# EUROPEAN PATENT APPLICATION

(11) **EP 4 480 950 A1**
(43) Date of publication of application: **25.12.2024**
(21) Application number: 23796527.2
(22) Date of filing: 27.04.2023
(51) Int. Cl.: C07D 401/04, A01N 43/50, A01N 43/56, A01N 43/653, A01N 43/78, A01P 13/00, C07D 249/08, C07D 417/04

(54) **NITROGEN-CONTAINING HETEROCYCLIC COMPOUND HAVING OXIME GROUP, AGRICULTURAL/HORTICULTURAL HERBICIDE CONTAINING SAID COMPOUND, AND USE OF BOTH OF FOREGOING**

(30) Priority: 28.04.2022 JP 2022075388
(71) Applicant: Nihon Nohyaku Co., Ltd., Tokyo 104-8386 (JP)
(72) Inventor: INOUE, Hayato, Kawachinagano-shi, Osaka 586-0094 (JP); SANO, Yusuke, Kawachinagano-shi, Osaka 586-0094 (JP); MORITA, Tomoya, Kawachinagano-shi, Osaka 586-0094 (JP); HIRAIDE, Yuto, Kawachinagano-shi, Osaka 586-0094 (JP); MATSUMOTO, Seitaro, Kawachinagano-shi, Osaka 586-0094 (JP); SUDA, Yusuke, Kawachinagano-shi, Osaka 586-0094 (JP)
(74) Representative: Witte, Weller & Partner Patentanwälte mbB
(86) International application number: PCT/JP2023/016778
(87) International publication number: WO 2023/210792

(57) **Abstract**

The problem to be solved is to provide a novel herbicide having both high safety for crops and excellent herbicidal activity against weeds in order to resolve the food crisis that is anticipated to come in the near future due to global population growth.

The problem is solved by the compound represented by the following general formula (1): 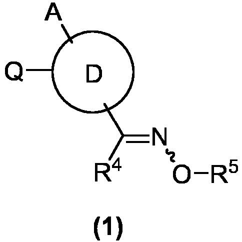 or a salt thereof, an agricultural or horticultural herbicide comprising the compound or the salt thereof as an active ingredient, and a method for using the herbicide.

## Description

### TECHNICAL FIELD

The present invention relates to a nitrogen-containing heterocyclic compound having an oxime group and a salt thereof, an agricultural or horticultural herbicide comprising the compound or the salt thereof as an active ingredient, and a method for using the compound or the salt thereof or the herbicide.

### BACKGROUND ART

Patent literature 1 describes certain kinds of nitrogen-containing condensed heterocyclic compounds having an oxime group that have insecticidal activity. However, the literature does not describe the specific structure of the compound of the present invention, nor does it disclose or suggest any compounds useful as herbicides.

### CITATION LIST

### Patent Literature

Patent Literature 1: WO 2017/065183

### SUMMARY OF INVENTION

### TECHNICAL PROBLEM

A stable and secure food supply is essential to resolve the food crisis that is anticipated to come in the near future due to global population growth. The stable and secure food supply requires economical and efficient elimination or control of weeds that interfere with crop cultivation and harvest. Therefore, it is becoming increasingly important to develop new herbicides and plant growth regulators that can provide solutions to this problem. In order to respond to such social demands, the present invention is intended to provide a novel herbicide having both high safety for crops and excellent herbicidal activity against weeds. In addition, in view of aging of farmers, there is a demand for various kinds of labor-saving application methods and for the creation of agricultural or horticultural herbicides suitable for such application methods.

### SOLUTION TO PROBLEM

After conducting extensive research to develop a novel agricultural or horticultural herbicide, the present inventors found that the nitrogen-containing heterocyclic compound having an oxime group represented by the general formula (1) of the present invention or a salt thereof is useful as an agricultural or horticultural herbicide. Based on this finding, the present inventors completed the present invention.

That is, the present invention includes the following.
[1] A compound represented by the general formula (1): 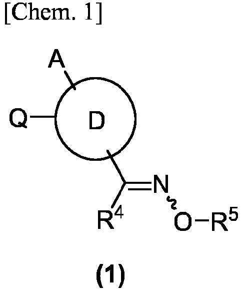 {wherein Q represents a group represented by the following Q1, Q2, Q3, Q4, Q5, Q6, Q7, Q8, Q9, Q10, Q11, Q12, Q13, Q14, Q15, Q16, Q17, or Q18: 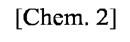 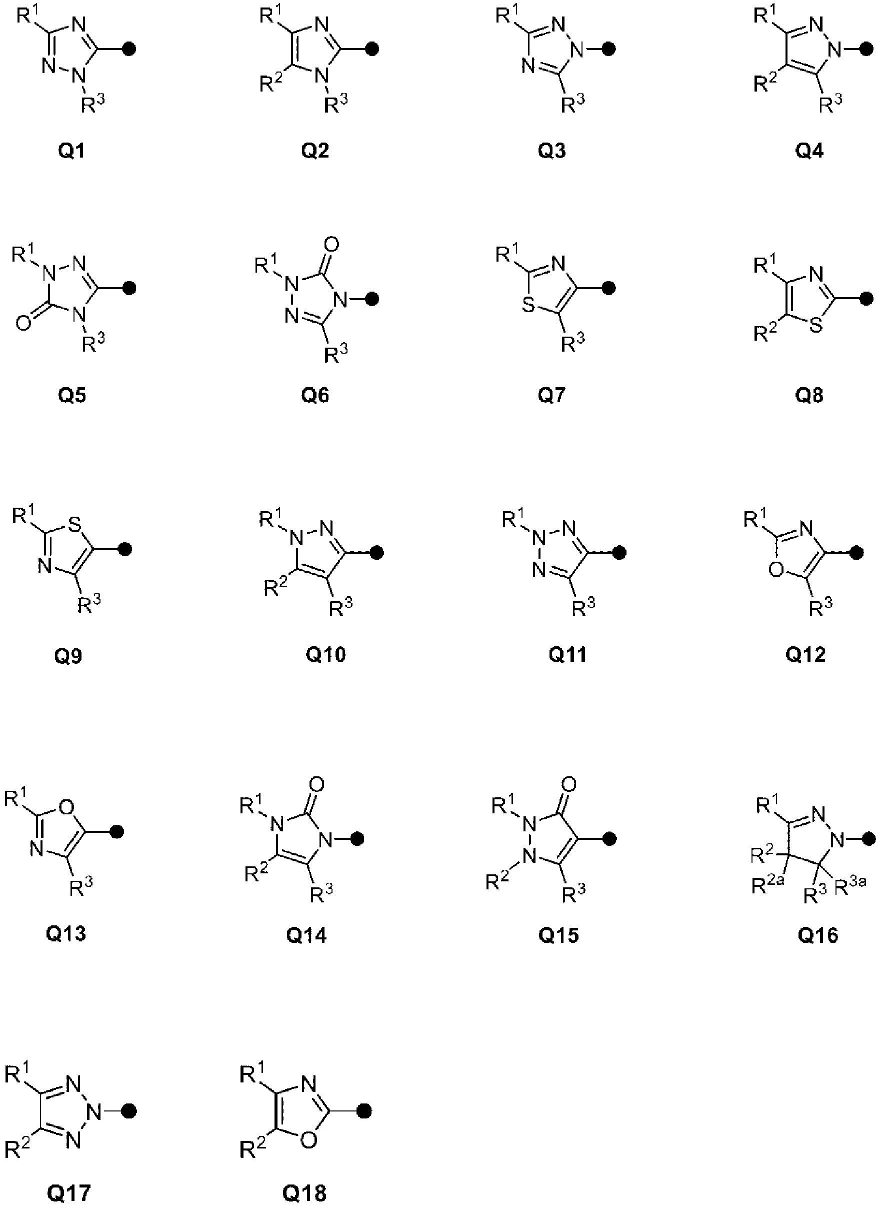 (wherein R¹ represents
   (a1) a halogen atom;
   (a2) a (C₁-C₆) alkyl group;
   (a3) a (C₂-C₆) alkenyl group;
   (a4) a (C₂-C₆) alkynyl group;
   (a5) a (C₃-C₆) cycloalkyl group;
   (a6) a halo (C₁-C₆) alkyl group;
   (a7) a halo (C₂-C₆) alkenyl group;
   (a8) a halo (C₂-C₆) alkynyl group;
   (a9) a halo (C₃-C₆) cycloalkyl group;
   (a10) a dioxolanyl group;
   (a11) a dioxanyl group;
   (a12) a dioxepanyl group;
   (a13) a dihydropyranyl group;
   (a14) a tetrahydropyranyl group;
   (a15) a tetrahydrothiopyranyl group;
   (a16) a piperidinyl group;
   (a17) a substituted piperidinyl group having 1 to 3 substituents on the ring, each independently selected from the group consisting of a (C₁-C₆) alkyl group, a (C₁-C₆) alkylcarbonyl group, and a (C₁-C₆) alkoxycarbonyl group;
   (a18) a thienyl group;
   (a19) a substituted thienyl group having 1 to 3 substituents on the ring, each independently selected from a set Y of substituents;
   (a20) a thiazolyl group;
   (a21) a substituted thiazolyl group having 1 or 2 substituents on the ring, each independently selected from the set Y of substituents;
   (a22) a thiadiazolyl group;
   (a23) a substituted thiadiazolyl group having one substituent on the ring, selected from the set Y of substituents;
   (a24) a pyrazolyl group;
   (a25) a substituted pyrazolyl group having 1 to 3 substituents on the ring, each independently selected from the set Y of substituents;
   (a26) a phenyl group;
   (a27) a substituted phenyl group having 1 to 5 substituents on the ring, each independently selected from the set Y of substituents;
   (a28) a pyridyl group;
   (a29) a substituted pyridyl group having 1 to 4 substituents on the ring, each independently selected from the set Y of substituents;
   (a30) a pyridazinyl group;
   (a31) a substituted pyridazinyl group having 1 to 3 substituents on the ring, each independently selected from the set Y of substituents;
   (a32) a pyrimidinyl group;
   (a33) a substituted pyrimidinyl group having 1 to 3 substituents on the ring, each independently selected from the set Y of substituents;
   (a34) a pyrazinyl group;
   (a35) a substituted pyrazinyl group having 1 to 3 substituents on the ring, each independently selected from the set Y of substituents;
   (a36) an amino group;
   (a37) a carboxyl group;
   (a38) a (C₁-C₆) alkoxy group;
   (a39) a halo (C₁-C₆) alkoxy group;
   (a40) a (C₁-C₆) alkylsulfanyl group;
   (a41) a (C₁-C₆) alkylsulfinyl group;
   (a42) a (C₁-C₆) alkylsulfonyl group;
   (a43) a (C₃-C₆) cycloalkenyl group;
   (a44) a (C₁-C₆) alkoxy (C₁-C₆) alkyl group;
   (a45) a (C₁-C₆) alkylsulfanyl (C₁-C₆) alkyl group;
   (a46) a (C₁-C₆) alkylsulfinyl (C₁-C₆) alkyl group;
   (a47) a (C₁-C₆) alkylsulfonyl (C₁-C₆) alkyl group;
   (a48) a (C₁-C₆) alkylcarbonyl group;
   (a49) a (C₁-C₆) alkoxycarbonyl group;
   (a50) a halo (C₁-C₆) alkylcarbonyl group;
   (a51) a halo (C₁-C₆) alkoxycarbonyl group;
   (a52) a phenylcarbonyl group;
   (a53) a substituted phenylcarbonyl group having 1 to 5 substituents on the ring, each independently selected from the set Y of substituents;
   (a54) a phenylaminocarbonyl group;
   (a55) a substituted phenylaminocarbonyl group having 1 to 5 substituents on the ring, each independently selected from the set Y of substituents;
   (a56) a benzhydrylidene amino group;
   (a57) an oxopyrrolidinyl group;
   (a58) an oxopyridyl group;
   (a59) a substituted dioxolanyl group having 1 to 4 substituents on the ring, each independently selected from the group consisting of a halogen atom and a (C₁-C₆) alkyl group;
   (a60) a substituted dioxanyl group having 1 to 4 substituents on the ring, each independently selected from the group consisting of a halogen atom and a (C₁-C₆) alkyl group;
   (a61) a furanyl group;
   (a62) a substituted furanyl group having 1 to 3 substituents on the ring, each independently selected from the set Y of substituents;
   (a63) an isothiazolyl group;
   (a64) a substituted isothiazolyl group having 1 or 2 substituents on the ring, each independently selected from the set Y of substituents;
   (a65) an oxazolyl group;
   (a66) a substituted oxazolyl group having 1 or 2 substituents on the ring, each independently selected from the set Y of substituents;
   (a67) an isoxazolyl group;
   (a68) a substituted isoxazolyl group having 1 or 2 substituents on the ring, each independently selected from the set Y of substituents;
   (a69) a quinolinyl group;
   (a70) a substituted quinolinyl group having 1 to 6 substituents on the ring, each independently selected from the set Y of substituents;
   (a71) a benzothienyl group;
   (a72) a substituted benzothienyl group having 1 to 5 substituents on the ring, each independently selected from the set Y of substituents;
   (a73) a phenoxy group;
   (a74) a substituted phenoxy group having 1 to 5 substituents on the ring, each independently selected from the set Y of substituents;
   (a75) a phenyl (C₁-C₆) alkyl group;
   (a76) a substituted phenyl (C₁-C₆) alkyl group having 1 to 5 substituents on the ring, each independently selected from the set Y of substituents;
   (a77) a phenyl (C₁-C₆) alkoxy group;
   (a78) a substituted phenyl (C₁-C₆) alkoxy group having 1 to 5 substituents on the ring, each independently selected from the set Y of substituents;
   (a79) a (C₁-C₆) alkylamino group;
   (a80) a halo (C₁-C₆) alkoxycarbonylamino group;
   (a81) an oxazolidinonyl group;
   (a82) a (C₁-C₆) alkoxy (C₁-C₆) alkoxy group;
   (a83) a tetrahydrofuranyl (C₁-C₆) alkoxy group; or
   (a84) a (C₃-C₆) cycloalkyl (C₁-C₆) alkoxy group,

   R² represents
      (b1) a hydrogen atom;
      (b2) a halogen atom; or
      (b3) a (C₁-C₆) alkyl group,
   R^{2a} represents
      (b1') a hydrogen atom; or
      (b2') a (C₁-C₆) alkyl group,
   R³ represents
      (c1) a hydrogen atom;
      (c2) a halogen atom;
      (c3) a hydroxy group;
      (c4) a (C₁-C₆) alkyl group;
      (c5) a (C₂-C₆) alkenyl group;
      (c6) a (C₂-C₆) alkynyl group;
      (c7) a (C₃-C₆) cycloalkyl group;
      (c8) a halo (C₁-C₆) alkyl group;
      (c9) a halo (C₂-C₆) alkenyl group;
      (c10) a halo (C₂-C₆) alkynyl group;
      (c11) a halo (C₃-C₆) cycloalkyl group;
      (c12) a (C₁-C₆) alkoxy group;
      (c13) a (C₁-C₆) alkylsulfanyl group;
      (c14) a (C₁-C₆) alkylsulfinyl group;
      (c15) a (C₁-C₆) alkylsulfonyl group;
      (c16) a halo (C₁-C₆) alkoxy group;
      (c17) a halo (C₁-C₆) alkylsulfanyl group;
      (c18) a halo (C₁-C₆) alkylsulfinyl group;
      (c19) a halo (C₁-C₆) alkylsulfonyl group;
      (c20) a (C₁-C₆) alkoxycarbonyl group; or
      (c21) a (C₁-C₆) alkylamino group, or
      R² and R³ may be bound to each other to form a 5- or 6-membered ring,
   R^{3a} represents
      (c1') a hydrogen atom; or
      (c2') a (C₁-C₆) alkyl group,
   the set Y of substituents consists of
      (d1) a halogen atom;
      (d2) a cyano group;
      (d3) a nitro group;
      (d4) an amino group;
      (d5) a (C₁-C₆) alkyl group;
      (d6) a (C₂-C₆) alkenyl group;
      (d7) a (C₂-C₆) alkynyl group;
      (d8) a (C₃-C₆) cycloalkyl group;
      (d9) a halo (C₁-C₆) alkyl group;
      (d10) a halo (C₂-C₆) alkenyl group;
      (d11) a halo (C₂-C₆) alkynyl group;
      (d12) a halo (C₃-C₆) cycloalkyl group;
      (d13) a (C₁-C₆) alkoxy group;
      (d14) a (C₁-C₆) alkylsulfanyl group;
      (d15) a (C₁-C₆) alkylsulfinyl group;
      (d16) a (C₁-C₆) alkylsulfonyl group;
      (d17) a halo (C₁-C₆) alkoxy group;
      (d18) a halo (C₁-C₆) alkylsulfanyl group;
      (d19) a halo (C₁-C₆) alkylsulfinyl group;
      (d20) a halo (C₁-C₆) alkylsulfonyl group;
      (d21) an N-((C₁-C₆) alkylcarbonyl)amino group;
      (d22) an N,N-di-((C₁-C₆) alkylcarbonyl)amino group wherein the (C₁-C₆) alkylcarbonyl groups may be the same or different;
      (d23) a di-(C₁-C₆) alkoxyphosphanyl group wherein the (C₁-C₆) alkoxy groups may be the same or different;
      (d24) an imidazolyl group;
      (d25) an imidazolyl group having 1 to 3 substituents on the ring, each independently selected from the group consisting of a halogen atom and a (C₁-C₆) alkyl group;
      (d26) a phenyl group;
      (d27) a phenyl group having 1 to 5 substituents on the ring, each independently selected from the group consisting of a halogen atom and a (C₁-C₆) alkyl group;
      (d28) a pyridyl group;
      (d29) a pyridyl group having 1 to 4 substituents on the ring, each independently selected from the group consisting of a halogen atom and a (C₁-C₆) alkyl group;
      (d30) a di-(C₁-C₆) alkylamino group;
      (d31) a (C₁-C₆) alkoxycarbonyl group; and
      (d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group, and
   each solid circle represents the position of binding to D),
   R⁴ represents
      (e1) a hydrogen atom;
      (e2) an amino group;
      (e3) a (C₁-C₆) alkyl group;
      (e4) an N-(C₁-C₆) alkylamino group;
      (e5) an N,N-di-(C₁-C₆) alkylamino group wherein the (C₁-C₆) alkyl groups may be the same or different;
      (e6) an N-((C₁-C₆) alkylcarbonyl)amino group;
      (e7) an N,N-di-((C₁-C₆) alkylcarbonyl)amino group wherein the (C₁-C₆) alkylcarbonyl groups may be the same or different;
      (e8) an N-(halo (C₁-C₆) alkylcarbonyl)amino group;
      (e9) an N-((C₁-C₆) alkoxycarbonyl)amino group; or
      (e10) an N-(C₁-C₆) alkyl-N-((C₁-C₆) alkylcarbonyl)amino group,
   R⁵ represents
      (f1) a (C₁-C₆) alkyl group;
      (f2) a (C₂-C₆) alkenyl group;
      (f3) a (C₂-C₆) alkynyl group;
      (f4) a (C₃-C₆) cycloalkyl group;
      (f5) a halo (C₁-C₆) alkyl group;
      (f6) a halo (C₂-C₆) alkenyl group;
      (f7) a halo (C₂-C₆) alkynyl group;
      (f8) a halo (C₃-C₆) cycloalkyl group;
      (f9) a (C₃-C₆) cycloalkyl (C₁-C₆) alkyl group;
      (f10) a (C₁-C₆) alkoxy (C₁-C₆) alkyl group;
      (f11) a (C₁-C₆) alkylsulfanyl (C₁-C₆) alkyl group;
      (f12) a (C₁-C₆) alkylsulfinyl (C₁-C₆) alkyl group;
      (f13) a (C₁-C₆) alkylsulfonyl (C₁-C₆) alkyl group; or
      (f14) a phenyl (C₁-C₆) alkyl group,
   A represents
      (g1) a (C₁-C₆) alkoxy group;
      (g2) a halo (C₁-C₆) alkoxy group;
      (g3) an N-(C₁-C₆) alkylaminocarbonyl group;
      (g4) an N,N-di-(C₁-C₆) alkylaminocarbonyl group wherein the (C₁-C₆) alkylcarbonyl groups may be the same or different;
      (g5) CO₂R⁷ wherein R⁷ represents
         (h1) a hydrogen atom,
         (h2) a (C₁-C₆) alkyl group,
         (h3) a (C₂-C₆) alkenyl group,
         (h4) a (C₂-C₆) alkynyl group,
         (h5) a (C₃-C₆) cycloalkyl group,
         (h6) a halo (C₁-C₆) alkyl group,
         (h7) a halo (C₂-C₆) alkenyl group,
         (h8) a halo (C₂-C₆) alkynyl group,
         (h9) a halo (C₃-C₆) cycloalkyl group, or
         (h10) a phenyl (C₁-C₆) alkyl group;
      (g6) SOₙR⁸ wherein R⁸ represents
         (i1) a (C₁-C₆) alkyl group,
         (i2) a (C₂-C₆) alkenyl group,
         (i3) a (C₂-C₆) alkynyl group,
         (i4) a (C₃-C₆) cycloalkyl group,
         (i5) a halo (C₁-C₆) alkyl group,
         (i6) a halo (C₂-C₆) alkenyl group,
         (i7) a halo (C₂-C₆) alkynyl group,
         (i8) a halo (C₃-C₆) cycloalkyl group, or
         (i9) a (C₁-C₆) alkylcarbonyl (C₁-C₆) alkyl group, and
         n represents 0, 1, or 2; or
      (g7) SO₂N(R⁹)R¹⁰ wherein R⁹ and R¹⁰ represent
         (j 1) a hydrogen atom,
         (j2) a (C₁-C₆) alkyl group,
         (j3) a (C₂-C₆) alkenyl group,
         (j4) a (C₂-C₆) alkynyl group,
         (j5) a (C₃-C₆) cycloalkyl group,
         (j6) a halo (C₁-C₆) alkyl group,
         (j7) a halo (C₂-C₆) alkenyl group,
         (j8) a halo (C₂-C₆) alkynyl group,
         (j9) a halo (C₃-C₆) cycloalkyl group,
         (j10) a phenyl (C₁-C₆) alkyl group
         (j 11) a (C₁-C₆) alkylcarbonyl group,
         (j12) a (C₁-C₆) alkoxycarbonyl group,
         (j13) a halo (C₁-C₆) alkylcarbonyl group, or
         (j14) a halo (C₁-C₆) alkoxycarbonyl group, and
         R⁹ and R¹⁰ may be the same or different, and
         D substituted with A, Q, and C(R⁴)=N~OR⁵ represents a ring represented by the following D1, D2, D3, or D4: 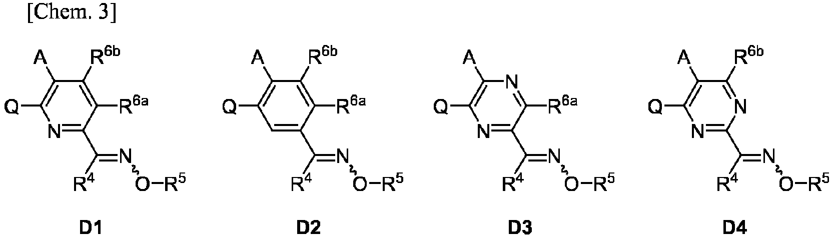 wherein R^{6a} and R^{6b} represent
            (k1) a hydrogen atom;
            (k2) a halogen atom; or
            (k3) a (C₁-C₆) alkyl group, and
            R^{6a} and R^{6b} may be the same or different}, or
      a salt thereof.
[2] The compound or the salt thereof according to the above [1], wherein
   Q and D are as defined in the above [1],
   R¹ is
      (a1) a halogen atom;
      (a2) a (C₁-C₆) alkyl group;
      (a3) a (C₂-C₆) alkenyl group;
      (a5) a (C₃-C₆) cycloalkyl group;
      (a9) a halo (C₃-C₆) cycloalkyl group;
      (a10) a dioxolanyl group;
      (a11) a dioxanyl group;
      (a12) a dioxepanyl group;
      (a13) a dihydropyranyl group;
      (a14) a tetrahydropyranyl group;
      (a15) a tetrahydrothiopyranyl group;
      (a16) a piperidinyl group;
      (a17) a substituted piperidinyl group having 1 to 3 substituents on the ring, each independently selected from the group consisting of a (C₁-C₆) alkyl group, a (C₁-C₆) alkylcarbonyl group, and a (C₁-C₆) alkoxycarbonyl group;
      (a18) a thienyl group;
      (a19) a substituted thienyl group having 1 to 3 substituents on the ring, each independently selected from a set Y of substituents;
      (a20) a thiazolyl group;
      (a21) a substituted thiazolyl group having 1 or 2 substituents on the ring, each independently selected from the set Y of substituents;
      (a22) a thiadiazolyl group;
      (a23) a substituted thiadiazolyl group having one substituent on the ring, selected from the set Y of substituents;
      (a24) a pyrazolyl group;
      (a25) a substituted pyrazolyl group having 1 to 3 substituents on the ring, each independently selected from the set Y of substituents;
      (a26) a phenyl group;
      (a27) a substituted phenyl group having 1 to 5 substituents on the ring, each independently selected from the set Y of substituents;
      (a28) a pyridyl group;
      (a29) a substituted pyridyl group having 1 to 4 substituents on the ring, each independently selected from the set Y of substituents;
      (a30) a pyridazinyl group;
      (a31) a substituted pyridazinyl group having 1 to 3 substituents on the ring, each independently selected from the set Y of substituents;
      (a32) a pyrimidinyl group;
      (a33) a substituted pyrimidinyl group having 1 to 3 substituents on the ring, each independently selected from the set Y of substituents;
      (a34) a pyrazinyl group;
      (a35) a substituted pyrazinyl group having 1 to 3 substituents on the ring, each independently selected from the set Y of substituents;
      (a36) an amino group;
      (a37) a carboxyl group;
      (a38) a (C₁-C₆) alkoxy group;
      (a39) a halo (C₁-C₆) alkoxy group;
      (a40) a (C₁-C₆) alkylsulfanyl group;
      (a41) a (C₁-C₆) alkylsulfinyl group;
      (a42) a (C₁-C₆) alkylsulfonyl group;
      (a43) a (C₃-C₆) cycloalkenyl group;
      (a44) a (C₁-C₆) alkoxy (C₁-C₆) alkyl group;
      (a45) a (C₁-C₆) alkylsulfanyl (C₁-C₆) alkyl group;
      (a46) a (C₁-C₆) alkylsulfinyl (C₁-C₆) alkyl group;
      (a47) a (C₁-C₆) alkylsulfonyl (C₁-C₆) alkyl group;
      (a48) a (C₁-C₆) alkylcarbonyl group;
      (a49) a (C₁-C₆) alkoxycarbonyl group;
      (a50) a halo (C₁-C₆) alkylcarbonyl group;
      (a51) a halo (C₁-C₆) alkoxycarbonyl group;
      (a52) a phenylcarbonyl group;
      (a53) a substituted phenylcarbonyl group having 1 to 5 substituents on the ring, each independently selected from the set Y of substituents;
      (a54) a phenylaminocarbonyl group;
      (a55) a substituted phenylaminocarbonyl group having 1 to 5 substituents on the ring, each independently selected from the set Y of substituents;
      (a56) a benzhydrylidene amino group;
      (a57) an oxopyrrolidinyl group;
      (a58) an oxopyridyl group;
      (a59) a substituted dioxolanyl group having 1 to 4 substituents on the ring, each independently selected from the group consisting of a halogen atom and a (C₁-C₆) alkyl group;
      (a60) a substituted dioxanyl group having 1 to 4 substituents on the ring, each independently selected from the group consisting of a halogen atom and a (C₁-C₆) alkyl group;
      (a61) a furanyl group;
      (a62) a substituted furanyl group having 1 to 3 substituents on the ring, each independently selected from the set Y of substituents;
      (a63) an isothiazolyl group;
      (a64) a substituted isothiazolyl group having 1 or 2 substituents on the ring, each independently selected from the set Y of substituents;
      (a69) a quinolinyl group;
      (a70) a substituted quinolinyl group having 1 to 6 substituents on the ring, each independently selected from the set Y of substituents;
      (a71) a benzothienyl group;
      (a72) a substituted benzothienyl group having 1 to 5 substituents on the ring, each independently selected from the set Y of substituents;
      (a73) a phenoxy group;
      (a74) a substituted phenoxy group having 1 to 5 substituents on the ring, each independently selected from the set Y of substituents;
      (a75) a phenyl (C₁-C₆) alkyl group;
      (a76) a substituted phenyl (C₁-C₆) alkyl group having 1 to 5 substituents on the ring, each independently selected from the set Y of substituents;
      (a77) a phenyl (C₁-C₆) alkoxy group;
      (a78) a substituted phenyl (C₁-C₆) alkoxy group having 1 to 5 substituents on the ring, each independently selected from the set Y of substituents;
      (a79) a (C₁-C₆) alkylamino group;
      (a80) a halo (C₁-C₆) alkoxycarbonylamino group;
      (a81) an oxazolidinonyl group;
      (a82) a (C₁-C₆) alkoxy (C₁-C₆) alkoxy group;
      (a83) a tetrahydrofuranyl (C₁-C₆) alkoxy group; or
      (a84) a (C₃-C₆) cycloalkyl (C₁-C₆) alkoxy group,
   R² is
      (b1) a hydrogen atom;
      (b2) a halogen atom; or
      (b3) a (C₁-C₆) alkyl group,
   R^{2a} is
      (b1') a hydrogen atom; or
      (b2') a (C₁-C₆) alkyl group,
   R³ is
      (c1) a hydrogen atom;
      (c2) a halogen atom;
      (c3) a hydroxy group;
      (c4) a (C₁-C₆) alkyl group;
      (c5) a (C₂-C₆) alkenyl group;
      (c6) a (C₂-C₆) alkynyl group;
      (c7) a (C₃-C₆) cycloalkyl group;
      (c8) a halo (C₁-C₆) alkyl group;
      (c12) a (C₁-C₆) alkoxy group;
      (c13) a (C₁-C₆) alkylsulfanyl group;
      (c14) a (C₁-C₆) alkylsulfinyl group;
      (c15) a (C₁-C₆) alkylsulfonyl group;
      (c16) a halo (C₁-C₆) alkoxy group;
      (c17) a halo (C₁-C₆) alkylsulfanyl group;
      (c18) a halo (C₁-C₆) alkylsulfinyl group;
      (c19) a halo (C₁-C₆) alkylsulfonyl group;
      (c20) a (C₁-C₆) alkoxycarbonyl group; or
      (c21) a (C₁-C₆) alkylamino group, or
      R² and R³ may be bound to each other to form a 5- or 6-membered ring,
   R^{3a} is
      (c1') a hydrogen atom; or
      (c2') a (C₁-C₆) alkyl group,
   the set Y of substituents consists of
      (d1) a halogen atom;
      (d2) a cyano group;
      (d3) a nitro group;
      (d4) an amino group;
      (d5) a (C₁-C₆) alkyl group;
      (d8) a (C₃-C₆) cycloalkyl group;
      (d9) a halo (C₁-C₆) alkyl group;
      (d13) a (C₁-C₆) alkoxy group;
      (d14) a (C₁-C₆) alkylsulfanyl group;
      (d15) a (C₁-C₆) alkylsulfinyl group;
      (d16) a (C₁-C₆) alkylsulfonyl group;
      (d17) a halo (C₁-C₆) alkoxy group;
      (d18) a halo (C₁-C₆) alkylsulfanyl group;
      (d19) a halo (C₁-C₆) alkylsulfinyl group;
      (d20) a halo (C₁-C₆) alkylsulfonyl group;
      (d21) an N-((C₁-C₆) alkylcarbonyl)amino group;
      (d22) an N,N-di-((C₁-C₆) alkylcarbonyl)amino group wherein the (C₁-C₆) alkylcarbonyl groups may be the same or different;
      (d23) a di-(C₁-C₆) alkoxyphosphanyl group wherein the (C₁-C₆) alkoxy groups may be the same or different;
      (d24) an imidazolyl group;
      (d25) an imidazolyl group having 1 to 3 substituents on the ring, each independently selected from the group consisting of a halogen atom and a (C₁-C₆) alkyl group;
      (d26) a phenyl group;
      (d27) a phenyl group having 1 to 5 substituents on the ring, each independently selected from the group consisting of a halogen atom and a (C₁-C₆) alkyl group;
      (d28) a pyridyl group;
      (d29) a pyridyl group having 1 to 4 substituents on the ring, each independently selected from the group consisting of a halogen atom and a (C₁-C₆) alkyl group;
      (d30) a di-(C₁-C₆) alkylamino group;
      (d31) a (C₁-C₆) alkoxycarbonyl group; and
      (d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group,
   R⁴ is
      (e1) a hydrogen atom;
      (e2) an amino group;
      (e3) a (C₁-C₆) alkyl group;
      (e4) an N-(C₁-C₆) alkylamino group;
      (e5) an N,N-di-(C₁-C₆) alkylamino group wherein the (C₁-C₆) alkyl groups may be the same or different;
      (e6) an N-((C₁-C₆) alkylcarbonyl)amino group;
      (e7) an N,N-di-((C₁-C₆) alkylcarbonyl)amino group wherein the (C₁-C₆) alkylcarbonyl groups may be the same or different; or
      (e8) an N-(halo (C₁-C₆) alkylcarbonyl)amino group,
   R⁵ is
      (f1) a (C₁-C₆) alkyl group;
      (f2) a (C₂-C₆) alkenyl group;
      (f3) a (C₂-C₆) alkynyl group;
      (f4) a (C₃-C₆) cycloalkyl group;
      (f5) a halo (C₁-C₆) alkyl group;
      (f9) a (C₃-C₆) cycloalkyl (C₁-C₆) alkyl group;
      (f10) a (C₁-C₆) alkoxy (C₁-C₆) alkyl group;
      (f11) a (C₁-C₆) alkylsulfanyl (C₁-C₆) alkyl group;
      (f12) a (C₁-C₆) alkylsulfinyl (C₁-C₆) alkyl group; or
      (f13) a (C₁-C₆) alkylsulfonyl (C₁-C₆) alkyl group,
   A is
      (g1) a (C₁-C₆) alkoxy group;
      (g3) an N-(C₁-C₆) alkylaminocarbonyl group;
      (g4) an N,N-di-(C₁-C₆) alkylaminocarbonyl group wherein the (C₁-C₆) alkylcarbonyl groups may be the same or different;
      (g5') CO₂R⁷ wherein R⁷ represents
         (h1) a hydrogen atom,
         (h2) a (C₁-C₆) alkyl group,
         (h3) a (C₂-C₆) alkenyl group,
         (h4) a (C₂-C₆) alkynyl group,
         (h5) a (C₃-C₆) cycloalkyl group,
         (h6) a halo (C₁-C₆) alkyl group, or
         (h10) a phenyl (C₁-C₆) alkyl group;
      (g6') SOₙR⁸ wherein R⁸ represents
         (i1) a (C₁-C₆) alkyl group,
         (i2) a (C₂-C₆) alkenyl group,
         (i3) a (C₂-C₆) alkynyl group,
         (i4) a (C₃-C₆) cycloalkyl group,
         (i5) a halo (C₁-C₆) alkyl group, or
         (i9) a (C₁-C₆) alkylcarbonyl (C₁-C₆) alkyl group, and
         n represents 0, 1, or 2; or
      (g7') SO₂N(R⁹)R¹⁰ wherein R⁹ and R¹⁰ represent
         (j 1) a hydrogen atom,
         (j2) a (C₁-C₆) alkyl group,
         (j3) a (C₂-C₆) alkenyl group,
         (j4) a (C₂-C₆) alkynyl group,
         (j5) a (C₃-C₆) cycloalkyl group,
         (j 11) a (C₁-C₆) alkylcarbonyl group,
         (j12) a (C₁-C₆) alkoxycarbonyl group,
         (j13) a halo (C₁-C₆) alkylcarbonyl group, or
         (j14) a halo (C₁-C₆) alkoxycarbonyl group, and
         R⁹ and R¹⁰ may be the same or different, and
         R^{6a} and R^{6b} are each
      (k1) a hydrogen atom;
      (k2) a halogen atom; or
      (k3) a (C₁-C₆) alkyl group, and
      R^{6a} and R^{6b} may be the same or different.
[3] The compound or the salt thereof according to the above [1], wherein
   Q and D are as defined in the above [1],
   R¹ is
      (a1) a halogen atom;
      (a2) a (C₁-C₆) alkyl group;
      (a3) a (C₂-C₆) alkenyl group;
      (a5) a (C₃-C₆) cycloalkyl group;
      (a13) a dihydropyranyl group;
      (a14) a tetrahydropyranyl group;
      (a16) a piperidinyl group;
      (a18) a thienyl group;
      (a19) a substituted thienyl group having 1 to 3 substituents on the ring, each independently selected from a set Y of substituents;
      (a20) a thiazolyl group;
      (a21) a substituted thiazolyl group having 1 or 2 substituents on the ring, each independently selected from the set Y of substituents;
      (a22) a thiadiazolyl group;
      (a23) a substituted thiadiazolyl group having one substituent on the ring, selected from the set Y of substituents;
      (a24) a pyrazolyl group;
      (a25) a substituted pyrazolyl group having 1 to 3 substituents on the ring, each independently selected from a set Y of substituents;
      (a26) a phenyl group;
      (a27) a substituted phenyl group having 1 to 5 substituents on the ring, each independently selected from the set Y of substituents;
      (a28) a pyridyl group;
      (a29) a substituted pyridyl group having 1 to 4 substituents on the ring, each independently selected from the set Y of substituents;
      (a32) a pyrimidinyl group;
      (a34) a pyrazinyl group;
      (a36) an amino group;
      (a37) a carboxyl group;
      (a38) a (C₁-C₆) alkoxy group;
      (a39) a halo (C₁-C₆) alkoxy group;
      (a40) a (C₁-C₆) alkylsulfanyl group;
      (a42) a (C₁-C₆) alkylsulfonyl group;
      (a43) a (C₃-C₆) cycloalkenyl group;
      (a44) a (C₁-C₆) alkoxy (C₁-C₆) alkyl group;
      (a45) a (C₁-C₆) alkylsulfanyl (C₁-C₆) alkyl group;
      (a46) a (C₁-C₆) alkylsulfinyl (C₁-C₆) alkyl group;
      (a47) a (C₁-C₆) alkylsulfonyl (C₁-C₆) alkyl group;
      (a48) a (C₁-C₆) alkylcarbonyl group;
      (a49) a (C₁-C₆) alkoxycarbonyl group;
      (a52) a phenylcarbonyl group;
      (a54) a phenylaminocarbonyl group;
      (a56) a benzhydrylidene amino group;
      (a57) an oxopyrrolidinyl group;
      (a58) an oxopyridyl group;
      (a59) a substituted dioxolanyl group having 1 to 4 substituents on the ring, each independently selected from the group consisting of a halogen atom and a (C₁-C₆) alkyl group;
      (a60) a substituted dioxanyl group having 1 to 4 substituents on the ring, each independently selected from the group consisting of a halogen atom and a (C₁-C₆) alkyl group;
      (a61) a furanyl group;
      (a63) an isothiazolyl group;
      (a64) a substituted isothiazolyl group having 1 or 2 substituents on the ring, each independently selected from the set Y of substituents;
      (a69) a quinolinyl group;
      (a71) a benzothienyl group;
      (a74) a substituted phenoxy group having 1 to 5 substituents on the ring, each independently selected from the set Y of substituents;
      (a75) a phenyl (C₁-C₆) alkyl group;
      (a82) a (C₁-C₆) alkoxy (C₁-C₆) alkoxy group;
      (a83) a tetrahydrofuranyl (C₁-C₆) alkoxy group; or
      (a84) a (C₃-C₆) cycloalkyl (C₁-C₆) alkoxy group,
   R² is
      (b1) a hydrogen atom;
      (b2) a halogen atom; or
      (b3) a (C₁-C₆) alkyl group,
   R^{2a} is
      (b1') a hydrogen atom,
   R³ is
      (c1) a hydrogen atom;
      (c2) a halogen atom;
      (c3) a hydroxy group;
      (c4) a (C₁-C₆) alkyl group;
      (c5) a (C₂-C₆) alkenyl group;
      (c8) a halo (C₁-C₆) alkyl group;
      (c12) a (C₁-C₆) alkoxy group;
      (c13) a (C₁-C₆) alkylsulfanyl group;
      (c14) a (C₁-C₆) alkylsulfinyl group;
      (c15) a (C₁-C₆) alkylsulfonyl group; or
      (c20) a (C₁-C₆) alkoxycarbonyl group, or
      R² and R³ may be bound to each other to form a 5- or 6-membered ring,
   R^{3a} is
      (c1') a hydrogen atom,
   the set Y of substituents consists of
      (d1) a halogen atom;
      (d2) a cyano group;
      (d3) a nitro group;
      (d4) an amino group;
      (d5) a (C₁-C₆) alkyl group;
      (d8) a (C₃-C₆) cycloalkyl group;
      (d9) a halo (C₁-C₆) alkyl group;
      (d13) a (C₁-C₆) alkoxy group;
      (d14) a (C₁-C₆) alkylsulfanyl group;
      (d17) a halo (C₁-C₆) alkoxy group;
      (d21) an N-((C₁-C₆) alkylcarbonyl)amino group;
      (d24) an imidazolyl group;
      (d26) a phenyl group;
      (d28) a pyridyl group;
      (d30) a di-(C₁-C₆) alkylamino group;
      (d31) a (C₁-C₆) alkoxycarbonyl group; and
      (d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group,
   R⁴ is
      (e2) an amino group;
      (e3) a (C₁-C₆) alkyl group;
      (e6) an N-((C₁-C₆) alkylcarbonyl)amino group;
      (e7) an N,N-di-((C₁-C₆) alkylcarbonyl)amino group wherein the (C₁-C₆) alkylcarbonyl groups may be the same or different; or
      (e8) an N-(halo (C₁-C₆) alkylcarbonyl)amino group,
   R⁵ is
      (f1) a (C₁-C₆) alkyl group;
      (f5) a halo (C₁-C₆) alkyl group; or
      (f10) a (C₁-C₆) alkoxy (C₁-C₆) alkyl group,
   A is
      (g1) a (C₁-C₆) alkoxy group;
      (g4) an N,N-di-(C₁-C₆) alkylaminocarbonyl group wherein the (C₁-C₆) alkylcarbonyl groups may be the same or different;
      (g5") CO₂R⁷ wherein R⁷ represents
         (h1) a hydrogen atom, or
         (h2) a (C₁-C₆) alkyl group;
      (g6") SOₙR⁸ wherein R⁸ represents
         (i1) a (C₁-C₆) alkyl group, or
         (i9) a (C₁-C₆) alkylcarbonyl (C₁-C₆) alkyl group, and
         n represents 2; or
      (g7") SO₂N(R⁹)R¹⁰ wherein R⁹ and R¹⁰ represent
         (j 1) a hydrogen atom,
         (j2) a (C₁-C₆) alkyl group,
         (j 11) a (C₁-C₆) alkylcarbonyl group, or
         (j12) a (C₁-C₆) alkoxycarbonyl group, and
         R⁹ and R¹⁰ may be the same or different, and
         R^{6a} and R^{6b} are each
      (k1) a hydrogen atom.
[4] The compound or the salt thereof according to the above [1], wherein
   R¹, R², R³, R⁴, R⁵, R^{6a}, R^{6b}, A, and the set Y of substituents are as defined in the above [1],
   Q is Q1, Q2, Q3, Q4, Q5, Q6, Q7, Q8, Q9, or Q10, and
   D is D1 or D2.
[5] The compound or the salt thereof according to the above [1], wherein
   R¹, R², R³, R⁴, R⁵, R^{6a}, R^{6b}, A, and the set Y of substituents are as defined in the above [1],
   Q is Q1, Q2, Q3, Q4, Q5, Q6, Q7, Q8, or Q10, and
   D is D1.
[6] The compound or the salt thereof according to the above [2], wherein
   R¹, R², R³, R⁴, R⁵, R^{6a}, R^{6b}, A, and the set Y of substituents are as defined in the above [2],
   Q is Q1, Q2, Q3, Q4, Q5, Q6, Q7, Q8, Q9, or Q10, and
   D is D1 or D2.
[7] The compound or the salt thereof according to the above [2], wherein
   R¹, R², R³, R⁴, R⁵, R^{6a}, R^{6b}, A, and the set Y of substituents are as defined in the above [2],
   Q is Q1, Q2, Q3, Q4, Q5, Q6, Q7, Q8, or Q10, and
   D is D1.
[8] The compound or the salt thereof according to the above [3], wherein
   R¹, R², R³, R⁴, R⁵, R^{6a}, R^{6b}, A, and the set Y of substituents are as defined in the above [3],
   Q is Q1, Q2, Q3, Q4, Q5, Q6, Q7, Q8, Q9, or Q10, and
   D is D1 or D2.
[9] The compound or the salt thereof according to the above [3], wherein
   R¹, R², R³, R⁴, R⁵, R^{6a}, R^{6b}, A, and the set Y of substituents are as defined in the above [3],
   Q is Q1, Q2, Q3, Q4, Q5, Q6, Q7, Q8, or Q10, and
   D is D1.
[10] An agricultural or horticultural herbicide comprising the compound or the salt thereof according to any one of the above [1] to [9] as an active ingredient.
[11] A method for using an agricultural or horticultural herbicide, comprising treating weeds, soil, paddy fields, or growing media with an effective amount of the agricultural or horticultural herbicide according to the above [10].
[12] A method for controlling weeds, comprising treating weeds, soil, paddy fields, or growing media with an effective amount of the agricultural or horticultural herbicide according to the above [10].

### ADVANTAGEOUS EFFECTS OF INVENTION

The nitrogen-containing heterocyclic compound having an oxime group of the present invention or a salt thereof is a highly effective agricultural or horticultural herbicide.

### DESCRIPTION OF EMBODIMENTS

In the definitions of the general formula (1) representing the compound of the present invention, "halo" means "halogen atom" and is usually exemplified by a chlorine atom, a bromine atom, an iodine atom, or a fluorine atom.

The "(C₁-C₆) alkyl group" refers to a straight-chain or branched-chain alkyl group of 1 to 6 carbon atoms, for example, a methyl group, an ethyl group, a n-propyl group, an isopropyl group, a n-butyl group, an isobutyl group, a sec-butyl group, a tert-butyl group, a n-pentyl group, an isopentyl group, a tert-pentyl group, a neopentyl group, a 2,3-dimethylpropyl group, an 1-ethylpropyl group, a 1-methylbutyl group, a 2-methylbutyl group, a n-hexyl group, an isohexyl group, a 2-hexyl group, a 3-hexyl group, a 2-methylpentyl group, a 3-methylpentyl group, a 1,1,2-trimethyl propyl group, a 3,3-dimethylbutyl group or the like.

The "(C₂-C₆) alkenyl group" refers to a straight-chain or branched-chain alkenyl group of 2 to 6 carbon atoms, for example, a vinyl group, an allyl group, an isopropenyl group, a 1-butenyl group, a 2-butenyl group, a 2-methyl-2-propenyl group, a 1-methyl-2-propenyl group, a 2-methyl-1-propenyl group, a pentenyl group, a 1-hexenyl group, a 3,3-dimethyl-1-butenyl group or the like. The "(C₂-C₆) alkynyl group" refers to a straight-chain or branched-chain alkynyl group of 2 to 6 carbon atoms, for example, an ethynyl group, a 1-propynyl group, a 2-propynyl group, a 1-butynyl group, a 2-butynyl group, a 3-butynyl group, a 3-methyl-1-propynyl group, a 2-methyl-3-propynyl group, a pentynyl group, a 1-hexynyl group, a 3-methyl-1-butynyl group, a 3,3-dimethyl-1-butynyl group or the like.

The "(C₃-C₆) cycloalkyl group" refers to a cyclic alkyl group of 3 to 6 carbon atoms, for example, a cyclopropyl group, a cyclobutyl group, a cyclopentyl group, a cyclohexyl group or the like. The "(C₁-C₆) alkoxy group" refers to a straight-chain or branched-chain alkoxy group of 1 to 6 carbon atoms, for example, a methoxy group, an ethoxy group, a n-propoxy group, an isopropoxy group, a n-butoxy group, a sec-butoxy group, a tert-butoxy group, a n-pentyloxy group, an isopentyloxy group, a tert-pentyloxy group, a neopentyloxy group, a 2,3-dimethylpropyloxy group, an 1-ethylpropyloxy group, a 1-methylbutyloxy group, a n-hexyloxy group, an isohexyloxy group, a 1,1,2-trimethylpropyloxy group or the like. The "(C₂-C₆) alkenyloxy group" refers to a straight-chain or branched-chain alkenyloxy group of 2 to 6 carbon atoms, for example, a propenyloxy group, a butenyloxy group, a pentenyloxy group, a hexenyloxy group or the like. The "(C₂-C₆) alkynyloxy group" refers to a straight-chain or branched-chain alkynyloxy group of 2 to 6 carbon atoms, for example, a propynyloxy group, a butynyloxy group, a pentynyloxy group, a hexynyloxy group or the like.

The "(C₁-C₆) alkylsulfanyl group" refers to a straight-chain or branched-chain alkylsulfanyl group of 1 to 6 carbon atoms, for example, a methylthio group, an ethylthio group, a n-propylthio group, an isopropylthio group, a n-butylthio group, a sec-butylthio group, a tert-butylthio group, a n-pentylthio group, an isopentylthio group, a tert-pentylthio group, a neopentylthio group, a 2,3-dimethylpropylthio group, an 1-ethylpropylthio group, a 1-methylbutylthio group, a n-hexylthio group, an isohexylthio group, a 1,1,2-trimethylpropylthio group or the like.

The "(C₁-C₆) alkylsulfinyl group" refers to a straight-chain or branched-chain alkylsulfinyl group of 1 to 6 carbon atoms, for example, a methylsulfinyl group, an ethylsulfinyl group, a n-propylsulfinyl group, an isopropylsulfinyl group, a n-butylsulfinyl group, a sec-butylsulfinyl group, a tert-butylsulfinyl group, a n-pentylsulfinyl group, an isopentylsulfinyl group, a tert-pentylsulfinyl group, a neopentylsulfinyl group, a 2,3-dimethylpropylsulfinyl group, an 1-ethylpropylsulfinyl group, a 1-methylbutylsulfinyl group, a n-hexylsulfinyl group, an isohexylsulfinyl group, a 1,1,2-trimethylpropylsulfinyl group or the like.

The "(C₁-C₆) alkylsulfonyl group" refers to a straight-chain or branched-chain alkylsulfonyl group of 1 to 6 carbon atoms, for example, a methylsulfonyl group, an ethylsulfonyl group, a n-propylsulfonyl group, an isopropylsulfonyl group, a n-butylsulfonyl group, a sec-butylsulfonyl group, a tert-butylsulfonyl group, a n-pentylsulfonyl group, an isopentylsulfonyl group, a tert-pentylsulfonyl group, a neopentylsulfonyl group, a 2,3-dimethylpropylsulfonyl group, an 1-ethylpropylsulfonyl group, a 1-methylbutylsulfonyl group, a n-hexylsulfonyl group, an isohexylsulfonyl group, a 1,1,2-trimethylpropylsulfonyl group or the like.

The "(C₁-C₆) alkylcarbonyl group" refers to an alkylcarbonyl group of 2 to 7 carbon atoms, for example, an alkylcarbonyl group in which the alkyl group is a (C₁-C₆) alkyl group as defined above, such as an acetyl group, a propanoyl group, a butanoyl group, a 2-methylpropanoyl group, a pentanoyl group, a 2-methylbutanoyl group, a 3-methylbutanoyl group, a pivaloyl group, a hexanoyl group, or the like.

The "(C₁-C₆) alkylcarbonyloxy group" refers to an alkylcarbonyloxy group of 2 to 7 carbon atoms, for example, an alkylcarbonyloxy group in which the alkyl group is a (C₁-C₆) alkyl group as defined above, such as an acetyloxy group, a propanoyloxy group, a butanoyloxy group, a 2-methylpropanoyloxy group, a pentanoyloxy group, a 2-methylbutanoyloxy group, a 3-methylbutanoyloxy group, a pivaloyloxy group, a hexanoyloxy group, or the like.

The "N-(C₁-C₆) alkylaminocarbonyl group" refers to an alkylaminocarbonyl group of 2 to 7 carbon atoms in which the alkyl group is a straight-chain or branched-chain alkyl group of 1 to 6 carbon atoms, for example, an N-methylaminocarbonyl group, an N-ethylaminocarbonyl group, an N-n-propylaminocarbonyl group, an N-isopropylaminocarbonyl group, an N-n-butylaminocarbonyl group, an N-isobutylaminocarbonyl group, an N-sec-butylaminocarbonyl group, an N-tert-butylaminocarbonyl group, an N-n-pentylaminocarbonyl group, an N-isopentylaminocarbonyl group, an N-tert-pentylaminocarbonyl group, an N-neopentylaminocarbonyl group, an N-n-hexylaminocarbonyl group, an N-isohexylaminocarbonyl group, or the like.

The "N,N-di-(C₁-C₆) alkylaminocarbonyl group" refers to an dialkylaminocarbonyl group of 3 to 13 carbon atoms in which the alkyl groups are straight-chain or branched-chain alkyl groups of 1 to 6 carbon atoms, for example, N,N-dimethylaminocarbonyl group, N,N-diethylaminocarbonyl group, N,N-di-n-propylaminocarbonyl group, N,N-diisopropylaminocarbonyl group, N,N-di-n-butylaminocarbonyl group, N,N-di-sec-butylaminocarbonyl group, N,N-di-tert-butylaminocarbonyl group, N-methyl-N-ethylaminocarbonyl group, N-methyl-N-n-propylaminocarbonyl group, N-methyl-N-isopropylaminocarbonyl group, N-methyl-N-n-butylaminocarbonyl group, N-methyl-N-sec-butylaminocarbonyl group, N-methyl-N-tert-butylaminocarbonyl group, N-methyl-N-n-pentylaminocarbonyl group, N-methyl-N-isopentylaminocarbonyl group, N-methyl-N-tert-pentylaminocarbonyl group, N-methyl-N-neopentylaminocarbonyl group, N-methyl-N-(2,3-dimethylpropyl)aminocarbonyl group, N-methyl-N-(1-ethylpropyl)aminocarbonyl group, N-methyl-N-(1-methylbutyl)aminocarbonyl group, N-methyl-N-n-hexylaminocarbonyl group, N-methyl-N-isohexylaminocarbonyl group, N-methyl-N-(1,1,2-trimethylpropyl)aminocarbonyl group, or the like.

The "(C₁-C₆) alkoxycarbonyl group" refers to an alkoxycarbonyl group of 2 to 7 carbon atoms, for example, an alkoxycarbonyl group in which the alkoxy group is a (C₁-C₆) alkoxy group as defined above, such as a methoxycarbonyl group, an ethoxycarbonyl group, a n-propoxycarbonyl group, an isopropoxycarbonyl group, a n-butoxycarbonyl group, an isobutoxycarbonyl group, a sec-butoxycarbonyl group, a tert-butoxycarbonyl group, a pentyloxycarbonyl group, or the like.

The above-mentioned "(C₁-C₆) alkyl group", "(C₂-C₆) alkenyl group", "(C₂-C₆) alkynyl group", "(C₁-C₆) alkoxy group", "(C₁-C₆) alkylsulfanyl group", "(C₁-C₆) alkylsulfinyl group", "(C₁-C₆) alkylsulfonyl group", "(C₃-C₆) cycloalkyl group", "(C₁-C₆) alkylcarbonyl group", "(C₁-C₆) alkoxycarbonyl group", "(C₁-C₆) alkylcarbonyloxy group", etc. may be substituted with one or more halogen atoms at a substitutable position(s), and in the case where any of the above-listed groups is substituted with two or more halogen atoms, the halogen atoms may be the same or different.

The above-mentioned groups substituted with one or more halogen atoms are expressed as a "halo (C₁-C₆) alkyl group", a "halo (C₂-C₆) alkenyl group", a "halo (C₂-C₆) alkynyl group", a "halo (C₁-C₆) alkoxy group", a "halo (C₁-C₆) alkylsulfanyl group", a "halo (C₁-C₆) alkylsulfinyl group", a "halo (C₁-C₆) alkylsulfonyl group", a "halo (C₃-C₆) cycloalkyl group", a "halo (C₁-C₆) alkylcarbonyl group", a "halo (C₁-C₆) alkoxycarbonyl group", a "halo (C₁-C₆) alkylcarbonyloxy group", etc.

In the present invention, the expressions "(C₁-C₆)", "(C₂-C₆)", "(C₃-C₆)", etc. each represent the range of the number of carbon atoms in each group. The same definition holds true for groups in which two or more of the above-mentioned groups are coupled together, and for example, the "(C₁-C₆) alkoxy (C₁-C₆) alkyl group" means that a straight-chain or branched-chain alkoxy group of 1 to 6 carbon atoms is bound to a straight-chain or branched-chain alkyl group of 1 to 6 carbon atoms.

Examples of the salt of the compound represented by the general formula (1) of the present invention include inorganic acid salts, such as hydrochlorides, sulfates, nitrates and phosphates; organic acid salts, such as acetates, fumarates, maleates, oxalates, methanesulfonates, benzenesulfonates and p-toluenesulfonates; and salts with an inorganic or organic base such as a sodium ion, a potassium ion, a calcium ion and a trimethylammonium ion.

The compound represented by the general formula (1) of the present invention and a salt thereof can have one or more chiral centers in the structural formula and can exist as two or more kinds of optical isomers or diastereomers. All the optical isomers and mixtures of the isomers at any ratio are also included in the present invention. Further, the compound represented by the general formula (1) of the present invention and a salt thereof can exist as two kinds of geometric isomers due to a carbon-carbon double bond and a carbon-nitrogen double bond in the structural formula. All the geometric isomers and mixtures of the isomers at any ratio are also included in the present invention. In other words, the oxime group in the compound represented by the general formula (1) of the present invention may be in the form of an E (entgegen) isomer, a Z (zusammen) isomer, or a mixture of these isomers.

Preferable embodiments of the compound represented by the general formula (1) of the present invention are shown below.
Q is preferably Q1, Q2, Q3, Q4, Q5, Q6, Q7, Q8, Q9, or Q10, and more preferably Q1, Q2, Q3, Q4, Q5, Q6, Q7, Q8, or Q10.
R¹ is preferably
   (a1) a halogen atom;
   (a2) a (C₁-C₆) alkyl group;
   (a3) a (C₂-C₆) alkenyl group;
   (a5) a (C₃-C₆) cycloalkyl group;
   (a9) a halo (C₃-C₆) cycloalkyl group;
   (a10) a dioxolanyl group;
   (a11) a dioxanyl group;
   (a12) a dioxepanyl group;
   (a13) a dihydropyranyl group;
   (a14) a tetrahydropyranyl group;
   (a15) a tetrahydrothiopyranyl group;
   (a16) a piperidinyl group;
   (a17) a substituted piperidinyl group having 1 to 3 substituents on the ring, each independently selected from the group consisting of a (C₁-C₆) alkyl group, a (C₁-C₆) alkylcarbonyl group, and a (C₁-C₆) alkoxycarbonyl group;
   (a18) a thienyl group;
   (a19) a substituted thienyl group having 1 to 3 substituents on the ring, each independently selected from a set Y of substituents;
   (a20) a thiazolyl group;
   (a21) a substituted thiazolyl group having 1 or 2 substituents on the ring, each independently selected from the set Y of substituents;
   (a22) a thiadiazolyl group;
   (a23) a substituted thiadiazolyl group having one substituent on the ring, selected from the set Y of substituents;
   (a24) a pyrazolyl group;
   (a25) a substituted pyrazolyl group having 1 to 3 substituents on the ring, each independently selected from the set Y of substituents;
   (a26) a phenyl group;
   (a27) a substituted phenyl group having 1 to 5 substituents on the ring, each independently selected from the set Y of substituents;
   (a28) a pyridyl group;
   (a29) a substituted pyridyl group having 1 to 4 substituents on the ring, each independently selected from the set Y of substituents;
   (a30) a pyridazinyl group;
   (a31) a substituted pyridazinyl group having 1 to 3 substituents on the ring, each independently selected from the set Y of substituents;
   (a32) a pyrimidinyl group;
   (a33) a substituted pyrimidinyl group having 1 to 3 substituents on the ring, each independently selected from the set Y of substituents;
   (a34) a pyrazinyl group;
   (a35) a substituted pyrazinyl group having 1 to 3 substituents on the ring, each independently selected from the set Y of substituents;
   (a36) an amino group;
   (a37) a carboxyl group;
   (a38) a (C₁-C₆) alkoxy group;
   (a39) a halo (C₁-C₆) alkoxy group;
   (a40) a (C₁-C₆) alkylsulfanyl group;
   (a41) a (C₁-C₆) alkylsulfinyl group;
   (a42) a (C₁-C₆) alkylsulfonyl group;
   (a43) a (C₃-C₆) cycloalkenyl group;
   (a44) a (C₁-C₆) alkoxy (C₁-C₆) alkyl group;
   (a45) a (C₁-C₆) alkylsulfanyl (C₁-C₆) alkyl group;
   (a46) a (C₁-C₆) alkylsulfinyl (C₁-C₆) alkyl group;
   (a47) a (C₁-C₆) alkylsulfonyl (C₁-C₆) alkyl group;
   (a48) a (C₁-C₆) alkylcarbonyl group;
   (a49) a (C₁-C₆) alkoxycarbonyl group;
   (a50) a halo (C₁-C₆) alkylcarbonyl group;
   (a51) a halo (C₁-C₆) alkoxycarbonyl group;
   (a52) a phenylcarbonyl group;
   (a53) a substituted phenylcarbonyl group having 1 to 5 substituents on the ring, each independently selected from the set Y of substituents;
   (a54) a phenylaminocarbonyl group;
   (a55) a substituted phenylaminocarbonyl group having 1 to 5 substituents on the ring, each independently selected from the set Y of substituents;
   (a56) a benzhydrylidene amino group;
   (a57) an oxopyrrolidinyl group;
   (a58) an oxopyridyl group;
   (a59) a substituted dioxolanyl group having 1 to 4 substituents on the ring, each independently selected from the group consisting of a halogen atom and a (C₁-C₆) alkyl group;
   (a60) a substituted dioxanyl group having 1 to 4 substituents on the ring, each independently selected from the group consisting of a halogen atom and a (C₁-C₆) alkyl group;
   (a61) a furanyl group;
   (a62) a substituted furanyl group having 1 to 3 substituents on the ring, each independently selected from the set Y of substituents;
   (a63) an isothiazolyl group;
   (a64) a substituted isothiazolyl group having 1 or 2 substituents on the ring, each independently selected from the set Y of substituents;
   (a69) a quinolinyl group;
   (a70) a substituted quinolinyl group having 1 to 6 substituents on the ring, each independently selected from the set Y of substituents;
   (a71) a benzothienyl group;
   (a72) a substituted benzothienyl group having 1 to 5 substituents on the ring, each independently selected from the set Y of substituents;
   (a73) a phenoxy group;
   (a74) a substituted phenoxy group having 1 to 5 substituents on the ring, each independently selected from the set Y of substituents;
   (a75) a phenyl (C₁-C₆) alkyl group;
   (a76) a substituted phenyl (C₁-C₆) alkyl group having 1 to 5 substituents on the ring, each independently selected from the set Y of substituents;
   (a77) a phenyl (C₁-C₆) alkoxy group;
   (a78) a substituted phenyl (C₁-C₆) alkoxy group having 1 to 5 substituents on the ring, each independently selected from the set Y of substituents;
   (a79) a (C₁-C₆) alkylamino group;
   (a80) a halo (C₁-C₆) alkoxycarbonylamino group;
   (a81) an oxazolidinonyl group;
   (a82) a (C₁-C₆) alkoxy (C₁-C₆) alkoxy group;
   (a83) a tetrahydrofuranyl (C₁-C₆) alkoxy group; or
   (a84) a (C₃-C₆) cycloalkyl (C₁-C₆) alkoxy group.
R¹ is more preferably
   (a1) a halogen atom;
   (a2) a (C₁-C₆) alkyl group;
   (a3) a (C₂-C₆) alkenyl group;
   (a5) a (C₃-C₆) cycloalkyl group;
   (a13) a dihydropyranyl group;
   (a14) a tetrahydropyranyl group;
   (a16) a piperidinyl group;
   (a18) a thienyl group;
   (a19) a substituted thienyl group having 1 to 3 substituents on the ring, each independently selected from a set Y of substituents;
   (a20) a thiazolyl group;
   (a21) a substituted thiazolyl group having 1 or 2 substituents on the ring, each independently selected from the set Y of substituents;
   (a22) a thiadiazolyl group;
   (a23) a substituted thiadiazolyl group having one substituent on the ring, selected from the set Y of substituents;
   (a24) a pyrazolyl group;
   (a25) a substituted pyrazolyl group having 1 to 3 substituents on the ring, each independently selected from a set Y of substituents;
   (a26) a phenyl group;
   (a27) a substituted phenyl group having 1 to 5 substituents on the ring, each independently selected from the set Y of substituents;
   (a28) a pyridyl group;
   (a29) a substituted pyridyl group having 1 to 4 substituents on the ring, each independently selected from the set Y of substituents;
   (a32) a pyrimidinyl group;
   (a34) a pyrazinyl group;
   (a36) an amino group;
   (a37) a carboxyl group;
   (a38) a (C₁-C₆) alkoxy group;
   (a39) a halo (C₁-C₆) alkoxy group;
   (a40) a (C₁-C₆) alkylsulfanyl group;
   (a42) a (C₁-C₆) alkylsulfonyl group;
   (a43) a (C₃-C₆) cycloalkenyl group;
   (a44) a (C₁-C₆) alkoxy (C₁-C₆) alkyl group;
   (a45) a (C₁-C₆) alkylsulfanyl (C₁-C₆) alkyl group;
   (a46) a (C₁-C₆) alkylsulfinyl (C₁-C₆) alkyl group;
   (a47) a (C₁-C₆) alkylsulfonyl (C₁-C₆) alkyl group;
   (a48) a (C₁-C₆) alkylcarbonyl group;
   (a49) a (C₁-C₆) alkoxycarbonyl group;
   (a52) a phenylcarbonyl group;
   (a54) a phenylaminocarbonyl group;
   (a56) a benzhydrylidene amino group;
   (a57) an oxopyrrolidinyl group;
   (a58) an oxopyridyl group;
   (a59) a substituted dioxolanyl group having 1 to 4 substituents on the ring, each independently selected from the group consisting of a halogen atom and a (C₁-C₆) alkyl group;
   (a60) a substituted dioxanyl group having 1 to 4 substituents on the ring, each independently selected from the group consisting of a halogen atom and a (C₁-C₆) alkyl group;
   (a61) a furanyl group;
   (a63) an isothiazolyl group;
   (a64) a substituted isothiazolyl group having 1 or 2 substituents on the ring, each independently selected from the set Y of substituents;
   (a69) a quinolinyl group;
   (a71) a benzothienyl group;
   (a74) a substituted phenoxy group having 1 to 5 substituents on the ring, each independently selected from the set Y of substituents;
   (a75) a phenyl (C₁-C₆) alkyl group;
   (a82) a (C₁-C₆) alkoxy (C₁-C₆) alkoxy group;
   (a83) a tetrahydrofuranyl (C₁-C₆) alkoxy group; or
   (a84) a (C₃-C₆) cycloalkyl (C₁-C₆) alkoxy group.
R² is preferably
   (b1) a hydrogen atom;
   (b2) a halogen atom; or
   (b3) a (C₁-C₆) alkyl group.
R² is more preferably
   (b1) a hydrogen atom; or
   (b2) a halogen atom.
R^{2a} is preferably
   (b1') a hydrogen atom; or
   (b2') a (C₁-C₆) alkyl group.
R^{2a} is more preferably
   (b1') a hydrogen atom.
R³ is preferably
   (c1) a hydrogen atom;
   (c2) a halogen atom;
   (c3) a hydroxy group;
   (c4) a (C₁-C₆) alkyl group;
   (c5) a (C₂-C₆) alkenyl group;
   (c6) a (C₂-C₆) alkynyl group;
   (c7) a (C₃-C₆) cycloalkyl group;
   (c8) a halo (C₁-C₆) alkyl group;
   (c12) a (C₁-C₆) alkoxy group;
   (c13) a (C₁-C₆) alkylsulfanyl group;
   (c14) a (C₁-C₆) alkylsulfinyl group;
   (c15) a (C₁-C₆) alkylsulfonyl group;
   (c16) a halo (C₁-C₆) alkoxy group;
   (c17) a halo (C₁-C₆) alkylsulfanyl group;
   (c18) a halo (C₁-C₆) alkylsulfinyl group;
   (c19) a halo (C₁-C₆) alkylsulfonyl group;
   (c20) a (C₁-C₆) alkoxycarbonyl group; or
   (c21) a (C₁-C₆) alkylamino group, or
   R² and R³ may be bound to each other to form a 5- or 6-membered ring.
R³ is more preferably
   (c1) a hydrogen atom;
   (c2) a halogen atom;
   (c3) a hydroxy group;
   (c4) a (C₁-C₆) alkyl group;
   (c5) a (C₂-C₆) alkenyl group;
   (c8) a halo (C₁-C₆) alkyl group;
   (c12) a (C₁-C₆) alkoxy group;
   (c13) a (C₁-C₆) alkylsulfanyl group;
   (c14) a (C₁-C₆) alkylsulfinyl group;
   (c15) a (C₁-C₆) alkylsulfonyl group; or
   (c20) a (C₁-C₆) alkoxycarbonyl group, or
   R² and R³ may be bound to each other to form a 5- or 6-membered ring.
R^{3a} is preferably
   (c1') a hydrogen atom; or
   (c2') a (C₁-C₆) alkyl group.
R^{3a} is more preferably
(c1') a hydrogen atom.

The set Y of substituents preferably consists of
(d1) a halogen atom;
(d2) a cyano group;
(d3) a nitro group;
(d4) an amino group;
(d5) a (C₁-C₆) alkyl group;
(d8) a (C₃-C₆) cycloalkyl group;
(d9) a halo (C₁-C₆) alkyl group;
(d13) a (C₁-C₆) alkoxy group;
(d14) a (C₁-C₆) alkylsulfanyl group;
(d15) a (C₁-C₆) alkylsulfinyl group;
(d16) a (C₁-C₆) alkylsulfonyl group;
(d17) a halo (C₁-C₆) alkoxy group;
(d18) a halo (C₁-C₆) alkylsulfanyl group;
(d19) a halo (C₁-C₆) alkylsulfinyl group;
(d20) a halo (C₁-C₆) alkylsulfonyl group;
(d21) an N-((C₁-C₆) alkylcarbonyl)amino group;
(d22) an N,N-di-((C₁-C₆) alkylcarbonyl)amino group wherein the (C₁-C₆) alkylcarbonyl groups may be the same or different;
(d23) a di-(C₁-C₆) alkoxyphosphanyl group wherein the (C₁-C₆) alkoxy groups may be the same or different;
(d24) an imidazolyl group;
(d25) an imidazolyl group having 1 to 3 substituents on the ring, each independently selected from the group consisting of a halogen atom and a (C₁-C₆) alkyl group;
(d26) a phenyl group;
(d27) a phenyl group having 1 to 5 substituents on the ring, each independently selected from the group consisting of a halogen atom and a (C₁-C₆) alkyl group;
(d28) a pyridyl group;
(d29) a pyridyl group having 1 to 4 substituents on the ring, each independently selected from the group consisting of a halogen atom and a (C₁-C₆) alkyl group;
(d30) a di-(C₁-C₆) alkylamino group;
(d31) a (C₁-C₆) alkoxycarbonyl group; and
(d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group.

The set Y of substituents more preferably consists of
(d1) a halogen atom;
(d2) a cyano group;
(d3) a nitro group;
(d4) an amino group;
(d5) a (C₁-C₆) alkyl group;
(d8) a (C₃-C₆) cycloalkyl group;
(d9) a halo (C₁-C₆) alkyl group;
(d13) a (C₁-C₆) alkoxy group;
(d14) a (C₁-C₆) alkylsulfanyl group;
(d17) a halo (C₁-C₆) alkoxy group;
(d21) an N-((C₁-C₆) alkylcarbonyl)amino group;
(d24) an imidazolyl group;
(d26) a phenyl group;
(d28) a pyridyl group;
(d30) a di-(C₁-C₆) alkylamino group;
(d31) a (C₁-C₆) alkoxycarbonyl group; and
(d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group.

R⁴ is preferably
(e1) a hydrogen atom;
(e2) an amino group;
(e3) a (C₁-C₆) alkyl group;
(e4) an N-(C₁-C₆) alkylamino group;
(e5) an N,N-di-(C₁-C₆) alkylamino group wherein the (C₁-C₆) alkyl groups may be the same or different;
(e6) an N-((C₁-C₆) alkylcarbonyl)amino group;
(e7) an N,N-di-((C₁-C₆) alkylcarbonyl)amino group wherein the (C₁-C₆) alkylcarbonyl groups may be the same or different; or
(e8) an N-(halo (C₁-C₆) alkylcarbonyl)amino group.
R⁴ is more preferably
(e2) an amino group;
(e3) a (C₁-C₆) alkyl group;
(e6) an N-((C₁-C₆) alkylcarbonyl)amino group;
(e7) an N,N-di-((C₁-C₆) alkylcarbonyl)amino group wherein the (C₁-C₆) alkylcarbonyl groups may be the same or different; or
(e8) an N-(halo (C₁-C₆) alkylcarbonyl)amino group.

R⁵ is preferably
(f1) a (C₁-C₆) alkyl group;
(f2) a (C₂-C₆) alkenyl group;
(f3) a (C₂-C₆) alkynyl group;
(f4) a (C₃-C₆) cycloalkyl group;
(f5) a halo (C₁-C₆) alkyl group;
(f9) a (C₃-C₆) cycloalkyl (C₁-C₆) alkyl group;
(f10) a (C₁-C₆) alkoxy (C₁-C₆) alkyl group;
(f11) a (C₁-C₆) alkylsulfanyl (C₁-C₆) alkyl group;
(f12) a (C₁-C₆) alkylsulfinyl (C₁-C₆) alkyl group; or
(f13) a (C₁-C₆) alkylsulfonyl (C₁-C₆) alkyl group.
R⁵ is more preferably
(f1) a (C₁-C₆) alkyl group;
(f5) a halo (C₁-C₆) alkyl group; or
(f10) a (C₁-C₆) alkoxy (C₁-C₆) alkyl group.

A is preferably
(g1) a (C₁-C₆) alkoxy group;
(g3) an N-(C₁-C₆) alkylaminocarbonyl group;
(g4) an N,N-di-(C₁-C₆) alkylaminocarbonyl group wherein the (C₁-C₆) alkylcarbonyl groups may be the same or different;
(g5') CO₂R⁷ wherein R⁷ represents
   (h1) a hydrogen atom,
   (h2) a (C₁-C₆) alkyl group,
   (h3) a (C₂-C₆) alkenyl group,
   (h4) a (C₂-C₆) alkynyl group,
   (h5) a (C₃-C₆) cycloalkyl group,
   (h6) a halo (C₁-C₆) alkyl group, or
   (h10) a phenyl (C₁-C₆) alkyl group;
(g6') SOₙR⁸ wherein R⁸ represents
   (i1) a (C₁-C₆) alkyl group,
   (i2) a (C₂-C₆) alkenyl group,
   (i3) a (C₂-C₆) alkynyl group,
   (i4) a (C₃-C₆) cycloalkyl group,
   (i5) a halo (C₁-C₆) alkyl group, or
   (i9) a (C₁-C₆) alkylcarbonyl (C₁-C₆) alkyl group, and
   n represents 0, 1, or 2; or
(g7') SO₂N(R⁹)R¹⁰ wherein R⁹ and R¹⁰ represent
   (j 1) a hydrogen atom,
   (j2) a (C₁-C₆) alkyl group,
   (j3) a (C₂-C₆) alkenyl group,
   (j4) a (C₂-C₆) alkynyl group,
   (j5) a (C₃-C₆) cycloalkyl group,
   (j 11) a (C₁-C₆) alkylcarbonyl group,
   (j12) a (C₁-C₆) alkoxycarbonyl group,
   (j13) a halo (C₁-C₆) alkylcarbonyl group, or
   (j14) a halo (C₁-C₆) alkoxycarbonyl group, and
   R⁹ and R¹⁰ may be the same or different.
   A is more preferably
(g1) a (C₁-C₆) alkoxy group;
(g4) an N,N-di-(C₁-C₆) alkylaminocarbonyl group wherein the (C₁-C₆) alkylcarbonyl groups may be the same or different;
(g5") CO₂R⁷ wherein R⁷ represents
   (h1) a hydrogen atom, or
   (h2) a (C₁-C₆) alkyl group;
(g6") SOₙR⁸ wherein R⁸ represents
   (i1) a (C₁-C₆) alkyl group, or
   (i9) a (C₁-C₆) alkylcarbonyl (C₁-C₆) alkyl group, and
   n represents 2; or
(g7") SO₂N(R⁹)R¹⁰ wherein R⁹ and R¹⁰ represent
   (j 1) a hydrogen atom,
   (j2) a (C₁-C₆) alkyl group,
   (j 11) a (C₁-C₆) alkylcarbonyl group, or
   (j 12) a (C₁-C₆) alkoxycarbonyl group, and
   R⁹ and R¹⁰ may be the same or different.

D is preferably D1 or D2, and more preferably D1.

R^{6a} and R^{6b} preferably represent
(k1) a hydrogen atom;
(k2) a halogen atom; or
(k3) a (C₁-C₆) alkyl group.
   R^{6a} and R^{6b} more preferably represent
(k1) a hydrogen atom.

R^{6a} and R^{6b} may be the same or different.

The compounds of the present invention can be produced according to, for example, the production methods described below, which are non-limiting examples.

### Production Method 1

The compounds represented by the general formulae (1a) and (1a-1) of the present invention can be produced from the compound represented by the general formula (2a) through the steps [a], [b], and [c] described below.

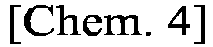

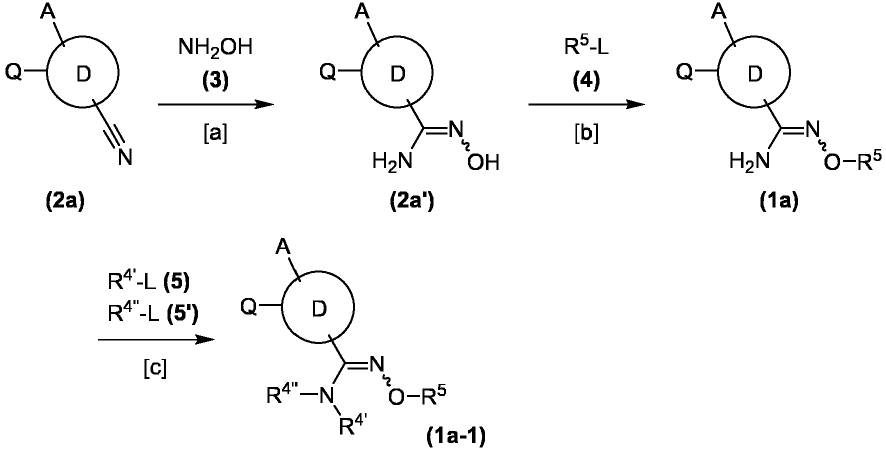

In the formula, A, D, Q, and R⁵ are the same as above; L represents a leaving group such as chlorine, bromine, iodine, or a (C₁-C₆) alkylcarbonyloxy group; and R^{4'} and R^{4"} represent a (C₁-C₆) alkyl group, a (C₁-C₆) alkylcarbonyl group, a halo (C₁-C₆) alkylcarbonyl group, or a (C₁-C₆) alkoxycarbonyl group, and R^{4'} and R^{4"} may be the same or different.

### Production method at step [a]

The compound represented by the general formula (2a') can be produced by reacting the compound represented by the general formula (2a) with the compound represented by the general formula (3) or a salt thereof in the presence of a base and an inert solvent.

Examples of the base that can be used in this reaction include inorganic bases such as sodium hydroxide, potassium hydroxide, sodium carbonate, potassium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; and organic bases including tertiary amines such as triethylamine, N,N-diisopropylethylamine and 1,8-diazabicyclo[5.4.0]undec-7-ene (DBU), and nitrogen-containing aromatic compounds such as pyridine and N,N-dimethyl-4-aminopyridine. The amount of the base used is usually in the range of a 1- to 10-fold molar amount relative to the compound represented by the general formula (2a).

The inert solvent used in this reaction may be any solvent that does not markedly inhibit the progress of the reaction, and examples include chain or cyclic saturated hydrocarbons such as pentane, hexane, and cyclohexane; aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; nitriles such as acetonitrile and propionitrile; esters such as methyl acetate; ketones such as acetone and methyl ethyl ketone; aprotic polar solvents such as N,N-dimethylformamide, N,N-dimethylacetamide, dimethyl sulfoxide, and 1,3-dimethyl-2-imidazolidinone; and alcohols such as methanol, ethanol, propanol, butanol, and 2-propanol. One of these inert solvents may be used alone, and also two or more of them may be used as a mixture. The amount of the inert solvent used is usually selected as appropriate from the range of 0.1 to 100 L relative to 1 mol of the compound represented by the general formula (2a).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [b]

The compound represented by the general formula (1a) can be produced by reacting the compound represented by the general formula (2a') with the compound represented by the general formula (4) in the presence of an inert solvent and a base.

Examples of the base that can be used in this reaction include inorganic bases such as sodium hydroxide, potassium hydroxide, sodium carbonate, potassium carbonate, cesium carbonate, sodium hydrogen carbonate, potassium hydrogen carbonate and sodium hydride; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (2a').

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2a').

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production method at step [c]

The compound represented by the general formula (1a-1) can be produced by reacting the compound represented by the general formula (1a) with the compound represented by the general formula (5) and/or (5') in the presence of an inert solvent and a base.

Examples of the base that can be used in this reaction include alkyllithiums such as methyllithium, n-butyllithium, sec-butyllithium and tert-butyllithium; organometallic compounds such as lithium hexamethyldisilazane and sodium hexamethyldisilazane; hydroxides such as lithium hydroxide, sodium hydroxide, potassium hydroxide and calcium hydroxide; carbonates such as lithium carbonate, lithium hydrogen carbonate, sodium carbonate, sodium hydrogen carbonate, potassium carbonate, cesium carbonate, potassium hydrogen carbonate, calcium carbonate and magnesium carbonate; acetates such as lithium acetate, sodium acetate and potassium acetate; alkoxides such as sodium methoxide, sodium ethoxide, sodium tert-butoxide and potassium tert-butoxide; metal hydrides such as sodium hydride and potassium hydride; and organic bases such as pyridine, picoline, lutidine, triethylamine, tributylamine and N,N-diisopropylethylamine. The amount of the base used is usually in the range of a 1- to 10-fold molar amount relative to the compound represented by the general formula (1a).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (1a).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production Method 2

The compound represented by the general formula (1a) can be produced from the compound represented by the general formula (2a) through the step [a'] described below.

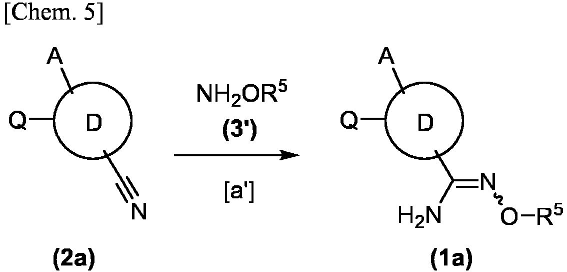

In the formula, A, D, Q, and R⁵ are the same as above.

### Production method at step [a']

The compound represented by the general formula (1a) can be produced by reacting the compound represented by the general formula (2a) with the compound represented by the general formula (3') or a salt thereof in the presence of a base and an inert solvent.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; and organic bases including tertiary amines such as triethylamine, N,N-diisopropylethylamine and DBU, and nitrogen-containing aromatic compounds such as pyridine and N,N-dimethyl-4-aminopyridine. The amount of the base used is usually in the range of a 1- to 10-fold molar amount relative to the compound represented by the general formula (2a).

The inert solvent used in this reaction may be any solvent that does not markedly inhibit the progress of the reaction, and examples include chain or cyclic saturated hydrocarbons such as pentane, hexane, and cyclohexane; aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; nitriles such as acetonitrile and propionitrile; esters such as methyl acetate; ketones such as acetone and methyl ethyl ketone; aprotic polar solvents such as N,N-dimethylformamide, N,N-dimethylacetamide, dimethyl sulfoxide, and 1,3-dimethyl-2-imidazolidinone; and alcohols such as methanol, ethanol, propanol, butanol, and 2-propanol. One of these inert solvents may be used alone, and also two or more of them may be used as a mixture. The amount of the inert solvent used is usually selected as appropriate from the range of 0.1 to 100 L relative to 1 mol of the compound represented by the general formula (2a).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production Method 3

The compound represented by the general formula (1a) can be produced from the compound represented by the general formula (2b) through the step [d] or [e] described below.

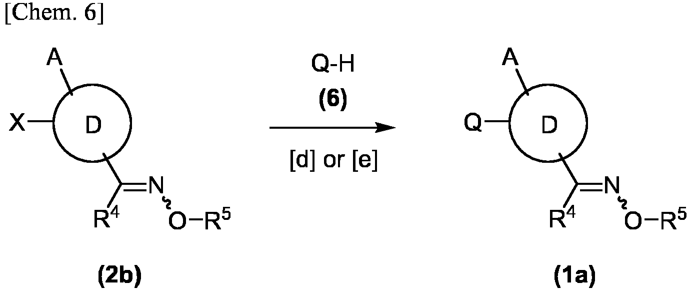

In the formula, A, D, Q, R⁴, and R⁵ are the same as above; and X represents a leaving group such as chlorine, bromine, or iodine.

### Production method at step [d]

The compound represented by the general formula (1a) can be produced by reacting the compound represented by the general formula (2b) with the compound represented by the general formula (6) in the presence of a base and an inert solvent.

Examples of the base that can be used in this reaction include inorganic bases such as sodium hydroxide, potassium hydroxide, sodium carbonate, potassium carbonate, cesium carbonate, sodium hydrogen carbonate, potassium hydrogen carbonate and sodium hydride; acetates such as sodium acetate and potassium acetate; alkali metal alkoxides such as potassium tert-butoxide, sodium methoxide and sodium ethoxide; organic bases including tertiary amines such as triethylamine, N,N-diisopropylethylamine and DBU, and nitrogen-containing aromatic compounds such as pyridine and N,N-dimethyl-4-aminopyridine; alkyllithiums such as methyllithium, n-butyllithium, sec-butyllithium and tert-butyllithium; and organometallic compounds such as lithium hexamethyldisilazane and sodium hexamethyldisilazane. The amount of the base used is usually in the range of a 1- to 10-fold molar amount relative to the compound represented by the general formula (2b).

The inert solvent used in this reaction may be any solvent that does not markedly inhibit the progress of the reaction, and examples include chain or cyclic saturated hydrocarbons such as pentane, hexane, and cyclohexane; aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; nitriles such as acetonitrile and propionitrile; esters such as methyl acetate; ketones such as acetone and methyl ethyl ketone; aprotic polar solvents such as N,N-dimethylformamide, N,N-dimethylacetamide, dimethyl sulfoxide, and 1,3-dimethyl-2-imidazolidinone; and alcohols such as methanol, ethanol, propanol, butanol, and 2-propanol. One of these inert solvents may be used alone, and also two or more of them may be used as a mixture. The amount of the inert solvent used is usually selected as appropriate from the range of 0.1 to 100 L relative to 1 mol of the compound represented by the general formula (2b).

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production method at step [e]

The compound represented by the general formula (1a) can be produced by reacting the compound represented by the general formula (2b) with the compound represented by the general formula (6) in the presence of a metal catalyst and an inert solvent.

Examples of the metal catalyst that can be used in this reaction include a palladium catalyst, a nickel catalyst, an iron catalyst, a ruthenium catalyst, a platinum catalyst, a rhodium catalyst and an iridium catalyst. Such a metal catalyst can be used in the form of a metal; a supported metal; a metal salt such as a metal chloride, a metal bromide, a metal iodide, a metal nitrate, a metal sulfate, a metal carbonate, a metal oxalate, a metal acetate and a metal oxide; or a complex compound such as an olefin complex, a phosphine complex, an amine complex, an ammine complex and an acetylacetonate complex.

Among these metal catalysts, palladium catalysts are particularly preferred. Examples of the palladium catalyst include palladium metals such as palladium black and palladium sponge; supported palladium metals such as palladium/alumina, palladium/carbon, palladium/silica and palladium/type Y zeolite; palladium metal salts such as palladium chloride, palladium bromide, palladium iodide and palladium acetate; and palladium complex compounds such as π-allylpalladium chloride dimer, palladium acetylacetonate, dichlorobis(acetonitrile)palladium, dichlorobis(benzonitrile)palladium, bis(dibenzylideneacetone)palladium, tris(dibenzylideneacetone)dipalladium, tris(dibenzylideneacetone)dipalladium (chloroform adduct), dichlorodiamine palladium, dichlorobis(triphenylphosphine)palladium, dichlorobis(tricyclohexylphosphine)palladium, tetrakis(triphenylphosphine)palladium, dichloro[1,2-bis(diphenylphosphino)ethane]palladium, dichloro[1,3-bis(diphenylphosphino)propane]palladium, dichloro [1,4-bis(diphenylphosphino)butane]palladium, dichloro[1,1'-bis(diphenylphosphino)ferrocene]palladium and a [(diphenylphosphino)ferrocene]dichloropalladium-dichloromethane complex. The amount of the metal catalyst used is usually selected as appropriate from the range of a 0.001- to 0.5-fold molar amount relative to the compound represented by the general formula (2b).

These palladium catalysts may be used alone or in combination with a tertiary phosphine. Examples of the tertiary phosphine that can be used in combination with the palladium catalyst include triphenylphosphine, trimethylphosphine, triethylphosphine, tributylphosphine, tri(tert-butyl)phosphine, tricyclohexylphosphine, tri-o-tolylphosphine, trioctylphosphine, 9,9-dimethyl-4,5-bis(diphenylphosphino)xanthene, 2-(di-tert-butylphosphino)biphenyl, 2-(dicyclohexylphosphino)biphenyl, 1,2-bis(diphenylphosphino)ethane, 1,3-bis(diphenylphosphino)propane, 1,4-bis(diphenylphosphino)butane, 1,1'-bis(diphenylphosphino)ferrocene, (R)-(+)-2,2'-bis(diphenylphosphino)-1,1'-binaphthyl, (S)-(-)-2,2'-bis(diphenylphosphino)-1,1'-binaphthyl and (+)-2,2'-bis(diphenylphosphino)-1,1'-binaphthyl. The amount of the tertiary phosphine used is usually selected as appropriate from the range of a 0.5- to 10-fold molar amount relative to the metal catalyst.

The inert solvent used in this reaction may be any solvent that does not markedly inhibit the reaction, and examples include alcohols such as methanol, ethanol, propanol, butanol and 2-propanol; chain or cyclic ethers such as diethyl ether, tetrahydrofuran, dioxane and 1,2-dimethoxyethane (DME); aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; nitriles such as acetonitrile; esters such as ethyl acetate; polar solvents such as N,N-dimethylformamide, N,N-dimethylacetamide, dimethyl sulfoxide and 1,3-dimethyl-2-imidazolidinone; and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2b).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like, but is basically selected as appropriate from the range of a few minutes to 48 hours. This reaction may be conducted under the atmosphere of an inert gas such as nitrogen gas and argon gas. After the reaction is completed, the compound of interest is isolated from the post-reaction mixture by the usual method. As needed, recrystallization, column chromatography, etc. can be employed for the purification of the compound of interest.

### Production Method 4

The compound represented by the general formula (1b) can be produced from the compound represented by the general formula (1a-2) through the steps [l] and [f] described below.

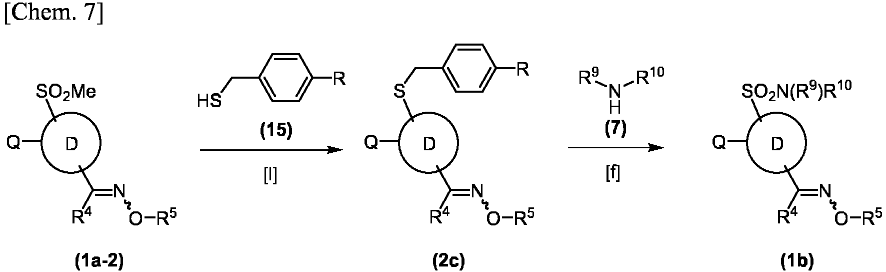

In the formula, D, Q, R⁴, R⁵, R⁹, and R¹⁰ are the same as above; and R represents a (C₁-C₄) alkyl group such as a tert-butyl group or a hydrogen atom.

### Production method at step [l]

The compound represented by the general formula (2c) can be produced by reacting the compound represented by the general formula (1a-2) with the thiol compound represented by the general formula (15) in the presence of a base and an inert solvent.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (1a-2). In the case where an alkali salt of the compound represented by the general formula (15) is used, it is not necessary to use a base.

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (1a-2).

Since this reaction is an equimolar reaction of the compounds, they are basically used in equimolar amounts, but either of them may be used in an excess amount. The reaction temperature in this reaction is usually in the range of -20°C to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [f]

The compound represented by the general formula (1b) of the present invention can be produced by chlorosulfonylation of the compound represented by the general formula (2c) with a halogenating agent in the presence of an inert solvent, followed by amination with the compound represented by the general formula (7) in the presence of an inert solvent with or without a base.

Examples of the halogenating agent that can be used in the chlorosulfonylation include thionyl chloride, chlorine, sulfuryl chloride, 1,3-dichloro-5,5-dimethylhydantoin, and N-chlorosuccinimide. The amount of the halogenating agent used is usually selected as appropriate from the range of a 0.5- to 5-fold molar amount relative to the compound represented by the general formula (2c).

The inert solvent used in the chlorosulfonylation may be any solvent that does not markedly inhibit the progress of the reaction, and examples include halogenated hydrocarbons such as methylene chloride, chloroform and carbon tetrachloride; halogenated aromatic hydrocarbons such as chlorobenzene and dichlorobenzene; nitriles such as acetonitrile and propionitrile; organic acids such as propionic acid; and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2c).

Since the chlorosulfonylation is an equimolar reaction of the reactants, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, when the chlorosulfonylated product is stable, the product is isolated from the post-reaction mixture by the usual method. As needed, recrystallization, column chromatography, etc. can be employed for the purification of the product. When the chlorosulfonylated product is unstable, the post-reaction mixture is directly used in the next reaction without purification.

Examples of the base that can be used in the amination include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (2c).

The inert solvent used in the amination may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; amides such as N,N-dimethylformamide and N,N-dimethylacetamide; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2c).

Since the amination is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest.

### Production Method 5

Among examples of the compound represented by the general formula (1), the compounds represented by the general formulae (1c) and (1c-1) wherein Q is the above-defined Q7 can be produced from the compound represented by the general formula (2b) through the steps [g], [h], and [i] described below.

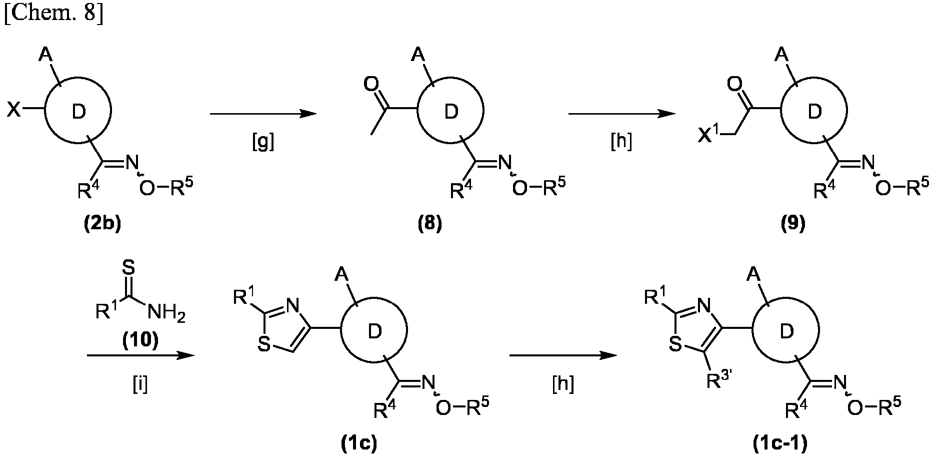

In the formula, A, D, R¹, R⁴, and R⁵ are the same as above; R^{3'} represents a halogen atom; X represents a leaving group such as chlorine, bromine, or iodine; and X¹ represents a halogen atom such as chlorine, bromine, or iodine.

### Production method at step [g]

The compound represented by the general formula (8) can be produced by reacting the compound represented by the general formula (2b) with tributyl(1-ethoxyvinyl)tin in the presence of a palladium catalyst and an inert solvent, followed by acid treatment.

Examples of the palladium catalyst that can be used in this reaction include bis(triphenylphosphine)palladium(II) chloride, palladium(II) acetate, palladium(II) chloride, tetrakis(triphenylphosphine)palladium(0), and bis(tri-tert-butylphosphine)palladium(0). The amount of the palladium catalyst used is in the range of a 0.001- to 0.5-fold molar amount relative to the compound represented by the general formula (2b).

The inert solvent used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; esters such as ethyl acetate; amides such as N,N-dimethylformamide and N,N-dimethylacetamide; ketones such as acetone and methyl ethyl ketone; alcohols such as methanol, ethanol, propanol, butanol, and 2-propanol; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2b).

Examples of the acid that can be used in this reaction include inorganic acids such as hydrochloric acid, sulfuric acid and nitric acid; organic acids such as formic acid, acetic acid, propionic acid, trifluoroacetic acid and benzoic acid; and sulfonic acids such as methanesulfonic acid and trifluoromethanesulfonic acid. The amount of the acid used is usually in the range of a 1- to 10-fold molar amount relative to the compound represented by the general formula (2b).

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production method at step [h]

The compound represented by the general formula (9) can be produced by halogenating the compound represented by the general formula (8) with a halogenating agent in the presence of an inert solvent. The compound represented by the general formula (1c-1) can be produced by halogenating the compound represented by the general formula (1c) with a halogenating agent in the presence of an inert solvent.

Examples of the halogenating agent include halogen molecules such as chlorine, bromine, and iodine; ammonium halide salts formed of halogen molecules; halogenated succinimides such as N-chlorocosuccinimide and N-bromocosuccinimide; halogenated hydantoins such as diiodohydantoin; and sulfuryl chloride. The amount of the halogenating agent used is usually in the range of a 0.5- to 10-fold molar amount relative to the compound represented by the general formula (8) or (1c-1).

The inert solvent used in this reaction may be any solvent that does not markedly inhibit the reaction, and examples include alcohols such as methanol, ethanol, propanol, butanol and 2-propanol; chain or cyclic ethers such as diethyl ether, tetrahydrofuran and dioxane; aromatic hydrocarbons such as benzene, toluene and xylene; esters such as ethyl acetate; and polar solvents such as N,N-dimethylformamide, N,N-dimethylacetamide, 1,3-dimethyl-2-imidazolidinone, water and acetic acid. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (8) or (1c-1).

The reaction temperature in this reaction is usually in the range of -30°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [i]

The compound represented by the general formula (1c) can be produced by reacting the compound represented by the general formula (9) with the compound represented by the general formula (10) in the presence of an inert solvent with or without a base.

The base used in this reaction may be an inorganic base or an organic base. Examples of the inorganic base include alkali metal hydroxides such as sodium hydroxide and potassium hydroxide; alkali metal hydrides such as sodium hydride and potassium hydride; alkali metal alkoxides such as sodium ethoxide and potassium tert-butoxide; and carbonates such as sodium carbonate, potassium carbonate, cesium carbonate, and sodium hydrogen carbonate. Examples of the organic base include triethylamine, pyridine, and DBU. The amount of the base used is usually in the range of a 0.01- to 10-fold molar amount relative to the compound represented by the general formula (9).

The inert solvent used in this reaction may be any solvent that does not markedly inhibit the progress of the reaction, and examples include alcohols such as methanol, ethanol, propanol, butanol and 2-propanol; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (9).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production Method 6

Among examples of the compound represented by the general formula (1), the compounds represented by the general formulae (1c') and (1c'-1) wherein Q is the above-defined Q12 can be produced from the compound represented by the general formula (9) through the steps [i] and [h] described below. The reaction conditions for the steps [i] and [h] are the same as described above.

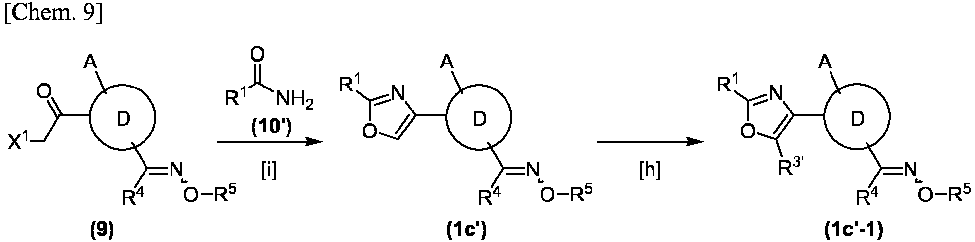

In the formula, A, D, R¹, R⁴, and R⁵ are the same as above; R^{3'} represents a halogen atom; and X¹ represents a halogen atom such as chlorine, bromine, or iodine.

### Production Method 7

Among examples of the compound represented by the general formula (1), the compound represented by the general formula (1d) wherein Q is the above-defined Q8 can be produced from the compound represented by the general formula (2d) through the steps [j], [k], and [i] described below. The reaction conditions for the step [i] are the same as described above.

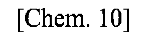

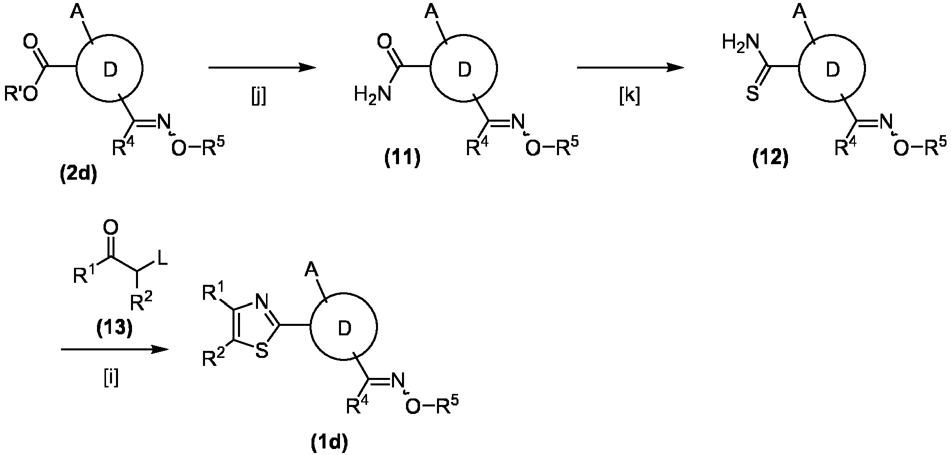

In the formula, A, D, R¹, R², R⁴, and R⁵ are the same as above; R' represents a (C₁-C₆) alkyl group such as a methyl group or an ethyl group; and L represents a leaving group such as chlorine, bromine, iodine, or a (C₁-C₆) alkylcarbonyloxy group.

### Production method at step [j]

The compound represented by the general formula (11) can be produced by reacting the compound represented by the general formula (2d) with ammonia in the presence of an inert solvent with or without a base.

The amount of the ammonia used in this reaction is usually in the range of an about 1- to 10-fold molar amount relative to the compound represented by the general formula (2d).

The base used in this reaction may be an inorganic base or an organic base. Examples of the inorganic base include alkali metal hydroxides such as sodium hydroxide and potassium hydroxide; alkali metal hydrides such as sodium hydride and potassium hydride; alkali metal alkoxides such as sodium ethoxide and potassium tert-butoxide; and carbonates such as sodium carbonate, potassium carbonate, cesium carbonate, and sodium hydrogen carbonate. Examples of the organic base include triethylamine, pyridine, and DBU. The amount of the base used is usually in the range of a 0.01- to 10-fold molar amount relative to the compound represented by the general formula (2d).

The inert solvent used in this reaction may be any solvent that does not markedly inhibit the progress of the reaction, and examples include aromatic hydrocarbons such as benzene, toluene, and xylene; halogenated aliphatic hydrocarbons such as methylene chloride, chloroform, and carbon tetrachloride; halogenated aromatic hydrocarbons such as chlorobenzene and dichlorobenzene; chain or cyclic ethers such as diethyl ether, dioxane, and tetrahydrofuran; esters such as ethyl acetate; amides such as N,N-dimethylformamide and N,N-dimethylacetamide; and other inert solvents such as dimethyl sulfoxide, 1,3-dimethyl-2-imidazolidinone, acetone, methyl ethyl ketone, and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2d).

The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture is directly used in the next reaction without purification.

### Production method at step [k]

The compound represented by the general formula (12) can be produced by reacting the compound represented by the general formula (11) with a sulfurizing agent in the presence of an inert solvent.

Examples of the sulfurizing agent that can be used in this reaction include Lawesson's reagent and diphosphorus pentasulfide. The amount of the sulfurizing agent used is usually in the range of a 1.0- to 10-fold molar amount relative to the compound represented by the general formula (11).

This reaction may be conducted with or without a solvent. The solvent used in this reaction may be any solvent that does not markedly inhibit the progress of the reaction, and examples include chain or cyclic saturated hydrocarbons such as pentane, hexane, and cyclohexane; aromatic hydrocarbons such as benzene, toluene, and xylene; halogenated aromatic hydrocarbons such as chlorobenzene and dichlorobenzene; chain or cyclic ethers such as diethyl ether, methyl tert-butyl ether, dioxane, and tetrahydrofuran; nitriles such as acetonitrile and propionitrile; aprotic polar solvents such as N,N-dimethylformamide, N,N-dimethylacetamide, dimethyl sulfoxide, and 1,3-dimethyl-2-imidazolidinone; and alcohols such as methanol, ethanol, propanol, butanol, and 2-propanol. One of these inert solvents may be used alone, and also two or more of them may be used as a mixture. The amount of the solvent used is usually selected as appropriate from the range of 0.1 to 100 L relative to 1 mol of the compound represented by the general formula (11).

The reaction temperature in this reaction is usually in the range of about 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production Method 8

Among examples of the compound represented by the general formula (1), the compound represented by the general formulae (1e) wherein Q is the above-defined Q10 can be produced from the compound represented by the general formula (2e) through the step [c'] described below.

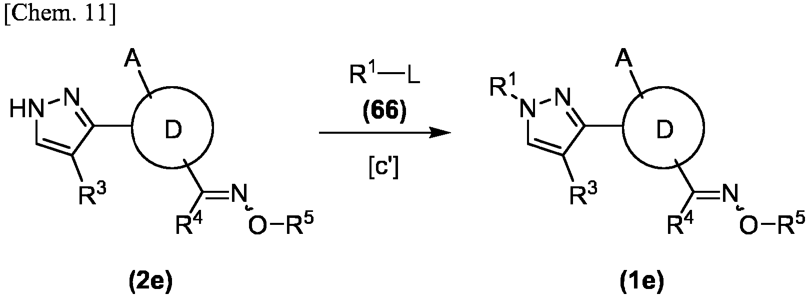

In the formula, A, D, R¹, R³, R⁴, and R⁵ are the same as above; and L represents a leaving group such as chlorine, bromine, iodine, or a (C₁-C₆) alkylcarbonyloxy group.

### Production method at step [c']

The compound represented by the general formula (1e) can be produced by reacting the compound represented by the general formula (2e) with the compound represented by the general formula (66) in the presence of an inert solvent and a base with or without copper acetate.

The copper acetate used in this reaction may be a hydrate. The amount of the copper acetate used is usually in the range of 0.1- to 1-fold molar amount relative to the compound represented by the general formula (2e).

Examples of the base that can be used in this reaction include alkyllithiums such as methyllithium, n-butyllithium, sec-butyllithium and tert-butyllithium; organometallic compounds such as lithium hexamethyldisilazane and sodium hexamethyldisilazane; hydroxides such as lithium hydroxide, sodium hydroxide, potassium hydroxide and calcium hydroxide; carbonates such as lithium carbonate, lithium hydrogen carbonate, sodium carbonate, sodium hydrogen carbonate, potassium carbonate, cesium carbonate, potassium hydrogen carbonate, calcium carbonate and magnesium carbonate; acetates such as lithium acetate, sodium acetate and potassium acetate; alkoxides such as sodium methoxide, sodium ethoxide, sodium tert-butoxide and potassium tert-butoxide; metal hydrides such as sodium hydride and potassium hydride; and organic bases such as pyridine, picoline, lutidine, triethylamine, tributylamine and N,N-diisopropylethylamine. The amount of the base used is usually in the range of a 1- to 10-fold molar amount relative to the compound represented by the general formula (2e).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2e).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production Method 9

Among examples of the compound represented by the general formula (1), the compound represented by the general formula (1f) wherein Q is the above-defined Q18 can be produced from the compound represented by the general formula (11) through the step [i] described below. The reaction conditions for the step [i] are the same as described above.

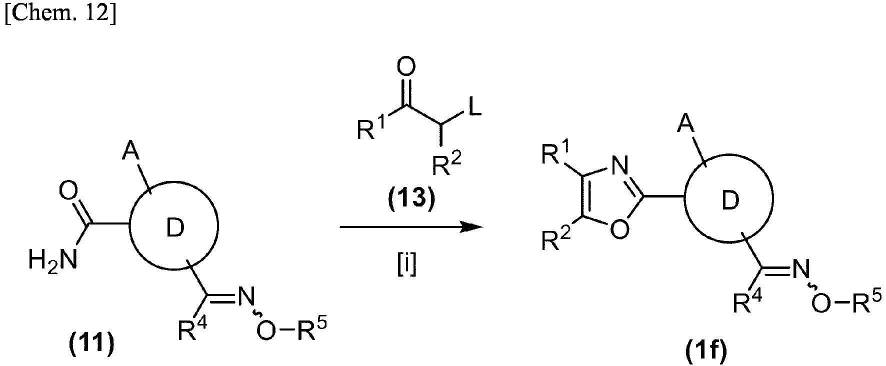

In the formula, A, D, R¹, R², R⁴, and R⁵ are the same as above; and L represents a leaving group such as chlorine, bromine, iodine, or a (C₁-C₆) alkylcarbonyloxy group.

### Production Method 10

Among examples of the compound represented by the general formula (1), the compound represented by the general formula (1g) wherein Q is the above-defined Q1 1 can be produced from the compound represented by the general formula (2f) through the step [c'] described below. The reaction conditions for the step [c'] are the same as described above.

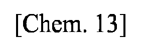

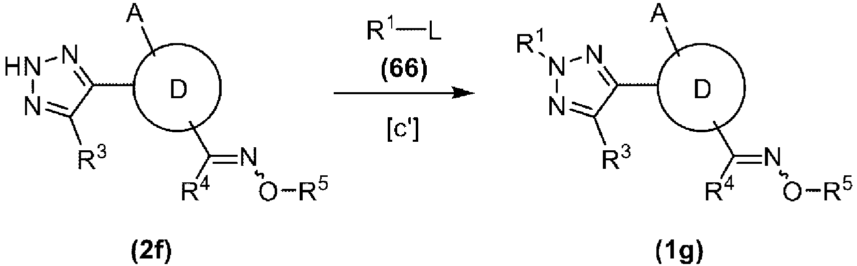

In the formula, A, D, R¹, R³, R⁴, and R⁵ are the same as above; and L represents a leaving group such as chlorine, bromine, iodine, or a (C₁-C₆) alkylcarbonyloxy group.

### Production method 1 for starting compound

Among examples of the compound represented by the general formula (2a) as the starting compound for Production Methods 1 and 2, the compound represented by the general formula (2a-1) wherein Q is the above-defined Q1 and A is the above-defined SO₂N(R⁹)R¹⁰ can be produced from the compound represented by the general formula (14) through the steps [l], [m], [n], [o], [f], and [p] described below. The reaction conditions for the steps [l] and [f] are the same as described above.

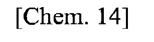

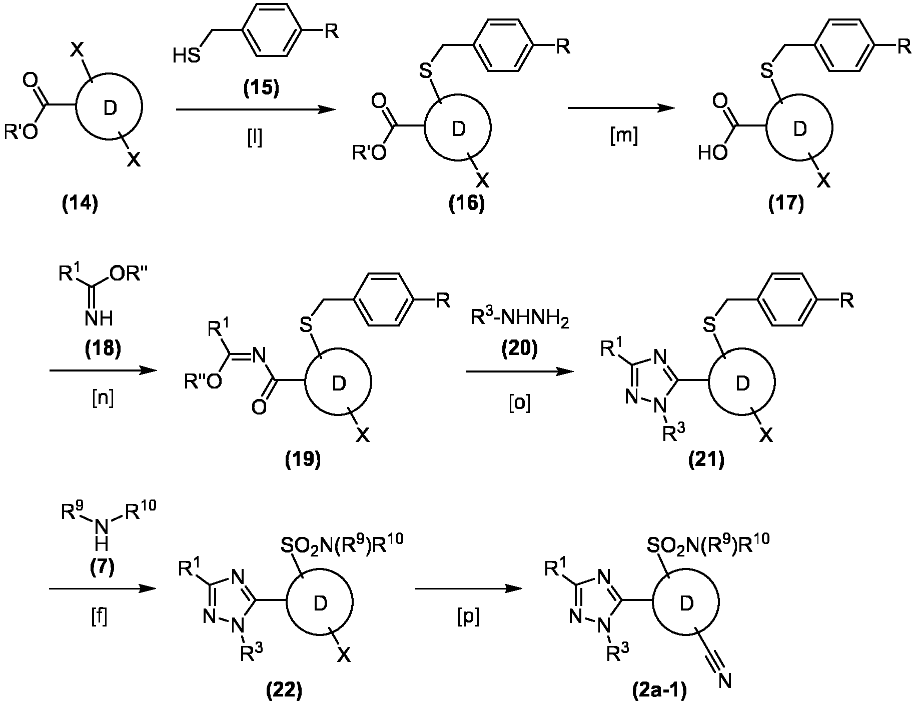

In the formula, D, R¹, R³, R⁹, and R¹⁰ are the same as above; R represents a (C₁-C₄) alkyl group such as a tert-butyl group or a hydrogen atom; R' and R" represent a (C₁-C₆) alkyl group such as a methyl group or an ethyl group; and X represents a leaving group such as chlorine, bromine, or iodine.

### Production method at step [m]

The compound represented by the general formula (17) can be produced by hydrolyzing the compound represented by the general formula (16) in the presence of a base, water, and an inert solvent.

Examples of the base that can be used in this reaction include organic bases such as lithium hydroxide, sodium hydroxide, potassium hydroxide, calcium hydroxide and other hydroxides. The amount of the base used is usually in the range of a 1- to 10-fold molar amount relative to the compound represented by the general formula (16).

The inert solvent used in this reaction may be any solvent that does not markedly inhibit the reaction, and examples include chain or cyclic saturated hydrocarbons such as pentane, hexane, and cyclohexane; chain or cyclic ethers such as diethyl ether, tetrahydrofuran (THF), and dioxane; and nonpolar solvents including aromatic hydrocarbons such as benzene, toluene, and xylene. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (16).

The reaction temperature in this reaction is usually in the range of 0°C to the boiling point of the solvent used. The reaction time varies with the reaction scale, the reaction temperature and the like and is not the same in every case, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [n]

The compound represented by the general formula (19) can be produced by reacting the compound represented by the general formula (17) with the compound represented by the general formula (18) in the presence of a base, a condensing agent, and an inert solvent.

Examples of the condensing agent that can be used in this condensation include acid-activating reagents such as phosgene, phosphorus trichloride, phosphorus oxychloride, oxalyl chloride, and thionyl chloride; carbodiimides such as N,N'-dicyclohexylcarbodiimide (DCC) and 1-(3-dimethylaminopropyl)-3-ethylcarbodiimide (EDCI); and other reagents such as phosphorus pentoxide, polyphosphoric acid, N,N'-carbonyldiimidazole, 2-chloropyridine-1-methoiodide (Mukaiyama reagent), 2-ethoxy-N-ethoxycarbonyl-1,2-dihydroquinoline (EEDQ), triphenylphosphine/carbon tetrachloride, bromotripyrrolidinophosphonium hexafluorophosphate (BROP), O-(1H-benzotriazol-1-yloxy)tris(dimethylamino)phosphonium hexafluorophosphate (BOP), N,N,N',N'-bis(tetramethylene)chlorouronium tetrafluoroborate, O-(1H-benzotriazol-1-yl)-N,N,N',N'-tetramethyluronium hexafluorophosphate (HBTU), O-(1H-benzotriazol-1 -yl)-N,N,N',N'-bis(tetramethylene)uronium hexafluorophosphate, O-(1H-benzotriazol-1-yl)-N,N,N',N'-tetramethyluronium tetrafluoroborate (TBTU), O-(1H-benzotriazol-1-yl)-N,N,N',N'-bis(tetramethylene)uronium tetrafluoroborate, O-(7-azabenzotriazol-1-yl)-N,N,N',N'-tetramethyluronium hexafluorophosphate (HATU), 1-hydroxybenzotriazole (HOBt), propylphosphonic anhydride (T₃P), and 4-(4,6-dimethoxy-1,3,5-triazin-2-yl)-4-methylmorpholinium salt (DMT-MM). One of these condensing agents may be used alone, and also two or more of them may be used as a mixture. The amount of the condensing agent used is usually selected as appropriate from the range of a 0.5- to 5-fold molar amount relative to the compound represented by the general formula (17).

Examples of the base that can be used in the condensation include carbonates such as lithium carbonate, lithium hydrogen carbonate, sodium carbonate, sodium hydrogen carbonate, potassium carbonate, cesium carbonate, potassium hydrogen carbonate, calcium carbonate and magnesium carbonate; acetates such as lithium acetate, sodium acetate and potassium acetate; and organic bases such as pyridine, picoline, lutidine, triethylamine, tributylamine and N,N-diisopropylethylamine. The amount of the base used is usually selected as appropriate from the range of a 0.5- to 5-fold molar amount relative to the compound represented by the general formula (17). In some cases, the base can be used as the solvent as well.

The inert solvent used in the condensation may be any solvent that does not markedly inhibit the reaction, and examples include chain or cyclic saturated hydrocarbons such as pentane, hexane, and cyclohexane; chain or cyclic ethers such as diethyl ether, THF, and dioxane; aromatic hydrocarbons such as benzene, toluene, and xylene; halogenated hydrocarbons such as methylene chloride, chloroform, and carbon tetrachloride; nitriles such as acetonitrile and isopropylnitrile; and polar solvents such as N,N-dimethylformamide, N,N-dimethylacetamide, dimethyl sulfoxide, and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (17). In the case where the base is used also as the solvent, it is not necessary to use another solvent.

Since the condensation is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [o]

The compound represented by the general formula (21) can be produced by reacting the compound represented by the general formula (19) with the compound represented by the general formula (20) or a salt thereof in the presence of an inert solvent with or without a base.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (19).

The inert solvent used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (19).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [p]

The compound represented by the general formula (2a-1) can be produced by reacting the compound represented by the general formula (22) with a cyanizing agent in the presence of a base and an inert solvent with or without a metal catalyst.

Examples of the cyanizing agent that can be used in this reaction include sodium cyanide, potassium cyanide, trimethylsilyl cyanide, and zinc cyanide. The amount of the cyanizing agent used is usually in the range of a 1- to 10-fold molar amount relative to the compound represented by the general formula (22).

Examples of the base that can be used in this reaction include carbonates such as lithium carbonate, lithium hydrogen carbonate, sodium carbonate, sodium hydrogen carbonate, potassium carbonate, cesium carbonate, potassium hydrogen carbonate, calcium carbonate, and magnesium carbonate; acetates such as lithium acetate, sodium acetate, and potassium acetate; and organic bases such as pyridine, picoline, lutidine, triethylamine, tributylamine, N,N-diisopropylethylamine, and 1,4-diazabicyclo[2.2.2]octane. The amount of the base used is usually in the range of a 1- to 5-fold molar amount relative to the compound represented by the general formula (22).

Examples of the metal catalyst that can be used in this reaction include a palladium catalyst, a nickel catalyst, an iron catalyst, a ruthenium catalyst, a platinum catalyst, a rhodium catalyst and an iridium catalyst. Such a metal catalyst can be used in the form of a metal; a supported metal; a metal salt such as a metal chloride, a metal bromide, a metal iodide, a metal nitrate, a metal sulfate, a metal carbonate, a metal oxalate, a metal acetate and a metal oxide; or a complex compound such as an olefin complex, a phosphine complex, an amine complex, an ammine complex and an acetylacetonate complex.

Among these metal catalysts, palladium catalysts are particularly preferred. Examples of the palladium catalyst include palladium metals such as palladium black and palladium sponge; and supported palladium metals such as palladium/alumina, palladium/carbon, palladium/silica and palladium/type Y zeolite. Also included are palladium metal salts such as palladium chloride, palladium bromide, palladium iodide and palladium acetate. Other examples of the palladium catalyst include palladium complex compounds such as π-allylpalladium chloride dimer, palladium acetylacetonate, dichlorobis(acetonitrile)palladium, dichlorobis(benzonitrile)palladium, bis(dibenzylideneacetone)palladium, tris(dibenzylideneacetone)dipalladium, tris(dibenzylideneacetone)dipalladium (chloroform adduct), dichlorodiamine palladium, dichlorobis(triphenylphosphine)palladium, dichlorobis(tricyclohexylphosphine)palladium, tetrakis(triphenylphosphine)palladium, dichloro[1,2-bis(diphenylphosphino)ethane]palladium, dichloro[1,3-bis(diphenylphosphino)propane]palladium, dichloro [1,4-bis(diphenylphosphino)butane]palladium, dichloro[1,1'-bis(diphenylphosphino)ferrocene]palladium and a [(diphenylphosphino)ferrocene]dichloropalladium-dichloromethane complex. The amount of the metal catalyst used is usually selected as appropriate from the range of a 0.001- to 0.5-fold molar amount relative to the compound represented by the general formula (22).

These palladium catalysts may be used alone or in combination with a tertiary phosphine. Examples of the tertiary phosphine that can be used in combination with the palladium catalyst include triphenylphosphine, trimethylphosphine, triethylphosphine, tributylphosphine, tri(tert-butyl)phosphine, tricyclohexylphosphine, tri-o-tolylphosphine, trioctylphosphine, 9,9-dimethyl-4,5-bis(diphenylphosphino)xanthene, 2-(di-tert-butylphosphino)biphenyl, 2-(dicyclohexylphosphino)biphenyl, 1,2-bis(diphenylphosphino)ethane, 1,3-bis(diphenylphosphino)propane, 1,4-bis(diphenylphosphino)butane, 1,1'-bis(diphenylphosphino)ferrocene, (R)-(+)-2,2'-bis(diphenylphosphino)-1,1'-binaphthyl, (S)-(-)-2,2'-bis(diphenylphosphino)-1,1'-binaphthyl and (±)-2,2'-bis(diphenylphosphino)-1,1'-binaphthyl. The amount of the tertiary phosphine used is usually selected as appropriate from the range of a 0.5- to 10-fold molar amount relative to the metal catalyst.

The inert solvent used in this reaction may be any solvent that does not markedly inhibit the reaction, and examples include alcohols such as methanol, ethanol, propanol, butanol, and 2-propanol; chain or cyclic saturated hydrocarbons such as pentane, hexane, and cyclohexane; aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, tetrahydrofuran, and cyclopentyl methyl ether; esters such as ethyl acetate; nitriles such as acetonitrile and propionitrile; amides such as N,N-dimethylformamide and N,N-dimethylacetamide; ketones such as acetone and methyl ethyl ketone; polar solvents such as dimethyl sulfoxide and 1,3-dimethyl-2-imidazolidinone; and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (22).

The reaction temperature in this reaction is usually in the range of 0°C to the boiling point of the solvent used. The reaction time varies with the reaction scale, the reaction temperature and the like and is not the same in every case, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next reaction without isolation of the compound of interest.

### Production method 2 for starting compound

Among examples of the compound represented by the general formula (2a) as the starting compound for Production Methods 1 and 2, the compound represented by the general formula (2a-2) wherein Q is the above-defined Q2 and A is the above-defined SO₂N(R⁹)R¹⁰ can be produced from the compound represented by the general formula (17) through the steps [q], [r], [s], [f], and [p] described below. The reaction conditions for the steps [f] and [p] are the same as described above.

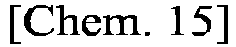

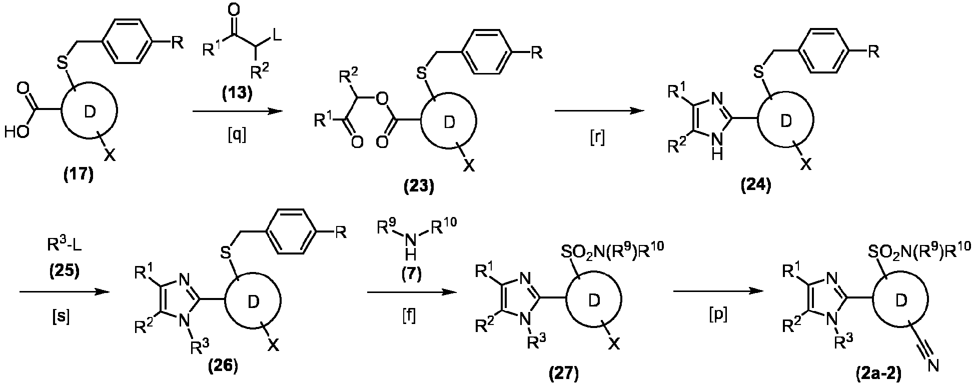

In the formula, D, R¹, R², R³, R⁹, and R¹⁰ are the same as above; R represents a (C₁-C₄) alkyl group such as a tert-butyl group or a hydrogen atom; X represents a leaving group such as chlorine, bromine, or iodine; and L represents a leaving group such as chlorine, bromine, iodine, or a (C₁-C₆) alkylcarbonyloxy group.

### Production method at step [q]

The compound represented by the general formula (23) can be produced by reacting the compound represented by the general formula (17) with the compound represented by the general formula (13) in the presence of a base and an inert solvent.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (17).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (17).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [r]

The compound represented by the general formula (24) can be produced by reacting the compound represented by the general formula (23) with a nitrogen source in the presence of an acid and an inert solvent.

Examples of the nitrogen source that can be used in this reaction include ammonia, ammonium carbamate, ammonium acetate, and ammonium carbonate. The amount of the nitrogen source used is usually selected as appropriate from the range of a 1- to 20-fold molar amount relative to the compound represented by the general formula (23).

Examples of the acid that can be used in this reaction include inorganic acids such as hydrochloric acid, sulfuric acid and nitric acid; organic acids such as formic acid, acetic acid, propionic acid, trifluoroacetic acid and benzoic acid; sulfonic acids such as methanesulfonic acid, trifluoromethanesulfonic acid and p-toluenesulfonic acid; and phosphoric acid. The amount of the acid used is usually selected as appropriate from the range of a 0.01- to 10-fold molar amount relative to the compound represented by the general formula (23). In some cases, the acid can be used as the solvent as well.

The inert solvent used in the dehydration may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (23) obtained by the condensation. In the case where the acid is used also as the solvent, it is not necessary to use another solvent.

The reaction temperature in the dehydration is usually in the range of room temperature to the boiling point of the solvent used. The reaction time varies with the reaction scale and the reaction temperature, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [s]

The compound represented by the general formula (26) can be produced by reacting the compound represented by the general formula (24) with the compound represented by the general formula (25) in the presence of an inert solvent and a base.

Examples of the base that can be used in this reaction include inorganic bases such as sodium hydroxide, potassium hydroxide, sodium carbonate, potassium carbonate, cesium carbonate, sodium hydrogen carbonate, potassium hydrogen carbonate and sodium hydride; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (24).

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (24).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest.

### Production method 3 for starting compound

Among examples of the compound represented by the general formula (2a) as the starting compound for Production Methods 1 and 2, the compound represented by the general formula (2a-3) wherein Q is the above-defined Q5 and A is the above-defined CO₂R⁷ can be produced from the compound represented by the general formula (28) through the steps [t], [u], [v], [w], [x], [j], and [y] described below. The reaction conditions for the step [j] are the same as described above.

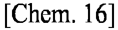

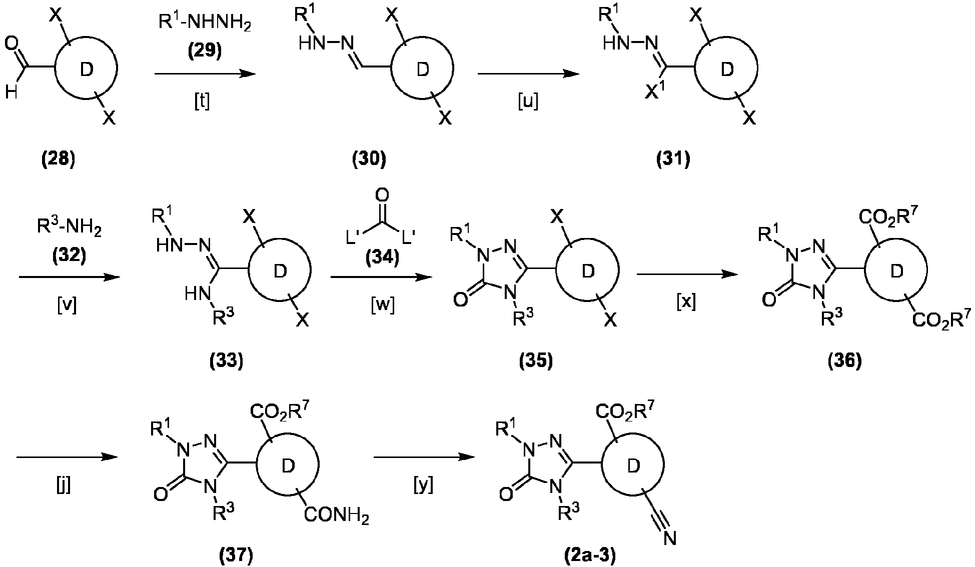

In the formula, D, R¹, R³, and R⁷ are the same as above; X represents a leaving group such as chlorine, bromine, or iodine; X¹ represents a halogen atom such as chlorine, bromine, or iodine; and L' represents a leaving group such as chlorine, bromine, iodine, a halo (C₁-C₆) alkoxy group, or an imidazolyl group.

### Production method at step [t]

The compound represented by the general formula (30) can be produced by reacting the compound represented by the general formula (28) with the compound represented by the general formula (29) in the presence of an inert solvent with or without an acid.

Examples of the acid that can be used in this reaction include inorganic acids such as hydrochloric acid, sulfuric acid and nitric acid; organic acids such as formic acid, acetic acid, propionic acid, trifluoroacetic acid and benzoic acid; sulfonic acids such as methanesulfonic acid, trifluoromethanesulfonic acid and p-toluenesulfonic acid; and phosphoric acid. The amount of the acid used is usually in the range of a 0.01- to 10-fold molar amount relative to the compound represented by the general formula (28).

The inert solvent used in this reaction may be any solvent that does not markedly inhibit the progress of the reaction, and examples include alcohols such as methanol, ethanol, propanol, butanol and 2-propanol; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (28) obtained by the condensation.

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [u]

The compound represented by the general formula (31) can be produced by reacting the compound represented by the general formula (30) with a halogenating agent in the presence of an inert solvent with or without a base.

Examples of the halogenating agent that can be used in this reaction include N-halosuccinimides such as N-chlorosuccinimide and N-bromosuccinimide; hypohalogenous alkali metal salts such as sodium hypochlorite; hypohalogenous acid esters such as tert-butyl hypochlorite; simple halogens such as chlorine gas; phosphorus pentachloride; and sulfuryl chloride. The amount of the halogenating agent used is usually in the range of a 1- to 10-fold molar amount relative to the compound represented by the general formula (30).

Examples of the base that can be used in this reaction include alkali metal hydrides such as sodium hydride; alkali metal hydroxides such as sodium hydroxide and potassium hydroxide; alkali metal carbonates such as sodium carbonate, potassium carbonate, and cesium carbonate; inorganic salts; and organic bases such as pyridine, DBU, and triethylamine. The amount of the base used is usually in the range of a 1- to 10-fold molar amount relative to the compound represented by the general formula (30).

The inert solvent used in this reaction may be any solvent that does not markedly inhibit the reaction, and examples include aromatic hydrocarbons such as benzene, toluene, and xylene; halogenated hydrocarbons such as chloroform and dichloromethane; esters such as ethyl acetate and methyl acetate; chain or cyclic ethers such as tetrahydrofuran, diethyl ether, methyl tert-butyl ether, 1,2-dimethoxyethane, and dioxane; nitriles such as acetonitrile; and polar solvents such as N,N-dimethylformamide, N,N-dimethylacetamide, N-methylpyrrolidone, dimethyl sulfoxide, and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (30).

The reaction temperature in this reaction is usually selected as appropriate from the range of -50°C to the boiling point of the inert solvent used. The reaction time varies with the reaction scale and the reaction temperature, but is basically in the range of a few minutes to 48 hours. After the reaction is completed, when the compound represented by the general formula (31) is stable, the compound of interest is isolated from the post-reaction mixture by the usual method. As needed, recrystallization, column chromatography, etc. can be employed for the purification of the compound of interest. Alternatively, the post-reaction mixture may be directly used in the next reaction without isolation of the compound of interest. When the compound represented by the general formula (31) is unstable, the post-reaction mixture is directly used in the next step without purification.

### Production method at step [v]

The compound represented by the general formula (33) can be produced by aminating the compound represented by the general formula (31) with the compound represented by the general formula (32) in the presence of an inert solvent with or without a base.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (31).

The inert solvent that can be used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; amides such as N,N-dimethylformamide and N,N-dimethylacetamide; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (31).

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [w]

The compound represented by the general formula (35) can be produced by reacting the compound represented by the general formula (33) with the compound represented by the general formula (34) in the presence of a base and an inert solvent.

Examples of the base that can be used in this reaction include inorganic bases including alkali metal hydroxides such as sodium hydroxide and potassium hydroxide; alkali metal hydrides such as sodium hydride and potassium hydride; alkali metal alkoxides such as sodium ethoxide and potassium tert-butoxide; and carbonates such as sodium carbonate, potassium carbonate, cesium carbonate and sodium hydrogen carbonate; and organic bases such as triethylamine, pyridine, N,N-dimethyl-4-aminopyridine and DBU. The amount of the base used is usually selected as appropriate from the range of a 1- to 10-fold molar amount relative to the compound represented by the general formula (33). In some cases, the base can be used as the solvent as well.

The inert solvent used in this reaction may be any solvent that does not markedly inhibit the progress of the reaction, and examples include chain or cyclic saturated hydrocarbons such as pentane, hexane, and cyclohexane; aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; nitriles such as acetonitrile and propionitrile; esters such as methyl acetate; ketones such as acetone and methyl ethyl ketone; aprotic polar solvents such as N,N-dimethylformamide, N,N-dimethylacetamide, dimethyl sulfoxide, and 1,3-dimethyl-2-imidazolidinone; and alcohols such as methanol, ethanol, propanol, butanol, and 2-propanol. One of these inert solvents may be used alone, and also two or more of them may be used as a mixture. The amount of the inert solvent used is usually selected as appropriate from the range of 0.1 to 100 L relative to 1 mol of the compound represented by the general formula (33). In the case where the base is used also as the solvent, it is not necessary to use another solvent.

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [x]

The compound represented by the general formula (36), which has ester groups introduced at C-3 and C-6 positions, can be synthesized from the compound represented by the general formula (35) according to the method described in JP 2005-272338 (Heck reaction). After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [y]

The compound represented by the general formula (2a-3) can be produced by reacting the compound represented by the general formula (37) with a dehydrating agent in the presence of an inert solvent with or without a base.

Examples of the dehydrating agent used in this reaction include trifluoroacetic anhydride, phosphorus oxychloride, and phosphorus pentoxide. The amount of the dehydrating agent used is usually in the range of a 1- to 10-fold molar amount relative to the compound represented by the general formula (37).

The base used in this reaction may be an inorganic base or an organic base. Examples of the inorganic base include alkali metal hydroxides such as sodium hydroxide and potassium hydroxide; alkali metal hydrides such as sodium hydride and potassium hydride; alkali metal alkoxides such as sodium ethoxide and potassium tert-butoxide; and carbonates such as sodium carbonate, potassium carbonate, cesium carbonate, and sodium hydrogen carbonate. Examples of the organic base include triethylamine, pyridine, and DBU. The amount of the base used is usually selected as appropriate from the range of a 0.01- to 10-fold molar amount relative to the compound represented by the general formula (37).

The inert solvent used in this reaction may be any solvent that does not markedly inhibit the progress of the reaction, and examples include aromatic hydrocarbons such as benzene, toluene and xylene; halogenated aliphatic hydrocarbons such as methylene chloride, chloroform and carbon tetrachloride; halogenated aromatic hydrocarbons such as chlorobenzene and dichlorobenzene; chain or cyclic ethers such as diethyl ether, dioxane and tetrahydrofuran; esters such as ethyl acetate; amides such as N,N-dimethylformamide and N,N-dimethylacetamide; and other inert solvents such as dimethyl sulfoxide, 1,3-dimethyl-2-imidazolidinone, acetone and methyl ethyl ketone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (37).

The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture is directly used in the next reaction without purification.

### Production method 4 for starting compound

Among examples of the compound represented by the general formula (2a) as the starting compound for Production Methods 1 and 2, the compound represented by the general formula (2a-4) wherein Q is the above-defined Q7 or Q12 and A is the above-defined CO₂R⁷ can be produced from the compound represented by the general formula (38) through the steps [z], [aa], [h], [i], [x], [j], and [y] described below. The reaction conditions for the steps [h], [i], [x], [j], and [y] are the same as described above.

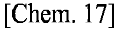

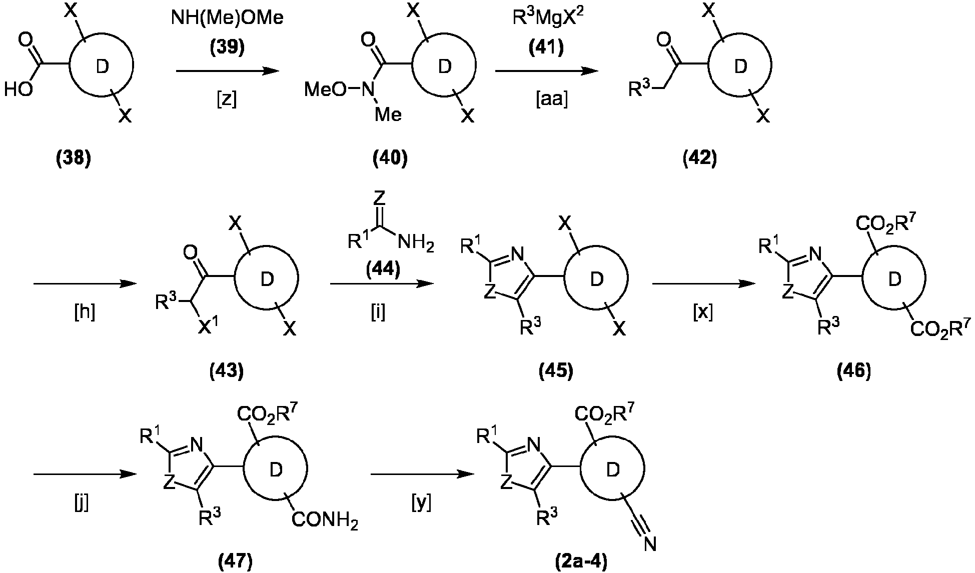

In the formula, D, R¹, R³, and R⁷ are the same as above; Z represents an oxygen atom or a sulfur atom; X represents a leaving group such as chlorine, bromine, or iodine; and X¹ and X² represent a halogen atom such as chlorine, bromine, or iodine.

### Production method at step [z]

The compound represented by the general formula (40) can be produced by reacting the compound represented by the general formula (38) with a halogenating agent in the presence of an inert solvent to form an acid halide and then reacting the acid halide with the compound represented by the formula (39) in the presence of an inert solvent with or without a base.

Examples of the halogenating agent that can be used in this hydrogenation include oxalyl chloride, thionyl chloride, phosphorus trichloride, phosphorus tribromide, phosphorus pentachloride, and phosphorus oxychloride. The amount of the halogenating agent used is usually in the range of a 1- to 10-fold molar amount relative to the compound represented by the general formula (38).

The inert solvent used in this hydrogenation may be any solvent that does not markedly inhibit the reaction, and examples include aromatic hydrocarbons such as benzene, toluene, and xylene; halogenated hydrocarbons such as chloroform and dichloromethane; esters such as ethyl acetate and methyl acetate; chain or cyclic ethers such as tetrahydrofuran, diethyl ether, methyl tert-butyl ether, 1,2-dimethoxyethane, and dioxane; nitriles such as acetonitrile; and polar solvents such as N,N-dimethylformamide, N,N-dimethylacetamide, N-methylpyrrolidone, and dimethyl sulfoxide. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (38).

The reaction temperature in the hydrogenation is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. Alternatively, the post-reaction mixture is directly used in the next reaction without purification.

Examples of the base that can be used in the amidation include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (38).

The inert solvent used in the amidation may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; amides such as N,N-dimethylformamide and N,N-dimethylacetamide; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (38).

Since the amidation is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture is directly used in the next reaction without purification.

### Production method at step [aa]

The compound represented by the general formula (42) can be produced by reacting the compound represented by the general formula (40) with an organomagnesium compound represented by the general formula (41) in the presence of an inert solvent.

The inert solvent used in this reaction may be any solvent that does not markedly inhibit the reaction, and examples include aromatic hydrocarbons such as benzene, toluene, and xylene; halogenated hydrocarbons such as chloroform and dichloromethane; and polar solvents including chain or cyclic ethers such as tetrahydrofuran, diethyl ether, methyl tert-butyl ether, 1,2-dimethoxyethane, and dioxane. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (40).

Since this reaction is an equimolar reaction of the compounds, they are basically used in equimolar amounts, but either of them may be used in an excess amount. The reaction temperature in this reaction is usually in the range of -78°C to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture is directly used in the next reaction without purification.

### Production method 5 for starting compound

Among examples of the compound represented by the general formula (2b) as the starting compound for Production Method 3, the compound represented by the general formula (2b-1) wherein A is the above-defined SO₂N(R⁹)R¹⁰ can be produced from the compound represented by the general formula (48) through the steps [ab], [ac], [p], and [a'] described below. The reaction conditions for the steps [p] and [a'] are the same as described above.

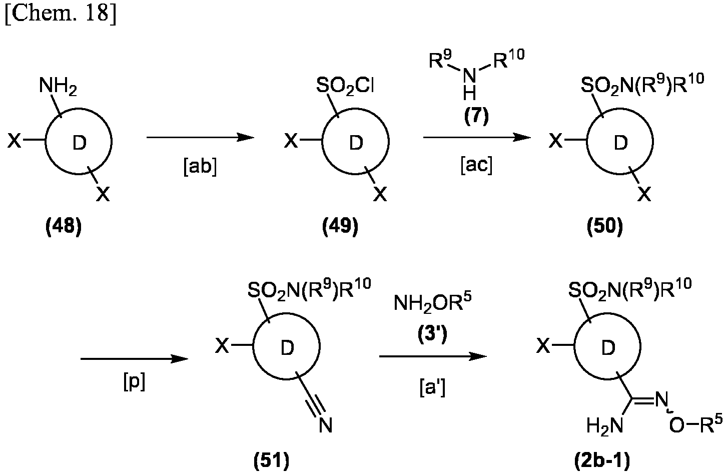

In the formula, D, R⁵, R⁹, and R¹⁰ are the same as above; and X represents a leaving group such as chlorine, bromine, or iodine.

### Production method at step [ab]

The compound represented by the general formula (49) can be produced through chlorosulfonylation of the compound represented by the general formula (48) according to the method described in Organic Process Research & Development, 2009, 13, 875-879 or WO 2020/041169.

After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [ac]

The compound represented by the general formula (50) can be produced by reacting the compound represented by the general formula (49) with the compound represented by the general formula (7) in the presence of an inert solvent with or without a base.

Examples of the base that can be used in this reaction include inorganic bases such as sodium hydroxide, potassium hydroxide, sodium carbonate, potassium carbonate, cesium carbonate, sodium hydrogen carbonate and potassium hydrogen carbonate; acetates such as sodium acetate and potassium acetate; alkali metal alkoxides such as potassium tert-butoxide, sodium methoxide and sodium ethoxide; tertiary amines such as triethylamine, N,N-diisopropylethylamine and DBU; and nitrogen-containing aromatic compounds such as pyridine and N,N-dimethyl-4-aminopyridine. The amount of the base used is usually in the range of a 1- to 10-fold molar amount relative to the compound represented by the general formula (49).

The inert solvent that can be used in this reaction may be any solvent that does not markedly inhibit the progress of the reaction, and examples include aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; alcohols such as methanol, ethanol, propanol, and isopropyl alcohol; amides such as N,N-dimethylformamide and N,N-dimethylacetamide; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (49).

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale and the reaction temperature, but is basically in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method 6 for starting compound

Among examples of the compound represented by the general formula (2d) as the starting compound for Production Method 7, the compound represented by the general formula (2d-1) wherein A is the above-defined SO₂R⁸ can be produced from the compound represented by the general formula (14) through the steps [l], [ad], [p], and [a'] described below. Among examples of the compound represented by the general formula (2b) as the starting compound for Production Method 3, the compound represented by the general formula (2b-2) wherein A is the above-defined SO₂R⁸ can be produced from the compound represented by the general formula (2d-1) through the steps [m], [ae], and [af] described below. The reaction conditions for the steps [l], [p], [a'], and [m] are the same as described above.

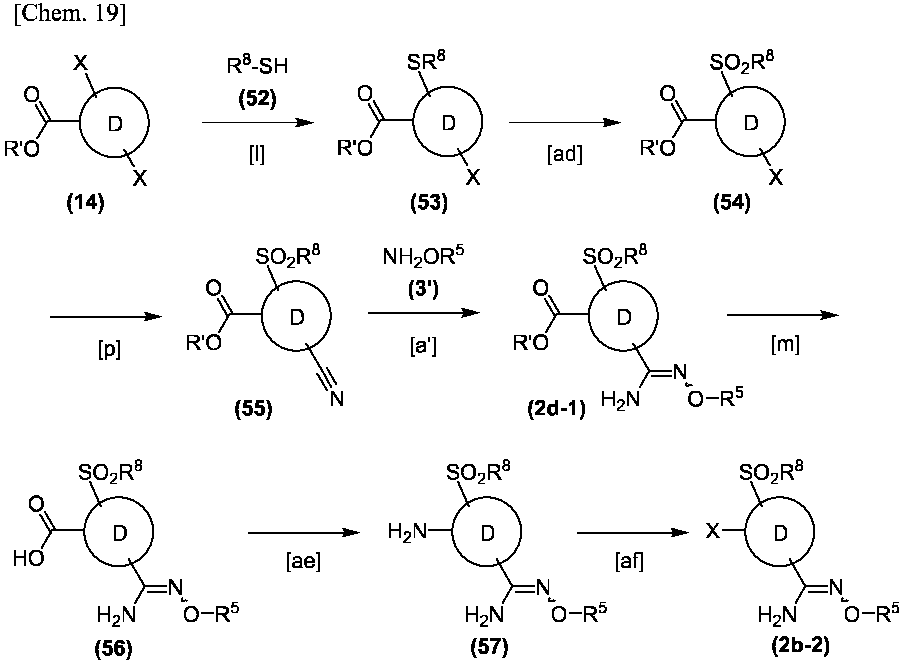

In the formula, D, R⁵, and R⁸ are the same as above; X represents a leaving group such as chlorine, bromine, or iodine; and R' represents a (C₁-C₆) alkyl group such as a methyl group or an ethyl group.

### Production method at step [ad]

The compound represented by the general formula (54) can be produced by reacting the compound represented by the general formula (53) with an oxidizing agent in the presence of an inert solvent.

Examples of the oxidizing agent that can be used in this reaction include peroxides such as a hydrogen peroxide solution, peroxybenzoic acid and m-chloroperoxybenzoic acid. The amount of the oxidizing agent used is usually in the range of a 1- to 5-fold molar amount relative to the compound represented by the general formula (53).

The inert solvent that can be used in this reaction may be any solvent that does not markedly inhibit the reaction, and examples include chain or cyclic ethers such as diethyl ether, tetrahydrofuran and dioxane; aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; nitriles such as acetonitrile; esters such as ethyl acetate; organic acids such as formic acid and acetic acid; and polar solvents such as N,N-dimethylformamide, N,N-dimethylacetamide, 1,3-dimethyl-2-imidazolidinone and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (53).

The reaction temperature in this reaction is usually in the range of -10°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [ae]

The compound represented by the general formula (57) can be produced by reacting the compound represented by the general formula (56) with azide diphenyl phosphate in the presence of tert-butyl alcohol according to the Curtius rearrangement, that is, the method described in J. A. Chem. Soc. 1972, 94, 6203-6205, followed by treatment with an acid and an inert solvent.

Examples of the acid that can be used in this reaction include inorganic acids such as hydrochloric acid, sulfuric acid and nitric acid; organic acids such as formic acid, acetic acid, propionic acid, trifluoroacetic acid and benzoic acid; and sulfonic acids such as methanesulfonic acid and trifluoromethanesulfonic acid. The amount of the acid used is usually selected as appropriate from the range of a 1- to 10-fold molar amount relative to the compound represented by the general formula (56). In some cases, the acid can be used as the solvent as well.

The inert solvent used in this reaction may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (56).

The reaction temperature in this reaction is usually in the range of -10°C to the boiling point of the solvent used. The reaction time varies with the reaction scale and the reaction temperature, but is usually in the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method at step [af]

The compound represented by the general formula (2b-2) can be produced by conversion of the amino group of the compound represented by the general formula (57) to a halogen atom according to the method described in Chem. Rev. 1988, 88, 765, i.e., the Sandmeyer reaction.

After the reaction is completed, the compound of interest is isolated from the post-reaction mixture by the usual method. As needed, recrystallization, column chromatography, etc. can be employed for the purification of the compound of interest.

### Production method 7 for starting compound

Among examples of the compound represented by the general formula (2c) as the starting compound for Production Method 4, the compound represented by the general formula (2c-1) wherein Q is the above-defined Q1 can be produced from the compound represented by the general formula (38) through the steps [ag], [p], [l], [a], and [b] described below. The reaction conditions for the steps [p], [l], [a], and [b] are the same as described above. The compound represented by the general formula (58) can be produced by the method described in Chem. Ber., 1982, 115, 2807-2818 or WO 2018/160845, or its modified method.

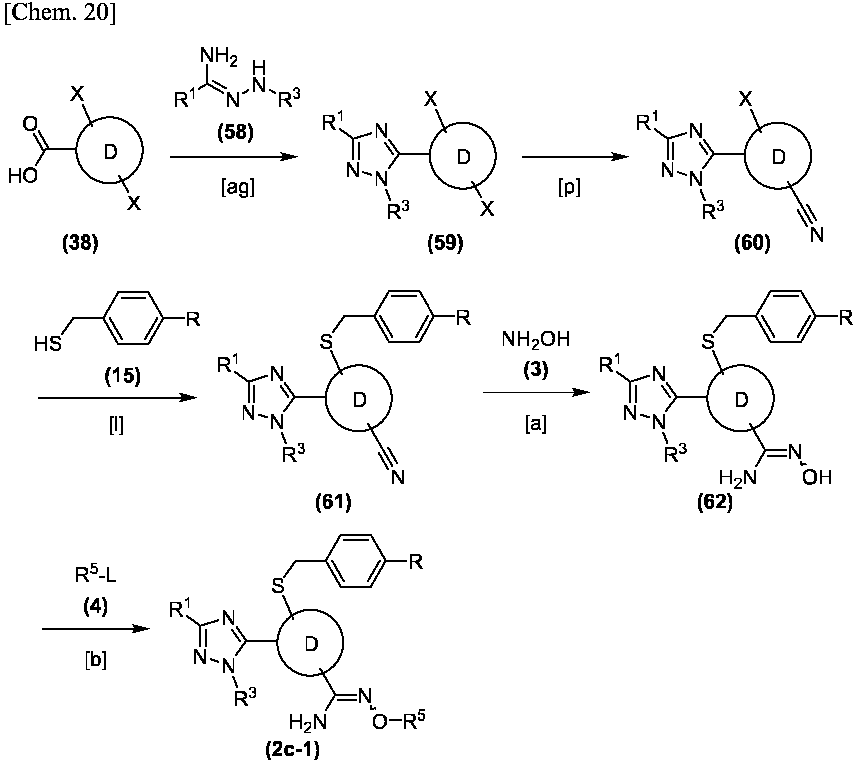

In the formula, D, R¹, R³, and R⁵ are the same as above; L represents a leaving group such as chlorine, bromine, iodine, or a (C₁-C₆) alkylcarbonyloxy group; R represents a (C₁-C₄) alkyl group such as a tert-butyl group or a hydrogen atom; and X represents a leaving group such as chlorine, bromine, or iodine.

### Production method at step [ag]

The compound represented by the general formula (59) can be produced by condensation of the compound represented by the general formula (38) with the compound represented by the general formula (58) in the presence of a base, a condensing agent and an inert solvent to form an amide compound, followed by dehydration in the presence of an acid and an inert solvent.

Examples of the condensing agent that can be used in this condensation include acid-activating reagents such as phosgene, phosphorus trichloride, phosphorus oxychloride, oxalyl chloride, and thionyl chloride; carbodiimides such as N,N'-dicyclohexylcarbodiimide (DCC) and 1-(3-dimethylaminopropyl)-3-ethylcarbodiimide (EDCI); and other reagents such as phosphorus pentoxide, polyphosphoric acid, N,N'-carbonyldiimidazole, 2-chloropyridine-1-methoiodide (Mukaiyama reagent), 2-ethoxy-N-ethoxycarbonyl-1,2-dihydroquinoline (EEDQ), triphenylphosphine/carbon tetrachloride, bromotripyrrolidinophosphonium hexafluorophosphate (BROP), O-(1H-benzotriazol-1-yloxy)tris(dimethylamino)phosphonium hexafluorophosphate (BOP), N,N,N',N'-bis(tetramethylene)chlorouronium tetrafluoroborate, O-(1H-benzotriazol-1-yl)-N,N,N',N'-tetramethyluronium hexafluorophosphate (HBTU), O-(1H-benzotriazol-1 -yl)-N,N,N',N'-bis(tetramethylene)uronium hexafluorophosphate, O-(1H-benzotriazol-1-yl)-N,N,N',N'-tetramethyluronium tetrafluoroborate (TBTU), O-(1H-benzotriazol-1-yl)-N,N,N',N'-bis(tetramethylene)uronium tetrafluoroborate, O-(7-azabenzotriazol-1-yl)-N,N,N',N'-tetramethyluronium hexafluorophosphate (HATU), 1-hydroxybenzotriazole (HOBt), propylphosphonic anhydride (T₃P), and 4-(4,6-dimethoxy-1,3,5-triazin-2-yl)-4-methylmorpholinium salt (DMT-MM). One of these condensing agents may be used alone, and also two or more of them may be used as a mixture. The amount of the condensing agent used is usually in the range of a 0.5- to 5-fold molar amount relative to the compound represented by the general formula (38).

Examples of the base that can be used in the condensation include carbonates such as lithium carbonate, lithium hydrogen carbonate, sodium carbonate, sodium hydrogen carbonate, potassium carbonate, cesium carbonate, potassium hydrogen carbonate, calcium carbonate and magnesium carbonate; acetates such as lithium acetate, sodium acetate and potassium acetate; and organic bases such as pyridine, picoline, lutidine, triethylamine, tributylamine and N,N-diisopropylethylamine. The amount of the base used is usually selected as appropriate from the range of a 0.5- to 5-fold molar amount relative to the compound represented by the general formula (38). In some cases, the base can be used as the solvent as well.

The inert solvent used in the condensation may be any solvent that does not markedly inhibit the reaction, and examples include chain or cyclic saturated hydrocarbons such as pentane, hexane, and cyclohexane; chain or cyclic ethers such as diethyl ether, THF, and dioxane; aromatic hydrocarbons such as benzene, toluene, and xylene; halogenated hydrocarbons such as methylene chloride, chloroform, and carbon tetrachloride; nitriles such as acetonitrile and isopropylnitrile; and polar solvents such as N,N-dimethylformamide, N,N-dimethylacetamide, dimethyl sulfoxide, and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (38). In the case where the base is used also as the solvent, it is not necessary to use another solvent.

Since the condensation is an equimolar reaction of the compounds, they are basically used in equimolar amounts, but either of them may be used in an excess amount. The reaction temperature in the condensation is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next dehydration without isolation of the compound of interest.

Examples of the acid that can be used in the dehydration include inorganic acids such as hydrochloric acid, sulfuric acid and nitric acid; organic acids such as formic acid, acetic acid, propionic acid, trifluoroacetic acid and benzoic acid; sulfonic acids such as methanesulfonic acid, trifluoromethanesulfonic acid and p-toluenesulfonic acid; and phosphoric acid. The amount of the acid used is usually selected as appropriate from the range of a 0.01- to 10-fold molar amount relative to the amide compound. In some cases, the acid can be used as the solvent as well.

The inert solvent used in the dehydration may be any solvent that does not markedly inhibit the progress of the reaction, and examples include aromatic hydrocarbons such as benzene, toluene and xylene; halogenated hydrocarbons such as methylene chloride, chloroform and carbon tetrachloride; halogenated aromatic hydrocarbons such as chlorobenzene and dichlorobenzene; chain or cyclic ethers such as diethyl ether, methyl tert-butyl ether, dioxane and tetrahydrofuran; esters such as ethyl acetate; amides such as N,N-dimethylformamide and N,N-dimethylacetamide; ketones such as acetone and methyl ethyl ketone; and polar solvents such as dimethyl sulfoxide and 1,3-dimethyl-2-imidazolidinone. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the amide compound obtained by the condensation. In the case where the acid is used also as the solvent, it is not necessary to use another solvent.

The reaction temperature in the dehydration is usually in the range of room temperature to the boiling point of the solvent used. The reaction time varies with the reaction scale and the reaction temperature, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method 8 for starting compound

Among examples of the compound represented by the general formula (2a) as the starting compound for Production Methods 1 and 2, the compound represented by the general formula (2a-5) wherein Q is the above-defined Q5 and A is the above-defined SO₂N(R⁹)R¹⁰ can be produced from the compound represented by the general formula (16) through the steps [ah], [ai], [c'], [f], and [p] described below. The reaction conditions for the steps [c'], [f], and [p] are the same as described above.

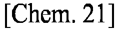

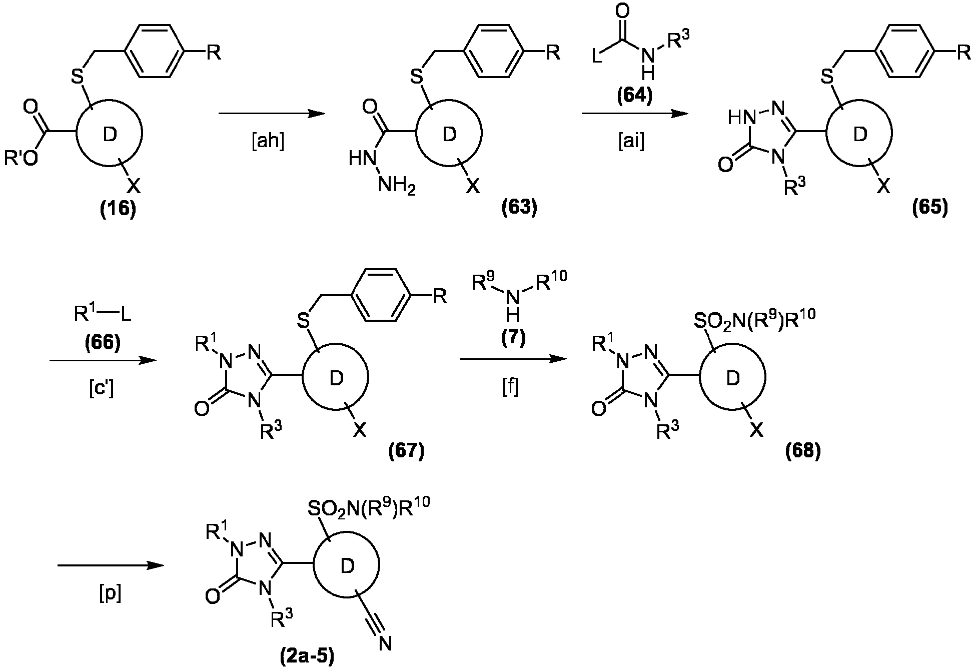

In the formula, D, R¹, R³, R⁹, and R¹⁰ are the same as above; R' represents a (C₁-C₆) alkyl group such as a methyl group or an ethyl group; X represents a leaving group such as chlorine, bromine, or iodine; and L represents a leaving group such as chlorine, bromine, iodine, or a (C₁-C₆) alkylcarbonyloxy group.

### Production method at step [ah]

The compound represented by the general formula (63) can be produced by reacting the compound represented by the general formula (16) with hydrazine in the presence of an inert solvent with or without a base.

The hydrazine used in this reaction can be in the form of a salt such as hydrochloride or a hydrate. The amount of the hydrazine used is usually in the range of a 1- to 10-fold molar amount relative to the compound represented by the general formula (16).

The base used in this reaction may be an inorganic base or an organic base. Examples of the inorganic base include alkali metal hydroxides such as sodium hydroxide and potassium hydroxide; alkali metal hydrides such as sodium hydride and potassium hydride; alkali metal alkoxides such as sodium ethoxide and potassium tert-butoxide; and carbonates such as sodium carbonate, potassium carbonate, cesium carbonate, and sodium hydrogen carbonate. Examples of the organic base include triethylamine, pyridine, and DBU. The amount of the base used is usually in the range of a 0.01- to 10-fold molar amount relative to the compound represented by the general formula (16).

The inert solvent used in this reaction may be any solvent that does not markedly inhibit the progress of the reaction, and examples include aromatic hydrocarbons such as benzene, toluene, and xylene; halogenated aliphatic hydrocarbons such as methylene chloride, chloroform, and carbon tetrachloride; halogenated aromatic hydrocarbons such as chlorobenzene and dichlorobenzene; chain or cyclic ethers such as diethyl ether, dioxane, and tetrahydrofuran; esters such as ethyl acetate; amides such as N,N-dimethylformamide and N,N-dimethylacetamide; and other inert solvents such as dimethyl sulfoxide, 1,3-dimethyl-2-imidazolidinone, acetone, methyl ethyl ketone, and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (16).

The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture is directly used in the next reaction without purification.

### Production method at step [ai]

The compound represented by the general formula (65) can be produced by reacting the compound represented by the general formula (63) with the compound represented by the general formula (64) in the presence of a base and an inert solvent.

Examples of the base that can be used in this reaction include inorganic bases including alkali metal hydroxides such as sodium hydroxide and potassium hydroxide; alkali metal hydrides such as sodium hydride and potassium hydride; alkali metal alkoxides such as sodium ethoxide and potassium tert-butoxide; and carbonates such as sodium carbonate, potassium carbonate, cesium carbonate and sodium hydrogen carbonate; and organic bases such as triethylamine, pyridine, N,N-dimethyl-4-aminopyridine and DBU. The amount of the base used is usually selected as appropriate from the range of a 1- to 10-fold molar amount relative to the compound represented by the general formula (63). In some cases, the base can be used as the solvent as well.

The inert solvent used in this reaction may be any solvent that does not markedly inhibit the progress of the reaction, and examples include chain or cyclic saturated hydrocarbons such as pentane, hexane, and cyclohexane; aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; chain or cyclic ethers such as diethyl ether, methyl tert-butyl ether, dioxane, and tetrahydrofuran; nitriles such as acetonitrile and propionitrile; esters such as methyl acetate; ketones such as acetone and methyl ethyl ketone; aprotic polar solvents such as N,N-dimethylformamide, N,N-dimethylacetamide, dimethyl sulfoxide, and 1,3-dimethyl-2-imidazolidinone; and alcohols such as methanol, ethanol, propanol, butanol, and 2-propanol. One of these inert solvents may be used alone, and also two or more of them may be used as a mixture. The amount of the inert solvent used is usually selected as appropriate from the range of 0.1 to 100 L relative to 1 mol of the compound represented by the general formula (63). In the case where the base is used also as the solvent, it is not necessary to use another solvent.

Since this reaction is an equimolar reaction of the compounds, they are basically used in equimolar amounts, but either of them may be used in an excess amount. The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture may be directly used in the next step without isolation of the compound of interest.

### Production method 9 for starting compound

Among examples of the compound represented by the general formula (2c) as the starting compound for Production Method 4, the compound represented by the general formula (2c-2) wherein Q is the above-defined Q8 can be produced from the compound represented by the general formula (1d-1) wherein A is the above-defined SO₂R⁸ as an example of the compound represented by the general formula (1d) through the step [l] described below. The reaction conditions for the step [l] are the same as described above.

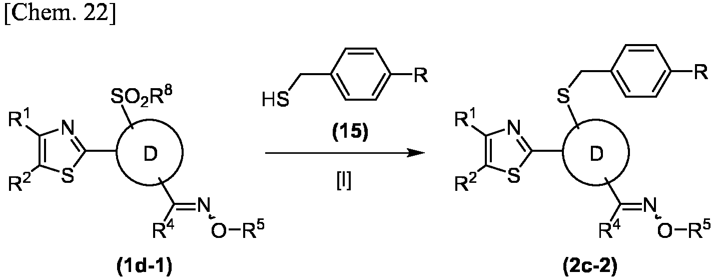

In the formula, D, R¹, R², R⁵, and R⁸ are the same as above; and R represents a (C₁-C₄) alkyl group such as a tert-butyl group or a hydrogen atom.

### Production method 10 for starting compound

Among examples of the compound represented by the general formula (2e) as the starting compound for Production Method 8, the compound represented by the general formula (2e-1) wherein A is the above-defined SO₂R⁸ can be produced from the compound represented by the general formula (69) through the steps [z], [aa], [aj], [ad], [p], and [a'] described below. The reaction conditions for the steps [z], [aa], [ad], [p], and [a'] are the same as described above.

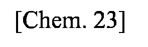

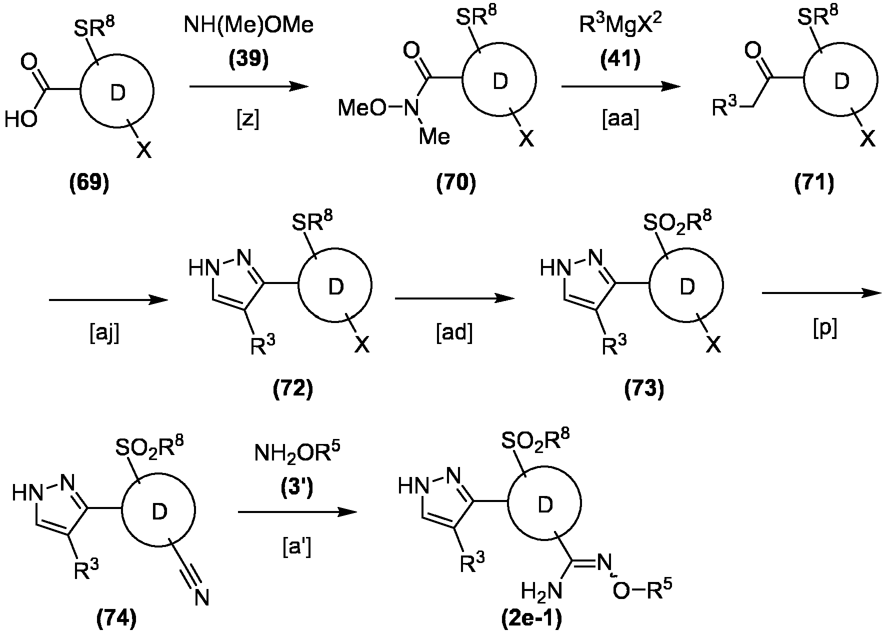

In the formula, D, R³, R⁵, and R⁸ are the same as above; X represents a leaving group such as chlorine, bromine, or iodine; and X² represents a halogen atom such as chlorine, bromine, or iodine.

### Production method at step [aj]

The compound represented by the general formula (72) can be produced by reacting the compound represented by the general formula (71) with a homologation agent and hydrazine in the presence of an inert solvent with or without a base.

Examples of the homologation agent used in this reaction include N,N-dimethylformamide dimethyl acetal, N,N-dimethylformamide di-tert-butyl acetal, tert-butoxybis(dimethylamino)methane, trimethyl orthoformate, methyl formate, and ethyl formate. The amount of the homologation agent used is usually in the range of a 1- to 20-fold molar amount relative to the compound represented by the general formula (71).

The hydrazine used in this reaction can be in the form of a salt such as hydrochloride or a hydrate. The amount of the hydrazine used is usually in the range of a 1- to 10-fold molar amount relative to the compound represented by the general formula (71).

Examples of the base that can be used in this reaction include inorganic bases including alkali metal hydroxides such as sodium hydroxide and potassium hydroxide; alkali metal hydrides such as sodium hydride and potassium hydride; alkali metal alkoxides such as sodium ethoxide and potassium tert-butoxide; and carbonates such as sodium carbonate, potassium carbonate, cesium carbonate and sodium hydrogen carbonate; and organic bases such as triethylamine, pyridine, N,N-dimethyl-4-aminopyridine and DBU. The amount of the base used is usually in the range of a 1- to 10-fold molar amount relative to the compound represented by the general formula (71).

The inert solvent used in this reaction may be any solvent that does not markedly inhibit the progress of the reaction, and examples include aromatic hydrocarbons such as benzene, toluene, and xylene; halogenated aliphatic hydrocarbons such as methylene chloride, chloroform, and carbon tetrachloride; halogenated aromatic hydrocarbons such as chlorobenzene and dichlorobenzene; chain or cyclic ethers such as diethyl ether, dioxane, and tetrahydrofuran; esters such as ethyl acetate; amides such as N,N-dimethylformamide and N,N-dimethylacetamide; and other inert solvents such as dimethyl sulfoxide, 1,3-dimethyl-2-imidazolidinone, acetone, methyl ethyl ketone, and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (71).

The reaction temperature in this reaction is usually in the range of 0°C to the boiling point of the solvent used. The reaction time varies with the reaction scale, the reaction temperature and the like and is not the same in every case, but is usually selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. Alternatively, the post-reaction mixture is directly used in the next reaction without purification.

### Production method 11 for starting compound

Among examples of the compound represented by the general formula (2c) as the starting compound for Production Method 4, the compound represented by the general formula (2c-3) wherein Q is the above-defined Q10 can be produced from the compound represented by the general formula (1e-1) wherein A is the above-defined SO₂R⁸ as an example of the compound represented by the general formula (1e) through the step [l] described below. The reaction conditions for the step [l] are the same as described above.

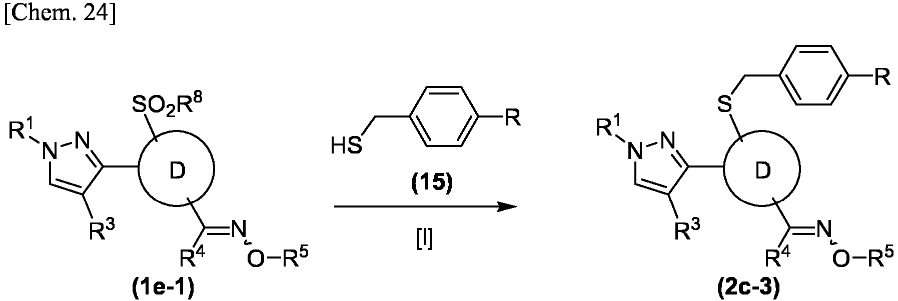

In the formula, D, R¹, R³, R⁴, R⁵, and R⁸ are the same as above; and R represents a (C₁-C₄) alkyl group such as a tert-butyl group or a hydrogen atom.

### Production method 12 for starting compound

Among examples of the compound represented by the general formula (2b) as the starting compound for Production Method 4, the compound represented by the general formula (2b-3) wherein A is the above-defined SO₂R⁸ can be produced from the compound represented by the general formula (54) through the steps [g], [a'], [m], [ae], and [at] described below. The reaction conditions for the steps [g], [a'], [m], [ae], and [af] are the same as described above.

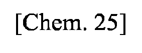

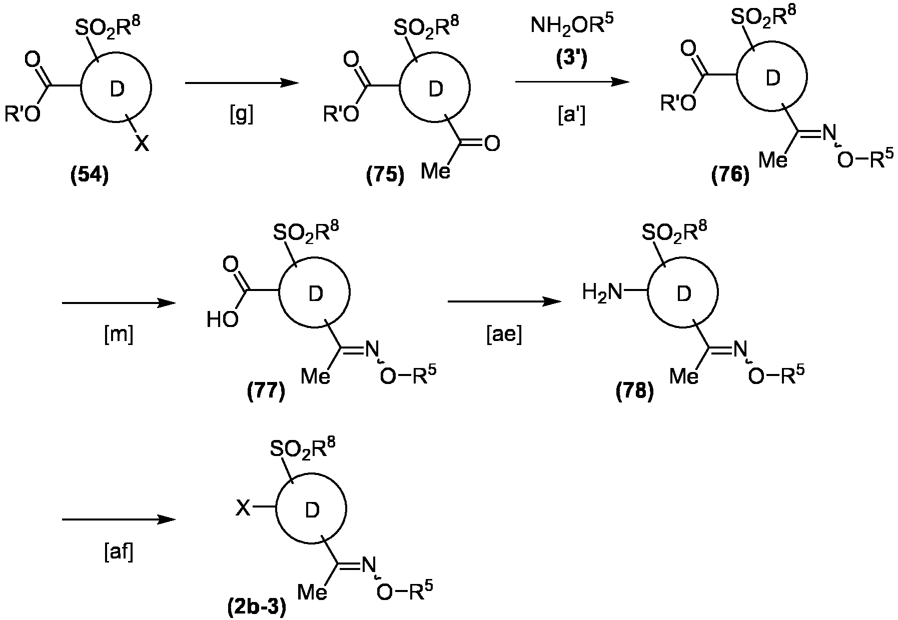

In the formula, D, R⁵, and R⁸ are the same as above; R' represents a (C₁-C₆) alkyl group such as a methyl group or an ethyl group; and X represents a leaving group such as chlorine, bromine, or iodine.

### Production method 13 for starting compound

Among examples of the compound represented by the general formula (f) as the starting compound for Production Method 10, the compound represented by the general formula (2f-1) wherein A is the above-defined SO₂R⁸ can be produced from the compound represented by the general formula (2b-2) through the steps [ak] and [al] described below.

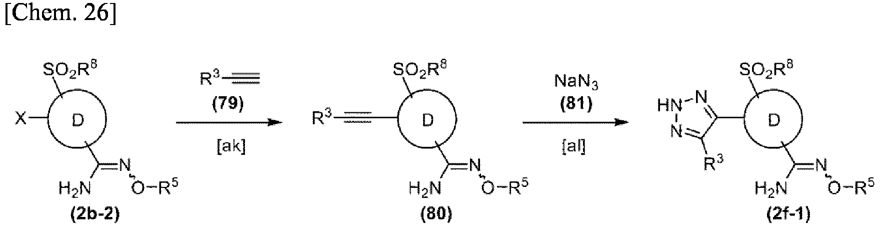

In the formula, D, R³, R⁵, and R⁸ are the same as above; and X represents a leaving group such as chlorine, bromine, or iodine.

### Production method at step [ak]

The compound represented by the general formula (80) can be produced by reacting the compound represented by the general formula (2b-2) with the compound represented by the general formula (79) in the presence of a palladium catalyst, copper iodide, a base and an inert solvent.

Examples of the palladium catalyst used in this reaction include palladium metal salts such as palladium chloride, palladium bromide, palladium iodide and palladium acetate; and palladium complex compounds such as π-allylpalladium chloride dimer, palladium acetylacetonate, dichlorobis(acetonitrile)palladium, dichlorobis(benzonitrile)palladium, bis(dibenzylideneacetone)palladium, tris(dibenzylideneacetone)dipalladium, tris(dibenzylideneacetone)dipalladium (chloroform adduct), dichlorodiamine palladium, dichlorobis(triphenylphosphine)palladium, dichlorobis(tricyclohexylphosphine)palladium, tetrakis(triphenylphosphine)palladium, dichloro[1,2-bis(diphenylphosphino)ethane]palladium, dichloro[1,3-bis(diphenylphosphino)propane]palladium, dichloro [1,4-bis(diphenylphosphino)butane]palladium, dichloro[1,1'-bis(diphenylphosphino)ferrocene]palladium and a [(diphenylphosphino)ferrocene]dichloropalladium-dichloromethane complex. The amount of the metal catalyst used is usually selected as appropriate from the range of a 0.001- to 0.5-fold molar amount relative to the compound represented by the general formula (2b-2).

These palladium catalysts may be used alone or in combination with a tertiary phosphine. Examples of the tertiary phosphine that can be used in combination with the palladium catalyst include triphenylphosphine, trimethylphosphine, triethylphosphine, tributylphosphine, tri(tert-butyl)phosphine, tricyclohexylphosphine, tri-o-tolylphosphine, trioctylphosphine, 9,9-dimethyl-4,5-bis(diphenylphosphino)xanthene, 2-(di-tert-butylphosphino)biphenyl, 2-(dicyclohexylphosphino)biphenyl, 1,2-bis(diphenylphosphino)ethane, 1,3-bis(diphenylphosphino)propane, 1,4-bis(diphenylphosphino)butane, 1,1'-bis(diphenylphosphino)ferrocene, (R)-(+)-2,2'-bis(diphenylphosphino)-1,1'-binaphthyl, (S)-(-)-2,2'-bis(diphenylphosphino)-1,1'-binaphthyl and (±)-2,2'-bis(diphenylphosphino)-1,1'-binaphthyl. The amount of the tertiary phosphine used is usually selected as appropriate from the range of a 0.5- to 10-fold molar amount relative to the palladium catalyst.

The amount of the copper iodide used in this reaction is usually in the range of a 0.01- to 0.5-fold molar amount relative to the compound represented by the general formula (2b-2).

Examples of the base that can be used in this reaction include inorganic bases including carbonates such as sodium carbonate, potassium carbonate, cesium carbonate and sodium hydrogen carbonate; and organic bases such as triethylamine, pyridine, N,N-dimethyl-4-aminopyridine and DBU. The amount of the base used is usually selected as appropriate from the range of a 1- to 10-fold molar amount relative to the compound represented by the general formula (2b-2). In some cases, the base can be used as the solvent as well.

The inert solvent used in this reaction may be any solvent that does not markedly inhibit the reaction, and examples include alcohols such as methanol, ethanol, propanol, butanol, and 2-propanol; chain or cyclic ethers such as diethyl ether, tetrahydrofuran, dioxane, and 1,2-dimethoxyethane (DME); aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; nitriles such as acetonitrile; esters such as ethyl acetate; polar solvents such as N,N-dimethylformamide, N,N-dimethylacetamide, dimethyl sulfoxide, and 1,3-dimethyl-2-imidazolidinone; and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (2b-2). In the case where the base is used also as the solvent, it is not necessary to use another solvent.

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like, but is basically selected as appropriate from the range of a few minutes to 48 hours. This reaction may be conducted under the atmosphere of an inert gas such as nitrogen gas and argon gas. After the reaction is completed, the compound of interest is isolated from the post-reaction mixture by the usual method. As needed, recrystallization, column chromatography, etc. can be employed for the purification of the compound of interest. The post-reaction mixture may be directly used in the next reaction without isolation of the compound of interest.

### Production method at step [al]

The compound represented by the general formula (2f-1) can be produced by reacting the compound represented by the general formula (80) with sodium azide (81) in the presence of diacetoxy iodobenzene and an inert solvent.

The amount of the diacetoxy iodobenzene used in this reaction is usually in the range of an about 1- to 10-fold molar amount relative to the compound represented by the general formula (80).

The inert solvent used in this reaction may be any solvent that does not markedly inhibit the reaction, and examples include alcohols such as methanol, ethanol, propanol, butanol, and 2-propanol; chain or cyclic ethers such as diethyl ether, tetrahydrofuran, dioxane, and 1,2-dimethoxyethane (DME); aromatic hydrocarbons such as benzene, toluene, and xylene; halogenated hydrocarbons such as methylene chloride, chloroform, and carbon tetrachloride; halogenated aromatic hydrocarbons such as chlorobenzene and dichlorobenzene; nitriles such as acetonitrile; esters such as ethyl acetate; polar solvents such as N,N-dimethylformamide, N,N-dimethylacetamide, dimethyl sulfoxide, and 1,3-dimethyl-2-imidazolidinone; and water. One of these inert solvents may be used alone, and also two or more of them may be used as a mixture. The amount of the inert solvent used is not particularly limited as long as it is sufficient to dissolve the reaction reagents, and is usually selected as appropriate from the range of 0.5 L to 100 L relative to 1 mole of the compound represented by the general formula (80). In the case where the base is used also as the solvent, it is not necessary to use another solvent.

Since this reaction is an equimolar reaction of the compounds, they are basically used in equimolar amounts, but either of them may be used in an excess amount. The reaction temperature in this reaction is usually in the range of room temperature to the boiling point of the solvent used. The reaction time varies with the reaction scale, the reaction temperature and the like, but is basically selected as appropriate from the range of a few minutes to 48 hours. After the reaction is completed, the compound of interest is isolated from the post-reaction mixture by the usual method. As needed, recrystallization, column chromatography, etc. can be employed for the purification of the compound of interest. The post-reaction mixture may be directly used in the next reaction without isolation of the compound of interest.

### Production method 14 for starting compound

The compound Q-H represented by the general formula (6) in Production Method 3 can be produced by any of the methods described below or their modified methods. The production method for Q1-H is, for example, described in Organic Letters, 2015, 17, 2894-2897. The production method for Q2-H is, for example, described in Bioorganic & Medicinal Chemistry Letters, 2012, 22, 7036-7040. The production method for Q3-H is, for example, described in Heterocycles, 1983, 20, 1243-1246. The production method for Q4-H is, for example, described in Organic Process Research & Development, 2004, 8, 28-32. The production method for Q5-H is, for example, described in Bioorganic & Medicinal Chemistry Letters, 2021, 41, 127984. The production method for Q6-H is, for example, described in U.S. Patent No. 5449784. The production method for Q7-H is, for example, described in Journal of the American Chemical Society, 2015, 137, 2738-2747. The production method for Q8-H is, for example, described in U.S. Patent No. 4282364. The production method for Q9-H is, for example, described in Organic Letters, 2007, 9, 4009-4012. The production method for Q10-H is, for example, described in Chem. Ber., 1960, 93, 1208-11. The production method for Q11-H is, for example, described in Organic Letters, 2020, 22, 1396-1401. The production method for Q12-H is, for example, described in Synlett, (1999), (10), 1642-1644. The production method for Q13-H is, for example, described in Journal of the American Chemical Society, 1937, 59, 2262-2264. The production method for Q14-H is, for example, described in Tetrahedron Letters, 1994, 35, 1585-1586. The production method for Q15-H is, for example, described in Journal of Chemical and Pharmaceutical Research, 2012, 4, 1772-1781. The production method for Q16-H is, for example, described in Journal of Medicinal Chemistry, 2016, 59, 8398-8411. The production method for Q17-H is, for example, described in Chemistry-A European Journal, 2016, 22(3), 911-915. The production method for Q18-H is, for example, described in Journal of Organic Chemistry, 1990, 55(3), 929-35.

Representative examples of the compound represented by the general formula (1) of the present invention are shown in Tables 1 to 21, but the present invention is not limited thereto. In Tables 1 to 21, some descriptions for R¹ may be descriptions for both R¹ and a substituent from the set Y on R¹.

Specific examples of the compound of the present invention are shown below. In the tables below, Me stands for a methyl group, Et stands for an ethyl group, i-Pr stands for an isopropyl group, n-Pr stands for a n-propyl group, c-Pr stands for a cyclopropyl group, i-Bu stands for an isobutyl group, n-Br stands for a n-butyl group, c-Bu stands for a cyclobutyl group, t-Bu stands for a tert-butyl group, c-Pen stands for a cyclopentyl group, i-pen stands for an isopentyl group, c-Hex stands for a cyclohexyl group, Ac stands for an acetyl group, Ph stands for a phenyl group, Bn stands for a benzyl group, Bz stands for a benzoyl group, and i-allyl stands for an isoallyl group. Shown in the column of "Physical property value" is a melting point (°C), a refractive index (n_{D}), or "¹H-NMR". The number in the parentheses for the refractive index represents measurement temperature (°C). ¹H-NMR data are shown in Table 22.

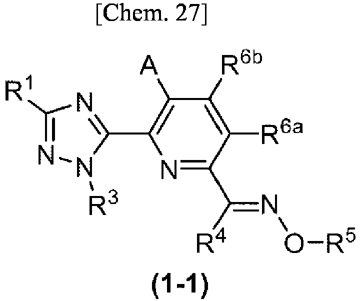

**Table 1 (In Table 1, R^{6a} and R^{6b} represent hydrogen atoms.)**

| [Table 1-1] | | | | | | |
|---|---|---|---|---|---|---|
| Compound No. | R¹ | R³ | R⁴ | R⁵ | A | Physical Property value |
| 1-1 | 2-Cl-Ph | Me | NH₂ | Et | SO₂NHMe | NMR |
| 1-2 | Ph | Me | NHAc | Et | CO₂Et | NMR |
| 1-3 | Ph | Me | NHCOCF₃ | Et | CO₂Et | 176-177 |
| 1-4 | Ph | Me | NH₂ | Et | SO₂NHMe | NMR |
| 1-5 | 2-Cl-Ph | Me | NH₂ | Et | SO₂Me | 197-198 |
| 1-6 | Ph | Me | Me | Et | SO₂Me | 202-203 |
| 1-7 | Ph | Me | NH₂ | Et | SO₂Me | 208-209 |
| 1-8 | Ph | Me | NH₂ | Et | CO₂Et | NMR |
| 1-9 | 2,6-F₂-Ph | Me | NH₂ | Et | SO₂NHMe | 161-162 |
| 1-10 | 3-(pyridin-3-yl)-Ph | Me | NH₂ | Me | SO₂NHMe | 174-175 |
| 1-11 | 3-F-Ph | Me | NH₂ | Me | SO₂NMe₂ | 113-114 |
| 1-12 | 2-Cl-Ph | Me | NAc₂ | Et | SO₂N(Ac)Me | NMR |
| 1-13 | Ph | Me | NH₂ | Me | SO₂NMe₂ | 160-161 |
| 1-14 | 3,5-F₂-Ph | Me | NH₂ | Me | SO₂NHMe | 199-200 |
| 1-15 | 3,5-F₂-Ph | Me | NH₂ | Et | SO₂NHMe | 155-156 |
| 1-16 | 3-CHF₂-Ph | Me | NH₂ | Me | SO₂NHMe | 193-194 |
| 1-17 | 3-CHF₂-Ph | Me | NH₂ | Et | SO₂NHMe | 158-159 |
| 1-18 | Ph | Me | NH₂ | i-Pr | SO₂NMe₂ | NMR |
| 1-19 | 2-Cl-Ph | Me | NH₂ | Me | SO₂NMe₂ | 122-123 |
| 1-20 | 2-Cl-Ph | Me | NH₂ | i-Pr | SO₂NMe₂ | NMR |
| 1-21 | 2-OMe-Ph | Me | NH₂ | Me | SO₂NHMe | NMR |
| 1-22 | 2-OMe-Ph | Me | NH₂ | Et | SO₂NHMe | NMR |
| 1-23 | 2,3-Cl₂-Ph | Me | NH₂ | Me | SO₂NHMe | 225-226 |
| 1-24 | 2,3-Cl₂-Ph | Me | NH₂ | Et | SO₂NHMe | 181-182 |
| 1-25 | 2,3-Cl₂-Ph | Me | NH₂ | i-Pr | SO₂NHMe | 82-83 |
| 1-26 | 3-Cl-Ph | Me | NH₂ | Et | SO₂NHMe | 123-124 |
| 1-27 | 3-Cl-Ph | Me | NH₂ | Me | SO₂NHMe | 151-152 |
| 1-28 | Ph | Me | NH₂ | i-Pr | SO₂NHMe | NMR |
| 1-29 | 2-Cl-Ph | Me | NH₂ | Me | SO₂NHMe | 178-179 |
| 1-30 | 2-Cl-Ph | Me | NH₂ | i-Pr | SO₂NHMe | 113-114 |

**[Table 1-2]**

| Compound No. | R¹ | R³ | R⁴ | R⁵ | A | Physical Property value |
|---|---|---|---|---|---|---|
| 1-31 | 2-Cl-Ph | Me | NH₂ | Et | SO₂NMe₂ | 140-141 |
| 1-32 | Ph | Me | NH₂ | Et | SO₂NMe₂ | NMR |
| 1-33 | 2,4-F₂-Ph | Me | NH₂ | Me | SO₂NHMe | 196-197 |
| 1-34 | 2,4-F₂-Ph | Me | NH₂ | Et | SO₂NHMe | 156-157 |
| 1-35 | 2,5-F₂-Ph | Me | NH₂ | Me | SO₂NHMe | 190-191 |
| 1-36 | 2,5-F₂-Ph | Me | NH₂ | Et | SO₂NHMe | 155-156 |
| 1-37 | 2-Cl-6-F-Ph | Me | NH₂ | Me | SO₂NHMe | 154-155 |
| 1-38 | 2-Cl-6-F-Ph | Me | NH₂ | Et | SO₂NHMe | NMR |
| 1-39 | 2-F-Ph | Me | NH₂ | Et | SO₂NHMe | NMR |
| 1-40 | 4-Cl-Ph | Me | NH₂ | Et | SO₂NHMe | NMR |
| 1-41 | 3-F-Ph | Me | NH₂ | i-Pr | SO₂NMe₂ | NMR |
| 1-42 | 2,6-F₂-Ph | Me | NH₂ | Et | SO₂NMe₂ | NMR |
| 1-43 | 3-Br-Ph | Me | NH₂ | Me | SO₂NHMe | 161-162 |
| 1-44 | 3-Br-Ph | Me | NH₂ | Et | SO₂NHMe | 171-172 |
| 1-45 | 3-F-Ph | Me | NH₂ | Et | SO₂NHMe | 138-139 |
| 1-46 | 3-F-Ph | Me | NH₂ | Me | SO₂NHMe | 153-154 |
| 1-47 | 3-F-Ph | Me | NH₂ | i-Pr | SO₂NHMe | 143-144 |
| 1-48 | Ph | Me | NH₂ | Me | SO₂NHMe | 223-224 |
| 1-49 | 2,6-F₂-Ph | Me | NH₂ | Me | SO₂NHMe | 199-200 |
| 1-50 | 3-Me-Ph | Me | NH₂ | Me | SO₂NHMe | 184-185 |
| 1-51 | 3-Me-Ph | Me | NH₂ | Et | SO₂NHMe | 126-127 |
| 1-52 | 3-Me-Ph | Me | NH₂ | C(Me)₂OMe | SO₂NHMe | 180-181 |
| 1-53 | 2-Me-Ph | Me | NH₂ | Et | SO₂NHMe | NMR |
| 1-54 | 2,6-F₂-Ph | Me | NH₂ | i-Pr | SO₂NHMe | |
| 1-55 | 2,5-F₂-Ph | Me | NH₂ | i-Pr | SO₂NHMe | |
| 1-56 | 2,4-F₂-Ph | Me | NH₂ | i-Pr | SO₂NHMe | |
| 1-57 | 4-F-Ph | Me | NH₂ | Me | SO₂NHMe | |
| 1-58 | 4-F-Ph | Me | NH₂ | Et | SO₂NHMe | |
| 1-59 | 4-F-Ph | Me | NH₂ | i-Pr | SO₂NHMe | |
| 1-60 | 2,3-F₂-Ph | Me | NH₂ | Me | SO₂NHMe | |

**[Table 1-3]**

| Compound No. | R¹ | R³ | R⁴ | R⁵ | A | Physical Property value |
|---|---|---|---|---|---|---|
| 1-61 | 2,3-F₂-Ph | Me | NH₂ | Et | SO₂NHMe | |
| 1-62 | 2,3-F₂-Ph | Me | NH₂ | i-Pr | SO₂NHMe | |
| 1-63 | 2,4,6-F₃-Ph | Me | NH₂ | Me | SO₂NHMe | |
| 1-64 | 2,4,6-F₃-Ph | Me | NH₂ | Et | SO₂NHMe | |
| 1-65 | 2,4,6-F₃-Ph | Me | NH₂ | i-Pr | SO₂NHMe | |
| 1-66 | 2-SMe-Ph | Me | NH₂ | Me | SO₂NHMe | |
| 1-67 | 2-SMe-Ph | Me | NH₂ | Et | SO₂NHMe | |
| 1-68 | 2-SMe-Ph | Me | NH₂ | i-Pr | SO₂NHMe | |
| 1-69 | 2-SOMe-Ph | Me | NH₂ | Me | SO₂NHMe | |
| 1-70 | 2-SOMe-Ph | Me | NH₂ | Et | SO₂NHMe | |
| 1-71 | 2-SOMe-Ph | Me | NH₂ | i-Pr | SO₂NHMe | |
| 1-72 | 2-SO₂Me-Ph | Me | NH₂ | Me | SO₂NHMe | |
| 1-73 | 2-SO₂Me-Ph | Me | NH₂ | Et | SO₂NHMe | |
| 1-74 | 2-SO₂Me-Ph | Me | NH₂ | i-Pr | SO₂NHMe | |
| 1-75 | 2-CN-Ph | Me | NH₂ | Me | SO₂NHMe | |
| 1-76 | 2-CN-Ph | Me | NH₂ | Et | SO₂NHMe | |
| 1-77 | 2-CN-Ph | Me | NH₂ | i-Pr | SO₂NHMe | |
| 1-78 | 2-OCHF₂-Ph | Me | NH₂ | Me | SO₂NHMe | |
| 1-79 | 2-OCHF₂-Ph | Me | NH₂ | Et | SO₂NHMe | |
| 1-80 | 2-OCHF₂-Ph | Me | NH₂ | i-Pr | SO₂NHMe | |
| 1-81 | 2-c-Pr-Ph | Me | NH₂ | Me | SO₂NHMe | |
| 1-82 | 2-c-Pr-Ph | Me | NH₂ | Et | SO₂NHMe | |
| 1-83 | 2-c-Pr-Ph | Me | NH₂ | i-Pr | SO₂NHMe | |
| 1-84 | 2-CHF₂-Ph | Me | NH₂ | Me | SO₂NHMe | |
| 1-85 | 2-CHF₂-Ph | Me | NH₂ | Et | SO₂NHMe | |
| 1-86 | 2-CHF₂-Ph | Me | NH₂ | i-Pr | SO₂NHMe | |
| 1-87 | 2-NHAc-Ph | Me | NH₂ | Me | SO₂NHMe | |
| 1-88 | 2-NHAc-Ph | Me | NH₂ | Et | SO₂NHMe | |
| 1-89 | 2-NHAc-Ph | Me | NH₂ | i-Pr | SO₂NHMe | |
| 1-90 | 2-P(OMe)₂-Ph | Me | NH₂ | Me | SO₂NHMe | |

**[Table 1-4]**

| Compound No. | R¹ | R³ | R⁴ | R⁵ | A | Physical Property value |
|---|---|---|---|---|---|---|
| 1-91 | 2-P(OMe)₂-Ph | Me | NH₂ | Et | SO₂NHMe | |
| 1-92 | 2-P(OMe)₂-Ph | Me | NH₂ | i-Pr | SO₂NHMe | |
| 1-93 | Ph | Me | NH₂ | Me | SO₂N(Ac)Me | |
| 1-94 | Ph | Me | NH₂ | Et | SO₂N(Ac)Me | |
| 1-95 | Ph | Me | NH₂ | i-Pr | SO₂N(Ac)Me | |
| 1-96 | Ph | Me | NH₂ | Me | SO₂N(CO₂Me)Me | |
| 1-97 | Ph | Me | NH₂ | Et | SO₂N(CO₂Me)Me | |
| 1-98 | Ph | Me | NH₂ | i-Pr | SO₂N(CO₂Me)Me | |
| 1-99 | 2-F-Ph | Me | NH₂ | Me | SO₂N(Ac)Me | |
| 1-100 | 2-F-Ph | Me | NH₂ | Et | SO₂N(Ac)Me | |
| 1-101 | 2-F-Ph | Me | NH₂ | i-Pr | SO₂N(Ac)Me | |
| 1-102 | 2-F-Ph | Me | NH₂ | Me | SO₂N(CO₂Me)Me | |
| 1-103 | 2-F-Ph | Me | NH₂ | Et | SO₂N(CO₂Me)Me | |
| 1-104 | 2-F-Ph | Me | NH₂ | i-Pr | SO₂N(CO₂Me)Me | |
| 1-105 | 3-F-Ph | Me | NH₂ | Me | SO₂N(Ac)Me | |
| 1-106 | 3-F-Ph | Me | NH₂ | Et | SO₂N(Ac)Me | |
| 1-107 | 3-F-Ph | Me | NH₂ | i-Pr | SO₂N(Ac)Me | |
| 1-108 | 3-F-Ph | Me | NH₂ | Me | SO₂N(CO₂Me)Me | |
| 1-109 | 3-F-Ph | Me | NH₂ | Et | SO₂N(CO₂Me)Me | |
| 1-110 | 3-F-Ph | Me | NH₂ | i-Pr | SO₂N(CO₂Me)Me | |
| 1-111 | 4-F-Ph | Me | NH₂ | Me | SO₂N(Ac)Me | |
| 1-112 | 4-F-Ph | Me | NH₂ | Et | SO₂N(Ac)Me | |
| 1-113 | 4-F-Ph | Me | NH₂ | i-Pr | SO₂N(Ac)Me | |
| 1-114 | 4-F-Ph | Me | NH₂ | Me | SO₂N(CO₂Me)Me | |
| 1-115 | 4-F-Ph | Me | NH₂ | Et | SO₂N(CO₂Me)Me | |
| 1-116 | 4-F-Ph | Me | NH₂ | i-Pr | SO₂N(CO₂Me)Me | |
| 1-117 | 2,6-F₂-Ph | Me | NH₂ | Me | SO₂N(Ac)Me | |
| 1-118 | 2,6-F₂-Ph | Me | NH₂ | Et | SO₂N(Ac)Me | |
| 1-119 | 2,6-F₂-Ph | Me | NH₂ | i-Pr | SO₂N(Ac)Me | |
| 1-120 | 2,6-F₂-Ph | Me | NH₂ | Me | SO₂N(CO₂Me)Me | |

**[Table 1-5]**

| Compound No. | R¹ | R³ | R⁴ | R⁵ | A | Physical Property value |
|---|---|---|---|---|---|---|
| 1-121 | 2,6-F₂-Ph | Me | NH₂ | Et | SO₂N(CO₂Me)Me | |
| 1-122 | 2,6-F₂-Ph | Me | NH₂ | i-Pr | SO₂N(CO₂Me)Me | |
| 1-123 | 2,5-F₂-Ph | Me | NH₂ | Me | SO₂N(Ac)Me | |
| 1-124 | 2,5-F₂-Ph | Me | NH₂ | Et | SO₂N(Ac)Me | |
| 1-125 | 2,5-F₂-Ph | Me | NH₂ | i-Pr | SO₂N(Ac)Me | |
| 1-126 | 2,5-F₂-Ph | Me | NH₂ | Me | SO₂N(CO₂Me)Me | |
| 1-127 | 2,5-F₂-Ph | Me | NH₂ | Et | SO₂N(CO₂Me)Me | |
| 1-128 | 2,5-F₂-Ph | Me | NH₂ | i-Pr | SO₂N(CO₂Me)Me | |
| 1-129 | 2,4-F₂-Ph | Me | NH₂ | Me | SO₂N(Ac)Me | |
| 1-130 | 2,4-F₂-Ph | Me | NH₂ | Et | SO₂N(Ac)Me | |
| 1-131 | 2,4-F₂-Ph | Me | NH₂ | i-Pr | SO₂N(Ac)Me | |
| 1-132 | 2,4-F₂-Ph | Me | NH₂ | Me | SO₂N(CO₂Me)Me | |
| 1-133 | 2,4-F₂-Ph | Me | NH₂ | Et | SO₂N(CO₂Me)Me | |
| 1-134 | 2,4-F₂-Ph | Me | NH₂ | i-Pr | SO₂N(CO₂Me)Me | |
| 1-135 | 2,3-F₂-Ph | Me | NH₂ | Me | SO₂N(Ac)Me | |
| 1-136 | 2,3-F₂-Ph | Me | NH₂ | Et | SO₂N(Ac)Me | |
| 1-137 | 2,3-F₂-Ph | Me | NH₂ | i-Pr | SO₂N(Ac)Me | |
| 1-138 | 2,3-F₂-Ph | Me | NH₂ | Me | SO₂N(CO₂Me)Me | |
| 1-139 | 2,3-F₂-Ph | Me | NH₂ | Et | SO₂N(CO₂Me)Me | |
| 1-140 | 2,3-F₂-Ph | Me | NH₂ | i-Pr | SO₂N(CO₂Me)Me | |
| 1-141 | 3-Ph-Ph | Me | NH₂ | Me | SO₂NHMe | 96-97 |
| 1-142 | pyridin-2-yl | Me | NH₂ | Et | SO₂NHMe | 199-200 |
| 1-143 | pyrazin-2-yl | Me | NH₂ | i-Pr | SO₂NHMe | 211-212 |
| 1-144 | c-Hex | Me | NH₂ | Me | SO₂NHMe | 162-163 |
| 1-145 | c-Hex | Me | NH₂ | Et | SO₂NHMe | 177-178 |
| 1-146 | c-Hex | Me | NH₂ | i-Pr | SO₂NHMe | |
| 1-147 | c-Pen | Me | NH₂ | Me | SO₂NHMe | |
| 1-148 | c-Pen | Me | NH₂ | Et | SO₂NHMe | |
| 1-149 | c-Pen | Me | NH₂ | i-Pr | SO₂NHMe | |
| 1-150 | c-Bu | Me | NH₂ | Me | SO₂NHMe | |

**[Table 1-6]**

| Compound No. | R¹ | R³ | R⁴ | R⁵ | A | Physical Property value |
|---|---|---|---|---|---|---|
| 1-151 | c-Bu | Me | NH₂ | Et | SO₂NHMe | |
| 1-152 | c-Bu | Me | NH₂ | i-Pr | SO₂NHMe | |
| 1-153 | c-Pr | Me | NH₂ | Me | SO₂NHMe | 156-157 |
| 1-154 | c-Pr | Me | NH₂ | Et | SO₂NHMe | 164-165 |
| 1-155 | c-Pr | Me | NH₂ | i-Pr | SO₂NHMe | |
| 1-156 | 3,4-dihydro-2H-pyran-6-yl | Me | NH₂ | Me | SO₂NHMe | |
| 1-157 | 3,4-dihydro-2H-pyran-6-yl | Me | NH₂ | Et | SO₂NHMe | |
| 1-158 | 3,4-dihydro-2H-pyran-6-yl | Me | NH₂ | i-Pr | SO₂NHMe | |
| 1-159 | tetrahydropyran-2-yl | Me | NH₂ | Me | SO₂NHMe | |
| 1-160 | tetrahydropyran-2-yl | Me | NH₂ | Et | SO₂NHMe | |
| 1-161 | tetrahydropyran-2-yl | Me | NH₂ | i-Pr | SO₂NHMe | |
| 1-162 | 1,3-dioxan-2-yl | Me | NH₂ | Me | SO₂NHMe | |
| 1-163 | 1,3-dioxan-2-yl | Me | NH₂ | Et | SO₂NHMe | |
| 1-164 | 1,3-dioxan-2-yl | Me | NH₂ | i-Pr | SO₂NHMe | |
| 1-165 | tetrahydropyran-3-yl | Me | NH₂ | Me | SO₂NHMe | |
| 1-166 | tetrahydropyran-3-yl | Me | NH₂ | Et | SO₂NHMe | |
| 1-167 | tetrahydropyran-3-yl | Me | NH₂ | i-Pr | SO₂NHMe | |
| 1-168 | tetrahydropyran-4-yl | Me | NH₂ | Me | SO₂NHMe | |
| 1-169 | tetrahydropyran-4-yl | Me | NH₂ | Et | SO₂NHMe | |
| 1-170 | tetrahydropyran-4-yl | Me | NH₂ | i-Pr | SO₂NHMe | |
| 1-171 | tetrahydrothiopyran-4-yl | Me | NH₂ | Me | SO₂NHMe | |
| 1-172 | tetrahydrothiopyran-4-yl | Me | NH₂ | Et | SO₂NHMe | |
| 1-173 | tetrahydrothiopyran-4-yl | Me | NH₂ | i-Pr | SO₂NHMe | |
| 1-174 | 1-Ac-piperidin-4-yl | Me | NH₂ | Me | SO₂NHMe | |
| 1-175 | 1-Ac-piperidin-4-yl | Me | NH₂ | Et | SO₂NHMe | |
| 1-176 | 1-Ac-piperidin-4-yl | Me | NH₂ | i-Pr | SO₂NHMe | |
| 1-177 | piperidin-1-yl | Me | NH₂ | Me | SO₂NHMe | |
| 1-178 | piperidin-1-yl | Me | NH₂ | Et | SO₂NHMe | |
| 1-179 | piperidin-1-yl | Me | NH₂ | i-Pr | SO₂NHMe | |
| 1-180 | 1,3-dioxolan-2-yl | Me | NH₂ | Me | SO₂NHMe | |

**[Table 1-7]**

| Compound No. | R¹ | R³ | R⁴ | R⁵ | A | Physical Property value |
|---|---|---|---|---|---|---|
| 1-181 | 1,3-dioxolan-2-yl | Me | NH₂ | Et | SO₂NHMe | |
| 1-182 | 1,3-dioxolan-2-yl | Me | NH₂ | i-Pr | SO₂NHMe | |
| 1-183 | 1,3-dioxepan-2-yl | Me | NH₂ | Me | SO₂NHMe | |
| 1-184 | 1,3-dioxepan-2-yl | Me | NH₂ | Et | SO₂NHMe | |
| 1-185 | 1,3-dioxepan-2-yl | Me | NH₂ | i-Pr | SO₂NHMe | |
| 1-186 | pyrimidin-2-yl | Me | NH₂ | Et | SO₂NHMe | 241-242 |
| 1-187 | pyrimidin-2-yl | Me | NH₂ | i-Pr | SO₂NHMe | |
| 1-188 | thiazol-5-yl | Me | NH₂ | Me | SO₂NHMe | NMR |
| 1-189 | thiazol-5-yl | Me | NH₂ | Et | SO₂NHMe | NMR |
| 1-190 | thiazol-5-yl | Me | NH₂ | i-Pr | SO₂NHMe | |
| 1-191 | thiazol-4-yl | Me | NH₂ | Me | SO₂NHMe | |
| 1-192 | thiazol-4-yl | Me | NH₂ | Et | SO₂NHMe | 86-89 |
| 1-193 | thiazol-4-yl | Me | NH₂ | i-Pr | SO₂NHMe | |
| 1-194 | 1,2,3-thiadiazol-5-yl | Me | NH₂ | Me | SO₂NHMe | |
| 1-195 | 1,2,3-thiadiazol-5-yl | Me | NH₂ | Et | SO₂NHMe | |
| 1-196 | 1,2,3-thiadiazol-5-yl | Me | NH₂ | i-Pr | SO₂NHMe | |
| 1-197 | thiophen-2-yl | Me | NH₂ | Me | SO₂NHMe | 181-182 |
| 1-198 | thiophen-2-yl | Me | NH₂ | Et | SO₂NHMe | 129-130 |
| 1-199 | thiophen-2-yl | Me | NH₂ | i-Pr | SO₂NHMe | |
| 1-200 | thiophen-3-yl | Me | NH₂ | Me | SO₂NHMe | NMR |
| 1-201 | thiophen-3-yl | Me | NH₂ | Et | SO₂NHMe | NMR |
| 1-202 | thiophen-3-yl | Me | NH₂ | i-Pr | SO₂NHMe | |
| 1-203 | pyridazin-3-yl | Me | NH₂ | Me | SO₂NHMe | |
| 1-204 | pyridazin-3-yl | Me | NH₂ | Et | SO₂NHMe | |
| 1-205 | pyridazin-3-yl | Me | NH₂ | i-Pr | SO₂NHMe | |

**[Table 1-8]**

| Compound No. | R¹ | R³ | R⁴ | R⁵ | A | Physical Property value |
|---|---|---|---|---|---|---|
| 1-206 | thiazol-2-yl | Me | NH₂ | Me | SO₂NHMe | NMR |
| 1-207 | thiazol-2-yl | Me | NH₂ | Et | SO₂NHMe | NMR |
| 1-208 | thiazol-2-yl | Me | NH₂ | i-Pr | SO₂NHMe | |
| 1-209 | pyridin-4-yl | Me | NH₂ | Et | SO₂NHMe | 168-169 |
| 1-210 | 2,6-F₂-Ph | Me | NH₂ | Et | SO₂N(Boc)Me | 187-188 |
| 1-211 | 3-CF₃-pyridin-2-yl | Me | NH₂ | Me | SO₂NHMe | NMR |
| 1-212 | 3-CF₃-pyridin-2-yl | Me | NH₂ | Et | SO₂NHMe | NMR |
| 1-213 | CH₂SMe | Me | NH₂ | Me | SO₂NHMe | NMR |
| 1-214 | CH₂SMe | Me | NH₂ | Et | SO₂NHMe | NMR |
| 1-215 | CH₂SO₂Me | Me | NH₂ | Me | SO₂NHMe | NMR |
| 1-216 | CH₂SOMe | Me | NH₂ | Et | SO₂NHMe | NMR |
| 1-217 | CH₂SO₂Me | Me | NH₂ | Et | SO₂NHMe | NMR |
| 1-218 | i-pen | Me | NH₂ | Me | SO₂NHMe | NMR |
| 1-219 | i-Pr | Me | NH₂ | Me | SO₂NHMe | NMR |
| 1-220 | i-Pr | Me | NH₂ | Et | SO₂NHMe | NMR |
| 1-221 | CH₂CH₂OMe | Me | NH₂ | Me | SO₂NHMe | NMR |
| 1-222 | CH₂CH₂OMe | Me | NH₂ | Et | SO₂NHMe | NMR |
| 1-223 | CH₂OMe | Me | NH₂ | Et | SO₂NHMe | NMR |
| 1-224 | CH₂OMe | Me | NH₂ | iPr | SO₂NHMe | NMR |
| 1-225 | furan-2-yl | Me | NH₂ | Me | SO₂NHMe | 216-217 |
| 1-226 | furan-2-yl | Me | NH₂ | Et | SO₂NHMe | 153-154 |
| 1-227 | 5-Me-pyridin-2-yl | Me | NH₂ | Et | SO₂NHMe | NMR |
| 1-228 | 5-Me-pyridin-2-yl | Me | NH₂ | Me | SO₂NHMe | NMR |
| 1-229 | pyridin-2-yl | Me | NH₂ | Me | SO₂NHMe | NMR |
| 1-230 | quinolin-2-yl | Me | NH₂ | Et | SO₂NHMe | 83-88 |
| 1-231 | 2,6-F₂-Ph | Me | Me | Et | SO₂NHMe | 164-165 |
| 1-232 | 2,6-F₂-Ph | Me | Me | Me | SO₂NHMe | 189-190 |
| 1-233 | 5-Cl-thiophen-2-yl | Me | NH₂ | Me | SO₂NHMe | 187-188 |
| 1-234 | 5-Cl-thiophen-2-yl | Me | NH₂ | Et | SO₂NHMe | NMR |
| 1-235 | 5-Cl-pyridin-2-yl | Me | NH₂ | Et | SO₂NHMe | 226-230 |

**[Table 1-9]**

| Compound No. | R¹ | R³ | R⁴ | R⁵ | A | Physical Property value |
|---|---|---|---|---|---|---|
| 1-236 | n-Pr | Me | NH₂ | Me | SO₂NHMe | NMR |
| 1-237 | n-Pr | Me | NH₂ | Et | SO₂NHMe | NMR |
| 1-238 | n-Pr | Me | NH₂ | i-Pr | SO₂NHMe | |
| 1-239 | thiazol-5-yl | Et | NH₂ | Me | SO₂NHMe | |
| 1-240 | thiazol-5-yl | Et | NH₂ | Et | SO₂NHMe | |
| 1-241 | thiazol-5-yl | Et | NH₂ | i-Pr | SO₂NHMe | |
| 1-242 | thiazol-4-yl | Et | NH₂ | Me | SO₂NHMe | |
| 1-243 | thiazol-4-yl | Et | NH₂ | Et | SO₂NHMe | |
| 1-244 | thiazol-4-yl | Et | NH₂ | i-Pr | SO₂NHMe | |
| 1-245 | thiazol-2-yl | Et | NH₂ | Me | SO₂NHMe | |
| 1-246 | thiazol-2-yl | Et | NH₂ | Et | SO₂NHMe | |
| 1-247 | thiazol-2-yl | Et | NH₂ | i-Pr | SO₂NHMe | |
| 1-248 | thiophen-2-yl | Et | NH₂ | Me | SO₂NHMe | NMR |
| 1-249 | thiophen-2-yl | Et | NH₂ | Et | SO₂NHMe | NMR |
| 1-250 | thiophen-2-yl | Et | NH₂ | i-Pr | SO₂NHMe | |
| 1-251 | thiophen-3-yl | Et | NH₂ | Me | SO₂NHMe | |
| 1-252 | thiophen-3-yl | Et | NH₂ | Et | SO₂NHMe | |
| 1-253 | thiophen-3-yl | Et | NH₂ | i-Pr | SO₂NHMe | |
| 1-254 | pyridazin-3-yl | Et | NH₂ | Me | SO₂NHMe | |
| 1-255 | pyridazin-3-yl | Et | NH₂ | Et | SO₂NHMe | |
| 1-256 | pyridazin-3-yl | Et | NH₂ | i-Pr | SO₂NHMe | |
| 1-257 | pyrazin-2-yl | Et | NH₂ | Me | SO₂NHMe | |
| 1-258 | pyrazin-2-yl | Et | NH₂ | Et | SO₂NHMe | |
| 1-259 | pyrazin-2-yl | Et | NH₂ | i-Pr | SO₂NHMe | |
| 1-260 | pyrimidin-2-yl | Et | NH₂ | Me | SO₂NHMe | |
| 1-261 | pyrimidin-2-yl | Et | NH₂ | Et | SO₂NHMe | |
| 1-262 | pyrimidin-2-yl | Et | NH₂ | i-Pr | SO₂NHMe | |
| 1-263 | pyrimidin-4-yl | Et | NH₂ | Me | SO₂NHMe | |
| 1-264 | pyrimidin-4-yl | Et | NH₂ | Et | SO₂NHMe | |
| 1-265 | pyrimidin-4-yl | Et | NH₂ | i-Pr | SO₂NHMe | |

**[Table 1-10]**

| Compound No. | R¹ | R³ | R⁴ | R⁵ | A | Physical Property value |
|---|---|---|---|---|---|---|
| 1-266 | pyrimidin-5-yl | Et | NH₂ | Me | SO₂NHMe | |
| 1-267 | pyrimidin-5-yl | Et | NH₂ | Et | SO₂NHMe | |
| 1-268 | pyrimidin-5-yl | Et | NH₂ | i-Pr | SO₂NHMe | |
| 1-269 | Ph | Et | NH₂ | Me | SO₂NHMe | NMR |
| 1-270 | Ph | Et | NH₂ | Et | SO₂NHMe | NMR |
| 1-271 | Ph | Et | NH₂ | i-Pr | SO₂NHMe | |
| 1-272 | 2-F-Ph | Et | NH₂ | Me | SO₂NHMe | |
| 1-273 | 2-F-Ph | Et | NH₂ | Et | SO₂NHMe | |
| 1-274 | 2-F-Ph | Et | NH₂ | i-Pr | SO₂NHMe | |
| 1-275 | 3-F-Ph | Et | NH₂ | Me | SO₂NHMe | |
| 1-276 | 3-F-Ph | Et | NH₂ | Et | SO₂NHMe | |
| 1-277 | 3-F-Ph | Et | NH₂ | i-Pr | SO₂NHMe | |
| 1-278 | 4-F-Ph | Et | NH₂ | Me | SO₂NHMe | |
| 1-279 | 4-F-Ph | Et | NH₂ | Et | SO₂NHMe | |
| 1-280 | 4-F-Ph | Et | NH₂ | i-Pr | SO₂NHMe | |
| 1-281 | 2,6-F₂-Ph | Et | NH₂ | Me | SO₂NHMe | |
| 1-282 | 2,6-F₂-Ph | Et | NH₂ | Et | SO₂NHMe | |
| 1-283 | 2,6-F₂-Ph | Et | NH₂ | i-Pr | SO₂NHMe | |
| 1-284 | n-Bu | Me | NH₂ | Me | SO₂NHMe | NMR |
| 1-285 | n-Bu | Me | NH₂ | Et | SO₂NHMe | NMR |
| 1-286 | n-Bu | Me | NH₂ | i-Pr | SO₂NHMe | |
| 1-287 | 5-Cl-thiazol-2-yl | Me | NH₂ | Me | SO₂NHMe | NMR |
| 1-288 | 4-Me-thiazol-5-yl | Me | NH₂ | Me | SO₂NHMe | NMR |
| 1-289 | 4-Me-thiazol-5-yl | Me | NH₂ | Et | SO₂NHMe | NMR |
| 1-290 | 4-Me-thiazol-5-yl | Me | NH₂ | i-Pr | SO₂NHMe | NMR |
| 1-291 | i-Pr | Et | NH₂ | Me | SO₂NHMe | 103-104 |
| 1-292 | i-Pr | Et | NH₂ | Et | SO₂NHMe | 95-96 |
| 1-293 | i-Bu | Me | NH₂ | Me | SO₂NHMe | 148-149 |
| 1-294 | i-Bu | Me | NH₂ | Et | SO₂NHMe | 101-102 |
| 1-295 | thiophen-2-yl | Me | NH₂ | Me | SO₂NHEt | 211-212 |

**[Table 1-11]**

| Compound No. | R¹ | R³ | R⁴ | R⁵ | A | Physical Property value |
|---|---|---|---|---|---|---|
| 1-296 | thiophen-2-yl | Me | NH₂ | Et | SO₂NHEt | 185-186 |
| 1-297 | thiophen-2-yl | Me | NH₂ | Me | CO₂t-Bu | |
| 1-298 | thiophen-2-yl | Me | NH₂ | Et | CO₂t-Bu | |
| 1-299 | i-Pr | Et | NH₂ | i-Pr | SO₂NHMe | NMR |
| 1-300 | BnO | Me | NH₂ | Et | SO₂NHMe | |
| 1-301 | CF₃CH₂O | Me | NH₂ | Me | SO₂NHMe | |
| 1-302 | CF₃CH₂O | Me | NH₂ | Et | SO₂NHMe | |
| 1-303 | CF₃CH₂O | Me | NH₂ | i-Pr | SO₂NHMe | |
| 1-304 | CHF₂CH₂O | Me | NH₂ | Me | SO₂NHMe | |
| 1-305 | CHF₂CH₂O | Me | NH₂ | Et | SO₂NHMe | |
| 1-306 | CHF₂CH₂O | Me | NH₂ | i-Pr | SO₂NHMe | |
| 1-307 | MeO | Me | NH₂ | Et | SO₂NHMe | |
| 1-308 | BuO | Me | NH₂ | Me | SO₂NHMe | |
| 1-309 | BuO | Me | NH₂ | Et | SO₂NHMe | |
| 1-310 | BuO | Me | NH₂ | i-Pr | SO₂NHMe | |
| 1-311 | PhO | Me | NH₂ | Et | SO₂NHMe | |
| 1-312 | 2-Cl-PhO | Me | NH₂ | Et | SO₂NHMe | |
| 1-313 | 2-OMe-PhO | Me | NH₂ | Et | SO₂NHMe | |
| 1-314 | pyridine-2-yl | CF₃CH₂ | NH₂ | Et | SO₂NHMe | |
| 1-315 | Ph | Me | NH₂ | Me | SO₂NHEt | |
| 1-316 | Ph | Me | NH₂ | Et | SO₂NHEt | |
| 1-317 | Ph | Me | NH₂ | i-Pr | SO₂NHEt | |
| 1-318 | 2-F-Ph | Me | NH₂ | Me | SO₂NHEt | |
| 1-319 | 2-F-Ph | Me | NH₂ | Et | SO₂NHEt | |
| 1-320 | 2-F-Ph | Me | NH₂ | i-Pr | SO₂NHEt | |
| 1-321 | 3-F-Ph | Me | NH₂ | Me | SO₂NHEt | |
| 1-322 | 3-F-Ph | Me | NH₂ | Et | SO₂NHEt | |
| 1-323 | 3-F-Ph | Me | NH₂ | i-Pr | SO₂NHEt | |
| 1-324 | 4-F-Ph | Me | NH₂ | Me | SO₂NHEt | |
| 1-325 | 4-F-Ph | Me | NH₂ | Et | SO₂NHEt | |
| 1-326 | 4-F-Ph | Me | NH₂ | i-Pr | SO₂NHEt | |

**[Table 1-12]**

| Compound No. | R¹ | R³ | R⁴ | R⁵ | A | Physical Property value |
|---|---|---|---|---|---|---|
| 1-327 | 2,6-F₂-Ph | Me | NH₂ | Me | SO₂NHEt | |
| 1-328 | 2,6-F₂-Ph | Me | NH₂ | Et | SO₂NHEt | |
| 1-329 | 2,6-F₂-Ph | Me | NH₂ | i-Pr | SO₂NHEt | |
| 1-330 | thiazol-5-yl | Me | NH₂ | Me | SO₂NHEt | |
| 1-331 | thiazol-5-yl | Me | NH₂ | Et | SO₂NHEt | |
| 1-332 | thiazol-5-yl | Me | NH₂ | i-Pr | SO₂NHEt | |
| 1-333 | thiazol-4-yl | Me | NH₂ | Me | SO₂NHEt | |
| 1-334 | thiazol-4-yl | Me | NH₂ | Et | SO₂NHEt | |
| 1-335 | thiazol-4-yl | Me | NH₂ | i-Pr | SO₂NHEt | |
| 1-336 | thiazol-2-yl | Me | NH₂ | Me | SO₂NHEt | |
| 1-337 | thiazol-2-yl | Me | NH₂ | Et | SO₂NHEt | |
| 1-338 | thiazol-2-yl | Me | NH₂ | i-Pr | SO₂NHEt | |
| 1-339 | thiophen-2-yl | Me | NH₂ | i-Pr | SO₂NHEt | |
| 1-340 | thiophen-3-yl | Me | NH₂ | Me | SO₂NHEt | |
| 1-341 | thiophen-3-yl | Me | NH₂ | Et | SO₂NHEt | |
| 1-342 | thiophen-3-yl | Me | NH₂ | i-Pr | SO₂NHEt | |
| 1-343 | pyridazin-3-yl | Me | NH₂ | Me | SO₂NHEt | |
| 1-344 | pyridazin-3-yl | Me | NH₂ | Et | SO₂NHEt | |
| 1-345 | pyridazin-3-yl | Me | NH₂ | i-Pr | SO₂NHEt | |
| 1-346 | pyrazin-2-yl | Me | NH₂ | Me | SO₂NHEt | |
| 1-347 | pyrazin-2-yl | Me | NH₂ | Et | SO₂NHEt | |
| 1-348 | pyrazin-2-yl | Me | NH₂ | i-Pr | SO₂NHEt | |
| 1-349 | pyrimidin-2-yl | Me | NH₂ | Me | SO₂NHEt | |
| 1-350 | pyrimidin-2-yl | Me | NH₂ | Et | SO₂NHEt | |
| 1-351 | pyrimidin-2-yl | Me | NH₂ | i-Pr | SO₂NHEt | |
| 1-352 | pyrimidin-4-yl | Me | NH₂ | Me | SO₂NHEt | |
| 1-353 | pyrimidin-4-yl | Me | NH₂ | Et | SO₂NHEt | |
| 1-354 | pyrimidin-4-yl | Me | NH₂ | i-Pr | SO₂NHEt | |
| 1-355 | pyrimidin-5-yl | Me | NH₂ | Me | SO₂NHEt | |
| 1-356 | pyrimidin-5-yl | Me | NH₂ | Et | SO₂NHEt | |
| 1-357 | pyrimidin-5-yl | Me | NH₂ | i-Pr | SO₂NHEt | |
| 1-358 | t-Bu | Me | NH₂ | Me | SO₂NHMe | NMR |
| 1-359 | t-Bu | Me | NH₂ | Et | SO₂NHMe | NMR |

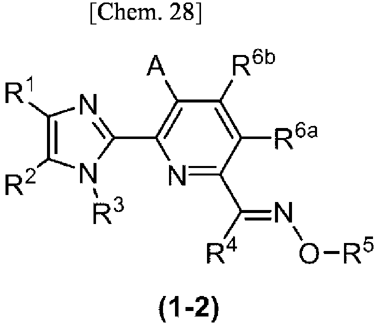

**Table 2 (In Table 2, R^{6a} and R^{6b} represent hydrogen atoms.)**

| [Table 2-1] | | | | | | | |
|---|---|---|---|---|---|---|---|
| Compound No. | R¹ | R² | R³ | R⁴ | R⁵ | A | Physical Property value |
| 2-1 | 2,6-F₂-Ph | H | Me | NH₂ | Et | CO₂Et | 120-121 |
| 2-2 | 2-Cl-Ph | H | Me | NH₂ | Et | CO₂Et | 117-118 |
| 2-3 | 2-Me-Ph | H | Me | NH₂ | Et | CO₂Et | 115-116 |
| 2-4 | 3-Me-Ph | H | Me | NH₂ | Et | CO₂Et | NMR |
| 2-5 | 4-Me-Ph | H | Me | NH₂ | Et | CO₂Et | 77-78 |
| 2-6 | 2-CF₃-Ph | H | Me | NH₂ | Et | CO₂Et | NMR |
| 2-7 | 3-CF₃-Ph | H | Me | NH₂ | Et | CO₂Et | 124-125 |
| 2-8 | 4-CF₃-Ph | H | Me | NH₂ | Et | CO₂Et | NMR |
| 2-9 | 2-OMe-Ph | H | Me | NH₂ | Et | CO₂Et | 107-108 |
| 2-10 | 3-OMe-Ph | H | Me | NH₂ | Et | CO₂Et | 51-52 |
| 2-11 | 4-OMe-Ph | H | Me | NH₂ | Et | CO₂Et | 132-133 |
| 2-12 | 2-F-Ph | H | Me | NH₂ | Et | CO₂Et | 117-118 |
| 2-13 | 3-F-Ph | H | Me | NH₂ | Et | CO₂Et | 59-60 |
| 2-14 | 4-F-Ph | H | Me | NH₂ | Et | CO₂Et | 70-71 |
| 2-15 | 2-Cl-Ph | H | Me | Me | Et | SO₂CH₂Ac | NMR |
| 2-16 | 2-Cl-Ph | H | Me | Me | Et | OMe | 154-155 |
| 2-17 | 2-Cl-Ph | H | Me | Me | Et | OEt | NMR |
| 2-18 | 2-Cl-Ph | Cl | Me | Me | Et | SO₂Me | 143-144 |
| 2-19 | Ph | H | Me | NH₂ | Et | CO₂Et | 62-63 |
| 2-20 | Ph | H | Me | NH₂ | Et | CO₂H | 220-221 |
| 2-21 | Ph | H | Me | NH₂ | Et | CO₂Me | 146-147 |
| 2-22 | Ph | H | Me | NH₂ | CH₂CF₃ | CO₂Me | NMR |
| 2-23 | Ph | Br | Me | NH₂ | Et | CO₂Me | 113-114 |
| 2-24 | Ph | Cl | Me | NH₂ | Et | CO₂Me | 101-102 |
| 2-25 | 3-CF₃-Ph | H | Me | NH₂ | Et | CO₂Me | 56-58 |
| 2-26 | 2,3-F₂-Ph | H | Me | NH₂ | Et | CO₂Et | 134-135 |
| 2-27 | 2,4-F₂-Ph | H | Me | NH₂ | Et | CO₂Et | 100-101 |
| 2-28 | 2,5-F₂-Ph | H | Me | NH₂ | Et | CO₂Et | 122-123 |
| 2-29 | 2-CN-Ph | H | Me | NH₂ | Et | CO₂Et | 103-104 |
| 2-30 | 2,4,6-F₃-Ph | H | Me | NH₂ | Et | CO₂Et | |

**[Table 2-2]**

| Compound No. | R¹ | R² | R³ | R⁴ | R⁵ | A | Physical Property value |
|---|---|---|---|---|---|---|---|
| 2-31 | 2-Cl-Ph | H | Me | NH₂ | Et | SO₂Me | 116-117 |
| 2-32 | 2-Cl-Ph | H | Me | Me | Et | SO₂Me | NMR |
| 2-33 | Ph | H | Me | NH₂ | Et | SO₂NHMe | 237-238 |
| 2-34 | pyridin-3-yl | H | Me | NH₂ | Et | CO₂Et | 57-58 |
| 2-35 | 1-Me-pyrazol-5-yl | H | Me | NH₂ | Et | CO₂Et | 122-123 |
| 2-36 | c-Pr | H | Me | NH₂ | Et | CO₂Et | 107-108 |
| 2-37 | 3,4-dihydro-2H-pyran-6-yl | H | Me | NH₂ | Et | CO₂Et | NMR |
| 2-38 | thiophen-3-yl | H | Me | NH₂ | Et | CO₂Me | 52-53 |

**[Table 2-3]**

| Compound No. | R¹ | R² | R³ | R⁴ | R⁵ | A | Physical Property value |
|---|---|---|---|---|---|---|---|
| 2-39 | thiazol-5-yl | H | Me | NH₂ | Me | SO₂NHMe | |
| 2-40 | thiazol-5-yl | H | Me | NH₂ | Et | SO₂NHMe | |
| 2-41 | thiazol-5-yl | H | Me | NH₂ | i-Pr | SO₂NHMe | |
| 2-42 | thiazol-4-yl | H | Me | NH₂ | Me | SO₂NHMe | |
| 2-43 | thiazol-4-yl | H | Me | NH₂ | Et | SO₂NHMe | |
| 2-44 | thiazol-4-yl | H | Me | NH₂ | i-Pr | SO₂NHMe | |
| 2-45 | thiazol-2-yl | H | Me | NH₂ | Me | SO₂NHMe | |
| 2-46 | thiazol-2-yl | H | Me | NH₂ | Et | SO₂NHMe | |
| 2-47 | thiazol-2-yl | H | Me | NH₂ | i-Pr | SO₂NHMe | |
| 2-48 | thiophen-2-yl | H | Me | NH₂ | Me | SO₂NHMe | |
| 2-49 | thiophen-2-yl | H | Me | NH₂ | Et | SO₂NHMe | |
| 2-50 | thiophen-2-yl | H | Me | NH₂ | i-Pr | SO₂NHMe | |
| 2-51 | thiophen-3-yl | H | Me | NH₂ | Me | SO₂NHMe | |
| 2-52 | thiophen-3-yl | H | Me | NH₂ | Et | SO₂NHMe | |
| 2-53 | thiophen-3-yl | H | Me | NH₂ | i-Pr | SO₂NHMe | |
| 2-54 | pyridazin-3-yl | H | Me | NH₂ | Me | SO₂NHMe | |
| 2-55 | pyridazin-3-yl | H | Me | NH₂ | Et | SO₂NHMe | |
| 2-56 | pyridazin-3-yl | H | Me | NH₂ | i-Pr | SO₂NHMe | |
| 2-57 | pyrazin-2-yl | H | Me | NH₂ | Me | SO₂NHMe | |
| 2-58 | pyrazin-2-yl | H | Me | NH₂ | Et | SO₂NHMe | |
| 2-59 | pyrazin-2-yl | H | Me | NH₂ | i-Pr | SO₂NHMe | |
| 2-60 | pyrimidin-2-yl | H | Me | NH₂ | Me | SO₂NHMe | |
| 2-61 | pyrimidin-2-yl | H | Me | NH₂ | Et | SO₂NHMe | |
| 2-62 | pyrimidin-2-yl | H | Me | NH₂ | i-Pr | SO₂NHMe | |
| 2-63 | pyrimidin-4-yl | H | Me | NH₂ | Me | SO₂NHMe | |
| 2-64 | pyrimidin-4-yl | H | Me | NH₂ | Et | SO₂NHMe | |
| 2-65 | pyrimidin-4-yl | H | Me | NH₂ | i-Pr | SO₂NHMe | |
| 2-66 | pyrimidin-5-yl | H | Me | NH₂ | Me | SO₂NHMe | |
| 2-67 | pyrimidin-5-yl | H | Me | NH₂ | Et | SO₂NHMe | |
| 2-68 | pyrimidin-5-yl | H | Me | NH₂ | i-Pr | SO₂NHMe | |

**[Table 2-4]**

| Compound No. | R¹ | R² | R³ | R⁴ | R⁵ | A | Physical Property value |
|---|---|---|---|---|---|---|---|
| 2-69 | thiazol-5-yl | H | Et | NH₂ | Me | SO₂NHMe | |
| 2-70 | thiazol-5-yl | H | Et | NH₂ | Et | SO₂NHMe | |
| 2-71 | thiazol-5-yl | H | Et | NH₂ | i-Pr | SO₂NHMe | |
| 2-72 | thiazol-4-yl | H | Et | NH₂ | Me | SO₂NHMe | |
| 2-73 | thiazol-4-yl | H | Et | NH₂ | Et | SO₂NHMe | |
| 2-74 | thiazol-4-yl | H | Et | NH₂ | i-Pr | SO₂NHMe | |
| 2-75 | thiazol-2-yl | H | Et | NH₂ | Me | SO₂NHMe | |
| 2-76 | thiazol-2-yl | H | Et | NH₂ | Et | SO₂NHMe | |
| 2-77 | thiazol-2-yl | H | Et | NH₂ | i-Pr | SO₂NHMe | |
| 2-78 | thiophen-2-yl | H | Et | NH₂ | Me | SO₂NHMe | |
| 2-79 | thiophen-2-yl | H | Et | NH₂ | Et | SO₂NHMe | |
| 2-80 | thiophen-2-yl | H | Et | NH₂ | i-Pr | SO₂NHMe | |
| 2-81 | thiophen-3-yl | H | Et | NH₂ | Me | SO₂NHMe | |
| 2-82 | thiophen-3-yl | H | Et | NH₂ | Et | SO₂NHMe | |
| 2-83 | thiophen-3-yl | H | Et | NH₂ | i-Pr | SO₂NHMe | |
| 2-84 | pyridazin-3-yl | H | Et | NH₂ | Me | SO₂NHMe | |
| 2-85 | pyridazin-3-yl | H | Et | NH₂ | Et | SO₂NHMe | |
| 2-86 | pyridazin-3-yl | H | Et | NH₂ | i-Pr | SO₂NHMe | |
| 2-87 | pyrazin-2-yl | H | Et | NH₂ | Me | SO₂NHMe | |
| 2-88 | pyrazin-2-yl | H | Et | NH₂ | Et | SO₂NHMe | |
| 2-89 | pyrazin-2-yl | H | Et | NH₂ | i-Pr | SO₂NHMe | |
| 2-90 | pyrimidin-2-yl | H | Et | NH₂ | Me | SO₂NHMe | |
| 2-91 | pyrimidin-2-yl | H | Et | NH₂ | Et | SO₂NHMe | |
| 2-92 | pyrimidin-2-yl | H | Et | NH₂ | i-Pr | SO₂NHMe | |
| 2-93 | pyrimidin-4-yl | H | Et | NH₂ | Me | SO₂NHMe | |
| 2-94 | pyrimidin-4-yl | H | Et | NH₂ | Et | SO₂NHMe | |
| 2-95 | pyrimidin-4-yl | H | Et | NH₂ | i-Pr | SO₂NHMe | |
| 2-96 | pyrimidin-5-yl | H | Et | NH₂ | Me | SO₂NHMe | |
| 2-97 | pyrimidin-5-yl | H | Et | NH₂ | Et | SO₂NHMe | |
| 2-98 | pyrimidin-5-yl | H | Et | NH₂ | i-Pr | SO₂NHMe | |

**[Table 2-5]**

| Compound No. | R¹ | R² | R³ | R⁴ | R⁵ | A | Physical Property value |
|---|---|---|---|---|---|---|---|
| 2-99 | Ph | H | Et | NH₂ | Me | SO₂NHMe | |
| 2-100 | Ph | H | Et | NH₂ | Et | SO₂NHMe | |
| 2-101 | Ph | H | Et | NH₂ | i-Pr | SO₂NHMe | |
| 2-102 | 2-F-Ph | H | Et | NH₂ | Me | SO₂NHMe | |
| 2-103 | 2-F-Ph | H | Et | NH₂ | Et | SO₂NHMe | |
| 2-104 | 2-F-Ph | H | Et | NH₂ | i-Pr | SO₂NHMe | |
| 2-105 | 3-F-Ph | H | Et | NH₂ | Me | SO₂NHMe | |
| 2-106 | 3-F-Ph | H | Et | NH₂ | Et | SO₂NHMe | |
| 2-107 | 3-F-Ph | H | Et | NH₂ | i-Pr | SO₂NHMe | |
| 2-108 | 4-F-Ph | H | Et | NH₂ | Me | SO₂NHMe | |
| 2-109 | 4-F-Ph | H | Et | NH₂ | Et | SO₂NHMe | |
| 2-110 | 4-F-Ph | H | Et | NH₂ | i-Pr | SO₂NHMe | |
| 2-111 | 2,6-F₂-Ph | H | Et | NH₂ | Me | SO₂NHMe | |
| 2-112 | 2,6-F₂-Ph | H | Et | NH₂ | Et | SO₂NHMe | |
| 2-113 | 2,6-F₂-Ph | H | Et | NH₂ | i-Pr | SO₂NHMe | |
| 2-114 | c-Pr | H | Me | NH₂ | Me | SO₂NHMe | |
| 2-115 | c-Pr | H | Me | NH₂ | Et | SO₂NHMe | |
| 2-116 | c-Pr | H | Me | NH₂ | i-Pr | SO₂NHMe | |
| 2-117 | c-Pr | Cl | Me | NH₂ | Me | SO₂NHMe | |
| 2-118 | c-Pr | Cl | Me | NH₂ | Et | SO₂NHMe | |
| 2-119 | c-Pr | Cl | Me | NH₂ | i-Pr | SO₂NHMe | |
| 2-120 | Ph | H | Me | NH₂ | Me | SO₂NHEt | |
| 2-121 | Ph | H | Me | NH₂ | Et | SO₂NHEt | |
| 2-122 | Ph | H | Me | NH₂ | i-Pr | SO₂NHEt | |
| 2-123 | 2-F-Ph | H | Me | NH₂ | Me | SO₂NHEt | |
| 2-124 | 2-F-Ph | H | Me | NH₂ | Et | SO₂NHEt | |
| 2-125 | 2-F-Ph | H | Me | NH₂ | i-Pr | SO₂NHEt | |
| 2-126 | 3-F-Ph | H | Me | NH₂ | Me | SO₂NHEt | |
| 2-127 | 3-F-Ph | H | Me | NH₂ | Et | SO₂NHEt | |
| 2-128 | 3-F-Ph | H | Me | NH₂ | i-Pr | SO₂NHEt | |

**[Table 2-6]**

| Compound No. | R¹ | R² | R³ | R⁴ | R⁵ | A | Physical Property value |
|---|---|---|---|---|---|---|---|
| 2-129 | 4-F-Ph | H | Me | NH₂ | Me | SO₂NHEt | |
| 2-130 | 4-F-Ph | H | Me | NH₂ | Et | SO₂NHEt | |
| 2-131 | 4-F-Ph | H | Me | NH₂ | i-Pr | SO₂NHEt | |
| 2-132 | 2,6-F₂-Ph | H | Me | NH₂ | Me | SO₂NHEt | |
| 2-133 | 2,6-F₂-Ph | H | Me | NH₂ | Et | SO₂NHEt | |
| 2-134 | 2,6-F₂-Ph | H | Me | NH₂ | i-Pr | SO₂NHEt | |
| 2-135 | thiazol-5-yl | H | Me | NH₂ | Me | SO₂NHEt | |
| 2-136 | thiazol-5-yl | H | Me | NH₂ | Et | SO₂NHEt | |
| 2-137 | thiazol-5-yl | H | Me | NH₂ | i-Pr | SO₂NHEt | |
| 2-138 | thiazol-4-yl | H | Me | NH₂ | Me | SO₂NHEt | |
| 2-139 | thiazol-4-yl | H | Me | NH₂ | Et | SO₂NHEt | |
| 2-140 | thiazol-4-yl | H | Me | NH₂ | i-Pr | SO₂NHEt | |
| 2-141 | thiazol-2-yl | H | Me | NH₂ | Me | SO₂NHEt | |
| 2-142 | thiazol-2-yl | H | Me | NH₂ | Et | SO₂NHEt | |
| 2-143 | thiazol-2-yl | H | Me | NH₂ | i-Pr | SO₂NHEt | |
| 2-144 | thiophen-2-yl | H | Me | NH₂ | Me | SO₂NHEt | |
| 2-145 | thiophen-2-yl | H | Me | NH₂ | Et | SO₂NHEt | |
| 2-146 | thiophen-2-yl | H | Me | NH₂ | i-Pr | SO₂NHEt | |
| 2-147 | thiophen-3-yl | H | Me | NH₂ | Me | SO₂NHEt | |
| 2-148 | thiophen-3-yl | H | Me | NH₂ | Et | SO₂NHEt | |
| 2-149 | thiophen-3-yl | H | Me | NH₂ | i-Pr | SO₂NHEt | |
| 2-150 | pyridazin-3-yl | H | Me | NH₂ | Me | SO₂NHEt | |
| 2-151 | pyridazin-3-yl | H | Me | NH₂ | Et | SO₂NHEt | |
| 2-152 | pyridazin-3-yl | H | Me | NH₂ | i-Pr | SO₂NHEt | |
| 2-153 | pyrazin-2-yl | H | Me | NH₂ | Me | SO₂NHEt | |
| 2-154 | pyrazin-2-yl | H | Me | NH₂ | Et | SO₂NHEt | |
| 2-155 | pyrazin-2-yl | H | Me | NH₂ | i-Pr | SO₂NHEt | |
| 2-156 | pyrimidin-2-yl | H | Me | NH₂ | Me | SO₂NHEt | |
| 2-157 | pyrimidin-2-yl | H | Me | NH₂ | Et | SO₂NHEt | |
| 2-158 | pyrimidin-2-yl | H | Me | NH₂ | i-Pr | SO₂NHEt | |

**[Table 2-7]**

| Compound No. | R¹ | R² | R³ | R⁴ | R⁵ | A | Physical Property value |
|---|---|---|---|---|---|---|---|
| 2-159 | pyrimidin-4-yl | H | Me | NH₂ | Me | SO₂NHEt | |
| 2-160 | pyrimidin-4-yl | H | Me | NH₂ | Et | SO₂NHEt | |
| 2-161 | pyrimidin-4-yl | H | Me | NH₂ | i-Pr | SO₂NHEt | |
| 2-162 | pyrimidin-5-yl | H | Me | NH₂ | Me | SO₂NHEt | |
| 2-163 | pyrimidin-5-yl | H | Me | NH₂ | Et | SO₂NHEt | |
| 2-164 | pyrimidin-5-yl | H | Me | NH₂ | i-Pr | SO₂NHEt | |

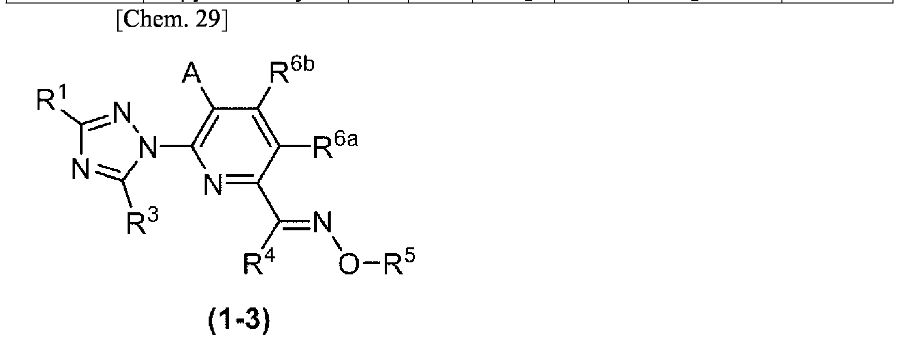

Table 3 (In Table 3, R^{6a} and R^{6b} represent hydrogen atoms.)

**[Table 3-1]**

| Compound No. | R¹ | R³ | R⁴ | R⁵ | A | Physical Property value |
|---|---|---|---|---|---|---|
| 3-1 | 2,3-F₂-Ph | Me | NH₂ | Et | SO₂NHMe | NMR |
| 3-2 | 2-Cl-3-F-Ph | Me | NH₂ | Et | SO₂NHMe | 194-195 |
| 3-3 | Ph | Me | NH₂ | Et | SO₂NHMe | NMR |
| 3-4 | 2-Cl-Ph | Me | NH₂ | Et | SO₂NHMe | NMR |
| 3-5 | 2-Br-Ph | Me | NH₂ | Et | SO₂NHMe | 175-176 |
| 3-6 | 2-Cl-4-F-Ph | Me | NH₂ | Et | SO₂NHMe | NMR |
| 3-7 | 3-F-Ph | Me | NH₂ | Me | SO₂NHMe | 202-203 |
| 3-8 | 2,6-F₂-Ph | Me | NH₂ | Me | SO₂NHMe | NMR |
| 3-9 | Ph | Me | NH₂ | Et | SO₂Me | NMR |
| 3-10 | 2-NHAc-Ph | Me | NH₂ | Et | SO₂NHMe | 196-197 |
| 3-11 | 2,6-F₂-Ph | Me | NH₂ | Et | SO₂NHMe | 144-145 |
| 3-12 | 2-F-Ph | Me | NH₂ | Et | SO₂NHMe | NMR |
| 3-13 | 2-Cl-6-F-Ph | Me | NH₂ | Et | SO₂NHMe | NMR |
| 3-14 | 2,4,6-F₃-Ph | Me | NH₂ | Et | SO₂NHMe | NMR |
| 3-15 | 2,4-F₂-Ph | Me | NH₂ | Et | SO₂NHMe | 136-137 |
| 3-16 | 2-NO₂-Ph | Me | NH₂ | Et | SO₂NHMe | 187-188 |
| 3-17 | 2-Cl-Ph | Me | NH₂ | Me | SO₂NHMe | NMR |
| 3-18 | 2-OMe-Ph | Me | NH₂ | Et | SO₂NHMe | NMR |
| 3-19 | 2-CN-Ph | Me | NH₂ | Et | SO₂NHMe | 237 |
| 3-20 | 3-Cl-Ph | Me | NH₂ | Me | SO₂NHMe | 164-165 |
| 3-21 | Ph | Me | NH₂ | Me | SO₂NHMe | 202-203 |
| 3-22 | 2,6-F₂-Ph | Me | NH₂ | i-Pr | SO₂NHMe | |
| 3-23 | 2-Cl-6-F-Ph | Me | NH₂ | Me | SO₂NHMe | |
| 3-24 | 2-Cl-6-F-Ph | Me | NH₂ | i-Pr | SO₂NHMe | |
| 3-25 | 2-F-Ph | Me | NH₂ | Me | SO₂NHMe | |
| 3-26 | 2-F-Ph | Me | NH₂ | i-Pr | SO₂NHMe | |
| 3-27 | 3-F-Ph | Me | NH₂ | Et | SO₂NHMe | |
| 3-28 | 3-F-Ph | Me | NH₂ | i-Pr | SO₂NHMe | |
| 3-29 | 4-F-Ph | Me | NH₂ | Me | SO₂NHMe | |
| 3-30 | 4-F-Ph | Me | NH₂ | Et | SO₂NHMe | |

**[Table 3-2]**

| Compound No. | R¹ | R³ | R⁴ | R⁵ | A | Physical Property value |
|---|---|---|---|---|---|---|
| 3-31 | 4-F-Ph | Me | NH₂ | i-Pr | SO₂NHMe | |
| 3-32 | 2,5-F₂-Ph | Me | NH₂ | Me | SO₂NHMe | |
| 3-33 | 2,5-F₂-Ph | Me | NH₂ | Et | SO₂NHMe | |
| 3-34 | 2,5-F₂-Ph | Me | NH₂ | i-Pr | SO₂NHMe | |
| 3-35 | 2,4-F₂-Ph | Me | NH₂ | Me | SO₂NHMe | |
| 3-36 | 2,4-F₂-Ph | Me | NH₂ | i-Pr | SO₂NHMe | |
| 3-37 | 2,3-F₂-Ph | Me | NH₂ | Me | SO₂NHMe | |
| 3-38 | 2,3-F₂-Ph | Me | NH₂ | i-Pr | SO₂NHMe | |
| 3-39 | 2,4,6-F₃-Ph | Me | NH₂ | Me | SO₂NHMe | |
| 3-40 | 2,4,6-F₃-Ph | Me | NH₂ | i-Pr | SO₂NHMe | |
| 3-41 | 2-SMe-Ph | Me | NH₂ | Me | SO₂NHMe | |
| 3-42 | 2-SMe-Ph | Me | NH₂ | Et | SO₂NHMe | |
| 3-43 | 2-SMe-Ph | Me | NH₂ | i-Pr | SO₂NHMe | |
| 3-44 | 2-SOMe-Ph | Me | NH₂ | Me | SO₂NHMe | |
| 3-45 | 2-SOMe-Ph | Me | NH₂ | Et | SO₂NHMe | |
| 3-46 | 2-SOMe-Ph | Me | NH₂ | i-Pr | SO₂NHMe | |
| 3-47 | 2-SO₂Me-Ph | Me | NH₂ | Me | SO₂NHMe | |
| 3-48 | 2-SO₂Me-Ph | Me | NH₂ | Et | SO₂NHMe | |
| 3-49 | 2-SO₂Me-Ph | Me | NH₂ | i-Pr | SO₂NHMe | |
| 3-50 | 2-CN-Ph | Me | NH₂ | Me | SO₂NHMe | |
| 3-51 | 2-CN-Ph | Me | NH₂ | i-Pr | SO₂NHMe | |
| 3-52 | 2-OCHF₂-Ph | Me | NH₂ | Me | SO₂NHMe | |
| 3-53 | 2-OCHF₂-Ph | Me | NH₂ | Et | SO₂NHMe | |
| 3-54 | 2-OCHF₂-Ph | Me | NH₂ | i-Pr | SO₂NHMe | |
| 3-55 | 2-c-Pr-Ph | Me | NH₂ | Me | SO₂NHMe | |
| 3-56 | 2-c-Pr-Ph | Me | NH₂ | Et | SO₂NHMe | |
| 3-57 | 2-c-Pr-Ph | Me | NH₂ | i-Pr | SO₂NHMe | |
| 3-58 | 2-CHF₂-Ph | Me | NH₂ | Me | SO₂NHMe | |
| 3-59 | 2-CHF₂-Ph | Me | NH₂ | Et | SO₂NHMe | |
| 3-60 | 2-CHF₂-Ph | Me | NH₂ | i-Pr | SO₂NHMe | |

**[Table 3-3]**

| Compound No. | R¹ | R³ | R⁴ | R⁵ | A | Physical Property value |
|---|---|---|---|---|---|---|
| 3-61 | 2-NHAc-Ph | Me | NH₂ | Me | SO₂NHMe | |
| 3-62 | 2-NHAc-Ph | Me | NH₂ | i-Pr | SO₂NHMe | |
| 3-63 | 2-P(OMe)₂-Ph | Me | NH₂ | Me | SO₂NHMe | |
| 3-64 | 2-P(OMe)₂-Ph | Me | NH₂ | Et | SO₂NHMe | |
| 3-65 | 2-P(OMe)₂-Ph | Me | NH₂ | i-Pr | SO₂NHMe | |
| 3-66 | Ph | Me | NH₂ | Me | SO₂N(Ac)Me | |
| 3-67 | Ph | Me | NH₂ | Et | SO₂N(Ac)Me | |
| 3-68 | Ph | Me | NH₂ | i-Pr | SO₂N(Ac)Me | |
| 3-69 | Ph | Me | NH₂ | Me | SO₂N(CO₂Me)Me | |
| 3-70 | Ph | Me | NH₂ | Et | SO₂N(CO₂Me)Me | |
| 3-71 | Ph | Me | NH₂ | i-Pr | SO₂N(CO₂Me)Me | |
| 3-72 | 2-F-Ph | Me | NH₂ | Me | SO₂N(Ac)Me | |
| 3-73 | 2-F-Ph | Me | NH₂ | Et | SO₂N(Ac)Me | |
| 3-74 | 2-F-Ph | Me | NH₂ | i-Pr | SO₂N(Ac)Me | |
| 3-75 | 2-F-Ph | Me | NH₂ | Me | SO₂N(CO₂Me)Me | |
| 3-76 | 2-F-Ph | Me | NH₂ | Et | SO₂N(CO₂Me)Me | |
| 3-77 | 2-F-Ph | Me | NH₂ | i-Pr | SO₂N(CO₂Me)Me | |
| 3-78 | 3-F-Ph | Me | NH₂ | Me | SO₂N(Ac)Me | |
| 3-79 | 3-F-Ph | Me | NH₂ | Et | SO₂N(Ac)Me | |
| 3-80 | 3-F-Ph | Me | NH₂ | i-Pr | SO₂N(Ac)Me | |
| 3-81 | 3-F-Ph | Me | NH₂ | Me | SO₂N(CO₂Me)Me | |
| 3-82 | 3-F-Ph | Me | NH₂ | Et | SO₂N(CO₂Me)Me | |
| 3-83 | 3-F-Ph | Me | NH₂ | i-Pr | SO₂N(CO₂Me)Me | |
| 3-84 | 4-F-Ph | Me | NH₂ | Me | SO₂N(Ac)Me | |
| 3-85 | 4-F-Ph | Me | NH₂ | Et | SO₂N(Ac)Me | |
| 3-86 | 4-F-Ph | Me | NH₂ | i-Pr | SO₂N(Ac)Me | |
| 3-87 | 4-F-Ph | Me | NH₂ | Me | SO₂N(CO₂Me)Me | |
| 3-88 | 4-F-Ph | Me | NH₂ | Et | SO₂N(CO₂Me)Me | |
| 3-89 | 4-F-Ph | Me | NH₂ | i-Pr | SO₂N(CO₂Me)Me | |
| 3-90 | 2,6-F₂-Ph | Me | NH₂ | Me | SO₂N(Ac)Me | |

**[Table 3-4]**

| Compound No. | R¹ | R³ | R⁴ | R⁵ | A | Physical Property value |
|---|---|---|---|---|---|---|
| 3-91 | 2,6-F₂-Ph | Me | NH₂ | Et | SO₂N(Ac)Me | |
| 3-92 | 2,6-F₂-Ph | Me | NH₂ | i-Pr | SO₂N(Ac)Me | |
| 3-93 | 2,6-F₂-Ph | Me | NH₂ | Me | SO₂N(CO₂Me)Me | |
| 3-94 | 2,6-F₂-Ph | Me | NH₂ | Et | SO₂N(CO₂Me)Me | |
| 3-95 | 2,6-F₂-Ph | Me | NH₂ | i-Pr | SO₂N(CO₂Me)Me | |
| 3-96 | 2,5-F₂-Ph | Me | NH₂ | Me | SO₂N(Ac)Me | |
| 3-97 | 2,5-F₂-Ph | Me | NH₂ | Et | SO₂N(Ac)Me | |
| 3-98 | 2,5-F₂-Ph | Me | NH₂ | i-Pr | SO₂N(Ac)Me | |
| 3-99 | 2,5-F₂-Ph | Me | NH₂ | Me | SO₂N(CO₂Me)Me | |
| 3-100 | 2,5-F₂-Ph | Me | NH₂ | Et | SO₂N(CO₂Me)Me | |
| 3-101 | 2,5-F₂-Ph | Me | NH₂ | i-Pr | SO₂N(CO₂Me)Me | |
| 3-102 | 2,4-F₂-Ph | Me | NH₂ | Me | SO₂N(Ac)Me | |
| 3-103 | 2,4-F₂-Ph | Me | NH₂ | Et | SO₂N(Ac)Me | |
| 3-104 | 2,4-F₂-Ph | Me | NH₂ | i-Pr | SO₂N(Ac)Me | |
| 3-105 | 2,4-F₂-Ph | Me | NH₂ | Me | SO₂N(CO₂Me)Me | |
| 3-106 | 2,4-F₂-Ph | Me | NH₂ | Et | SO₂N(CO₂Me)Me | |
| 3-107 | 2,4-F₂-Ph | Me | NH₂ | i-Pr | SO₂N(CO₂Me)Me | |
| 3-108 | 2,3-F₂-Ph | Me | NH₂ | Me | SO₂N(Ac)Me | |
| 3-109 | 2,3-F₂-Ph | Me | NH₂ | Et | SO₂N(Ac)Me | |
| 3-110 | 2,3-F₂-Ph | Me | NH₂ | i-Pr | SO₂N(Ac)Me | |
| 3-111 | 2,3-F₂-Ph | Me | NH₂ | Me | SO₂N(CO₂Me)Me | |
| 3-112 | 2,3-F₂-Ph | Me | NH₂ | Et | SO₂N(CO₂Me)Me | |
| 3-113 | 2,3-F₂-Ph | Me | NH₂ | i-Pr | SO₂N(CO₂Me)Me | |
| 3-114 | c-Hex | Me | NH₂ | Et | SO₂NHMe | NMR |
| 3-115 | pyridin-2-yl | Me | NH₂ | Me | SO₂NHMe | NMR |
| 3-116 | 3-Cl-pyridin-4-yl | Me | NH₂ | Me | SO₂NHMe | 193-194 |
| 3-117 | 3-Cl-pyridin-4-yl | Me | NH₂ | Et | SO₂NHMe | 188-189 |
| 3-118 | 3-Cl-pyridin-4-yl | Me | NH₂ | i-Pr | SO₂NHMe | NMR |
| 3-119 | 2-Cl-pyridin-3-yl | Me | NH₂ | Et | SO₂NHMe | 212-213 |
| 3-120 | 2-Cl-pyridin-3-yl | Me | NH₂ | i-Pr | SO₂NHMe | 221-222 |

**[Table 3-5]**

| Compound No. | R¹ | R³ | R⁴ | R⁵ | A | Physical Property value |
|---|---|---|---|---|---|---|
| 3-121 | pyridin-2-yl | Me | NH₂ | Et | SO₂NHMe | 127-128 |
| 3-122 | pyridin-4-yl | Me | NH₂ | Et | SO₂NHMe | NMR |
| 3-123 | pyridin-3-yl | Me | NH₂ | Et | SO₂NHMe | NMR |
| 3-124 | 3-Cl-pyridin-2-yl | Me | NH₂ | Et | SO₂NHMe | NMR |
| 3-125 | 3-Cl-pyridin-2-yl | Me | NH₂ | Me | SO₂NHMe | NMR |
| 3-126 | c-Hex | Me | NH₂ | Me | SO₂NHMe | 185-186 |
| 3-127 | c-Hex | Me | NH₂ | i-Pr | SO₂NHMe | NMR |
| 3-128 | c-Pen | Me | NH₂ | Me | SO₂NHMe | |
| 3-129 | c-Pen | Me | NH₂ | Et | SO₂NHMe | NMR |
| 3-130 | c-Pen | Me | NH₂ | i-Pr | SO₂NHMe | NMR |
| 3-131 | c-Bu | Me | NH₂ | Me | SO₂NHMe | |
| 3-132 | c-Bu | Me | NH₂ | Et | SO₂NHMe | |
| 3-133 | c-Bu | Me | NH₂ | i-Pr | SO₂NHMe | |
| 3-134 | c-Pr | Me | NH₂ | Me | SO₂NHMe | |
| 3-135 | c-Pr | Me | NH₂ | Et | SO₂NHMe | |
| 3-136 | c-Pr | Me | NH₂ | i-Pr | SO₂NHMe | |
| 3-137 | 3,4-dihydro-2H-pyran-6-yl | Me | NH₂ | Me | SO₂NHMe | |
| 3-138 | 3,4-dihydro-2H-pyran-6-yl | Me | NH₂ | Et | SO₂NHMe | |
| 3-139 | 3,4-dihydro-2H-pyran-6-yl | Me | NH₂ | i-Pr | SO₂NHMe | |
| 3-140 | tetrahydropyran-2-yl | Me | NH₂ | Me | SO₂NHMe | |
| 3-141 | tetrahydropyran-2-yl | Me | NH₂ | Et | SO₂NHMe | |
| 3-142 | tetrahydropyran-2-yl | Me | NH₂ | i-Pr | SO₂NHMe | |
| 3-143 | 1,3-dioxan-2-yl | Me | NH₂ | Me | SO₂NHMe | |
| 3-144 | 1,3-dioxan-2-yl | Me | NH₂ | Et | SO₂NHMe | |
| 3-145 | 1,3-dioxan-2-yl | Me | NH₂ | i-Pr | SO₂NHMe | |
| 3-146 | tetrahydropyran-3-yl | Me | NH₂ | Me | SO₂NHMe | |
| 3-147 | tetrahydropyran-3-yl | Me | NH₂ | Et | SO₂NHMe | |
| 3-148 | tetrahydropyran-3-yl | Me | NH₂ | i-Pr | SO₂NHMe | |
| 3-149 | tetrahydropyran-4-yl | Me | NH₂ | Me | SO₂NHMe | |
| 3-150 | tetrahydropyran-4-yl | Me | NH₂ | Et | SO₂NHMe | |
| 3-151 | tetrahydropyran-4-yl | Me | NH₂ | i-Pr | SO₂NHMe | NMR |
| 3-152 | tetrahydrothiopyran-4-yl | Me | NH₂ | Me | SO₂NHMe | |
| 3-153 | tetrahydrothiopyran-4-yl | Me | NH₂ | Et | SO₂NHMe | |

**[Table 3-6]**

| Compound No. | R¹ | R³ | R⁴ | R⁵ | A | Physical Property value |
|---|---|---|---|---|---|---|
| 3-154 | tetrahydrothiopyran-4-yl | Me | NH₂ | i-Pr | SO₂NHMe | |
| 3-155 | 1-Ac-piperidin-4-yl | Me | NH₂ | Me | SO₂NHMe | |
| 3-156 | 1-Ac-piperidin-4-yl | Me | NH₂ | Et | SO₂NHMe | |
| 3-157 | 1-Ac-piperidin-4-yl | Me | NH₂ | i-Pr | SO₂NHMe | |
| 3-158 | piperidin-1-yl | Me | NH₂ | Me | SO₂NHMe | |
| 3-159 | piperidin-1-yl | Me | NH₂ | Et | SO₂NHMe | |
| 3-160 | piperidin-1-yl | Me | NH₂ | i-Pr | SO₂NHMe | |
| 3-161 | 1,3-dioxolan-2-yl | Me | NH₂ | Me | SO₂NHMe | |
| 3-162 | 1,3-dioxolan-2-yl | Me | NH₂ | Et | SO₂NHMe | |
| 3-163 | 1,3-dioxolan-2-yl | Me | NH₂ | i-Pr | SO₂NHMe | |
| 3-164 | 1,3-dioxepan-2-yl | Me | NH₂ | Me | SO₂NHMe | |
| 3-165 | 1,3-dioxepan-2-yl | Me | NH₂ | Et | SO₂NHMe | |
| 3-166 | 1,3-dioxepan-2-yl | Me | NH₂ | i-Pr | SO₂NHMe | |
| 3-167 | thiazol-5-yl | Me | NH₂ | Me | SO₂NHMe | |
| 3-168 | thiazol-5-yl | Me | NH₂ | Et | SO₂NHMe | NMR |
| 3-169 | thiazol-5-yl | Me | NH₂ | i-Pr | SO₂NHMe | |
| 3-170 | thiazol-4-yl | Me | NH₂ | Me | SO₂NHMe | |
| 3-171 | thiazol-4-yl | Me | NH₂ | Et | SO₂NHMe | NMR |
| 3-172 | thiazol-4-yl | Me | NH₂ | i-Pr | SO₂NHMe | |
| 3-173 | 1,2,3-thiadiazol-5-yl | Me | NH₂ | Me | SO₂NHMe | |
| 3-174 | 1,2,3-thiadiazol-5-yl | Me | NH₂ | Et | SO₂NHMe | NMR |
| 3-175 | 1,2,3-thiadiazol-5-yl | Me | NH₂ | i-Pr | SO₂NHMe | |
| 3-176 | thiophen-2-yl | Me | NH₂ | Me | SO₂NHMe | NMR |
| 3-177 | thiophen-2-yl | Me | NH₂ | Et | SO₂NHMe | NMR |
| 3-178 | thiophen-2-yl | Me | NH₂ | i-Pr | SO₂NHMe | |
| 3-179 | thiophen-3-yl | Me | NH₂ | Me | SO₂NHMe | NMR |
| 3-180 | thiophen-3-yl | Me | NH₂ | Et | SO₂NHMe | NMR |
| 3-181 | thiophen-3-yl | Me | NH₂ | i-Pr | SO₂NHMe | |
| 3-182 | pyridazin-3-yl | Me | NH₂ | Me | SO₂NHMe | |
| 3-183 | pyridazin-3-yl | Me | NH₂ | Et | SO₂NHMe | |
| 3-184 | pyridazin-3-yl | Me | NH₂ | i-Pr | SO₂NHMe | |
| 3-185 | pyrimidin-2-yl | Me | NH₂ | Et | SO₂NHMe | |
| 3-186 | pyrimidin-2-yl | Me | NH₂ | i-Pr | SO₂NHMe | |

**[Table 3-7]**

| Compound No. | R¹ | R³ | R⁴ | R⁵ | A | Physical Property value |
|---|---|---|---|---|---|---|
| 3-187 | thiazol-2-yl | Me | NH₂ | Me | SO₂NHMe | |
| 3-188 | thiazol-2-yl | Me | NH₂ | Et | SO₂NHMe | |
| 3-189 | thiazol-2-yl | Me | NH₂ | i-Pr | SO₂NHMe | |
| 3-190 | n-Pr | Me | NH₂ | Et | SO₂NHMe | NMR |
| 3-191 | thiophen-2-yl | Me | NH₂ | Et | SO₂Me | NMR |
| 3-192 | 3-Me-thiophen-2-yl | Me | NH₂ | Et | SO₂NHMe | NMR |
| 3-193 | 3-Cl-thiophen-2-yl | Me | NH₂ | Et | SO₂NHMe | NMR |
| 3-194 | 4-OMe-thiophen-3-yl | Me | NH₂ | Et | SO₂NHMe | NMR |
| 3-195 | 3,5-Cl₂-thiophen-2-yl | Me | NH₂ | Me | SO₂NHMe | NMR |
| 3-196 | 3-Cl-5-Br-thiophen-2-yl | Me | NH₂ | Me | SO₂NHMe | NMR |
| 3-197 | 3-F-thiophen-2-yl | Me | NH₂ | Et | SO₂NHMe | NMR |
| 3-198 | n-Bu | Me | NH₂ | Et | SO₂NHMe | 108-109 |
| 3-199 | Me | n-Bu | NH₂ | Et | SO₂NHMe | 110-111 |
| 3-200 | i-allyl | Me | NH₂ | Et | SO₂NHMe | 129-130 |
| 3-201 | 3-CI-thiophen-2-yl | Me | NH₂ | Me | SO₂NHMe | NMR |
| 3-202 | n-Pr | Me | NH₂ | Et | SO₂NMe2 | 148-149 |
| 3-203 | i-Bu | Me | NH₂ | Et | SO₂NHMe | 125-126 |
| 3-204 | i-Pen | Me | NH₂ | Et | SO₂NHMe | NMR |
| 3-205 | c-Pr | Me | NH₂ | Et | SO₂NHMe | 136-137 |
| 3-206 | Me | i-allyl | NH₂ | Et | SO₂NHMe | 191-192 |
| 3-207 | Me | Me | NH₂ | Et | SO₂NHMe | NMR |
| 3-208 | n-propylsulfanyl | Me | NH₂ | Et | SO₂NHMe | 111-112 |
| 3-209 | ethylsulfanyl | Me | NH₂ | Et | SO₂NHMe | 99-100 |
| 3-210 | n-propylsulfonyl | Me | NH₂ | Et | SO₂NHMe | 129-130 |
| 3-211 | pyrazin-2-yl | Me | NH₂ | Me | SO₂NHMe | |
| 3-212 | pyrazin-2-yl | Me | NH₂ | Et | SO₂NHMe | |
| 3-213 | pyrazin-2-yl | Me | NH₂ | i-Pr | SO₂NHMe | |
| 3-214 | pyrimidin-4-yl | Me | NH₂ | Me | SO₂NHMe | |
| 3-215 | pyrimidin-4-yl | Me | NH₂ | Et | SO₂NHMe | |
| 3-216 | pyrimidin-4-yl | Me | NH₂ | i-Pr | SO₂NHMe | |

**[Table 3-8]**

| Compound No. | R¹ | R³ | R⁴ | R⁵ | A | Physical Property value |
|---|---|---|---|---|---|---|
| 3-217 | pyrimidin-5-yl | Me | NH₂ | Me | SO₂NHMe | |
| 3-218 | pyrimidin-5-yl | Me | NH₂ | Et | SO₂NHMe | |
| 3-219 | pyrimidin-5-yl | Me | NH₂ | i-Pr | SO₂NHMe | |
| 3-220 | thiazol-5-yl | Et | NH₂ | Me | SO₂NHMe | |
| 3-221 | thiazol-5-yl | Et | NH₂ | Et | SO₂NHMe | |
| 3-222 | thiazol-5-yl | Et | NH₂ | i-Pr | SO₂NHMe | |
| 3-223 | thiazol-4-yl | Et | NH₂ | Me | SO₂NHMe | |
| 3-224 | thiazol-4-yl | Et | NH₂ | Et | SO₂NHMe | |
| 3-225 | thiazol-4-yl | Et | NH₂ | i-Pr | SO₂NHMe | |
| 3-226 | thiazol-2-yl | Et | NH₂ | Me | SO₂NHMe | |
| 3-227 | thiazol-2-yl | Et | NH₂ | Et | SO₂NHMe | |
| 3-228 | thiazol-2-yl | Et | NH₂ | i-Pr | SO₂NHMe | |
| 3-229 | thiophen-2-yl | Et | NH₂ | Me | SO₂NHMe | NMR |
| 3-230 | thiophen-2-yl | Et | NH₂ | Et | SO₂NHMe | NMR |
| 3-231 | thiophen-2-yl | Et | NH₂ | i-Pr | SO₂NHMe | |
| 3-232 | thiophen-3-yl | Et | NH₂ | Me | SO₂NHMe | |
| 3-233 | thiophen-3-yl | Et | NH₂ | Et | SO₂NHMe | |
| 3-234 | thiophen-3-yl | Et | NH₂ | i-Pr | SO₂NHMe | |
| 3-235 | pyridazin-3-yl | Et | NH₂ | Me | SO₂NHMe | |
| 3-236 | pyridazin-3-yl | Et | NH₂ | Et | SO₂NHMe | |
| 3-237 | pyridazin-3-yl | Et | NH₂ | i-Pr | SO₂NHMe | |
| 3-238 | pyrazin-2-yl | Et | NH₂ | Me | SO₂NHMe | |
| 3-239 | pyrazin-2-yl | Et | NH₂ | Et | SO₂NHMe | |
| 3-240 | pyrazin-2-yl | Et | NH₂ | i-Pr | SO₂NHMe | |
| 3-241 | pyrimidin-2-yl | Et | NH₂ | Me | SO₂NHMe | |
| 3-242 | pyrimidin-2-yl | Et | NH₂ | Et | SO₂NHMe | |
| 3-243 | pyrimidin-2-yl | Et | NH₂ | i-Pr | SO₂NHMe | |
| 3-244 | pyrimidin-4-yl | Et | NH₂ | Me | SO₂NHMe | |
| 3-245 | pyrimidin-4-yl | Et | NH₂ | Et | SO₂NHMe | |
| 3-246 | pyrimidin-4-yl | Et | NH₂ | i-Pr | SO₂NHMe | |

**[Table 3-9]**

| Compound No. | R¹ | R³ | R⁴ | R⁵ | A | Physical Property value |
|---|---|---|---|---|---|---|
| 3-247 | pyrimidin-5-yl | Et | NH₂ | Me | SO₂NHMe | |
| 3-248 | pyrimidin-5-yl | Et | NH₂ | Et | SO₂NHMe | |
| 3-249 | pyrimidin-5-yl | Et | NH₂ | i-Pr | SO₂NHMe | |
| 3-250 | Ph | Et | NH₂ | Me | SO₂NHMe | NMR |
| 3-251 | Ph | Et | NH₂ | Et | SO₂NHMe | NMR |
| 3-252 | Ph | Et | NH₂ | i-Pr | SO₂NHMe | |
| 3-253 | 2-F-Ph | Et | NH₂ | Me | SO₂NHMe | |
| 3-254 | 2-F-Ph | Et | NH₂ | Et | SO₂NHMe | |
| 3-255 | 2-F-Ph | Et | NH₂ | i-Pr | SO₂NHMe | |
| 3-256 | 3-F-Ph | Et | NH₂ | Me | SO₂NHMe | |
| 3-257 | 3-F-Ph | Et | NH₂ | Et | SO₂NHMe | |
| 3-258 | 3-F-Ph | Et | NH₂ | i-Pr | SO₂NHMe | |
| 3-259 | 4-F-Ph | Et | NH₂ | Me | SO₂NHMe | |
| 3-260 | 4-F-Ph | Et | NH₂ | Et | SO₂NHMe | |
| 3-261 | 4-F-Ph | Et | NH₂ | i-Pr | SO₂NHMe | |
| 3-262 | 2,6-F₂-Ph | Et | NH₂ | Me | SO₂NHMe | |
| 3-263 | 2,6-F₂-Ph | Et | NH₂ | Et | SO₂NHMe | |
| 3-264 | 2,6-F₂-Ph | Et | NH₂ | i-Pr | SO₂NHMe | |
| 3-265 | 3-Me-isothiazol-5-yl | Me | NH₂ | Et | SO₂NHMe | NMR |
| 3-266 | ethylsulfonyl | Me | NH₂ | Et | SO₂NHMe | 146-147 |
| 3-267 | 3-Br-thiophen-2-yl | Me | NH₂ | Et | SO₂NHMe | NMR |
| 3-268 | 3-F-5-Cl-thiophen-2-yl | Me | NH₂ | Et | SO₂NHMe | NMR |
| 3-269 | 3-F-5-Br-thiophen-2-yl | Me | NH₂ | Et | SO₂NHMe | NMR |
| 3-270 | 4-Me-thiadiazol-5-yl | Me | NH₂ | Et | SO₂NHMe | NMR |
| 3-271 | 5-Me-1,2,3-thiadiazol-4-yl | Me | NH₂ | Et | SO₂NHMe | NMR |
| 3-272 | furan-3-yl | Me | NH₂ | Et | SO₂NHMe | NMR |
| 3-273 | isothiazol-5-yl | Me | NH₂ | Et | SO₂NHMe | NMR |
| 3-274 | furan-2-yl | Me | NH₂ | Et | SO₂NHMe | NMR |
| 3-275 | I | Me | NH₂ | Et | SO₂NHMe | |
| 3-276 | vinyl | Me | NH₂ | Et | SO₂NHMe | |

**[Table 3-10]**

| Compound No. | R¹ | R³ | R⁴ | R⁵ | A | Physical Property value |
|---|---|---|---|---|---|---|
| 3-277 | Et | Me | NH₂ | Et | SO₂NHMe | |
| 3-278 | Et | Et | NH₂ | Et | SO₂NHMe | 125-126 |
| 3-279 | 1-Me-pyrazol-5-yl | Me | NH₂ | Et | SO₂NHMe | NMR |
| 3-280 | OEt | Me | NH₂ | Et | SO₂NHMe | |
| 3-281 | NHEt | Me | NH₂ | Et | SO₂NHMe | |
| 3-282 | Ph | OMe | NH₂ | Me | SO₂NHMe | |
| 3-283 | Ph | OMe | NH₂ | Et | SO₂NHMe | |
| 3-284 | Ph | OMe | NH₂ | Et | SO₂Me | |
| 3-285 | Ph | OH | NH₂ | Me | SO₂NHMe | |
| 3-286 | Ph | OH | NH₂ | Et | SO₂NHMe | NMR |
| 3-287 | Ph | OH | NH₂ | Et | SO₂Me | 259-260 |
| 3-288 | Ph | SMe | NH₂ | Me | SO₂NHMe | |
| 3-289 | Ph | SMe | NH₂ | Et | SO₂NHMe | |
| 3-290 | Ph | SMe | NH₂ | Et | SO₂Me | |
| 3-291 | Ph | SOMe | NH₂ | Me | SO₂NHMe | |
| 3-292 | Ph | SOMe | NH₂ | Et | SO₂NHMe | |
| 3-293 | Ph | SOMe | NH₂ | Et | SO₂Me | |
| 3-294 | Ph | SO₂Me | NH₂ | Me | SO₂NHMe | |
| 3-295 | Ph | SO₂Me | NH₂ | Et | SO₂NHMe | |
| 3-296 | Ph | SO₂Me | NH₂ | Et | SO₂Me | |
| 3-297 | Ph | vinyl | NH₂ | Me | SO₂NHMe | |
| 3-298 | Ph | vinyl | NH₂ | Et | SO₂NHMe | |
| 3-299 | Ph | vinyl | NH₂ | Et | SO₂Me | |
| 3-300 | Ph | NHMe | NH₂ | Me | SO₂NHMe | |
| 3-301 | Ph | NHMe | NH₂ | Et | SO₂NHMe | |
| 3-302 | Ph | NHMe | NH₂ | Et | SO₂Me | |
| 3-303 | Ph | CF₃ | NH₂ | Me | SO₂NHMe | |
| 3-304 | Ph | CF₃ | NH₂ | Et | SO₂NHMe | |
| 3-305 | Ph | CHF₂ | NH₂ | Me | SO₂NHMe | |
| 3-306 | Ph | CHF₂ | NH₂ | Et | SO₂NHMe | |

**[Table 3-11]**

| Compound No. | R¹ | R³ | R⁴ | R⁵ | A | Physical Property value |
|---|---|---|---|---|---|---|
| 3-307 | thiophen-2-yl | OMe | NH₂ | Me | SO₂NHMe | |
| 3-308 | thiophen-2-yl | OMe | NH₂ | Et | SO₂NHMe | |
| 3-309 | thiophen-2-yl | OMe | NH₂ | Et | SO₂Me | |
| 3-310 | thiophen-2-yl | OH | NH₂ | Me | SO₂NHMe | |
| 3-311 | thiophen-2-yl | OH | NH₂ | Et | SO₂NHMe | |
| 3-312 | thiophen-2-yl | OH | NH₂ | Et | SO₂Me | |
| 3-313 | thiophen-2-yl | SMe | NH₂ | Me | SO₂NHMe | |
| 3-314 | thiophen-2-yl | SMe | NH₂ | Et | SO₂NHMe | |
| 3-315 | thiophen-2-yl | SMe | NH₂ | Et | SO₂Me | 106-107 |
| 3-316 | thiophen-2-yl | SOMe | NH₂ | Me | SO₂NHMe | |
| 3-317 | thiophen-2-yl | SOMe | NH₂ | Et | SO₂NHMe | |
| 3-318 | thiophen-2-yl | SOMe | NH₂ | Et | SO₂Me | NMR |
| 3-319 | thiophen-2-yl | SO₂Me | NH₂ | Me | SO₂NHMe | |
| 3-320 | thiophen-2-yl | SO₂Me | NH₂ | Et | SO₂NHMe | |
| 3-321 | thiophen-2-yl | SO₂Me | NH₂ | Et | SO₂Me | NMR |
| 3-322 | thiophen-2-yl | vinyl | NH₂ | Me | SO₂NHMe | |
| 3-323 | thiophen-2-yl | vinyl | NH₂ | Et | SO₂NHMe | |
| 3-324 | thiophen-2-yl | vinyl | NH₂ | Et | SO₂Me | NMR |
| 3-325 | thiophen-2-yl | NHMe | NH₂ | Me | SO₂NHMe | |
| 3-326 | thiophen-2-yl | NHMe | NH₂ | Et | SO₂NHMe | |
| 3-327 | thiophen-2-yl | NHMe | NH₂ | Et | SO₂Me | |
| 3-328 | thiophen-2-yl | CF₃ | NH₂ | Me | SO₂NHMe | |
| 3-329 | thiophen-2-yl | CF₃ | NH₂ | Et | SO₂NHMe | |
| 3-330 | thiophen-2-yl | CHF₂ | NH₂ | Me | SO₂NHMe | |
| 3-331 | thiophen-2-yl | CHF₂ | NH₂ | Et | SO₂NHMe | |
| 3-332 | Ph | Me | NH₂ | Me | SO₂NHEt | |
| 3-333 | Ph | Me | NH₂ | Et | SO₂NHEt | |
| 3-334 | Ph | Me | NH₂ | i-Pr | SO₂NHEt | |
| 3-335 | 2-F-Ph | Me | NH₂ | Me | SO₂NHEt | |
| 3-336 | 2-F-Ph | Me | NH₂ | Et | SO₂NHEt | |

**[Table 3-12]**

| Compound No. | R¹ | R³ | R⁴ | R⁵ | A | Physical Property value |
|---|---|---|---|---|---|---|
| 3-337 | 2-F-Ph | Me | NH₂ | i-Pr | SO₂NHEt | |
| 3-338 | 3-F-Ph | Me | NH₂ | Me | SO₂NHEt | |
| 3-339 | 3-F-Ph | Me | NH₂ | Et | SO₂NHEt | |
| 3-340 | 3-F-Ph | Me | NH₂ | i-Pr | SO₂NHEt | |
| 3-341 | 4-F-Ph | Me | NH₂ | Me | SO₂NHEt | |
| 3-342 | 4-F-Ph | Me | NH₂ | Et | SO₂NHEt | |
| 3-343 | 4-F-Ph | Me | NH₂ | i-Pr | SO₂NHEt | |
| 3-344 | 2,6-F₂-Ph | Me | NH₂ | Me | SO₂NHEt | |
| 3-345 | 2,6-F₂-Ph | Me | NH₂ | Et | SO₂NHEt | |
| 3-346 | 2,6-F₂-Ph | Me | NH₂ | i-Pr | SO₂NHEt | |
| 3-347 | thiazol-5-yl | Me | NH₂ | Me | SO₂NHEt | |
| 3-348 | thiazol-5-yl | Me | NH₂ | Et | SO₂NHEt | |
| 3-349 | thiazol-5-yl | Me | NH₂ | i-Pr | SO₂NHEt | |
| 3-350 | thiazol-4-yl | Me | NH₂ | Me | SO₂NHEt | |
| 3-351 | thiazol-4-yl | Me | NH₂ | Et | SO₂NHEt | |
| 3-352 | thiazol-4-yl | Me | NH₂ | i-Pr | SO₂NHEt | |
| 3-353 | thiazol-2-yl | Me | NH₂ | Me | SO₂NHEt | |
| 3-354 | thiazol-2-yl | Me | NH₂ | Et | SO₂NHEt | |
| 3-355 | thiazol-2-yl | Me | NH₂ | i-Pr | SO₂NHEt | |
| 3-356 | thiophen-2-yl | Me | NH₂ | Me | SO₂NHEt | |
| 3-357 | thiophen-2-yl | Me | NH₂ | Et | SO₂NHEt | |
| 3-358 | thiophen-2-yl | Me | NH₂ | i-Pr | SO₂NHEt | |
| 3-359 | thiophen-3-yl | Me | NH₂ | Me | SO₂NHEt | |
| 3-360 | thiophen-3-yl | Me | NH₂ | Et | SO₂NHEt | |
| 3-361 | thiophen-3-yl | Me | NH₂ | i-Pr | SO₂NHEt | |
| 3-362 | pyridazin-3-yl | Me | NH₂ | Me | SO₂NHEt | |
| 3-363 | pyridazin-3-yl | Me | NH₂ | Et | SO₂NHEt | |
| 3-364 | pyridazin-3-yl | Me | NH₂ | i-Pr | SO₂NHEt | |
| 3-365 | pyrazin-2-yl | Me | NH₂ | Me | SO₂NHEt | |
| 3-366 | pyrazin-2-yl | Me | NH₂ | Et | SO₂NHEt | |

**[Table 3-13]**

| Compound No. | R¹ | R³ | R⁴ | R⁵ | A | Physical Property value |
|---|---|---|---|---|---|---|
| 3-367 | pyrazin-2-yl | Me | NH₂ | i-Pr | SO₂NHEt | |
| 3-368 | pyrimidin-2-yl | Me | NH₂ | Me | SO₂NHEt | |
| 3-369 | pyrimidin-2-yl | Me | NH₂ | Et | SO₂NHEt | |
| 3-370 | pyrimidin-2-yl | Me | NH₂ | i-Pr | SO₂NHEt | |
| 3-371 | pyrimidin-4-yl | Me | NH₂ | Me | SO₂NHEt | |
| 3-372 | pyrimidin-4-yl | Me | NH₂ | Et | SO₂NHEt | |
| 3-373 | pyrimidin-4-yl | Me | NH₂ | i-Pr | SO₂NHEt | |
| 3-374 | pyrimidin-5-yl | Me | NH₂ | Me | SO₂NHEt | |
| 3-375 | pyrimidin-5-yl | Me | NH₂ | Et | SO₂NHEt | |
| 3-376 | pyrimidin-5-yl | Me | NH₂ | i-Pr | SO₂NHEt | |
| 3-377 | thiophen-2-yl | Et | NH₂ | Et | SO₂Me | NMR |
| 3-378 | thiophen-2-yl | CHF₂ | NH₂ | Et | SO₂Me | NMR |
| 3-379 | thiophen-2-yl | CF₃ | NH₂ | Et | SO₂Me | NMR |

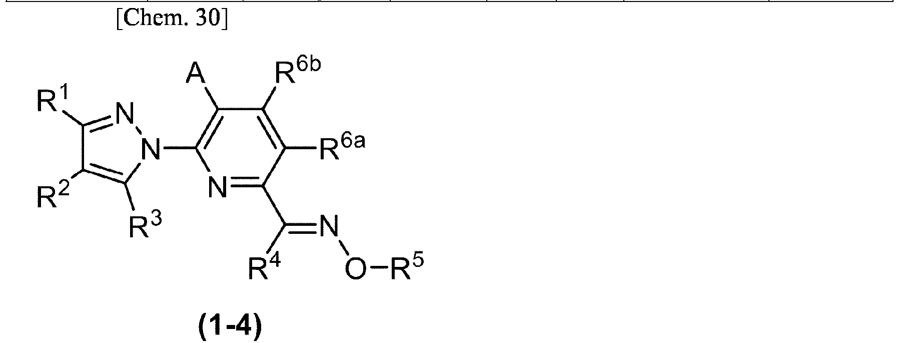

Table 4 (In Table 4, R^{6a} and R^{6b} represent hydrogen atoms.)

**[Table 4-1]**

| Compound No. | R¹ | R² | R³ | R⁴ | R⁵ | A | Physical Property value |
|---|---|---|---|---|---|---|---|
| 4-1 | Ph | H | Me | NH₂ | Et | SO₂Me | NMR |
| 4-2 | 2-Cl-Ph | H | Me | NH₂ | Et | SO₂Me | NMR |
| 4-3 | 4-CN-Ph | H | Me | NH₂ | Et | SO₂NHMe | 170-171 |
| 4-4 | 4-(imidazol-1-yl)-Ph | H | Me | NH₂ | Et | SO₂NHMe | 217-218 |
| 4-5 | 2-Cl-Ph | H | Me | NH₂ | Et | SO₂NHMe | NMR |
| 4-6 | Ph | H | Me | NH₂ | Et | SO₂NHMe | NMR |
| 4-7 | 2,4-Cl₂-Ph | H | Me | NH₂ | Et | SO₂NHMe | NMR |
| 4-8 | 4-OMe-Ph | H | Me | NH₂ | Et | SO₂NHMe | NMR |
| 4-9 | 2-Br-Ph | H | Me | NH₂ | Me | SO₂NHMe | 153-155 |
| 4-10 | 2-CN-Ph | H | Me | NH₂ | Me | SO₂NHMe | NMR |
| 4-11 | 4-Cl-Ph | H | Et | NH₂ | Et | SO₂NHMe | NMR |
| 4-12 | Ph | H | Me | NH₂ | Me | SO₂NHMe | 206-207 |
| 4-13 | 2-Cl-Ph | H | Me | NH₂ | Me | SO₂NHMe | 181-182 |
| 4-14 | 4-Cl-Ph | H | Me | NH₂ | Et | SO₂NHMe | NMR |
| 4-15 | 2-Me-Ph | H | Me | NH₂ | Et | SO₂NHMe | NMR |
| 4-16 | 3-Cl-Ph | H | Me | NH₂ | Et | SO₂NHMe | NMR |
| 4-17 | 2-Cl-Ph | H | Me | NH₂ | Et | SO₂NMe₂ | 100-101 |
| 4-18 | Ph | H | OEt | NH₂ | Et | SO₂Me | NMR |
| 4-19 | Ph | H | OMe | NH₂ | Et | SO₂Me | NMR |
| 4-20 | Ph | H | OH | NH₂ | Et | SO₂Me | NMR |
| 4-21 | 2-Cl-Ph | H | OH | NH₂ | Et | SO₂NHMe | NMR |
| 4-22 | Ph | H | OH | NH₂ | Et | SO₂NHMe | NMR |
| 4-23 | 4-Cl-Ph | H | SMe | NH₂ | Et | SO₂NHMe | NMR |
| 4-24 | 4-Cl-Ph | H | SOMe | NH₂ | Et | SO₂NHMe | NMR |
| 4-25 | 4-Cl-Ph | H | SO₂Me | NH₂ | Et | SO₂NHMe | NMR |
| 4-26 | 2-F-Ph | H | OH | NH₂ | Et | SO₂NHMe | NMR |
| 4-27 | 2-F-Ph | H | Me | NH₂ | Me | SO₂NHMe | |
| 4-28 | 2-F-Ph | H | Me | NH₂ | Et | SO₂NHMe | |
| 4-29 | 2-F-Ph | H | Me | NH₂ | i-Pr | SO₂NHMe | |
| 4-30 | 3-F-Ph | H | Me | NH₂ | Me | SO₂NHMe | |

**[Table 4-2]**

| Compound No. | R¹ | R² | R³ | R⁴ | R⁵ | A | Physical Property value |
|---|---|---|---|---|---|---|---|
| 4-31 | 3-F-Ph | H | Me | NH₂ | Et | SO₂NHMe | |
| 4-32 | 3-F-Ph | H | Me | NH₂ | i-Pr | SO₂NHMe | |
| 4-33 | 4-F-Ph | H | Me | NH₂ | Me | SO₂NHMe | |
| 4-34 | 4-F-Ph | H | Me | NH₂ | Et | SO₂NHMe | |
| 4-35 | 4-F-Ph | H | Me | NH₂ | i-Pr | SO₂NHMe | |
| 4-36 | 2,6-F₂-Ph | H | Me | NH₂ | Me | SO₂NHMe | |
| 4-37 | 2,6-F₂-Ph | H | Me | NH₂ | Et | SO₂NHMe | |
| 4-38 | 2,6-F₂-Ph | H | Me | NH₂ | i-Pr | SO₂NHMe | |
| 4-39 | 2,5-F₂-Ph | H | Me | NH₂ | Me | SO₂NHMe | |
| 4-40 | 2,5-F₂-Ph | H | Me | NH₂ | Et | SO₂NHMe | |
| 4-41 | 2,5-F₂-Ph | H | Me | NH₂ | i-Pr | SO₂NHMe | |
| 4-42 | 2,4-F₂-Ph | H | Me | NH₂ | Me | SO₂NHMe | |
| 4-43 | 2,4-F₂-Ph | H | Me | NH₂ | Et | SO₂NHMe | |
| 4-44 | 2,4-F₂-Ph | H | Me | NH₂ | i-Pr | SO₂NHMe | |
| 4-45 | 2,3-F₂-Ph | H | Me | NH₂ | Me | SO₂NHMe | |
| 4-46 | 2,3-F₂-Ph | H | Me | NH₂ | Et | SO₂NHMe | |
| 4-47 | 2,3-F₂-Ph | H | Me | NH₂ | i-Pr | SO₂NHMe | |
| 4-48 | 2,3-F₂-Ph | H | Me | NH₂ | Me | SO₂NHMe | |
| 4-49 | 2,3-F₂-Ph | H | Me | NH₂ | Et | SO₂NHMe | |
| 4-50 | 2,3-F₂-Ph | H | Me | NH₂ | i-Pr | SO₂NHMe | |
| 4-51 | 2-SMe-Ph | H | Me | NH₂ | Me | SO₂NHMe | |
| 4-52 | 2-SMe-Ph | H | Me | NH₂ | Et | SO₂NHMe | NMR |
| 4-53 | 2-SMe-Ph | H | Me | NH₂ | i-Pr | SO₂NHMe | |
| 4-54 | 2-SOMe-Ph | H | Me | NH₂ | Me | SO₂NHMe | |
| 4-55 | 2-SOMe-Ph | H | Me | NH₂ | Et | SO₂NHMe | |
| 4-56 | 2-SOMe-Ph | H | Me | NH₂ | i-Pr | SO₂NHMe | |
| 4-57 | 2-SO₂Me-Ph | H | Me | NH₂ | Me | SO₂NHMe | |
| 4-58 | 2-SO₂Me-Ph | H | Me | NH₂ | Et | SO₂NHMe | |
| 4-59 | 2-SO₂Me-Ph | H | Me | NH₂ | i-Pr | SO₂NHMe | |
| 4-60 | 2-CN-Ph | H | Me | NH₂ | Et | SO₂NHMe | |

**[Table 4-3]**

| Compound No. | R¹ | R² | R³ | R⁴ | R⁵ | A | Physical Property value |
|---|---|---|---|---|---|---|---|
| 4-61 | 2-CN-Ph | H | Me | NH₂ | i-Pr | SO₂NHMe | |
| 4-62 | 2-OCHF₂-Ph | H | Me | NH₂ | Me | SO₂NHMe | |
| 4-63 | 2-OCHF₂-Ph | H | Me | NH₂ | Et | SO₂NHMe | |
| 4-64 | 2-OCHF₂-Ph | H | Me | NH₂ | i-Pr | SO₂NHMe | |
| 4-65 | 2-c-Pr-Ph | H | Me | NH₂ | Me | SO₂NHMe | |
| 4-66 | 2-c-Pr-Ph | H | Me | NH₂ | Et | SO₂NHMe | |
| 4-67 | 2-c-Pr-Ph | H | Me | NH₂ | i-Pr | SO₂NHMe | |
| 4-68 | 2-CHF₂-Ph | H | Me | NH₂ | Me | SO₂NHMe | |
| 4-69 | 2-CHF₂-Ph | H | Me | NH₂ | Et | SO₂NHMe | |
| 4-70 | 2-CHF₂-Ph | H | Me | NH₂ | i-Pr | SO₂NHMe | |
| 4-71 | 2-NHAc-Ph | H | Me | NH₂ | Me | SO₂NHMe | |
| 4-72 | 2-NHAc-Ph | H | Me | NH₂ | Et | SO₂NHMe | |
| 4-73 | 2-NHAc-Ph | H | Me | NH₂ | i-Pr | SO₂NHMe | |
| 4-74 | 2-P(OMe)₂-Ph | H | Me | NH₂ | Me | SO₂NHMe | |
| 4-75 | 2-P(OMe)₂-Ph | H | Me | NH₂ | Et | SO₂NHMe | |
| 4-76 | 2-P(OMe)₂-Ph | H | Me | NH₂ | i-Pr | SO₂NHMe | |
| 4-77 | pyridin-2-yl | H | Me | NH₂ | Et | SO₂NHMe | NMR |
| 4-78 | 3-Cl-pyridin-2-yl | H | Me | NH₂ | Me | SO₂NHMe | 153-154 |
| 4-79 | 3-Cl-pyridin-2-yl | H | Me | NH₂ | Et | SO₂NHMe | 136-137 |
| 4-80 | c-Hex | H | Me | NH₂ | Me | SO₂NHMe | |
| 4-81 | c-Hex | H | Me | NH₂ | Et | SO₂NHMe | |
| 4-82 | c-Hex | H | Me | NH₂ | i-Pr | SO₂NHMe | |
| 4-83 | c-Pen | H | Me | NH₂ | Me | SO₂NHMe | |
| 4-84 | c-Pen | H | Me | NH₂ | Et | SO₂NHMe | |
| 4-85 | c-Pen | H | Me | NH₂ | i-Pr | SO₂NHMe | |
| 4-86 | c-Bu | H | Me | NH₂ | Me | SO₂NHMe | |
| 4-87 | c-Bu | H | Me | NH₂ | Et | SO₂NHMe | |
| 4-88 | c-Bu | H | Me | NH₂ | i-Pr | SO₂NHMe | |
| 4-89 | c-Pr | H | Me | NH₂ | Me | SO₂NHMe | |
| 4-90 | c-Pr | H | Me | NH₂ | Et | SO₂NHMe | |

**[Table 4-4]**

| Compound No. | R¹ | R² | R³ | R⁴ | R⁵ | A | Physical Property value |
|---|---|---|---|---|---|---|---|
| 4-91 | c-Pr | H | Me | NH₂ | i-Pr | SO₂NHMe | |
| 4-92 | 3,4-dihydro-2H-pyran-6-yl | H | Me | NH₂ | Me | SO₂NHMe | |
| 4-93 | 3,4-dihydro-2H-pyran-6-yl | H | Me | NH₂ | Et | SO₂NHMe | |
| 4-94 | 3,4-dihydro-2H-pyran-6-yl | H | Me | NH₂ | i-Pr | SO₂NHMe | |
| 4-95 | tetrahydropyran-2-yl | H | Me | NH₂ | Me | SO₂NHMe | |
| 4-96 | tetrahydropyran-2-yl | H | Me | NH₂ | Et | SO₂NHMe | |
| 4-97 | tetrahydropyran-2-yl | H | Me | NH₂ | i-Pr | SO₂NHMe | |
| 4-98 | 1,3-dioxan-2-yl | H | Me | NH₂ | Me | SO₂NHMe | |
| 4-99 | 1,3-dioxan-2-yl | H | Me | NH₂ | Et | SO₂NHMe | |
| 4-100 | 1,3-dioxan-2-yl | H | Me | NH₂ | i-Pr | SO₂NHMe | |
| 4-101 | tetrahydropyran-3-yl | H | Me | NH₂ | Me | SO₂NHMe | |
| 4-102 | tetrahydropyran-3-yl | H | Me | NH₂ | Et | SO₂NHMe | |
| 4-103 | tetrahydropyran-3-yl | H | Me | NH₂ | i-Pr | SO₂NHMe | |
| 4-104 | tetrahydropyran-4-yl | H | Me | NH₂ | Me | SO₂NHMe | |
| 4-105 | tetrahydropyran-4-yl | H | Me | NH₂ | Et | SO₂NHMe | |
| 4-106 | tetrahydropyran-4-yl | H | Me | NH₂ | i-Pr | SO₂NHMe | |
| 4-107 | tetrahydrothiopyran-4-yl | H | Me | NH₂ | Me | SO₂NHMe | |
| 4-108 | tetrahydrothiopyran-4-yl | H | Me | NH₂ | Et | SO₂NHMe | |
| 4-109 | tetrahydrothiopyran-4-yl | H | Me | NH₂ | i-Pr | SO₂NHMe | |
| 4-110 | 1-Ac-piperidin-4-yl | H | Me | NH₂ | Me | SO₂NHMe | |
| 4-111 | 1-Ac-piperidin-4-yl | H | Me | NH₂ | Et | SO₂NHMe | |
| 4-112 | 1-Ac-piperidin-4-yl | H | Me | NH₂ | i-Pr | SO₂NHMe | |
| 4-113 | piperidin-1-yl | H | Me | NH₂ | Me | SO₂NHMe | |
| 4-114 | piperidin-1-yl | H | Me | NH₂ | Et | SO₂NHMe | |
| 4-115 | piperidin-1-yl | H | Me | NH₂ | i-Pr | SO₂NHMe | NMR |
| 4-116 | 1,3-dioxolan-2-yl | H | Me | NH₂ | Me | SO₂NHMe | |
| 4-117 | 1,3-dioxolan-2-yl | H | Me | NH₂ | Et | SO₂NHMe | |
| 4-118 | 1,3-dioxolan-2-yl | H | Me | NH₂ | i-Pr | SO₂NHMe | |
| 4-119 | 1,3-dioxepan-2-yl | H | Me | NH₂ | Me | SO₂NHMe | |
| 4-120 | 1,3-dioxepan-2-yl | H | Me | NH₂ | Et | SO₂NHMe | |

**[Table 4-5]**

| Compound No. | R¹ | R² | R³ | R⁴ | R⁵ | A | Physical Property value |
|---|---|---|---|---|---|---|---|
| 4-121 | 1,3-dioxepan-2-yl | H | Me | NH₂ | i-Pr | SO₂NHMe | |
| 4-122 | thiazol-5-yl | H | Me | NH₂ | Me | SO₂NHMe | |
| 4-123 | thiazol-5-yl | H | Me | NH₂ | Et | SO₂NHMe | |
| 4-124 | thiazol-5-yl | H | Me | NH₂ | i-Pr | SO₂NHMe | |
| 4-125 | thiazol-4-yl | H | Me | NH₂ | Me | SO₂NHMe | |
| 4-126 | thiazol-4-yl | H | Me | NH₂ | Et | SO₂NHMe | |
| 4-127 | thiazol-4-yl | H | Me | NH₂ | i-Pr | SO₂NHMe | |
| 4-128 | 1,2,3-thiadiazol-5-yl | H | Me | NH₂ | Me | SO₂NHMe | |
| 4-129 | 1,2,3-thiadiazol-5-yl | H | Me | NH₂ | Et | SO₂NHMe | |
| 4-130 | 1,2,3-thiadiazol-5-yl | H | Me | NH₂ | i-Pr | SO₂NHMe | |
| 4-131 | thiophen-2-yl | H | Me | NH₂ | Me | SO₂NHMe | 194-195 |
| 4-132 | thiophen-2-yl | H | Me | NH₂ | Et | SO₂NHMe | 179-180 |
| 4-133 | thiophen-2-yl | H | Me | NH₂ | i-Pr | SO₂NHMe | |
| 4-134 | thiophen-3-yl | H | Me | NH₂ | Me | SO₂NHMe | 194-195 |
| 4-135 | thiophen-3-yl | H | Me | NH₂ | Et | SO₂NHMe | NMR |
| 4-136 | thiophen-3-yl | H | Me | NH₂ | i-Pr | SO₂NHMe | |
| 4-137 | pyridazin-3-yl | H | Me | NH₂ | Me | SO₂NHMe | |
| 4-138 | pyridazin-3-yl | H | Me | NH₂ | Et | SO₂NHMe | |
| 4-139 | pyridazin-3-yl | H | Me | NH₂ | i-Pr | SO₂NHMe | |
| 4-140 | pyrimidin-2-yl | H | Me | NH₂ | Me | SO₂NHMe | |
| 4-141 | pyrimidin-2-yl | H | Me | NH₂ | Et | SO₂NHMe | |
| 4-142 | pyrimidin-2-yl | H | Me | NH₂ | i-Pr | SO₂NHMe | |
| 4-143 | pyrazin-2-yl | H | Me | NH₂ | Me | SO₂NHMe | |
| 4-144 | pyrazin-2-yl | H | Me | NH₂ | Et | SO₂NHMe | |
| 4-145 | pyrazin-2-yl | H | Me | NH₂ | i-Pr | SO₂NHMe | |

**[Table 4-6]**

| Compound No. | R¹ | R² | R³ | R⁴ | R⁵ | A | Physical Property value |
|---|---|---|---|---|---|---|---|
| 4-146 | thiazol-2-yl | H | Me | NH₂ | Me | SO₂NHMe | |
| 4-147 | thiazol-2-yl | H | Me | NH₂ | Et | SO₂NHMe | |
| 4-148 | thiazol-2-yl | H | Me | NH₂ | i-Pr | SO₂NHMe | |
| 4-149 | Ac | H | Me | NH₂ | Et | SO₂NHMe | NMR |
| 4-150 | CO₂Et | H | Me | NH₂ | Et | SO₂Me | NMR |
| 4-151 | I | H | Me | NH₂ | i-Pr | SO₂NHMe | NMR |
| 4-152 | I | H | Me | NH₂ | Me | SO₂NHMe | NMR |
| 4-153 | CO₂Et | H | Me | NH₂ | i-Pr | SO₂NHMe | NMR |
| 4-154 | CO₂H | H | Me | NH₂ | i-Pr | SO₂NHMe | 73-74 |
| 4-155 | 4-NMe₂-Ph | H | Me | NH₂ | Et | SO₂NHMe | NMR |
| 4-156 | 1,3-benzodioxol-5-yl | H | Me | NH₂ | Et | SO₂NHMe | NMR |
| 4-157 | 2-F-4-Cl-Ph | H | Me | NH₂ | Et | SO₂NHMe | NMR |
| 4-158 | 2-fluoro-pyridin-3-yl | H | Me | NH₂ | Et | SO₂NHMe | NMR |
| 4-159 | 3-CN-Ph | H | Me | NH₂ | Et | SO₂NHMe | NMR |
| 4-160 | 6-Cl-pyridin-3-yl | H | Me | NH₂ | Et | SO₂NHMe | NMR |
| 4-161 | quinolin-3-yl | H | Me | NH₂ | Et | SO₂NHMe | NMR |
| 4-162 | benzothiophen-2-yl | H | Me | NH₂ | Et | SO₂NHMe | NMR |
| 4-163 | 4-CO₂Me-Ph | H | Me | NH₂ | Et | SO₂NHMe | NMR |
| 4-164 | 2,2-difluoro-1,3-benzodioxol-4-yl | H | Me | NH₂ | Et | SO₂NHMe | NMR |
| 4-165 | Me | Me | CO₂Et | NH₂ | Et | SO₂NHMe | NMR |
| 4-166 | OMe | H | CO₂Et | NH₂ | Et | SO₂NHMe | NMR |
| 4-167 | 4-CN-Ph | Et | Me | NH₂ | Et | SO₂NHMe | NMR |
| 4-168 | 2-oxopyrrolidin-1-yl | H | Me | NH₂ | Et | SO₂NHMe | NMR |
| 4-169 | 2-oxopyrrolidin-1-yl | Cl | Me | NH₂ | Et | SO₂NHMe | NMR |
| 4-170 | 4-Cl-Ph | Et | Me | NH₂ | Et | SO₂NHMe | NMR |
| 4-171 | Me | I | Me | NH₂ | Et | SO₂NHMe | NMR |
| 4-172 | 4-Cl-Ph | Cl | Me | NH₂ | Et | SO₂NHMe | NMR |
| 4-173 | 4-Cl-Ph | (-CH₂)₄ | | NH₂ | Et | SO₂NHMe | NMR |
| 4-174 | 4-Cl-Ph | i-Pr | Me | NH₂ | Et | SO₂NHMe | NMR |
| 4-175 | 4-c-Hex-Ph | H | Me | NH₂ | Et | SO₂NHMe | NMR |

**[Table 4-7]**

| Compound No. | R¹ | R² | R³ | R⁴ | R⁵ | A | Physical Property value |
|---|---|---|---|---|---|---|---|
| 4-176 | 4-Cl-Ph | H | CF₃ | NH₂ | Et | SO₂NHMe | NMR |
| 4-177 | 4-CN-Ph | Br | Me | NH₂ | Et | SO₂NHMe | NMR |
| 4-178 | 4-Cl-Ph | Br | Me | NH₂ | Et | SO₂NHMe | NMR |
| 4-179 | 4-SMe-Ph | H | Me | NH₂ | Et | SO₂NHMe | NMR |
| 4-180 | 2-Me-1,3-dioxolan-2-yl | H | Me | NH₂ | Et | SO₂NHMe | NMR |
| 4-181 | 2-Me-1,3-dioxan-2-yl | H | Me | NH₂ | Et | SO₂NHMe | NMR |
| 4-182 | 3-Me-thiophen-2-yl | H | Me | NH₂ | Et | SO₂NHMe | NMR |
| 4-183 | 2-Me-pyrazol-3-yl | H | Me | NH₂ | Et | SO₂NHMe | NMR |
| 4-184 | 4-CF₃-Ph | H | Me | NH₂ | Et | SO₂NHMe | NMR |
| 4-185 | 2-OMe-Ph | H | Me | NH₂ | Et | SO₂NHMe | NMR |
| 4-186 | 3,5-OMe₂-Ph | H | Me | NH₂ | Et | SO₂NHMe | NMR |
| 4-187 | 3-OMe-Ph | H | Me | NH₂ | Et | SO₂NHMe | NMR |
| 4-188 | 2,4-F₂-Ph | H | Me | NH₂ | Et | SO₂NHMe | NMR |
| 4-189 | Me | Br | Me | NH₂ | Et | SO₂NHMe | 86-87 |
| 4-190 | pyridin-3-yl | H | Me | NH₂ | Et | SO₂NHMe | 36-41 |
| 4-191 | CO₂H | H | Me | NH₂ | Et | SO₂Me | 202-203 |
| 4-192 | thiazol-5-yl | H | Et | NH₂ | Me | SO₂NHMe | |
| 4-193 | thiazol-5-yl | H | Et | NH₂ | Et | SO₂NHMe | |
| 4-194 | thiazol-5-yl | H | Et | NH₂ | i-Pr | SO₂NHMe | |
| 4-195 | thiazol-4-yl | H | Et | NH₂ | Me | SO₂NHMe | |
| 4-196 | thiazol-4-yl | H | Et | NH₂ | Et | SO₂NHMe | |
| 4-197 | thiazol-4-yl | H | Et | NH₂ | i-Pr | SO₂NHMe | |
| 4-198 | thiazol-2-yl | H | Et | NH₂ | Me | SO₂NHMe | |
| 4-199 | thiazol-2-yl | H | Et | NH₂ | Et | SO₂NHMe | |
| 4-200 | thiazol-2-yl | H | Et | NH₂ | i-Pr | SO₂NHMe | |
| 4-201 | thiophen-2-yl | H | Et | NH₂ | Me | SO₂NHMe | |
| 4-202 | thiophen-2-yl | H | Et | NH₂ | Et | SO₂NHMe | |
| 4-203 | thiophen-2-yl | H | Et | NH₂ | i-Pr | SO₂NHMe | |
| 4-204 | thiophen-3-yl | H | Et | NH₂ | Me | SO₂NHMe | |
| 4-205 | thiophen-3-yl | H | Et | NH₂ | Et | SO₂NHMe | |

**[Table 4-8]**

| Compound No. | R¹ | R² | R³ | R⁴ | R⁵ | A | Physical Property value |
|---|---|---|---|---|---|---|---|
| 4-206 | thiophen-3-yl | H | Et | NH₂ | i-Pr | SO₂NHMe | |
| 4-207 | pyridazin-3-yl | H | Et | NH₂ | Me | SO₂NHMe | |
| 4-208 | pyridazin-3-yl | H | Et | NH₂ | Et | SO₂NHMe | |
| 4-209 | pyridazin-3-yl | H | Et | NH₂ | i-Pr | SO₂NHMe | |
| 4-210 | pyrazin-2-yl | H | Et | NH₂ | Me | SO₂NHMe | |
| 4-211 | pyrazin-2-yl | H | Et | NH₂ | Et | SO₂NHMe | |
| 4-212 | pyrazin-2-yl | H | Et | NH₂ | i-Pr | SO₂NHMe | |
| 4-213 | pyrimidin-2-yl | H | Et | NH₂ | Me | SO₂NHMe | |
| 4-214 | pyrimidin-2-yl | H | Et | NH₂ | Et | SO₂NHMe | |
| 4-215 | pyrimidin-2-yl | H | Et | NH₂ | i-Pr | SO₂NHMe | |
| 4-216 | pyrimidin-4-yl | H | Et | NH₂ | Me | SO₂NHMe | |
| 4-217 | pyrimidin-4-yl | H | Et | NH₂ | Et | SO₂NHMe | |
| 4-218 | pyrimidin-4-yl | H | Et | NH₂ | i-Pr | SO₂NHMe | |
| 4-219 | pyrimidin-5-yl | H | Et | NH₂ | Me | SO₂NHMe | |
| 4-220 | pyrimidin-5-yl | H | Et | NH₂ | Et | SO₂NHMe | |
| 4-221 | pyrimidin-5-yl | H | Et | NH₂ | i-Pr | SO₂NHMe | |
| 4-222 | Ph | H | Et | NH₂ | Me | SO₂NHMe | |
| 4-223 | Ph | H | Et | NH₂ | Et | SO₂NHMe | |
| 4-224 | Ph | H | Et | NH₂ | i-Pr | SO₂NHMe | |
| 4-225 | 2-F-Ph | H | Et | NH₂ | Me | SO₂NHMe | |
| 4-226 | 2-F-Ph | H | Et | NH₂ | Et | SO₂NHMe | |
| 4-227 | 2-F-Ph | H | Et | NH₂ | i-Pr | SO₂NHMe | |
| 4-228 | 3-F-Ph | H | Et | NH₂ | Me | SO₂NHMe | |
| 4-229 | 3-F-Ph | H | Et | NH₂ | Et | SO₂NHMe | |
| 4-230 | 3-F-Ph | H | Et | NH₂ | i-Pr | SO₂NHMe | |
| 4-231 | 4-F-Ph | H | Et | NH₂ | Me | SO₂NHMe | |
| 4-232 | 4-F-Ph | H | Et | NH₂ | Et | SO₂NHMe | |
| 4-233 | 4-F-Ph | H | Et | NH₂ | i-Pr | SO₂NHMe | |
| 4-234 | 2,6-F₂-Ph | H | Et | NH₂ | Me | SO₂NHMe | |
| 4-235 | 2,6-F₂-Ph | H | Et | NH₂ | Et | SO₂NHMe | |

**[Table 4-9]**

| Compound No. | R¹ | R² | R³ | R⁴ | R⁵ | A | Physical Property value |
|---|---|---|---|---|---|---|---|
| 4-236 | 2,6-F₂-Ph | H | Et | NH₂ | i-Pr | SO₂NHMe | |
| 4-237 | vinyl | H | Me | NH₂ | Et | SO₂NHMe | NMR |
| 4-238 | Et | H | Me | NH₂ | Et | SO₂NHMe | NMR |
| 4-239 | 2-oxopyridin-1-yl | H | Me | NH₂ | Et | SO₂NHMe | NMR |
| 4-240 | furan-2-yl | H | Me | NH₂ | Et | SO₂NHMe | 118-119 |
| 4-241 | cyclopenten-1-yl | H | Me | NH₂ | Et | SO₂NHMe | 170-171 |
| 4-242 | (E)-prop-1-enyl | H | Me | NH₂ | Et | SO₂NHMe | 113-114 |
| 4-243 | pyrazol-1-yl | H | Me | NH₂ | Et | SO₂NHMe | NMR |
| 4-244 | 2-Cl-ethyl carbamate | H | Me | NH₂ | Et | SO₂NHMe | |
| 4-245 | 2-oxo-1,3-oxazolidin-3-yl | H | Me | NH₂ | Et | SO₂NHMe | |
| 4-246 | NH₂ | H | Me | NH₂ | Et | SO₂NHMe | 59-61 |
| 4-247 | benzhydrylideneamino | H | Me | NH₂ | Et | SO₂NHMe | NMR |
| 4-248 | Me | H | Me | NH₂ | Et | SO₂NHMe | 110-111 |
| 4-249 | Et | H | Et | NH₂ | Et | SO₂NHMe | NMR |
| 4-250 | n-Pr | H | Me | NH₂ | Et | SO₂NHMe | 97-98 |
| 4-251 | 4-OCF₃-Ph | H | Me | NH₂ | Et | SO₂NHMe | NMR |
| 4-252 | i-Pr | H | Me | NH₂ | Et | SO₂NHMe | |
| 4-253 | isopropenyl | H | Me | NH₂ | Et | SO₂NHMe | |
| 4-254 | Et | H | Me | NH₂ | Me | SO₂NHMe | 109-110 |
| 4-255 | vinyl | H | Me | NH₂ | Me | SO₂NHMe | |
| 4-256 | i-Pr | H | Me | NH₂ | Me | SO₂NHMe | |
| 4-257 | n-Pr | H | Me | NH₂ | Me | SO₂NHMe | |
| 4-258 | Ph | H | Me | NH₂ | Me | SO₂NHEt | |
| 4-259 | Ph | H | Me | NH₂ | Et | SO₂NHEt | |
| 4-260 | Ph | H | Me | NH₂ | i-Pr | SO₂NHEt | |
| 4-261 | 2-F-Ph | H | Me | NH₂ | Me | SO₂NHEt | |
| 4-262 | 2-F-Ph | H | Me | NH₂ | Et | SO₂NHEt | |
| 4-263 | 2-F-Ph | H | Me | NH₂ | i-Pr | SO₂NHEt | |
| 4-264 | 3-F-Ph | H | Me | NH₂ | Me | SO₂NHEt | |
| 4-265 | 3-F-Ph | H | Me | NH₂ | Et | SO₂NHEt | |

**[Table 4-10]**

| Compound No. | R¹ | R² | R³ | R⁴ | R⁵ | A | Physical Property value |
|---|---|---|---|---|---|---|---|
| 4-266 | 3-F-Ph | H | Me | NH₂ | i-Pr | SO₂NHEt | |
| 4-267 | 4-F-Ph | H | Me | NH₂ | Me | SO₂NHEt | |
| 4-268 | 4-F-Ph | H | Me | NH₂ | Et | SO₂NHEt | |
| 4-269 | 4-F-Ph | H | Me | NH₂ | i-Pr | SO₂NHEt | |
| 4-270 | 2,6-F₂-Ph | H | Me | NH₂ | Me | SO₂NHEt | |
| 4-271 | 2,6-F₂-Ph | H | Me | NH₂ | Et | SO₂NHEt | |
| 4-272 | 2,6-F₂-Ph | H | Me | NH₂ | i-Pr | SO₂NHEt | |
| 4-273 | thiazol-5-yl | H | Me | NH₂ | Me | SO₂NHEt | |
| 4-274 | thiazol-5-yl | H | Me | NH₂ | Et | SO₂NHEt | |
| 4-275 | thiazol-5-yl | H | Me | NH₂ | i-Pr | SO₂NHEt | |
| 4-276 | thiazol-4-yl | H | Me | NH₂ | Me | SO₂NHEt | |
| 4-277 | thiazol-4-yl | H | Me | NH₂ | Et | SO₂NHEt | |
| 4-278 | thiazol-4-yl | H | Me | NH₂ | i-Pr | SO₂NHEt | |
| 4-279 | thiazol-2-yl | H | Me | NH₂ | Me | SO₂NHEt | |
| 4-280 | thiazol-2-yl | H | Me | NH₂ | Et | SO₂NHEt | |
| 4-281 | thiazol-2-yl | H | Me | NH₂ | i-Pr | SO₂NHEt | |
| 4-282 | thiophen-2-yl | H | Me | NH₂ | Me | SO₂NHEt | 211-212 |
| 4-283 | thiophen-2-yl | H | Me | NH₂ | Et | SO₂NHEt | 185-186 |
| 4-284 | thiophen-2-yl | H | Me | NH₂ | i-Pr | SO₂NHEt | |
| 4-285 | thiophen-3-yl | H | Me | NH₂ | Me | SO₂NHEt | |
| 4-286 | thiophen-3-yl | H | Me | NH₂ | Et | SO₂NHEt | |
| 4-287 | thiophen-3-yl | H | Me | NH₂ | i-Pr | SO₂NHEt | |
| 4-288 | pyridazin-3-yl | H | Me | NH₂ | Me | SO₂NHEt | |
| 4-289 | pyridazin-3-yl | H | Me | NH₂ | Et | SO₂NHEt | |
| 4-290 | pyridazin-3-yl | H | Me | NH₂ | i-Pr | SO₂NHEt | |
| 4-291 | pyrazin-2-yl | H | Me | NH₂ | Me | SO₂NHEt | |
| 4-292 | pyrazin-2-yl | H | Me | NH₂ | Et | SO₂NHEt | |
| 4-293 | pyrazin-2-yl | H | Me | NH₂ | i-Pr | SO₂NHEt | |
| 4-294 | pyrimidin-2-yl | H | Me | NH₂ | Me | SO₂NHEt | |
| 4-295 | pyrimidin-2-yl | H | Me | NH₂ | Et | SO₂NHEt | |

**[Table 4-11]**

| Compound No. | R¹ | R² | R³ | R⁴ | R⁵ | A | Physical Property value |
|---|---|---|---|---|---|---|---|
| 4-296 | pyrimidin-2-yl | H | Me | NH₂ | i-Pr | SO₂NHEt | |
| 4-297 | pyrimidin-4-yl | H | Me | NH₂ | Me | SO₂NHEt | |
| 4-298 | pyrimidin-4-yl | H | Me | NH₂ | Et | SO₂NHEt | |
| 4-299 | pyrimidin-4-yl | H | Me | NH₂ | i-Pr | SO₂NHEt | |
| 4-300 | pyrimidin-5-yl | H | Me | NH₂ | Me | SO₂NHEt | |
| 4-301 | pyrimidin-5-yl | H | Me | NH₂ | Et | SO₂NHEt | |
| 4-302 | pyrimidin-5-yl | H | Me | NH₂ | i-Pr | SO₂NHEt | |
| 4-303 | Et | H | Et | NH₂ | Me | SO₂NHMe | NMR |
| 4-304 | OEt | H | Me | NH₂ | Et | SO₂NHMe | NMR |
| 4-305 | OCH₂CH₂OMe | H | Me | NH₂ | Et | SO₂NHMe | NMR |
| 4-306 | (tetrahydro-3-furanyl)methoxy | H | Me | NH₂ | Et | SO₂NHMe | NMR |
| 4-307 | (tetrahydro-2-furanyl)methoxy | H | Me | NH₂ | Et | SO₂NHMe | NMR |
| 4-308 | OCH₂c-Pr | H | Me | NH₂ | Et | SO₂NHMe | NMR |
| 4-309 | OCH₂CHF₂ | H | Me | NH₂ | Et | SO₂NHMe | NMR |
| 4-310 | 4-NO₂-PhO | H | Me | NH₂ | Et | SO₂NHMe | NMR |

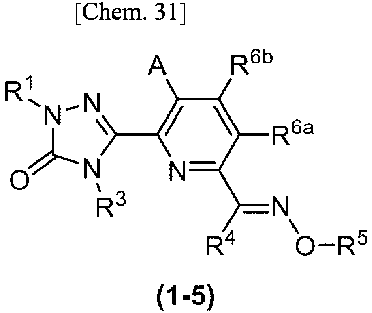

Table 5 (In Table 5, R^{6a} and R^{6b} represent hydrogen atoms.)

**[Table 5-1]**

| Compound No. | R¹ | R³ | R⁴ | R⁵ | A | Physical Property value |
|---|---|---|---|---|---|---|
| 5-1 | Ph | Me | NH₂ | Et | CONMe₂ | NMR |
| 5-2 | Ph | Me | NH₂ | Et | CO₂H | 227-228 |
| 5-3 | Ph | Me | NH₂ | Et | CO₂Et | 1.4692(20.1) |
| 5-4 | Ph | Me | NH₂ | Me | SO₂NHMe | |
| 5-5 | Ph | Me | NH₂ | Et | SO₂NHMe | |
| 5-6 | Ph | Me | NH₂ | i-Pr | SO₂NHMe | |
| 5-7 | 2-F-Ph | Me | NH₂ | Me | SO₂NHMe | |
| 5-8 | 2-F-Ph | Me | NH₂ | Et | SO₂NHMe | |
| 5-9 | 2-F-Ph | Me | NH₂ | i-Pr | SO₂NHMe | |
| 5-10 | 3-F-Ph | Me | NH₂ | Me | SO₂NHMe | |
| 5-11 | 3-F-Ph | Me | NH₂ | Et | SO₂NHMe | |
| 5-12 | 3-F-Ph | Me | NH₂ | i-Pr | SO₂NHMe | |
| 5-13 | 4-F-Ph | Me | NH₂ | Me | SO₂NHMe | |
| 5-14 | 4-F-Ph | Me | NH₂ | Et | SO₂NHMe | |
| 5-15 | 4-F-Ph | Me | NH₂ | i-Pr | SO₂NHMe | |
| 5-16 | 2,6-F₂-Ph | Me | NH₂ | Me | SO₂NHMe | |
| 5-17 | 2,6-F₂-Ph | Me | NH₂ | Et | SO₂NHMe | |
| 5-18 | 2,6-F₂-Ph | Me | NH₂ | i-Pr | SO₂NHMe | |
| 5-19 | 2-NO₂-Ph | Me | NH₂ | Et | SO₂NHMe | NMR |
| 5-20 | 2-NH₂-Ph | Me | NH₂ | Et | SO₂NHMe | 135-136 |
| 5-21 | 2-Cl-Ph | Me | NH₂ | Et | SO₂NHMe | 160-161 |

**[Table 5-2]**

| Compound No. | R¹ | R³ | R⁴ | R⁵ | A | Physical Property value |
|---|---|---|---|---|---|---|
| 5-22 | thiazol-5-yl | Me | NH₂ | Me | SO₂NHMe | |
| 5-23 | thiazol-5-yl | Me | NH₂ | Et | SO₂NHMe | |
| 5-24 | thiazol-5-yl | Me | NH₂ | i-Pr | SO₂NHMe | |
| 5-25 | thiazol-4-yl | Me | NH₂ | Me | SO₂NHMe | |
| 5-26 | thiazol-4-yl | Me | NH₂ | Et | SO₂NHMe | |
| 5-27 | thiazol-4-yl | Me | NH₂ | i-Pr | SO₂NHMe | |
| 5-28 | thiazol-2-yl | Me | NH₂ | Me | SO₂NHMe | |
| 5-29 | thiazol-2-yl | Me | NH₂ | Et | SO₂NHMe | |
| 5-30 | thiazol-2-yl | Me | NH₂ | i-Pr | SO₂NHMe | |
| 5-31 | thiophen-2-yl | Me | NH₂ | Me | SO₂NHMe | |
| 5-32 | thiophen-2-yl | Me | NH₂ | Et | SO₂NHMe | |
| 5-33 | thiophen-2-yl | Me | NH₂ | i-Pr | SO₂NHMe | |
| 5-34 | thiophen-3-yl | Me | NH₂ | Me | SO₂NHMe | |
| 5-35 | thiophen-3-yl | Me | NH₂ | Et | SO₂NHMe | |
| 5-36 | thiophen-3-yl | Me | NH₂ | i-Pr | SO₂NHMe | |
| 5-37 | pyridazin-3-yl | Me | NH₂ | Me | SO₂NHMe | |
| 5-38 | pyridazin-3-yl | Me | NH₂ | Et | SO₂NHMe | |
| 5-39 | pyridazin-3-yl | Me | NH₂ | i-Pr | SO₂NHMe | |
| 5-40 | pyrazin-2-yl | Me | NH₂ | Me | SO₂NHMe | |
| 5-41 | pyrazin-2-yl | Me | NH₂ | Et | SO₂NHMe | |
| 5-42 | pyrazin-2-yl | Me | NH₂ | i-Pr | SO₂NHMe | |
| 5-43 | pyrimidin-2-yl | Me | NH₂ | Me | SO₂NHMe | |
| 5-44 | pyrimidin-2-yl | Me | NH₂ | Et | SO₂NHMe | |
| 5-45 | pyrimidin-2-yl | Me | NH₂ | i-Pr | SO₂NHMe | |
| 5-46 | pyrimidin-4-yl | Me | NH₂ | Me | SO₂NHMe | |
| 5-47 | pyrimidin-4-yl | Me | NH₂ | Et | SO₂NHMe | |
| 5-48 | pyrimidin-4-yl | Me | NH₂ | i-Pr | SO₂NHMe | |
| 5-49 | pyrimidin-5-yl | Me | NH₂ | Me | SO₂NHMe | |
| 5-50 | pyrimidin-5-yl | Me | NH₂ | Et | SO₂NHMe | |
| 5-51 | pyrimidin-5-yl | Me | NH₂ | i-Pr | SO₂NHMe | |

**[Table 5-3]**

| Compound No. | R¹ | R³ | R⁴ | R⁵ | A | Physical Property value |
|---|---|---|---|---|---|---|
| 5-52 | thiazol-5-yl | Et | NH₂ | Me | SO₂NHMe | |
| 5-53 | thiazol-5-yl | Et | NH₂ | Et | SO₂NHMe | |
| 5-54 | thiazol-5-yl | Et | NH₂ | i-Pr | SO₂NHMe | |
| 5-55 | thiazol-4-yl | Et | NH₂ | Me | SO₂NHMe | |
| 5-56 | thiazol-4-yl | Et | NH₂ | Et | SO₂NHMe | |
| 5-57 | thiazol-4-yl | Et | NH₂ | i-Pr | SO₂NHMe | |
| 5-58 | thiazol-2-yl | Et | NH₂ | Me | SO₂NHMe | |
| 5-59 | thiazol-2-yl | Et | NH₂ | Et | SO₂NHMe | |
| 5-60 | thiazol-2-yl | Et | NH₂ | i-Pr | SO₂NHMe | |
| 5-61 | thiophen-2-yl | Et | NH₂ | Me | SO₂NHMe | |
| 5-62 | thiophen-2-yl | Et | NH₂ | Et | SO₂NHMe | |
| 5-63 | thiophen-2-yl | Et | NH₂ | i-Pr | SO₂NHMe | |
| 5-64 | thiophen-3-yl | Et | NH₂ | Me | SO₂NHMe | |
| 5-65 | thiophen-3-yl | Et | NH₂ | Et | SO₂NHMe | |
| 5-66 | thiophen-3-yl | Et | NH₂ | i-Pr | SO₂NHMe | |
| 5-67 | pyridazin-3-yl | Et | NH₂ | Me | SO₂NHMe | |
| 5-68 | pyridazin-3-yl | Et | NH₂ | Et | SO₂NHMe | |
| 5-69 | pyridazin-3-yl | Et | NH₂ | i-Pr | SO₂NHMe | |
| 5-70 | pyrazin-2-yl | Et | NH₂ | Me | SO₂NHMe | |
| 5-71 | pyrazin-2-yl | Et | NH₂ | Et | SO₂NHMe | |
| 5-72 | pyrazin-2-yl | Et | NH₂ | i-Pr | SO₂NHMe | |
| 5-73 | pyrimidin-2-yl | Et | NH₂ | Me | SO₂NHMe | |
| 5-74 | pyrimidin-2-yl | Et | NH₂ | Et | SO₂NHMe | |
| 5-75 | pyrimidin-2-yl | Et | NH₂ | i-Pr | SO₂NHMe | |
| 5-76 | pyrimidin-4-yl | Et | NH₂ | Me | SO₂NHMe | |
| 5-77 | pyrimidin-4-yl | Et | NH₂ | Et | SO₂NHMe | |
| 5-78 | pyrimidin-4-yl | Et | NH₂ | i-Pr | SO₂NHMe | |
| 5-79 | pyrimidin-5-yl | Et | NH₂ | Me | SO₂NHMe | |
| 5-80 | pyrimidin-5-yl | Et | NH₂ | Et | SO₂NHMe | |
| 5-81 | pyrimidin-5-yl | Et | NH₂ | i-Pr | SO₂NHMe | |

**[Table 5-4]**

| Compound No. | R¹ | R³ | R⁴ | R⁵ | A | Physical Property value |
|---|---|---|---|---|---|---|
| 5-82 | Ph | Et | NH₂ | Me | SO₂NHMe | |
| 5-83 | Ph | Et | NH₂ | Et | SO₂NHMe | |
| 5-84 | Ph | Et | NH₂ | i-Pr | SO₂NHMe | |
| 5-85 | 2-F-Ph | Et | NH₂ | Me | SO₂NHMe | |
| 5-86 | 2-F-Ph | Et | NH₂ | Et | SO₂NHMe | |
| 5-87 | 2-F-Ph | Et | NH₂ | i-Pr | SO₂NHMe | |
| 5-88 | 3-F-Ph | Et | NH₂ | Me | SO₂NHMe | |
| 5-89 | 3-F-Ph | Et | NH₂ | Et | SO₂NHMe | |
| 5-90 | 3-F-Ph | Et | NH₂ | i-Pr | SO₂NHMe | |
| 5-91 | 4-F-Ph | Et | NH₂ | Me | SO₂NHMe | |
| 5-92 | 4-F-Ph | Et | NH₂ | Et | SO₂NHMe | |
| 5-93 | 4-F-Ph | Et | NH₂ | i-Pr | SO₂NHMe | |
| 5-94 | 2,6-F₂-Ph | Et | NH₂ | Me | SO₂NHMe | |
| 5-95 | 2,6-F₂-Ph | Et | NH₂ | Et | SO₂NHMe | |
| 5-96 | 2,6-F₂-Ph | Et | NH₂ | i-Pr | SO₂NHMe | |
| 5-97 | 2,4-Cl₂-Ph | Me | NH₂ | Me | SO₂NHMe | |
| 5-98 | 2,4-Cl₂-Ph | Me | NH₂ | Et | SO₂NHMe | |
| 5-99 | 2,4-Cl₂-Ph | Me | NH₂ | i-Pr | SO₂NHMe | |
| 5-100 | 2,5-Cl₂-Ph | Me | NH₂ | Me | SO₂NHMe | |
| 5-101 | 2,5-Cl₂-Ph | Me | NH₂ | Et | SO₂NHMe | |
| 5-102 | 2,5-Cl₂-Ph | Me | NH₂ | i-Pr | SO₂NHMe | |
| 5-103 | 2,6-Cl₂-Ph | Me | NH₂ | Me | SO₂NHMe | |
| 5-104 | 2,6-Cl₂-Ph | Me | NH₂ | Et | SO₂NHMe | |
| 5-105 | 2,6-Cl₂-Ph | Me | NH₂ | i-Pr | SO₂NHMe | |
| 5-106 | c-Hex | Me | NH₂ | Et | SO₂NHMe | |
| 5-107 | n-Bu | Me | NH₂ | Et | SO₂NHMe | |
| 5-108 | t-Bu | Me | NH₂ | Et | SO₂NHMe | |
| 5-109 | Ph | Me | NH₂ | Me | SO₂NHEt | |
| 5-110 | Ph | Me | NH₂ | Et | SO₂NHEt | |
| 5-111 | Ph | Me | NH₂ | i-Pr | SO₂NHEt | |

**[Table 5-5]**

| Compound No. | R¹ | R³ | R⁴ | R⁵ | A | Physical Property value |
|---|---|---|---|---|---|---|
| 5-112 | 2-F-Ph | Me | NH₂ | Me | SO₂NHEt | |
| 5-113 | 2-F-Ph | Me | NH₂ | Et | SO₂NHEt | |
| 5-114 | 2-F-Ph | Me | NH₂ | i-Pr | SO₂NHEt | |
| 5-115 | 3-F-Ph | Me | NH₂ | Me | SO₂NHEt | |
| 5-116 | 3-F-Ph | Me | NH₂ | Et | SO₂NHEt | |
| 5-117 | 3-F-Ph | Me | NH₂ | i-Pr | SO₂NHEt | |
| 5-118 | 4-F-Ph | Me | NH₂ | Me | SO₂NHEt | |
| 5-119 | 4-F-Ph | Me | NH₂ | Et | SO₂NHEt | |
| 5-120 | 4-F-Ph | Me | NH₂ | i-Pr | SO₂NHEt | |
| 5-121 | 2,6-F₂-Ph | Me | NH₂ | Me | SO₂NHEt | |
| 5-122 | 2,6-F₂-Ph | Me | NH₂ | Et | SO₂NHEt | |
| 5-123 | 2,6-F₂-Ph | Me | NH₂ | i-Pr | SO₂NHEt | |
| 5-124 | thiazol-5-yl | Me | NH₂ | Me | SO₂NHEt | |
| 5-125 | thiazol-5-yl | Me | NH₂ | Et | SO₂NHEt | |
| 5-126 | thiazol-5-yl | Me | NH₂ | i-Pr | SO₂NHEt | |
| 5-127 | thiazol-4-yl | Me | NH₂ | Me | SO₂NHEt | |
| 5-128 | thiazol-4-yl | Me | NH₂ | Et | SO₂NHEt | |
| 5-129 | thiazol-4-yl | Me | NH₂ | i-Pr | SO₂NHEt | |
| 5-130 | thiazol-2-yl | Me | NH₂ | Me | SO₂NHEt | |
| 5-131 | thiazol-2-yl | Me | NH₂ | Et | SO₂NHEt | |
| 5-132 | thiazol-2-yl | Me | NH₂ | i-Pr | SO₂NHEt | |
| 5-133 | thiophen-2-yl | Me | NH₂ | Me | SO₂NHEt | |
| 5-134 | thiophen-2-yl | Me | NH₂ | Et | SO₂NHEt | |
| 5-135 | thiophen-2-yl | Me | NH₂ | i-Pr | SO₂NHEt | |
| 5-136 | thiophen-3-yl | Me | NH₂ | Me | SO₂NHEt | |
| 5-137 | thiophen-3-yl | Me | NH₂ | Et | SO₂NHEt | |
| 5-138 | thiophen-3-yl | Me | NH₂ | i-Pr | SO₂NHEt | |
| 5-139 | pyridazin-3-yl | Me | NH₂ | Me | SO₂NHEt | |
| 5-140 | pyridazin-3-yl | Me | NH₂ | Et | SO₂NHEt | |
| 5-141 | pyridazin-3-yl | Me | NH₂ | i-Pr | SO₂NHEt | |

**[Table 5-6]**

| Compound No. | R¹ | R³ | R⁴ | R⁵ | A | Physical Property value |
|---|---|---|---|---|---|---|
| 5-142 | pyrazin-2-yl | Me | NH₂ | Me | SO₂NHEt | |
| 5-143 | pyrazin-2-yl | Me | NH₂ | Et | SO₂NHEt | |
| 5-144 | pyrazin-2-yl | Me | NH₂ | i-Pr | SO₂NHEt | |
| 5-145 | pyrimidin-2-yl | Me | NH₂ | Me | SO₂NHEt | |
| 5-146 | pyrimidin-2-yl | Me | NH₂ | Et | SO₂NHEt | |
| 5-147 | pyrimidin-2-yl | Me | NH₂ | i-Pr | SO₂NHEt | |
| 5-148 | pyrimidin-4-yl | Me | NH₂ | Me | SO₂NHEt | |
| 5-149 | pyrimidin-4-yl | Me | NH₂ | Et | SO₂NHEt | |
| 5-150 | pyrimidin-4-yl | Me | NH₂ | i-Pr | SO₂NHEt | |
| 5-151 | pyrimidin-5-yl | Me | NH₂ | Me | SO₂NHEt | |
| 5-152 | pyrimidin-5-yl | Me | NH₂ | Et | SO₂NHEt | |
| 5-153 | pyrimidin-5-yl | Me | NH₂ | i-Pr | SO₂NHEt | |

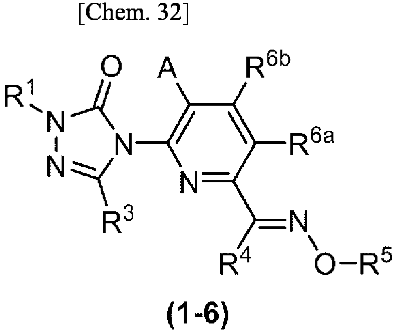

Table 6 (In Table 6, R^{6a} and R^{6b} represent hydrogen atoms.)

**[Table 6-1]**

| Compound No. | R¹ | R³ | R⁴ | R⁵ | A | Physical Property value |
|---|---|---|---|---|---|---|
| 6-1 | Ph | Me | Me | Et | SO₂Me | NMR |
| 6-2 | Ph | Me | NH₂ | Me | SO₂NHMe | |
| 6-3 | Ph | Me | NH₂ | Et | SO₂NHMe | |
| 6-4 | Ph | Me | NH₂ | i-Pr | SO₂NHMe | |
| 6-5 | 2-F-Ph | Me | NH₂ | Me | SO₂NHMe | |
| 6-6 | 2-F-Ph | Me | NH₂ | Et | SO₂NHMe | |
| 6-7 | 2-F-Ph | Me | NH₂ | i-Pr | SO₂NHMe | |
| 6-8 | 3-F-Ph | Me | NH₂ | Me | SO₂NHMe | |
| 6-9 | 3-F-Ph | Me | NH₂ | Et | SO₂NHMe | |
| 6-10 | 3-F-Ph | Me | NH₂ | i-Pr | SO₂NHMe | |
| 6-11 | 4-F-Ph | Me | NH₂ | Me | SO₂NHMe | |
| 6-12 | 4-F-Ph | Me | NH₂ | Et | SO₂NHMe | |
| 6-13 | 4-F-Ph | Me | NH₂ | i-Pr | SO₂NHMe | |
| 6-14 | 2,6-F₂-Ph | Me | NH₂ | Me | SO₂NHMe | |
| 6-15 | 2,6-F₂-Ph | Me | NH₂ | Et | SO₂NHMe | |
| 6-16 | 2,6-F₂-Ph | Me | NH₂ | i-Pr | SO₂NHMe | |

**[Table 6-2]**

| Compound No. | R¹ | R³ | R⁴ | R⁵ | A | Physical Property value |
|---|---|---|---|---|---|---|
| 6-17 | thiazol-5-yl | Me | NH₂ | Me | SO₂NHMe | |
| 6-18 | thiazol-5-yl | Me | NH₂ | Et | SO₂NHMe | |
| 6-19 | thiazol-5-yl | Me | NH₂ | i-Pr | SO₂NHMe | |
| 6-20 | thiazol-4-yl | Me | NH₂ | Me | SO₂NHMe | |
| 6-21 | thiazol-4-vl | Me | NH₂ | Et | SO₂NHMe | |
| 6-22 | thiazol-4-yl | Me | NH₂ | i-Pr | SO₂NHMe | |
| 6-23 | thiazol-2-yl | Me | NH₂ | Me | SO₂NHMe | |
| 6-24 | thiazol-2-yl | Me | NH₂ | Et | SO₂NHMe | |
| 6-25 | thiazol-2-yl | Me | NH₂ | i-Pr | SO₂NHMe | |
| 6-26 | thiophen-2-yl | Me | NH₂ | Me | SO₂NHMe | |
| 6-27 | thiophen-2-yl | Me | NH₂ | Et | SO₂NHMe | |
| 6-28 | thiophen-2-yl | Me | NH₂ | i-Pr | SO₂NHMe | |
| 6-29 | thiophen-3-yl | Me | NH₂ | Me | SO₂NHMe | |
| 6-30 | thiophen-3-yl | Me | NH₂ | Et | SO₂NHMe | |
| 6-31 | thiophen-3-yl | Me | NH₂ | i-Pr | SO₂NHMe | |
| 6-32 | pyridazin-3-yl | Me | NH₂ | Me | SO₂NHMe | |
| 6-33 | pyridazin-3-yl | Me | NH₂ | Et | SO₂NHMe | |
| 6-34 | pyridazin-3-yl | Me | NH₂ | i-Pr | SO₂NHMe | |
| 6-35 | pyrazin-2-yl | Me | NH₂ | Me | SO₂NHMe | |
| 6-36 | pyrazin-2-yl | Me | NH₂ | Et | SO₂NHMe | |
| 6-37 | pyrazin-2-yl | Me | NH₂ | i-Pr | SO₂NHMe | |
| 6-38 | pyrimidin-2-yl | Me | NH₂ | Me | SO₂NHMe | |
| 6-39 | pyrimidin-2-yl | Me | NH₂ | Et | SO₂NHMe | |
| 6-40 | pyrimidin-2-yl | Me | NH₂ | i-Pr | SO₂NHMe | |
| 6-41 | pyrimidin-4-yl | Me | NH₂ | Me | SO₂NHMe | |
| 6-42 | pyrimidin-4-yl | Me | NH₂ | Et | SO₂NHMe | |
| 6-43 | pyrimidin-4-yl | Me | NH₂ | i-Pr | SO₂NHMe | |
| 6-44 | pyrimidin-5-yl | Me | NH₂ | Me | SO₂NHMe | |
| 6-45 | pyrimidin-5-yl | Me | NH₂ | Et | SO₂NHMe | |
| 6-46 | pyrimidin-5-yl | Me | NH₂ | i-Pr | SO₂NHMe | |

**[Table 6-3]**

| Compound No. | R¹ | R³ | R⁴ | R⁵ | A | Physical Property value |
|---|---|---|---|---|---|---|
| 6-47 | thiazol-5-yl | Et | NH₂ | Me | SO₂NHMe | |
| 6-48 | thiazol-5-yl | Et | NH₂ | Et | SO₂NHMe | |
| 6-49 | thiazol-5-yl | Et | NH₂ | i-Pr | SO₂NHMe | |
| 6-50 | thiazol-4-yl | Et | NH₂ | Me | SO₂NHMe | |
| 6-51 | thiazol-4-yl | Et | NH₂ | Et | SO₂NHMe | |
| 6-52 | thiazol-4-yl | Et | NH₂ | i-Pr | SO₂NHMe | |
| 6-53 | thiazol-2-yl | Et | NH₂ | Me | SO₂NHMe | |
| 6-54 | thiazol-2-yl | Et | NH₂ | Et | SO₂NHMe | |
| 6-55 | thiazol-2-yl | Et | NH₂ | i-Pr | SO₂NHMe | |
| 6-56 | thiophen-2-yl | Et | NH₂ | Me | SO₂NHMe | |
| 6-57 | thiophen-2-yl | Et | NH₂ | Et | SO₂NHMe | |
| 6-58 | thiophen-2-yl | Et | NH₂ | i-Pr | SO₂NHMe | |
| 6-59 | thiophen-3-yl | Et | NH₂ | Me | SO₂NHMe | |
| 6-60 | thiophen-3-yl | Et | NH₂ | Et | SO₂NHMe | |
| 6-61 | thiophen-3-yl | Et | NH₂ | i-Pr | SO₂NHMe | |
| 6-62 | pyridazin-3-yl | Et | NH₂ | Me | SO₂NHMe | |
| 6-63 | pyridazin-3-yl | Et | NH₂ | Et | SO₂NHMe | |
| 6-64 | pyridazin-3-yl | Et | NH₂ | i-Pr | SO₂NHMe | |
| 6-65 | pyrazin-2-yl | Et | NH₂ | Me | SO₂NHMe | |
| 6-66 | pyrazin-2-yl | Et | NH₂ | Et | SO₂NHMe | |
| 6-67 | pyrazin-2-yl | Et | NH₂ | i-Pr | SO₂NHMe | |
| 6-68 | pyrimidin-2-yl | Et | NH₂ | Me | SO₂NHMe | |
| 6-69 | pyrimidin-2-yl | Et | NH₂ | Et | SO₂NHMe | |
| 6-70 | pyrimidin-2-yl | Et | NH₂ | i-Pr | SO₂NHMe | |
| 6-71 | pyrimidin-4-yl | Et | NH₂ | Me | SO₂NHMe | |
| 6-72 | pyrimidin-4-yl | Et | NH₂ | Et | SO₂NHMe | |
| 6-73 | pyrimidin-4-yl | Et | NH₂ | i-Pr | SO₂NHMe | |
| 6-74 | pyrimidin-5-yl | Et | NH₂ | Me | SO₂NHMe | |
| 6-75 | pyrimidin-5-yl | Et | NH₂ | Et | SO₂NHMe | |
| 6-76 | pyrimidin-5-yl | Et | NH₂ | i-Pr | SO₂NHMe | |

**[Table 6-4]**

| Compound No. | R¹ | R³ | R⁴ | R⁵ | A | Physical Property value |
|---|---|---|---|---|---|---|
| 6-77 | Ph | Et | NH₂ | Me | SO₂NHMe | |
| 6-78 | Ph | Et | NH₂ | Et | SO₂NHMe | |
| 6-79 | Ph | Et | NH₂ | i-Pr | SO₂NHMe | |
| 6-80 | 2-F-Ph | Et | NH₂ | Me | SO₂NHMe | |
| 6-81 | 2-F-Ph | Et | NH₂ | Et | SO₂NHMe | |
| 6-82 | 2-F-Ph | Et | NH₂ | i-Pr | SO₂NHMe | |
| 6-83 | 3-F-Ph | Et | NH₂ | Me | SO₂NHMe | |
| 6-84 | 3-F-Ph | Et | NH₂ | Et | SO₂NHMe | |
| 6-85 | 3-F-Ph | Et | NH₂ | i-Pr | SO₂NHMe | |
| 6-86 | 4-F-Ph | Et | NH₂ | Me | SO₂NHMe | |
| 6-87 | 4-F-Ph | Et | NH₂ | Et | SO₂NHMe | |
| 6-88 | 4-F-Ph | Et | NH₂ | i-Pr | SO₂NHMe | |
| 6-89 | 2,6-F₂-Ph | Et | NH₂ | Me | SO₂NHMe | |
| 6-90 | 2,6-F₂-Ph | Et | NH₂ | Et | SO₂NHMe | |
| 6-91 | 2,6-F₂-Ph | Et | NH₂ | i-Pr | SO₂NHMe | |
| 6-92 | Ph | Me | NH₂ | Me | SO₂NHEt | |
| 6-93 | Ph | Me | NH₂ | Et | SO₂NHEt | |
| 6-94 | Ph | Me | NH₂ | i-Pr | SO₂NHEt | |
| 6-95 | 2-F-Ph | Me | NH₂ | Me | SO₂NHEt | |
| 6-96 | 2-F-Ph | Me | NH₂ | Et | SO₂NHEt | |
| 6-97 | 2-F-Ph | Me | NH₂ | i-Pr | SO₂NHEt | |
| 6-98 | 3-F-Ph | Me | NH₂ | Me | SO₂NHEt | |
| 6-99 | 3-F-Ph | Me | NH₂ | Et | SO₂NHEt | |
| 6-100 | 3-F-Ph | Me | NH₂ | i-Pr | SO₂NHEt | |
| 6-101 | 4-F-Ph | Me | NH₂ | Me | SO₂NHEt | |
| 6-102 | 4-F-Ph | Me | NH₂ | Et | SO₂NHEt | |
| 6-103 | 4-F-Ph | Me | NH₂ | i-Pr | SO₂NHEt | |
| 6-104 | 2,6-F₂-Ph | Me | NH₂ | Me | SO₂NHEt | |
| 6-105 | 2,6-F₂-Ph | Me | NH₂ | Et | SO₂NHEt | |
| 6-106 | 2,6-F₂-Ph | Me | NH₂ | i-Pr | SO₂NHEt | |

**[Table 6-5]**

| Compound No. | R¹ | R³ | R⁴ | R⁵ | A | Physical Property value |
|---|---|---|---|---|---|---|
| 6-107 | thiazol-5-yl | Me | NH₂ | Me | SO₂NHEt | |
| 6-108 | thiazol-5-yl | Me | NH₂ | Et | SO₂NHEt | |
| 6-109 | thiazol-5-yl | Me | NH₂ | i-Pr | SO₂NHEt | |
| 6-110 | thiazol-4-yl | Me | NH₂ | Me | SO₂NHEt | |
| 6-111 | thiazol-4-yl | Me | NH₂ | Et | SO₂NHEt | |
| 6-112 | thiazol-4-yl | Me | NH₂ | i-Pr | SO₂NHEt | |
| 6-113 | thiazol-2-yl | Me | NH₂ | Me | SO₂NHEt | |
| 6-114 | thiazol-2-yl | Me | NH₂ | Et | SO₂NHEt | |
| 6-115 | thiazol-2-yl | Me | NH₂ | i-Pr | SO₂NHEt | |
| 6-116 | thiophen-2-yl | Me | NH₂ | Me | SO₂NHEt | |
| 6-117 | thiophen-2-yl | Me | NH₂ | Et | SO₂NHEt | |
| 6-118 | thiophen-2-yl | Me | NH₂ | i-Pr | SO₂NHEt | |
| 6-119 | thiophen-3-yl | Me | NH₂ | Me | SO₂NHEt | |
| 6-120 | thiophen-3-yl | Me | NH₂ | Et | SO₂NHEt | |
| 6-121 | thiophen-3-yl | Me | NH₂ | i-Pr | SO₂NHEt | |
| 6-122 | pyridazin-3-yl | Me | NH₂ | Me | SO₂NHEt | |
| 6-123 | pyridazin-3-yl | Me | NH₂ | Et | SO₂NHEt | |
| 6-124 | pyridazin-3-yl | Me | NH₂ | i-Pr | SO₂NHEt | |
| 6-125 | pyrazin-2-yl | Me | NH₂ | Me | SO₂NHEt | |
| 6-126 | pyrazin-2-yl | Me | NH₂ | Et | SO₂NHEt | |
| 6-127 | pyrazin-2-yl | Me | NH₂ | i-Pr | SO₂NHEt | |
| 6-128 | pyrimidin-2-yl | Me | NH₂ | Me | SO₂NHEt | |
| 6-129 | pyrimidin-2-yl | Me | NH₂ | Et | SO₂NHEt | |
| 6-130 | pyrimidin-2-yl | Me | NH₂ | i-Pr | SO₂NHEt | |
| 6-131 | pyrimidin-4-yl | Me | NH₂ | Me | SO₂NHEt | |
| 6-132 | pyrimidin-4-yl | Me | NH₂ | Et | SO₂NHEt | |
| 6-133 | pyrimidin-4-yl | Me | NH₂ | i-Pr | SO₂NHEt | |
| 6-134 | pyrimidin-5-yl | Me | NH₂ | Me | SO₂NHEt | |
| 6-135 | pyrimidin-5-yl | Me | NH₂ | Et | SO₂NHEt | |
| 6-136 | pyrimidin-5-yl | Me | NH₂ | i-Pr | SO₂NHEt | |

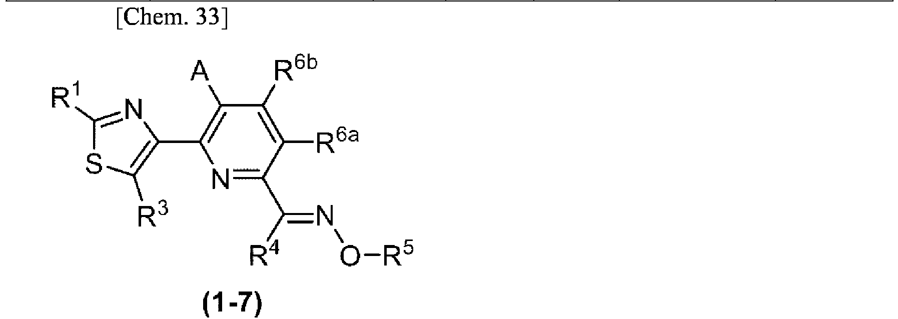

Table 7 (In Table 7, R^{6a} and R^{6b} represent hydrogen atoms.)

**[Table 7-1]**

| Compound No. | R¹ | R³ | R⁴ | R⁵ | A | Physical Property value |
|---|---|---|---|---|---|---|
| 7-1 | Ph | Me | NH₂ | Et | CO₂Et | NMR |
| 7-2 | Ph | Me | NH₂ | Et | CO₂Me | |
| 7-3 | Ph | Me | NH₂ | Me | SO₂NHMe | |
| 7-4 | Ph | Me | NH₂ | Et | SO₂NHMe | |
| 7-5 | Ph | Me | NH₂ | i-Pr | SO₂NHMe | |
| 7-6 | 2-F-Ph | Me | NH₂ | Me | SO₂NHMe | |
| 7-7 | 2-F-Ph | Me | NH₂ | Et | SO₂NHMe | |
| 7-8 | 2-F-Ph | Me | NH₂ | i-Pr | SO₂NHMe | |
| 7-9 | 3-F-Ph | Me | NH₂ | Me | SO₂NHMe | |
| 7-10 | 3-F-Ph | Me | NH₂ | Et | SO₂NHMe | |
| 7-11 | 3-F-Ph | Me | NH₂ | i-Pr | SO₂NHMe | |
| 7-12 | 4-F-Ph | Me | NH₂ | Me | SO₂NHMe | |
| 7-13 | 4-F-Ph | Me | NH₂ | Et | SO₂NHMe | |
| 7-14 | 4-F-Ph | Me | NH₂ | i-Pr | SO₂NHMe | |
| 7-15 | 2,6-F₂-Ph | Me | NH₂ | Me | SO₂NHMe | |
| 7-16 | 2,6-F₂-Ph | Me | NH₂ | Et | SO₂NHMe | |
| 7-17 | 2,6-F₂-Ph | Me | NH₂ | i-Pr | SO₂NHMe | |
| 7-18 | Ph | H | NH₂ | Et | SO₂NHMe | 189-190 |
| 7-19 | Ph | Br | NH₂ | Et | SO₂NHMe | 159-160 |

**[Table 7-2]**

| Compound No. | R¹ | R³ | R⁴ | R⁵ | A | Physical Property value |
|---|---|---|---|---|---|---|
| 7-20 | Ph | Me | NH₂ | Et | SO₂Me | 144-146 |
| 7-21 | Ph | Me | NH₂ | Et | SO₂NHMe | 187-188 |
| 7-22 | Ph | Me | NH₂ | Et | SO₂NMe₂ | 58-59 |
| 7-23 | CONHPh | H | NH₂ | Et | SO₂NHMe | 161-163 |
| 7-24 | thiazol-5-yl | Me | NH₂ | Me | SO₂NHMe | |
| 7-25 | thiazol-5-yl | Me | NH₂ | Et | SO₂NHMe | |
| 7-26 | thiazol-5-yl | Me | NH₂ | i-Pr | SO₂NHMe | |
| 7-27 | thiazol-4-yl | Me | NH₂ | Me | SO₂NHMe | |
| 7-28 | thiazol-4-yl | Me | NH₂ | Et | SO₂NHMe | |
| 7-29 | thiazol-4-yl | Me | NH₂ | i-Pr | SO₂NHMe | |
| 7-30 | thiazol-2-yl | Me | NH₂ | Me | SO₂NHMe | |
| 7-31 | thiazol-2-yl | Me | NH₂ | Et | SO₂NHMe | |
| 7-32 | thiazol-2-yl | Me | NH₂ | i-Pr | SO₂NHMe | |
| 7-33 | thiophen-2-yl | Me | NH₂ | Me | SO₂NHMe | |
| 7-34 | thiophen-2-yl | Me | NH₂ | Et | SO₂NHMe | |
| 7-35 | thiophen-2-yl | Me | NH₂ | i-Pr | SO₂NHMe | |
| 7-36 | thiophen-3-yl | Me | NH₂ | Me | SO₂NHMe | |
| 7-37 | thiophen-3-yl | Me | NH₂ | Et | SO₂NHMe | |
| 7-38 | thiophen-3-yl | Me | NH₂ | i-Pr | SO₂NHMe | |
| 7-39 | pyridazin-3-yl | Me | NH₂ | Me | SO₂NHMe | |
| 7-40 | pyridazin-3-yl | Me | NH₂ | Et | SO₂NHMe | |
| 7-41 | pyridazin-3-yl | Me | NH₂ | i-Pr | SO₂NHMe | |
| 7-42 | pyrazin-2-yl | Me | NH₂ | Me | SO₂NHMe | |
| 7-43 | pyrazin-2-yl | Me | NH₂ | Et | SO₂NHMe | |
| 7-44 | pyrazin-2-yl | Me | NH₂ | i-Pr | SO₂NHMe | |
| 7-45 | pyrimidin-2-yl | Me | NH₂ | Me | SO₂NHMe | |
| 7-46 | pyrimidin-2-yl | Me | NH₂ | Et | SO₂NHMe | |
| 7-47 | pyrimidin-2-yl | Me | NH₂ | i-Pr | SO₂NHMe | |
| 7-48 | pyrimidin-4-yl | Me | NH₂ | Me | SO₂NHMe | |
| 7-49 | pyrimidin-4-yl | Me | NH₂ | Et | SO₂NHMe | |

**[Table 7-3]**

| Compound No. | R¹ | R³ | R⁴ | R⁵ | A | Physical Property value |
|---|---|---|---|---|---|---|
| 7-50 | pyrimidin-4-yl | Me | NH₂ | i-Pr | SO₂NHMe | |
| 7-51 | pyrimidin-5-yl | Me | NH₂ | Me | SO₂NHMe | |
| 7-52 | pyrimidin-5-yl | Me | NH₂ | Et | SO₂NHMe | |
| 7-53 | pyrimidin-5-yl | Me | NH₂ | i-Pr | SO₂NHMe | |
| 7-54 | thiazol-5-yl | Et | NH₂ | Me | SO₂NHMe | |
| 7-55 | thiazol-5-yl | Et | NH₂ | Et | SO₂NHMe | |
| 7-56 | thiazol-5-yl | Et | NH₂ | i-Pr | SO₂NHMe | |
| 7-57 | thiazol-4-yl | Et | NH₂ | Me | SO₂NHMe | |
| 7-58 | thiazol-4-vl | Et | NH₂ | Et | SO₂NHMe | |
| 7-59 | thiazol-4-yl | Et | NH₂ | i-Pr | SO₂NHMe | |
| 7-60 | thiazol-2-yl | Et | NH₂ | Me | SO₂NHMe | |
| 7-61 | thiazol-2-yl | Et | NH₂ | Et | SO₂NHMe | |
| 7-62 | thiazol-2-yl | Et | NH₂ | i-Pr | SO₂NHMe | |
| 7-63 | thiophen-2-yl | Et | NH₂ | Me | SO₂NHMe | |
| 7-64 | thiophen-2-yl | Et | NH₂ | Et | SO₂NHMe | |
| 7-65 | thiophen-2-yl | Et | NH₂ | i-Pr | SO₂NHMe | |
| 7-66 | thiophen-3-yl | Et | NH₂ | Me | SO₂NHMe | |
| 7-67 | thiophen-3-yl | Et | NH₂ | Et | SO₂NHMe | |
| 7-68 | thiophen-3-yl | Et | NH₂ | i-Pr | SO₂NHMe | |
| 7-69 | pyridazin-3-yl | Et | NH₂ | Me | SO₂NHMe | |
| 7-70 | pyridazin-3-yl | Et | NH₂ | Et | SO₂NHMe | |
| 7-71 | pyridazin-3-yl | Et | NH₂ | i-Pr | SO₂NHMe | |
| 7-72 | pyrazin-2-yl | Et | NH₂ | Me | SO₂NHMe | |
| 7-73 | pyrazin-2-yl | Et | NH₂ | Et | SO₂NHMe | |
| 7-74 | pyrazin-2-yl | Et | NH₂ | i-Pr | SO₂NHMe | |
| 7-75 | pyrimidin-2-yl | Et | NH₂ | Me | SO₂NHMe | |
| 7-76 | pyrimidin-2-yl | Et | NH₂ | Et | SO₂NHMe | |
| 7-77 | pyrimidin-2-yl | Et | NH₂ | i-Pr | SO₂NHMe | |
| 7-78 | pyrimidin-4-yl | Et | NH₂ | Me | SO₂NHMe | |
| 7-79 | pyrimidin-4-yl | Et | NH₂ | Et | SO₂NHMe | |

**[Table 7-4]**

| Compound No. | R¹ | R³ | R⁴ | R⁵ | A | Physical Property value |
|---|---|---|---|---|---|---|
| 7-80 | pyrimidin-4-yl | Et | NH₂ | i-Pr | SO₂NHMe | |
| 7-81 | pyrimidin-5-yl | Et | NH₂ | Me | SO₂NHMe | |
| 7-82 | pyrimidin-5-yl | Et | NH₂ | Et | SO₂NHMe | |
| 7-83 | pyrimidin-5-yl | Et | NH₂ | i-Pr | SO₂NHMe | |
| 7-84 | Ph | Et | NH₂ | Me | SO₂NHMe | |
| 7-85 | Ph | Et | NH₂ | Et | SO₂NHMe | |
| 7-86 | Ph | Et | NH₂ | i-Pr | SO₂NHMe | |
| 7-87 | 2-F-Ph | Et | NH₂ | Me | SO₂NHMe | |
| 7-88 | 2-F-Ph | Et | NH₂ | Et | SO₂NHMe | |
| 7-89 | 2-F-Ph | Et | NH₂ | i-Pr | SO₂NHMe | |
| 7-90 | 3-F-Ph | Et | NH₂ | Me | SO₂NHMe | |
| 7-91 | 3-F-Ph | Et | NH₂ | Et | SO₂NHMe | |
| 7-92 | 3-F-Ph | Et | NH₂ | i-Pr | SO₂NHMe | |
| 7-93 | 4-F-Ph | Et | NH₂ | Me | SO₂NHMe | |
| 7-94 | 4-F-Ph | Et | NH₂ | Et | SO₂NHMe | |
| 7-95 | 4-F-Ph | Et | NH₂ | i-Pr | SO₂NHMe | |
| 7-96 | 2,6-F₂-Ph | Et | NH₂ | Me | SO₂NHMe | |
| 7-97 | 2,6-F₂-Ph | Et | NH₂ | Et | SO₂NHMe | |
| 7-98 | 2,6-F₂-Ph | Et | NH₂ | i-Pr | SO₂NHMe | |
| 7-99 | Ph | Me | NH₂ | Me | SO₂NHEt | |
| 7-100 | Ph | Me | NH₂ | Et | SO₂NHEt | |
| 7-101 | Ph | Me | NH₂ | i-Pr | SO₂NHEt | |
| 7-102 | 2-F-Ph | Me | NH₂ | Me | SO₂NHEt | |
| 7-103 | 2-F-Ph | Me | NH₂ | Et | SO₂NHEt | |
| 7-104 | 2-F-Ph | Me | NH₂ | i-Pr | SO₂NHEt | |
| 7-105 | 3-F-Ph | Me | NH₂ | Me | SO₂NHEt | |
| 7-106 | 3-F-Ph | Me | NH₂ | Et | SO₂NHEt | |
| 7-107 | 3-F-Ph | Me | NH₂ | i-Pr | SO₂NHEt | |
| 7-108 | 4-F-Ph | Me | NH₂ | Me | SO₂NHEt | |
| 7-109 | 4-F-Ph | Me | NH₂ | Et | SO₂NHEt | |

**[Table 7-5]**

| Compound No. | R¹ | R³ | R⁴ | R⁵ | A | Physical Property value |
|---|---|---|---|---|---|---|
| 7-110 | 4-F-Ph | Me | NH₂ | i-Pr | SO₂NHEt | |
| 7-111 | 2,6-F2-Ph | Me | NH₂ | Me | SO₂NHEt | |
| 7-112 | 2,6-F2-Ph | Me | NH₂ | Et | SO₂NHEt | |
| 7-113 | 2,6-F2-Ph | Me | NH₂ | i-Pr | SO₂NHEt | |
| 7-114 | thiazol-5-yl | Me | NH₂ | Me | SO₂NHEt | |
| 7-115 | thiazol-5-yl | Me | NH₂ | Et | SO₂NHEt | |
| 7-116 | thiazol-5-vl | Me | NH₂ | i-Pr | SO₂NHEt | |
| 7-117 | thiazol-4-yl | Me | NH₂ | Me | SO₂NHEt | |
| 7-118 | thiazol-4-yl | Me | NH₂ | Et | SO₂NHEt | |
| 7-119 | thiazol-4-yl | Me | NH₂ | i-Pr | SO₂NHEt | |
| 7-120 | thiazol-2-yl | Me | NH₂ | Me | SO₂NHEt | |
| 7-121 | thiazol-2-vl | Me | NH₂ | Et | SO₂NHEt | |
| 7-122 | thiazol-2-yl | Me | NH₂ | i-Pr | SO₂NHEt | |
| 7-123 | thiophen-2-yl | Me | NH₂ | Me | SO₂NHEt | |
| 7-124 | thiophen-2-yl | Me | NH₂ | Et | SO₂NHEt | |
| 7-125 | thiophen-2-yl | Me | NH₂ | i-Pr | SO₂NHEt | |
| 7-126 | thiophen-3-yl | Me | NH₂ | Me | SO₂NHEt | |
| 7-127 | thiophen-3-yl | Me | NH₂ | Et | SO₂NHEt | |
| 7-128 | thiophen-3-yl | Me | NH₂ | i-Pr | SO₂NHEt | |
| 7-129 | pyridazin-3-yl | Me | NH₂ | Me | SO₂NHEt | |
| 7-130 | pyridazin-3-yl | Me | NH₂ | Et | SO₂NHEt | |
| 7-131 | pyridazin-3-yl | Me | NH₂ | i-Pr | SO₂NHEt | |
| 7-132 | pyrazin-2-yl | Me | NH₂ | Me | SO₂NHEt | |
| 7-133 | pyrazin-2-yl | Me | NH₂ | Et | SO₂NHEt | |
| 7-134 | pyrazin-2-yl | Me | NH₂ | i-Pr | SO₂NHEt | |
| 7-135 | pyrimidin-2-yl | Me | NH₂ | Me | SO₂NHEt | |
| 7-136 | pyrimidin-2-yl | Me | NH₂ | Et | SO₂NHEt | |
| 7-137 | pyrimidin-2-yl | Me | NH₂ | i-Pr | SO₂NHEt | |
| 7-138 | pyrimidin-4-yl | Me | NH₂ | Me | SO₂NHEt | |
| 7-139 | pyrimidin-4-yl | Me | NH₂ | Et | SO₂NHEt | |

**[Table 7-6]**

| Compound No. | R¹ | R³ | R⁴ | R⁵ | A | Physical Property value |
|---|---|---|---|---|---|---|
| 7-140 | pyrimidin-4-yl | Me | NH₂ | i-Pr | SO₂NHEt | |
| 7-141 | pyrimidin-5-yl | Me | NH₂ | Me | SO₂NHEt | |
| 7-142 | pyrimidin-5-yl | Me | NH₂ | Et | SO₂NHEt | |
| 7-143 | pyrimidin-5-yl | Me | NH₂ | i-Pr | SO₂NHEt | |

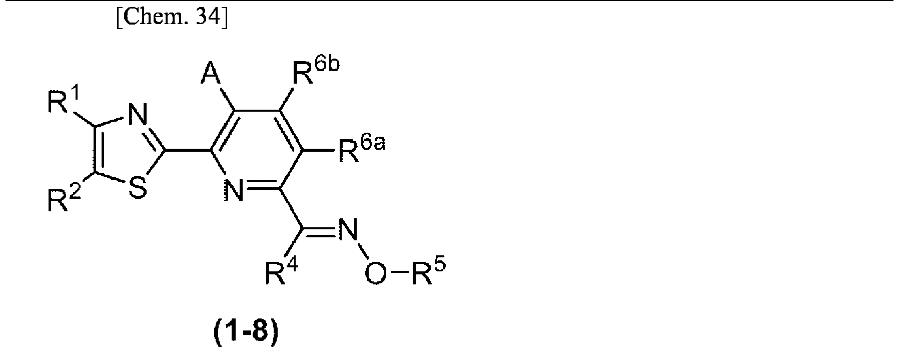

Table 8 (In Table 8, R^{6a} and R^{6b} represent hydrogen atoms. In Table 8-1, R² represents a hydrogen atom.)

**[Table 8-1]**

| Compound No. | R¹ | R⁴ | R⁵ | A | Physical Property value |
|---|---|---|---|---|---|
| 8-1 | Ph | NH₂ | Me | SO₂NHMe | 190-191 |
| 8-2 | Ph | NH₂ | Et | SO₂NHMe | 157-158 |
| 8-3 | Ph | NH₂ | i-Pr | SO₂NHMe | |
| 8-4 | 2-F-Ph | NH₂ | Me | SO₂NHMe | 208-210 |
| 8-5 | 2-F-Ph | NH₂ | Et | SO₂NHMe | 150-151 |
| 8-6 | 2-F-Ph | NH₂ | i-Pr | SO₂NHMe | |
| 8-7 | 3-F-Ph | NH₂ | Me | SO₂NHMe | |
| 8-8 | 3-F-Ph | NH₂ | Et | SO₂NHMe | |
| 8-9 | 3-F-Ph | NH₂ | i-Pr | SO₂NHMe | |
| 8-10 | 4-F-Ph | NH₂ | Me | SO₂NHMe | |
| 8-11 | 4-F-Ph | NH₂ | Et | SO₂NHMe | |
| 8-12 | 4-F-Ph | NH₂ | i-Pr | SO₂NHMe | |
| 8-13 | 2,6-F₂-Ph | NH₂ | Me | SO₂NHMe | |
| 8-14 | 2,6-F₂-Ph | NH₂ | Et | SO₂NHMe | |
| 8-15 | 2,6-F₂-Ph | NH₂ | i-Pr | SO₂NHMe | |
| 8-16 | Ph | NH₂ | Me | SO₂Me | 236-237 |
| 8-17 | Ph | NH₂ | Et | SO₂Me | 177-179 |
| 8-18 | Ph | NH₂ | i-Pr | SO₂Me | |
| 8-19 | 2-F-Ph | NH₂ | Me | SO₂Me | |
| 8-20 | 2-F-Ph | NH₂ | Et | SO₂Me | |
| 8-21 | 2-F-Ph | NH₂ | i-Pr | SO₂Me | |

**[Table 8-2]**

| Compound No. | R¹ | R² | R⁴ | R⁵ | A | Physical Property value |
|---|---|---|---|---|---|---|
| 8-22 | Ph | Me | NH₂ | Me | SO₂Me | 226-228 |
| 8-23 | Ph | Me | NH₂ | Et | SO₂Me | 179-180 |
| 8-24 | pyridin-2-yl | H | NH₂ | Me | SO₂Me | 230-232 |
| 8-25 | pyridin-2-yl | H | NH₂ | Et | SO₂Me | 204-205 |
| 8-26 | pyridin-2-yl | H | NH₂ | Me | SO₂NHMe | 234-236 |
| 8-27 | pyridin-2-yl | H | NH₂ | Et | SO₂NHMe | 222-223 |
| 8-28 | Ph | Me | NH₂ | Et | SO₂NHMe | 184-185 |
| 8-29 | Ph | Cl | NH₂ | Me | SO₂NHMe | 232-233 |
| 8-30 | thiazol-5-yl | Me | NH₂ | Me | SO₂NHMe | |
| 8-31 | thiazol-5-yl | Me | NH₂ | Et | SO₂NHMe | |
| 8-32 | thiazol-5-yl | Me | NH₂ | i-Pr | SO₂NHMe | |
| 8-33 | thiazol-4-yl | Me | NH₂ | Me | SO₂NHMe | |
| 8-34 | thiazol-4-yl | Me | NH₂ | Et | SO₂NHMe | |
| 8-35 | thiazol-4-yl | Me | NH₂ | i-Pr | SO₂NHMe | |
| 8-36 | thiazol-2-yl | Me | NH₂ | Me | SO₂NHMe | |
| 8-37 | thiazol-2-yl | Me | NH₂ | Et | SO₂NHMe | |
| 8-38 | thiazol-2-yl | Me | NH₂ | i-Pr | SO₂NHMe | |
| 8-39 | thiophen-2-yl | Me | NH₂ | Me | SO₂NHMe | |
| 8-40 | thiophen-2-yl | Me | NH₂ | Et | SO₂NHMe | |
| 8-41 | thiophen-2-yl | Me | NH₂ | i-Pr | SO₂NHMe | |
| 8-42 | thiophen-3-yl | Me | NH₂ | Me | SO₂NHMe | |
| 8-43 | thiophen-3-yl | Me | NH₂ | Et | SO₂NHMe | |
| 8-44 | thiophen-3-yl | Me | NH₂ | i-Pr | SO₂NHMe | |
| 8-45 | pyridazin-3-yl | Me | NH₂ | Me | SO₂NHMe | |
| 8-46 | pyridazin-3-yl | Me | NH₂ | Et | SO₂NHMe | |
| 8-47 | pyridazin-3-yl | Me | NH₂ | i-Pr | SO₂NHMe | |
| 8-48 | pyrazin-2-yl | Me | NH₂ | Me | SO₂NHMe | |
| 8-49 | pyrazin-2-yl | Me | NH₂ | Et | SO₂NHMe | |
| 8-50 | pyrazin-2-yl | Me | NH₂ | i-Pr | SO₂NHMe | |
| 8-51 | pyrimidin-2-yl | Me | NH₂ | Me | SO₂NHMe | |

**[Table 8-3]**

| Compound No. | R¹ | R² | R⁴ | R⁵ | A | Physical Property value |
|---|---|---|---|---|---|---|
| 8-52 | pyrimidin-2-yl | Me | NH₂ | Et | SO₂NHMe | |
| 8-53 | pyrimidin-2-yl | Me | NH₂ | i-Pr | SO₂NHMe | |
| 8-54 | pyrimidin-4-yl | Me | NH₂ | Me | SO₂NHMe | |
| 8-55 | pyrimidin-4-yl | Me | NH₂ | Et | SO₂NHMe | |
| 8-56 | pyrimidin-4-yl | Me | NH₂ | i-Pr | SO₂NHMe | |
| 8-57 | pyrimidin-5-yl | Me | NH₂ | Me | SO₂NHMe | |
| 8-58 | pyrimidin-5-yl | Me | NH₂ | Et | SO₂NHMe | |
| 8-59 | pyrimidin-5-yl | Me | NH₂ | i-Pr | SO₂NHMe | |
| 8-60 | Ph | Me | NH₂ | Me | SO₂NHEt | |
| 8-61 | Ph | Me | NH₂ | Et | SO₂NHEt | |
| 8-62 | Ph | Me | NH₂ | i-Pr | SO₂NHEt | |
| 8-63 | 2-F-Ph | Me | NH₂ | Me | SO₂NHEt | |
| 8-64 | 2-F-Ph | Me | NH₂ | Et | SO₂NHEt | |
| 8-65 | 2-F-Ph | Me | NH₂ | i-Pr | SO₂NHEt | |
| 8-66 | 3-F-Ph | Me | NH₂ | Me | SO₂NHEt | |
| 8-67 | 3-F-Ph | Me | NH₂ | Et | SO₂NHEt | |
| 8-68 | 3-F-Ph | Me | NH₂ | i-Pr | SO₂NHEt | |
| 8-69 | 4-F-Ph | Me | NH₂ | Me | SO₂NHEt | |
| 8-70 | 4-F-Ph | Me | NH₂ | Et | SO₂NHEt | |
| 8-71 | 4-F-Ph | Me | NH₂ | i-Pr | SO₂NHEt | |
| 8-72 | 2,6-F₂-Ph | Me | NH₂ | Me | SO₂NHEt | |
| 8-73 | 2,6-F₂-Ph | Me | NH₂ | Et | SO₂NHEt | |
| 8-74 | 2,6-F₂-Ph | Me | NH₂ | i-Pr | SO₂NHEt | |
| 8-75 | thiazol-5-yl | Me | NH₂ | Me | SO₂NHEt | |
| 8-76 | thiazol-5-yl | Me | NH₂ | Et | SO₂NHEt | |
| 8-77 | thiazol-5-yl | Me | NH₂ | i-Pr | SO₂NHEt | |
| 8-78 | thiazol-4-yl | Me | NH₂ | Me | SO₂NHEt | |
| 8-79 | thiazol-4-yl | Me | NH₂ | Et | SO₂NHEt | |
| 8-80 | thiazol-4-yl | Me | NH₂ | i-Pr | SO₂NHEt | |
| 8-81 | thiazol-2-yl | Me | NH₂ | Me | SO₂NHEt | |

**[Table 8-4]**

| Compound No. | R¹ | R² | R⁴ | R⁵ | A | Physical Property value |
|---|---|---|---|---|---|---|
| 8-82 | thiazol-2-yl | Me | NH₂ | Et | SO₂NHEt | |
| 8-83 | thiazol-2-yl | Me | NH₂ | i-Pr | SO₂NHEt | |
| 8-84 | thiophen-2-yl | Me | NH₂ | Me | SO₂NHEt | |
| 8-85 | thiophen-2-yl | Me | NH₂ | Et | SO₂NHEt | |
| 8-86 | thiophen-2-yl | Me | NH₂ | i-Pr | SO₂NHEt | |
| 8-87 | thiophen-3-yl | Me | NH₂ | Me | SO₂NHEt | |
| 8-88 | thiophen-3-yl | Me | NH₂ | Et | SO₂NHEt | |
| 8-89 | thiophen-3-yl | Me | NH₂ | i-Pr | SO₂NHEt | |
| 8-90 | pyridazin-3-yl | Me | NH₂ | Me | SO₂NHEt | |
| 8-91 | pyridazin-3-yl | Me | NH₂ | Et | SO₂NHEt | |
| 8-92 | pyridazin-3-yl | Me | NH₂ | i-Pr | SO₂NHEt | |
| 8-93 | pyrazin-2-yl | Me | NH₂ | Me | SO₂NHEt | |
| 8-94 | pyrazin-2-yl | Me | NH₂ | Et | SO₂NHEt | |
| 8-95 | pyrazin-2-yl | Me | NH₂ | i-Pr | SO₂NHEt | |
| 8-96 | pyrimidin-2-yl | Me | NH₂ | Me | SO₂NHEt | |
| 8-97 | pyrimidin-2-yl | Me | NH₂ | Et | SO₂NHEt | |
| 8-98 | pyrimidin-2-yl | Me | NH₂ | i-Pr | SO₂NHEt | |
| 8-99 | pyrimidin-4-yl | Me | NH₂ | Me | SO₂NHEt | |
| 8-100 | pyrimidin-4-yl | Me | NH₂ | Et | SO₂NHEt | |
| 8-101 | pyrimidin-4-yl | Me | NH₂ | i-Pr | SO₂NHEt | |
| 8-102 | pyrimidin-5-yl | Me | NH₂ | Me | SO₂NHEt | |
| 8-103 | pyrimidin-5-yl | Me | NH₂ | Et | SO₂NHEt | |
| 8-104 | pyrimidin-5-yl | Me | NH₂ | i-Pr | SO₂NHEt | |

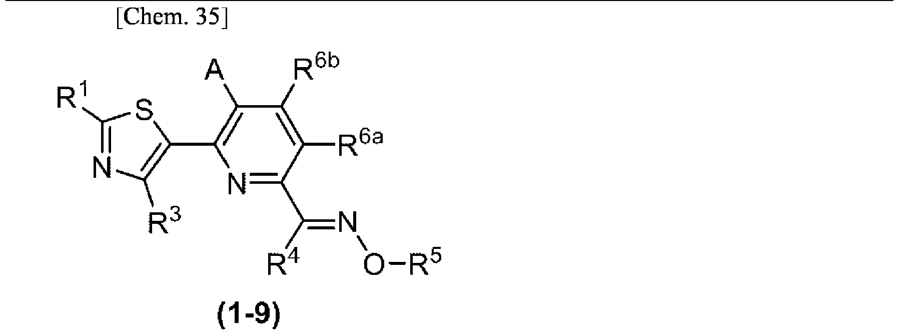

Table 9 (In Table 9, R^{6a} and R^{6b} represent hydrogen atoms.)

**[Table 9-1]**

| Compound No. | R¹ | R³ | R⁴ | R⁵ | A | Physical Property value |
|---|---|---|---|---|---|---|
| 9-1 | Ph | Me | NH₂ | Me | SO₂NHMe | |
| 9-2 | Ph | Me | NH₂ | Et | SO₂NHMe | |
| 9-3 | Ph | Me | NH₂ | i-Pr | SO₂NHMe | |
| 9-4 | 2-F-Ph | Me | NH₂ | Me | SO₂NHMe | |
| 9-5 | 2-F-Ph | Me | NH₂ | Et | SO₂NHMe | |
| 9-6 | 2-F-Ph | Me | NH₂ | i-Pr | SO₂NHMe | |
| 9-7 | 3-F-Ph | Me | NH₂ | Me | SO₂NHMe | |
| 9-8 | 3-F-Ph | Me | NH₂ | Et | SO₂NHMe | |
| 9-9 | 3-F-Ph | Me | NH₂ | i-Pr | SO₂NHMe | |
| 9-10 | 4-F-Ph | Me | NH₂ | Me | SO₂NHMe | |
| 9-11 | 4-F-Ph | Me | NH₂ | Et | SO₂NHMe | |
| 9-12 | 4-F-Ph | Me | NH₂ | i-Pr | SO₂NHMe | |
| 9-13 | 2,6-F₂-Ph | Me | NH₂ | Me | SO₂NHMe | |
| 9-14 | 2,6-F₂-Ph | Me | NH₂ | Et | SO₂NHMe | |
| 9-15 | 2,6-F₂-Ph | Me | NH₂ | i-Pr | SO₂NHMe | |

**[Table 9-2]**

| Compound No. | R¹ | R³ | R⁴ | R⁵ | A | Physical Property value |
|---|---|---|---|---|---|---|
| 9-16 | thiazol-5-yl | Me | NH₂ | Me | SO₂NHMe | |
| 9-17 | thiazol-5-yl | Me | NH₂ | Et | SO₂NHMe | |
| 9-18 | thiazol-5-yl | Me | NH₂ | i-Pr | SO₂NHMe | |
| 9-19 | thiazol-4-yl | Me | NH₂ | Me | SO₂NHMe | |
| 9-20 | thiazol-4-yl | Me | NH₂ | Et | SO₂NHMe | |
| 9-21 | thiazol-4-yl | Me | NH₂ | i-Pr | SO₂NHMe | |
| 9-22 | thiazol-2-yl | Me | NH₂ | Me | SO₂NHMe | |
| 9-23 | thiazol-2-yl | Me | NH₂ | Et | SO₂NHMe | |
| 9-24 | thiazol-2-yl | Me | NH₂ | i-Pr | SO₂NHMe | |
| 9-25 | thiophen-2-yl | Me | NH₂ | Me | SO₂NHMe | |
| 9-26 | thiophen-2-yl | Me | NH₂ | Et | SO₂NHMe | |
| 9-27 | thiophen-2-yl | Me | NH₂ | i-Pr | SO₂NHMe | |
| 9-28 | thiophen-3-yl | Me | NH₂ | Me | SO₂NHMe | |
| 9-29 | thiophen-3-yl | Me | NH₂ | Et | SO₂NHMe | |
| 9-30 | thiophen-3-yl | Me | NH₂ | i-Pr | SO₂NHMe | |
| 9-31 | pyridazin-3-yl | Me | NH₂ | Me | SO₂NHMe | |
| 9-32 | pyridazin-3-yl | Me | NH₂ | Et | SO₂NHMe | |
| 9-33 | pyridazin-3-yl | Me | NH₂ | i-Pr | SO₂NHMe | |
| 9-34 | pyrazin-2-yl | Me | NH₂ | Me | SO₂NHMe | |
| 9-35 | pyrazin-2-yl | Me | NH₂ | Et | SO₂NHMe | |
| 9-36 | pyrazin-2-yl | Me | NH₂ | i-Pr | SO₂NHMe | |
| 9-37 | pyrimidin-2-yl | Me | NH₂ | Me | SO₂NHMe | |
| 9-38 | pyrimidin-2-yl | Me | NH₂ | Et | SO₂NHMe | |
| 9-39 | pyrimidin-2-yl | Me | NH₂ | i-Pr | SO₂NHMe | |
| 9-40 | pyrimidin-4-yl | Me | NH₂ | Me | SO₂NHMe | |
| 9-41 | pyrimidin-4-yl | Me | NH₂ | Et | SO₂NHMe | |
| 9-42 | pyrimidin-4-yl | Me | NH₂ | i-Pr | SO₂NHMe | |
| 9-43 | pyrimidin-5-yl | Me | NH₂ | Me | SO₂NHMe | |
| 9-44 | pyrimidin-5-yl | Me | NH₂ | Et | SO₂NHMe | |
| 9-45 | pyrimidin-5-yl | Me | NH₂ | i-Pr | SO₂NHMe | |

**[Table 9-3]**

| Compound No. | R¹ | R³ | R⁴ | R⁵ | A | Physical Property value |
|---|---|---|---|---|---|---|
| 9-46 | thiazol-5-yl | Et | NH₂ | Me | SO₂NHMe | |
| 9-47 | thiazol-5-yl | Et | NH₂ | Et | SO₂NHMe | |
| 9-48 | thiazol-5-yl | Et | NH₂ | i-Pr | SO₂NHMe | |
| 9-49 | thiazol-4-yl | Et | NH₂ | Me | SO₂NHMe | |
| 9-50 | thiazol-4-yl | Et | NH₂ | Et | SO₂NHMe | |
| 9-51 | thiazol-4-yl | Et | NH₂ | i-Pr | SO₂NHMe | |
| 9-52 | thiazol-2-yl | Et | NH₂ | Me | SO₂NHMe | |
| 9-53 | thiazol-2-yl | Et | NH₂ | Et | SO₂NHMe | |
| 9-54 | thiazol-2-yl | Et | NH₂ | i-Pr | SO₂NHMe | |
| 9-55 | thiophen-2-yl | Et | NH₂ | Me | SO₂NHMe | |
| 9-56 | thiophen-2-yl | Et | NH₂ | Et | SO₂NHMe | |
| 9-57 | thiophen-2-yl | Et | NH₂ | i-Pr | SO₂NHMe | |
| 9-58 | thiophen-3-yl | Et | NH₂ | Me | SO₂NHMe | |
| 9-59 | thiophen-3-yl | Et | NH₂ | Et | SO₂NHMe | |
| 9-60 | thiophen-3-yl | Et | NH₂ | i-Pr | SO₂NHMe | |
| 9-61 | pyridazin-3-yl | Et | NH₂ | Me | SO₂NHMe | |
| 9-62 | pyridazin-3-yl | Et | NH₂ | Et | SO₂NHMe | |
| 9-63 | pyridazin-3-yl | Et | NH₂ | i-Pr | SO₂NHMe | |
| 9-64 | pyrazin-2-yl | Et | NH₂ | Me | SO₂NHMe | |
| 9-65 | pyrazin-2-yl | Et | NH₂ | Et | SO₂NHMe | |
| 9-66 | pyrazin-2-yl | Et | NH₂ | i-Pr | SO₂NHMe | |
| 9-67 | pyrimidin-2-yl | Et | NH₂ | Me | SO₂NHMe | |
| 9-68 | pyrimidin-2-yl | Et | NH₂ | Et | SO₂NHMe | |
| 9-69 | pyrimidin-2-yl | Et | NH₂ | i-Pr | SO₂NHMe | |
| 9-70 | pyrimidin-4-yl | Et | NH₂ | Me | SO₂NHMe | |
| 9-71 | pyrimidin-4-yl | Et | NH₂ | Et | SO₂NHMe | |
| 9-72 | pyrimidin-4-yl | Et | NH₂ | i-Pr | SO₂NHMe | |
| 9-73 | pyrimidin-5-yl | Et | NH₂ | Me | SO₂NHMe | |
| 9-74 | pyrimidin-5-yl | Et | NH₂ | Et | SO₂NHMe | |
| 9-75 | pyrimidin-5-yl | Et | NH₂ | i-Pr | SO₂NHMe | |

**[Table 9-4]**

| Compound No. | R¹ | R³ | R⁴ | R⁵ | A | Physical Property value |
|---|---|---|---|---|---|---|
| 9-76 | Ph | Et | NH₂ | Me | SO₂NHMe | |
| 9-77 | Ph | Et | NH₂ | Et | SO₂NHMe | |
| 9-78 | Ph | Et | NH₂ | i-Pr | SO₂NHMe | |
| 9-79 | 2-F-Ph | Et | NH₂ | Me | SO₂NHMe | |
| 9-80 | 2-F-Ph | Et | NH₂ | Et | SO₂NHMe | |
| 9-81 | 2-F-Ph | Et | NH₂ | i-Pr | SO₂NHMe | |
| 9-82 | 3-F-Ph | Et | NH₂ | Me | SO₂NHMe | |
| 9-83 | 3-F-Ph | Et | NH₂ | Et | SO₂NHMe | |
| 9-84 | 3-F-Ph | Et | NH₂ | i-Pr | SO₂NHMe | |
| 9-85 | 4-F-Ph | Et | NH₂ | Me | SO₂NHMe | |
| 9-86 | 4-F-Ph | Et | NH₂ | Et | SO₂NHMe | |
| 9-87 | 4-F-Ph | Et | NH₂ | i-Pr | SO₂NHMe | |
| 9-88 | 2,6-F₂-Ph | Et | NH₂ | Me | SO₂NHMe | |
| 9-89 | 2,6-F₂-Ph | Et | NH₂ | Et | SO₂NHMe | |
| 9-90 | 2,6-F₂-Ph | Et | NH₂ | i-Pr | SO₂NHMe | |
| 9-91 | Ph | Me | NH₂ | Me | SO₂NHEt | |
| 9-92 | Ph | Me | NH₂ | Et | SO₂NHEt | |
| 9-93 | Ph | Me | NH₂ | i-Pr | SO₂NHEt | |
| 9-94 | 2-F-Ph | Me | NH₂ | Me | SO₂NHEt | |
| 9-95 | 2-F-Ph | Me | NH₂ | Et | SO₂NHEt | |
| 9-96 | 2-F-Ph | Me | NH₂ | i-Pr | SO₂NHEt | |
| 9-97 | 3-F-Ph | Me | NH₂ | Me | SO₂NHEt | |
| 9-98 | 3-F-Ph | Me | NH₂ | Et | SO₂NHEt | |
| 9-99 | 3-F-Ph | Me | NH₂ | i-Pr | SO₂NHEt | |
| 9-100 | 4-F-Ph | Me | NH₂ | Me | SO₂NHEt | |
| 9-101 | 4-F-Ph | Me | NH₂ | Et | SO₂NHEt | |
| 9-102 | 4-F-Ph | Me | NH₂ | i-Pr | SO₂NHEt | |
| 9-103 | 2,6-F₂-Ph | Me | NH₂ | Me | SO₂NHEt | |
| 9-104 | 2,6-F₂-Ph | Me | NH₂ | Et | SO₂NHEt | |
| 9-105 | 2,6-F₂-Ph | Me | NH₂ | i-Pr | SO₂NHEt | |

**[Table 9-5]**

| Compound No. | R¹ | R³ | R⁴ | R⁵ | A | Physical Property value |
|---|---|---|---|---|---|---|
| 9-106 | thiazol-5-yl | Me | NH₂ | Me | SO₂NHEt | |
| 9-107 | thiazol-5-yl | Me | NH₂ | Et | SO₂NHEt | |
| 9-108 | thiazol-5-yl | Me | NH₂ | i-Pr | SO₂NHEt | |
| 9-109 | thiazol-4-yl | Me | NH₂ | Me | SO₂NHEt | |
| 9-110 | thiazol-4-yl | Me | NH₂ | Et | SO₂NHEt | |
| 9-111 | thiazol-4-yl | Me | NH₂ | i-Pr | SO₂NHEt | |
| 9-112 | thiazol-2-yl | Me | NH₂ | Me | SO₂NHEt | |
| 9-113 | thiazol-2-yl | Me | NH₂ | Et | SO₂NHEt | |
| 9-114 | thiazol-2-yl | Me | NH₂ | i-Pr | SO₂NHEt | |
| 9-115 | thiophen-2-yl | Me | NH₂ | Me | SO₂NHEt | |
| 9-116 | thiophen-2-yl | Me | NH₂ | Et | SO₂NHEt | |
| 9-117 | thiophen-2-yl | Me | NH₂ | i-Pr | SO₂NHEt | |
| 9-118 | thiophen-3-yl | Me | NH₂ | Me | SO₂NHEt | |
| 9-119 | thiophen-3-yl | Me | NH₂ | Et | SO₂NHEt | |
| 9-120 | thiophen-3-yl | Me | NH₂ | i-Pr | SO₂NHEt | |
| 9-121 | pyridazin-3-yl | Me | NH₂ | Me | SO₂NHEt | |
| 9-122 | pyridazin-3-yl | Me | NH₂ | Et | SO₂NHEt | |
| 9-123 | pyridazin-3-yl | Me | NH₂ | i-Pr | SO₂NHEt | |
| 9-124 | pyrazin-2-yl | Me | NH₂ | Me | SO₂NHEt | |
| 9-125 | pyrazin-2-yl | Me | NH₂ | Et | SO₂NHEt | |
| 9-126 | pyrazin-2-yl | Me | NH₂ | i-Pr | SO₂NHEt | |
| 9-127 | pyrimidin-2-yl | Me | NH₂ | Me | SO₂NHEt | |
| 9-128 | pyrimidin-2-yl | Me | NH₂ | Et | SO₂NHEt | |
| 9-129 | pyrimidin-2-yl | Me | NH₂ | i-Pr | SO₂NHEt | |
| 9-130 | pyrimidin-4-yl | Me | NH₂ | Me | SO₂NHEt | |
| 9-131 | pyrimidin-4-yl | Me | NH₂ | Et | SO₂NHEt | |
| 9-132 | pyrimidin-4-yl | Me | NH₂ | i-Pr | SO₂NHEt | |
| 9-133 | pyrimidin-5-yl | Me | NH₂ | Me | SO₂NHEt | |
| 9-134 | pyrimidin-5-yl | Me | NH₂ | Et | SO₂NHEt | |
| 9-135 | pyrimidin-5-yl | Me | NH₂ | i-Pr | SO₂NHEt | |

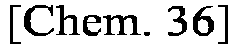

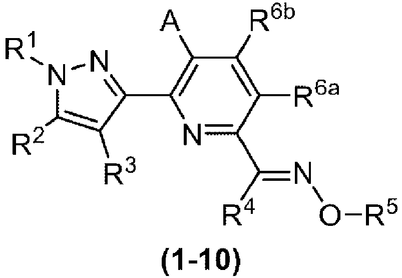

Table 10 (In Table 10, R^{6a} and R^{6b} represent hydrogen atoms.)

**[Table 10-1]**

| Compound No. | R¹ | R² | R³ | R⁴ | R⁵ | A | Physical Property value |
|---|---|---|---|---|---|---|---|
| 10-1 | Ph | H | Me | NH₂ | Me | SO₂NHMe | |
| 10-2 | Ph | H | Me | NH₂ | Et | SO₂NHMe | 130-131 |
| 10-3 | Ph | H | Me | NH₂ | i-Pr | SO₂NHMe | |
| 10-4 | 2-F-Ph | H | Me | NH₂ | Me | SO₂NHMe | |
| 10-5 | 2-F-Ph | H | Me | NH₂ | Et | SO₂NHMe | |
| 10-6 | 2-F-Ph | H | Me | NH₂ | i-Pr | SO₂NHMe | |
| 10-7 | 3-F-Ph | H | Me | NH₂ | Me | SO₂NHMe | |
| 10-8 | 3-F-Ph | H | Me | NH₂ | Et | SO₂NHMe | |
| 10-9 | 3-F-Ph | H | Me | NH₂ | i-Pr | SO₂NHMe | |
| 10-10 | 4-F-Ph | H | Me | NH₂ | Me | SO₂NHMe | |
| 10-11 | 4-F-Ph | H | Me | NH₂ | Et | SO₂NHMe | |
| 10-12 | 4-F-Ph | H | Me | NH₂ | i-Pr | SO₂NHMe | |
| 10-13 | 2,6-F₂-Ph | H | Me | NH₂ | Me | SO₂NHMe | |
| 10-14 | 2,6-F₂-Ph | H | Me | NH₂ | Et | SO₂NHMe | |
| 10-15 | 2,6-F₂-Ph | H | Me | NH₂ | i-Pr | SO₂NHMe | |
| 10-16 | Ph | H | Me | NH₂ | Et | SO₂Me | 150-151 |

**[Table 10-2]**

| Compound No. | R¹ | R² | R³ | R⁴ | R⁵ | A | Physical Property value |
|---|---|---|---|---|---|---|---|
| 10-17 | Bn | H | Me | NH₂ | Et | SO₂Me | NMR |
| 10-18 | 2-F-Ph | H | Me | NH₂ | Et | SO₂Me | NMR |
| 10-19 | Bz | H | Me | NH₂ | Et | SO₂Me | NMR |
| 10-20 | Ph | H | Me | NH₂ | Et | SO₂NMe₂ | NMR |
| 10-21 | thiazol-5-yl | H | Me | NH₂ | Me | SO₂NHMe | |
| 10-22 | thiazol-5-yl | H | Me | NH₂ | Et | SO₂NHMe | |
| 10-23 | thiazol-5-yl | H | Me | NH₂ | i-Pr | SO₂NHMe | |
| 10-24 | thiazol-4-yl | H | Me | NH₂ | Me | SO₂NHMe | |
| 10-25 | thiazol-4-yl | H | Me | NH₂ | Et | SO₂NHMe | |
| 10-26 | thiazol-4-yl | H | Me | NH₂ | i-Pr | SO₂NHMe | |
| 10-27 | thiazol-2-yl | H | Me | NH₂ | Me | SO₂NHMe | |
| 10-28 | thiazol-2-yl | H | Me | NH₂ | Et | SO₂NHMe | |
| 10-29 | thiazol-2-yl | H | Me | NH₂ | i-Pr | SO₂NHMe | |
| 10-30 | thiophen-2-yl | H | Me | NH₂ | Me | SO₂NHMe | |
| 10-31 | thiophen-2-yl | H | Me | NH₂ | Et | SO₂NHMe | |
| 10-32 | thiophen-2-yl | H | Me | NH₂ | i-Pr | SO₂NHMe | |
| 10-33 | thiophen-3-yl | H | Me | NH₂ | Me | SO₂NHMe | |
| 10-34 | thiophen-3-yl | H | Me | NH₂ | Et | SO₂NHMe | |
| 10-35 | thiophen-3-yl | H | Me | NH₂ | i-Pr | SO₂NHMe | |
| 10-36 | pyridazin-3-yl | H | Me | NH₂ | Me | SO₂NHMe | |
| 10-37 | pyridazin-3-yl | H | Me | NH₂ | Et | SO₂NHMe | |
| 10-38 | pyridazin-3-yl | H | Me | NH₂ | i-Pr | SO₂NHMe | |
| 10-39 | pyrazin-2-yl | H | Me | NH₂ | Me | SO₂NHMe | |
| 10-40 | pyrazin-2-yl | H | Me | NH₂ | Et | SO₂NHMe | |
| 10-41 | pyrazin-2-yl | H | Me | NH₂ | i-Pr | SO₂NHMe | |
| 10-42 | pyrimidin-2-yl | H | Me | NH₂ | Me | SO₂NHMe | |
| 10-43 | pyrimidin-2-yl | H | Me | NH₂ | Et | SO₂NHMe | |
| 10-44 | pyrimidin-2-yl | H | Me | NH₂ | i-Pr | SO₂NHMe | |
| 10-45 | pyrimidin-4-yl | H | Me | NH₂ | Me | SO₂NHMe | |
| 10-46 | pyrimidin-4-yl | H | Me | NH₂ | Et | SO₂NHMe | |

**[Table 10-3]**

| Compound No. | R¹ | R² | R³ | R⁴ | R⁵ | A | Physical Property value |
|---|---|---|---|---|---|---|---|
| 10-47 | pyrimidin-4-yl | H | Me | NH₂ | i-Pr | SO₂NHMe | |
| 10-48 | pyrimidin-5-yl | H | Me | NH₂ | Me | SO₂NHMe | |
| 10-49 | pyrimidin-5-yl | H | Me | NH₂ | Et | SO₂NHMe | |
| 10-50 | pyrimidin-5-yl | H | Me | NH₂ | i-Pr | SO₂NHMe | |
| 10-51 | thiazol-5-yl | H | Et | NH₂ | Me | SO₂NHMe | |
| 10-52 | thiazol-5-yl | H | Et | NH₂ | Et | SO₂NHMe | |
| 10-53 | thiazol-5-yl | H | Et | NH₂ | i-Pr | SO₂NHMe | |
| 10-54 | thiazol-4-yl | H | Et | NH₂ | Me | SO₂NHMe | |
| 10-55 | thiazol-4-yl | H | Et | NH₂ | Et | SO₂NHMe | |
| 10-56 | thiazol-4-yl | H | Et | NH₂ | i-Pr | SO₂NHMe | |
| 10-57 | thiazol-2-yl | H | Et | NH₂ | Me | SO₂NHMe | |
| 10-58 | thiazol-2-yl | H | Et | NH₂ | Et | SO₂NHMe | |
| 10-59 | thiazol-2-yl | H | Et | NH₂ | i-Pr | SO₂NHMe | |
| 10-60 | thiophen-2-yl | H | Et | NH₂ | Me | SO₂NHMe | |
| 10-61 | thiophen-2-yl | H | Et | NH₂ | Et | SO₂NHMe | |
| 10-62 | thiophen-2-yl | H | Et | NH₂ | i-Pr | SO₂NHMe | |
| 10-63 | thiophen-3-yl | H | Et | NH₂ | Me | SO₂NHMe | |
| 10-64 | thiophen-3-yl | H | Et | NH₂ | Et | SO₂NHMe | |
| 10-65 | thiophen-3-yl | H | Et | NH₂ | i-Pr | SO₂NHMe | |
| 10-66 | pyridazin-3-yl | H | Et | NH₂ | Me | SO₂NHMe | |
| 10-67 | pyridazin-3-yl | H | Et | NH₂ | Et | SO₂NHMe | |
| 10-68 | pyridazin-3-yl | H | Et | NH₂ | i-Pr | SO₂NHMe | |
| 10-69 | pyrazin-2-yl | H | Et | NH₂ | Me | SO₂NHMe | |
| 10-70 | pyrazin-2-yl | H | Et | NH₂ | Et | SO₂NHMe | |
| 10-71 | pyrazin-2-yl | H | Et | NH₂ | i-Pr | SO₂NHMe | |
| 10-72 | pyrimidin-2-yl | H | Et | NH₂ | Me | SO₂NHMe | |
| 10-73 | pyrimidin-2-yl | H | Et | NH₂ | Et | SO₂NHMe | |
| 10-74 | pyrimidin-2-yl | H | Et | NH₂ | i-Pr | SO₂NHMe | |
| 10-75 | pyrimidin-4-yl | H | Et | NH₂ | Me | SO₂NHMe | |
| 10-76 | pyrimidin-4-yl | H | Et | NH₂ | Et | SO₂NHMe | |

**[Table 10-4]**

| Compound No. | R¹ | R² | R³ | R⁴ | R⁵ | A | Physical Property value |
|---|---|---|---|---|---|---|---|
| 10-77 | pyrimidin-4-yl | H | Et | NH₂ | i-Pr | SO₂NHMe | |
| 10-78 | pyrimidin-5-yl | H | Et | NH₂ | Me | SO₂NHMe | |
| 10-79 | pyrimidin-5-yl | H | Et | NH₂ | Et | SO₂NHMe | |
| 10-80 | pyrimidin-5-yl | H | Et | NH₂ | i-Pr | SO₂NHMe | |
| 10-81 | Ph | H | Et | NH₂ | Me | SO₂NHMe | |
| 10-82 | Ph | H | Et | NH₂ | Et | SO₂NHMe | |
| 10-83 | Ph | H | Et | NH₂ | i-Pr | SO₂NHMe | |
| 10-84 | 2-F-Ph | H | Et | NH₂ | Me | SO₂NHMe | |
| 10-85 | 2-F-Ph | H | Et | NH₂ | Et | SO₂NHMe | |
| 10-86 | 2-F-Ph | H | Et | NH₂ | i-Pr | SO₂NHMe | |
| 10-87 | 3-F-Ph | H | Et | NH₂ | Me | SO₂NHMe | |
| 10-88 | 3-F-Ph | H | Et | NH₂ | Et | SO₂NHMe | |
| 10-89 | 3-F-Ph | H | Et | NH₂ | i-Pr | SO₂NHMe | |
| 10-90 | 4-F-Ph | H | Et | NH₂ | Me | SO₂NHMe | |
| 10-91 | 4-F-Ph | H | Et | NH₂ | Et | SO₂NHMe | |
| 10-92 | 4-F-Ph | H | Et | NH₂ | i-Pr | SO₂NHMe | |
| 10-93 | 2,6-F₂-Ph | H | Et | NH₂ | Me | SO₂NHMe | |
| 10-94 | 2,6-F₂-Ph | H | Et | NH₂ | Et | SO₂NHMe | |
| 10-95 | 2,6-F₂-Ph | H | Et | NH₂ | i-Pr | SO₂NHMe | |
| 10-96 | Ph | H | Me | NH₂ | Me | SO₂NHEt | |
| 10-97 | Ph | H | Me | NH₂ | Et | SO₂NHEt | |
| 10-98 | Ph | H | Me | NH₂ | i-Pr | SO₂NHEt | |
| 10-99 | 2-F-Ph | H | Me | NH₂ | Me | SO₂NHEt | |
| 10-100 | 2-F-Ph | H | Me | NH₂ | Et | SO₂NHEt | |
| 10-101 | 2-F-Ph | H | Me | NH₂ | i-Pr | SO₂NHEt | |
| 10-102 | 3-F-Ph | H | Me | NH₂ | Me | SO₂NHEt | |
| 10-103 | 3-F-Ph | H | Me | NH₂ | Et | SO₂NHEt | |
| 10-104 | 3-F-Ph | H | Me | NH₂ | i-Pr | SO₂NHEt | |
| 10-105 | 4-F-Ph | H | Me | NH₂ | Me | SO₂NHEt | |
| 10-106 | 4-F-Ph | H | Me | NH₂ | Et | SO₂NHEt | |

**[Table 10-5]**

| Compound No. | R¹ | R² | R³ | R⁴ | R⁵ | A | Physical Property value |
|---|---|---|---|---|---|---|---|
| 10-107 | 4-F-Ph | H | Me | NH₂ | i-Pr | SO₂NHEt | |
| 10-108 | 2,6-F₂-Ph | H | Me | NH₂ | Me | SO₂NHEt | |
| 10-109 | 2,6-F₂-Ph | H | Me | NH₂ | Et | SO₂NHEt | |
| 10-110 | 2,6-F₂-Ph | H | Me | NH₂ | i-Pr | SO₂NHEt | |
| 10-111 | thiazol-5-yl | H | Me | NH₂ | Me | SO₂NHEt | |
| 10-112 | thiazol-5-yl | H | Me | NH₂ | Et | SO₂NHEt | |
| 10-113 | thiazol-5-yl | H | Me | NH₂ | i-Pr | SO₂NHEt | |
| 10-114 | thiazol-4-yl | H | Me | NH₂ | Me | SO₂NHEt | |
| 10-115 | thiazol-4-yl | H | Me | NH₂ | Et | SO₂NHEt | |
| 10-116 | thiazol-4-yl | H | Me | NH₂ | i-Pr | SO₂NHEt | |
| 10-117 | thiazol-2-yl | H | Me | NH₂ | Me | SO₂NHEt | |
| 10-118 | thiazol-2-yl | H | Me | NH₂ | Et | SO₂NHEt | |
| 10-119 | thiazol-2-yl | H | Me | NH₂ | i-Pr | SO₂NHEt | |
| 10-120 | thiophen-2-yl | H | Me | NH₂ | Me | SO₂NHEt | |
| 10-121 | thiophen-2-yl | H | Me | NH₂ | Et | SO₂NHEt | |
| 10-122 | thiophen-2-yl | H | Me | NH₂ | i-Pr | SO₂NHEt | |
| 10-123 | thiophen-3-yl | H | Me | NH₂ | Me | SO₂NHEt | |
| 10-124 | thiophen-3-yl | H | Me | NH₂ | Et | SO₂NHEt | |
| 10-125 | thiophen-3-yl | H | Me | NH₂ | i-Pr | SO₂NHEt | |
| 10-126 | pyridazin-3-yl | H | Me | NH₂ | Me | SO₂NHEt | |
| 10-127 | pyridazin-3-yl | H | Me | NH₂ | Et | SO₂NHEt | |
| 10-128 | pyridazin-3-yl | H | Me | NH₂ | i-Pr | SO₂NHEt | |
| 10-129 | pyrazin-2-yl | H | Me | NH₂ | Me | SO₂NHEt | |
| 10-130 | pyrazin-2-yl | H | Me | NH₂ | Et | SO₂NHEt | |
| 10-131 | pyrazin-2-yl | H | Me | NH₂ | i-Pr | SO₂NHEt | |
| 10-132 | pyrimidin-2-yl | H | Me | NH₂ | Me | SO₂NHEt | |
| 10-133 | pyrimidin-2-yl | H | Me | NH₂ | Et | SO₂NHEt | |
| 10-134 | pyrimidin-2-yl | H | Me | NH₂ | i-Pr | SO₂NHEt | |
| 10-135 | pyrimidin-4-yl | H | Me | NH₂ | Me | SO₂NHEt | |
| 10-136 | pyrimidin-4-yl | H | Me | NH₂ | Et | SO₂NHEt | |

**[Table 10-6]**

| Compound No. | R¹ | R² | R³ | R⁴ | R⁵ | A | Physical Property value |
|---|---|---|---|---|---|---|---|
| 10-137 | pyrimidin-4-yl | H | Me | NH₂ | i-Pr | SO₂NHEt | |
| 10-138 | pyrimidin-5-yl | H | Me | NH₂ | Me | SO₂NHEt | |
| 10-139 | pyrimidin-5-yl | H | Me | NH₂ | Et | SO₂NHEt | |
| 10-140 | pyrimidin-5-yl | H | Me | NH₂ | i-Pr | SO₂NHEt | |

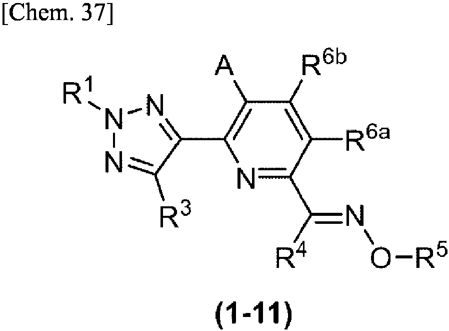

Table 11 (In Table 11, R^{6a} and R^{6b} represent hydrogen atoms.)

**[Table 11-1]**

| Compound No. | R¹ | R³ | R⁴ | R⁵ | A | Physical Property value |
|---|---|---|---|---|---|---|
| 11-1 | Ph | Me | NH₂ | Me | SO₂NHMe | |
| 11-2 | Ph | Me | NH₂ | Et | SO₂NHMe | NMR |
| 11-3 | Ph | Me | NH₂ | i-Pr | SO₂NHMe | |
| 11-4 | 2-F-Ph | Me | NH₂ | Me | SO₂NHMe | |
| 11-5 | 2-F-Ph | Me | NH₂ | Et | SO₂NHMe | |
| 11-6 | 2-F-Ph | Me | NH₂ | i-Pr | SO₂NHMe | |
| 11-7 | 3-F-Ph | Me | NH₂ | Me | SO₂NHMe | |
| 11-8 | 3-F-Ph | Me | NH₂ | Et | SO₂NHMe | |
| 11-9 | 3-F-Ph | Me | NH₂ | i-Pr | SO₂NHMe | |
| 11-10 | 4-F-Ph | Me | NH₂ | Me | SO₂NHMe | |
| 11-11 | 4-F-Ph | Me | NH₂ | Et | SO₂NHMe | |
| 11-12 | 4-F-Ph | Me | NH₂ | i-Pr | SO₂NHMe | |
| 11-13 | 2,6-F₂-Ph | Me | NH₂ | Me | SO₂NHMe | |
| 11-14 | 2,6-F₂-Ph | Me | NH₂ | Et | SO₂NHMe | |
| 11-15 | 2,6-F₂-Ph | Me | NH₂ | i-Pr | SO₂NHMe | |

**[Table 11-2]**

| Compound No. | R¹ | R³ | R⁴ | R⁵ | A | Physical Property value |
|---|---|---|---|---|---|---|
| 11-16 | thiazol-5-yl | Me | NH₂ | Me | SO₂NHMe | |
| 11-17 | thiazol-5-yl | Me | NH₂ | Et | SO₂NHMe | |
| 11-18 | thiazol-5-yl | Me | NH₂ | i-Pr | SO₂NHMe | |
| 11-19 | thiazol-4-yl | Me | NH₂ | Me | SO₂NHMe | |
| 11-20 | thiazol-4-yl | Me | NH₂ | Et | SO₂NHMe | |
| 11-21 | thiazol-4-yl | Me | NH₂ | i-Pr | SO₂NHMe | |
| 11-22 | thiazol-2-yl | Me | NH₂ | Me | SO₂NHMe | |
| 11-23 | thiazol-2-yl | Me | NH₂ | Et | SO₂NHMe | |
| 11-24 | thiazol-2-yl | Me | NH₂ | i-Pr | SO₂NHMe | |
| 11-25 | thiophen-2-yl | Me | NH₂ | Me | SO₂NHMe | |
| 11-26 | thiophen-2-yl | Me | NH₂ | Et | SO₂NHMe | |
| 11-27 | thiophen-2-yl | Me | NH₂ | i-Pr | SO₂NHMe | |
| 11-28 | thiophen-3-yl | Me | NH₂ | Me | SO₂NHMe | |
| 11-29 | thiophen-3-yl | Me | NH₂ | Et | SO₂NHMe | |
| 11-30 | thiophen-3-yl | Me | NH₂ | i-Pr | SO₂NHMe | |
| 11-31 | pyridazin-3-yl | Me | NH₂ | Me | SO₂NHMe | |
| 11-32 | pyridazin-3-yl | Me | NH₂ | Et | SO₂NHMe | |
| 11-33 | pyridazin-3-yl | Me | NH₂ | i-Pr | SO₂NHMe | |
| 11-34 | pyrazin-2-yl | Me | NH₂ | Me | SO₂NHMe | |
| 11-35 | pyrazin-2-yl | Me | NH₂ | Et | SO₂NHMe | |
| 11-36 | pyrazin-2-yl | Me | NH₂ | i-Pr | SO₂NHMe | |
| 11-37 | pyrimidin-2-yl | Me | NH₂ | Me | SO₂NHMe | |
| 11-38 | pyrimidin-2-yl | Me | NH₂ | Et | SO₂NHMe | |
| 11-39 | pyrimidin-2-yl | Me | NH₂ | i-Pr | SO₂NHMe | |
| 11-40 | pyrimidin-4-yl | Me | NH₂ | Me | SO₂NHMe | |
| 11-41 | pyrimidin-4-yl | Me | NH₂ | Et | SO₂NHMe | |
| 11-42 | pyrimidin-4-yl | Me | NH₂ | i-Pr | SO₂NHMe | |
| 11-43 | pyrimidin-5-yl | Me | NH₂ | Me | SO₂NHMe | |
| 11-44 | pyrimidin-5-yl | Me | NH₂ | Et | SO₂NHMe | |
| 11-45 | pyrimidin-5-yl | Me | NH₂ | i-Pr | SO₂NHMe | |

**[Table 11-3]**

| Compound No. | R¹ | R³ | R⁴ | R⁵ | A | Physical Property value |
|---|---|---|---|---|---|---|
| 11-46 | thiazol-5-yl | Et | NH₂ | Me | SO₂NHMe | |
| 11-47 | thiazol-5-yl | Et | NH₂ | Et | SO₂NHMe | |
| 11-48 | thiazol-5-yl | Et | NH₂ | i-Pr | SO₂NHMe | |
| 11-49 | thiazol-4-yl | Et | NH₂ | Me | SO₂NHMe | |
| 11-50 | thiazol-4-yl | Et | NH₂ | Et | SO₂NHMe | |
| 11-51 | thiazol-4-yl | Et | NH₂ | i-Pr | SO₂NHMe | |
| 11-52 | thiazol-2-yl | Et | NH₂ | Me | SO₂NHMe | |
| 11-53 | thiazol-2-yl | Et | NH₂ | Et | SO₂NHMe | |
| 11-54 | thiazol-2-yl | Et | NH₂ | i-Pr | SO₂NHMe | |
| 11-55 | thiophen-2-yl | Et | NH₂ | Me | SO₂NHMe | |
| 11-56 | thiophen-2-yl | Et | NH₂ | Et | SO₂NHMe | |
| 11-57 | thiophen-2-yl | Et | NH₂ | i-Pr | SO₂NHMe | |
| 11-58 | thiophen-3-yl | Et | NH₂ | Me | SO₂NHMe | |
| 11-59 | thiophen-3-yl | Et | NH₂ | Et | SO₂NHMe | |
| 11-60 | thiophen-3-yl | Et | NH₂ | i-Pr | SO₂NHMe | |
| 11-61 | pyridazin-3-yl | Et | NH₂ | Me | SO₂NHMe | |
| 11-62 | pyridazin-3-yl | Et | NH₂ | Et | SO₂NHMe | |
| 11-63 | pyridazin-3-yl | Et | NH₂ | i-Pr | SO₂NHMe | |
| 11-64 | pyrazin-2-yl | Et | NH₂ | Me | SO₂NHMe | |
| 11-65 | pyrazin-2-yl | Et | NH₂ | Et | SO₂NHMe | |
| 11-66 | pyrazin-2-yl | Et | NH₂ | i-Pr | SO₂NHMe | |
| 11-67 | pyrimidin-2-yl | Et | NH₂ | Me | SO₂NHMe | |
| 11-68 | pyrimidin-2-yl | Et | NH₂ | Et | SO₂NHMe | |
| 11-69 | pyrimidin-2-yl | Et | NH₂ | i-Pr | SO₂NHMe | |
| 11-70 | pyrimidin-4-yl | Et | NH₂ | Me | SO₂NHMe | |
| 11-71 | pyrimidin-4-yl | Et | NH₂ | Et | SO₂NHMe | |
| 11-72 | pyrimidin-4-yl | Et | NH₂ | i-Pr | SO₂NHMe | |
| 11-73 | pyrimidin-5-yl | Et | NH₂ | Me | SO₂NHMe | |
| 11-74 | pyrimidin-5-yl | Et | NH₂ | Et | SO₂NHMe | |
| 11-75 | pyrimidin-5-yl | Et | NH₂ | i-Pr | SO₂NHMe | |

**[Table 11-4]**

| Compound No. | R¹ | R³ | R⁴ | R⁵ | A | Physical Property value |
|---|---|---|---|---|---|---|
| 11-76 | Ph | Et | NH₂ | Me | SO₂NHMe | |
| 11-77 | Ph | Et | NH₂ | Et | SO₂NHMe | |
| 11-78 | Ph | Et | NH₂ | i-Pr | SO₂NHMe | |
| 11-79 | 2-F-Ph | Et | NH₂ | Me | SO₂NHMe | |
| 11-80 | 2-F-Ph | Et | NH₂ | Et | SO₂NHMe | |
| 11-81 | 2-F-Ph | Et | NH₂ | i-Pr | SO₂NHMe | |
| 11-82 | 3-F-Ph | Et | NH₂ | Me | SO₂NHMe | |
| 11-83 | 3-F-Ph | Et | NH₂ | Et | SO₂NHMe | |
| 11-84 | 3-F-Ph | Et | NH₂ | i-Pr | SO₂NHMe | |
| 11-85 | 4-F-Ph | Et | NH₂ | Me | SO₂NHMe | |
| 11-86 | 4-F-Ph | Et | NH₂ | Et | SO₂NHMe | |
| 11-87 | 4-F-Ph | Et | NH₂ | i-Pr | SO₂NHMe | |
| 11-88 | 2,6-F₂-Ph | Et | NH₂ | Me | SO₂NHMe | |
| 11-89 | 2,6-F₂-Ph | Et | NH₂ | Et | SO₂NHMe | |
| 11-90 | 2,6-F₂-Ph | Et | NH₂ | i-Pr | SO₂NHMe | |
| 11-91 | pyrimidin-2-yl | Me | NH₂ | Me | SO₂Me | |
| 11-92 | pyrimidin-2-yl | Et | NH₂ | Me | SO₂Me | |
| 11-93 | pyrimidin-2-yl | Me | NH₂ | Et | SO₂Me | 241-246 |
| 11-94 | 2-NO₂-Ph | Me | NH₂ | Me | SO₂Me | |
| 11-95 | 2-NO₂-Ph | Me | NH₂ | Et | SO₂Me | 179-184 |
| 11-96 | 2-NO₂-Ph | Me | NH₂ | Me | SO₂NHMe | |
| 11-97 | 2-NO₂-Ph | Me | NH₂ | Et | SO₂NHMe | |
| 11-98 | 2-NO₂-Ph | Et | NH₂ | Me | SO₂Me | |
| 11-99 | 2-NO₂-Ph | Et | NH₂ | Et | SO₂Me | |
| 11-100 | 2-NO₂-Ph | Et | NH₂ | Me | SO₂NHMe | |
| 11-101 | 2-NO₂-Ph | Et | NH₂ | Et | SO₂NHMe | |
| 11-102 | 2-NH₂-Ph | Me | NH₂ | Me | SO₂Me | |
| 11-103 | 2-NH₂-Ph | Me | NH₂ | Et | SO₂Me | 38-43 |
| 11-104 | 2-NH₂-Ph | Me | NH₂ | Me | SO₂NHMe | |
| 11-105 | 2-NH₂-Ph | Me | NH₂ | Et | SO₂NHMe | |

**[Table 11-5]**

| Compound No. | R¹ | R³ | R⁴ | R⁵ | A | Physical Property value |
|---|---|---|---|---|---|---|
| 11-106 | 2-NH₂-Ph | Et | NH₂ | Me | SO₂Me | |
| 11-107 | 2-NH₂-Ph | Et | NH₂ | Et | SO₂Me | |
| 11-108 | 2-NH₂-Ph | Et | NH₂ | Me | SO₂NHMe | |
| 11-109 | 2-NH₂-Ph | Et | NH₂ | Et | SO₂NHMe | |
| 11-110 | 2-F-Ph | Me | NH₂ | Me | SO₂Me | |
| 11-111 | 2-F-Ph | Et | NH₂ | Me | SO₂Me | |
| 11-112 | 2-F-Ph | Me | NH₂ | Et | SO₂Me | |
| 11-113 | 3-F-pyridin-2-yl | Me | NH₂ | Me | SO₂Me | |
| 11-114 | 3-F-pyridin-2-yl | Me | NH₂ | Et | SO₂Me | |
| 11-115 | 3-F-pyridin-2-yl | Me | NH₂ | Me | SO₂NHMe | |
| 11-116 | 3-F-pyridin-2-yl | Me | NH₂ | Et | SO₂NHMe | |
| 11-117 | 3-F-pyridin-2-yl | Et | NH₂ | Me | SO₂Me | |
| 11-118 | 3-F-pyridin-2-yl | Et | NH₂ | Et | SO₂Me | |
| 11-119 | 3-F-pyridin-2-yl | Et | NH₂ | Me | SO₂NHMe | |
| 11-120 | 3-F-pyridin-2-yl | Et | NH₂ | Et | SO₂NHMe | |
| 11-121 | 3-NO₂-pyridin-2-yl | Me | NH₂ | Me | SO₂Me | |
| 11-122 | 3-NO₂-pyridin-2-yl | Me | NH₂ | Et | SO₂Me | NMR |
| 11-123 | 3-NO₂-pyridin-2-yl | Me | NH₂ | Me | SO₂NHMe | |
| 11-124 | 3-NO₂-pyridin-2-yl | Me | NH₂ | Et | SO₂NHMe | |
| 11-125 | 3-NO₂-pyridin-2-yl | Et | NH₂ | Me | SO₂Me | |
| 11-126 | 3-NO₂-pyridin-2-yl | Et | NH₂ | Et | SO₂Me | |
| 11-127 | 3-NO₂-pyridin-2-yl | Et | NH₂ | Me | SO₂NHMe | |
| 11-128 | 3-NO₂-pyridin-2-yl | Et | NH₂ | Et | SO₂NHMe | |
| 11-129 | 3-NH₂-pyridin-2-yl | Me | NH₂ | Me | SO₂Me | |
| 11-130 | 3-NH₂-pyridin-2-yl | Me | NH₂ | Et | SO₂Me | 193-198 |
| 11-131 | 3-NH₂-pyridin-2-yl | Me | NH₂ | Me | SO₂NHMe | |
| 11-132 | 3-NH₂-pyridin-2-yl | Me | NH₂ | Et | SO₂NHMe | |
| 11-133 | 3-NH₂-pyridin-2-yl | Et | NH₂ | Me | SO₂Me | |
| 11-134 | 3-NH₂-pyridin-2-yl | Et | NH₂ | Et | SO₂Me | |
| 11-135 | 3-NH₂-pyridin-2-yl | Et | NH₂ | Me | SO₂NHMe | |

**[Table 11-6]**

| Compound No. | R¹ | R³ | R⁴ | R⁵ | A | Physical Property value |
|---|---|---|---|---|---|---|
| 11-136 | 3-NH₂-pyridin-2-yl | Et | NH₂ | Et | SO₂NHMe | |
| 11-137 | Me | Me | NH₂ | Me | SO₂Me | |
| 11-138 | Me | Me | NH₂ | Et | SO₂Me | |
| 11-139 | Me | Me | NH₂ | Me | SO₂NHMe | |
| 11-140 | Me | Me | NH₂ | Et | SO₂NHMe | |
| 11-141 | Me | Et | NH₂ | Me | SO₂Me | |
| 11-142 | Me | Et | NH₂ | Et | SO₂Me | |
| 11-143 | Me | Et | NH₂ | Me | SO₂NHMe | |
| 11-144 | Me | Et | NH₂ | Et | SO₂NHMe | |
| 11-145 | Et | Me | NH₂ | Me | SO₂Me | |
| 11-146 | Et | Me | NH₂ | Et | SO₂Me | |
| 11-147 | Et | Me | NH₂ | Me | SO₂NHMe | |
| 11-148 | Et | Me | NH₂ | Et | SO₂NHMe | |
| 11-149 | Et | Et | NH₂ | Me | SO₂Me | |
| 11-150 | Et | Et | NH₂ | Et | SO₂Me | |
| 11-151 | Et | Et | NH₂ | Me | SO₂NHMe | |
| 11-152 | Et | Et | NH₂ | Et | SO₂NHMe | |
| 11-153 | n-Pr | Me | NH₂ | Me | SO₂Me | |
| 11-154 | n-Pr | Me | NH₂ | Et | SO₂Me | |
| 11-155 | n-Pr | Me | NH₂ | Me | SO₂NHMe | |
| 11-156 | n-Pr | Me | NH₂ | Et | SO₂NHMe | |
| 11-157 | n-Pr | Et | NH₂ | Me | SO₂Me | |
| 11-158 | n-Pr | Et | NH₂ | Et | SO₂Me | |
| 11-159 | n-Pr | Et | NH₂ | Me | SO₂NHMe | |
| 11-160 | n-Pr | Et | NH₂ | Et | SO₂NHMe | |
| 11-161 | n-Bu | Me | NH₂ | Me | SO₂Me | |
| 11-162 | n-Bu | Me | NH₂ | Et | SO₂Me | |
| 11-163 | n-Bu | Me | NH₂ | Me | SO₂NHMe | |
| 11-164 | n-Bu | Me | NH₂ | Et | SO₂NHMe | |
| 11-165 | n-Bu | Et | NH₂ | Me | SO₂Me | |

**[Table 11-7]**

| Compound No. | R¹ | R³ | R⁴ | R⁵ | A | Physical Property value |
|---|---|---|---|---|---|---|
| 11-166 | n-Bu | Et | NH₂ | Et | SO₂Me | |
| 11-167 | n-Bu | Et | NH₂ | Me | SO₂NHMe | |
| 11-168 | n-Bu | Et | NH₂ | Et | SO₂NHMe | |
| 11-169 | Ph | Me | NH₂ | Me | SO₂NHEt | |
| 11-170 | Ph | Me | NH₂ | Et | SO₂NHEt | |
| 11-171 | Ph | Me | NH₂ | i-Pr | SO₂NHEt | |
| 11-172 | 2-F-Ph | Me | NH₂ | Me | SO₂NHEt | |
| 11-173 | 2-F-Ph | Me | NH₂ | Et | SO₂NHEt | |
| 11-174 | 2-F-Ph | Me | NH₂ | i-Pr | SO₂NHEt | |
| 11-175 | 3-F-Ph | Me | NH₂ | Me | SO₂NHEt | |
| 11-176 | 3-F-Ph | Me | NH₂ | Et | SO₂NHEt | |
| 11-177 | 3-F-Ph | Me | NH₂ | i-Pr | SO₂NHEt | |
| 11-178 | 4-F-Ph | Me | NH₂ | Me | SO₂NHEt | |
| 11-179 | 4-F-Ph | Me | NH₂ | Et | SO₂NHEt | |
| 11-180 | 4-F-Ph | Me | NH₂ | i-Pr | SO₂NHEt | |
| 11-181 | 2,6-F₂-Ph | Me | NH₂ | Me | SO₂NHEt | |
| 11-182 | 2,6-F₂-Ph | Me | NH₂ | Et | SO₂NHEt | |
| 11-183 | 2,6-F₂-Ph | Me | NH₂ | i-Pr | SO₂NHEt | |
| 11-184 | thiazol-5-yl | Me | NH₂ | Me | SO₂NHEt | |
| 11-185 | thiazol-5-yl | Me | NH₂ | Et | SO₂NHEt | |
| 11-186 | thiazol-5-yl | Me | NH₂ | i-Pr | SO₂NHEt | |
| 11-187 | thiazol-4-yl | Me | NH₂ | Me | SO₂NHEt | |
| 11-188 | thiazol-4-yl | Me | NH₂ | Et | SO₂NHEt | |
| 11-189 | thiazol-4-yl | Me | NH₂ | i-Pr | SO₂NHEt | |
| 11-190 | thiazol-2-yl | Me | NH₂ | Me | SO₂NHEt | |
| 11-191 | thiazol-2-yl | Me | NH₂ | Et | SO₂NHEt | |
| 11-192 | thiazol-2-yl | Me | NH₂ | i-Pr | SO₂NHEt | |
| 11-193 | thiophen-2-yl | Me | NH₂ | Me | SO₂NHEt | |
| 11-194 | thiophen-2-yl | Me | NH₂ | Et | SO₂NHEt | |
| 11-195 | thiophen-2-yl | Me | NH₂ | i-Pr | SO₂NHEt | |
| 11-196 | thiophen-3-yl | Me | NH₂ | Me | SO₂NHEt | |

**[Table 11-8]**

| Compound No. | R¹ | R³ | R⁴ | R⁵ | A | Physical Property value |
|---|---|---|---|---|---|---|
| 11-197 | thiophen-3-yl | Me | NH₂ | Et | SO₂NHEt | |
| 11-198 | thiophen-3-yl | Me | NH₂ | i-Pr | SO₂NHEt | |
| 11-199 | pyridazin-3-yl | Me | NH₂ | Me | SO₂NHEt | |
| 11-200 | pyridazin-3-yl | Me | NH₂ | Et | SO₂NHEt | |
| 11-201 | pyridazin-3-yl | Me | NH₂ | i-Pr | SO₂NHEt | |
| 11-202 | pyrazin-2-yl | Me | NH₂ | Me | SO₂NHEt | |
| 11-203 | pyrazin-2-yl | Me | NH₂ | Et | SO₂NHEt | |
| 11-204 | pyrazin-2-yl | Me | NH₂ | i-Pr | SO₂NHEt | |
| 11-205 | pyrimidin-2-yl | Me | NH₂ | Me | SO₂NHEt | |
| 11-206 | pyrimidin-2-yl | Me | NH₂ | Et | SO₂NHEt | |
| 11-207 | pyrimidin-2-yl | Me | NH₂ | i-Pr | SO₂NHEt | |
| 11-208 | pyrimidin-4-yl | Me | NH₂ | Me | SO₂NHEt | |
| 11-209 | pyrimidin-4-yl | Me | NH₂ | Et | SO₂NHEt | |
| 11-210 | pyrimidin-4-yl | Me | NH₂ | i-Pr | SO₂NHEt | |
| 11-211 | pyrimidin-5-yl | Me | NH₂ | Me | SO₂NHEt | |
| 11-212 | pyrimidin-5-yl | Me | NH₂ | Et | SO₂NHEt | |
| 11-213 | pyrimidin-5-yl | Me | NH₂ | i-Pr | SO₂NHEt | |
| 11-214 | Ph | Me | NH₂ | Et | SO₂Me | 36-41 |
| 11-215 | i-Bu | Me | NH₂ | Et | SO₂Me | 54-59 |

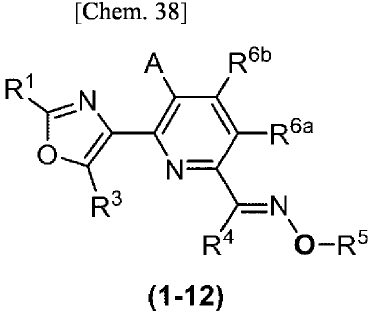

Table 12 (In Table 12, R^{6a} and R^{6b} represent hydrogen atoms.)

**[Table 12-1]**

| Compound No. | R¹ | R³ | R⁴ | R⁵ | A | Physical Property value |
|---|---|---|---|---|---|---|
| 12-1 | Ph | Me | NH₂ | Me | SO₂NHMe | |
| 12-2 | Ph | Me | NH₂ | Et | SO₂NHMe | 163-164 |
| 12-3 | Ph | Me | NH₂ | i-Pr | SO₂NHMe | |
| 12-4 | 2-F-Ph | Me | NH₂ | Me | SO₂NHMe | |
| 12-5 | 2-F-Ph | Me | NH₂ | Et | SO₂NHMe | |
| 12-6 | 2-F-Ph | Me | NH₂ | i-Pr | SO₂NHMe | |
| 12-7 | 3-F-Ph | Me | NH₂ | Me | SO₂NHMe | |
| 12-8 | 3-F-Ph | Me | NH₂ | Et | SO₂NHMe | |
| 12-9 | 3-F-Ph | Me | NH₂ | i-Pr | SO₂NHMe | |
| 12-10 | 4-F-Ph | Me | NH₂ | Me | SO₂NHMe | |
| 12-11 | 4-F-Ph | Me | NH₂ | Et | SO₂NHMe | |
| 12-12 | 4-F-Ph | Me | NH₂ | i-Pr | SO₂NHMe | |
| 12-13 | 2,6-F₂-Ph | Me | NH₂ | Me | SO₂NHMe | |
| 12-14 | 2,6-F₂-Ph | Me | NH₂ | Et | SO₂NHMe | |
| 12-15 | 2,6-F₂-Ph | Me | NH₂ | i-Pr | SO₂NHMe | |

**[Table 12-2]**

| Compound No. | R¹ | R³ | R⁴ | R⁵ | A | Physical Property value |
|---|---|---|---|---|---|---|
| 12-16 | thiazol-5-yl | Me | NH₂ | Me | SO₂NHMe | |
| 12-17 | thiazol-5-yl | Me | NH₂ | Et | SO₂NHMe | |
| 12-18 | thiazol-5-yl | Me | NH₂ | i-Pr | SO₂NHMe | |
| 12-19 | thiazol-4-yl | Me | NH₂ | Me | SO₂NHMe | |
| 12-20 | thiazol-4-yl | Me | NH₂ | Et | SO₂NHMe | |
| 12-21 | thiazol-4-yl | Me | NH₂ | i-Pr | SO₂NHMe | |
| 12-22 | thiazol-2-yl | Me | NH₂ | Me | SO₂NHMe | |
| 12-23 | thiazol-2-yl | Me | NH₂ | Et | SO₂NHMe | |
| 12-24 | thiazol-2-yl | Me | NH₂ | i-Pr | SO₂NHMe | |
| 12-25 | thiophen-2-yl | Me | NH₂ | Me | SO₂NHMe | |
| 12-26 | thiophen-2-yl | Me | NH₂ | Et | SO₂NHMe | |
| 12-27 | thiophen-2-yl | Me | NH₂ | i-Pr | SO₂NHMe | |
| 12-28 | thiophen-3-yl | Me | NH₂ | Me | SO₂NHMe | |
| 12-29 | thiophen-3-yl | Me | NH₂ | Et | SO₂NHMe | |
| 12-30 | thiophen-3-yl | Me | NH₂ | i-Pr | SO₂NHMe | |
| 12-31 | pyridazin-3-yl | Me | NH₂ | Me | SO₂NHMe | |
| 12-32 | pyridazin-3-yl | Me | NH₂ | Et | SO₂NHMe | |
| 12-33 | pyridazin-3-yl | Me | NH₂ | i-Pr | SO₂NHMe | |
| 12-34 | pyrazin-2-yl | Me | NH₂ | Me | SO₂NHMe | |
| 12-35 | pyrazin-2-yl | Me | NH₂ | Et | SO₂NHMe | |
| 12-36 | pyrazin-2-yl | Me | NH₂ | i-Pr | SO₂NHMe | |
| 12-37 | pyrimidin-2-yl | Me | NH₂ | Me | SO₂NHMe | |
| 12-38 | pyrimidin-2-yl | Me | NH₂ | Et | SO₂NHMe | |
| 12-39 | pyrimidin-2-yl | Me | NH₂ | i-Pr | SO₂NHMe | |
| 12-40 | pyrimidin-4-yl | Me | NH₂ | Me | SO₂NHMe | |
| 12-41 | pyrimidin-4-yl | Me | NH₂ | Et | SO₂NHMe | |
| 12-42 | pyrimidin-4-yl | Me | NH₂ | i-Pr | SO₂NHMe | |
| 12-43 | pyrimidin-5-yl | Me | NH₂ | Me | SO₂NHMe | |
| 12-44 | pyrimidin-5-yl | Me | NH₂ | Et | SO₂NHMe | |
| 12-45 | pyrimidin-5-yl | Me | NH₂ | i-Pr | SO₂NHMe | |

**[Table 12-3]**

| Compound No. | R¹ | R³ | R⁴ | R⁵ | A | Physical Property value |
|---|---|---|---|---|---|---|
| 12-46 | thiazol-5-yl | Et | NH₂ | Me | SO₂NHMe | |
| 12-47 | thiazol-5-yl | Et | NH₂ | Et | SO₂NHMe | |
| 12-48 | thiazol-5-yl | Et | NH₂ | i-Pr | SO₂NHMe | |
| 12-49 | thiazol-4-yl | Et | NH₂ | Me | SO₂NHMe | |
| 12-50 | thiazol-4-yl | Et | NH₂ | Et | SO₂NHMe | |
| 12-51 | thiazol-4-yl | Et | NH₂ | i-Pr | SO₂NHMe | |
| 12-52 | thiazol-2-yl | Et | NH₂ | Me | SO₂NHMe | |
| 12-53 | thiazol-2-yl | Et | NH₂ | Et | SO₂NHMe | |
| 12-54 | thiazol-2-yl | Et | NH₂ | i-Pr | SO₂NHMe | |
| 12-55 | thiophen-2-yl | Et | NH₂ | Me | SO₂NHMe | |
| 12-56 | thiophen-2-yl | Et | NH₂ | Et | SO₂NHMe | |
| 12-57 | thiophen-2-yl | Et | NH₂ | i-Pr | SO₂NHMe | |
| 12-58 | thiophen-3-yl | Et | NH₂ | Me | SO₂NHMe | |
| 12-59 | thiophen-3-yl | Et | NH₂ | Et | SO₂NHMe | |
| 12-60 | thiophen-3-yl | Et | NH₂ | i-Pr | SO₂NHMe | |
| 12-61 | pyridazin-3-yl | Et | NH₂ | Me | SO₂NHMe | |
| 12-62 | pyridazin-3-yl | Et | NH₂ | Et | SO₂NHMe | |
| 12-63 | pyridazin-3-yl | Et | NH₂ | i-Pr | SO₂NHMe | |
| 12-64 | pyrazin-2-yl | Et | NH₂ | Me | SO₂NHMe | |
| 12-65 | pyrazin-2-yl | Et | NH₂ | Et | SO₂NHMe | |
| 12-66 | pyrazin-2-yl | Et | NH₂ | i-Pr | SO₂NHMe | |
| 12-67 | pyrimidin-2-yl | Et | NH₂ | Me | SO₂NHMe | |
| 12-68 | pyrimidin-2-yl | Et | NH₂ | Et | SO₂NHMe | |
| 12-69 | pyrimidin-2-yl | Et | NH₂ | i-Pr | SO₂NHMe | |
| 12-70 | pyrimidin-4-yl | Et | NH₂ | Me | SO₂NHMe | |
| 12-71 | pyrimidin-4-yl | Et | NH₂ | Et | SO₂NHMe | |
| 12-72 | pyrimidin-4-yl | Et | NH₂ | i-Pr | SO₂NHMe | |
| 12-73 | pyrimidin-5-yl | Et | NH₂ | Me | SO₂NHMe | |
| 12-74 | pyrimidin-5-yl | Et | NH₂ | Et | SO₂NHMe | |
| 12-75 | pyrimidin-5-yl | Et | NH₂ | i-Pr | SO₂NHMe | |

**[Table 12-4]**

| Compound No. | R¹ | R³ | R⁴ | R⁵ | A | Physical Property value |
|---|---|---|---|---|---|---|
| 12-76 | Ph | Et | NH₂ | Me | SO₂NHMe | |
| 12-77 | Ph | Et | NH₂ | Et | SO₂NHMe | |
| 12-78 | Ph | Et | NH₂ | i-Pr | SO₂NHMe | |
| 12-79 | 2-F-Ph | Et | NH₂ | Me | SO₂NHMe | |
| 12-80 | 2-F-Ph | Et | NH₂ | Et | SO₂NHMe | |
| 12-81 | 2-F-Ph | Et | NH₂ | i-Pr | SO₂NHMe | |
| 12-82 | 3-F-Ph | Et | NH₂ | Me | SO₂NHMe | |
| 12-83 | 3-F-Ph | Et | NH₂ | Et | SO₂NHMe | |
| 12-84 | 3-F-Ph | Et | NH₂ | i-Pr | SO₂NHMe | |
| 12-85 | 4-F-Ph | Et | NH₂ | Me | SO₂NHMe | |
| 12-86 | 4-F-Ph | Et | NH₂ | Et | SO₂NHMe | |
| 12-87 | 4-F-Ph | Et | NH₂ | i-Pr | SO₂NHMe | |
| 12-88 | 2,6-F₂-Ph | Et | NH₂ | Me | SO₂NHMe | |
| 12-89 | 2,6-F₂-Ph | Et | NH₂ | Et | SO₂NHMe | |
| 12-90 | 2,6-F₂-Ph | Et | NH₂ | i-Pr | SO₂NHMe | |
| 12-91 | Ph | Me | NH₂ | Me | SO₂NHEt | |
| 12-92 | Ph | Me | NH₂ | Et | SO₂NHEt | |
| 12-93 | Ph | Me | NH₂ | i-Pr | SO₂NHEt | |
| 12-94 | 2-F-Ph | Me | NH₂ | Me | SO₂NHEt | |
| 12-95 | 2-F-Ph | Me | NH₂ | Et | SO₂NHEt | |
| 12-96 | 2-F-Ph | Me | NH₂ | i-Pr | SO₂NHEt | |
| 12-97 | 3-F-Ph | Me | NH₂ | Me | SO₂NHEt | |
| 12-98 | 3-F-Ph | Me | NH₂ | Et | SO₂NHEt | |
| 12-99 | 3-F-Ph | Me | NH₂ | i-Pr | SO₂NHEt | |
| 12-100 | 4-F-Ph | Me | NH₂ | Me | SO₂NHEt | |
| 12-101 | 4-F-Ph | Me | NH₂ | Et | SO₂NHEt | |
| 12-102 | 4-F-Ph | Me | NH₂ | i-Pr | SO₂NHEt | |
| 12-103 | 2,6-F₂-Ph | Me | NH₂ | Me | SO₂NHEt | |
| 12-104 | 2,6-F₂-Ph | Me | NH₂ | Et | SO₂NHEt | |
| 12-105 | 2,6-F₂-Ph | Me | NH₂ | i-Pr | SO₂NHEt | |

**[Table 12-5]**

| Compound No. | R¹ | R³ | R⁴ | R⁵ | A | Physical Property value |
|---|---|---|---|---|---|---|
| 12-106 | thiazol-5-yl | Me | NH₂ | Me | SO₂NHEt | |
| 12-107 | thiazol-5-yl | Me | NH₂ | Et | SO₂NHEt | |
| 12-108 | thiazol-5-yl | Me | NH₂ | i-Pr | SO₂NHEt | |
| 12-109 | thiazol-4-yl | Me | NH₂ | Me | SO₂NHEt | |
| 12-110 | thiazol-4-yl | Me | NH₂ | Et | SO₂NHEt | |
| 12-111 | thiazol-4-yl | Me | NH₂ | i-Pr | SO₂NHEt | |
| 12-112 | thiazol-2-yl | Me | NH₂ | Me | SO₂NHEt | |
| 12-113 | thiazol-2-yl | Me | NH₂ | Et | SO₂NHEt | |
| 12-114 | thiazol-2-yl | Me | NH₂ | i-Pr | SO₂NHEt | |
| 12-115 | thiophen-2-yl | Me | NH₂ | Me | SO₂NHEt | |
| 12-116 | thiophen-2-yl | Me | NH₂ | Et | SO₂NHEt | |
| 12-117 | thiophen-2-yl | Me | NH₂ | i-Pr | SO₂NHEt | |
| 12-118 | thiophen-3-yl | Me | NH₂ | Me | SO₂NHEt | |
| 12-119 | thiophen-3-yl | Me | NH₂ | Et | SO₂NHEt | |
| 12-120 | thiophen-3-yl | Me | NH₂ | i-Pr | SO₂NHEt | |
| 12-121 | pyridazin-3-yl | Me | NH₂ | Me | SO₂NHEt | |
| 12-122 | pyridazin-3-yl | Me | NH₂ | Et | SO₂NHEt | |
| 12-123 | pyridazin-3-yl | Me | NH₂ | i-Pr | SO₂NHEt | |
| 12-124 | pyrazin-2-yl | Me | NH₂ | Me | SO₂NHEt | |
| 12-125 | pyrazin-2-yl | Me | NH₂ | Et | SO₂NHEt | |
| 12-126 | pyrazin-2-yl | Me | NH₂ | i-Pr | SO₂NHEt | |
| 12-127 | pyrimidin-2-yl | Me | NH₂ | Me | SO₂NHEt | |
| 12-128 | pyrimidin-2-yl | Me | NH₂ | Et | SO₂NHEt | |
| 12-129 | pyrimidin-2-yl | Me | NH₂ | i-Pr | SO₂NHEt | |
| 12-130 | pyrimidin-4-yl | Me | NH₂ | Me | SO₂NHEt | |
| 12-131 | pyrimidin-4-yl | Me | NH₂ | Et | SO₂NHEt | |
| 12-132 | pyrimidin-4-yl | Me | NH₂ | i-Pr | SO₂NHEt | |
| 12-133 | pyrimidin-5-yl | Me | NH₂ | Me | SO₂NHEt | |
| 12-134 | pyrimidin-5-yl | Me | NH₂ | Et | SO₂NHEt | |
| 12-135 | pyrimidin-5-yl | Me | NH₂ | i-Pr | SO₂NHEt | |

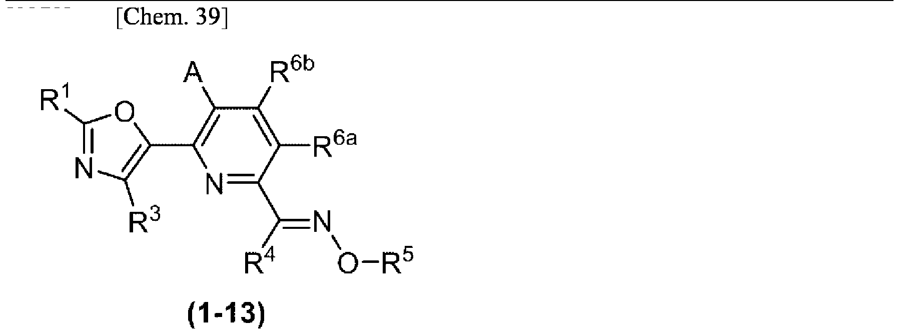

Table 13 (In Table 13, R^{6a} and R^{6b} represent hydrogen atoms.)

**[Table 13-1]**

| Compound No. | R¹ | R³ | R⁴ | R⁵ | A | Physical Property value |
|---|---|---|---|---|---|---|
| 13-1 | Ph | Me | NH₂ | Me | SO₂NHMe | |
| 13-2 | Ph | Me | NH₂ | Et | SO₂NHMe | |
| 13-3 | Ph | Me | NH₂ | i-Pr | SO₂NHMe | |
| 13-4 | 2-F-Ph | Me | NH₂ | Me | SO₂NHMe | |
| 13-5 | 2-F-Ph | Me | NH₂ | Et | SO₂NHMe | |
| 13-6 | 2-F-Ph | Me | NH₂ | i-Pr | SO₂NHMe | |
| 13-7 | 3-F-Ph | Me | NH₂ | Me | SO₂NHMe | |
| 13-8 | 3-F-Ph | Me | NH₂ | Et | SO₂NHMe | |
| 13-9 | 3-F-Ph | Me | NH₂ | i-Pr | SO₂NHMe | |
| 13-10 | 4-F-Ph | Me | NH₂ | Me | SO₂NHMe | |
| 13-11 | 4-F-Ph | Me | NH₂ | Et | SO₂NHMe | |
| 13-12 | 4-F-Ph | Me | NH₂ | i-Pr | SO₂NHMe | |
| 13-13 | 2,6-F₂-Ph | Me | NH₂ | Me | SO₂NHMe | |
| 13-14 | 2,6-F₂-Ph | Me | NH₂ | Et | SO₂NHMe | |
| 13-15 | 2,6-F₂-Ph | Me | NH₂ | i-Pr | SO₂NHMe | |

**[Table 13-2]**

| Compound No. | R¹ | R³ | R⁴ | R⁵ | A | Physical Property value |
|---|---|---|---|---|---|---|
| 13-16 | thiazol-5-yl | Me | NH₂ | Me | SO₂NHMe | |
| 13-17 | thiazol-5-yl | Me | NH₂ | Et | SO₂NHMe | |
| 13-18 | thiazol-5-yl | Me | NH₂ | i-Pr | SO₂NHMe | |
| 13-19 | thiazol-4-yl | Me | NH₂ | Me | SO₂NHMe | |
| 13-20 | thiazol-4-yl | Me | NH₂ | Et | SO₂NHMe | |
| 13-21 | thiazol-4-yl | Me | NH₂ | i-Pr | SO₂NHMe | |
| 13-22 | thiazol-2-yl | Me | NH₂ | Me | SO₂NHMe | |
| 13-23 | thiazol-2-yl | Me | NH₂ | Et | SO₂NHMe | |
| 13-24 | thiazol-2-yl | Me | NH₂ | i-Pr | SO₂NHMe | |
| 13-25 | thiophen-2-yl | Me | NH₂ | Me | SO₂NHMe | |
| 13-26 | thiophen-2-yl | Me | NH₂ | Et | SO₂NHMe | |
| 13-27 | thiophen-2-yl | Me | NH₂ | i-Pr | SO₂NHMe | |
| 13-28 | thiophen-3-yl | Me | NH₂ | Me | SO₂NHMe | |
| 13-29 | thiophen-3-yl | Me | NH₂ | Et | SO₂NHMe | |
| 13-30 | thiophen-3-yl | Me | NH₂ | i-Pr | SO₂NHMe | |
| 13-31 | pyridazin-3-yl | Me | NH₂ | Me | SO₂NHMe | |
| 13-32 | pyridazin-3-yl | Me | NH₂ | Et | SO₂NHMe | |
| 13-33 | pyridazin-3-yl | Me | NH₂ | i-Pr | SO₂NHMe | |
| 13-34 | pyrazin-2-yl | Me | NH₂ | Me | SO₂NHMe | |
| 13-35 | pyrazin-2-yl | Me | NH₂ | Et | SO₂NHMe | |
| 13-36 | pyrazin-2-yl | Me | NH₂ | i-Pr | SO₂NHMe | |
| 13-37 | pyrimidin-2-yl | Me | NH₂ | Me | SO₂NHMe | |
| 13-38 | pyrimidin-2-yl | Me | NH₂ | Et | SO₂NHMe | |
| 13-39 | pyrimidin-2-yl | Me | NH₂ | i-Pr | SO₂NHMe | |
| 13-40 | pyrimidin-4-yl | Me | NH₂ | Me | SO₂NHMe | |
| 13-41 | pyrimidin-4-yl | Me | NH₂ | Et | SO₂NHMe | |
| 13-42 | pyrimidin-4-yl | Me | NH₂ | i-Pr | SO₂NHMe | |
| 13-43 | pyrimidin-5-yl | Me | NH₂ | Me | SO₂NHMe | |
| 13-44 | pyrimidin-5-yl | Me | NH₂ | Et | SO₂NHMe | |
| 13-45 | pyrimidin-5-yl | Me | NH₂ | i-Pr | SO₂NHMe | |

**[Table 13-3]**

| Compound No. | R¹ | R³ | R⁴ | R⁵ | A | Physical Property value |
|---|---|---|---|---|---|---|
| 13-46 | thiazol-5-yl | Et | NH₂ | Me | SO₂NHMe | |
| 13-47 | thiazol-5-yl | Et | NH₂ | Et | SO₂NHMe | |
| 13-48 | thiazol-5-yl | Et | NH₂ | i-Pr | SO₂NHMe | |
| 13-49 | thiazol-4-yl | Et | NH₂ | Me | SO₂NHMe | |
| 13-50 | thiazol-4-yl | Et | NH₂ | Et | SO₂NHMe | |
| 13-51 | thiazol-4-yl | Et | NH₂ | i-Pr | SO₂NHMe | |
| 13-52 | thiazol-2-yl | Et | NH₂ | Me | SO₂NHMe | |
| 13-53 | thiazol-2-yl | Et | NH₂ | Et | SO₂NHMe | |
| 13-54 | thiazol-2-yl | Et | NH₂ | i-Pr | SO₂NHMe | |
| 13-55 | thiophen-2-yl | Et | NH₂ | Me | SO₂NHMe | |
| 13-56 | thiophen-2-yl | Et | NH₂ | Et | SO₂NHMe | |
| 13-57 | thiophen-2-yl | Et | NH₂ | i-Pr | SO₂NHMe | |
| 13-58 | thiophen-3-yl | Et | NH₂ | Me | SO₂NHMe | |
| 13-59 | thiophen-3-yl | Et | NH₂ | Et | SO₂NHMe | |
| 13-60 | thiophen-3-yl | Et | NH₂ | i-Pr | SO₂NHMe | |
| 13-61 | pyridazin-3-yl | Et | NH₂ | Me | SO₂NHMe | |
| 13-62 | pyridazin-3-yl | Et | NH₂ | Et | SO₂NHMe | |
| 13-63 | pyridazin-3-yl | Et | NH₂ | i-Pr | SO₂NHMe | |
| 13-64 | pyrazin-2-yl | Et | NH₂ | Me | SO₂NHMe | |
| 13-65 | pyrazin-2-yl | Et | NH₂ | Et | SO₂NHMe | |
| 13-66 | pyrazin-2-yl | Et | NH₂ | i-Pr | SO₂NHMe | |
| 13-67 | pyrimidin-2-yl | Et | NH₂ | Me | SO₂NHMe | |
| 13-68 | pyrimidin-2-yl | Et | NH₂ | Et | SO₂NHMe | |
| 13-69 | pyrimidin-2-yl | Et | NH₂ | i-Pr | SO₂NHMe | |
| 13-70 | pyrimidin-4-yl | Et | NH₂ | Me | SO₂NHMe | |
| 13-71 | pyrimidin-4-yl | Et | NH₂ | Et | SO₂NHMe | |
| 13-72 | pyrimidin-4-yl | Et | NH₂ | i-Pr | SO₂NHMe | |
| 13-73 | pyrimidin-5-yl | Et | NH₂ | Me | SO₂NHMe | |
| 13-74 | pyrimidin-5-yl | Et | NH₂ | Et | SO₂NHMe | |
| 13-75 | pyrimidin-5-yl | Et | NH₂ | i-Pr | SO₂NHMe | |

**[Table 13-4]**

| Compound No. | R¹ | R³ | R⁴ | R⁵ | A | Physical Property value |
|---|---|---|---|---|---|---|
| 13-76 | Ph | Et | NH₂ | Me | SO₂NHMe | |
| 13-77 | Ph | Et | NH₂ | Et | SO₂NHMe | |
| 13-78 | Ph | Et | NH₂ | i-Pr | SO₂NHMe | |
| 13-79 | 2-F-Ph | Et | NH₂ | Me | SO₂NHMe | |
| 13-80 | 2-F-Ph | Et | NH₂ | Et | SO₂NHMe | |
| 13-81 | 2-F-Ph | Et | NH₂ | i-Pr | SO₂NHMe | |
| 13-82 | 3-F-Ph | Et | NH₂ | Me | SO₂NHMe | |
| 13-83 | 3-F-Ph | Et | NH₂ | Et | SO₂NHMe | |
| 13-84 | 3-F-Ph | Et | NH₂ | i-Pr | SO₂NHMe | |
| 13-85 | 4-F-Ph | Et | NH₂ | Me | SO₂NHMe | |
| 13-86 | 4-F-Ph | Et | NH₂ | Et | SO₂NHMe | |
| 13-87 | 4-F-Ph | Et | NH₂ | i-Pr | SO₂NHMe | |
| 13-88 | 2,6-F₂-Ph | Et | NH₂ | Me | SO₂NHMe | |
| 13-89 | 2,6-F₂-Ph | Et | NH₂ | Et | SO₂NHMe | |
| 13-90 | 2,6-F₂-Ph | Et | NH₂ | i-Pr | SO₂NHMe | |
| 13-91 | Ph | Me | NH₂ | Me | SO₂NHEt | |
| 13-92 | Ph | Me | NH₂ | Et | SO₂NHEt | |
| 13-93 | Ph | Me | NH₂ | i-Pr | SO₂NHEt | |
| 13-94 | 2-F-Ph | Me | NH₂ | Me | SO₂NHEt | |
| 13-95 | 2-F-Ph | Me | NH₂ | Et | SO₂NHEt | |
| 13-96 | 2-F-Ph | Me | NH₂ | i-Pr | SO₂NHEt | |
| 13-97 | 3-F-Ph | Me | NH₂ | Me | SO₂NHEt | |
| 13-98 | 3-F-Ph | Me | NH₂ | Et | SO₂NHEt | |
| 13-99 | 3-F-Ph | Me | NH₂ | i-Pr | SO₂NHEt | |
| 13-100 | 4-F-Ph | Me | NH₂ | Me | SO₂NHEt | |
| 13-101 | 4-F-Ph | Me | NH₂ | Et | SO₂NHEt | |
| 13-102 | 4-F-Ph | Me | NH₂ | i-Pr | SO₂NHEt | |
| 13-103 | 2,6-F₂-Ph | Me | NH₂ | Me | SO₂NHEt | |
| 13-104 | 2,6-F₂-Ph | Me | NH₂ | Et | SO₂NHEt | |
| 13-105 | 2,6-F₂-Ph | Me | NH₂ | i-Pr | SO₂NHEt | |

**[Table 13-5]**

| Compound No. | R¹ | R³ | R⁴ | R⁵ | A | Physical Property value |
|---|---|---|---|---|---|---|
| 13-106 | thiazol-5-yl | Me | NH₂ | Me | SO₂NHEt | |
| 13-107 | thiazol-5-yl | Me | NH₂ | Et | SO₂NHEt | |
| 13-108 | thiazol-5-yl | Me | NH₂ | i-Pr | SO₂NHEt | |
| 13-109 | thiazol-4-yl | Me | NH₂ | Me | SO₂NHEt | |
| 13-110 | thiazol-4-yl | Me | NH₂ | Et | SO₂NHEt | |
| 13-111 | thiazol-4-yl | Me | NH₂ | i-Pr | SO₂NHEt | |
| 13-112 | thiazol-2-yl | Me | NH₂ | Me | SO₂NHEt | |
| 13-113 | thiazol-2-yl | Me | NH₂ | Et | SO₂NHEt | |
| 13-114 | thiazol-2-yl | Me | NH₂ | i-Pr | SO₂NHEt | |
| 13-115 | thiophen-2-yl | Me | NH₂ | Me | SO₂NHEt | |
| 13-116 | thiophen-2-yl | Me | NH₂ | Et | SO₂NHEt | |
| 13-117 | thiophen-2-yl | Me | NH₂ | i-Pr | SO₂NHEt | |
| 13-118 | thiophen-3-yl | Me | NH₂ | Me | SO₂NHEt | |
| 13-119 | thiophen-3-yl | Me | NH₂ | Et | SO₂NHEt | |
| 13-120 | thiophen-3-yl | Me | NH₂ | i-Pr | SO₂NHEt | |
| 13-121 | pyridazin-3-yl | Me | NH₂ | Me | SO₂NHEt | |
| 13-122 | pyridazin-3-yl | Me | NH₂ | Et | SO₂NHEt | |
| 13-123 | pyridazin-3-yl | Me | NH₂ | i-Pr | SO₂NHEt | |
| 13-124 | pyrazin-2-yl | Me | NH₂ | Me | SO₂NHEt | |
| 13-125 | pyrazin-2-yl | Me | NH₂ | Et | SO₂NHEt | |
| 13-126 | pyrazin-2-yl | Me | NH₂ | i-Pr | SO₂NHEt | |
| 13-127 | pyrimidin-2-yl | Me | NH₂ | Me | SO₂NHEt | |
| 13-128 | pyrimidin-2-yl | Me | NH₂ | Et | SO₂NHEt | |
| 13-129 | pyrimidin-2-yl | Me | NH₂ | i-Pr | SO₂NHEt | |
| 13-130 | pyrimidin-4-yl | Me | NH₂ | Me | SO₂NHEt | |
| 13-131 | pyrimidin-4-yl | Me | NH₂ | Et | SO₂NHEt | |
| 13-132 | pyrimidin-4-yl | Me | NH₂ | i-Pr | SO₂NHEt | |
| 13-133 | pyrimidin-5-yl | Me | NH₂ | Me | SO₂NHEt | |
| 13-134 | pyrimidin-5-yl | Me | NH₂ | Et | SO₂NHEt | |
| 13-135 | pyrimidin-5-yl | Me | NH₂ | i-Pr | SO₂NHEt | |

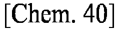

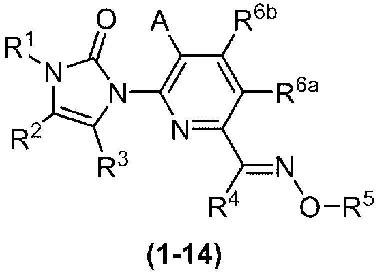

Table 14 (In Table 14, R², R^{6a}, and R^{6b} represent hydrogen atoms.)

**[Table 14-1]**

| Compound No. | R¹ | R³ | R⁴ | R⁵ | A | Physical Property value |
|---|---|---|---|---|---|---|
| 14-1 | Ph | Me | NH₂ | Me | SO₂NHMe | |
| 14-2 | Ph | Me | NH₂ | Et | SO₂NHMe | |
| 14-3 | Ph | Me | NH₂ | i-Pr | SO₂NHMe | |
| 14-4 | 2-F-Ph | Me | NH₂ | Me | SO₂NHMe | |
| 14-5 | 2-F-Ph | Me | NH₂ | Et | SO₂NHMe | |
| 14-6 | 2-F-Ph | Me | NH₂ | i-Pr | SO₂NHMe | |
| 14-7 | 3-F-Ph | Me | NH₂ | Me | SO₂NHMe | |
| 14-8 | 3-F-Ph | Me | NH₂ | Et | SO₂NHMe | |
| 14-9 | 3-F-Ph | Me | NH₂ | i-Pr | SO₂NHMe | |
| 14-10 | 4-F-Ph | Me | NH₂ | Me | SO₂NHMe | |
| 14-11 | 4-F-Ph | Me | NH₂ | Et | SO₂NHMe | |
| 14-12 | 4-F-Ph | Me | NH₂ | i-Pr | SO₂NHMe | |
| 14-13 | 2,6-F₂-Ph | Me | NH₂ | Me | SO₂NHMe | |
| 14-14 | 2,6-F₂-Ph | Me | NH₂ | Et | SO₂NHMe | |
| 14-15 | 2,6-F₂-Ph | Me | NH₂ | i-Pr | SO₂NHMe | |

**[Table 14-2]**

| Compound No. | R¹ | R² | R³ | R⁴ | R⁵ | A | Physical Property value |
|---|---|---|---|---|---|---|---|
| 14-16 | thiazol-5-yl | H | Me | NH₂ | Me | SO₂NHMe | |
| 14-17 | thiazol-5-yl | H | Me | NH₂ | Et | SO₂NHMe | |
| 14-18 | thiazol-5-yl | H | Me | NH₂ | i-Pr | SO₂NHMe | |
| 14-19 | thiazol-4-yl | H | Me | NH₂ | Me | SO₂NHMe | |
| 14-20 | thiazol-4-yl | H | Me | NH₂ | Et | SO₂NHMe | |
| 14-21 | thiazol-4-yl | H | Me | NH₂ | i-Pr | SO₂NHMe | |
| 14-22 | thiazol-2-yl | H | Me | NH₂ | Me | SO₂NHMe | |
| 14-23 | thiazol-2-yl | H | Me | NH₂ | Et | SO₂NHMe | |
| 14-24 | thiazol-2-yl | H | Me | NH₂ | i-Pr | SO₂NHMe | |
| 14-25 | thiophen-2-yl | H | Me | NH₂ | Me | SO₂NHMe | |
| 14-26 | thiophen-2-yl | H | Me | NH₂ | Et | SO₂NHMe | |
| 14-27 | thiophen-2-yl | H | Me | NH₂ | i-Pr | SO₂NHMe | |
| 14-28 | thiophen-3-yl | H | Me | NH₂ | Me | SO₂NHMe | |
| 14-29 | thiophen-3-yl | H | Me | NH₂ | Et | SO₂NHMe | |
| 14-30 | thiophen-3-yl | H | Me | NH₂ | i-Pr | SO₂NHMe | |
| 14-31 | pyridazin-3-yl | H | Me | NH₂ | Me | SO₂NHMe | |
| 14-32 | pyridazin-3-yl | H | Me | NH₂ | Et | SO₂NHMe | |
| 14-33 | pyridazin-3-yl | H | Me | NH₂ | i-Pr | SO₂NHMe | |
| 14-34 | pyrazin-2-yl | H | Me | NH₂ | Me | SO₂NHMe | |
| 14-35 | pyrazin-2-yl | H | Me | NH₂ | Et | SO₂NHMe | |
| 14-36 | pyrazin-2-yl | H | Me | NH₂ | i-Pr | SO₂NHMe | |
| 14-37 | pyrimidin-2-yl | H | Me | NH₂ | Me | SO₂NHMe | |
| 14-38 | pyrimidin-2-yl | H | Me | NH₂ | Et | SO₂NHMe | |
| 14-39 | pyrimidin-2-yl | H | Me | NH₂ | i-Pr | SO₂NHMe | |
| 14-40 | pyrimidin-4-yl | H | Me | NH₂ | Me | SO₂NHMe | |
| 14-41 | pyrimidin-4-yl | H | Me | NH₂ | Et | SO₂NHMe | |
| 14-42 | pyrimidin-4-yl | H | Me | NH₂ | i-Pr | SO₂NHMe | |
| 14-43 | pyrimidin-5-yl | H | Me | NH₂ | Me | SO₂NHMe | |
| 14-44 | pyrimidin-5-yl | H | Me | NH₂ | Et | SO₂NHMe | |
| 14-45 | pyrimidin-5-yl | H | Me | NH₂ | i-Pr | SO₂NHMe | |

**[Table 14-3]**

| Compound No. | R¹ | R² | R³ | R⁴ | R⁵ | A | Physical Property value |
|---|---|---|---|---|---|---|---|
| 14-46 | thiazol-5-yl | H | Et | NH₂ | Me | SO₂NHMe | |
| 14-47 | thiazol-5-yl | H | Et | NH₂ | Et | SO₂NHMe | |
| 14-48 | thiazol-5-yl | H | Et | NH₂ | i-Pr | SO₂NHMe | |
| 14-49 | thiazol-4-yl | H | Et | NH₂ | Me | SO₂NHMe | |
| 14-50 | thiazol-4-yl | H | Et | NH₂ | Et | SO₂NHMe | |
| 14-51 | thiazol-4-yl | H | Et | NH₂ | i-Pr | SO₂NHMe | |
| 14-52 | thiazol-2-yl | H | Et | NH₂ | Me | SO₂NHMe | |
| 14-53 | thiazol-2-yl | H | Et | NH₂ | Et | SO₂NHMe | |
| 14-54 | thiazol-2-yl | H | Et | NH₂ | i-Pr | SO₂NHMe | |
| 14-55 | thiophen-2-yl | H | Et | NH₂ | Me | SO₂NHMe | |
| 14-56 | thiophen-2-yl | H | Et | NH₂ | Et | SO₂NHMe | |
| 14-57 | thiophen-2-yl | H | Et | NH₂ | i-Pr | SO₂NHMe | |
| 14-58 | thiophen-3-yl | H | Et | NH₂ | Me | SO₂NHMe | |
| 14-59 | thiophen-3-yl | H | Et | NH₂ | Et | SO₂NHMe | |
| 14-60 | thiophen-3-yl | H | Et | NH₂ | i-Pr | SO₂NHMe | |
| 14-61 | pyridazin-3-yl | H | Et | NH₂ | Me | SO₂NHMe | |
| 14-62 | pyridazin-3-yl | H | Et | NH₂ | Et | SO₂NHMe | |
| 14-63 | pyridazin-3-yl | H | Et | NH₂ | i-Pr | SO₂NHMe | |
| 14-64 | pyrazin-2-yl | H | Et | NH₂ | Me | SO₂NHMe | |
| 14-65 | pyrazin-2-yl | H | Et | NH₂ | Et | SO₂NHMe | |
| 14-66 | pyrazin-2-yl | H | Et | NH₂ | i-Pr | SO₂NHMe | |
| 14-67 | pyrimidin-2-yl | H | Et | NH₂ | Me | SO₂NHMe | |
| 14-68 | pyrimidin-2-yl | H | Et | NH₂ | Et | SO₂NHMe | |
| 14-69 | pyrimidin-2-yl | H | Et | NH₂ | i-Pr | SO₂NHMe | |
| 14-70 | pyrimidin-4-yl | H | Et | NH₂ | Me | SO₂NHMe | |
| 14-71 | pyrimidin-4-yl | H | Et | NH₂ | Et | SO₂NHMe | |
| 14-72 | pyrimidin-4-yl | H | Et | NH₂ | i-Pr | SO₂NHMe | |
| 14-73 | pyrimidin-5-yl | H | Et | NH₂ | Me | SO₂NHMe | |
| 14-74 | pyrimidin-5-yl | H | Et | NH₂ | Et | SO₂NHMe | |
| 14-75 | pyrimidin-5-yl | H | Et | NH₂ | i-Pr | SO₂NHMe | |

**[Table 14-4]**

| Compound No. | R¹ | R² | R³ | R⁴ | R⁵ | A | Physical Property value |
|---|---|---|---|---|---|---|---|
| 14-76 | Ph | H | Et | NH₂ | Me | SO₂NHMe | |
| 14-77 | Ph | H | Et | NH₂ | Et | SO₂NHMe | |
| 14-78 | Ph | H | Et | NH₂ | i-Pr | SO₂NHMe | |
| 14-79 | 2-F-Ph | H | Et | NH₂ | Me | SO₂NHMe | |
| 14-80 | 2-F-Ph | H | Et | NH₂ | Et | SO₂NHMe | |
| 14-81 | 2-F-Ph | H | Et | NH₂ | i-Pr | SO₂NHMe | |
| 14-82 | 3-F-Ph | H | Et | NH₂ | Me | SO₂NHMe | |
| 14-83 | 3-F-Ph | H | Et | NH₂ | Et | SO₂NHMe | |
| 14-84 | 3-F-Ph | H | Et | NH₂ | i-Pr | SO₂NHMe | |
| 14-85 | 4-F-Ph | H | Et | NH₂ | Me | SO₂NHMe | |
| 14-86 | 4-F-Ph | H | Et | NH₂ | Et | SO₂NHMe | |
| 14-87 | 4-F-Ph | H | Et | NH₂ | i-Pr | SO₂NHMe | |
| 14-88 | 2,6-F₂-Ph | H | Et | NH₂ | Me | SO₂NHMe | |
| 14-89 | 2,6-F₂-Ph | H | Et | NH₂ | Et | SO₂NHMe | |
| 14-90 | 2,6-F₂-Ph | H | Et | NH₂ | i-Pr | SO₂NHMe | |
| 14-91 | Ph | H | Me | NH₂ | Me | SO₂NHEt | |
| 14-92 | Ph | H | Me | NH₂ | Et | SO₂NHEt | |
| 14-93 | Ph | H | Me | NH₂ | i-Pr | SO₂NHEt | |
| 14-94 | 2-F-Ph | H | Me | NH₂ | Me | SO₂NHEt | |
| 14-95 | 2-F-Ph | H | Me | NH₂ | Et | SO₂NHEt | |
| 14-96 | 2-F-Ph | H | Me | NH₂ | i-Pr | SO₂NHEt | |
| 14-97 | 3-F-Ph | H | Me | NH₂ | Me | SO₂NHEt | |
| 14-98 | 3-F-Ph | H | Me | NH₂ | Et | SO₂NHEt | |
| 14-99 | 3-F-Ph | H | Me | NH₂ | i-Pr | SO₂NHEt | |
| 14-100 | 4-F-Ph | H | Me | NH₂ | Me | SO₂NHEt | |
| 14-101 | 4-F-Ph | H | Me | NH₂ | Et | SO₂NHEt | |
| 14-102 | 4-F-Ph | H | Me | NH₂ | i-Pr | SO₂NHEt | |
| 14-103 | 2,6-F₂-Ph | H | Me | NH₂ | Me | SO₂NHEt | |
| 14-104 | 2,6-F₂-Ph | H | Me | NH₂ | Et | SO₂NHEt | |
| 14-105 | 2,6-F₂-Ph | H | Me | NH₂ | i-Pr | SO₂NHEt | |

**[Table 14-5]**

| Compound No. | R¹ | R² | R³ | R⁴ | R⁵ | A | Physical Property value |
|---|---|---|---|---|---|---|---|
| 14-106 | thiazol-5-yl | H | Me | NH₂ | Me | SO₂NHEt | |
| 14-107 | thiazol-5-yl | H | Me | NH₂ | Et | SO₂NHEt | |
| 14-108 | thiazol-5-yl | H | Me | NH₂ | i-Pr | SO₂NHEt | |
| 14-109 | thiazol-4-yl | H | Me | NH₂ | Me | SO₂NHEt | |
| 14-110 | thiazol-4-yl | H | Me | NH₂ | Et | SO₂NHEt | |
| 14-111 | thiazol-4-yl | H | Me | NH₂ | i-Pr | SO₂NHEt | |
| 14-112 | thiazol-2-yl | H | Me | NH₂ | Me | SO₂NHEt | |
| 14-113 | thiazol-2-yl | H | Me | NH₂ | Et | SO₂NHEt | |
| 14-114 | thiazol-2-yl | H | Me | NH₂ | i-Pr | SO₂NHEt | |
| 14-115 | thiophen-2-yl | H | Me | NH₂ | Me | SO₂NHEt | |
| 14-116 | thiophen-2-yl | H | Me | NH₂ | Et | SO₂NHEt | |
| 14-117 | thiophen-2-yl | H | Me | NH₂ | i-Pr | SO₂NHEt | |
| 14-118 | thiophen-3-yl | H | Me | NH₂ | Me | SO₂NHEt | |
| 14-119 | thiophen-3-yl | H | Me | NH₂ | Et | SO₂NHEt | |
| 14-120 | thiophen-3-yl | H | Me | NH₂ | i-Pr | SO₂NHEt | |
| 14-121 | pyridazin-3-yl | H | Me | NH₂ | Me | SO₂NHEt | |
| 14-122 | pyridazin-3-yl | H | Me | NH₂ | Et | SO₂NHEt | |
| 14-123 | pyridazin-3-yl | H | Me | NH₂ | i-Pr | SO₂NHEt | |
| 14-124 | pyrazin-2-yl | H | Me | NH₂ | Me | SO₂NHEt | |
| 14-125 | pyrazin-2-yl | H | Me | NH₂ | Et | SO₂NHEt | |
| 14-126 | pyrazin-2-yl | H | Me | NH₂ | i-Pr | SO₂NHEt | |
| 14-127 | pyrimidin-2-yl | H | Me | NH₂ | Me | SO₂NHEt | |
| 14-128 | pyrimidin-2-yl | H | Me | NH₂ | Et | SO₂NHEt | |
| 14-129 | pyrimidin-2-yl | H | Me | NH₂ | i-Pr | SO₂NHEt | |
| 14-130 | pyrimidin-4-yl | H | Me | NH₂ | Me | SO₂NHEt | |
| 14-131 | pyrimidin-4-yl | H | Me | NH₂ | Et | SO₂NHEt | |
| 14-132 | pyrimidin-4-yl | H | Me | NH₂ | i-Pr | SO₂NHEt | |
| 14-133 | pyrimidin-5-yl | H | Me | NH₂ | Me | SO₂NHEt | |
| 14-134 | pyrimidin-5-yl | H | Me | NH₂ | Et | SO₂NHEt | |
| 14-135 | pyrimidin-5-yl | H | Me | NH₂ | i-Pr | SO₂NHEt | |

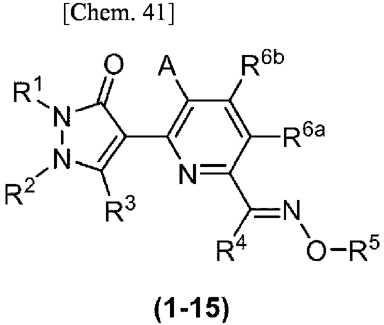

Table 15 (In Table 15, R², R^{6a}, and R^{6b} represent hydrogen atoms.)

**[Table 15-1]**

| Compound No. | R¹ | R³ | R⁴ | R⁵ | A | Physical Property value |
|---|---|---|---|---|---|---|
| 15-1 | Ph | Me | NH₂ | Me | SO₂NHMe | |
| 15-2 | Ph | Me | NH₂ | Et | SO₂NHMe | |
| 15-3 | Ph | Me | NH₂ | i-Pr | SO₂NHMe | |
| 15-4 | 2-F-Ph | Me | NH₂ | Me | SO₂NHMe | |
| 15-5 | 2-F-Ph | Me | NH₂ | Et | SO₂NHMe | |
| 15-6 | 2-F-Ph | Me | NH₂ | i-Pr | SO₂NHMe | |
| 15-7 | 3-F-Ph | Me | NH₂ | Me | SO₂NHMe | |
| 15-8 | 3-F-Ph | Me | NH₂ | Et | SO₂NHMe | |
| 15-9 | 3-F-Ph | Me | NH₂ | i-Pr | SO₂NHMe | |
| 15-10 | 4-F-Ph | Me | NH₂ | Me | SO₂NHMe | |
| 15-11 | 4-F-Ph | Me | NH₂ | Et | SO₂NHMe | |
| 15-12 | 4-F-Ph | Me | NH₂ | i-Pr | SO₂NHMe | |
| 15-13 | 2,6-F₂-Ph | Me | NH₂ | Me | SO₂NHMe | |
| 15-14 | 2,6-F₂-Ph | Me | NH₂ | Et | SO₂NHMe | |
| 15-15 | 2,6-F₂-Ph | Me | NH₂ | i-Pr | SO₂NHMe | |

**[Table 15-2]**

| Compound No. | R¹ | R² | R³ | R⁴ | R⁵ | A | Physical Property value |
|---|---|---|---|---|---|---|---|
| 15-16 | thiazol-5-yl | H | Me | NH₂ | Me | SO₂NHMe | |
| 15-17 | thiazol-5-yl | H | Me | NH₂ | Et | SO₂NHMe | |
| 15-18 | thiazol-5-yl | H | Me | NH₂ | i-Pr | SO₂NHMe | |
| 15-19 | thiazol-4-yl | H | Me | NH₂ | Me | SO₂NHMe | |
| 15-20 | thiazol-4-yl | H | Me | NH₂ | Et | SO₂NHMe | |
| 15-21 | thiazol-4-yl | H | Me | NH₂ | i-Pr | SO₂NHMe | |
| 15-22 | thiazol-2-yl | H | Me | NH₂ | Me | SO₂NHMe | |
| 15-23 | thiazol-2-yl | H | Me | NH₂ | Et | SO₂NHMe | |
| 15-24 | thiazol-2-yl | H | Me | NH₂ | i-Pr | SO₂NHMe | |
| 15-25 | thiophen-2-yl | H | Me | NH₂ | Me | SO₂NHMe | |
| 15-26 | thiophen-2-yl | H | Me | NH₂ | Et | SO₂NHMe | |
| 15-27 | thiophen-2-yl | H | Me | NH₂ | i-Pr | SO₂NHMe | |
| 15-28 | thiophen-3-yl | H | Me | NH₂ | Me | SO₂NHMe | |
| 15-29 | thiophen-3-yl | H | Me | NH₂ | Et | SO₂NHMe | |
| 15-30 | thiophen-3-yl | H | Me | NH₂ | i-Pr | SO₂NHMe | |
| 15-31 | pyridazin-3-yl | H | Me | NH₂ | Me | SO₂NHMe | |
| 15-32 | pyridazin-3-yl | H | Me | NH₂ | Et | SO₂NHMe | |
| 15-33 | pyridazin-3-yl | H | Me | NH₂ | i-Pr | SO₂NHMe | |
| 15-34 | pyrazin-2-yl | H | Me | NH₂ | Me | SO₂NHMe | |
| 15-35 | pyrazin-2-yl | H | Me | NH₂ | Et | SO₂NHMe | |
| 15-36 | pyrazin-2-yl | H | Me | NH₂ | i-Pr | SO₂NHMe | |
| 15-37 | pyrimidin-2-yl | H | Me | NH₂ | Me | SO₂NHMe | |
| 15-38 | pyrimidin-2-yl | H | Me | NH₂ | Et | SO₂NHMe | |
| 15-39 | pyrimidin-2-yl | H | Me | NH₂ | i-Pr | SO₂NHMe | |
| 15-40 | pyrimidin-4-yl | H | Me | NH₂ | Me | SO₂NHMe | |
| 15-41 | pyrimidin-4-yl | H | Me | NH₂ | Et | SO₂NHMe | |
| 15-42 | pyrimidin-4-yl | H | Me | NH₂ | i-Pr | SO₂NHMe | |
| 15-43 | pyrimidin-5-yl | H | Me | NH₂ | Me | SO₂NHMe | |
| 15-44 | pyrimidin-5-yl | H | Me | NH₂ | Et | SO₂NHMe | |
| 15-45 | pyrimidin-5-yl | H | Me | NH₂ | i-Pr | SO₂NHMe | |

**[Table 15-3]**

| Compound No. | R¹ | R² | R³ | R⁴ | R⁵ | A | Physical Property value |
|---|---|---|---|---|---|---|---|
| 15-46 | thiazol-5-yl | H | Et | NH₂ | Me | SO₂NHMe | |
| 15-47 | thiazol-5-yl | H | Et | NH₂ | Et | SO₂NHMe | |
| 15-48 | thiazol-5-yl | H | Et | NH₂ | i-Pr | SO₂NHMe | |
| 15-49 | thiazol-4-yl | H | Et | NH₂ | Me | SO₂NHMe | |
| 15-50 | thiazol-4-yl | H | Et | NH₂ | Et | SO₂NHMe | |
| 15-51 | thiazol-4-yl | H | Et | NH₂ | i-Pr | SO₂NHMe | |
| 15-52 | thiazol-2-yl | H | Et | NH₂ | Me | SO₂NHMe | |
| 15-53 | thiazol-2-yl | H | Et | NH₂ | Et | SO₂NHMe | |
| 15-54 | thiazol-2-yl | H | Et | NH₂ | i-Pr | SO₂NHMe | |
| 15-55 | thiophen-2-yl | H | Et | NH₂ | Me | SO₂NHMe | |
| 15-56 | thiophen-2-yl | H | Et | NH₂ | Et | SO₂NHMe | |
| 15-57 | thiophen-2-yl | H | Et | NH₂ | i-Pr | SO₂NHMe | |
| 15-58 | thiophen-3-yl | H | Et | NH₂ | Me | SO₂NHMe | |
| 15-59 | thiophen-3-yl | H | Et | NH₂ | Et | SO₂NHMe | |
| 15-60 | thiophen-3-yl | H | Et | NH₂ | i-Pr | SO₂NHMe | |
| 15-61 | pyridazin-3-yl | H | Et | NH₂ | Me | SO₂NHMe | |
| 15-62 | pyridazin-3-yl | H | Et | NH₂ | Et | SO₂NHMe | |
| 15-63 | pyridazin-3-yl | H | Et | NH₂ | i-Pr | SO₂NHMe | |
| 15-64 | pyrazin-2-yl | H | Et | NH₂ | Me | SO₂NHMe | |
| 15-65 | pyrazin-2-yl | H | Et | NH₂ | Et | SO₂NHMe | |
| 15-66 | pyrazin-2-yl | H | Et | NH₂ | i-Pr | SO₂NHMe | |
| 15-67 | pyrimidin-2-yl | H | Et | NH₂ | Me | SO₂NHMe | |
| 15-68 | pyrimidin-2-yl | H | Et | NH₂ | Et | SO₂NHMe | |
| 15-69 | pyrimidin-2-yl | H | Et | NH₂ | i-Pr | SO₂NHMe | |
| 15-70 | pyrimidin-4-yl | H | Et | NH₂ | Me | SO₂NHMe | |
| 15-71 | pyrimidin-4-yl | H | Et | NH₂ | Et | SO₂NHMe | |
| 15-72 | pyrimidin-4-yl | H | Et | NH₂ | i-Pr | SO₂NHMe | |
| 15-73 | pyrimidin-5-yl | H | Et | NH₂ | Me | SO₂NHMe | |
| 15-74 | pyrimidin-5-yl | H | Et | NH₂ | Et | SO₂NHMe | |
| 15-75 | pyrimidin-5-yl | H | Et | NH₂ | i-Pr | SO₂NHMe | |

**[Table 15-4]**

| Compound No. | R¹ | R² | R³ | R⁴ | R⁵ | A | Physical Property value |
|---|---|---|---|---|---|---|---|
| 15-76 | Ph | H | Et | NH₂ | Me | SO₂NHMe | |
| 15-77 | Ph | H | Et | NH₂ | Et | SO₂NHMe | |
| 15-78 | Ph | H | Et | NH₂ | i-Pr | SO₂NHMe | |
| 15-79 | 2-F-Ph | H | Et | NH₂ | Me | SO₂NHMe | |
| 15-80 | 2-F-Ph | H | Et | NH₂ | Et | SO₂NHMe | |
| 15-81 | 2-F-Ph | H | Et | NH₂ | i-Pr | SO₂NHMe | |
| 15-82 | 3-F-Ph | H | Et | NH₂ | Me | SO₂NHMe | |
| 15-83 | 3-F-Ph | H | Et | NH₂ | Et | SO₂NHMe | |
| 15-84 | 3-F-Ph | H | Et | NH₂ | i-Pr | SO₂NHMe | |
| 15-85 | 4-F-Ph | H | Et | NH₂ | Me | SO₂NHMe | |
| 15-86 | 4-F-Ph | H | Et | NH₂ | Et | SO₂NHMe | |
| 15-87 | 4-F-Ph | H | Et | NH₂ | i-Pr | SO₂NHMe | |
| 15-88 | 2,6-F₂-Ph | H | Et | NH₂ | Me | SO₂NHMe | |
| 15-89 | 2,6-F₂-Ph | H | Et | NH₂ | Et | SO₂NHMe | |
| 15-90 | 2,6-F₂-Ph | H | Et | NH₂ | i-Pr | SO₂NHMe | |
| 15-91 | Ph | H | Me | NH₂ | Me | SO₂NHEt | |
| 15-92 | Ph | H | Me | NH₂ | Et | SO₂NHEt | |
| 15-93 | Ph | H | Me | NH₂ | i-Pr | SO₂NHEt | |
| 15-94 | 2-F-Ph | H | Me | NH₂ | Me | SO₂NHEt | |
| 15-95 | 2-F-Ph | H | Me | NH₂ | Et | SO₂NHEt | |
| 15-96 | 2-F-Ph | H | Me | NH₂ | i-Pr | SO₂NHEt | |
| 15-97 | 3-F-Ph | H | Me | NH₂ | Me | SO₂NHEt | |
| 15-98 | 3-F-Ph | H | Me | NH₂ | Et | SO₂NHEt | |
| 15-99 | 3-F-Ph | H | Me | NH₂ | i-Pr | SO₂NHEt | |
| 15-100 | 4-F-Ph | H | Me | NH₂ | Me | SO₂NHEt | |
| 15-101 | 4-F-Ph | H | Me | NH₂ | Et | SO₂NHEt | |
| 15-102 | 4-F-Ph | H | Me | NH₂ | i-Pr | SO₂NHEt | |
| 15-103 | 2,6-F₂-Ph | H | Me | NH₂ | Me | SO₂NHEt | |
| 15-104 | 2,6-F₂-Ph | H | Me | NH₂ | Et | SO₂NHEt | |
| 15-105 | 2,6-F₂-Ph | H | Me | NH₂ | i-Pr | SO₂NHEt | |

**[Table 15-5]**

| Compound No. | R¹ | R² | R³ | R⁴ | R⁵ | A | Physical Property value |
|---|---|---|---|---|---|---|---|
| 15-106 | thiazol-5-yl | H | Me | NH₂ | Me | SO₂NHEt | |
| 15-107 | thiazol-5-yl | H | Me | NH₂ | Et | SO₂NHEt | |
| 15-108 | thiazol-5-yl | H | Me | NH₂ | i-Pr | SO₂NHEt | |
| 15-109 | thiazol-4-yl | H | Me | NH₂ | Me | SO₂NHEt | |
| 15-110 | thiazol-4-yl | H | Me | NH₂ | Et | SO₂NHEt | |
| 15-111 | thiazol-4-yl | H | Me | NH₂ | i-Pr | SO₂NHEt | |
| 15-112 | thiazol-2-yl | H | Me | NH₂ | Me | SO₂NHEt | |
| 15-113 | thiazol-2-yl | H | Me | NH₂ | Et | SO₂NHEt | |
| 15-114 | thiazol-2-yl | H | Me | NH₂ | i-Pr | SO₂NHEt | |
| 15-115 | thiophen-2-yl | H | Me | NH₂ | Me | SO₂NHEt | |
| 15-116 | thiophen-2-yl | H | Me | NH₂ | Et | SO₂NHEt | |
| 15-117 | thiophen-2-yl | H | Me | NH₂ | i-Pr | SO₂NHEt | |
| 15-118 | thiophen-3-yl | H | Me | NH₂ | Me | SO₂NHEt | |
| 15-119 | thiophen-3-yl | H | Me | NH₂ | Et | SO₂NHEt | |
| 15-120 | thiophen-3-yl | H | Me | NH₂ | i-Pr | SO₂NHEt | |
| 15-121 | pyridazin-3-yl | H | Me | NH₂ | Me | SO₂NHEt | |
| 15-122 | pyridazin-3-yl | H | Me | NH₂ | Et | SO₂NHEt | |
| 15-123 | pyridazin-3-yl | H | Me | NH₂ | i-Pr | SO₂NHEt | |
| 15-124 | pyrazin-2-yl | H | Me | NH₂ | Me | SO₂NHEt | |
| 15-125 | pyrazin-2-yl | H | Me | NH₂ | Et | SO₂NHEt | |
| 15-126 | pyrazin-2-yl | H | Me | NH₂ | i-Pr | SO₂NHEt | |
| 15-127 | pyrimidin-2-yl | H | Me | NH₂ | Me | SO₂NHEt | |
| 15-128 | pyrimidin-2-yl | H | Me | NH₂ | Et | SO₂NHEt | |
| 15-129 | pyrimidin-2-yl | H | Me | NH₂ | i-Pr | SO₂NHEt | |
| 15-130 | pyrimidin-4-yl | H | Me | NH₂ | Me | SO₂NHEt | |
| 15-131 | pyrimidin-4-yl | H | Me | NH₂ | Et | SO₂NHEt | |
| 15-132 | pyrimidin-4-yl | H | Me | NH₂ | i-Pr | SO₂NHEt | |
| 15-133 | pyrimidin-5-yl | H | Me | NH₂ | Me | SO₂NHEt | |
| 15-134 | pyrimidin-5-yl | H | Me | NH₂ | Et | SO₂NHEt | |
| 15-135 | pyrimidin-5-yl | H | Me | NH₂ | i-Pr | SO₂NHEt | |

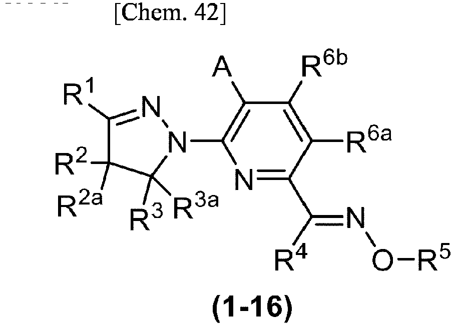

Table 16 (In Table 16, R², R^{2a}, R^{6a}, and R^{6b} represent hydrogen atoms.)

**[Table 16-1]**

| Compound No. | R¹ | R³ | R^{3a} | R⁴ | R⁵ | A | Physical Property value |
|---|---|---|---|---|---|---|---|
| 16-1 | Ph | Me | H | NH₂ | Me | SO₂NHMe | |
| 16-2 | Ph | Me | H | NH₂ | Et | SO₂NHMe | |
| 16-3 | Ph | Me | H | NH₂ | i-Pr | SO₂NHMe | |
| 16-4 | 2-F-Ph | Me | H | NH₂ | Me | SO₂NHMe | |
| 16-5 | 2-F-Ph | Me | H | NH₂ | Et | SO₂NHMe | |
| 16-6 | 2-F-Ph | Me | H | NH₂ | i-Pr | SO₂NHMe | |
| 16-7 | 3-F-Ph | Me | H | NH₂ | Me | SO₂NHMe | |
| 16-8 | 3-F-Ph | Me | H | NH₂ | Et | SO₂NHMe | |
| 16-9 | 3-F-Ph | Me | H | NH₂ | i-Pr | SO₂NHMe | |
| 16-10 | 4-F-Ph | Me | H | NH₂ | Me | SO₂NHMe | |
| 16-11 | 4-F-Ph | Me | H | NH₂ | Et | SO₂NHMe | |
| 16-12 | 4-F-Ph | Me | H | NH₂ | i-Pr | SO₂NHMe | |
| 16-13 | 2,6-F₂-Ph | Me | H | NH₂ | Me | SO₂NHMe | |
| 16-14 | 2,6-F₂-Ph | Me | H | NH₂ | Et | SO₂NHMe | |
| 16-15 | 2,6-F₂-Ph | Me | H | NH₂ | i-Pr | SO₂NHMe | |
| 16-16 | Ph | Me | Me | NH₂ | Me | SO₂NHMe | |
| 16-17 | Ph | Me | Me | NH₂ | Et | SO₂NHMe | |
| 16-18 | Ph | Me | Me | NH₂ | i-Pr | SO₂NHMe | |
| 16-19 | 2-F-Ph | Me | Me | NH₂ | Me | SO₂NHMe | |
| 16-20 | 2-F-Ph | Me | Me | NH₂ | Et | SO₂NHMe | |
| 16-21 | 2-F-Ph | Me | Me | NH₂ | i-Pr | SO₂NHMe | |
| 16-22 | 3-F-Ph | Me | Me | NH₂ | Me | SO₂NHMe | |
| 16-23 | 3-F-Ph | Me | Me | NH₂ | Et | SO₂NHMe | |
| 16-24 | 3-F-Ph | Me | Me | NH₂ | i-Pr | SO₂NHMe | |
| 16-25 | 4-F-Ph | Me | Me | NH₂ | Me | SO₂NHMe | |
| 16-26 | 4-F-Ph | Me | Me | NH₂ | Et | SO₂NHMe | |
| 16-27 | 4-F-Ph | Me | Me | NH₂ | i-Pr | SO₂NHMe | |
| 16-28 | 2,6-F₂-Ph | Me | Me | NH₂ | Me | SO₂NHMe | |
| 16-29 | 2,6-F₂-Ph | Me | Me | NH₂ | Et | SO₂NHMe | |
| 16-30 | 2,6-F₂-Ph | Me | Me | NH₂ | i-Pr | SO₂NHMe | |

**[Table 16-2]**

| Compound No. | R¹ | R² | R^{2a} | R³ | R^{3a} | R⁴ | R⁵ | A | Physical Property value |
|---|---|---|---|---|---|---|---|---|---|
| 16-31 | thiazol-5-yl | H | H | Me | H | NH₂ | Me | SO₂NHMe | |
| 16-32 | thiazol-5-yl | H | H | Me | H | NH₂ | Et | SO₂NHMe | |
| 16-33 | thiazol-5-yl | H | H | Me | H | NH₂ | i-Pr | SO₂NHMe | |
| 16-34 | thiazol-4-yl | H | H | Me | H | NH₂ | Me | SO₂NHMe | |
| 16-35 | thiazol-4-yl | H | H | Me | H | NH₂ | Et | SO₂NHMe | |
| 16-36 | thiazol-4-yl | H | H | Me | H | NH₂ | i-Pr | SO₂NHMe | |
| 16-37 | thiazol-2-yl | H | H | Me | H | NH₂ | Me | SO₂NHMe | |
| 16-38 | thiazol-2-yl | H | H | Me | H | NH₂ | Et | SO₂NHMe | |
| 16-39 | thiazol-2-yl | H | H | Me | H | NH₂ | i-Pr | SO₂NHMe | |
| 16-40 | thiophen-2-yl | H | H | Me | H | NH₂ | Me | SO₂NHMe | |
| 16-41 | thiophen-2-yl | H | H | Me | H | NH₂ | Et | SO₂NHMe | |
| 16-42 | thiophen-2-yl | H | H | Me | H | NH₂ | i-Pr | SO₂NHMe | |
| 16-43 | thiophen-3-yl | H | H | Me | H | NH₂ | Me | SO₂NHMe | |
| 16-44 | thiophen-3-yl | H | H | Me | H | NH₂ | Et | SO₂NHMe | |
| 16-45 | thiophen-3-yl | H | H | Me | H | NH₂ | i-Pr | SO₂NHMe | |
| 16-46 | pyridazin-3-yl | H | H | Me | H | NH₂ | Me | SO₂NHMe | |
| 16-47 | pyridazin-3-yl | H | H | Me | H | NH₂ | Et | SO₂NHMe | |
| 16-48 | pyridazin-3-yl | H | H | Me | H | NH₂ | i-Pr | SO₂NHMe | |
| 16-49 | pyrazin-2-yl | H | H | Me | H | NH₂ | Me | SO₂NHMe | |
| 16-50 | pyrazin-2-yl | H | H | Me | H | NH₂ | Et | SO₂NHMe | |
| 16-51 | pyrazin-2-yl | H | H | Me | H | NH₂ | i-Pr | SO₂NHMe | |
| 16-52 | pyrimidin-2-yl | H | H | Me | H | NH₂ | Me | SO₂NHMe | |
| 16-53 | pyrimidin-2-yl | H | H | Me | H | NH₂ | Et | SO₂NHMe | |
| 16-54 | pyrimidin-2-yl | H | H | Me | H | NH₂ | i-Pr | SO₂NHMe | |
| 16-55 | pyrimidin-4-yl | H | H | Me | H | NH₂ | Me | SO₂NHMe | |
| 16-56 | pyrimidin-4-yl | H | H | Me | H | NH₂ | Et | SO₂NHMe | |
| 16-57 | pyrimidin-4-yl | H | H | Me | H | NH₂ | i-Pr | SO₂NHMe | |
| 16-58 | pyrimidin-5-yl | H | H | Me | H | NH₂ | Me | SO₂NHMe | |
| 16-59 | pyrimidin-5-yl | H | H | Me | H | NH₂ | Et | SO₂NHMe | |
| 16-60 | pyrimidin-5-yl | H | H | Me | H | NH₂ | i-Pr | SO₂NHMe | |

**[Table 16-3]**

| Compound No. | R¹ | R² | R^{2a} | R³ | R^{3a} | R⁴ | R⁵ | A | Physical Property value |
|---|---|---|---|---|---|---|---|---|---|
| 16-61 | thiazol-5-yl | H | H | Me | Me | NH₂ | Me | SO₂NHMe | |
| 16-62 | thiazol-5-yl | H | H | Me | Me | NH₂ | Et | SO₂NHMe | |
| 16-63 | thiazol-5-yl | H | H | Me | Me | NH₂ | i-Pr | SO₂NHMe | |
| 16-64 | thiazol-4-yl | H | H | Me | Me | NH₂ | Me | SO₂NHMe | |
| 16-65 | thiazol-4-yl | H | H | Me | Me | NH₂ | Et | SO₂NHMe | |
| 16-66 | thiazol-4-yl | H | H | Me | Me | NH₂ | i-Pr | SO₂NHMe | |
| 16-67 | thiazol-2-yl | H | H | Me | Me | NH₂ | Me | SO₂NHMe | |
| 16-68 | thiazol-2-yl | H | H | Me | Me | NH₂ | Et | SO₂NHMe | |
| 16-69 | thiazol-2-yl | H | H | Me | Me | NH₂ | i-Pr | SO₂NHMe | |
| 16-70 | thiophen-2-yl | H | H | Me | Me | NH₂ | Me | SO₂NHMe | |
| 16-71 | thiophen-2-yl | H | H | Me | Me | NH₂ | Et | SO₂NHMe | |
| 16-72 | thiophen-2-yl | H | H | Me | Me | NH₂ | i-Pr | SO₂NHMe | |
| 16-73 | thiophen-3-yl | H | H | Me | Me | NH₂ | Me | SO₂NHMe | |
| 16-74 | thiophen-3-yl | H | H | Me | Me | NH₂ | Et | SO₂NHMe | |
| 16-75 | thiophen-3-yl | H | H | Me | Me | NH₂ | i-Pr | SO₂NHMe | |
| 16-76 | pyridazin-3-yl | H | H | Me | Me | NH₂ | Me | SO₂NHMe | |
| 16-77 | pyridazin-3-yl | H | H | Me | Me | NH₂ | Et | SO₂NHMe | |
| 16-78 | pyridazin-3-yl | H | H | Me | Me | NH₂ | i-Pr | SO₂NHMe | |
| 16-79 | pyrazin-2-yl | H | H | Me | Me | NH₂ | Me | SO₂NHMe | |
| 16-80 | pyrazin-2-yl | H | H | Me | Me | NH₂ | Et | SO₂NHMe | |
| 16-81 | pyrazin-2-yl | H | H | Me | Me | NH₂ | i-Pr | SO₂NHMe | |
| 16-82 | pyrimidin-2-yl | H | H | Me | Me | NH₂ | Me | SO₂NHMe | |
| 16-83 | pyrimidin-2-yl | H | H | Me | Me | NH₂ | Et | SO₂NHMe | |
| 16-84 | pyrimidin-2-yl | H | H | Me | Me | NH₂ | i-Pr | SO₂NHMe | |
| 16-85 | pyrimidin-4-yl | H | H | Me | Me | NH₂ | Me | SO₂NHMe | |
| 16-86 | pyrimidin-4-yl | H | H | Me | Me | NH₂ | Et | SO₂NHMe | |
| 16-87 | pyrimidin-4-yl | H | H | Me | Me | NH₂ | i-Pr | SO₂NHMe | |
| 16-88 | pyrimidin-5-yl | H | H | Me | Me | NH₂ | Me | SO₂NHMe | |
| 16-89 | pyrimidin-5-yl | H | H | Me | Me | NH₂ | Et | SO₂NHMe | |
| 16-90 | pyrimidin-5-yl | H | H | Me | Me | NH₂ | i-Pr | SO₂NHMe | |

**[Table 16-4]**

| Compound No. | R¹ | R² | R^{2a} | R³ | R^{3a} | R⁴ | R⁵ | A | Physical Property value |
|---|---|---|---|---|---|---|---|---|---|
| 16-91 | Ph | H | H | H | H | NH₂ | Me | SO₂NHMe | |
| 16-92 | Ph | H | H | H | H | NH₂ | Et | SO₂NHMe | |
| 16-93 | Ph | H | H | H | H | NH₂ | i-Pr | SO₂NHMe | |
| 16-94 | 2,6-F₂-Ph | H | H | H | H | NH₂ | Me | SO₂NHMe | |
| 16-95 | 2,6-F₂-Ph | H | H | H | H | NH₂ | Et | SO₂NHMe | |
| 16-96 | 2,6-F₂-Ph | H | H | H | H | NH₂ | i-Pr | SO₂NHMe | |
| 16-97 | thiophen-2-yl | H | H | H | H | NH₂ | Me | SO₂NHMe | |
| 16-98 | thiophen-2-yl | H | H | H | H | NH₂ | Et | SO₂NHMe | |
| 16-99 | thiophen-2-yl | H | H | H | H | NH₂ | i-Pr | SO₂NHMe | |
| 16-100 | thiophen-3-yl | H | H | H | H | NH₂ | Me | SO₂NHMe | |
| 16-101 | thiophen-3-yl | H | H | H | H | NH₂ | Et | SO₂NHMe | |
| 16-102 | thiophen-3-yl | H | H | H | H | NH₂ | i-Pr | SO₂NHMe | |
| 16-103 | thiazol-5-yl | H | H | Me | H | NH₂ | Me | SO₂NHEt | |
| 16-104 | thiazol-5-yl | H | H | Me | H | NH₂ | Et | SO₂NHEt | |
| 16-105 | thiazol-5-yl | H | H | Me | H | NH₂ | i-Pr | SO₂NHEt | |
| 16-106 | thiazol-4-yl | H | H | Me | H | NH₂ | Me | SO₂NHEt | |
| 16-107 | thiazol-4-yl | H | H | Me | H | NH₂ | Et | SO₂NHEt | |
| 16-108 | thiazol-4-yl | H | H | Me | H | NH₂ | i-Pr | SO₂NHEt | |
| 16-109 | thiazol-2-yl | H | H | Me | H | NH₂ | Me | SO₂NHEt | |
| 16-110 | thiazol-2-yl | H | H | Me | H | NH₂ | Et | SO₂NHEt | |
| 16-111 | thiazol-2-yl | H | H | Me | H | NH₂ | i-Pr | SO₂NHEt | |
| 16-112 | thiophen-2-yl | H | H | Me | H | NH₂ | Me | SO₂NHEt | |
| 16-113 | thiophen-2-yl | H | H | Me | H | NH₂ | Et | SO₂NHEt | |
| 16-114 | thiophen-2-yl | H | H | Me | H | NH₂ | i-Pr | SO₂NHEt | |
| 16-115 | thiophen-3-yl | H | H | Me | H | NH₂ | Me | SO₂NHEt | |
| 16-116 | thiophen-3-yl | H | H | Me | H | NH₂ | Et | SO₂NHEt | |
| 16-117 | thiophen-3-yl | H | H | Me | H | NH₂ | i-Pr | SO₂NHEt | |
| 16-118 | pyridazin-3-yl | H | H | Me | H | NH₂ | Me | SO₂NHEt | |
| 16-119 | pyridazin-3-yl | H | H | Me | H | NH₂ | Et | SO₂NHEt | |
| 16-120 | pyridazin-3-yl | H | H | Me | H | NH₂ | i-Pr | SO₂NHEt | |

**[Table 16-5]**

| Compound No. | R¹ | R² | R^{2a} | R³ | R^{3a} | R⁴ | R⁵ | A | Physical Property value |
|---|---|---|---|---|---|---|---|---|---|
| 16-121 | pyrazin-2-yl | H | H | Me | H | NH₂ | Me | SO₂NHEt | |
| 16-122 | pyrazin-2-yl | H | H | Me | H | NH₂ | Et | SO₂NHEt | |
| 16-123 | pyrazin-2-yl | H | H | Me | H | NH₂ | i-Pr | SO₂NHEt | |
| 16-124 | pyrimidin-2-yl | H | H | Me | H | NH₂ | Me | SO₂NHEt | |
| 16-125 | pyrimidin-2-yl | H | H | Me | H | NH₂ | Et | SO₂NHEt | |
| 16-126 | pyrimidin-2-yl | H | H | Me | H | NH₂ | i-Pr | SO₂NHEt | |
| 16-127 | pyrimidin-4-yl | H | H | Me | H | NH₂ | Me | SO₂NHEt | |
| 16-128 | pyrimidin-4-yl | H | H | Me | H | NH₂ | Et | SO₂NHEt | |
| 16-129 | pyrimidin-4-yl | H | H | Me | H | NH₂ | i-Pr | SO₂NHEt | |
| 16-130 | pyrimidin-5-yl | H | H | Me | H | NH₂ | Me | SO₂NHEt | |
| 16-131 | pyrimidin-5-yl | H | H | Me | H | NH₂ | Et | SO₂NHEt | |
| 16-132 | pyrimidin-5-yl | H | H | Me | H | NH₂ | i-Pr | SO₂NHEt | |
| 16-133 | thiazol-5-yl | H | H | Me | Me | NH₂ | Me | SO₂NHEt | |
| 16-134 | thiazol-5-yl | H | H | Me | Me | NH₂ | Et | SO₂NHEt | |
| 16-135 | thiazol-5-yl | H | H | Me | Me | NH₂ | i-Pr | SO₂NHEt | |
| 16-136 | thiazol-4-yl | H | H | Me | Me | NH₂ | Me | SO₂NHEt | |
| 16-137 | thiazol-4-yl | H | H | Me | Me | NH₂ | Et | SO₂NHEt | |
| 16-138 | thiazol-4-yl | H | H | Me | Me | NH₂ | i-Pr | SO₂NHEt | |
| 16-139 | thiazol-2-yl | H | H | Me | Me | NH₂ | Me | SO₂NHEt | |
| 16-140 | thiazol-2-yl | H | H | Me | Me | NH₂ | Et | SO₂NHEt | |
| 16-141 | thiazol-2-yl | H | H | Me | Me | NH₂ | i-Pr | SO₂NHEt | |
| 16-142 | thiophen-2-yl | H | H | Me | Me | NH₂ | Me | SO₂NHEt | |
| 16-143 | thiophen-2-yl | H | H | Me | Me | NH₂ | Et | SO₂NHEt | |
| 16-144 | thiophen-2-yl | H | H | Me | Me | NH₂ | i-Pr | SO₂NHEt | |
| 16-145 | thiophen-3-yl | H | H | Me | Me | NH₂ | Me | SO₂NHEt | |
| 16-146 | thiophen-3-yl | H | H | Me | Me | NH₂ | Et | SO₂NHEt | |
| 16-147 | thiophen-3-yl | H | H | Me | Me | NH₂ | i-Pr | SO₂NHEt | |
| 16-148 | pyridazin-3-yl | H | H | Me | Me | NH₂ | Me | SO₂NHEt | |
| 16-149 | pyridazin-3-yl | H | H | Me | Me | NH₂ | Et | SO₂NHEt | |
| 16-150 | pyridazin-3-yl | H | H | Me | Me | NH₂ | i-Pr | SO₂NHEt | |

**[Table 16-6]**

| Compound No. | R¹ | R² | R^{2a} | R³ | R^{3a} | R⁴ | R⁵ | A | Physical Property value |
|---|---|---|---|---|---|---|---|---|---|
| 16-151 | pyrazin-2-yl | H | H | Me | Me | NH₂ | Me | SO₂NHEt | |
| 16-152 | pyrazin-2-yl | H | H | Me | Me | NH₂ | Et | SO₂NHEt | |
| 16-153 | pyrazin-2-yl | H | H | Me | Me | NH₂ | i-Pr | SO₂NHEt | |
| 16-154 | pyrimidin-2-yl | H | H | Me | Me | NH₂ | Me | SO₂NHEt | |
| 16-155 | pyrimidin-2-yl | H | H | Me | Me | NH₂ | Et | SO₂NHEt | |
| 16-156 | pyrimidin-2-yl | H | H | Me | Me | NH₂ | i-Pr | SO₂NHEt | |
| 16-157 | pyrimidin-4-yl | H | H | Me | Me | NH₂ | Me | SO₂NHEt | |
| 16-158 | pyrimidin-4-yl | H | H | Me | Me | NH₂ | Et | SO₂NHEt | |
| 16-159 | pyrimidin-4-yl | H | H | Me | Me | NH₂ | i-Pr | SO₂NHEt | |
| 16-160 | pyrimidin-5-yl | H | H | Me | Me | NH₂ | Me | SO₂NHEt | |
| 16-161 | pyrimidin-5-yl | H | H | Me | Me | NH₂ | Et | SO₂NHEt | |
| 16-162 | pyrimidin-5-yl | H | H | Me | Me | NH₂ | i-Pr | SO₂NHEt | |
| 16-163 | Ph | H | H | H | H | NH₂ | Me | SO₂NHEt | |
| 16-164 | Ph | H | H | H | H | NH₂ | Et | SO₂NHEt | |
| 16-165 | Ph | H | H | H | H | NH₂ | i-Pr | SO₂NHEt | |
| 16-166 | 2,6-F₂-Ph | H | H | H | H | NH₂ | Me | SO₂NHEt | |
| 16-167 | 2,6-F₂-Ph | H | H | H | H | NH₂ | Et | SO₂NHEt | |
| 16-168 | 2,6-F₂-Ph | H | H | H | H | NH₂ | i-Pr | SO₂NHEt | |
| 16-169 | thiophen-2-yl | H | H | H | H | NH₂ | Me | SO₂NHEt | |
| 16-170 | thiophen-2-yl | H | H | H | H | NH₂ | Et | SO₂NHEt | |
| 16-171 | thiophen-2-yl | H | H | H | H | NH₂ | i-Pr | SO₂NHEt | |
| 16-172 | thiophen-3-yl | H | H | H | H | NH₂ | Me | SO₂NHEt | |
| 16-173 | thiophen-3-yl | H | H | H | H | NH₂ | Et | SO₂NHEt | |
| 16-174 | thiophen-3-yl | H | H | H | H | NH₂ | i-Pr | SO₂NHEt | |

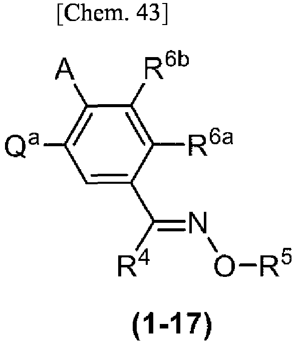

Table 17 (In Table 17, R^{6a} and R^{6b} represent hydrogen atoms, and each solid circle represents a binding position.)

**[Table 17-1]**

| Compound No. | Q^{a} | R⁴ | R⁵ | A | Physical Property value |
|---|---|---|---|---|---|
| 17-1 | 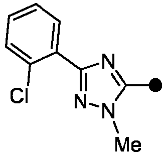 | NH₂ | Me | SO₂NHMe | 182-183 |
| 17-2 | 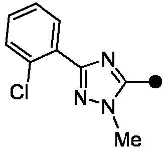 | NH₂ | Et | SO₂NHMe | 167-168 |
| 17-3 | 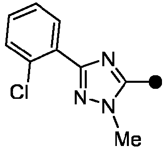 | NH₂ | i-Pr | SO₂NHMe | 199-200 |
| 17-4 | 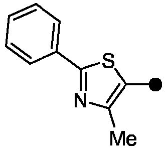 | NH₂ | Et | CO₂Me | NMR |
| 17-5 | 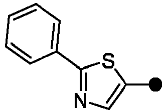 | NH₂ | Et | CO₂Me | NMR |
| 17-6 | 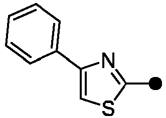 | NH₂ | Et | CO₂Et | NMR |
| 17-7 | 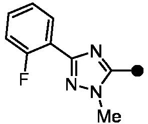 | NH₂ | Me | SO₂NHMe | |

**[Table 17-2]**

| Compound No. | Q^{a} | R⁴ | R⁵ | A | Physical Property value |
|---|---|---|---|---|---|
| 17-8 | 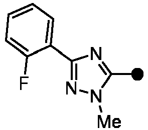 | NH₂ | Et | SO₂NHMe | |
| 17-9 | 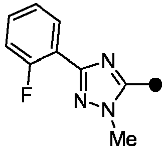 | NH₂ | i-Pr | SO₂NHMe | |
| 17-10 | 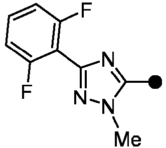 | NH₂ | Me | SO₂NHMe | |
| 17-11 | 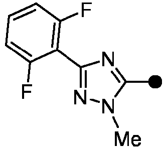 | NH₂ | Et | SO₂NHMe | |
| 17-12 | 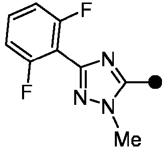 | NH₂ | i-Pr | SO₂NHMe | |
| 17-13 | 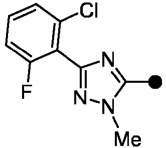 | NH₂ | Me | SO₂NHMe | |
| 17-14 | 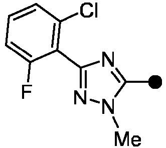 | NH₂ | Et | SO₂NHMe | |

**[Table 17-3]**

| Compound No. | Q^{a} | R⁴ | R⁵ | A | Physical Property value |
|---|---|---|---|---|---|
| 17-15 | 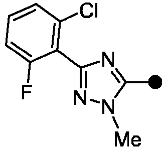 | NH₂ | i-Pr | SO₂NHMe | |
| 17-16 | 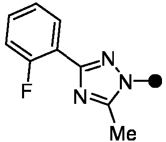 | NH₂ | Me | SO₂NHMe | |
| 17-17 | 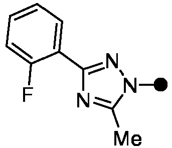 | NH₂ | Et | SO₂NHMe | |
| 17-18 | 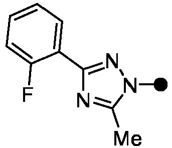 | NH₂ | i-Pr | SO₂NHMe | |
| 17-19 | 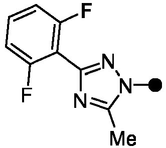 | NH₂ | Me | SO₂NHMe | |
| 17-20 | 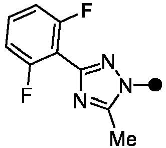 | NH₂ | Et | SO₂NHMe | |
| 17-21 | 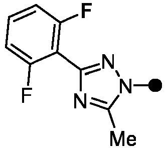 | NH₂ | i-Pr | SO₂NHMe | |

**[Table 17-4]**

| Compound No. | Q^{a} | R⁴ | R⁵ | A | Physical Property value |
|---|---|---|---|---|---|
| 17-22 | 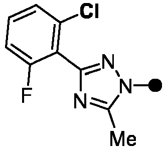 | NH₂ | Me | SO₂NHMe | |
| 17-23 | 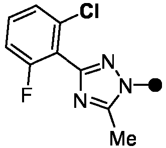 | NH₂ | Et | SO₂NHMe | |
| 17-24 | 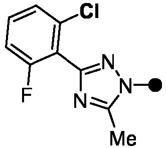 | NH₂ | i-Pr | SO₂NHMe | |
| 17-25 | 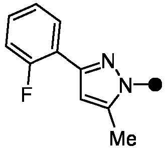 | NH₂ | Me | SO₂NHMe | |
| 17-26 | 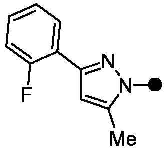 | NH₂ | Et | SO₂NHMe | |
| 17-27 | 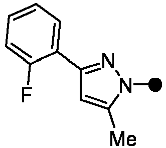 | NH₂ | i-Pr | SO₂NHMe | |
| 17-28 | 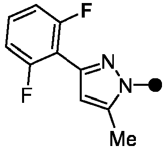 | NH₂ | Me | SO₂NHMe | |

**[Table 17-5]**

| Compound No. | Q^{a} | R⁴ | R⁵ | A | Physical Property value |
|---|---|---|---|---|---|
| 17-29 | 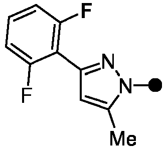 | NH₂ | Et | SO₂NHMe | |
| 17-30 | 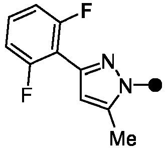 | NH₂ | i-Pr | SO₂NHMe | |
| 17-31 | 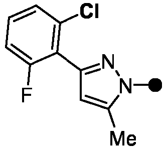 | NH₂ | Me | SO₂NHMe | |
| 17-32 | 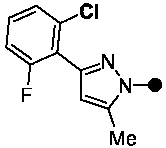 | NH₂ | Et | SO₂NHMe | |
| 17-33 | 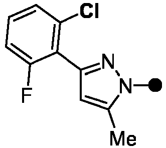 | NH₂ | i-Pr | SO₂NHMe | |

**[Table 17-6]**

| Compound No. | Q^{a} | R⁴ | R⁵ | A | Physical Property value |
|---|---|---|---|---|---|
| 17-34 | 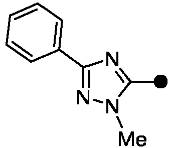 | NH₂ | Et | SO₂NHMe | |
| 17-35 | 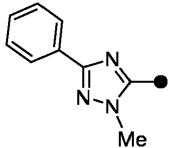 | NH₂ | Et | SO₂Me | |
| 17-36 | 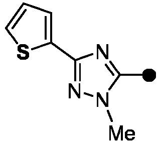 | NH₂ | Et | SO₂NHMe | |
| 17-37 | 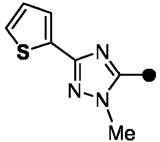 | NH₂ | Et | SO₂Me | |
| 17-38 | 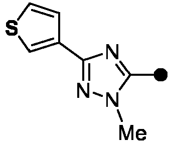 | NH₂ | Et | SO₂NHMe | |
| 17-39 | 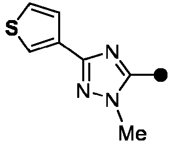 | NH₂ | Et | SO₂Me | |
| 17-40 | 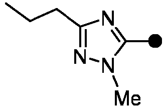 | NH₂ | Et | SO₂NHMe | |
| 17-41 | 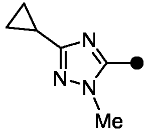 | NH2 | Et | SO2NHMe | |

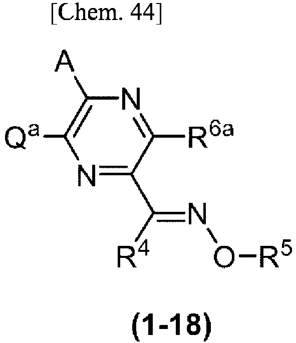

Table 18 (In Table 18, R^{6a} represents a hydrogen atom, and each solid circle represents a binding position.)

**[Table 18-1]**

| Compound No. | Q^{a} | R⁴ | R⁵ | A | Physical Property value |
|---|---|---|---|---|---|
| 18-1 | 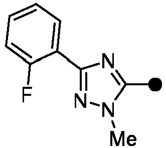 | NH₂ | Me | SO₂NHMe | |
| 18-2 | 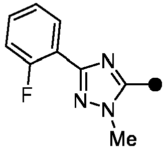 | NH₂ | Et | SO₂NHMe | |
| 18-3 | 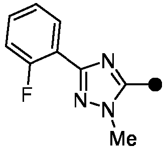 | NH₂ | i-Pr | SO₂NHMe | |
| 18-4 | 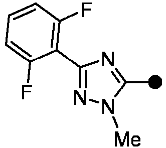 | NH₂ | Me | SO₂NHMe | |
| 18-5 | 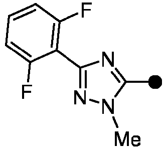 | NH₂ | Et | SO₂NHMe | |
| 18-6 | 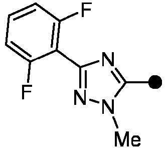 | NH₂ | i-Pr | SO₂NHMe | |
| 18-7 | 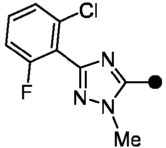 | NH₂ | Me | SO₂NHMe | |

**[Table 18-2]**

| Compound No. | Q^{a} | R⁴ | R⁵ | A | Physical Property value |
|---|---|---|---|---|---|
| 18-8 | 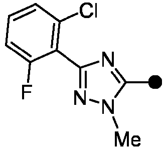 | NH₂ | Et | SO₂NHMe | |
| 18-9 | 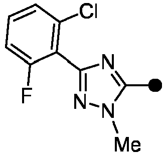 | NH₂ | i-Pr | SO₂NHMe | |
| 18-10 | 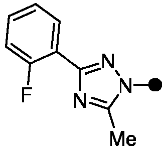 | NH₂ | Me | SO₂NHMe | |
| 18-11 | 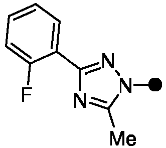 | NH₂ | Et | SO₂NHMe | |
| 18-12 | 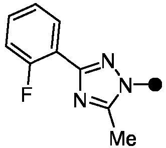 | NH₂ | i-Pr | SO₂NHMe | |
| 18-13 | 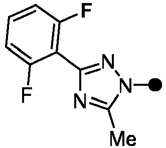 | NH₂ | Me | SO₂NHMe | |
| 18-14 | 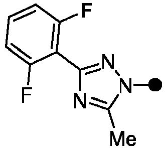 | NH₂ | Et | SO₂NHMe | |

**[Table 18-3]**

| Compound No. | Q^{a} | R⁴ | R⁵ | A | Physical Property value |
|---|---|---|---|---|---|
| 18-15 | 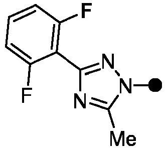 | NH₂ | i-Pr | SO₂NHMe | |
| 18-16 | 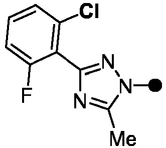 | NH₂ | Me | SO₂NHMe | |
| 18-17 | 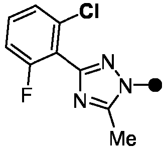 | NH₂ | Et | SO₂NHMe | |
| 18-18 | 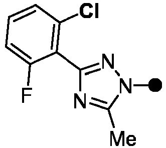 | NH₂ | i-Pr | SO₂NHMe | |
| 18-19 | 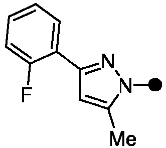 | NH₂ | Me | SO₂NHMe | |
| 18-20 | 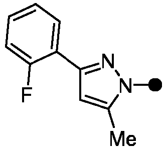 | NH₂ | Et | SO₂NHMe | |
| 18-21 | 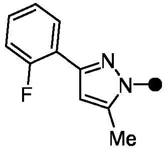 | NH₂ | i-Pr | SO₂NHMe | |

**[Table 18-4]**

| Compound No. | Q^{a} | R⁴ | R⁵ | A | Physical Property value |
|---|---|---|---|---|---|
| 18-22 | 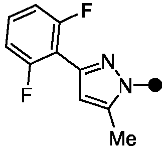 | NH₂ | Me | SO₂NHMe | |
| 18-23 | 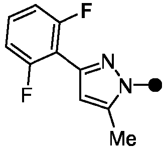 | NH₂ | Et | SO₂NHMe | |
| 18-24 | 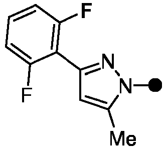 | NH₂ | i-Pr | SO₂NHMe | |
| 18-25 | 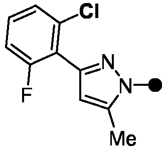 | NH₂ | Me | SO₂NHMe | |
| 18-26 | 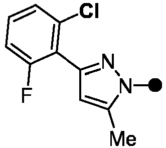 | NH₂ | Et | SO₂NHMe | |
| 18-27 | 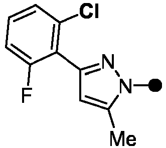 | NH₂ | i-Pr | SO₂NHMe | |

**[Table 18-5]**

| Compound No. | Q^{a} | R⁴ | R⁵ | A | Physical Property value |
|---|---|---|---|---|---|
| 18-28 | 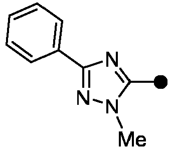 | NH₂ | Et | SO₂NHMe | |
| 18-29 | 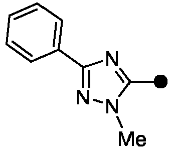 | NH₂ | Et | SO₂Me | |
| 18-30 | 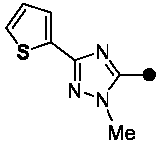 | NH₂ | Et | SO₂NHMe | |
| 18-31 | 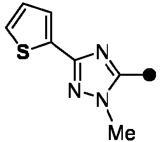 | NH₂ | Et | SO₂Me | |
| 18-32 | 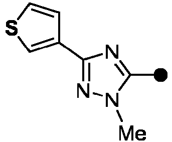 | NH₂ | Et | SO₂NHMe | |
| 18-33 | 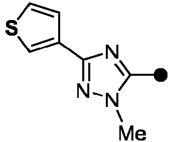 | NH₂ | Et | SO₂Me | |
| 18-34 | 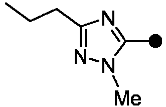 | NH₂ | Et | SO₂NHMe | |
| 18-35 | 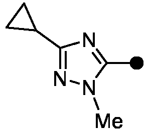 | NH2 | Et | SO2NHMe | |

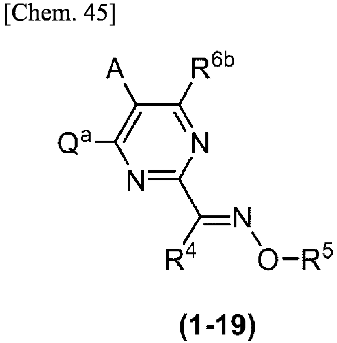

Table 19 (In Table 19, R^{6b} represents a hydrogen atom, and each solid circle represents a binding position.)

**[Table 19-1]**

| Compound No. | Q^{a} | R⁴ | R⁵ | A | Physical Property value |
|---|---|---|---|---|---|
| 19-1 | 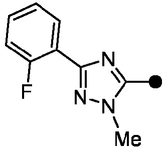 | NH₂ | Me | SO₂NHMe | |
| 19-2 | 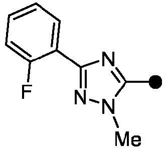 | NH₂ | Et | SO₂NHMe | |
| 19-3 | 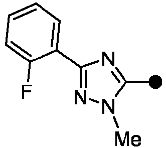 | NH₂ | i-Pr | SO₂NHMe | |
| 19-4 | 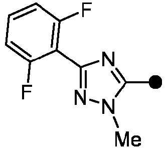 | NH₂ | Me | SO₂NHMe | |
| 19-5 | 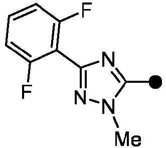 | NH₂ | Et | SO₂NHMe | |
| 19-6 | 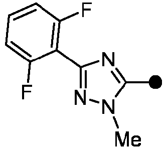 | NH₂ | i-Pr | SO₂NHMe | |

**[Table 19-2]**

| Compound No. | Q^{a} | R⁴ | R⁵ | A | Physical Property value |
|---|---|---|---|---|---|
| 19-7 | 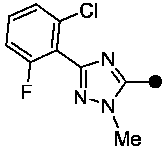 | NH₂ | Me | SO₂NHMe | |
| 19-8 | 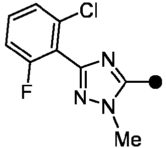 | NH₂ | Et | SO₂NHMe | |
| 19-9 | 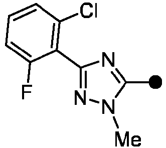 | NH₂ | i-Pr | SO₂NHMe | |
| 19-10 | 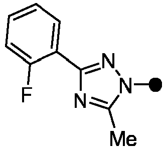 | NH₂ | Me | SO₂NHMe | |
| 19-11 | 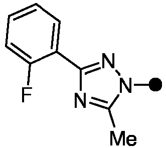 | NH₂ | Et | SO₂NHMe | |
| 19-12 | 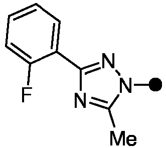 | NH₂ | i-Pr | SO₂NHMe | |
| 19-13 | 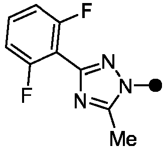 | NH₂ | Me | SO₂NHMe | |

**[Table 19-3]**

| Compound No. | Q^{a} | R⁴ | R⁵ | A | Physical Property value |
|---|---|---|---|---|---|
| 19-14 | 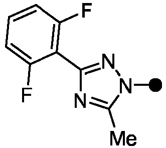 | NH₂ | Et | SO₂NHMe | |
| 19-15 | 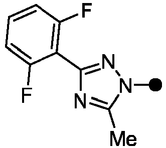 | NH₂ | i-Pr | SO₂NHMe | |
| 19-16 | 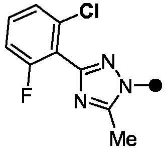 | NH₂ | Me | SO₂NHMe | |
| 19-17 | 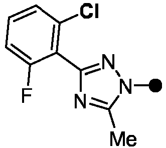 | NH₂ | Et | SO₂NHMe | |
| 19-18 | 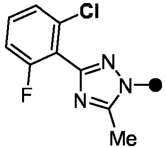 | NH₂ | i-Pr | SO₂NHMe | |
| 19-19 | 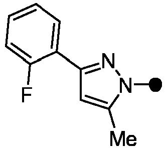 | NH₂ | Me | SO₂NHMe | |
| 19-20 | 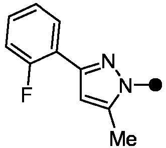 | NH₂ | Et | SO₂NHMe | |

**[Table 19-4]**

| Compound No. | Q^{a} | R⁴ | R⁵ | A | Physical Property value |
|---|---|---|---|---|---|
| 19-21 | 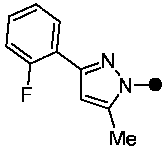 | NH₂ | i-Pr | SO₂NHMe | |
| 19-22 | 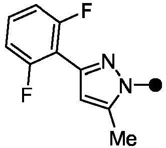 | NH₂ | Me | SO₂NHMe | |
| 19-23 | 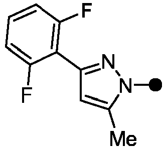 | NH₂ | Et | SO₂NHMe | |
| 19-24 | 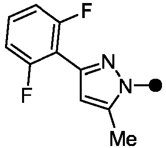 | NH₂ | i-Pr | SO₂NHMe | |
| 19-25 | 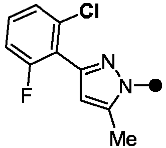 | NH₂ | Me | SO₂NHMe | |
| 19-26 | 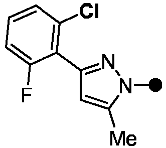 | NH₂ | Et | SO₂NHMe | |
| 19-27 | 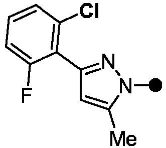 | NH₂ | i-Pr | SO₂NHMe | |

**[Table 19-5]**

| Compound No. | Q^{a} | R⁴ | R⁵ | A | Physical Property value |
|---|---|---|---|---|---|
| 19-28 | 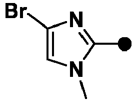 | NH₂ | Et | SO₂Me | NMR |
| 19-29 | 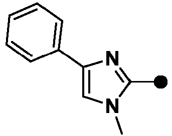 | NH₂ | Et | SO₂Me | NMR |
| 19-30 | 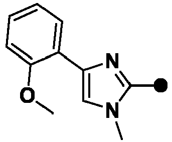 | NH₂ | Et | SO₂Me | NMR |
| 19-31 | 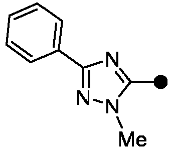 | NH₂ | Et | SO₂NHMe | |
| 19-32 | 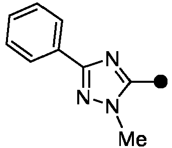 | NH₂ | Et | SO₂Me | |
| 19-33 | 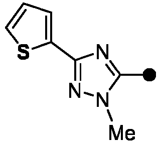 | NH₂ | Et | SO₂NHMe | |
| 19-34 | 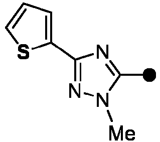 | NH₂ | Et | SO₂Me | |

**[Table 19-6]**

| Compound No. | Q^{a} | R⁴ | R⁵ | A | Physical Property value |
|---|---|---|---|---|---|
| 19-35 | 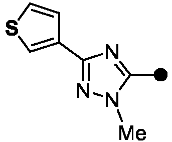 | NH₂ | Et | SO₂NHMe | |
| 19-36 | 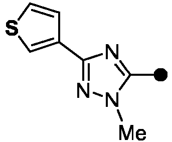 | NH₂ | Et | SO₂Me | |
| 19-37 | 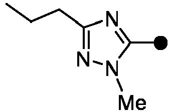 | NH₂ | Et | SO₂NHMe | |
| 19-38 | 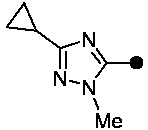 | NH₂ | Et | SO₂NHMe | |

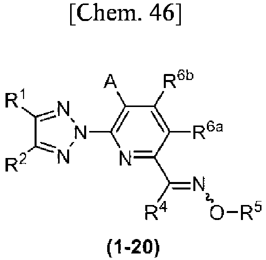

Table 20 (In Table 20, R^{6a} and R^{6b} represent hydrogen atoms.)

**[Table 20-1]**

| Compound No. | R¹ | R² | R⁴ | R⁵ | A | Physical Property value |
|---|---|---|---|---|---|---|
| 20-1 | Ph | H | NH₂ | Me | SO₂NHMe | |
| 20-2 | Ph | H | NH₂ | Et | SO₂NHMe | 38-40 |
| 20-3 | Ph | H | NH₂ | i-Pr | SO₂NHMe | |
| 20-4 | 2-F-Ph | H | NH₂ | Me | SO₂NHMe | |
| 20-5 | 2-F-Ph | H | NH₂ | Et | SO₂NHMe | |
| 20-6 | 2-F-Ph | H | NH₂ | i-Pr | SO₂NHMe | |
| 20-7 | 3-F-Ph | H | NH₂ | Me | SO₂NHMe | |
| 20-8 | 3-F-Ph | H | NH₂ | Et | SO₂NHMe | |
| 20-9 | 3-F-Ph | H | NH₂ | i-Pr | SO₂NHMe | |
| 20-10 | 4-F-Ph | H | NH₂ | Me | SO₂NHMe | |
| 20-11 | 4-F-Ph | H | NH₂ | Et | SO₂NHMe | |
| 20-12 | 4-F-Ph | H | NH₂ | i-Pr | SO₂NHMe | |
| 20-13 | 2,6-F₂-Ph | H | NH₂ | Me | SO₂NHMe | |
| 20-14 | 2,6-F₂-Ph | H | NH₂ | Et | SO₂NHMe | |
| 20-15 | 2,6-F₂-Ph | H | NH₂ | i-Pr | SO₂NHMe | |
| 20-16 | Ph | Me | NH₂ | Me | SO₂NHMe | |
| 20-17 | Ph | Me | NH₂ | Et | SO₂NHMe | 72-77 |
| 20-18 | Ph | Me | NH₂ | i-Pr | SO₂NHMe | |
| 20-19 | 2-F-Ph | Me | NH₂ | Me | SO₂NHMe | |
| 20-20 | 2-F-Ph | Me | NH₂ | Et | SO₂NHMe | NMR |
| 20-21 | 2-F-Ph | Me | NH₂ | i-Pr | SO₂NHMe | |
| 20-22 | 3-F-Ph | Me | NH₂ | Me | SO₂NHMe | |
| 20-23 | 3-F-Ph | Me | NH₂ | Et | SO₂NHMe | |
| 20-24 | 3-F-Ph | Me | NH₂ | i-Pr | SO₂NHMe | |
| 20-25 | 4-F-Ph | Me | NH₂ | Me | SO₂NHMe | |
| 20-26 | 4-F-Ph | Me | NH₂ | Et | SO₂NHMe | |
| 20-27 | 4-F-Ph | Me | NH₂ | i-Pr | SO₂NHMe | |
| 20-28 | 2,6-F₂-Ph | Me | NH₂ | Me | SO₂NHMe | |
| 20-29 | 2,6-F₂-Ph | Me | NH₂ | Et | SO₂NHMe | 38-43 |
| 20-30 | 2,6-F₂-Ph | Me | NH₂ | i-Pr | SO₂NHMe | |

**[Table 20-2]**

| Compound No. | R¹ | R² | R⁴ | R⁵ | A | Physical Property value |
|---|---|---|---|---|---|---|
| 20-31 | thiazol-5-yl | Me | NH₂ | Me | SO₂NHMe | |
| 20-32 | thiazol-5-yl | Me | NH₂ | Et | SO₂NHMe | |
| 20-33 | thiazol-5-yl | Me | NH₂ | i-Pr | SO₂NHMe | |
| 20-34 | thiazol-4-yl | Me | NH₂ | Me | SO₂NHMe | |
| 20-35 | thiazol-4-yl | Me | NH₂ | Et | SO₂NHMe | |
| 20-36 | thiazol-4-yl | Me | NH₂ | i-Pr | SO₂NHMe | |
| 20-37 | thiazol-2-yl | Me | NH₂ | Me | SO₂NHMe | |
| 20-38 | thiazol-2-yl | Me | NH₂ | Et | SO₂NHMe | |
| 20-39 | thiazol-2-yl | Me | NH₂ | i-Pr | SO₂NHMe | |
| 20-40 | thiophen-2-yl | Me | NH₂ | Me | SO₂NHMe | |
| 20-41 | thiophen-2-yl | Me | NH₂ | Et | SO₂NHMe | |
| 20-42 | thiophen-2-yl | Me | NH₂ | i-Pr | SO₂NHMe | |
| 20-43 | thiophen-3-yl | Me | NH₂ | Me | SO₂NHMe | |
| 20-44 | thiophen-3-yl | Me | NH₂ | Et | SO₂NHMe | |
| 20-45 | thiophen-3-yl | Me | NH₂ | i-Pr | SO₂NHMe | |
| 20-46 | pyridazin-3-yl | Me | NH₂ | Me | SO₂NHMe | |
| 20-47 | pyridazin-3-yl | Me | NH₂ | Et | SO₂NHMe | |
| 20-48 | pyridazin-3-yl | Me | NH₂ | i-Pr | SO₂NHMe | |
| 20-49 | pyrazin-2-yl | Me | NH₂ | Me | SO₂NHMe | |
| 20-50 | pyrazin-2-yl | Me | NH₂ | Et | SO₂NHMe | |
| 20-51 | pyrazin-2-yl | Me | NH₂ | i-Pr | SO₂NHMe | |
| 20-52 | pyrimidin-2-yl | Me | NH₂ | Me | SO₂NHMe | |
| 20-53 | pyrimidin-2-yl | Me | NH₂ | Et | SO₂NHMe | |
| 20-54 | pyrimidin-2-yl | Me | NH₂ | i-Pr | SO₂NHMe | |
| 20-55 | pyrimidin-4-yl | Me | NH₂ | Me | SO₂NHMe | |
| 20-56 | pyrimidin-4-yl | Me | NH₂ | Et | SO₂NHMe | |
| 20-57 | pyrimidin-4-yl | Me | NH₂ | i-Pr | SO₂NHMe | |
| 20-58 | pyrimidin-5-yl | Me | NH₂ | Me | SO₂NHMe | |
| 20-59 | pyrimidin-5-yl | Me | NH₂ | Et | SO₂NHMe | |
| 20-60 | pyrimidin-5-yl | Me | NH₂ | i-Pr | SO₂NHMe | |

**[Table 20-3]**

| Compound No. | R¹ | R² | R⁴ | R⁵ | A | Physical Property value |
|---|---|---|---|---|---|---|
| 20-61 | pyridin-2-yl | H | NH₂ | Et | SO₂NHMe | 75-80 |
| 20-62 | 2-F-Ph | Me | NH₂ | Et | SO₂Me | 59-64 |
| 20-63 | 2,6-F₂-Ph | Me | NH₂ | Et | SO₂Me | 50-55 |
| 20-64 | 3-Cl-pyrimidin-2-yl | Me | NH₂ | Et | SO₂Me | |
| 20-65 | 3-Cl-pyrimidin-2-yl | Me | NH₂ | Et | SO₂NHMe | |
| 20-66 | 3-Cl-pyridin-2-yl | H | NH₂ | Et | SO₂NHMe | 52-57 |

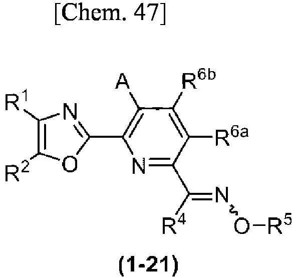

Table 21 (In Table 21, R^{6a} and R^{6b} represent hydrogen atoms.)

**[Table 21-1]**

| Compound No. | R¹ | R² | R⁴ | R⁵ | A | Physical Property value |
|---|---|---|---|---|---|---|
| 21-1 | Ph | H | NH₂ | Me | SO₂NHMe | |
| 21-2 | Ph | H | NH₂ | Et | SO₂NHMe | 165-166 |
| 21-3 | Ph | H | NH₂ | i-Pr | SO₂NHMe | |
| 21-4 | 2-F-Ph | H | NH₂ | Me | SO₂NHMe | |
| 21-5 | 2-F-Ph | H | NH₂ | Et | SO₂NHMe | |
| 21-6 | 2-F-Ph | H | NH₂ | i-Pr | SO₂NHMe | |
| 21-7 | 3-F-Ph | H | NH₂ | Me | SO₂NHMe | |
| 21-8 | 3-F-Ph | H | NH₂ | Et | SO₂NHMe | |
| 21-9 | 3-F-Ph | H | NH₂ | i-Pr | SO₂NHMe | |
| 21-10 | 4-F-Ph | H | NH₂ | Me | SO₂NHMe | |
| 21-11 | 4-F-Ph | H | NH₂ | Et | SO₂NHMe | |
| 21-12 | 4-F-Ph | H | NH₂ | i-Pr | SO₂NHMe | |
| 21-13 | 2,6-F₂-Ph | H | NH₂ | Me | SO₂NHMe | |
| 21-14 | 2,6-F₂-Ph | H | NH₂ | Et | SO₂NHMe | |
| 21-15 | 2,6-F₂-Ph | H | NH₂ | i-Pr | SO₂NHMe | |
| 21-16 | Ph | Me | NH₂ | Me | SO₂NHMe | |
| 21-17 | Ph | Me | NH₂ | Et | SO₂NHMe | |
| 21-18 | Ph | Me | NH₂ | i-Pr | SO₂NHMe | |
| 21-19 | 2-F-Ph | Me | NH₂ | Me | SO₂NHMe | |
| 21-20 | 2-F-Ph | Me | NH₂ | Et | SO₂NHMe | |
| 21-21 | 2-F-Ph | Me | NH₂ | i-Pr | SO₂NHMe | |
| 21-22 | 3-F-Ph | Me | NH₂ | Me | SO₂NHMe | |
| 21-23 | 3-F-Ph | Me | NH₂ | Et | SO₂NHMe | |
| 21-24 | 3-F-Ph | Me | NH₂ | i-Pr | SO₂NHMe | |
| 21-25 | 4-F-Ph | Me | NH₂ | Me | SO₂NHMe | |
| 21-26 | 4-F-Ph | Me | NH₂ | Et | SO₂NHMe | |
| 21-27 | 4-F-Ph | Me | NH₂ | i-Pr | SO₂NHMe | |
| 21-28 | 2,6-F₂-Ph | Me | NH₂ | Me | SO₂NHMe | |
| 21-29 | 2,6-F₂-Ph | Me | NH₂ | Et | SO₂NHMe | |
| 21-30 | 2,6-F₂-Ph | Me | NH₂ | i-Pr | SO₂NHMe | |

**[Table 21-2]**

| Compound No. | R¹ | R² | R⁴ | R⁵ | A | Physical Property value |
|---|---|---|---|---|---|---|
| 21-31 | thiazol-5-yl | Me | NH₂ | Me | SO₂NHMe | |
| 21-32 | thiazol-5-yl | Me | NH₂ | Et | SO₂NHMe | |
| 21-33 | thiazol-5-yl | Me | NH₂ | i-Pr | SO₂NHMe | |
| 21-34 | thiazol-4-yl | Me | NH₂ | Me | SO₂NHMe | |
| 21-35 | thiazol-4-yl | Me | NH₂ | Et | SO₂NHMe | |
| 21-36 | thiazol-4-yl | Me | NH₂ | i-Pr | SO₂NHMe | |
| 21-37 | thiophen-2-yl | Me | NH₂ | Me | SO₂NHMe | |
| 21-38 | thiophen-2-yl | Me | NH₂ | Et | SO₂NHMe | |
| 21-39 | thiophen-2-yl | Me | NH₂ | i-Pr | SO₂NHMe | |
| 21-40 | thiophen-3-yl | Me | NH₂ | Me | SO₂NHMe | |
| 21-41 | thiophen-3-yl | Me | NH₂ | Et | SO₂NHMe | |
| 21-42 | thiophen-3-yl | Me | NH₂ | i-Pr | SO₂NHMe | |
| 21-43 | pyridazin-3-yl | Me | NH₂ | Me | SO₂NHMe | |
| 21-44 | pyridazin-3-yl | Me | NH₂ | Et | SO₂NHMe | |
| 21-45 | pyridazin-3-yl | Me | NH₂ | i-Pr | SO₂NHMe | |
| 21-46 | pyrazin-2-yl | Me | NH₂ | Me | SO₂NHMe | |
| 21-47 | pyrazin-2-yl | Me | NH₂ | Et | SO₂NHMe | |
| 21-48 | pyrazin-2-yl | Me | NH₂ | i-Pr | SO₂NHMe | |
| 21-49 | pyrimidin-2-yl | Me | NH₂ | Me | SO₂NHMe | |
| 21-50 | pyrimidin-2-yl | Me | NH₂ | Et | SO₂NHMe | |
| 21-51 | pyrimidin-2-yl | Me | NH₂ | i-Pr | SO₂NHMe | |
| 21-52 | pyrimidin-4-yl | Me | NH₂ | Me | SO₂NHMe | |
| 21-53 | pyrimidin-4-yl | Me | NH₂ | Et | SO₂NHMe | |
| 21-54 | pyrimidin-4-yl | Me | NH₂ | i-Pr | SO₂NHMe | |
| 21-55 | pyrimidin-5-yl | Me | NH₂ | Me | SO₂NHMe | |
| 21-56 | pyrimidin-5-yl | Me | NH₂ | Et | SO₂NHMe | |
| 21-57 | pyrimidin-5-yl | Me | NH₂ | i-Pr | SO₂NHMe | |
| 21-58 | thiazol-5-yl | H | NH₂ | Me | SO₂NHMe | |
| 21-59 | thiazol-5-yl | H | NH₂ | Et | SO₂NHMe | |
| 21-60 | thiazol-5-yl | H | NH₂ | i-Pr | SO₂NHMe | |

**[Table 21-3]**

| Compound No. | R¹ | R² | R⁴ | R⁵ | A | Physical Property value |
|---|---|---|---|---|---|---|
| 21-61 | thiazol-4-yl | H | NH₂ | Me | SO₂NHMe | |
| 21-62 | thiazol-4-yl | H | NH₂ | Et | SO₂NHMe | |
| 21-63 | thiazol-4-yl | H | NH₂ | i-Pr | SO₂NHMe | |
| 21-64 | thiophen-2-yl | H | NH₂ | Me | SO₂NHMe | |
| 21-65 | thiophen-2-yl | H | NH₂ | Et | SO₂NHMe | |
| 21-66 | thiophen-2-yl | H | NH₂ | i-Pr | SO₂NHMe | |
| 21-67 | thiophen-3-yl | H | NH₂ | Me | SO₂NHMe | |
| 21-68 | thiophen-3-yl | H | NH₂ | Et | SO₂NHMe | |
| 21-69 | thiophen-3-yl | H | NH₂ | i-Pr | SO₂NHMe | |
| 21-70 | pyridazin-3-yl | H | NH₂ | Me | SO₂NHMe | |
| 21-71 | pyridazin-3-yl | H | NH₂ | Et | SO₂NHMe | |
| 21-72 | pyridazin-3-yl | H | NH₂ | i-Pr | SO₂NHMe | |
| 21-73 | pyrazin-2-yl | H | NH₂ | Me | SO₂NHMe | |
| 21-74 | pyrazin-2-yl | H | NH₂ | Et | SO₂NHMe | |
| 21-75 | pyrazin-2-yl | H | NH₂ | i-Pr | SO₂NHMe | |
| 21-76 | pyrimidin-2-yl | H | NH₂ | Me | SO₂NHMe | |
| 21-77 | pyrimidin-2-yl | H | NH₂ | Et | SO₂NHMe | |
| 21-78 | pyrimidin-2-yl | H | NH₂ | i-Pr | SO₂NHMe | |
| 21-79 | pyrimidin-4-yl | H | NH₂ | Me | SO₂NHMe | |
| 21-80 | pyrimidin-4-yl | H | NH₂ | Et | SO₂NHMe | |
| 21-81 | pyrimidin-4-yl | H | NH₂ | i-Pr | SO₂NHMe | |
| 21-82 | pyrimidin-5-yl | H | NH₂ | Me | SO₂NHMe | |
| 21-83 | pyrimidin-5-yl | H | NH₂ | Et | SO₂NHMe | |
| 21-84 | pyrimidin-5-yl | H | NH₂ | i-Pr | SO₂NHMe | |
| 21-85 | Ph | H | NH₂ | Me | SO₂NHEt | |
| 21-86 | Ph | H | NH₂ | Et | SO₂NHEt | |
| 21-87 | Ph | H | NH₂ | i-Pr | SO₂NHEt | |
| 21-88 | 2,6-F₂-Ph | H | NH₂ | Me | SO₂NHEt | |
| 21-89 | 2,6-F₂-Ph | H | NH₂ | Et | SO₂NHEt | |
| 21-90 | 2,6-F₂-Ph | H | NH₂ | i-Pr | SO₂NHEt | |

Table 22 (In Table 22, * indicates ¹H-NMR data in DMSO-d₆.)

**[Table 22-1]**

| Compound No. | ¹H-NMR Data (CDCl₃/TMS, ppm) |
|---|---|
| 1-1 | δ 8.47(d, 1H), 8.27(d, 1H), 7.94-7.89(m, 1H), 7.86-7.80(m, 1H), 7.54-7.50(m, 1H), 7.40-7.36(m, 2H), 5.47(s, 2H), 4.25(q, 2H), 4.16(s, 3H), 2.74(d, 3H), 1.37(t, 3H) |
| 1-2 | δ 8.27(d, 1H), 8.13-8.06(m, 2H), 7.90(s, 1H), 7.81 (d, 1H), 7.47-7.34(m, 3H), 4.32-4.26(q, 2H), 4.25-4.18(q, 2H), 3.99(s, 3H), 2.18(s, 3H), 1.37(t, 3H), 1.10(t, 3H) |
| 1-4 | δ 8.46(d, 1H), 8.26(d, 1H), 8.03-7.99(m, 2H), 7.79-7.73(m, 1H), 7.51-7.43(m, 3H), 5.44(s, 2H), 4.24(q, 2H), 4.10(s, 1H), 2.79(d, 3H), 1.37(t, 3H) |
| 1-8 | δ 8.26(d, 1H), 8.20(d, 1H), 8.13-8.10(m, 2H), 7.47-7.39(m, 3H), 5.49(s, 2H), 4.29-4.16(m, 4H), 3.98(s, 3H), 1.36(t, 3H), 1.13(t, 3H) |
| 1-12 | δ 8.79(d, 1H), 8.34(d, 1H), 7.93-7.90(m, 1H), 7.53-7.50(m, 1H), 7.38-7.35(m, 2H), 4.44(q, 2H), 3.85(s, 3H), 3.05(s, 3H), 2.32(s, 9H), 2.16(s, 3H), 1.40(t, 3H) |
| 1-18 | δ 8.37-8.24(m, 2H), 8.15-8.06(m, 2H), 7.49-7.34(m, 4H), 5.43(s, 2H), 4.47-4.35(m, 1H), 3.77(s, 3H), 2.75(s, 6H), 1.33(d, 6H) |
| 1-20 | δ 8.33-8.32(m, 2H), 7.95-7.91 (m, 1H), 7.52-7.49(m, 1H), 7.36-7.32(m, 2H), 5.45(s, 2H), 4.47-4.37(m, 1H), 3.82(s, 3H), 2.75(s, 6H), 1.33(d, 3H) |
| 1-21 | δ 8.49(d, 1H), 8.28-8.21 (m, 2H), 7.91 (d, 1H), 7.45-7.39(m, 1H), 7.08-7.04(m, 2H), 5.48(s, 2H), 4.14(s, 3H), 3.96(s, 3H), 3.94(s, 3H), 2.76(d, 3H) |
| 1-22 | δ 8.49(d, 1H), 8.27-8.22(m, 2H), 7.91(dd, 1H), 7.45-7.40(m, 1H), 7.08-7.04(m, 2H), 5.45(s, 2H), 4.24(q, 2H), 4.14(s, 3H), 3.96(s, 3H), 2.76(d, 3H), 1.37(t, 3H) |
| 1-28 | δ 8.46(d, 1H), 8.27(d, 1H), 8.03-7.99(m, 2H), 7.75(q, 1H), 7.49-7.44(m, 3H), 5.41(s, 2H), 4.49-4.39(m, 1H), 4.10(s, 3H), 2.79(d, 3H), 1.35(d, 6H) |
| 1-32 | δ 8.37-8.28(m, 2H), 8.13-8.09(m, 1H), 7.47-7.39(m, 2H), 5.47(s, 2H), 4.23(m, 2H), 3.79(s, 3H), 2.75(s, 6H), 1.36(t, 3H) |
| 1-38 | δ 8.46(d, 1H), 8.27(d, 1H), 7.44-7.28(m, 4H), 7.18-7.12(m, 1H), 5.47(s, 2H), 4.28-4.22(m, 2H), 4.14(s, 3H), 2.71(d, 3H), 1.37(t, 3H) |
| 1-39 | δ 8.46(d, 1H), 8.27(d, 1H), 7.96-7.93(m, 2H), 7.56(q, 1H), 7.46-7.43(m, 2H), 5.44(s, 2H), 4.28-4.21(q, 2H), 4.08(s, 3H), 2.79(d, 3H), 1.37(t, 3H) |

**[Table 22-2]**

| Compound No. | ¹H-NMR Data (CDCl₃/TMS, ppm) |
|---|---|
| 1-40 | δ 8.46(d, 1H), 8.27(d, 1H), 7.96-7.93(m, 2H), 7.56(q, 1H), 7.46-7.43(m, 2H), 5.44(s, 2H), 4.28-4.21(q, 2H), 4.08(s, 3H), 2.79(d, 3H), 1.37(t, 3H) |
| 1-41 | δ 8.36-8.30(m, 2H), 7.91(d, 1H), 7.82(d, 1H), 7.44-7.36(m, 1H), 7.13-7.06(m, 1H), 5.43(s, 2H), 4.47-4.38(m, 1H), 3.79(s, 3H), 2.78(s, 6H), 1.33(d, 6H) |
| 1-42 | δ 8.33(q, 2H), 7.42-7.33(m, 1H), 7.05-6.99(m, 2H), 5.48(s, 2H), 4.22(q, 2H), 3.84(s, 3H), 2.75(s, 6H), 1.36(t, 3H) |
| 1-53 | δ 8.46(d, 1H), 8.26(d, 1H), 7.85-7.77(m, 2H), 5.46(s, 2H), 4.28-4.22(m, 2H),4.13(s, 3H), 2.72(d, 3H), 2.62(s, 3H), 1.37(t, 3H) |
| 1-188 | δ 8.85(s, 1H), 8.47-8.40(m, 2H), 8.29-8.26(m, 1H), 7.24-7.17(m, 1H).5.42(brs, 2H), 4.07(s, 1H), 4.01(s, 3H), 2.79(d, 3H) |
| 1-189 | δ 8.85(s, 1H), 8.47-8.40(m, 2H), 8.30-8.27(m, 1H), 7.24-7.18(m, 1H), 5.42(brs, 2H), 4.24(q, Hz, 2H), 4.07(s, 3H), 2.79(d, 3H), 1.37(t, 3H) |
| 1-200 | δ 8.47-8.44(m, 1H), 8.27-8.24(m, 1H), 7.85(dd, 1H), 7.68(q, 1H), 7.60(dd, 1H), 7.41(dd, 1H), 5.43(brs, 2H), 4.06(s, 3H), 4.00(s, 3H), 2.78(d, 3H) |
| 1-201 | δ 8.47-8.42(m, 1H), 8.27-8.23(m, 1H), 7.85(dd, 1H), 7.71-7.59(m, 2H), 7.42-7.38(m, 1H), 5.43(brs, 2H), 4.28-4.21(m, 2H), 4.07-4.05(s, 3H), 2.79-2.75(m, 3H), 1.39-1.33(m, 3H) |
| 1-206 | δ 8.48-8.45(m, 1H), 8.29-8.26(m, 1H), 7.97(d, 1H), 7.45(d, 1H), 7.26-7.22(m, 1H), 5.43(brs, 1H), 4.12(s, 3H), 4.01(s, 3H), 2.81(d, 3H) |
| 1-207 | δ 8.48-8.45(m, 1H), 8.30-8.27(m, 1H), 7.98-7.97(m, 1H), 7.46-7.45(m, 1H), 7.26-7.23(m, 1H), 5.43(brs, 2H), 4.25(q, 2H), 4.12(s, 3H), 2.82-2.80(m, 3H), 1.37(t, 3H) |
| 1-211 | δ 8.96-8.93(m, 1H), 8.50-8.45(m, 1H), 8.28-8.15(m, 2H), 7.63-7.53(m, 1H), 7.26-7.18(m, 1H), 5.45(brs, 2H), 4.16(s, 3H), 4.00(s, 3H), 2.73(dd, 3H) |
| 1-212 | δ 8.97-8.91(m, 1H), 8.47-8.44(m, 1H), 8.29-8.15(m, 2H), 7.61-7.53(m, 1H), 7.26-7.18(m, 1H), 5.47-5.43(brs, 2H), 4.25(q, 2H), 4.17(s, 3H), 2.73(d, 3H), 1.37(t, 3H) |
| 1-213 | δ 8.43(d, 1H), 8.23(d, 1H), 7.53-7.47(m, 1H), 5.43(brs, 2H), 4.00-3.99(m, 6H), 3.77(s, 2H), 2.74(d, 3H), 2.14(s, 3H) |
| 1-214 | δ 8.44-8.41(d, 1H), 8.26-8.23(d, 1H), 7.53-7.43(m, 1H), 5.43(brs, 2H), 4.24(q, 2H), 4.00(s, 3H), 3.77(s, 2H), 2.75-2.73(d, 3H), 2.15(s, 3H), 1.37(t, 3H) |

**[Table 22-3]**

| Compound No. | ¹H-NMR Data (CDCl₃/TMS, ppm) |
|---|---|
| 1-215 | δ 8.44-8.41(d, 1H), 8.28-8.26(d, 1H), 6.81-6.75(m, 1H), 5.44-5.40(brs, 2H), 4.45(s, 2H), 4.00-3.99(m, 6H), 2.95(s, 3H), 2.75(d, 3H) |
| 1-216 | δ 8.43-8.40(d, 1H), 8.27-8.24(d, 1H), 7.18(q, 2H), 5.42(brs, 2H), 4.27-4.20(m, 2H), 4.05(s, 2H), 3.98(s, 3H), 2.72(d, 3H), 2.63(s, 1H), 1.36(t, 3H) |
| 1-217 | δ 8.43-8.41(d, 1H), 8.29-8.26(d, 1H), 6.79(q, 1H), 5.42(brs, 2H), 4.45(s, 2H), 4.24(q, 2H), 4.00-3.99(s, 3H), 2.95(s, 3H), 2.75(d, 3H), 1.37(t, 3H) |
| 1-218 | δ 8.43(d, 1H), 8.22(d, 1H), 7.83(q, 1H), 5.42(brs, 2H), 4.00-3.98(m, 6H), 2.80-2.71(m, 5H), 1.66(m, 3H), 0.97-0.95(m, 6H) |
| 1-219 | δ 8.42(d, 1H), 8.21(d, 1H), 7.94(q, 1H), 5.42(brs, 2H), 4.00-3.99(m, 6H), 3.16-3.08(m, 1H), 2.74-2.72(m, 3H), 1.37(d, 6H) |
| 1-220 | δ 8.42(d, 1H), 8.22(d, 1H), 7.94(q, 1H), 5.42(brs, 2H), 4.23(q, 2H), 4.00(s, 3H), 3.16-3.08(m, 1H), 2.74-2.71(m, 3H), 1.38-1.34(m, 9H) |
| 1-221 | δ 8.42(d, 1H), 8.21(d, 1H), 7.65(q, 1H), 5.42(brs, 2H), 3.99(m, 6H), 3.78(t, 2H), 3.40(d, 3H), 3.05(t, 2H), 2.72(d, 3H) |
| 1-222 | δ 8.42(d, 1H), 8.22(d, 1H), 7.66(q, 1H), 5.42-5.39(brs, 2H), 4.23(q, 2H), 3.99(s, 3H), 3.78(t, 2H), 3.40-3.39(s, 3H), 3.05(t, 2H), 2.72(d, 3H), 1.36(t, 3H) |
| 1-223 | δ 8.42(d, 1H), 8.24(d, 1H), 7.44(q, 1H), 5.42(brs, 2H), 4.58(s, 2H), 4.27-4.21(m, 2H), 4.02(s, 3H), 3.46(s, 3H), 2.7(d, 3H), 1.39-1.34(t, 3H) |
| 1-224 | δ 8.42(d, 1H), 8.25(d, 1H), 7.44(q, 1H), 5.38(brs, 2H), 4.58(s, 2H), 4.48-4.38(m, 1H), 4.03-4.02(s, 3H), 3.46(s, 3H), 2.73(d, 3H), 1.34(d, 6H) |
| 1-227 | δ 8.57(s, 1H), 8.47-8.45(m, 1H), 8.28-8.25(m, 1H), 7.92-7.88(m, 1H), 7.66-7.61(m, 2H), 5.44(s, 2H), 4.24(q, 2H), 4.13(s, 3H), 2.81(d, 3H), 2.41(s, 3H), 1.37(t, 3H) |
| 1-228* | δ 8.56-8.54(m, 1H), 8.46-8.43(m, 1H), 8.24-8.21(m, 1H), 8.01-7.98(m, 1H), 7.91-7.86(m, 1H), 7.78(dd, 1H), 6.34(brs, 2H), 4.02(s, 3H), 3.87(s, 3H), 2.63(d, 3H), 2.37(s, 3H) |
| 1-229 | δ 8.77-8.74(m, 1H), 8.49-8.46(m, 1H), 8.28-8.25(m, 1H), 8.05-8.00(m, 1H), 7.86-7.79(m, 1H), 7.60(q, 1H), 7.40-7.34(m, 1H), 5.44(brs, 2H), 4.14(s, 3H), 4.00(s, 3H), 2.82(d, 3H) |
| 1-234 | δ 8.45(dd, 1H), 8.26(dd, 1H), 7.64(dd, 1H), 7.41-7.37(m, 1H), 7.13(dd, 1H), 6.95-6.93(m, 1H), 5.45-5.40(brs, 2H), 4.24(q, 2H), 4.06(m, 3H), 2.80-2.77(m, 3H), 1.37(t, 3H) |
| 1-236 | δ 8.44-8.42(m, 1H), 8.23-8.20(m, 1H), 7.87(q, 1H), 5.43(brs, 2H), 4.00-3.99(m, 6H), 2.77-2.71(m, 5H), 1.83-1.76(m, 2H), 1.02(t, 3H) |

**[Table 22-4]**

| Compound No. | ¹H-NMR Data (CDCl₃/TMS, ppm) |
|---|---|
| 1-237 | δ 8.44-8.41(m, 1H), 8.24-8.21(m, 1H), 7.87(q, 1H), 5.43(brs, 2H), 4.23(q, 2H), 4.00(s, 3H), 2.77-2.71(m, 5H), 1.83-1.77(m, 2H), 1.36(t, 3H), 1.02(t, 3H) |
| 1-248 | δ 8.47-8.43(m, 1H), 8.27-8.24(m, 1H), 7.64(d, 1H), 7.39-7.36(m, 2H), 7.14-7.10(m, 1H), 5.43(brs, 2H), 4.37(q, 2H), 4.00(s, 3H), 2.79(d, 3H), 1.51(t, 3H) |
| 1-249 | δ 8.46-8.43(m, 1H), 8.28-8.25(m, 1H), 7.65-7.63(m, 1H), 7.39-7.36(m, 2H), 7.12(dd, 1H), 5.42(brs, 2H), 4.37(q, 2H), 4.24(q, 2H), 2.79(d, 3H), 1.51(t, 3H), 1.37(t, 3H) |
| 1-269 | δ 8.47-8.44(m, 1H), 8.27-8.24(m, 1H), 8.02(d, 2H), 7.59(q, 1H), 7.50-7.43(m, 3H), 5.44(brs, 2H), 4.43-4.36(m, 2H), 4.00(s, 3H), 2.80(d, 3H), 1.53(t, 3H) |
| 1-270 | δ 8.47-8.44(m, 1H), 8.28-8.25(m, 1H), 8.04-8.00(m, 2H), 7.60(q, 1H), 7.49-7.42(m, 3H), 5.44(brs, 2H), 4.40(q, 2H), 4.24(q, 2H), 2.79(d, 3H), 1.57(t, 3H), 1.37(t, 3H) |
| 1-284 | δ 8.44-8.41(m, 1H), 8.23-8.20(m, 1H), 7.84(q, 1H), 5.42(brs, 2H), 4.00-3.98(m, 6H), 2.79-2.71(m, 5H), 1.79-1.70(m, 2H), 1.48-1.38(m, 2H), 0.96(t, 3H) |
| 1-285 | δ 8.43-8.41(m, 1H), 8.23-8.21(m, 1H), 7.85(q, 1H), 5.43(brs, 2H), 4.23(q, 2H), 3.98(s, 3H), 2.79-2.71(m, 5H), 1.80-1.70(m, 2H), 1.39-1.34(m, 5H), 0.96(t, 3H) |
| 1-287 | δ 8.48-8.45(m, 1H), 8.30-8.27(m, 1H), 7.75(s, 1H), 7.09-6.99(m, 1H), 5.42(brs, 2H), 4.10(s, 3H), 4.00(s, 3H), 2.81-2.79(m, 3H) |
| 1-288 | δ 8.72(s, 1H), 8.48-8.45(m, 1H), 8.28-8.25(m, 1H), 7.35(q, 1H), 5.43(brs, 2H), 4.08(s, 3H), 4.01(s, 3H), 2.82-2.76(m, 6H) |
| 1-289 | δ 8.72(s, 1H), 8.47-8.44(m, 1H), 8.29-8.26(m, 1H), 7.38-7.32(m, 1H), 5.43(brs, 2H), 4.24(q, 2H), 4.08(s, 3H), 2.83-2.76(m, 6H), 1.40-1.34(t, 3H) |
| 1-290 | δ 8.72(s, 1H), 8.46-8.43(m, 1H), 8.30-8.27(m, 1H), 7.38-7.33(m, 1H), 5.48(brs, 2H), 4.49-4.39(m, 1H), 4.09(s, 3H), 2.83-2.76(m, 6H), 1.36-1.33(d, 6H) |
| 1-299 | δ 8.41(d, 1H), 8.23(d, 1H), 7.81(q, 1H), 5.40(brs, 2H), 4.47-4.38(m, 1H), 4.31(q, 2H), 3.19-3.07(m, 1H), 2.73(d, 3H), 1.45-1.44(t, 3H), 1.35(m, 12H) |
| 1-358 | δ 8.43 (d, 1H), 8.21 (d, 1H), 8.04 (q, 1H), 5.43 (brs, 2H), 4.00 - 3.99 (m, 6H), 2.73 (d, 3H), 1.41 (s, 9H). |
| 1-359 | δ 8.42 (d, 1H), 8.21 (d, 1H), 8.04 (q, 1H), 5.43 (brs, 2H), 4.23 (q, 2H), 4.00 (s, 3H), 2.72 (d, 3H), 1.42 - 1.34 (m, 12H). |

**[Table 22-5]**

| Compound No. | ¹H-NMR Data (CDCl₃/TMS, ppm) |
|---|---|
| 2-4 | δ 8.13-8.05(m, 2H), 7.63(s, 1H), 7.58-7.54(m, 1H), 7.30(s, 1H), 7.24(d, 1H), 7.07-7.03(m, 1H), 5.49(s, 2H), 4.28-4.18(m, 4H), 3.85(s, 3H), 2.37(s, 3H), 1.36(t, 3H), 1.15(t, 3H) |
| 2-6 | δ 8.14-8.06(m, 2H), 8.03-7.96(m, 1H), 7.74-7.68(m, 1H), 7.59-7.52(m,1H), 7.40-7.30(m,2H), 5.50(s, 2H), 4.27-4.16(m, 4H), 1.36(t, 3H), 1.15(t, 3H) |
| 2-8 | δ 8.12-8.06(m, 1H), 7.81-7.75(m, 2H), 7.58-7.52(m, 1H), 7.34-7.30(m, 1H), 5.39(s, 2H), 4.23-4.14(m, 4H), 3.70(s, 3H), 1.35(t, 3H), 1.17(t, 3H) |
| 2-15 | δ 8.45(d, 1H), 8.18(d, 1H), 8.02(dt, 1H), 7.81(s, 1H), 7.45(dd, 1H), 7.34-7.27(m, 1H), 7.22-7.17(m, 1H), 5.32(s, 2H), 4.65(q, 2H), 3.85(s, 3H), 2.32(s, 3H), 2.30(s, 3H), 1.38(t, 3H) |
| 2-17 | δ 8.27-8.19(m, 2H), 7.86(d, 1H), 7.65(s, 1H), 7.40(d, 1H), 7.23-7.15(m, 2H), 4.45(q, 2H), 4.27(q, 2H), 4.06(s, 3H), 2.50(s, 3H), 1.45(t, 3H), 1.10(t, 3H) |
| 2-22 | δ 8.07(d, 1H), 8.01(d, 1H), 7.80-7.76(m, 2H), 7.40-7.34(m, 2H), 7.32(s, 1H), 7.25-7.22(m, 1H), 5.59(s, 2H), 4.51(q, 2H), 3.94(s, 3H), 3.80(s, 3H) |
| 2-32* | δ 8.46(d, 1H), 8.17(d, 1H), 8.09-8.04(m, 1H), 7.44-7.39(m, 1H), 7.30-7.25(m, 1H), 7.19-7.13(m, 1H), 4.34(q, 2H), 3.77(s, 3H), 3.70(s, 3H), 2.32(s, 3H), 1.37(t, 3H) |
| 2-37 | δ 8.13-8.02(m, 2H), 7.02(s, 1H), 5.65-5.42(m, 3H), 4.29-4.17(m, 4H), 4.15-4.10(m, 2H), 3.75(s, 3H), 2.20-2.14(m, 2H), 1.93-1.86(m, 2H), 1.35(t, 3H), 1.20(t, 3H) |
| 3-1 | δ 8.49(d, 1H), 8.27(d, 1H), 8.02-7.98(m, 1H), 7.29-7.19(m, 2H), 6.72-6.67(m, 1H), 5.37(brs, 2H), 4.24(q, 2H), 2.81(d, 3H), 2.67(s, 3H), 1.37(t, 3H) |
| 3-3 | δ 8.48(d, 1H), 8.28(d, 1H), 8.04-8.01(m, 2H), 7.49-7.45(m, 3H), 6.34(q, 1H), 5.39(brs, 2H), 4.24(q, 2H), 2.79(d, 3H), 2.61(s, 3H), 1.37(t, 3H) |
| 3-4 | δ 8.48(d, 1H), 8.27(d, 1H), 8.18(dd, 1H), 7.50(dd, 1H) 7.42-7.35(m, 2H), 6.74(q, 1H), 5.41(brs, 2H), 4.24(q, 2H), 2.75(d, 3H), 2.65(s, 3H), 1.36(t, 3H) |
| 3-6 | δ 8.48(d, 1H), 8.28(d, 1H), 8.20-8.17(m, 1H), 7.26-7.23(m, 1H), 7.15-7.10(m, 1H), 6.65(q, 1H), 5.40(brs, 2H), 4.24(q, 2H), 2.74(d, 3H), 2.64(s, 3H), 1.36(t, 3H) |
| 3-8 | δ 8.49(d, 1H), 8.28(d, 1H), 7.46-7.38(m, 1H), 7.10-7.03(m, 2H), 6.32-6.28(m, 1H), 5.41(brs, 2H), 4.00(s, 3H), 2.76(d, 3H), 2.65(s, 3H) |
| 3-9 | δ 8.54(d, 1H), 8.34(d, 1H), 8.10-8.07(m, 2H), 7.46-7.41(m, 3H), 5.45(br.s, 2H), 4.23(q, 2H), 3.46(s, 3H), 2.52(s, 3H), 1.34(t, 3H) |

**[Table 22-6]**

| Compound No. | ¹H-NMR Data (CDCl₃/TMS, ppm) |
|---|---|
| 3-12 | δ 8.49(d, 1H), 8.25(d, 1H), 8.26-8.21(m, 1H), 7.46-7.40(m, 1H), 7.31-7.27(m, 1H) 7.22-7.16(m, 1H), 6.90-6.85(m, 1H), 5.38(brs, 2H), 4.24(q, 2H), 2.80(d, 3H), 2.68(s, 3H), 1.36(t, 3H) |
| 3-13 | δ 8.48(d, 1H), 8.30(d, 1H), 7.43-7.32(m, 2H), 7.17-7.12(m, 1H), 6.04(q, 1H), 5.43(brs, 2H), 4.24(q, 2H), 2.71(d, 3H), 2.63(s, 3H), 1.37(s, 3H) |
| 3-14 | δ 8.48(d, 1H), 8.30(d, 1H), 6.87-6.80(m, 1H), 6.23-6.19(m, 1H), 5.40(brs, 2H), 4.24(q, 2H), 2.75(d, 3H), 2.63(s, 3H), 1.36(s, 3H) |
| 3-17 | δ 8.49(d, 1H), 8.27(d, 1H), 8.19-8.17(m, 1H), 7.51-7.49(m, 1H), 7.43-7.36(m, 2H), 6.74(q, 1H), 5.40(brs, 2H), 4.00(s, 3H), 2.75(d, 3H), 2.65(s, 3H) |
| 3-18 | δ 8.49(d, 1H), 8.22(d, 1H), 8.15(d, 1H), 7.53(q, 1H),7.4-7.39(m, 1H), 7.12-7.04(m, 2H), 5.40(brs, 2H), 4.23(q, 2H), 3.95(s, 3H), 2.79(d, 3H), 2.68(s, 3H), 1.36(t, 3H) |
| 3-114 | δ 8.43(d, 1H), 8.22(d, 1H), 6.70(q, 1H), 5.37(brs, 2H), 4.23(q, 2H), 2.78(tt, 1H) 2.73(d, 3H), 2.54(s, 3H), 2.13-2.09(m, 2H), 1.85(dt, 2H), 1.77-1.72(m, 1H), 1.59-1.49(m, 2H), 1.46-1.36(m, 2H), 1.34-1.26(m, 1H) |
| 3-115 | δ 8.69(d, 1H), 8.50(d, 1H), 8.25(d, 1H), 7.85-7.81(m, 1H), 7.38-7.35(m, 1H), 6.90(q, 1H), 5.44(brs, 2H), 4.00(s, 3H), 2.84(d, 3H), 2.68(s, 3H) |
| 3-118 | δ 8.73(s, 1H), 8.63(d, 1H), 8.48(d, 1H), 8.32(d, 1H), 8.16(d, 1H), 6.47(q, 1H), 5.35(brs, 2H), 4.48-4.39(m, 1H), 2.76(d, 3H), 2.64(s, 3H), 1.34(d, 6H) |
| 3-122 | δ 8.74(brs, 2H), 8.49(d, 1H), 8.33(d, 1H), 7.90(d, 2H), 6.00(q, 1H), 5.40(brs, 2H), 4.25(q, 2H), 2.80(d, 3H), 2.60(s, 3H), 1.37(t, 3H) |
| 3-123 | δ 9.25(s, 1H), 8.69(dd, 1H), 8.48(d, 1H), 8.33(dt, 1H), 8.31(d, 1H), 7.44-7.41(m, 1H), 6.09(q, 1H), 5.39(brs, 2H), 4.24(q, 2H), 2.79(d, 3H), 2.61(s, 3H), 1.37(t, 3H) |
| 3-124 | δ 8.69(dd, 1H), 8.49(d, 1H), 8.28(d, 1H), 7.87(dd, 1H), 7.37-7.33(m, 1H), 6.75(q, 1H), 5.39(brs, 2H), 4.24(q, 2H), 2.77(d, 3H), 2.69(s, 3H), 1.36(t, 3H) |
| 3-125 | δ 8.69(brs, 1H), 8.50(d, 1H), 8.27(d, 1H), 7.87-7.85(m, 1H), 7.37-7.33(m, 1H), 6.76(q, 1H), 5.40(brs, 2H), 4.00(s, 3H), 2.78(d, 3H), 2.69(s, 3H) |
| 3-127 | δ 8.42(d, 1H), 8.23(d, 1H), 6.69(q, 1H), 5.34(brs, 2H), 4.45-4.43(m, 1H), 2.78(tt, 1H), 2.73(d, 3H), 2.54(s, 3H), 2.13-2.09(m, 2H), 1.85(dt, 2H), 1.76-1.71(m, 1H), 1.59-1.49(m, 2H), 1.46-1.36(m, 2H), 1.33(d, 6H), 1.33-1.26(m, 1H) |

**[Table 22-7]**

| Compound No. | ¹H-NMR Data (CDCl₃/TMS, ppm) |
|---|---|
| 3-129 | δ 8.43(d, 1H), 8.22(d, 1H), 6.65(q, 1H), 5.37(brs, 2H), 4.23(q, 2H), 3.22(tt, 1H), 2.73(d, 3H), 2.54(s, 3H), 2.16-2.07(m, 2H), 1.91-1.68(m, 6H), 1.36(t, 3H) |
| 3-130 | δ 8.42(d, 1H), 8.23(d, 1H), 6.65(q, 1H), 5.34(brs, 2H), 4.47-4.37(m, 1H), 3.22(tt, 1H), 2.73(d, 3H), 2.54(s, 3H), 2.16-2.08(m, 2H), 1.91-1.68(m, 6H), 1.33(d, 6H) |
| 3-151 | δ 8.43(d, 1H), 8.25(d, 1H), 6.53(q, 1H), 5.35(brs, 2H), 4.42(quin, 1H), 4.10-4.05(m, 2H), 3.56(dt, 2H), 3.01-3.09(m, 1H), 2.74(d, 3H), 2.55(s, 3H), 2.03-2.00(m, 2H), 1.95-1.85(m, 2H), 1.33(d, 6H) |
| 3-168 | δ 8.86(s, 1H), 8.47(s, 1H), 8.47(d, 1H), 8.30(d, 1H), 6.00(q, 1H), 5.37(brs, 2H), 4.24(q, 2H), 2.79(d, 3H), 2.59(s, 3H), 1.36(t, 3H) |
| 3-171 | δ 8.89(d, 1H), 8.48(d, 1H), 8.25(d, 1H), 8.07(d, 1H), 6.79(q, 1H), 5.41(s, 3H), 5.35(brs, 2H), 4.24(q, 2H), 2.82(d, 3H), 2.67(s, 3H), 1.36(t, 3H) |
| 3-174 | δ 9.19(s, 1H), 8.47(d, 1H), 8.35(d, 1H), 5.58(q, 1H), 5.37(brs, 2H), 4.24(q, 2H), 2.80(d, 3H), 2.59(s, 3H), 1.36(t, 3H) |
| 3-176 | δ 8.47(d, 1H), 8.26(d, 1H), 7.70(d, 1H), 7.39(d, 1H), 7.13(t, 1H)6.27(q, 1H), 5.38(brs, 2H), 4.00(s, 3H), 2.80(d, 3H), 2.60(s, 3H) |
| 3-177 | δ 8.46(d, 1H), 8.27(d, 1H), 7.70(dd, 1H), 7.39(dd, 1H), 7.13(dd, 1H)6.27(q, 1H), 5.37(brs, 2H), 4.24(q, 2H), 2.80(d, 3H), 2.60(s, 3H), 1.36(t, 3H) |
| 3-179 | δ 8.47(d, 1H), 8.27(d, 1H), 7.96(dd, 1H), 7.54(dd, 1H), 7.41(dd, 1H)6.32(q, 1H), 5.38(brs, 2H), 4.00(s, 3H), 2.78(d, 3H), 2.59(s, 3H) |
| 3-180 | δ 8.46(d, 1H), 8.27(d, 1H), 7.96(dd, 1H), 7.54(dd, 1H), 7.41(dd, 1H)6.32(q, 1H), 5.38(brs, 2H), 4.24(q, 2H), 2.78(d, 3H), 2.59(s, 3H), 1.36(t, 3H) |
| 3-190 | δ 8.43(d, 1H), 8.23(d, 1H), 6.60(q, 1H), 5.37(bs, 2H), 4.23(q, 2H), 2.75(s, 3H), 2.74(q, 2H), 2.54(s, 3H), 1.82(m, 2H), 1.36(t, 3H), 1.03(t, 3H) |
| 3-191 | δ 8.53(d, 1H), 8.33(d, 1H), 7.72(d, 1H), 7.37(dd, 1H), 7.12(dd, 1H)5.41(brs, 1H), 4.24(q, 2H), 3.47(s, 3H), 2.53(s, 3H), 1.36(t, 3H) |
| 3-192 | δ 8.47(d, 1H), 8.27(d, 1H), 7.28(d, 1H), 6.93(d, 1H), 6.41(q, 1H), 5.37(brs, 2H), 4.24(q, 2H), 2.76(d, 3H), 2.59(s, 3H), 1.36(t, 3H) |
| 3-193 | δ 8.47(d, 1H), 8.27(d, 1H), 7.39(d, 1H), 7.03(d, 1H), 6.78(q, 1H), 5.36(brs, 2H), 4.24(q, 2H), 2.77(d, 3H), 2.61(s, 3H), 1.36(t, 3H) |
| 3-194 | δ 8.48(d, 1H), 8.20(d, 1H), 8.00(d, 1H), 7.45(q, 1H), 6.39(d, 1H)5.36(brs, 2H), 4.23(q, 2H), 3.94(s, 3H), 2.80(d, 3H), 2.67(s, 3H), 1.36(t, 3H) |
| 3-195 | δ 8.47(d, 1H), 8.27(d, 1H), 6.89(s, 1H), 6.64(q, 1H), 5.36(brs, 2H), 4.00(s, 3H), 2.76(d, 3H), 2.59(s, 3H) |

**[Table 22-8]**

| Compound No. | ¹H-NMR Data (CDCl₃/TMS, ppm) |
|---|---|
| 3-196 | δ 8.46(d, 1H), 8.27(d, 1H), 7.01(s, 1H), 6.64(q, 1H), 5.36(brs, 2H), 4.00(s, 3H), 2.76(d, 3H), 2.59(s, 3H) |
| 3-197 | δ 8.48(d, 1H), 8.25(d, 1H), 7.29(dd, 1H), 6.91(d, 1H), 6.72(q, 1H), 5.36(brs, 2H), 4.24(q, 2H), 2.79(d, 3H), 2.65(s, 3H), 1.36(t, 3H) |
| 3-201 | δ 8.48(d, 1H), 8.26(d, 1H), 7.39(d, 1H), 7.03(d, 1H), 6.78(q, 1H), 5.37(brs, 2H), 4.00(s, 3H), 2.77(d, 3H), 2.61(s, 3H) |
| 3-204 | δ 8.43(d, 1H), 8.22(d, 1H), 6.58(q, 1H), 5.37(bs, 2H), 4.23(q, 2H), 2.76(t, 2H), 2.74(d, 3H), 2.54(s, 3H), 1.77(m, 2H), 1.44(m, 2H), 1.36(t, 3H), 0.97(t, 3H) |
| 3-207 | δ 8.43(d, 1H), 8.24(d, 1H), 6.48(q, 1H), 5.36(brs, 2H), 4.23(q, 2H), 2.74(d, 3H), 2.52(s, 3H), 2.44(s, 3H), 1.36(t, 3H) |
| 3-229 | δ 8.47(d, 1H), 8.26(d, 1H), 7.72(d, 1H), 7.38(d, 1H), 7.13(dd, 1H), 6.25(q, 1H), 5.39(brs, 2H), 4.00(s, 3H), 2.91(q, 2H), 2.79(d, 3H), 1.33(t, 3H) |
| 3-230 | δ 8.46(d, 1H), 8.27(d, 1H), 7.72(dd, 1H), 7.38(dd, 1H), 7.13(dd, 1H), 6.25(q, 1H), 5.38(brs, 2H), 4.24(q, 2H), 2.91(q, 2H), 2.79(d, 3H), 1.36(t, 3H), 1.33(t, 3H) |
| 3-250 | δ 8.48(d, 1H), 8.28(d, 1H), 8.05-8.02(m, 2H), 7.49-7.43(m, 3H), 6.31(q, 1H), 5.40(brs, 2H), 4.00(s, 3H), 2.91(q, 2H), 2.78(d, 3H), 1.35(t, 3H) |
| 3-251 | δ 8.47(d, 1H), 8.28(d, 1H), 8.05-8.03(m, 2H), 7.49-7.43(m, 3H), 6.31(q, 1H), 5.40(brs, 2H), 4.24(q, 3H), 2.91(q, 2H), 2.78(d, 3H), 1.36(t, 3H), 1.35(t, 3H) |
| 3-265 | δ 8.46(d, 1H), 8.30(d, 1H), 7.51(s, 1H), 5.88(q, 1H), 5.38(brs, 2H), 4.24(q, 2H), 2.79(d, 3H), 2.57(s, 3H), 1.36(t, 3H) |
| 3-267 | δ 8.47(d, 1H), 8.28(d, 1H), 7.38(d, 1H), 7.09(d, 1H), 6.74(q, 1H), 5.36(brs, 2H), 4.24(q, 2H), 2.77(d, 3H), 2.60(s, 3H), 1.36(t, 3H) |
| 3-268 | δ 8.86(d, 1H), 8.47(d, 1H), 8.47(d, 1H), 8.30(d, 1H), 6.00(q, 1H), 5.37(brs, 2H), 4.24(q, 2H), 2.79(d, 3H), 2.59(s, 3H), 1.36(t, 3H) |
| 3-269 | δ 8.47(d, 1H), 8.25(d, 1H), 6.81(dd, 1H), 6.56(s, 1H), 6.32(q, 1H), 5.35(brs, 2H), 4.23(q, 2H), 2.78(d, 3H), 2.62(s, 3H), 1.36(t, 3H) |
| 3-270 | δ 8.47(d, 1H), 8.34(d, 1H), 5.74(q, 1H), 5.38(brs, 2H), 4.24(q, 2H), 3.03(s, 3H), 2.78(d, 3H), 2.59(s, 3H), 1.36(t, 3H) |
| 3-271 | δ 8.50(d, 1H), 8.28(d, 1H), 6.62(q, 1H), 5.39(brs, 2H), 4.24(q, 3H), 3.02(s, 3H), 2.82(d, 3H), 2.69(s, 3H), 1.37(t, 3H) |
| 3-272 | δ 8.46(d, 1H), 8.27(d, 1H), 8.06(dd, 1H), 7.51(dd, 1H), 6.78(dd, 1H), 6.28(q, 1H), 5.38(brs, 2H), 4.24(q, 2H), 2.77(d, 3H), 2.57(s, 3H), 1.36(t, 3H) |

**[Table 22-9]**

| Compound No. | ¹H-NMR Data (CDCl₃/TMS, ppm) |
|---|---|
| 3-273 | δ 8.54(d, 1H), 8.47(d, 1H), 8.32(d, 1H), 7.75(d, 1H), 5.86(q, 1H), 5.40(brs, 2H), 4.24(q, 2H), 2.79(d, 3H), 2.60(s, 3H), 1.36(t, 3H) |
| 3-274 | δ 8.47(d, 1H), 8.26(d, 1H), 7.54(dd, 1H), 7.04(dd, 1H), 6.54(dd, 1H), 6.47(q, 1H), 5.37(brs, 2H), 4.24(q, 2H), 2.80(d, 3H), 2.62(s, 3H), 1.36(t, 3H) |
| 3-279 | δ 8.47(d, 1H), 8.31(d, 1H), 7.53(d, 1H), 6.75(d, 1H), 5.99(q, 1H), 5.38(brs, 2H), 4.24(s, 3H), 4.24(q, 2H), 2.75(d, 3H), 2.58(s, 3H), 1.36(t, 3H) |
| 3-286 | δ 11.36(s, 1H), 8.38(d, 1H), 8.22(d, 1H), 7.76(dd, 2H),7.49-7.40(m, 3H), 6.61(q, 1H), 5.49(brs, 2H), 4.22(q, 2H), 2.75(d, 3H), 1.36(t, 3H) |
| 3-318 | δ 8.56(d, 1H), 8.31(d, 1H), 7.80(dd, 1H), 7.44(dd, 1H), 7.15(dd, 1H), 6.02(brs, 2H), 4.23(q, 2H), 3.67(s, 3H), 3.27(s, 3H), 1.36(t, 3H) |
| 3-321 | δ 8.50(d, 1H), 8.36(d, 1H), 7.79(dd, 1H), 7.45(dd, 1H), 7.15(dd, 1H), 5.58(brs, 2H), 4.22(q, 2H), 3.47(s, 3H), 3.45(s, 3H), 1.35(t, 3H) |
| 3-324 | δ 8.55(d, 1H), 8.32(d, 1H), 7.78(d, 1H), 7.38(d, 1H), 7.13(t, 1H), 6.55(dd, 1H), 6.53(dd, 1H), 5.70(dd, 1H), 5.40(brs, 2H), 4.24(q, 2H), 3.52(s, 3H), 1.36(t, 3H) |
| 3-377 | δ 8.53(d, 1H), 8.33(d, 1H), 7.73(m, 1H), 7.35(m, 1H), 7.11(m, 1H), 5.40(brs, 2H), 4.23(q, 2H), 3.47(s, 3H), 2.83(q, 2H), 1.35(t, 3H), 1.32(t, 3H) |
| 3-378 | δ 8.54(d, 1H), 8.36(d, 1H), 7.79(d, 1H), 7.43(d, 1H), 7.15(dd, 1H), 6.92(t, 1H), 5.44(brs, 2H), 4.24(q, 2H), 3.50(s, 3H), 1.36(t, 3H) |
| 3-379 | δ 8.56(d, 1H), 8.34(d, 1H), 7.82(dd, 1H), 7.39(dd, 1H), 7.14(dd, 1H), 5.34(brs, 2H), 4.23(q, 2H), 3.42(s, 3H), 1.35(t, 3H) |
| 4-1 | δ 8.54(d, 1H), 8.26(d, 1H), 7.80-7.77(m, 2H), 7.42-7.38(m, 2H), 7.35-7.33(m, 1H), 6.61(s, 1H), 5.43(br.s, 2H), 4.23(q, 2H), 3.80(s, 3H), 2.36(s, 3H), 1.36(t, 3H) |
| 4-2 | δ 8.54(d, 1H), 8.26(d, 1H), 7.74-7.72(m, 1H), 7.48-7.46(m, 1H), 7.30-7.28(m, 2H), 6.77(s, 1H), 5.44(br.s, 2H), 4.23(q, 2H), 3.46(s, 3H), 2.39(s, 3H), 1.36(t, 3H) |
| 4-5 | δ 8.46(d, 1H), 8.18(d, 1H), 7.67-7.65(m, 1H), 7.49-7.47(m, 1H), 7.36-7.29(m, 2H), 6.91(q, 1H), 6.69(s, 1H), 5.42(brs, 2H), 4.23(q, 2H), 2.70(d, 3H), 2.47(s, 3H), 1.36(t, 3H) |
| 4-6 | δ 8.46(d, 1H), 8.20(d, 1H), 7.72-7.69(m, 1H), 7.45-7.40(m, 2H), 7.38-7.34(m, 1H), 6.79(q, 1H), 6.60(s, 1H), 5.41(brs, 2H), 4.23(q, 2H), 2.77(d, 3H), 2.43(s, 3H), 1.37(t, 3H) |

**[Table 22-10]**

| Compound No. | ¹H-NMR Data (CDCl₃/TMS, ppm) |
|---|---|
| 4-7 | δ 8.46(d, 1H), 8.19(d, 1H), 7.60(d, 1H), 7.50(d, 1H), 7.32(dd, 1H), 6.77(q, 1H), 6.67(s, 1H), 5.41(brs, 2H), 4.23(q, 2H), 2.70(d, 3H), 2.46(s, 3H), 1.37(t, 3H) |
| 4-8 | δ 8.46(d, 1H), 8.18(d, 1H), 7.64(d, 2H), 6.96(d, 2H), 6.88(q, 1H), 6.53(s, 1H), 5.40(brs, 2H), 4.23(q, 2H), 3.86(s, 3H), 2.77(d, 3H), 2.42(s, 3H), 1.36(t, 3H) |
| 4-10 | δ 8.47(d, 1H), 8.22(d, 1H), 7.81(d, 1H), 7.77(d, 1H), 7.68-7.64(m, 1H), 7.48-7.37(m, 1H), 6.78(s, 1H), 6.65(q, 1H), 5.41(brs, 2H), 4.00(s, 3H), 2.77(d, 2H), 2.43(s, 2H) |
| 4-11 | δ 8.45(d, 1H), 8.21(d, 1H), 7.66(d, 2H), 7.40(d, 2H), 6.60(s, 1H), 6.57(q, 1H), 5.40(brs, 2H), 4.23(q, 2H), 2.77(q, 2H), 2.76(d, 3H), 1.36(t, 3H), 1.26(t, 3H) |
| 4-14 | δ 8.46(d, 1H), 8.21(d, 1H), 7.65-7.62(m, 2H), 7.41-7.38(m, 2H), 6.61(q, 1H), 6.58(s, 1H), 5.40(brs, 2H), 4.23(q, 2H), 2.76(d, 3H), 2.41(s, 3H), 1.36(t, 3H) |
| 4-15 | δ 8.45(d, 1H), 8.17(d, 1H), 7.51(d, 1H), 7.32-7.25(m, 3H), 6.94(q, 1H), 6.42(s, 1H), 5.43(brs, 2H), 4.24(q, 2H), 2.68(d, 3H), 2.47(s, 3H), 2.46(s, 3H), 1.36(t, 3H) |
| 4-16 | δ 8.46(d, 1H), 8.22(d, 1H), 7.70-7.69(m, 1H), 7.60-7.58(m, 1H), 7.38-7.31(m, 2H), 6.60(s, 1H), 6.56(q, 1H), 5.41(brs, 2H), 4.23(q, 2H), 2.78(d, 3H), 2.41(s, 3H), 1.36(t, 3H) |
| 4-18 | δ 8.38(d, 1H), 7.96(d, 1H), 7.80-7.78(m, 2H), 7.42-7.39(m, 2H), 7.34-7.30(m, 1H), 6.41(s, 1H), 5.15(br.s, 2H), 4.18(q, 4H), 3.34(s, 3H), 1.47(t, 3H), 1.32(t, 3H) |
| 4-19 | δ 8.49(d, 1H), 8.23(d, 1H), 7.79-7.76(m, 2H), 7.68-7.65(m, 1H), 7.34-7.30(m, 2H), 6.05(s, 1H), 5.46(br.s, 2H), 4.18(q, 2H), 3.94(s, 3H), 3.36(s, 3H), 1.32(t, 3H) |
| 4-20 | δ 12.0(br.s, 1H), 8.26(d, 1H), 7.81(d, 1H), 7.55-7.52(m, 2H), 7.32-7.27(m, 3H), 6.34(s, 1H), 5.16(br.s, 2H), 4.14(q, 2H), 3.21(s, 3H), 1.27(t, 3H) |
| 4-21 | δ 8.30(d, 1H), 7.87(d, 1H), 7.55-7.50(m, 2H), 7.39-7.34(m, 2H), 6.54(s, 1H), 5.63(q, 1H), 5.19(brs, 2H), 4.17(q, 2H), 2.73(d, 3H), 1.31(t, 3H) |
| 4-22 | δ 8.30(d, 1H), 7.86(d, 1H), 7.54(d, 2H), 7.47-7.36(m, 3H), 6.35(s, 1H), 6.12(brd, 1H), 5.16(brs, 2H), 4.16(q, 2H), 2.70(d, 3H), 1.30(t, 3H) |
| 4-23 | δ 8.48(d, 1H), 8.11(d, 1H), 7.64(d, 2H), 7.42(d, 2H), 7.12(q, 1H), 6.58(s, 1H), 5.54(brs, 2H), 4.23(q, 2H), 2.77(d, 3H), 2.53(s, 3H), 1.36(t, 3H) |

**[Table 22-11]**

| Compound No. | ¹H-NMR Data (CDCl₃/TMS, ppm) |
|---|---|
| 4-24 | δ 8.53(d, 1H), 8.18(d, 1H), 7.68(d, 2H), 7.48(d, 2H), 7.34(s, 1H), 6.91(q, 1H), 5.89(brs, 2H), 4.23(q, 2H), 2.99(s, 3H), 2.80(d, 3H), 1.36(t, 3H) |
| 4-25 | δ 8.45(d, 1H), 8.29(d, 1H), 7.68(d, 2H), 7.45(d, 2H), 7.39(s, 1H), 5.60(q, 1H), 5.51(brs, 2H), 4.23(q, 2H), 3.19(s, 3H), 2.76(d, 3H), 1.36(t, 3H) |
| 4-26 | δ 8.30(d, 1H), 7.87(d, 1H), 7.62-7.58(m, 1H), 7.41-7.35(m, 1H), 7.27-7.19(m, 2H), 6.54(s, 1H), 5.50(q, 1H), 5.16(brs, 2H), 4.17(q, 2H), 2.74(d, 3H), 1.31(t, 3H) |
| 4-52 | δ 8.43(d, 1H), 8.24(d, 1H), 7.60-7.55(m, 1H), 7.38-7.21(m, 3H), 6.61(m, 1H), 6.48(q, 1H), 5.36(brs, 2H), 4.23(q, 2H), 2.74(d, 3H), 2.52(m, 3H), 2.44(m, 3H), 1.36(t, 3H) |
| 4-77 | δ 8.66-8.64(m, 1H), 8.49(d, 1H), 8.17(d, 1H), 7.78-7.73(m, 1H), 7.70-7.68(m, 1H), 7.31(q, 1H), 7.28-7.24(m, 1H), 6.80(s, 1H), 5.41(brs, 2H), 4.23(q, 2H), 2.84(d, 3H), 2.51(s, 3H), 1.36(t, 3H) |
| 4-115 | δ 8.37(d, 1H), 8.06(d, 1H), 7.58(q, 1H), 5.78(s, 1H), 5.34(brs, 2H), 4.40(quin, 2H), 3.18(t, 4H), 2.69(d, 3H), 2.33(s, 3H), 1.70-1.56(m, 6H), 1.32(d, 6H) |
| 4-135 | δ 8.45(d, 1H), 8.19(d, 1H), 7.55(t, 1H), 7.39-7.38(m, 2H), 6.83(q, 1H), 6.51(s, 1H), 5.40(brs, 2H), 4.26-4.20(m, 2H), 3.21(s, 3H), 2.77(d, 3H), 2.41(s, 3H), 1.38-1.34(m, 3H) |
| 4-149 | δ 8.48-8.45(m, 1H), 8.28-8.25(m, 1H), 6.76(d, 1H), 6.31-6.24(m, 1H), 5.38(brs, 2H), 4.27-4.20(m, 2H), 2.80-2.77(m, 3H), 2.55(s, 3H), 1.39-1.34(m, 3H) |
| 4-150 | δ 8.52(d, 1H), 8.29(d, 1H), 6.78(s, 1H), 5.39(brs, 2H), 4.39(q, 2H), 4.24(q, 2H), 3.51(s, 3H), 2.36(s, 3H), 2.59(s, 3H), 1.38(t, 3H), 1.36(t, 3H) |
| 4-151 | δ 8.42(d, 1H), 8.21(d, 1H), 6.44(s, 1H), 6.17(q, 1H), 5.33(brs, 2H), 4.42(quin, 2H), 2.75(d, 3H), 2.35(s, 3H), 1.33(d, 6H) |
| 4-152 | δ 8.43(d, 1H), 8.19(d, 1H), 6.44(s, 1H), 6.17(q, 1H), 5.33(brs, 2H), 3.99(s, 3H), 2.75(d, 3H), 2.35(s, 3H) |
| 4-153 | δ 8.46(d, 1H), 8.22(d, 1H), 6.76(s, 1H), 6.68(q, 1H), 5.34(brs, 2H), 4.42(quin, 1H), 4.38(q, 2H), 2.79(d, 3H), 2.46(s, 3H), 1.39(t, 3H), 1.34(d, 6H) |
| 4-155 | δ 8.45(d, 1H), 8.17-8.14(m, 1H), 7.58(q, 2H), 7.09-6.99(m, 1H), 6.77-6.74(m, 2H), 6.51(s, 1H), 5.45(brs, 2H), 4.26-4.18(m, 2H), 3.01-3.00(m, 3H), 2.78-2.76(m, 3H), 2.69-2.67(m, 3H), 2.42(m, 3H), 1.37(d, 3H) |
| 4-156 | δ 8.47-8.44(m, 1H), 8.21-8.18(m, 1H), 7.23-7.17(m, 2H), 6.88-6.85(m, 1H), 6.76(q, 1H), 6.51(s, 1H), 6.01(s, 2H), 5.40(brs, 2H), 4.26-4.19(m, 2H), 2.77(d, 3H), 2.41(s, 3H), 1.36(t, 8H) |

**[Table 22-12]**

| Compound No. | ¹H-NMR Data (CDCl₃/TMS, ppm) |
|---|---|
| 4-157 | δ 8.48-8.41(m, 2H), 8.21(dd, 1H), 7.66(t, 1H), 7.24-7.19(m, 1H), 6.70-6.67(m, 1H), 6.18(q, 1H), 5.40-5.35(brs, 2H), 4.27-4.20(m, 2H), 2.77-2.74(m, 3H), 2.43(s, 3H), 1.39-1.33(t, 3H) |
| 4-158 | δ 8.49-8.46(m, 1H), 8.24-8.13(m, 3H), 7.32-7.26(m, 1H), 6.75(m, 1H), 6.66-6.61(m, 1H), 5.39(brs, 2H), 4.24(q, 2H), 2.78(d, 3H), 2.44(s, 3H), 1.37(t, 3H) |
| 4-159 | δ 8.48-8.45(m, 1H), 8.26-8.24(m, 1H), 8.00-7.92(m, 2H), 7.67-7.63(m, 1H), 7.57-7.52(m, 1H), 6.62(s, 1H), 6.35(q, 1H), 5.42-5.39(m, 2H), 4.27-4.20(m, 2H), 2.78(d, 3H), 2.41(s, 3H), 1.39-1.34(m, 3H) |
| 4-160 | δ 8.77(d, 1H), 8.48-8.45(m, 1H), 8.26-8.23(m, 1H), 7.95(dd, 1H), 7.42-7.39(m, 1H), 6.62(d, 1H), 6.34(q, 1H), 5.40(brs, 2H), 4.24(q, 2H), 2.76(d, 3H), 2.41-2.41(m, 3H), 1.36(t, 3H) |
| 4-161 | δ 9.33(d, 1H), 8.50-8.42(m, 2H), 8.27-8.24(m, 1H), 8.16-8.08(m, 1H), 7.90-7.87(m, 1H), 7.78-7.71(m, 1H), 7.63-7.56(m, 1H), 6.79-6.78(m, 1H), 6.52(q, 1H), 5.42(brs, 2H), 4.25(q, 2H), 2.81-2.79(m, 3H), 2.46(s, 3H), 1.37(t, 3H) |
| 4-162 | δ 8.49-8.46(m, 1H), 8.22-8.19(m, 1H), 7.83-7.77(m, 2H), 7.58(s, 1H), 7.39-7.32(m, 2H), 6.67-6.66(m, 1H), 6.63-6.60(m, 1H), 5.42-5.38(m, 2H), 4.27-4.19(m, 2H), 2.85(d, 3H), 2.45(s, 3H), 1.39-1.34(m, 3H) |
| 4-163 | δ 8.48-8.45(m, 1H), 8.24-8.22(m, 1H), 8.10(d, 2H), 7.80-7.77(m, 2H), 6.67(s, 1H), 6.55(q, 1H), 5.45(brs, 2H), 4.25-4.18(m, 2H), 3.95-3.94(m, 3H), 2.78(d, 3H), 1.37-1.32(m, 3H) |
| 4-164 | δ 8.47(d, 1H), 8.23-8.20(m, 1H), 7.43-7.40(m, 1H), 7.18-7.13(m, 1H), 7.06(dd, 1H), 6.91(q, 1H), 6.72(s, 1H), 5.42-5.36(m, 2H), 4.27-4.19(m, 2H), 2.81(d, 3H), 2.45(s, 3H), 1.39-1.34(m, 3H) |
| 4-165 | δ 8.47(d, 1H), 8.18(d, 1H), 6.91-6.86(m, 1H), 5.37(brs, 2H), 4.38(q, 2H), 4.26-4.20(m, 2H), 2.79(d, 3H), 2.37(s, 3H), 2.30(s, 3H), 1.42-1.34(m, 6H) |
| 4-166 | δ 8.29(d, 1H), 7.87(d, 1H), 6.67(s, 1H), 5.12(brs, 2H), 4.40-4.33(m, 2H), 4.19-4.14(m, 5H), 2.74-2.72(m, 3H), 1.40-1.30(m, 6H) |
| 4-167 | δ 8.48-8.40(m, 1H), 8.22-8.18(m, 1H), 7.77-7.70(m, 4H), 6.72(q, 1H), 5.52(brs, 2H), 4.25-4.19(m, 4H), 3.23(s, 3H), 2.71-2.69(m, 3H), 1.39-1.32(m, 6H) |
| 4-168 | δ 8.43(d, 1H), 8.12(d, 1H), 7.67(q, 1H), 6.40(s, 1H), 5.40(brs, 2H), 4.22(q, 2H), 3.85(t, 3H), 2.77(d, 3H), 2.60(t, 2H), 2.43(s, 3H), 2.21(quin, 2H), 1.35(t, 3H) |
| 4-169 | δ 8.45(d, 1H), 8.18(d, 1H), 7.59(q, 1H), 5.37(brs, 2H), 4.22(q, 2H), 4.09(t, 2H), 2.75(d, 3H), 2.57(t, 2H), 2.22(quin, 2H), 1.36(t, 3H) |

**[Table 22-13]**

| Compound No. | ¹H-NMR Data (CDCl₃/TMS, ppm) |
|---|---|
| 4-170 | δ 8.46-8.43(m, 1H), 8.17-8.14(m, 1H), 7.54-7.51(m, 2H), 7.45-7.41(m, 2H), 7.00-6.96(m, 1H), 5.41(brs, 2H), 4.23(q, 2H), 2.70(d, 3H), 2.60(q, 2H), 2.38(s, 3H), 1.39-1.34(m, 3H), 1.19-1.14(m, 3H) |
| 4-171 | δ 8.43-8.40(m, 1H), 8.18-8.15(m, 1H), 6.65(q, 1H), 5.36(brs, 2H), 4.25-4.19(m, 2H), 2.73(d, 3H), 2.38(s, 3H), 2.31(s, 3H), 1.36(t, 3H) |
| 4-172 | δ 8.48-8.45(m, 1H), 8.24-8.21(m, 1H), 7.80-7.75(m, 2H), 7.47-7.43(m, 2H), 6.42(q, 1H), 5.38(brs, 2H), 4.24(q, 2H), 2.73(d, 3H), 2.43(s, 3H), 1.36(t, 3H) |
| 4-173 | δ 8.47-8.44(m, 1H), 8.13-8.10(m, 1H), 7.62-7.59(m, 2H), 7.44-7.41(m, 2H), 7.17(d, 1H), 5.38(brs, 2H), 4.23(q, 2H), 2.85(m, 2H), 2.74(m, 5H), 1.90-1.84(m, 4H), 1.36(t, 3H) |
| 4-174 | δ 8.44-8.42(m, 1H), 8.17-8.15(m, 1H), 7.43(m, 4H), 6.85(s, 1H), 5.42(brs, 2H), 4.23(q, 2H), 3.07-2.99(m, 1H), 2.65(d, 3H), 2.42(s, 3H), 1.37(t, 3H), 1.27(d, 6H) |
| 4-175 | δ 8.47-8.44(m, 1H), 8.20-8.17(m, 1H), 7.64-7.61(m, 2H), 7.29-7.25(m, 2H), 6.88-6.85(m, 1H), 6.57(s, 1H), 5.43-5.38(m, 2H), 4.23(q, 2H), 2.77(d, 3H), 2.57-2.50(m, 1H), 2.43-2.42(m, 3H), 1.94-1.82(m, 4H), 1.78-1.73(m, 1H), 1.39-1.34(m, 8H) |
| 4-176 | δ 8.45-8.43(m, 1H), 8.16-8.14(m, 1H), 7.32-7.19(m, 4H), 6.84-6.83(m, 1H), 5.98(q, 1H), 4.84(brs, 2H), 4.16(q, 2H), 2.82-2.80(m, 3H), 1.33-1.29(m, 3H) |
| 4-177 | δ 8.48-8.45(m, 1H), 8.27-8.25(m, 1H), 7.99-7.96(m, 2H), 7.78-7.75(m, 2H), 6.19(q, 1H), 5.38(brs, 2H), 4.27-4.21(m, 2H), 2.73(d, 3H), 2.44(s, 3H), 1.37(t, 3H) |
| 4-178 | δ 8.47-8.45(m, 1H), 8.24-8.21(m, 1H), 7.78-7.75(m, 2H), 7.48-7.43(m, 2H), 6.44(q, 1H), 5.48(brs, 2H), 4.27-4.21(m, 2H), 2.72(d, 3H), 2.44(s, 3H), 1.39-1.34(m, 3H) |
| 4-179 | δ 8.47-8.44(m, 1H), 8.21-8.18(m, 1H), 7.64-7.61(m, 2H), 7.32-7.28(m, 2H), 6.75(m, 1H), 6.58(s, 1H), 5.45(brs, 2H), 4.25-4.18(m, 2H), 2.78-2.75(m, 3H), 2.52(s, 3H), 2.4(s, 3H), 1.38-1.32(m, 3H) |
| 4-180 | δ 8.45-8.42(m, 1H), 8.15-8.12(m, 1H), 7.17(q, 1H), 6.26(s, 1H), 5.40(brs, 2H), 4.26-4.19(m, 2H), 4.09-3.96(m, 4H), 2.75(d, 3H), 2.42(d, 3H), 1.38-1.33(m, 3H) |
| 4-181 | δ 8.45(d, 1H), 8.13(d, 1H), 6.77(d, 1H), 6.28-6.25(m, 1H), 5.36(s, 2H), 4.27-4.20(m, 2H), 4.13-4.05(m, 2H), 3.96-3.90(m, 2H), 2.79-2.77(m, 2H), 2.56-2.54(m, 3H), 2.51-2.49(m, 3H), 2.41-2.38(m, 3H), 1.39-1.34(m, 3H) |

**[Table 22-14]**

| Compound No. | ¹H-NMR Data (CDCl₃/TMS, ppm) |
|---|---|
| 4-182 | δ 8.46-8.44(m, 1H), 8.19-8.16(m, 1H), 7.15(d, 1H), 6.85(d, 1H), 6.70(q, 1H), 6.49(s, 1H), 5.40(brs, 2H), 4.23(q, 2H), 2.81-2.79(m, 3H), 2.42(s, 3H), 2.29(s, 3H), 1.38-1.34(m, 3H) |
| 4-183 | δ 8.47-8.44(m, 1H), 8.25-8.22(m, 1H), 7.52(d, 1H), 6.51-6.48(m, 2H), 6.37(q, 1H), 5.41(brs, 2H), 4.24(q, 2H), 4.06(s, 3H), 2.72(d, 3H), 2.42(s, 3H), 1.37(t, 3H) |
| 4-184 | δ 8.48-8.45(m, 1H), 8.25-8.22(m, 1H), 7.85-7.81(m, 2H), 7.71-7.67(m, 2H), 6.66(s, 1H), 6.51(q, 1H), 5.41(brs, 2H), 4.24(q, 2H), 2.77(d, 3H), 2.42(s, 3H), 1.36(t, 3H) |
| 4-185 | δ 8.48-8.45(m, 1H), 8.17-8.14(m, 1H), 7.61(dd, 1H), 7.39-7.28(m, 2H), 7.06-6.99(m, 2H), 6.63(s, 1H), 5.40(brs, 2H), 4.23(q, 2H), 3.92(s, 3H), 2.74(d, 3H), 2.45(s, 3H), 1.36(t, 3H) |
| 4-186 | δ 8.48-8.45(m, 1H), 8.21-8.18(m, 1H), 6.88-6.86(m, 2H), 6.84-6.75(m, 1H), 6.58-6.57(m, 1H), 6.48(t, 1H), 5.42(brs, 2H), 4.23(q, 2H), 3.84-3.82(m, 6H), 2.76(d, 3H), 2.43(s, 3H), 1.36(t, 3H) |
| 4-187 | δ 8.48-8.45(m, 1H), 8.21-8.18(m, 1H), 7.40-7.28(m, 3H), 6.94-6.90(m, 1H), 6.80(q, 1H), 6.60(s, 1H), 5.40(brs, 2H), 4.23(q, 2H), 3.85(s, 3H), 2.77(d, 3H), 2.43(s, 3H), 1.39-1.34(m, 3H) |
| 4-188 | δ 8.48-8.45(m, 1H), 8.22-8.19(m, 1H), 7.74-7.66(m, 1H), 6.98-6.90(m, 2H), 6.75(t, 1H), 6.65(s, 1H), 5.40(brs, 2H), 4.23(q, 2H), 2.76(d, 3H), 2.43(s, 3H), 1.36(t, 3H) |
| 4-237 | δ 8.42(d, 1H), 8.16(d, 1H), 6.72(q, 1H), 6.66(dd, 1H), 6.41(s, 1H), 5.78(dd, 1H), 5.41(dd, 1H), 5.38(bs, 2H), 4.23(q, 2H), 2.73(s, 3H), 2.37(s, 3H), 1.36(t, 3H) |
| 4-238 | δ 8.41(d, 1H), 8.13(d, 1H), 7.10(q, 1H), 6.11(s, 1H), 5.39(bs, 2H), 4.22(q, 2H), 2.72(d, 3H), 2.69(q, 2H), 2.36(s, 3H), 1.36(t, 3H), 1.28(t, 3H) |
| 4-239 | δ 8.47(d, 1H), 8.22(d, 1H), 7.50(m, 1H), 7.41(m, 1H), 7.21(m, 1H), 6.66(d, 1H), 6.42(s, 1H), 6.28(dt, 1H), 5.41(brs, 2H), 4.23(q, 2H), 2.70(d, 3H), 2.39(s, 3H), 1.36(t, 3H) |
| 4-243 | δ 8.45(d, 1H), 8.18(d, 1H), 7.87(d, 1H), 7.71(d, 1H), 6.78(q, 1H), 6.48(s, 1H), 6.44(t, 1H), 5.41(brs, 2H), 4.21(q, 3H), 2.78(d, 3H), 2.44(s, 3H), 1.35(t, 3H) |
| 4-247 | δ 8.41(d, 1H), 8.19(d, 1H), 7.57-7.55(m, 4H), 7.48-7.38(m, 6H), 6.41(s, 1H), 6.20(q, 1H), 5.38(brs, 2H), 4.21(q, 3H), 2.72(d, 3H), 2.34(s, 3H), 1.36(t, 3H) |

**[Table 22-15]**

| Compound No. | ¹H-NMR Data (CDCl₃/TMS, ppm) |
|---|---|
| 4-249 | δ 8.41(d, 1H), 8.14(d, 1H), 7.06(q, 1H), 6.14(s, 1H), 5.38(bs, 2H), 4.22(q, 2H), 2.73(q, 2H), 2.69(q, 2H), 1.36(t, 3H), 1.29(t, 3H), 1.19(t, 3H) |
| 4-251 | δ 8.46(d, 1H), 8.22(d, 1H), 7.74(d, 2H), 7.28(d, 2H), 6.60(q, 1H), 6.59(s, 1H), 5.40(bs, 2H), 4.23(q, 2H), 2.77(d, 3H), 2.42(s, 3H), 1.36(t, 3H) |
| 4-303 | δ 8.41(d,1H), 8.13(d,1H), 7.06(q, 1H), 6.14(s, 1H), 5.38(bs, 2H), 3.98(s, 3H), 2.73(q,2H), 2.71(s,3H), 2.69(q, 2H), 1.29(t,3H), 1.19(t, 3H) |
| 4-304 | δ 8.40 (d, 1H), 8.10 (d, 1H), 7.08 (q, 1H), 5.76 (s, 1H), 5.46 (brs, 2H), 4.25-4.12 (m, 4H), 2.71 (d, 3H), 2.34 (s, 3H), 1.45-1.33 (m, 6H). |
| 4-305 | δ 8.41 (d, 1H), 8.12 (d, 1H), 6.93 (q, 1H), 5.80 (s, 1H), 5.37 (brs, 2H), 4.32-4.27 (m, 2H), 4.25-4.18 (m, 2H), 3.75 - 3.70 (m, 2H), 3.43 (s, 3H), 2.70 (d, 3H), 2.33 (s, 3H), 1.35 (t, 3H). |
| 4-306 | δ 8.40 (d, 1H), 8.12 (d, 1H), 6.96 (q, 1H), 5.78 (s, 1H), 5.37 (brs, 2H), 4.26-4.19 (m, 2H), 4.09-3.99 (m, 2H), 3.94-3.85 (m, 2H), 3.80 - 3.66 (m, 2H), 2.79 - 2.69 (m, 4H), 2.34 (s, 3H), 2.16-2.04 (m, 1H), 1.76-1.65 (m, 1H), 1.36 (t, 3H). |
| 4-307 | δ 8.41 (d,1H), 8.11 (d, 1H), 6.95 (q, 1H), 5.79 (s, 1H), 5.37 (brs, 2H), 4.27-4.16 (m, 4H), 4.13-4.07 (m, 1H), 3.96-3.88 (m, 1H), 3.84-3.76 (m, 1H), 2.70(d, 3H), 2.33 (d, 3H), 2.12-1.88 (m, 3H), 1.81-1.70 (m, 1H), 1.35 (t, 3H). |
| 4-308 | δ 8.40 (d, 1H), 8.11 (d, 1H), 7.04 (q, 1H), 5.79 (s, 1H), 5.37 (brs, 2H), 4.22 (q, 2H), 3.93 (d, 2H), 2.71 (d, 3H), 2.34 (s, 3H), 1.36 (t, 3H), 0.68 - 0.62 (m, 2H), 0.39 - 0.32 (m, 2H). |
| 4-309 | δ 8.42 (d, 1H), 8.16 (d, 1H), 6.52 (q, 1H), 6.24 - 5.94 (m, 1H), 5.82 (s, 1H), 5.37 (brs, 2H), 4.37 - 4.28 (m, 2H), 4.23 (q, 2H), 2.72 (d, 3H), 2.32 (s, 3H), 1.35 (t, 3H). |
| 4-310 | 8.43-8.38 (m, 1H), 8.30-8.24 (m, 2H), 8.20-8.17(d, 1H), 7.34-7.29(m, 2H), 6.16(q, 1H), 5.99(s, 1H), 5.39(brs, 2H), 4.24(q, 2H), 2.59(d, 3H), 2.42(s, 3H), 1.37(t, 3H) |
| 5-1 | δ 8.17-8.12 (m, 1H), 8.00-7.92 (m, 2H), 7.76-7.70 (m, 1H), 7.47-7.40 (m, 2H), 7.26-7.21 (m, 1H), 5.42 (s, 2H), 4.22 (q, 2H), 3.66 (s, 3H), 3.14 (s, 3H), 2.95 (s, 3H), 1.36 (t, 3H) |
| 5-19 | δ 8.43(d, 1H), 8.29(d, 1H), 7.96-7.90(m, 2H), 7.78-7.72(m, 1H), 7.56-7.50(m, 1H), 5.84(q, 1H), 5.45(s, 2H), 4.24(q, 2H), 3.42(s, 3H), 2.71(d, 3H), 1.37(t, 3H) |
| 6-1 | δ 8.44 (d, 1H), 8.24 (d, 1H), 8.02-7.95 (m, 2H), 7.49-7.40 (m, 2H), 7.26-7.21 (m, 1H), 4.36 (q, 2H), 3.40 (s, 3H), 2.29 (s, 3H), 2.21 (s, 3H), 1.38 (t, 3H) |
| 7-1 | δ 8.06 (d, 1H), 8.01(d, 1H), 7.91-7.81 (m, 2H), 7.44-7.38 (m, 3H), 5.53 (s, 2H), 4.21 (q, 2H), 4.18 (q, 2H), 2.73 (s, 3H), 1.35 (t, 3H), 1.08 (t, 3H) |

**[Table 22-16]**

| Compound No. | ¹H-NMR Data (CDCl₃/TMS, ppm) |
|---|---|
| 10-17 | δ 8.43(d, 1H), 8.11(d, 1H), 7.41-7.27(m, 6H), 5.52(bs, 2H), 5.30(s, 2H), 4.22(q, 2H), 3.28(s, 3H), 2.12(s, 3H), 1.35(t, 3H) |
| 10-18 | δ 8.50(d, 1H), 8.17(d, 1H), 7.91(s, 1H), 7.80(t, 1H), 7.32-7.20(m, 3H), 5.54(bs, 2H), 4.23(q, 2H), 3.55(s, 3H), 2.23(s, 3H), 1.37(t, 3H) |
| 10-19 | δ 8.46(d, 1H), 8.30(s, 1H), 8.19(d, 1H), 8.01(d, 2H), 7.59(t, 1H), 7.47(t, 2H), 5.50(bs, 2H), 4.23(q, 2H), 3.15(s, 3H), 2.22(s, 3H), 1.36(t, 3H) |
| 10-20 | δ 8.37(d, 1H), 8.15(d, 1H), 7.83(s, 1H), 7.70(d, 2H), 7.44(t, 2H), 7.26(t, 1H), 5.52(bs, 2H), 4.22(q, 2H), 2.63(s, 6H), 2.10(s, 3H), 1.35(t, 3H) |
| 11-2 | δ 8.47(d, 1H), 8.15(d, 1H), 8.08-8.06(m, 3H), 7.95-7.92(m, 2H), 6.72(q, 1H), 5.50(brs, 2H), 4.24(q, 2H), 2.77(d, 3H), 2.63(s, 3H), 1.42(t, 3H) |
| 11-122 | δ 8.78-8.77(m, 1H), 8.49(d, 1H), 8.25(d, 1H), 8.20-8.18(m, 1H), 7.57-7.54(m, 1H), 5.51(brs, 2H), 4.23(q, 2H), 3.40(s, 3H), 2.50(s, 3H), 1.36(t, 3H) |
| 17-4 | δ 7.98(d, 1H),7.96-7.92(m, 2H), 7.75(dd, 1H), 7.71(d, 1H), 7.47-7.41(m, 3H), 4.83(brs, 2H), 4.19(q, 2H), 3.75(s, 3H), 2.27(s, 3H), 1.33(t, 2H) |
| 17-5 | δ 7.99-7.95(m, 2H),7.88(d, 1H), 7.79(dd, 1H), 7.76(s, 1H), 7.73(dd, 1H),7.49-7.43(m, 3H), 4.83(brs, 2H), 4.19(q, 2H), 3.79(s, 3H), 1.33(t, 2H) |
| 17-6 | δ 7.98-7.92(m, 3H), 7.83-7.79(m, 2H), 7.59(s, 1H), 7.46-7.40(m, 2H), 7.35(dd, 1H), 4.85(brs, 2H), 4.21(q, 2H),4.20(q, 2H), 1.35(t, 3H), 1.09(t, 3H) |
| 19-28 | δ 9.44(s, 1H), 7.08(s, 1H), 5.54(brs, 2H), 4.33(q, 2H), 3.93(s, 3H), 3.78(s, 3H), 1.39-1.34(m, 3H) |
| 19-29 | δ 9.48(s, 1H), 7.76-7.71(m, 3H), 7.45-7.37(m, 3H), 5.56(brs, 2H), 4.34(q, 2H), 4.01(s, 3H), 3.93(s, 3H), 1.38(t, 3H) |
| 19-30 | δ 9.47(s, 1H), 8.03-8.00(m, 1H), 7.72(s, 1H), 7.30-7.27(m, 1H), 7.06-6.98(m, 2H), 5.54(brs, 2H), 4.35(q, 2H), 4.02-3.93(m, 9H), 1.38(t, 3H) |
| 20-20 | δ 8.49(d, 1H), 8.20(d, 1H), 7.64(ddd, 1H), 7.50-7.45(m, 1H), 7.32-7.20(m, 2H), 6.66(q, 1H), 5.54(brs, 2H), 4.23(q, 2H), 2.78(d, 3H), 2.52(d, 3H), 1.36(t, 3H) |

Examples of useful plants for which the nitrogen-containing heterocyclic compound of the present invention or a salt thereof can be used include, but are not particularly limited to, cereals (e.g., rice, barley, wheat, rye, oats, corn, etc.), legumes (e.g., soybeans, azuki beans, broad beans, green peas, kidney beans, peanuts, etc.), fruit trees and fruits (e.g., apples, citrus fruits, pears, grapes, peaches, plums, cherries, walnuts, chestnuts, almonds, bananas, etc.), leaf and fruit vegetables (e.g., cabbages, tomatoes, spinach, broccoli, lettuce, onions, green onions (chives and Welsh onions), green peppers, eggplants, strawberries, pepper crops, okra, Chinese chives, etc.), root vegetables (e.g., carrots, potatoes, sweet potatoes, taros, Japanese radishes, turnips, lotus roots, burdock roots, garlic, Chinese scallions, etc.), crops for processing (e.g., cotton, hemp, beet, hops, sugarcane, sugar beet, olives, rubber, coffee, tobacco, tea, etc.), gourds (e.g., Japanese pumpkins, cucumbers, watermelons, oriental sweet melons, melons, etc.), pasture grass (e.g., orchardgrass, sorghum, timothy, clover, alfalfa, etc.), lawn grass (e.g., Korean lawn grass, bent grass, etc.), spice and aromatic crops and ornamental crops (e.g., lavender, rosemary, thyme, parsley, pepper, ginger, etc.), ornamental flowering plants (e.g., chrysanthemum, rose, carnation, orchid, tulip, lily, etc.), garden trees (e.g., ginkgo trees, cherry trees, Japanese aucuba, etc.) and forest trees (e.g., *Abies sachalinensis, Picea jezoensis,* pine, yellow cedar, Japanese cedar, hinoki cypress, eucalyptus, etc.).

The above-mentioned "plants" also include plants provided with herbicide tolerance by a classical breeding technique or a gene recombination technique. Examples of such herbicide tolerance include tolerance to HPPD inhibitors, such as isoxaflutole; ALS inhibitors, such as imazethapyr and thifensulfuron-methyl; EPSP synthase inhibitors, such as glyphosate; glutamine synthetase inhibitors, such as glufosinate; acetyl-CoA carboxylase inhibitors, such as sethoxydim; or other herbicides, such as bromoxynil, dicamba and 2,4-D.

Examples of the plants provided with herbicide tolerance by a classical breeding technique include varieties of rapeseed, wheat, sunflower and rice tolerant to the imidazolinone family of ALS-inhibiting herbicides such as imazethapyr. Such a rice variety is sold under the trade name of Clearfield (registered trademark). Also included is a variety of soybean provided with tolerance to the sulfonyl urea family of ALS-inhibiting herbicides such as thifensulfuron-methyl by a classical breeding technique, and this is sold under the trade name of STS soybean. Also included are plants provided with tolerance to acetyl-CoA carboxylase inhibitors such as trione oxime herbicides and aryloxy phenoxy propionic acid herbicides by a classical breeding technique, for example, SR corn and the like.
Plants provided with tolerance to acetyl-CoA carboxylase inhibitors are described in Proc. Natl. Acad. Sci. USA, 87, 7175-7179 (1990), and the like. Further, acetyl-CoA carboxylase mutants resistant to acetyl-CoA carboxylase inhibitors are reported in Weed Science, 53, 728-746 (2005), and the like, and by introducing the gene of such an acetyl-CoA carboxylase mutant into plants by a gene recombination technique, or introducing a resistance-conferring mutation into acetyl-CoA carboxylase of plants, plants tolerant to acetyl-CoA carboxylase inhibitors can be engineered. Alternatively, by introducing a nucleic acid causing base substitution mutation into plant cells (a typical example of this technique is chimeraplasty technique (Gura T. 1999. Repairing the Genome's Spelling Mistakes. Science 285: 316-318.)) to allow site-specific substitution mutation in the amino acids encoded by an acetyl-CoA carboxylase gene, an ALS gene or the like of plants, plants tolerant to acetyl-CoA carboxylase inhibitors, ALS inhibitors or the like can be engineered. The nitrogen-containing heterocyclic compound of the present invention or a salt thereof can be used for these plants as well. The compound of the present invention does not do damage to these useful plants.

Examples of the weed that can be controlled by the nitrogen-containing heterocyclic compound of the present invention or a salt thereof include dicotyledonous genera such as *Ipomoea, Lindernia, Sesbania, Abutilon, Matricaria, Rorippa, Urtica, Lamium, Xanthium, Sinapis, Rotala, Veronica, Papaver, Chenopodium, Trifolium, Portulaca, Viola, Pharbitis, Galeopsis, Datura, Solanum, Capsella, Cirsium, Sonchus, Galinsoga, Stellaria, Senecio, Amaranthus, Ambrosia, Kochia, Lamium, Leipidium, Polygonum, Galium, Centaurea,* and *Artemisia.*

Examples of the weed that can be controlled by the nitrogen-containing heterocyclic compound of the present invention or a salt thereof include monocotyledonous genera such as *Poa, Bolboschoenus, Festuca, Setaria, Eleusine, Sagittaria, Agropyron, Ischaemum, Cyperus, Avena, Bromus, Panicum, Cynodon, Monochoria, Alopecurus, Paspalum, Commelina, Fimbristylis, Lolium, Brachiaria, Agrostis, Eleocharis, Echinochloa esculenta, Scirpus, Schoenoplectus, Digitaria,* and *Sorghum.*

Other examples of the weed that can be controlled by the nitrogen-containing heterocyclic compound of the present invention or a salt thereof include *Spirogyra* sp., *Amaranthus retroflexus, Amaranthus viridis, Setaria faberi, Leersia japonica, Leptochloa chinensis, Lindernia angustifolia, Lindernia procumbens, Dopatrium junceum, Ipomoea hederacea, Lindernia dubia, Sida spinosa, Polygonum pensylvanicum, Sesbania exaltata, Geranium carolinense, Chenopodium ambrosioides, Conyza bonariensis, Setaria italica, Amaranthus powellii, Polygonum cuspidatum, Abutilon theophrasti, Matricaria perforata, Polygonum longisetum, Veronica polita, Echinochloa crus-galli, Amaranthus lividus, Solanum nigrum, Schoenoplectus juncoides* (Roxb.) Palla, *Bromus catharticus, Murdannia keisak, Bolboschoenus fluviatilis, Scirpus maritimus, Bromus tectorum, Sagittaria pygmaea* Miq, *Rumex obtusifolius, Leersia oryzoides* (L.) Sw., *Setaria viridis, Cassia obtusifolia, Conyza sumatrensis, Veronica persica, Spirodela polyrhiza, Xanthium canadens, Coreopsis lanceolata, Panicum dichotomiflorum, Asclepias syriaca, Euphorbia maculata, Plantago asiatica, Rudbeckia laciniata, Amaranthus palmeri, Avena sativa, Xanthium strumarium, Avena sterilis, Eleusine indica, Sagittaria trifolia, Erodium cicutarium, Cerastium glomeratum, Matricaria matricarioides, Matricaria chamomilla, Vicia angustifolia, Bromus secalinus, Avena fatua, Rotala indica* Koehne, *Rumex japonicus, Paspalum distichum, Bromus remotiflorus, Cyperus esculentus, Galium kinuta, Setaria glauca, Pueraria lobata, Eleocharis kuroguwai* Ohwi, *Sagittaria trifolia* Caerulea, *Ambrosia trifida, Hydrilla verticillata, Bolboschoenus maritimus* (L.) Palla, *Chrysanthemum segetum, Cyperus iria, Monochoria vaginalis, Echinochloa colona, Alisma plantago-aquatica, Oryza sativa, Polygonum lapathifolium, Eleusine coracana, Schoenoplectus nipponicus, Cyperus malaccensis, Agropyron repens, Sorghum vulgare, Apera spica-venti, Chenopodium album, Trifolium repens, Datura stramonium, Equisetum arvense, Poa annua, Bromus japonicus, Alopecurus aequalis, Portulaca oleracea, Solidago altissima, Sorghum halepense, Brassica juncea, Taraxacum officinale, Convolvulus arvensis, Oenanthe javanica, Polygonum convolvulus, Echinochloa oryzicola* Vasing, *Ischaemum rugosum, Veronica arvensis, Cyperus difformis L., Amaranthus rudis, Phleum pratense, Ludwigia prostrata* Roxburgh, *Commelina communis, Panicum texanum, Euphorbia helioscopia, Festuca parvigluma, Rumex crispus, Capsella bursa-pastoris, Euphorbia pseudochamaesyce, Brachiaria plantaginea, Lolium multiflorum, Cirsium japonicum, Alopecurus myosuroides, Sinapis arvensis, Senecio vulgaris, Galinsoga ciliata, Amaranthus tricolor, Stellaria media, Cyperus papyrus, Cyperus rotundus, Amaranthus spinosus, Polygonum persicaria, Senecio cannabifolius, Cyperus flaccidus, Papaver rhoeas, Helianthus annuus, Lamium purpureum, Kyllinga gracillima, Ammannia multiflora, Erigeron canadensis, Potamogeton distinctus* A. Benn, *Amaranthus tuberculatus, Viola arvensis, Cirsium purpuratum, Ambrosia artemisiifolia, Schoenoplectus tabernaemontani, Veronica hederaefolia, Alopecurus myosuroides, Desmodium tortuosum, Plantago lanceolata, Kochia scoparia, Lolium rigidum, Ammannia coccinea, Lolium perenne, Scirpus juncoides* Roxburgh, *Lamium amplexicaule, Najas graminea, Amaranthus hybridus, Eleocharis acicularis* L., *Portulaca grandiflora, Ipomoea lacunosa, Ipomoea purpurea, Ipomoea hederacea* var. *integriuscula, Commelina bengharensis, Monochoria korsakowii, Cyperus serotinus* Rottboel, *Elatine triandra* Schk, *Digitaria ciliaris, Digitaria sanguinalis, Sorghum bicolor, Galium aparine, Artemisia princeps, Viola tricolor, Raphanus raphanistrum, Myosotis arvensis,* and *Alisma canaliculatum.* The nitrogen-containing heterocyclic compound of the present invention or a salt thereof inhibits growth of these weeds.

When the nitrogen-containing heterocyclic compound of the present invention or a salt thereof is used, it is commonly formulated into a convenient form for application, which is prepared by the usual method for preparing agrochemical formulations.
That is, the compound represented by the general formula (1) of the present invention or a salt thereof and an appropriate inactive carrier, and if needed an adjuvant, are blended at an appropriate ratio, and through the step of dissolution, separation, suspension, mixing, impregnation, adsorption and/or adhesion, are formulated into an appropriate form for application, such as a suspension concentrate, an emulsifiable concentrate, a soluble concentrate, a wettable powder, a water-dispersible granule, a granule, a dust, a tablet and a pack.

The composition (agricultural or horticultural herbicide) of the present invention can optionally contain an additive usually used for agrochemical formulations or agricultural or horticultural herbicides in addition to the active ingredient. Examples of the additive include carriers such as solid or liquid carriers, surfactants, dispersants, wetting agents, binders, tackifiers, thickeners, colorants, spreaders, sticking/spreading agents, antifreezing agents, anti-caking agents, disintegrants and stabilizing agents. If needed, preservatives, plant fragments, etc. may also be used as the additive. One of these additives may be used alone, and also two or more of them may be used in combination.

Examples of the solid carrier include natural minerals, such as quartz, clay, kaolinite, pyrophyllite, sericite, talc, bentonite, acid clay, attapulgite, zeolite and diatomite; inorganic salts, such as calcium carbonate, ammonium sulfate, sodium sulfate and potassium chloride; organic solid carriers, such as synthetic silicic acid, synthetic silicates, starch, cellulose and plant powders (for example, sawdust, coconut shell, corn cob, tobacco stalk, etc.); plastics carriers, such as polyethylene, polypropylene and polyvinylidene chloride; urea; hollow inorganic materials; hollow plastic materials; and fumed silica (white carbon). One of these may be used alone, and also two or more of them may be used in combination.

Examples of the liquid carrier include alcohols including monohydric alcohols, such as methanol, ethanol, propanol, isopropanol and butanol, and polyhydric alcohols, such as ethylene glycol, diethylene glycol, propylene glycol, hexylene glycol, polyethylene glycol, polypropylene glycol and glycerin; polyol compounds, such as propylene glycol ether; ketones, such as acetone, methyl ethyl ketone, methyl isobutyl ketone, diisobutyl ketone and cyclohexanone; ethers, such as ethyl ether, dioxane, ethylene glycol monoethyl ether, dipropyl ether and tetrahydrofuran; aliphatic hydrocarbons, such as normal paraffin, naphthene, isoparaffin, kerosene and mineral oil; aromatic hydrocarbons, such as benzene, toluene, xylene, solvent naphtha and alkyl naphthalene; halogenated hydrocarbons, such as dichloromethane, chloroform and carbon tetrachloride; esters, such as ethyl acetate, diisopropyl phthalate, dibutyl phthalate, dioctyl phthalate and dimethyl adipate; lactones, such as gamma-butyrolactone; amides, such as N,N-dimethylformamide, N,N-diethylformamide, N,N-dimethylacetamide and N-alkyl pyrrolidinone; nitriles, such as acetonitrile; sulfur compounds, such as dimethyl sulfoxide; vegetable oils, such as soybean oil, rapeseed oil, cotton seed oil and castor oil; and water. One of these may be used alone, and also two or more of them may be used in combination.

Exemplary surfactants used as a dispersant, a wetting agent, a spreader, a sticking/spreading agent, etc. include nonionic surfactants, such as sorbitan fatty acid ester, polyoxyethylene sorbitan fatty acid ester, sucrose fatty acid ester, polyoxyethylene fatty acid ester, polyoxyethylene resin acid ester, polyoxyethylene fatty acid diester, polyoxyethylene alkyl ether, polyoxyethylene alkyl aryl ether, polyoxyethylene alkyl phenyl ether, polyoxyethylene dialkyl phenyl ether, polyoxyethylene alkyl phenyl ether-formaldehyde condensates, polyoxyethylene-polyoxypropylene block copolymers, polystyrene-polyoxyethylene block polymers, alkyl polyoxyethylene-polypropylene block copolymer ether, polyoxyethylene alkylamine, polyoxyethylene fatty acid amide, polyoxyethylene fatty acid bis(phenyl ether), polyalkylene benzyl phenyl ether, polyoxyalkylene styryl phenyl ether, acetylene diol, polyoxyalkylene-added acetylene diol, polyoxyethylene ether-type silicone, ester-type silicone, fluorosurfactants, polyoxyethylene castor oil and polyoxyethylene hydrogenated castor oil; anionic surfactants, such as alkyl sulfates, polyoxyethylene alkyl ether sulfates, polyoxyethylene alkyl phenyl ether sulfates, polyoxyethylene styryl phenyl ether sulfates, alkylbenzene sulfonates, alkylaryl sulfonates, lignosulfonates, alkyl sulfosuccinates, naphthalene sulfonates, alkylnaphthalene sulfonates, salts of naphthalenesulfonic acid-formaldehyde condensates, salts of alkylnaphthalenesulfonic acid-formaldehyde condensates, fatty acid salts, polycarboxylic acid salts, polyacrylates, N-methyl-fatty acid sarcosinates, resinates, polyoxyethylene alkyl ether phosphates and polyoxyethylene alkyl phenyl ether phosphates; cationic surfactants including alkyl amine salts, such as lauryl amine hydrochloride, stearyl amine hydrochloride, oleyl amine hydrochloride, stearyl amine acetate, stearyl aminopropyl amine acetate, alkyl trimethyl ammonium chloride and alkyl dimethyl benzalkonium chloride; and amphoteric surfactants, such as amino acid-type or betaine-type amphoteric surfactants. One of these surfactants may be used alone, and also two or more of them may be used in combination.

Examples of the binder or the tackifier include carboxymethyl cellulose or salts thereof, dextrin, soluble starch, xanthan gum, guar gum, sucrose, polyvinyl pyrrolidone, gum arabic, polyvinyl alcohol, polyvinyl acetate, sodium polyacrylate, polyethylene glycols with an average molecular weight of 6,000 to 20,000, polyethylene oxides with an average molecular weight of 100,000 to 5,000,000, phospholipids (for example, cephalin, lecithin, etc.), cellulose powder, dextrin, modified starch, polyaminocarboxylic acid chelating compounds, cross-linked polyvinyl pyrrolidone, maleic acid-styrene copolymers, (meth)acrylic acid copolymers, half esters of polyhydric alcohol polymer and dicarboxylic anhydride, water soluble polystyrene sulfonates, paraffin, terpene, polyamide resins, polyacrylates, polyoxyethylene, waxes, polyvinyl alkyl ether, alkylphenol-formaldehyde condensates and synthetic resin emulsions.

Examples of the thickener include water soluble polymers, such as xanthan gum, guar gum, diutan gum, carboxymethyl cellulose, polyvinyl pyrrolidone, carboxyvinyl polymers, acrylic polymers, starch compounds and polysaccharides; and inorganic fine powders, such as high grade bentonite and fumed silica (white carbon).

Examples of the colorant include inorganic pigments, such as iron oxide, titanium oxide and Prussian blue; and organic dyes, such as alizarin dyes, azo dyes and metal phthalocyanine dyes.

Examples of the antifreezing agent include polyhydric alcohols, such as ethylene glycol, diethylene glycol, propylene glycol and glycerin.

Examples of the adjuvant serving to prevent caking or facilitate disintegration include polysaccharides (starch, alginic acid, mannose, galactose, etc.), polyvinyl pyrrolidone, fumed silica (white carbon), ester gum, petroleum resin, sodium tripolyphosphate, sodium hexametaphosphate, metal stearates, cellulose powder, dextrin, methacrylate copolymers, polyvinyl pyrrolidone, polyaminocarboxylic acid chelating compounds, sulfonated styrene-isobutylene-maleic anhydride copolymers and starch-polyacrylonitrile graft copolymers.

Examples of the stabilizing agent include desiccants, such as zeolite, quicklime and magnesium oxide; antioxidants, such as phenolic compounds, amine compounds, sulfur compounds and phosphoric acid compounds; and ultraviolet absorbers, such as salicylic acid compounds and benzophenone compounds.

Examples of the preservative include potassium sorbate and 1,2-benzothiazolin-3-one.
Further, other adjuvants including functional spreading agents, activity enhancers such as metabolic inhibitors (piperonyl butoxide etc.), antifreezing agents (propylene glycol etc.), antioxidants (BHT etc.) and ultraviolet absorbers can also be used if needed.

The amount of the active ingredient compound in the agricultural or horticultural herbicide of the present invention can be adjusted as needed, and basically, the amount of the active ingredient compound is appropriately selected from the range of 0.01 to 90 parts by weight in 100 parts by weight of the agricultural or horticultural herbicide. For example, in the case where the agricultural or horticultural herbicide is a dust, a granule, an emulsifiable concentrate or a wettable powder, it is suitable that the amount of the active ingredient compound is 0.01 to 50% by weight relative to the total weight of the agricultural or horticultural herbicide.

The application rate of the nitrogen-containing heterocyclic compound of the present invention or a salt thereof may vary with various factors, for example, the purpose, the target weed, the growing conditions of crops, the tendency of weed infestation, the weather, the environmental conditions, the formulation, the application method, the application site, the application timing, etc., but basically, the application rate of the active ingredient compound is appropriately selected from the range of 0.001 g to 10 kg, and preferably 0.01 g to 1 kg per 10 ares depending on the purpose.

In order to control weeds, the agricultural or horticultural herbicide comprising the nitrogen-containing heterocyclic compound of the present invention or a salt thereof as an active ingredient, with or without appropriate dilution or suspension in water etc., is applied directly to the foliage of weeds in an amount effective for the control of the weeds. In addition to foliar application, seed treatment for useful plants, such as dipping, dust coating and calcium peroxide coating, can be performed. Further, treatment of soil or growing media may also be performed, and examples of such treatment include whole soil incorporation, planting row treatment, bed soil incorporation, plug seedling treatment, planting hole treatment, plant foot treatment, top-dressing, treatment of nursery boxes for paddy rice, and submerged application.

Exemplary methods of seed treatment for useful plants include dipping of seeds in a diluted or undiluted fluid of a liquid or solid formulation for the permeation of agrochemicals into the seeds; mixing or dust coating of seeds with a solid or liquid formulation for the adherence of the formulation onto the surfaces of the seeds; coating of seeds with a mixture of a solid or liquid formulation and an adhesive carrier such as resins and polymers; and application of a solid or liquid formulation to the vicinity of seeds at the same time as seeding.
The term "seed" in the seed treatment refers to a plant body which is in the early stages of cultivation and used for useful plant propagation. The examples include, in addition to a so-called seed, a plant body for vegetative propagation, such as a bulb, a tuber, a seed potato, a bulbil, a propagule, a discoid stem and a stem used for cuttage.

The term "soil" or "growing medium" in the method of the present invention for using an agricultural or horticultural herbicide refers to a support medium for crop cultivation, in particular a support medium which allows crop plants to spread their roots therein, and the materials are not particularly limited as long as they allow useful plants to grow. Examples of the support medium include what is called soils, seedling mats and water, and specific examples of the materials include sand, pumice, vermiculite, diatomite, agar, gelatinous substances, high-molecular-weight substances, rock wool, glass wool, wood chip and bark.

In the case of the application to nursery boxes for paddy rice, the type of the formulation may vary depending on whether the application is performed at the time of seeding, in the greening period, at the time of transplanting, or the like. For example, a dust, a water-dispersible granule, a granule, or the like may be used. Such a formulation can be applied by incorporation into nursery soil. For example, a dust, a water-dispersible granule, a granule, or the like may be incorporated into bed soil, covering soil, or the whole nursery soil. Simply, nursery soil and such a formulation may be alternately layered.

In the application to paddy fields, a solid formulation, such as a jumbo, a pack, a granule and a water-dispersible granule, or a liquid formulation, such as a flowable and an emulsifiable concentrate, is applied usually to flooded paddy fields. In a rice planting period, a suitable formulation, as it is or after mixed with a fertilizer, may be applied onto soil or injected into soil. In addition, an emulsifiable concentrate, a flowable or the like may be applied to the source of water supply for paddy fields, such as a water inlet and an irrigation device.
In this case, treatment can be accomplished with the supply of water and thus achieved in a labor-saving manner. In the case of using spraying equipment, it can be any equipment that is usually used, and examples include tractor mounted boom sprayers, manned helicopters, radio-controlled helicopters, radio-controlled boats, drones, one-shot sprayers, power (manual or automatic) sprayers, carry power sprayers, backpack power sprayers, and hand operated sprayers.

For the expansion of the range of target weeds and the appropriate time for weed control, or for dose reduction, the nitrogen-containing heterocyclic compound of the present invention or a salt thereof can be used after mixed with other herbicides, plant growth regulators, phytotoxicity reducers (safeners), soil conditioners, fertilizers, and/or the like. Further, the compound of the present invention or a salt thereof can be used after mixed with agricultural or horticultural insecticides, acaricides, nematicides, microbicides, biopesticides and/or the like depending on the situation. Non-limiting examples of typical compounds are described below.

Exemplary herbicides used for the same purposes as above include 1-naphthylacetamide, 2,4-PA, 2,3,6-TBA, 2,4,5-T, 2,4,5-TB, 2,4-D, 2,4-DB, 2,4-DEB, 2,4-DEP, 3,4-DA, 3,4-DB, 3,4-DP, 4-CPA, 4-CPB, 4-CPP, MCP, MCPA, MCPA-thioethyl, MCPB, ioxynil, icafolin, aclonifen, azafenidin, acifluorfen, aziprotryne, azimsulfuron, asulam, acetochlor, atrazine, atraton, anisuron, anilofos, aviglycine, abscisic acid, amicarbazone, amidosulfuron, amitrole, aminocyclopyrachlor, aminopyralid, amibuzin, amiprophos-methyl, ametridione, ametryn, alachlor, allidochlor, alloxydim, alorac, iofensulfuron, isouron, isocarbamid, isoxachlortole, isoxapyrifop, isoxaflutole, isoxaben, isocil, isonoruron, isoproturon, isopropalin, isopolinate, isomethiozin, inabenfide, ipazine, iptriazopyrid, ipfencarbazone, iprymidam, imazaquin, imazapic, imazapyr, imazamethapyr, imazamethabenz, imazamethabenz-methyl, imazamox, imazethapyr, imazosulfuron, indaziflam, indanofan, indolauxipyr, indolauxipyr-cyanomethyl, indolebutyric acid, uniconazole-P, eglinazine, esprocarb, ethametsulfuron, ethametsulfuron-methyl, ethalfluralin, ethiolate, ethychlozate-ethyl, ethidimuron, etinofen, ethephon, ethoxysulfuron, ethoxyfen, etnipromid, ethofumesate, etobenzanid, epyrifenacil, epronaz, erbon, endothal, oxadiazon, oxadiargyl, oxaziclomefone, oxasulfuron, oxapyrazon, oxyfluorfen, oryzalin, orthosulfamuron, orbencarb, cafenstrole, cambendichlor, carbasulam, carfentrazone, carfentrazone-ethyl, karbutilate, carbetamide, carboxazole, quizalofop, quizalofop-P, quizalofop-ethyl, xylachlor, quinoclamine, quinonamid, quinclorac, quinmerac, cumyluron, clacyfos, cliodinate, glyphosate, glufosinate, glufosinate-P, credazine, clethodim, cloxyfonac, clodinafop, clodinafop-propargyl, chlorotoluron, clopyralid, cloproxydim, cloprop, chlorbromuron, clofop, clomazone, chlomethoxynil, chlomethoxyfen, clomeprop, chlorazifop, chlorazine, cloransulam, chloranocryl, chloramben, cloransulam-methyl, chloridazon, chlorimuron, chlorimuron-ethyl, chlorsulfuron, chlorthal, chlorthiamid, chlornitrofen, chlorfenac, chlorfenprop, chlorbufam, chlorflurazole, chlorflurenol, chlorprocarb, chlorpropham, chlormequat, chloreturon, chloroxynil, chloroxuron, chloropon, saflufenacil, cyanazine, cyanatryn, di-allate, diuron, diethamquat, dioxopyritrione, dicamba, cycluron, cycloate, cycloxydim, diclosulam, cyclosulfamuron, cyclopyranil, cyclopyrimorate, dichlorprop, dichlorprop-P, dichlobenil, diclofop, diclofop-methyl, dichlormate, dichloralurea, diquat, cisanilide, disul, siduron, dithiopyr, dinitramine, cinidon-ethyl, dinosam, cinosulfuron, dinoseb, dinoterb, dinofenate, dinoprop, cyhalofop-butyl, cypyrafluone, diphenamid, difenoxuron, difenopenten, difenzoquat, cybutryne, cyprazine, cyprazole, diflufenican, diflufenzopyr, dipropetryn, cypromid, cyperquat, gibberellin, simazine, dimexano, dimesulfazet, dimethachlor, dimidazon, dimethametryn, dimethenamid, simetryn, simeton, dimepiperate, dimefuron, cinmethylin, swep, sulglycapin, sulcotrione, sulfallate, sulfentrazone, sulfosulfuron, sulfometuron, sulfometuron-methyl, secbumeton, sethoxydim, sebuthylazine, terbacil, daimuron, dazomet, dalapon, thiazafluron, thiazopyr, tiafenacil, thiencarbazone, thiencarbazone-methyl, tiocarbazil, tioclorim, thiobencarb, thidiazimin, thidiazuron, thifensulfuron, thifensulfuron-methyl, desmedipham, desmetryn, tetflupyrolimet, tetrafluron, thenylchlor, tebutam, tebuthiuron, terbumeton, tepraloxydim, tefuryltrione, tembotrione, delachlor, terbacil, terbucarb, terbuchlor, terbuthylazine, terbutryn, topramezone, tralkoxydim, triaziflam, triasulfuron, triafamone, tri-allate, trietazine, tricamba, triclopyr, tridiphane, tritac, tritosulfuron, tripyrasulfone, trifludimoxazin, triflusulfuron, triflusulfuron-methyl, trifluralin, trifloxysulfuron, tripropindan, tribenuron-methyl, tribenuron, trifop, trifopsime, trimeturon, tolpyralate, naptalam, naproanilide, napropamide, nicosulfuron, nitralin, nitrofen, nitrofluorfen, nipyraclofen, neburon, norflurazon, noruron, barban, paclobutrazol, paraquat, parafluron, haloxydine, halauxifen, haloxyfop, haloxyfop-P, haloxyfop-methyl, halosafen, halosulfuron, halosulfuron-methyl, bixlozone, picloram, picolinafen, bicyclopyrone, bispyribac, bispyribac-sodium, pydanon, pinoxaden, bipyrazone, bifenox, piperophos, hymexazol, pyraquinate, pyraclonil, pyrasulfotole, pyrazoxyfen, pyrazosulfuron, pyrazosulfuron-ethyl, pyrazolate, bilanafos, pyraflufenethyl, pyriclor, pyridafol, pyrithiobac, pyrithiobac-sodium, pyridate, pyriftalid, pyributicarb, pyriflubenzoxim, pyribenzoxim, pyrimisulfan, primisulfuron, pyriminobac-methyl, flusulfinam, broclozone, pyroxasulfone, pyroxsulam, fenasulam, phenisopham, fenuron, fenoxasulfone, fenoxaprop, fenoxaprop-P, fenoxaprop-ethyl, phenothiol, fenoprop, phenobenzuron, fenquinotrione, fenthiaprop, fenteracol, fentrazamide, fenpyrazone, phenmedipham, phenmedipham-ethyl, butachlor, butafenacil, butamifos, buthiuron, buthidazole, butylate, buturon, butenachlor, butroxydim, butralin, flazasulfuron, flamprop, furyloxyfen, prynachlor, primisulfuronmethyl, fluazifop, fluazifop-P, fluazifop-butyl, fluazolate, fluchloraminopyr, fluchloraminopyr-tefuryl, fluroxypyr, fluothiuron, fluometuron, fluoroglycofen, flurochloridone, fluorodifen, fluoronitrofen, fluoromidine, flucarbazone, flucarbazonesodium, fluchloralin, flucetosulfuron, fluthiacet, fluthiacet-methyl, flupyrsulfuron, flufenacet, flufenoximacil, flufenican, flufenpyr, flupropacil, flupropanate, flupoxam, flumioxazin, flumiclorac, flumiclorac-pentyl, flumipropyn, flumezin, fluometuron, flumetsulam, fluridone, flurtamone, fluroxypyr, pretilachlor, proxan, proglinazine, procyazine, prodiamine, prosulfalin, prosulfuron, prosulfocarb, propaquizafop, propachlor, propazine, propanil, propyzamide, propisochlor, prohydrojasmon, propyrisulfuron, propham, profluazol, profluralin, prohexadione-calcium, propoxycarbazone, propoxycarbazone-sodium, profoxydim, bromacil, brompyrazon, prometryn, prometon, bromoxynil, bromofenoxim, bromobutide, bromobonil, florasulam, florpyrauxifen, hexachloroacetone, hexazinone, pethoxamid, benazolin, penoxsulam, pebulate, beflubutamid, beflubutamid-M, vernolate, perfluidone, bencarbazone, benquitrione, benzadox, benzipram, benzylaminopurine, benzthiazuron, benzfendizone, bensulide, bensulfuron-methyl, benzoylprop, benzobicyclon, benzofenap, benzofluor, bentazone, pentanochlor, benthiocarb, pendimethalin, pentoxazone, benfluralin, benfuresate, fosamine, fomesafen, foramsulfuron, forchlorfenuron, maleic hydrazide, mecoprop, mecoprop-P, medinoterb, mesosulfuron, mesosulfuron-methyl, mesotrione, mesoprazine, methoprotryne, metazachlor, methazole, metazosulfuron, methabenzthiazuron, metamitron, metamifop, metam, methalpropalin, methiuron, methiozolin, methiobencarb, methyldymron, metoxuron, metosulam, metsulfuron, metsulfuron-methyl, metflurazon, metobromuron, metobenzuron, methometon, metolachlor, metribuzin, mepiquat-chloride, mefenacet, mefluidide, monalide, monisouron, monuron, monochloroacetic acid, monolinuron, molinate, morfamquat, iodosulfuron, iodosulfuron-methyl-sodium, iodobonil, iodomethane, lactofen, lancotrione, linuron, rimisoxafen, rimsulfuron, lenacil, rhodethanil, calcium peroxide and methyl bromide. Biopesticides available as herbicides, such as Xanthomonas campestris, can also be used after mixed with the compound of the present invention or a salt thereof.

Examples of the phytotoxicity reducer (safener) include 1,8-naphthalic anhydride, isoxadifen-ethyl, furilazole, cyprosulfamide, cyometrinil, dichlormid, dimepiperate, thiencarbazone-methyl, fenchlorazone-ethyl, fenclorim, fluxofenim, flurazole, benoxacor, metcamifen, and mefenpyr-diethyl.

Other examples of the biopesticide include natural predators such as *Encarsia formosa, Aphidius colemani, Aphidoletes aphidimyza, Diglyphus isaea, Dacnusa sibirica, Phytoseiulus persimilis, Amblyseius cucumeris,* and *Orius sauteri;* microbial pesticides such as *Beauveria brongniartii;* and pheromones such as (Z)-10-tetradecenyl acetate, (E,Z)-4,10-tetradecadienyl acetate, (Z)-8-dodecenyl acetate, (Z)-11-tetradecenyl acetate, (Z)-13-icosen-10-one, and 14-methyl-1-octadecene.

### EXAMPLES

Hereinafter, representative Examples in relation to the present invention are shown, but the present invention is not limited thereto.

### Production Example 1

Production of (Z)-ethyl 6-(N'-ethoxycarbamimidoyl)-2-(4-methyl-5-oxo-1-phenyl-4,5-dihydro-1H-1,2,4-triazol-3-yl)nicotinate (Compound No. 5-3)

### Example 1-1

Production of (Z)-ethyl 6-(N'-hydroxycarbamimidoyl)-2-(4-methyl-5-oxo-1-phenyl-4,5-dihydro-1H-1 ,2,4-triazol-3-yl)nicotinate

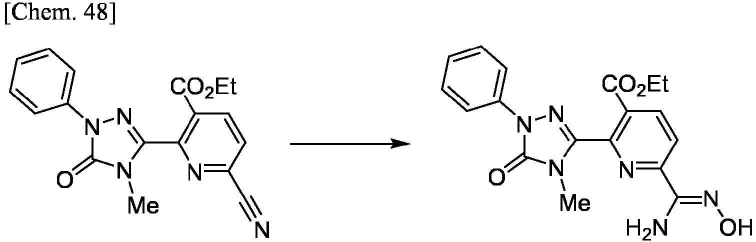

To a solution of ethyl 6-cyano-2-(4-methyl-5-oxo-1-phenyl-4,5-dihydro-1H-1,2,4-triazol-3-yl)nicotinate (0.73 g, 2.1 mmol) in ethanol (21 mL), hydroxylamine hydrochloride (0.22 g, 3.2 mmol) and sodium acetate (0.26 g, 3.2 mmol) were added, and the mixture was stirred and heated under reflux for 2 hours. The reaction mixture was concentrated in vacuo. A saturated aqueous sodium hydrogen carbonate solution was added to the residue, and ethyl acetate extraction was performed. The organic layer was dried over anhydrous magnesium sulfate and then concentrated in vacuo. The residue was purified by silica gel column chromatography to give (Z)-ethyl 6-(N'-hydroxycarbamimidoyl)-2-(4-methyl-5-oxo-1-phenyl-4,5-dihydro-1H-1,2,4-triazol-3-yl)nicotinate (0.72 g, 1.6 mmol).
Yield: 89%
Physical property: Melting point: 127 to 128°C

### Production Example 1-2

Production of (Z)-ethyl 6-(N'-ethoxycarbamimidoyl)-2-(4-methyl-5-oxo-1-phenyl-4,5-dihydro-1H-1,2,4-triazol-3-yl)nicotinate (Compound No. 5-3)

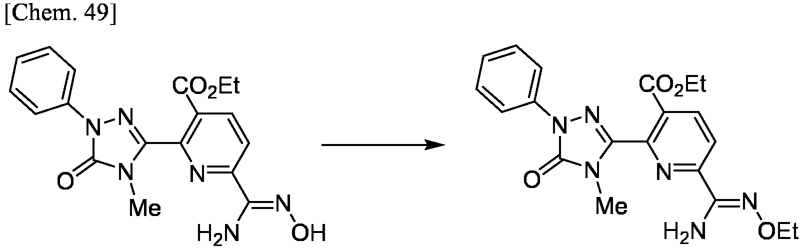

To a solution of (Z)-ethyl 6-(N'-hydroxycarbamimidoyl)-2-(4-methyl-5-oxo-1-phenyl-4,5-dihydro-1H-1,2,4-triazol-3-yl)nicotinate (0.30 g, 0.78 mmol) in N,N-dimethylformamide (8.0 mL), cesium carbonate (0.51 g, 1.6 mmol) and ethyl iodide (0.13 mL, 1.6 mmol) were added at room temperature, and the mixture was stirred at room temperature. A saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give (Z)-ethyl 6-(N'-ethoxycarbamimidoyl)-2-(4-methyl-5-oxo-1-phenyl-4,5-dihydro-1H-1,2,4-triazol-3-yl)nicotinate (0.35 mg, 0.78 mmol).
Yield: 100%
Physical property: Refractive index: 1.4692 (20.1°C)

### Production Example 2

Production of (Z)-ethyl 6-(N'-ethoxycarbamimidoyl)-2-(5-methyl-2-phenylthiazol-4-yl)nicotinate (Compound No. 7-1)

### Production Example 2-1

Production of (Z)-ethyl 6-(N'-hydroxycarbamimidoyl)-2-(5-methyl-2-phenylthiazol-4-yl)nicotinate

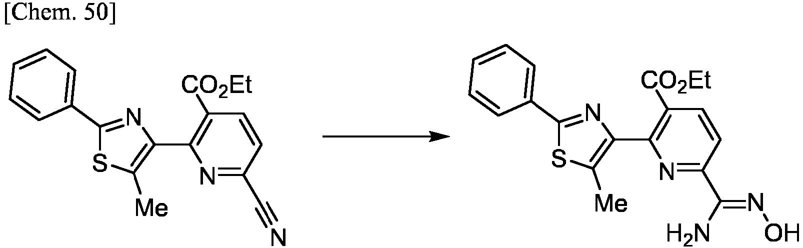

To a solution of ethyl 6-cyano-2-(5-methyl-2-phenylthiazol-4-yl)nicotinate (63 mg, 0.18 mmol) in ethanol (1.0 mL), hydroxylamine hydrochloride (20 mg, 0.27 mmol) and sodium acetate (23 mg, 0.27 mmol) were added at room temperature, and the mixture was stirred and heated under reflux for 3 hours. After the completion of the reaction, the reaction mixture was concentrated in vacuo. A saturated aqueous sodium hydrogen carbonate solution was added to the residue, and ethyl acetate extraction was performed. The organic layer was dried over anhydrous magnesium sulfate and then concentrated in vacuo to give (Z)-ethyl 6-(N'-hydroxycarbamimidoyl)-2-(5-methyl-2-phenylthiazol-4-yl)nicotinate (51 mg, 0.13 mmol).
Yield: 74%

### Production Example 2-2

Production of (Z)-ethyl 6-(N'-ethoxycarbamimidoyl)-2-(5-methyl-2-phenylthiazol-4-yl)nicotinate (Compound No. 7-1)

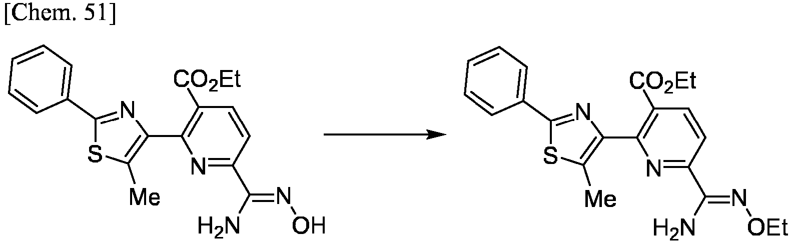

To a solution of (Z)-ethyl 6-(N'-hydroxycarbamimidoyl)-2-(5-methyl-2-phenylthiazol-4-yl)nicotinate (40 mg, 0.11 mmol) in N,N-dimethylformamide (8.0 mL), cesium carbonate (70 mg, 0.21 mmol) and ethyl iodide (20 µL, 0.21 mmol) were added at room temperature, and the mixture was stirred at room temperature for 2 hours. After the completion of the reaction, a saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give (Z)-ethyl 6-(N'-ethoxycarbamimidoyl)-2-(5-methyl-2-phenylthiazol-4-yl)nicotinate (36 mg, 0.088 mmol).
Yield: 83%
Physical property: ¹H-NMR (CDCl₃): δ 8.06 (d, 1H), 8.01 (d, 1H), 7.91-7.81 (m, 2H), 7.44-7.38 (m, 3H), 5.53 (s, 2H), 4.21 (q, 2H), 4.18 (q, 2H), 2.73 (s, 3H), 1.35 (t, 3H), 1.08 (t, 3H)

### Production Example 3

Production of (Z)-N'-ethoxy-6-(1-methyl-3-phenyl-1H-1,2,4-triazol-5-yl)-5-(N-methylsulfamoyl)picolinimidamide (Compound No. 1-4)

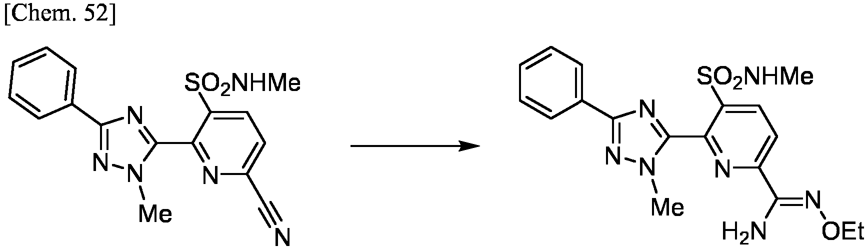

To a solution of 6-cyano-N-methyl-2-(1-methyl-3-phenyl-1H-1,2,4-triazol-5-yl)pyridine-3-sulfonamide (0.16 g, 0.45 mmol) in methanol (2.0 mL), a methanol solution of sodium methoxide (28 wt%, 0.10 mL, 0.45 mmol) was added, and the mixture was stirred at room temperature for 30 minutes. After the disappearance of the starting compound was confirmed, O-ethylhydroxylamine hydrochloride (67 mg, 0.69 mmol) was added to the reaction mixture, and the mixture was stirred at room temperature for 30 minutes. A saturated aqueous ammonium chloride solution was added, and ethyl acetate extraction was performed. The organic layer was concentrated in vacuo, and the residue was purified by silica gel column chromatography to give (Z)-N'-ethoxy-6-(1-methyl-3-phenyl-1H-1,2,4-triazol-5-yl)-5-(N-methylsulfamoyl)picolinimidamide (89.1 mg, 0.21 mmol).
Yield: 47%
Physical property: ¹H-NMR (CDCl₃): δ 8.46 (d, 1H), 8.26 (d, 1H), 8.03-7.99 (m, 2H), 7.79-7.73 (m, 1H), 7.51-7.43 (m, 3H), 5.44 (s, 2H), 4.24 (q, 2H), 4.10 (s, 1H), 2.79 (d, 3H), 1.37 (t, 3H)

### Production Example 4

Production of (Z)-N'-ethoxy-6-(1-methyl-4-phenyl-1H-imidazol-2-yl)-5-(N-methylsulfamoyl)picolinimidamide (Compound No. 2-33)

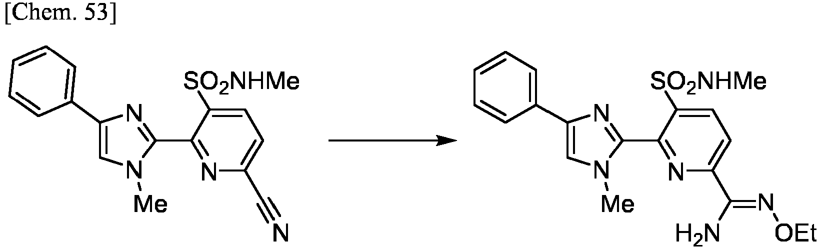

To a solution of 6-cyano-N-methyl-2-(1-methyl-4-phenyl-1H-imidazol-2-yl)pyridine-3-sulfonamide (0.10 g, 0.28 mmol) in methanol (1.0 mL), a methanol solution of sodium methoxide (28 wt%, 0.050 mL, 0.77 mmol) was added, and the mixture was stirred at room temperature for 30 minutes. After the disappearance of the starting compound was confirmed, O-ethylhydroxylamine hydrochloride (42 mg, 0.42 mmol) was added, and the mixture was stirred at room temperature for 30 minutes. A saturated aqueous ammonium chloride solution was added to the reaction mixture, and ethyl acetate extraction was performed. The organic layer was concentrated, and the residue was purified by silica gel column chromatography to give the desired product (83 mg, 0.20 mmol).
Yield: 72%
Physical property: Melting point: 237 to 238°C

### Production Example 5

Production of (Z)-N'-ethoxy-6-(5-methyl-3-phenyl-1H-1,2,4-triazol-1-yl)-5-(N-methylsulfamoyl)picolinimidamide (Compound No. 3-3)

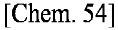

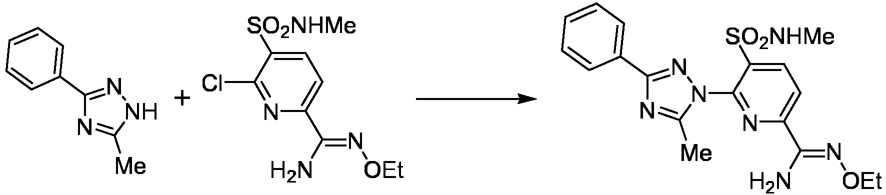

To a solution of (Z)-6-chloro-N'-ethoxy-5-(N-methylsulfamoyl)picolinimidamide (80 mg, 0.27 mmol) in N,N-dimethylacetamide (1.5 mL), potassium carbonate (0.13 g, 0.96 mmol) and 5-methyl-3-phenyl-1H-1,2,4-triazole (53 mg, 0.27 mmol) were added, and the mixture was stirred at 130°C for 4 hours. After the completion of the reaction, water and ethyl acetate were added for extraction. The organic layer was dried over anhydrous sodium sulfate and then concentrated in vacuo. The residue was purified by silica gel column chromatography to give (Z)-N'-ethoxy-6-(5-methyl-3-phenyl-1H-1,2,4-triazol-1-yl)-5-(N-methylsulfamoyl)picolinimidamide (84 mg, 0.19 mmol).
Yield: 68%
Physical property: ¹H-NMR (CDCl₃): δ 8.48 (d, 1H), 8.28 (d, 1H), 8.04-8.01 (m, 2H), 7.49-7.45 (m, 3H), 6.34 (q, 1H), 5.39 (brs, 2H), 4.24 (q, 2H), 2.79 (d, 3H), 2.61 (s, 3H), 1.37 (t, 3H)

### Production Example 6

Production of (Z)-N'-ethoxy-6-(5-methyl-3-phenyl-1H-pyrazol-1-yl)-5-(N-methylsulfamoyl)picolinimidamide (Compound No. 4-6)

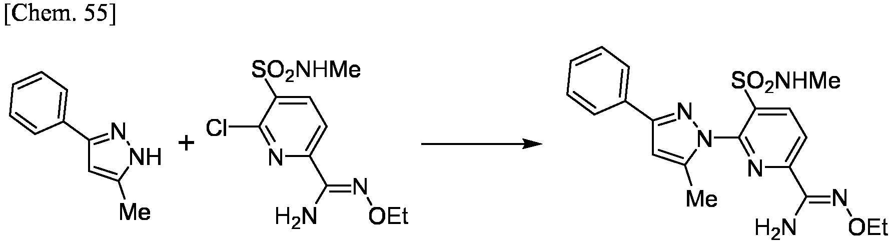

To a solution of (Z)-6-chloro-N'-ethoxy-5-(N-methylsulfamoyl)picolinimidamide (70 mg, 0.25 mmol) in N,N-dimethylacetamide (1.4 mL), potassium carbonate (0.12 g, 0.87 mmol) and 5-methyl-3-phenyl-1H-pyrazole (40 mg, 0.25 mmol) were added, and the mixture was stirred at 130°C for 5 hours. After the completion of the reaction, water and ethyl acetate were added to the reaction mixture for extraction. The organic layer was dried over anhydrous sodium sulfate and then concentrated in vacuo. The residue was purified by silica gel column chromatography to give (Z)-N'-ethoxy-6-(5-methyl-3-phenyl-1H-pyrazol-1-yl)-5-(N-methylsulfamoyl)picolinimidamide (28 mg, 0.070 mmol).
Yield: 28%
Physical property: ¹H-NMR (CDCl₃): δ 8.46 (d, 1H), 8.20 (d, 1H), 7.72-7.69 (m, 1H), 7.45-7.40 (m, 2H), 7.38-7.34 (m, 1H), 6.79 (q, 1H), 6.60 (s, 1H), 5.41 (brs, 2H), 4.23 (q, 2H), 2.77 (d, 3H), 2.43 (s, 3H), 1.37 (t, 3H)

### Production Example 7

Production of (E)-4-(6-(1-(ethoxyimino)ethyl)-3-(methylsulfonyl)pyridin-2-yl)-3-methyl-1-phenyl-1H-1,2,4-triazol-5(4H)-one (Compound No. 6-1)

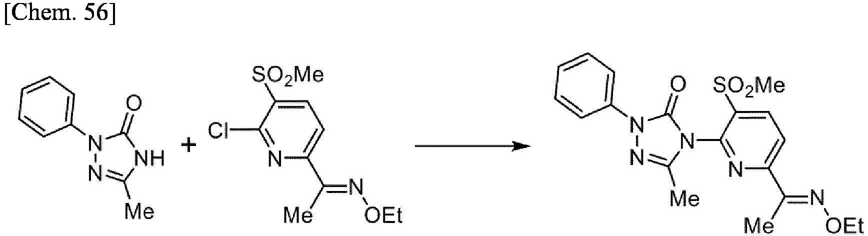

To an N,N-dimethylformamide solution (1.0 mL) of (E)-1-(6-chloro-5-(methylsulfonyl)pyridin-2-yl)ethanone O-ethyl oxime (60 mg, 0.22 mmol), 3-methyl-1-phenyl-1H-1,2,4-triazol-5(4H)-one (76 mg, 0.43 mmol), 4,5'-bis(diphenylphosphino)-9,9'-dimethyl xanthene (25 mg, 0.043 mmol), and tris(dibenzylideneacetone)dipalladium(0) (20 mg, 0.022 mmol) were added, and the mixture was stirred at 120°C for 3 hours. A saturated aqueous ammonium chloride solution was added to the reaction mixture, and ethyl acetate extraction was performed, followed by washing with saturated brine. The organic layer was dried over anhydrous magnesium sulfate and then concentrated in vacuo. The residue was purified by silica gel column chromatography to give (E)-4-(6-(1-(ethoxyimino)ethyl)-3-(methylsulfonyl)pyridin-2-yl)-3-methyl-1-phenyl-1H-1,2,4-triazol-5(4H)-one (4.0 mg, 0.0084 mmol).
Yield: 4%
Physical property: ¹H-NMR (CDCl₃): δ 8.44 (d, 1H), 8.24 (d, 1H), 8.02-7.95 (m, 2H), 7.49-7.40 (m, 2H), 7.26-7.21 (m, 1H), 4.36 (q, 2H), 3.40 (s, 3H), 2.29 (s, 3H), 2.21 (s, 3H), 1.38 (t, 3H)

### Production Example 8

Production of (Z)-3-(3-(2-chlorophenyl)-1-methyl-1H-1,2,4-triazol-5-yl)-N'-ethoxy-4-(N-methylsulfamoyl)benzimidamide (Compound No. 17-2)

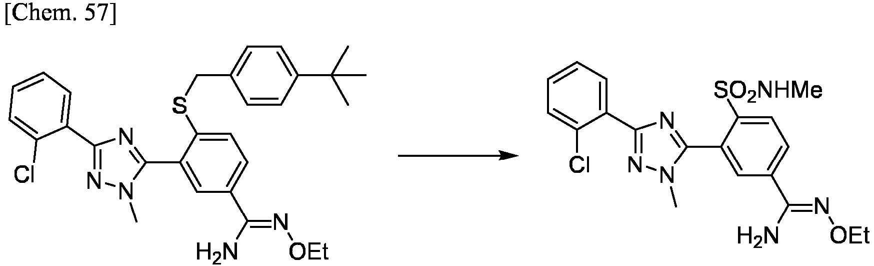

To a solution of (Z)-4-((4-(tert-butyl)benzyl)thio)-3-(3-(2-chlorophenyl)-1-methyl-1H-1,2,4-triazol-5-yl)-N'-ethoxybenzimidamide (0.23 g, 0.42 mmol) in chloroform (4.2 mL), 1,3-dichloro-5,5-dimethylhydantoin (0.25 g, 1.3 mmol), acetic acid (75 µL, 1.3 mmol), and water (45 µL, 2.5 mmol) were added at 0°C, and the mixture was stirred at the same temperature for 10 minutes. A methanol solution of methylamine (9.8 M, 0.43 mL, 4.2 mmol) was added, and the mixture was stirred for 10 minutes. Water was added to the reaction mixture, and chloroform extraction was performed. The organic layer was dried over anhydrous magnesium sulfate and then concentrated in vacuo. The residue was purified by silica gel column chromatography to give (Z)-3-(3-(2-chlorophenyl)-1-methyl-1H-1,2,4-triazol-5-yl)-N'-ethoxy-4-(N-methylsulfamoyl)benzimidamide (72 mg, 0.16 mmol).
Yield: 37%
Physical property: Melting point: 167 to 168°C

### Production Example 9

Production of (Z)-3-(3-(2-chlorophenyl)-1-methyl-1H-1,2,4-triazol-5-yl)-N'-ethoxy-4-(N-methylsulfamoyl)benzimidamide (Compound No. 1-12)

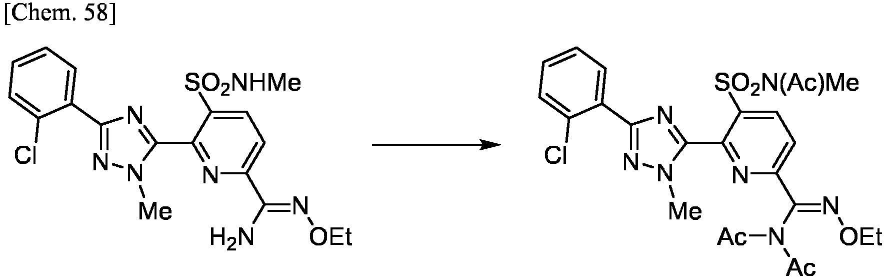

To a solution of (Z)-6-(3-(2-chlorophenyl)-1-methyl-1H-1,2,4-triazol-5-yl)-N'-ethoxy-5-(N-methylsulfanyl)picolinimidamide (0.10 g, 0.22 mmol) in N,N-dimethylacetamide (2 mL), acetic anhydride (0.067 g, 0.33 mmol) and cesium carbonate (0.22 g, 0.33 mmol) were added at room temperature, and the mixture was stirred at 50°C for 10 minutes. A methanol solution of methylamine (9.8 M, 0.43 mL, 4.2 mmol) was added, and the mixture was stirred for 10 minutes. Water was added to the reaction mixture, and chloroform extraction was performed. The organic layer was dried over anhydrous magnesium sulfate and then concentrated in vacuo. The residue was purified by silica gel column chromatography to give (Z)-N-acetyl-N-((5-(N-acetyl-N-methylsulfamoyl)-6-(3-(2-chlorophenyl)-1-methyl-1H-1,2,4-triazol-5-yl)pyridin-2-yl)(ethoxyimino)methyl)acetamide (62 mg, 0.11 mmol).
Yield: 50%
Physical property: ¹H-NMR (CDCl₃): δ 8.79 (d, 1H), 8.34 (d, 1H), 7.93-7.90 (m, 1H), 7.53-7.50 (m, 1H), 7.38-7.35 (m, 2H), 4.44 (q, 2H), 3.85 (s, 3H), 3.05 (s, 3H), 2.32 (s, 9H), 2.16 (s, 3H), 1.40 (t, 3H)

### Production Example 10

Production of (Z)-N'-ethoxy-6-(4-methyl-1-(2-nitrophenyl)-5-oxo-4,5-dihydro-1H-1,2,4-triazol-3-yl)-5-(N-methylsulfamoyl)picolinimidamide (Compound No. 5-19)

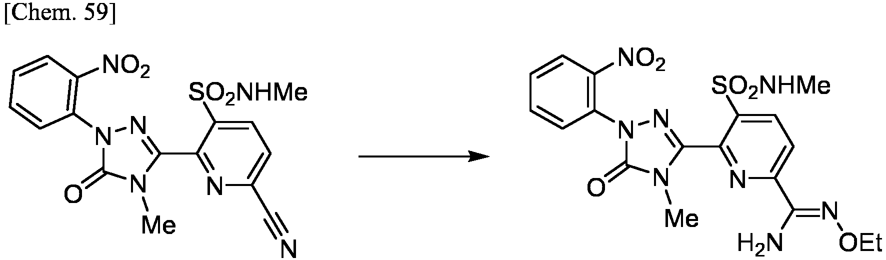

To a solution of 6-cyano-N-methyl-2-(4-methyl-1-(2-nitrophenyl)-5-oxo-4,5-dihydro-1H-1,2,4-triazol-3-yl)pyridine-3-sulfonamide (1.0 g, 2.4 mmol) in methanol (10 mL), a methanol solution of sodium methoxide (28 wt%, 0.55 mL, 2.4 mmol) was added, and the mixture was stirred at room temperature for 30 minutes. After the disappearance of the starting compound was confirmed, O-ethylhydroxylamine hydrochloride (0.36 g, 3.7 mmol) was added to the reaction mixture, and the mixture was stirred at room temperature for 30 minutes. A saturated aqueous ammonium chloride solution was added to the reaction mixture, and ethyl acetate extraction was performed. The organic layer was concentrated in vacuo, and the residue was purified by silica gel column chromatography to give (Z)-N'-ethoxy-6-(4-methyl-1-(2-nitrophenyl)-5-oxo-4,5-dihydro-1H-1,2,4-triazol-3-yl)-5-(N-methylsulfamoyl)picolinimidamide (0.92 g, 1.9 mmol).
Yield: 79%
Physical property: ¹H-NMR (CDCl₃): δ 8.43 (d, 1H), 8.29 (d, 1H), 7.96-7.90 (m, 2H), 7.78-7.72 (m, 1H), 7.56-7.50 (m, 1H), 5.84 (q, 1H), 5.45 (s, 2H), 4.24 (q, 2H), 3.42 (s, 3H), 2.71 (d, 3H), 1.37 (t, 3H)

### Production Example 11

Production of (Z)-N'-ethoxy-5-(N-methylsulfamoyl)-6-(2-phenylthiazol-4-yl)picolinimidamide (Compound No. 7-18) and (Z)-6-(5-bromo-2-phenylthiazol-4-yl)-N'-ethoxy-5-(N-methylsulfamoyl)picolinimidamide (Compound No. 7-19)

### Production Example 11-1

Production of (Z)-6-acetyl-N'-ethoxy-5-(N-methylsulfamoyl)picolinimidamide

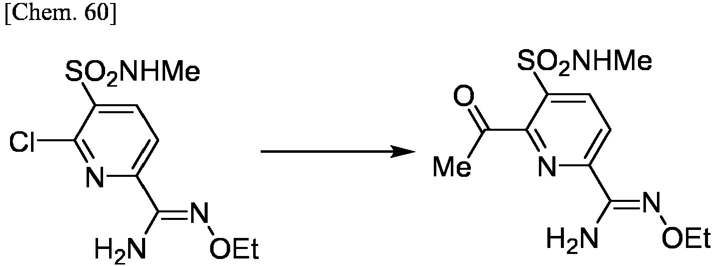

To a solution of (Z)-6-chloro-N'-ethoxy-5-(N-methylsulfamoyl)picolinimidamide (1.0 g, 3.4 mmol) in dimethoxyethane (20 mL), tetrakis(triphenylphosphine)palladium(0) (0.40 g, 0.34 mmol) and tributyl(1-ethoxyvinyl)tin (1.7 mL, 5.1 mmol) were added, and the mixture was stirred under an argon atmosphere at 110°C for 5 hours. After the completion of the reaction, tetrahydrofuran (10 mL) and 1 N hydrochloric acid (10 mL) were added to the reaction mixture, and the mixture was stirred overnight at room temperature. Water and ethyl acetate were added for extraction. The organic layer was dried over anhydrous sodium sulfate and then concentrated in vacuo. The residue was purified by silica gel column chromatography to give (Z)-6-acetyl-N'-ethoxy-5-(N-methylsulfamoyl)picolinimidamide (0.55 g, 1.8 mmol).
Yield: 54%
Physical property: Melting point: 100 to 101°C

### Production Example 11-2

Production of (Z)-6-(2-bromoacetyl)-N'-ethoxy-5-(N-methylsulfamoyl)picolinimidamide

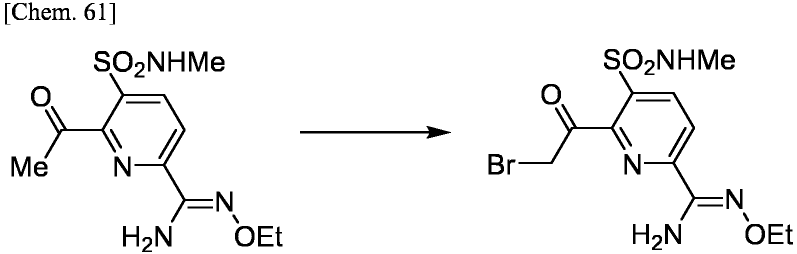

To a solution of (Z)-6-acetyl-N'-ethoxy-5-(N-methylsulfamoyl)picolinimidamide (0.50 g, 1.7 mmol) in tetrahydrofuran (10 mL), phenyltrimethylammonium tribromide (0.62 g, 1.7 mmol) was added, and the mixture was stirred at room temperature for 2 hours. After the completion of the reaction, water and ethyl acetate were added to the reaction mixture for extraction. The organic layer was dried over anhydrous sodium sulfate and then concentrated in vacuo to give (Z)-6-(2-bromoacetyl)-N'-ethoxy-5-(N-methylsulfamoyl)picolinimidamide (0.71 g, 1.8 mmol).
Yield: 100%
Physical property: ¹H-NMR (CDCl₃): δ 8.31-8.27 (m, 1H), 8.11-8.09 (m, 1H), 5.51 (brs, 2H), 4.23 (q, 2H), 3.00 (s, 2H), 1.36 (t, 3H)

### Production Example 11-3

Production of (Z)-N'-ethoxy-5-(N-methylsulfamoyl)-6-(2-phenylthiazol-4-yl)picolinimidamide (Compound No. 7-18)

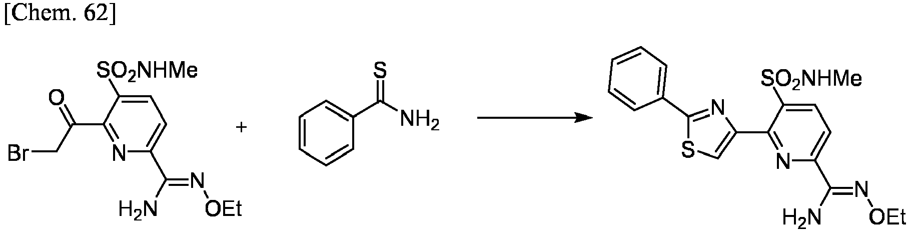

To a solution of (Z)-6-(2-bromoacetyl)-N'-ethoxy-5-(N-methylsulfamoyl)picolinimidamide (0.71 g, 1.8 mmol) in ethanol (10 mL), thiobenzamide (0.21 g, 1.5 mmol) was added, and the mixture was stirred and heated under reflux for 2 hours. After the completion of the reaction, the reaction mixture was concentrated in vacuo. A saturated aqueous sodium hydrogen carbonate solution and ethyl acetate were added to the residue for extraction. The organic layer was dried over anhydrous sodium sulfate and then concentrated in vacuo. The residue was purified by silica gel column chromatography to give (Z)-N'-ethoxy-5-(N-methylsulfamoyl)-6-(2-phenylthiazol-4-yl)picolinimidamide (0.28 g, 0.68 mmol).
Yield: 36%
Physical property: Melting point: 189 to 190°C

### Production Example 11-4

Production of (Z)-6-(5-bromo-2-phenylthiazol-4-yl)-N'-ethoxy-5-(N-methylsulfamoyl)picolinimidamide (Compound No. 7-19)

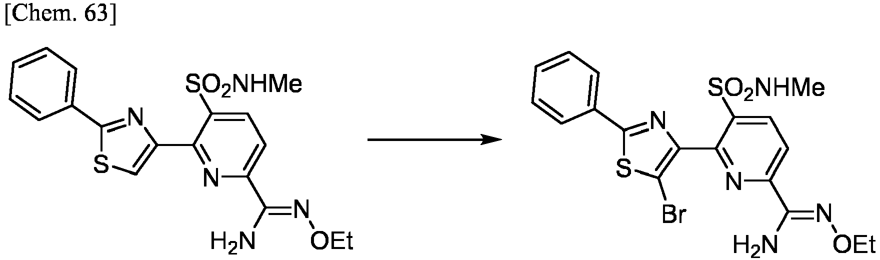

To a solution of (Z)-N'-ethoxy-5-(N-methylsulfamoyl)-6-(2-phenylthiazol-4-yl)picolinimidamide (0.24 g, 0.58 mmol) in chloroform (5.0 mL), N-bromosuccinimide (0.13 g, 0.70 mmol) was added, and the mixture was stirred at 50°C for 7 hours and then at room temperature overnight. After the completion of the reaction, water was added to the reaction mixture, and chloroform extraction was performed. The organic layer was dried over anhydrous sodium sulfate and then concentrated in vacuo. The residue was purified by silica gel column chromatography to give (Z)-6-(5-bromo-2-phenylthiazol-4-yl)-N'-ethoxy-5-(N-methylsulfamoyl)picolinimidamide (0.23 g, 0.46 mmol).
Yield: 79%
Physical property: Melting point: 159 to 160°C

### Production Example 12

Production of (Z)-N'-ethoxy-5-(methylsulfonyl)-6-(4-phenylthiazol-2-yl)picolinimidamide (Compound No. 8-17) and (Z)-N'-ethoxy-5-(N-methylsulfamoyl)-6-(4-phenylthiazol-2-yl)picolinimidamide (Compound No. 8-2)

### Production Example 12-1

Production of (Z)-6-(N'-ethoxycarbamimidoyl)-3-(methylsulfonyl)picolinamide

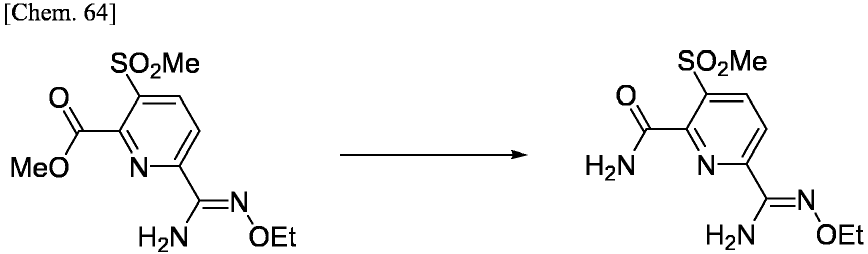

(Z)-Methyl 6-(N'-ethoxycarbamimidoyl)-3-(methylsulfonyl)picolinate (1.4 g, 4.5 mmol) was added to a methanol solution of ammonia (2 mol/L, 10 mL), and the mixture was stirred at room temperature overnight. After the completion of the reaction, the reaction mixture was concentrated in vacuo. Water and ethyl acetate were added to the residue for extraction. The organic layer was dried over anhydrous sodium sulfate and then concentrated in vacuo. The residue was purified by silica gel column chromatography to give (Z)-6-(N'-ethoxycarbamimidoyl)-3-(methylsulfonyl)picolinamide (0.32 g, 1.1 mmol).
Yield: 24%
Physical property: ¹H-NMR (CDCl₃): δ 8.49 (d, 1H), 8.28 (d, 1H), 6.79 (brs, 1H), 5.81 (brs, 1H), 5.43 (brs, 2H), 4.23 (q, 2H), 3.56 (s, 3H), 1.36 (t, 3H)

### Production Example 12-2

Production of (Z)-6-(N'-ethoxycarbamimidoyl)-3-(methylsulfonyl)pyridine-2-carbothioamide

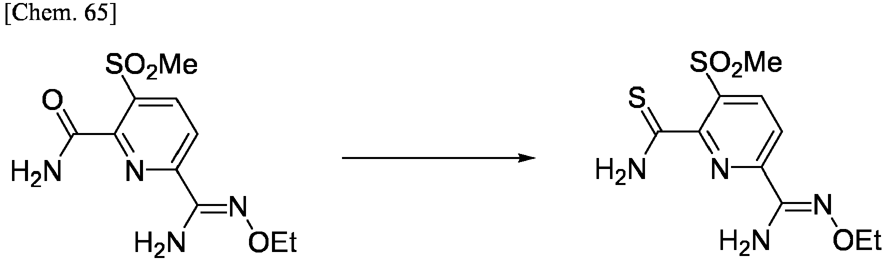

To a solution of (Z)-6-(N'-ethoxycarbamimidoyl)-3-(methylsulfonyl)picolinamide (0.32 g, 1.1 mmol) in toluene (5.0 mL), Lawesson's reagent (0.45 g, 1.1 mmol) was added, and the mixture was stirred at 90°C for 3 hours. After the completion of the reaction, the reaction mixture was concentrated in vacuo. The residue was purified by silica gel column chromatography to give (Z)-6-(N'-ethoxycarbamimidoyl)-3-(methylsulfonyl)pyridine-2-carbothioamide (0.27 g, 0.90 mmol).
Yield: 82%
Physical property: Melting point: 243 to 245°C

### Production Example 12-3

Production of (Z)-N'-ethoxy-5-(methylsulfonyl)-6-(4-phenylthiazol-2-yl)picolinimidamide (Compound No. 8-17)

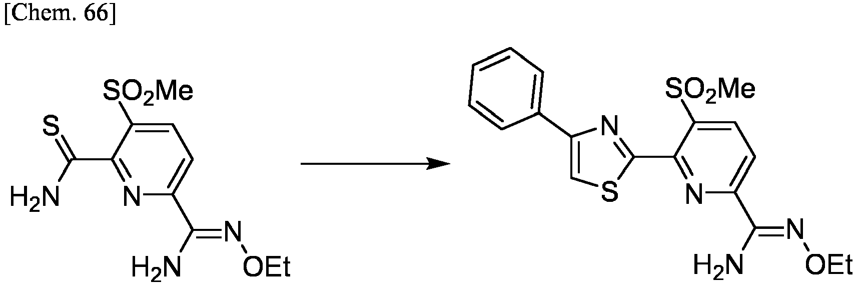

To a solution of (Z)-6-(N'-ethoxycarbamimidoyl)-3-(methylsulfonyl)pyridine-2-carbothioamide (0.23 g, 0.77 mmol) in ethanol (2.5 mL), phenacyl bromide (0.17 g, 0.84 mmol) was added, and the mixture was stirred and heated under reflux for 2 hours. After the completion of the reaction, the reaction mixture was concentrated in vacuo. A saturated aqueous sodium hydrogen carbonate solution and ethyl acetate were added to the residue for extraction. The organic layer was dried over anhydrous sodium sulfate and then concentrated in vacuo. The residue was purified by silica gel column chromatography to give (Z)-N'-ethoxy-5-(methylsulfonyl)-6-(4-phenylthiazol-2-yl)picolinimidamide (0.27 g, 0.67 mmol).
Yield: 87%
Physical property: Melting point: 177 to 179°C

### Production Example 12-4

Production of (Z)-5-((4-(tert-butyl)benzyl)thio)-N'-ethoxy-6-(4-phenylthiazol-2-yl)picolinimidamide

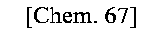

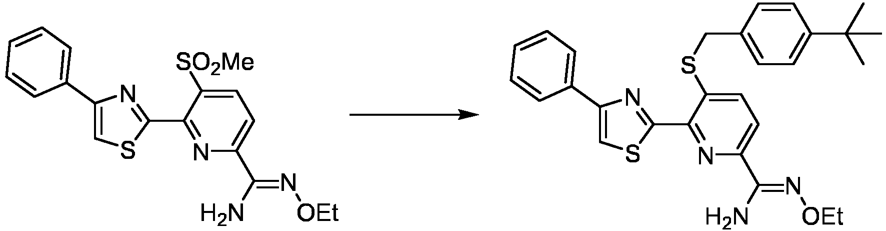

To a solution of (Z)-N'-ethoxy-5-(methylsulfonyl)-6-(4-phenylthiazol-2-yl)picolinimidamide (0.23 g, 0.58 mmol) in N,N-dimethylacetamide (3.0 mL), cesium carbonate (0.38 g, 1.2 mmol) and (4-(tert-butyl)phenyl)methanethiol (0.16 mL, 0.87 mmol) were added, and the mixture was stirred at 60°C for 1 hour. After the completion of the reaction, water and ethyl acetate were added to the reaction mixture for extraction. The organic layer was dried over anhydrous sodium sulfate and then concentrated in vacuo. The residue was purified by silica gel column chromatography to give (Z)-5-((4-(tert-butyl)benzyl)thio)-N'-ethoxy-6-(4-phenylthiazol-2-yl)picolinimidamide (0.23 g, 0.46 mmol).
Yield: 80%
Physical property: Melting point: 204 to 206°C

### Production Example 12-5

Production of (Z)-N'-ethoxy-5-(N-methylsulfamoyl)-6-(4-phenylthiazol-2-yl)picolinimidamide (Compound No. 8-2)

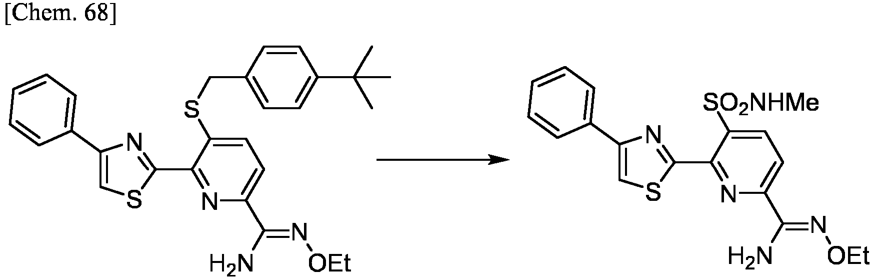

To a solution of (Z)-5-((4-(tert-butyl)benzyl)thio)-N'-ethoxy-6-(4-phenylthiazol-2-yl)picolinimidamide (0.11 g, 0.22 mmol) in chloroform (5.0 mL), acetic acid (0.038 mL, 0.66 mmol) and water (0.024 mL, 1.3 mmol) were added, and 1,3-dichloro-5,5-dimethylhydantoin (0.13 g, 0.66 mmol) was added with cooling in an ice bath. After stirring for 5 minutes, the reaction mixture was added dropwise to a methanol solution of methylamine (9.8 mol/L, 5.0 mL), and the mixture was stirred for 5 minutes. After the completion of the reaction, water was added to the reaction mixture, and chloroform extraction was performed. The organic layer was dried over anhydrous sodium sulfate and then concentrated in vacuo. The residue was purified by silica gel column chromatography to give (Z)-N'-ethoxy-5-(N-methylsulfamoyl)-6-(4-phenylthiazol-2-yl)picolinimidamide (44 mg, 0.11 mmol).
Yield: 48%
Physical property: Melting point: 157 to 158°C

### Production Example 13

Production of (Z)-N'-ethoxy-6-(4-methyl-1-phenyl-1H-pyrazol-3-yl)-5-(methylsulfonyl)picolinimidamide (Compound No. 10-16) and (Z)-N'-ethoxy-6-(4-methyl-1-phenyl-1H-pyrazol-3-yl)-5-(N-methylsulfamoyl)picolinimidamide (Compound No. 10-2)

### Production Example 13-1

Production of (Z)-N'-ethoxy-6-(4-methyl-1-phenyl-1H-pyrazol-3-yl)-5-(methylsulfonyl)picolinimidamide (Compound No. 10-16)

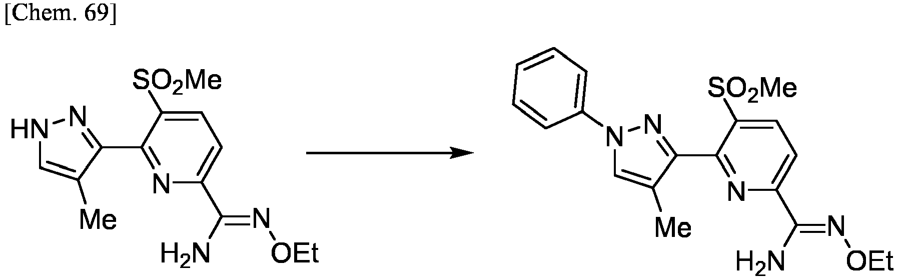

To a solution of (Z)-N'-ethoxy-6-(4-methyl-1H-pyrazol-3-yl)-5-(methylsulfonyl)picolinimidamide (0.58 g, 1.8 mmol) in N,N-dimethylacetamide (10 mL), iodobenzene (0.20 mL, 1.8 mmol), cesium carbonate (1.2 g, 3.7 mmol), and copper(II) acetate monohydrate (38 mg, 0.19 mmol) were added, and the mixture was stirred at 140°C for 3 hours. After the completion of the reaction, a saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give (Z)-N'-ethoxy-6-(4-methyl-1-phenyl-1H-pyrazol-3-yl)-5-(methylsulfonyl)picolinimidamide (0.15 g, 0.37 mmol).
Yield: 21%
Physical property: Melting point: 150 to 151°C

### Production Example 13-2

Production of (Z)-5-((4-(tert-butyl)benzyl)thio)-N'-ethoxy-6-(4-methyl-1-phenyl-1H-pyrazol-3 -yl)picolinimidamide

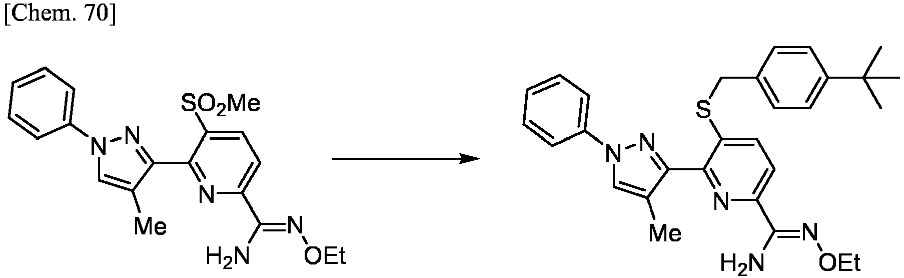

To a solution of (Z)-N'-ethoxy-6-(4-methyl-1-phenyl-1H-pyrazol-3-yl)-5-(methylsulfonyl)picolinimidamide (0.15 g, 0.42 mmol) in N,N-dimethylacetamide (2.0 mL), cesium carbonate (0.41 g, 1.3 mmol) and (4-(tert-butyl)phenyl)methanethiol (0.15 mL, 0.82 mmol) were added at room temperature, and the mixture was stirred at room temperature for 2 hours. After the completion of the reaction, a saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give (Z)-5-((4-(tert-butyl)benzyl)thio)-N'-ethoxy-6-(4-methyl-1-phenyl-1H-pyrazol-3-yl)picolinimidamide (0.10 g, 0.21 mmol).
Yield: 50%

### Production Example 13-3

Production of (Z)-N'-ethoxy-6-(4-methyl-1-phenyl-1H-pyrazol-3-yl)-5-(N-methylsulfamoyl)picolinimidamide (Compound No. 10-2)

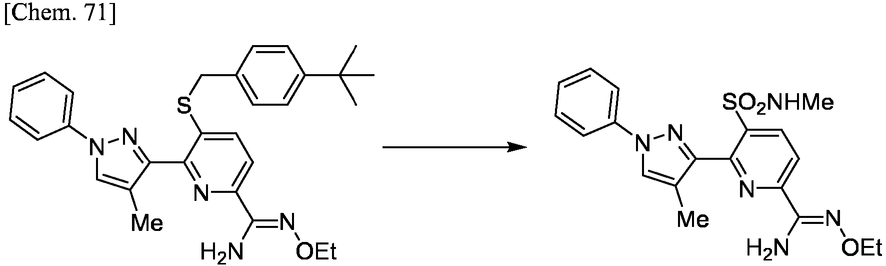

To a solution of (Z)-5-((4-(tert-butyl)benzyl)thio)-N'-ethoxy-6-(4-methyl-1-phenyl-1H-pyrazol-3-yl)picolinimidamide (0.10 g, 0.21 mmol) in chloroform (2.0 mL), 1,3-dichloro-5,5-dimethylhydantoin (0.12 g, 0.62 mmol), acetic acid (40 µL, 0.63 mmol), and water (25 µL, 1.2 mmol) were added at 0°C, and the mixture was stirred at the same temperature for 10 minutes. After the completion of the reaction, methylamine (40% solution in methanol, 0.21 mL, 2.1 mmol) was added to the reaction mixture, and the mixture was further stirred for 10 minutes. After the completion of the reaction, water was added to the reaction mixture, and chloroform extraction was performed. The organic layer was dried over anhydrous magnesium sulfate and then concentrated in vacuo. The residue was purified by silica gel column chromatography to give (Z)-N'-ethoxy-6-(4-methyl-1-phenyl-1H-pyrazol-3-yl)-5-(N-methylsulfamoyl)picolinimidamide (67 mg, 0.16 mmol).
Yield: 76%
Physical property: Melting point: 130 to 131°C

### Production Example 14

Production of (Z)-N'-ethoxy-6-(4-methyl-5-phenyl-2H-1,2,3-triazol-2-yl)-5-(N-methylsulfamoyl)picolinimidamide (Compound No. 20-17)

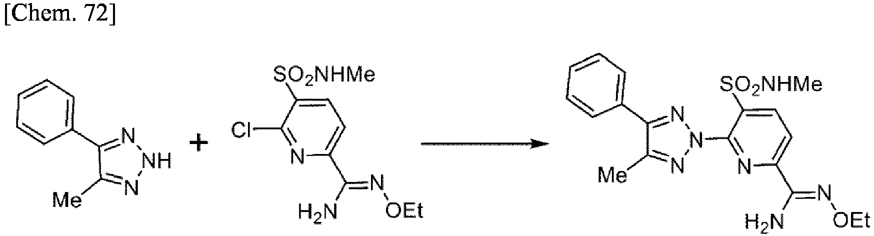

To a solution of (Z)-6-chloro-N'-ethoxy-5-(N-methylsulfamoyl)picolinimidamide (0.12 g, 0.41 mmol) in N,N-dimethylacetamide (2.0 mL), potassium carbonate (87 mg, 0.87 mmol) and 4-methyl-5-phenyl-2H-1,2,3-triazole (66 mg, 0.41 mmol) were added, and the mixture was stirred at 130°C for 17 hours. After the completion of the reaction, water and ethyl acetate were added to the reaction mixture for extraction. The extract was concentrated in vacuo, and the residue was purified by silica gel column chromatography to give (Z)-N'-ethoxy-6-(4-methyl-5-phenyl-2H-1,2,3-triazol-2-yl)-5-(N-methylsulfamoyl)picolinimidamide (28 mg, 0.067 mmol).
Yield: 16%
Physical property: Melting point: 72 to 77°C

### Production Example 15

Production of (Z)-N'-ethoxy-6-(5-methyl-2-(2-nitrophenyl)-2H-1,2,3-triazol-4-yl)-5-(methylsulfonyl)picolinimidamide (0.21 g, 0.47 mmol) (Compound No. 11-95)

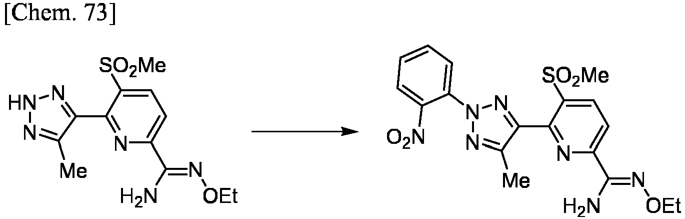

To a solution of (Z)-N'-ethoxy-6-(5-methyl-2H-1,2,3-triazol-4-yl)-5-(methylsulfonyl)picolinimidamide (0.18 g, 0.56 mmol) in N,N-dimethylacetamide (3 mL), potassium carbonate (0.17 g, 0.85 mmol) and 2-fluoronitrobenzene (0.059 mL, 0.56 mmol) were added, and the mixture was stirred at 130°C for 7 hours. The reaction mixture was cooled to room temperature, and ethyl acetate and water were added for extraction. The organic layer was concentrated in vacuo, and the residue was purified by silica gel chromatography to give (Z)-N'-ethoxy-6-(5-methyl-2-(2-nitrophenyl)-2H-1,2,3-triazol-4-yl)-5-(methylsulfonyl)picolinimidamide (0.21 g, 0.47 mmol).
Yield: 83%
Physical property: Melting point: 179 to 184°C

### Reference Example 1

Production of ethyl 6-cyano-2-(4-methyl-5-oxo-1-phenyl-4,5-dihydro-1H-1,2,4-triazol-3-yl)nicotinate (starting compound for Production Example 1-1)

### Reference Production Example 1-1

Production of (E)-3,6-dichloro-2-((2-phenylhydrazono)methyl)pyridine

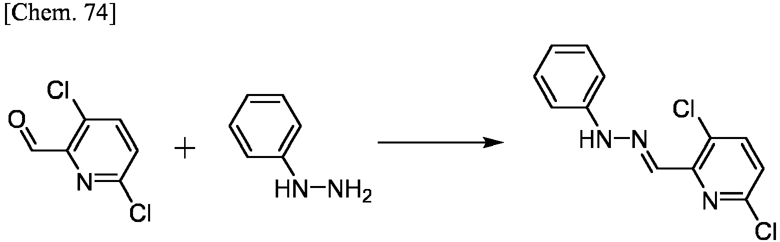

Phenylhydrazine (1.7 mL, 31 mmol) was added to an ethanol solution (60 mL) of 3,6-dichloropicolinaldehyde (3.0 g, 17 mmol), and the mixture was stirred for 3 hours. The resulting solid was collected by filtration, washed with ethanol, and dried to give the desired product (4.3 g, 16 mmol).
Yield: 96%

### Reference Production Example 1-2

Production of 3-(3,6-dichloropyridin-2-yl)-4-methyl-1-phenyl-1H-1,2,4-triazol-5(4H)-one

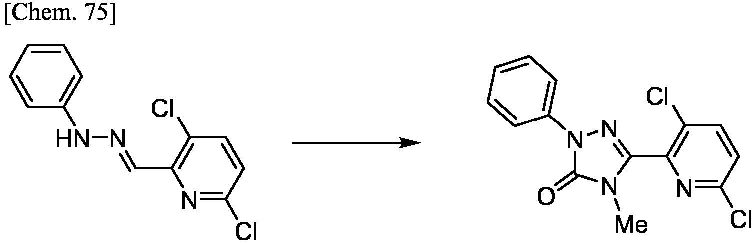

Phosphorus pentachloride (4.1 g, 20 mmol) was added to a solution of (E)-3,6-dichloro-2-((2-phenylhydrazono)methyl)pyridine (3.5 g, 13 mmol) in chloroform (65 mL) at room temperature, and the mixture was stirred at 80°C for 3 hours. Water was added to the reaction mixture, and chloroform extraction was performed. The organic layer was dried over anhydrous magnesium sulfate and then concentrated in vacuo. The residue was dissolved in tetrahydrofuran (65 mL), a 40% methylamine solution (6.6 mL, 65 mmol) was added, and the mixture was stirred for 1 hour. Water was further added to the reaction mixture, and ethyl acetate extraction was performed. The organic layer was dried over anhydrous magnesium sulfate and then concentrated in vacuo. The residue was dissolved in chloroform (130 mL), pyridine (2.6 mL, 33 mol) and triphosgene (5.8 g, 20 mmol) were added, and the mixture was stirred for 1 hour. Water was added to the reaction mixture, and chloroform extraction was performed. The organic layer was dried over anhydrous magnesium sulfate and then concentrated in vacuo. The residue was purified by silica gel column chromatography to give 3-(3,6-dichloropyridin-2-yl)-4-methyl-1-phenyl-1H-1,2,4-triazol-5(4H)-one (1.5 g, 4.7 mmol).
Yield: 36%

### Reference Production Example 1-3

Production of ethyl 6-carbamoyl-2-(4-methyl-5-oxo-1-phenyl-4,5-dihydro-1H-1,2,4-triazol-3-yl)nicotinate

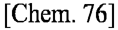

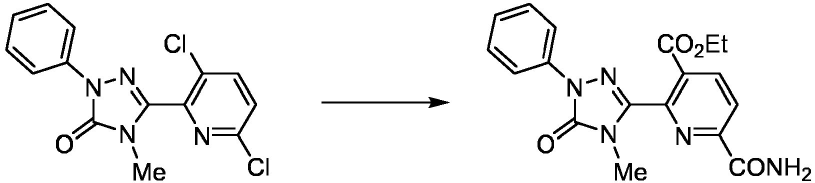

To a solution of 3-(3,6-dichloropyridin-2-yl)-4-methyl-1-phenyl-1H-1,2,4-triazol-5(4H)-one (1.5 g, 4.8 mmol) in ethanol (47 mL), 1,4-bis(diphenylphosphino)butane (tetramethylenebis(diphenylphosphine)) (72 mg, 0.0020 mmol), bis(triphenylphosphine)palladium(II) dichloride (67 mg, 0.0010 mmol), and triethylamine (2.0 mL, 1.4 mmol) were added, and the mixture was stirred under a carbon monoxide atmosphere of 4 MPa at 130°C for 3 hours. The reaction mixture was filtered, and the filtrate was concentrated in vacuo. The residue was purified by silica gel column chromatography. A 4% ammonia-ethanol solution (50 mL) was added to the resulting solid, and the mixture was stirred at room temperature overnight. Water was added to the reaction mixture, and ethyl acetate extraction was performed. The organic layer was dried over anhydrous magnesium sulfate and then concentrated in vacuo. The residue was purified by silica gel column chromatography to give ethyl 6-carbamoyl-2-(4-methyl-5-oxo-1-phenyl-4,5-dihydro-1H-1,2,4-triazol-3-yl)nicotinate (0.78 g, 2.1 mmol).
Yield: 45%
Physical property: Melting point: 137 to 138°C

### Reference Production Example 1-4

Production of ethyl 6-cyano-2-(4-methyl-5-oxo-1-phenyl-4,5-dihydro-1H-1,2,4-triazol-3 -yl)nicotinate

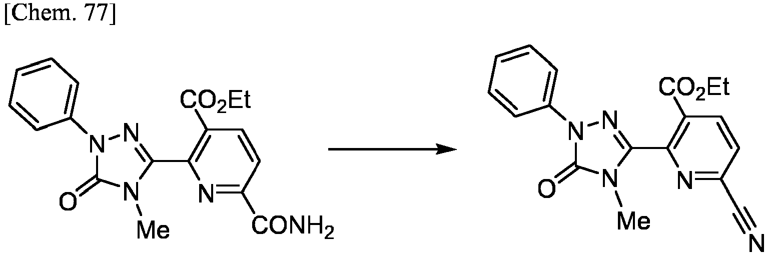

To a solution of ethyl 6-carbamoyl-2-(4-methyl-5-oxo-1-phenyl-4,5-dihydro-1H-1,2,4-triazol-3-yl)nicotinate (0.78 g, 2.1 mmol) in N,N-dimethylformamide (21 mL), phosphoryl chloride (0.79 mL, 8.5 mmol) was added at room temperature, and the mixture was stirred for 1 hour. A saturated aqueous sodium hydrogen carbonate solution was added to the reaction mixture, and ethyl acetate extraction was performed. The organic layer was dried over anhydrous magnesium sulfate and then concentrated in vacuo to give ethyl 6-cyano-2-(4-methyl-5-oxo-1-phenyl-4,5-dihydro-1H-1,2,4-triazol-3-yl)nicotinate (0.73 g, 2.1 mmol).
Yield: 100%
Physical property: Melting point: 138 to 139°C

### Reference Example 2

Production of ethyl 6-cyano-2-(5-methyl-2-phenylthiazol-4-yl)nicotinate (starting compound for Production Example 2)

### Reference Production Example 2-1

Production of 3,6-dichloro-N-methoxy-N-methylpicolinamide

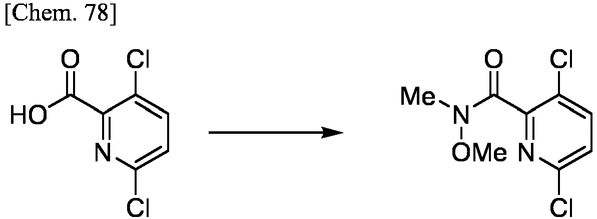

N,N-dimethylformamide (0.4 mL, 5.3 mmol) and oxalyl chloride (5.0 mL, 57 mmol) were added to a solution of 3,6-dichloropicolinic acid (10 g, 52 mmol) in tetrahydrofuran (0.10 L) at room temperature, and the mixture was stirred at room temperature for 0.5 hour. The reaction mixture was concentrated in vacuo, and chloroform (0.10 L), triethylamine (28 mL, 0.21 mol), and N-methoxy-N-methylamine hydrochloride (6.1 g, 62 mmol) were added to the residue. The mixture was stirred at room temperature overnight. After the completion of the reaction, water was added to the reaction mixture, and ethyl acetate extraction was performed. The organic layer was dried over anhydrous magnesium sulfate and then concentrated in vacuo. The residue was purified by silica gel column chromatography to give 3,6-dichloro-N-methoxy-N-methylpicolinamide (12 g, 52 mmol).
Yield: 100%
Physical property: ¹H-NMR (CDCl₃): δ 7.70 (d, 1H), 7.33 (d, 1H), 3.61 (s, 3H), 3.39 (s, 3H)

### Reference Production Example 2-2

Production of 1-(3,6-dichloropyridin-2-yl)propan-1-one

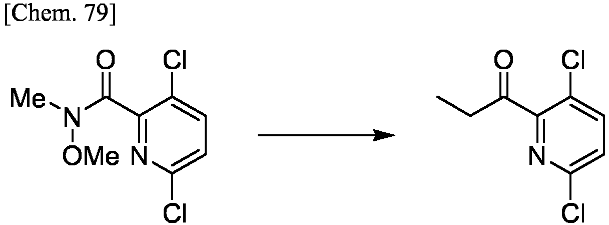

To a solution of 3,6-dichloro-N-methoxy-N-methylpicolinamide (5.0 g, 21 mmol) in tetrahydrofuran (0.10 L), a diethyl ether solution of ethylmagnesium bromide (27 mL, 81 mmol) was added at 0°C, and the mixture was stirred for 2 hours. After the completion of the reaction, a saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give 1-(3,6-dichloropyridin-2-yl)propan-1-one (1.8 g, 8.7 mmol).
Yield: 41%
Physical property: ¹H-NMR (CDCl₃): δ 7.74 (d, 1H), 7.39 (d, 1H), 3.10 (q, 2H), 1.20 (t, 3H)

### Reference Production Example 2-3

Production of 2-bromo-1-(3,6-dichloropyridin-2-yl)propan-1-one

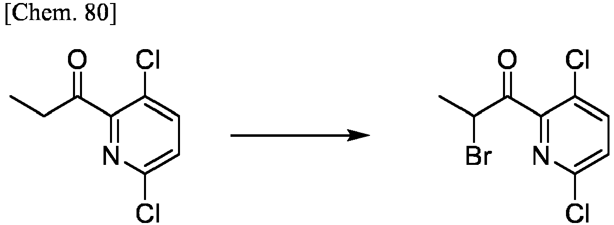

Bromine (0.30 mL, 5.6 mmol) was added to a solution of 1-(3,6-dichloropyridin-2-yl)propan-1-one (1.1 g, 5.2 mmol) in acetic acid (17 mL), and the mixture was stirred at 120°C for 2 hours. After the completion of the reaction, the reaction mixture was concentrated in vacuo. Water was added to the residue, and ethyl acetate extraction was performed. The organic layer was dried over anhydrous magnesium sulfate and then concentrated in vacuo. The residue was purified by silica gel column chromatography to give 2-bromo-1-(3,6-dichloropyridin-2-yl)propan-1-one (1.2 g, 4.3 mmol).
Yield: 85%

### Reference Production Example 2-4

Production of 4-(3,6-dichloropyridin-2-yl)-5-methyl-2-phenylthiazole

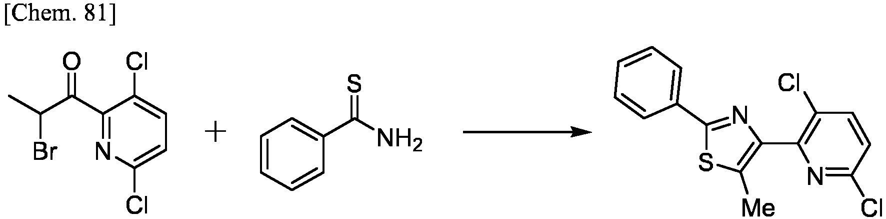

Benzothioamide (1.1 g, 3.6 mmol) was added to a solution of 2-bromo-1-(3,6-dichloropyridin-2-yl)propan-1-one (0.51 g, 3.6 mmol) in ethanol (7.0 mL), and the mixture was stirred at 80°C for 2.5 hours. After the completion of the reaction, the reaction mixture was concentrated in vacuo. A saturated aqueous sodium hydrogen carbonate solution was added to the residue, and ethyl acetate extraction was performed. The organic layer was dried over anhydrous magnesium sulfate and then concentrated in vacuo. The residue was purified by silica gel column chromatography to give 4-(3,6-dichloropyridin-2-yl)-5-methyl-2-phenylthiazole (0.95 g, 3.0 mmol).
Yield: 75%

### Reference Production Example 2-5

Production of diethyl 6-(5-methyl-2-phenylthiazol-4-yl)pyridine-2,5-dicarboxylate

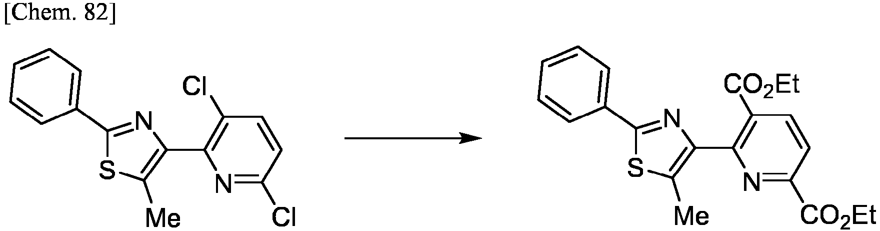

Into an autoclave reaction vessel, 4-(3,6-dichloropyridin-2-yl)-5-methyl-2-phenylthiazole (0.95 g, 3.0 mmol), dichlorobis(triphenylphosphine)palladium (42 mg, 0.060 mmol), bis(diphenylphosphino)butane (51 mg, 0.12 mmol), triethylamine (1.0 mL, 7.4 mmol), and ethanol (10 mL) were placed, and the mixture was stirred under an atmosphere of carbon monoxide (4.0 MPa) at 130°C for 3 hours. The reaction mixture was concentrated in vacuo, a saturated aqueous sodium hydrogen carbonate solution was added to the residue, and ethyl acetate extraction was performed. The organic layer was dried over anhydrous magnesium sulfate and then concentrated in vacuo to give diethyl 6-(5-methyl-2-phenylthiazol-4-yl)pyridine-2,5-dicarboxylate (0.82 g, 2.1 mmol).
Yield: 70%
Physical property: Melting point: 69 to 70°C

### Reference Production Example 2-6

Production of ethyl 6-carbamoyl-2-(5-methyl-2-phenylthiazol-4-yl)nicotinate

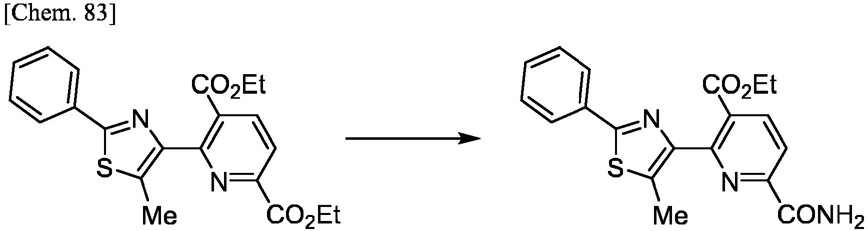

To a solution of diethyl 6-(5-methyl-2-phenylthiazol-4-yl)pyridine-2,5-dicarboxylate (0.80 g, 2.0 mmol) in ethanol (10 mL), ammonia (28% solution in water, 10 mL) was added at room temperature, and the mixture was stirred at room temperature overnight. The reaction mixture was filtered, and the residue was washed with ethanol to give ethyl 6-carbamoyl-2-(5-methyl-2-phenylthiazol-4-yl)nicotinate (0.74 g, 2.0 mmol).
Yield: 100% (in 3 steps)
Physical property: Melting point: 147 to 148°C

### Reference Production Example 2-7

Production of ethyl 6-cyano-2-(5-methyl-2-phenylthiazol-4-yl)nicotinate

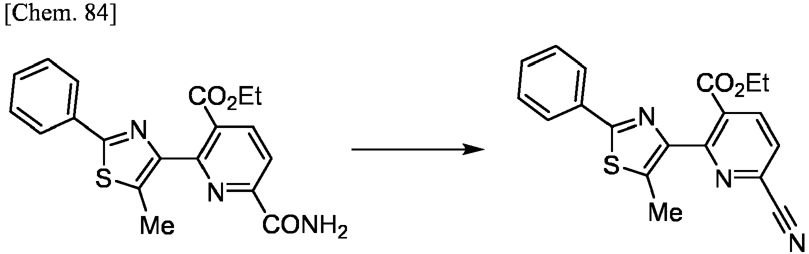

To a solution of ethyl 6-carbamoyl-2-(5-methyl-2-phenylthiazol-4-yl)nicotinate (0.50 g, 1.4 mmol) in N,N-dimethylformamide (6.8 mL), phosphoryl chloride (0.5 mL, 5.4 mmol) was added at 0°C, and the mixture was stirred at room temperature for 30 minutes. After the completion of the reaction, a saturated aqueous sodium hydrogen carbonate solution was added to the reaction mixture at 0°C, and ethyl acetate extraction was performed. The organic layer was dried over anhydrous magnesium sulfate and then concentrated in vacuo to give ethyl 6-cyano-2-(5-methyl-2-phenylthiazol-4-yl)nicotinate (0.48 g, 1.4 mmol).
Yield: 100%

### Reference Example 3

Production of 6-cyano-N-methyl-2-(1-methyl-3-phenyl-1H-1,2,4-triazol-5-yl)pyridine-3-sulfonamide (starting compound for Production Example 3)

### Reference Production Example 3-1

Production of methyl 3-((4-(tert-butyl)benzyl)thio)-6-chloropicolinate

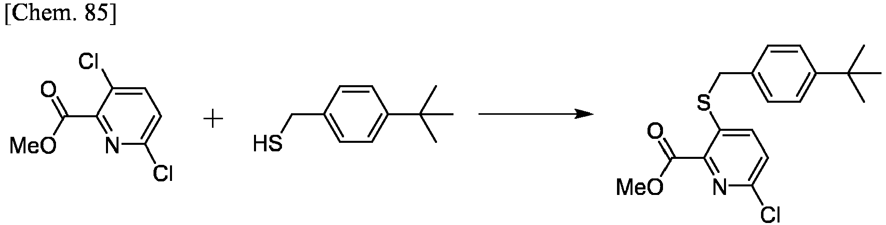

Sodium hydride (60 wt%, 4.70 g, 0.12 mol) was added to a solution of methyl 3,6-dichloropicolinate (20 g, 97 mmol) in tetrahydrofuran (0.50 L) with cooling in an ice bath, and the mixture was stirred for 15 minutes. 4-(Tert-butyl)phenyl)methanethiol (18 mL, 0.10 mol) was added dropwise, and the mixture was stirred at room temperature for 3 hours. A saturated aqueous ammonium chloride solution was added to the reaction mixture with cooling in an ice bath, and ethyl acetate extraction was performed. The organic layer was dried over anhydrous sodium sulfate and then concentrated. The residue was washed with hexane to give methyl 3-((4-(tert-butyl)benzyl)thio)-6-chloropicolinate (30 g, 86 mmol).
Yield: 88%
Physical property: ¹H-NMR (CDCl₃): δ 7.66-7.63 (m, 1H), 7.37-7.28 (m, 5H), 4.12 (s, 2H), 3.98 (s, 3H), 1.31 (s, 9H)

### Reference Production Example 3-2

Production of 3-((4-(tert-butyl)benzyl)thio)-6-chloropicolinic acid

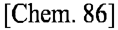

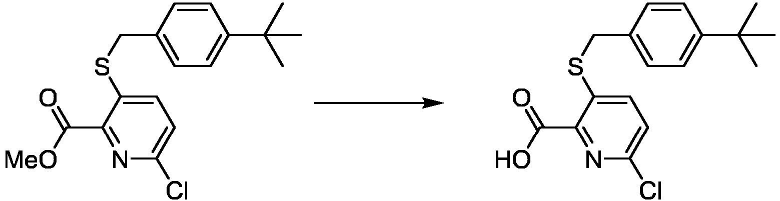

To a solution of methyl 3-((4-(tert-butyl)benzyl)thio)-6-chloropicolinate (30 g, 86 mmol) in methanol (200 mL) and tetrahydrofuran (50 mL), lithium hydroxide (4.3 g, 0.10 mol) was added, and the mixture was stirred at room temperature for 6 hours. The reaction mixture was concentrated in vacuo, and 2 M hydrochloric acid was added to the residue. The resulting solid was collected by filtration and washed with hexane to give 3-((4-(tert-butyl)benzyl)thio)-6-chloropicolinic acid (30 g, 85 mmol).
Yield: 100%

### Reference Production Example 3-3

Production of 3-((4-(tert-butyl)benzyl)thio)-6-chloro-2-(1-methyl-3-phenyl-1H-1,2,4-triazol-5-yl)pyridine

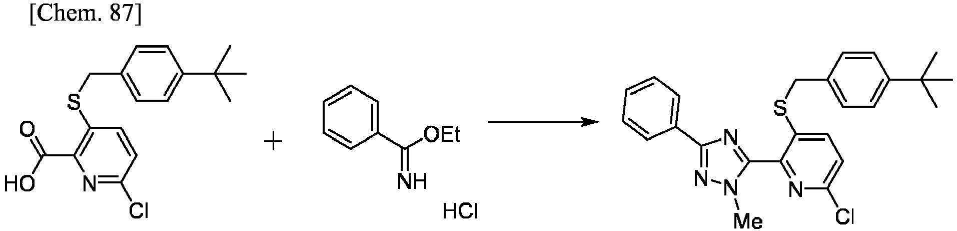

To a solution of 3-((4-(tert-butyl)benzyl)thio)-6-chloropicolinic acid (10 g, 30 mmol) and ethyl benzimidate hydrochloride (5.6 g, 30 mmol) in N,N-dimethylformamide (60 mL), N,N-diisopropylethylamine (15 mL, 90 mmol) and 1-[bis(dimethylamino)methylene]-lH-benzotriazolium 3-oxide hexafluorophosphate (17 g, 45 mmol) were added, and the mixture was stirred at room temperature overnight. Water was added to the reaction mixture, and chloroform extraction was performed. The organic layer was washed with saturated brine, dried over anhydrous sodium sulfate, and concentrated in vacuo. The residue was dissolved in ethanol (60 mL), methylhydrazine (2.4 mL, 45 mmol) was added, and the mixture was heated under reflux for 2 hours. The reaction mixture was concentrated in vacuo, and the residue was purified by silica gel column chromatography to give 3-((4-(tert-butyl)benzyl)thio)-6-chloro-2-(1-methyl-3-phenyl-1H-1,2,4-triazol-5-yl)pyridine (2.1 g, 4.6 mmol).
Yield: 5%

### Reference Production Example 3-4

Production of 6-chloro-N-methyl-2-(1-methyl-3-phenyl-1H-1,2,4-triazol-5-yl)pyridine-3-sulfonamide

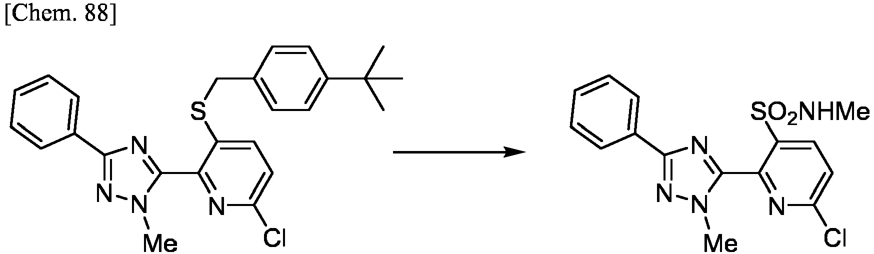

To a solution of 3-((4-(tert-butyl)benzyl)thio)-6-chloro-2-(1-methyl-3-phenyl-1H-1,2,4-triazol-5-yl)pyridine (2.1 g, 4.6 mmol) in chloroform (50 mL), water (0.50 mL, 27 mmol) and acetic acid (0.80 mL, 14 mmol) were added, and 1,3-dichloro-5,5-dimethylhydantoin (2.7 g, 14 mmol) was added with cooling in an ice bath. After the disappearance of the starting compound was confirmed, the reaction mixture was added dropwise to a methylamine-methanol solution (9.8 mol/L, 20 mL) with cooling in an ice bath, and the mixture was stirred for 30 minutes. A saturated aqueous ammonium chloride solution was further added, and ethyl acetate extraction was performed. The organic layer was concentrated, and the residue was purified by silica gel column chromatography to give 6-chloro-N-methyl-2-(1-methyl-3-phenyl-1H-1,2,4-triazol-5-yl)pyridine-3-sulfonamide (0.70 g, 1.9 mmol).
Yield: 42%
Physical property: ¹H-NMR (CDCl₃): δ 8.46 (d, 1H), 8.14 (q, 1H), 8.00-7.95 (m, 2H), 7.59 (d, 1H), 7.50-7.41 (m, 3H), 4.18 (s, 3H), 2.80 (s, 3H)

### Reference Production Example 3-5

Production of 6-cyano-N-methyl-2-(1-methyl-3-phenyl-1H-1,2,4-triazol-5-yl)pyridine-3-sulfonamide

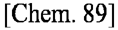

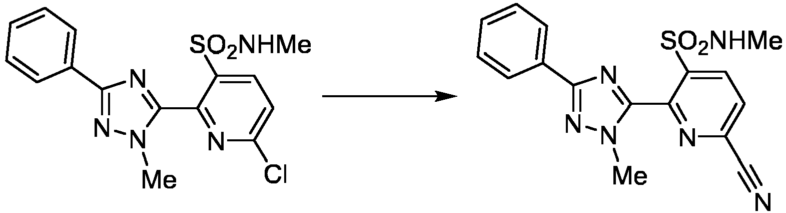

To a solution of 6-chloro-N-methyl-2-(1-methyl-3-phenyl-1H-1,2,4-triazol-5-yl)pyridine-3-sulfonamide (0.30 g, 0.83 mmol) in dimethyl sulfoxide (2.0 mL), sodium cyanide (61 mg, 1.2 mmol) and 1,4-diazabicyclo[2.2.2]octane (47 mg, 0.4 mmol) were added, and the mixture was stirred at 40°C for 2 hours. Saturated brine was added, and ethyl acetate extraction was performed. The organic layer was concentrated in vacuo, and the residue was purified by silica gel column chromatography to give 6-cyano-N-methyl-2-(1-methyl-3-phenyl-1H-1,2,4-triazol-5-yl)pyridine-3-sulfonamide (0.16 g, 0.45 mmol).
Yield: 55%

### Reference Example 4

Production of 6-cyano-N-methyl-2-(1-methyl-4-phenyl-1H-imidazol-2-yl)pyridine-3-sulfonamide (starting compound for Production Example 4)

### Reference Production Example 4-1

Production of 2-oxo-2-phenylethyl 3-((4-(tert-butyl)benzyl)thio)-6-chloropicolinate

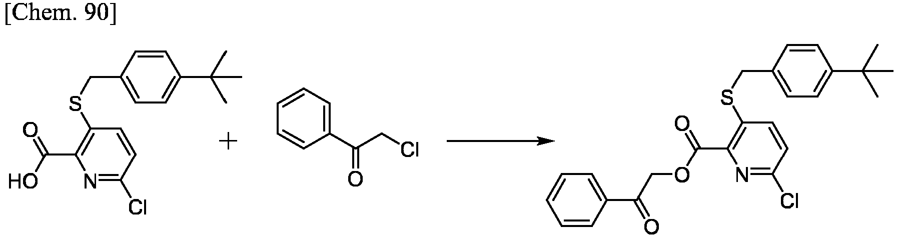

To a solution of 3-((4-(tert-butyl)benzyl)thio)-6-chloropicolinic acid (6.6 g, 20 mmol) in N,N-dimethylformamide (28 mL), phenacyl chloride (3.0 g, 20 mmol) and N,N-diisopropylethylamine (8.3 mL, 49 mmol) were added under ice cooling, and the mixture was stirred at room temperature overnight. After the completion of the reaction, water and ethyl acetate were added for extraction. The organic layer was dried over anhydrous sodium sulfate and then concentrated in vacuo to give 2-oxo-2-phenylethyl 3-((4-(tert-butyl)benzyl)thio)-6-chloropicolinate (7.8 g, 17 mmol) as a crude product.
Yield: 88%

### Reference Production Example 4-2

Production of 3-((4-(tert-butyl)benzyl)thio)-6-chloro-2-(4-phenyl-1H-imidazol-2-yl)pyridine

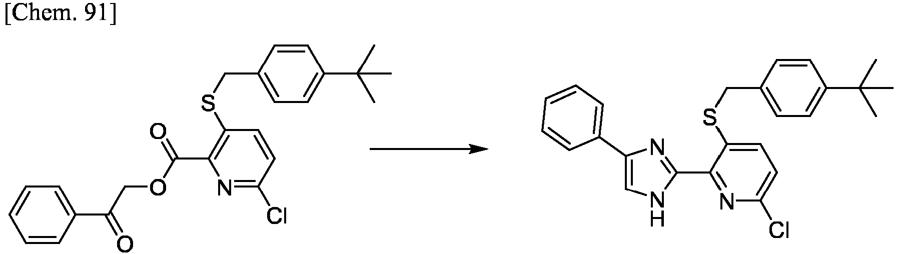

To a solution of 2-oxo-2-phenylethyl 3-((4-(tert-butyl)benzyl)thio)-6-chloropicolinate (7.8 g, 17 mmol) in acetic acid (86 mL), ammonium acetate (27 g, 0.34 mol) was added, and the mixture was stirred and heated under reflux. The reaction mixture was concentrated in vacuo. A saturated aqueous sodium hydrogen carbonate solution and ethyl acetate were added to the residue for extraction. The organic layer was dried over anhydrous sodium sulfate and then concentrated in vacuo. The residue was purified by silica gel column chromatography to give 3-((4-(tert-butyl)benzyl)thio)-6-chloro-2-(4-phenyl-1H-imidazol-2-yl)pyridine (7.4 g, 17 mmol).
Yield: 99%

### Reference Production Example 4-3

Production of 3-((4-(tert-butyl)benzyl)thio)-6-chloro-2-(1-methyl-4-phenyl-1H-imidazol-2-yl)pyridine

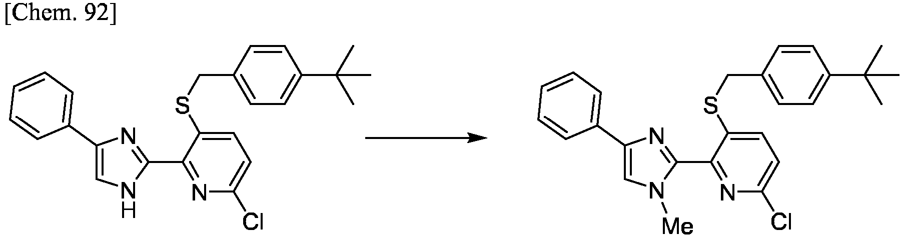

To a solution of 3-((4-(tert-butyl)benzyl)thio)-6-chloro-2-(4-phenyl-1H-imidazol-2-yl)pyridine (7.4 g, 17 mmol) in tetrahydrofuran (90 mL), potassium tert-butoxide (2.1 g, 18.8 mmol) and iodomethane (1.2 mL, 19 mmol) were added under ice cooling, and the mixture was stirred at room temperature for 2 hours. A saturated aqueous ammonium chloride solution and ethyl acetate were added for extraction. The organic layer was dried over sodium sulfate and then concentrated. The residue was purified by silica gel column chromatography to give 3-((4-(tert-butyl)benzyl)thio)-6-chloro-2-(1-methyl-4-phenyl-1H-imidazol-2-yl)pyridine (4.6 g, 10 mmol).
Yield: 59%

### Reference Production Example 4-4

Production of 6-chloro-N-methyl-2-(1-methyl-4-phenyl-1H-imidazol-2-yl)pyridine-3-sulfonamide

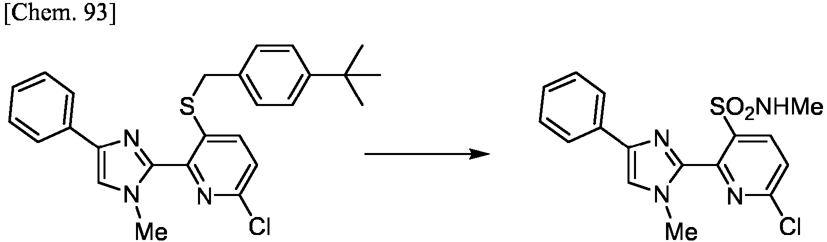

To a solution of 3-((4-(tert-butyl)benzyl)thio)-6-chloro-2-(1-methyl-4-phenyl-1H-imidazol-2-yl)pyridine (2.0 g, 4.5 mmol) in chloroform (50 mL), water (0.50 mL, 27 mmol) and acetic acid (0.77 mL, 13 mmol) were added, and 1,3-dichloro-5,5-dimethylhydantoin (2.6 g, 13 mmol) was added with cooling in an ice bath. After the disappearance of the starting compound was confirmed, the reaction mixture was added dropwise to a methylamine-methanol solution (30 mL) with cooling in an ice bath, and the mixture was stirred for 30 minutes. A saturated aqueous ammonium chloride solution was added, and ethyl acetate extraction was performed. The organic layer was concentrated, and the residue was purified by silica gel column chromatography to give 6-chloro-N-methyl-2-(1-methyl-4-phenyl-1H-imidazol-2-yl)pyridine-3-sulfonamide (0.51 mg, 1.4 mmol).
Yield: 31%
Physical property: ¹H-NMR (CDCl₃): δ 8.95 (q, 1H), 8.42 (d, 1H), 7.67-7.63 (m, 2H), 7.48-7.38 (m, 3H), 7.33-7.29 (m, 2H), 3.97 (s, 3H), 2.78 (d, 3H)

### Reference Production Example 4-5

Production of 6-cyano-N-methyl-2-(1-methyl-4-phenyl-1H-imidazol-2-yl)pyridine-3-sulfonamide

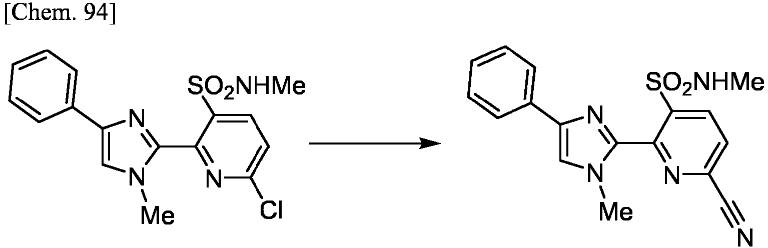

To a solution of 6-chloro-N-methyl-2-(1-methyl-4-phenyl-1H-imidazol-2-yl)pyridine-3-sulfonamide (0.43 g, 1.2 mmol) in dimethyl sulfoxide (10 mL), sodium cyanide (70 mg, 1.4 mmol) and 1,4-diazabicyclo[2.2.2]octane (67 mg, 0.60 mmol) were added, and the mixture was stirred at 40°C for 2 hours. Saturated brine was added, and ethyl acetate extraction was performed. The organic layer was concentrated in vacuo, and the residue was purified by silica gel column chromatography to give 6-cyano-N-methyl-2-(1-methyl-4-phenyl-1H-imidazol-2-yl)pyridine-3-sulfonamide (0.10 g, 0.28 mmol).
Yield: 24%

### Reference Example 5

Production of (Z)-6-chloro-N'-ethoxy-5-(N-methylsulfamoyl)picolinimidamide (starting compound for Production Examples 5 and 6)

### Reference Production Example 5-1

Production of 2,6-dichloro-N-methylpyridine-3-sulfonamide

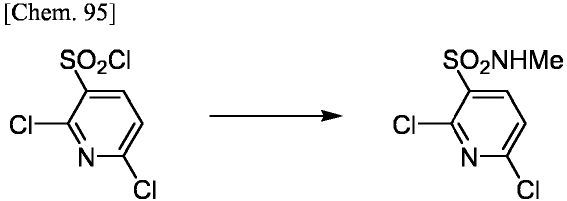

To a solution of 2,6-dichloropyridine-3-sulfonyl chloride in tetrahydrofuran (0.20 L), a 7.0% tetrahydrofuran solution of methylamine (31 mL, 61 mmol) was added under ice cooling, and the mixture was stirred at room temperature for 1 hour. After the completion of the reaction, a saturated aqueous ammonium chloride solution and ethyl acetate were added to the reaction mixture for extraction. The organic layer was dried over anhydrous sodium sulfate and then concentrated in vacuo. The residue was purified by silica gel column chromatography to give 2,6-dichloro-N-methylpyridine-3-sulfonamide (6.4 g, 27 mmol).
Yield: 44%
Physical property: ¹H-NMR (CDCl₃): δ 8.35 (d, 1H), 7.46 (d, 1H), 5.10 (br.s, 1H), 2.71 (d, 3H)

### Reference Production Example 5-2

Production of 2-chloro-6-cyano-N-methylpyridine-3-sulfonamide

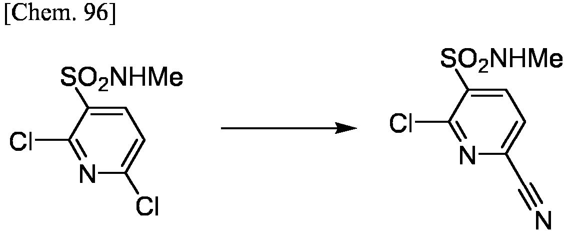

To a solution of 2,6-dichloro-N-methylpyridine-3-sulfonamide (9.8 g, 40 mmol) and 1,4-diazabicyclo[2.2.2]octane (0.81 g, 7.2 mmol) in dimethyl sulfoxide (0.10 L), a solution of sodium cyanide (2.3 g, 47 mmol) in water (10 mL) was added dropwise, and the mixture was stirred at room temperature. After the completion of the reaction, water and ethyl acetate were added to the reaction mixture for extraction. The organic layer was dried over anhydrous sodium sulfate and then concentrated in vacuo. The residue was purified by silica gel column chromatography to give 2-chloro-6-cyano-N-methylpyridine-3-sulfonamide (7.7 g, 33 mmol).
Yield: 82%
Physical property: ¹H-NMR (CDCl₃): δ 8.56 (d, 1H), 7.82 (d, 1H), 5.07 (q, 1H), 2.74 (d, 3H)

### Reference Production Example 5-3

Production of (Z)-6-chloro-N'-ethoxy-5-(N-methylsulfamoyl)picolinimidamide

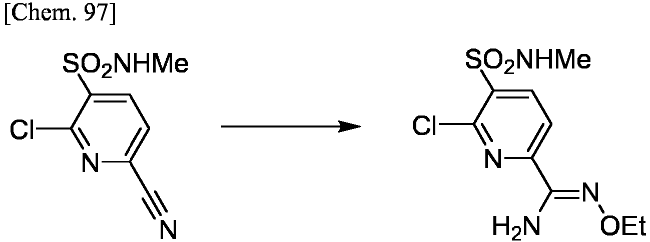

To a solution of 2-chloro-6-cyano-N-methylpyridine-3-sulfonamide (1.1 g, 2.9 mmol) in methanol (20 mL), a 28 wt% methanol solution of sodium methoxide (0.59 mL, 2.9 mmol) was added, and the mixture was stirred at room temperature. After the disappearance of the starting compound was confirmed, O-ethylhydroxylamine hydrochloride (0.37 g, 3.8 mmol) was added, and the mixture was stirred at room temperature. After the completion of the reaction, water and ethyl acetate were added for extraction. The organic layer was dried over anhydrous sodium sulfate and then concentrated in vacuo to give (Z)-6-chloro-N'-ethoxy-5-(N-methylsulfamoyl)picolinimidamide (1.2 g, 2.9 mmol).
Yield: 100%
Physical property: ¹H-NMR (CDCl₃): δ 8.35 (d, 1H), 8.07 (d, 1H), 5.45 (br.s, 2H), 5.05 (q, 1H), 4.20 (q, 2H), 2.68 (d, 3H), 1.34 (t, 3H)

### Reference Example 6

Production of (E)-1-(6-chloro-5-(methylsulfonyl)pyridin-2-yl)ethanone O-ethyl oxime (starting compound for Production Example 7)

### Reference Production Example 6-1

Production of methyl 6-chloro-3-(methylsulfanyl)picolinate

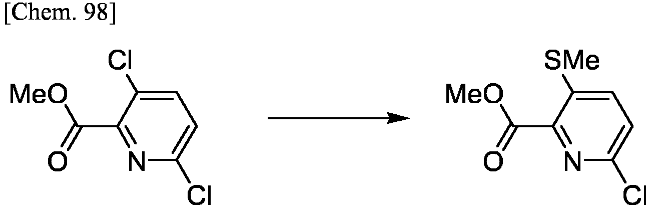

To a solution of methyl 3,6-dichloropicolinate (21 g, 0.10 mol) in N,N-dimethylformamide (0.20 L), sodium methanethiolate (8.2 g, 0.11 mol) was slowly added at 0°C, and the mixture was stirred at 0°C for 2 hours. After the completion of the reaction, water was added to the reaction mixture. The precipitated solid was collected by filtration, washed with ethanol, and dried to give methyl 6-chloro-3-(methylsulfanyl)picolinate (18 g, 80 mmol).
Yield: 80%

### Reference Production Example 6-2

Production of methyl 6-chloro-3-(methylsulfonyl)picolinate

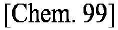

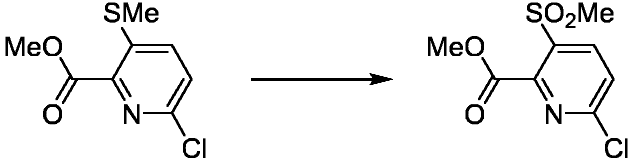

m-Chloroperbenzoic acid (22 g, 82 mmol) was added to a chloroform solution (160 mL) of methyl 6-chloro-3-(methylsulfanyl)picolinate (7.2 g, 33 mmol), and the mixture was stirred at room temperature for 2 hours. After the completion of the reaction, a saturated aqueous sodium hydrogen carbonate solution and a saturated aqueous sodium thiosulfate solution were added to the reaction mixture, and chloroform extraction was performed. The organic layer was dried over anhydrous magnesium sulfate and then concentrated in vacuo to give methyl 6-chloro-3-(methylsulfonyl)picolinate (8.1 g, 33 mmol).
Yield: 99%

### Reference Production Example 6-3

Production of (E)-methyl 6-(1 -(ethoxyimino)ethyl)-3-(methylsulfonyl)picolinate

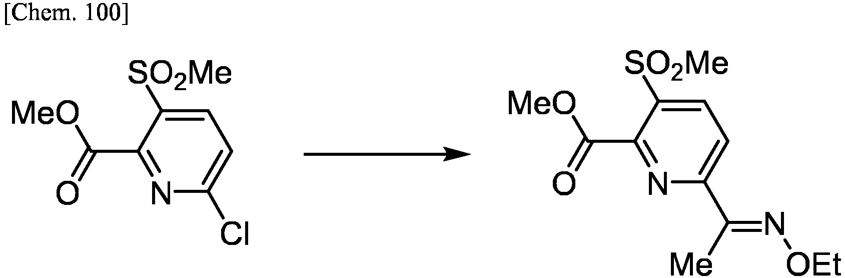

To a 1,2-dimethoxyethane solution (0.20 L) of methyl 6-chloro-3-(methylsulfonyl)picolinate (11 g, 41 mmol), tributyl(1-ethoxyvinyl)tin (17 mL, 49 mmol) and tetrakis(triphenylphosphine)palladium(0) (2.5 g, 2.2 mmol) were added under an argon atmosphere at room temperature, and the mixture was stirred at 110°C for 3 hours. After the reaction mixture was allowed to come to room temperature, tetrahydrofuran (0.10 L) and 2 N hydrochloric acid (0.10 L) were added, and the mixture was stirred at 50°C for 3 hours. After the completion of the reaction, ethyl acetate and water were added to the reaction mixture for extraction. The organic layer was dried over anhydrous magnesium sulfate and then concentrated in vacuo. The residue was purified by silica gel column chromatography to give methyl 6-acetyl-3-(methylsulfonyl)picolinate. The obtained methyl 6-acetyl-3-(methylsulfonyl)picolinate was dissolved in chloroform (0.20 L), and pyridine (60 mL) and O-ethylhydroxylamine hydrochloride (5.94 g, 60.9 mmol) were added. The mixture was stirred at room temperature overnight. 1 N Hydrochloric acid was added to the reaction mixture, and chloroform extraction was performed. The organic layer was dried over anhydrous magnesium sulfate and then concentrated in vacuo to give (E)-methyl 6-(1-(ethoxyimino)ethyl)-3-(methylsulfonyl)picolinate as a crude product. This crude product was used in the next step without further purification.

### Reference Production Example 6-4

Production of (E)-6-(1-(ethoxyimino)ethyl)-3-(methylsulfonyl)picolinic acid

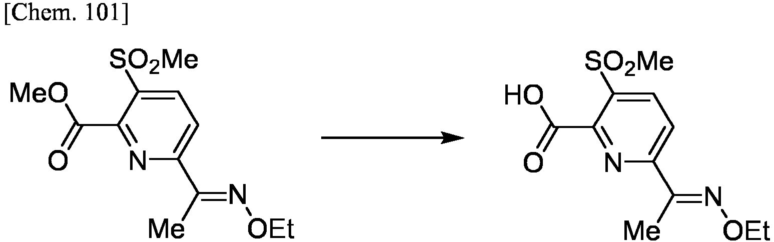

To a methanol solution (0.15 L) of (E)-methyl 6-(1-(ethoxyimino)ethyl)-3-(methylsulfonyl)picolinate, a 4 M aqueous lithium hydroxide solution (16 mL) was added at room temperature, and the mixture was stirred for 1 hour. 2 N Hydrochloric acid and chloroform were added to the reaction mixture for extraction. A 2 N aqueous sodium hydroxide solution was added, and the aqueous layer was separated. A 2 N aqueous hydrochloric acid solution was added to the aqueous layer, and ethyl acetate extraction was performed under acidic conditions. The organic layer was dried over anhydrous magnesium sulfate and then concentrated in vacuo. Methyl tert-butyl ether and hexane were added to the residue, and the precipitated solid was collected by filtration to give (E)-6-(1-(ethoxyimino)ethyl)-3-(methylsulfonyl)picolinic acid (8.4 g, 32 mmol).
Yield: 80% (in 3 steps)

### Reference Production Example 6-5

Production of (E)-1-(6-amino-5-(methylsulfonyl)pyridin-2-yl)ethanone O-ethyl oxime

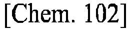

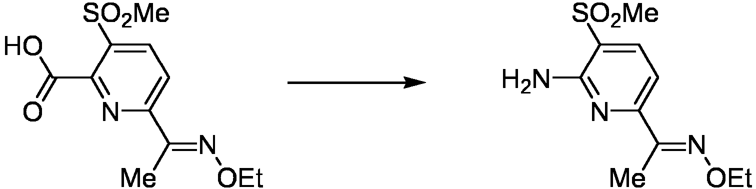

To a tert-butyl alcohol solution (20 mL) of (E)-6-(1-(ethoxyimino)ethyl)-3-(methylsulfonyl)picolinic acid (1.0 g, 3.5 mmol), triethylamine (0.73 mL, 5.2 mmol) and diphenylphosphoryl azide (1.2 mL, 5.2 mmol) were added, and the mixture was stirred at 100°C for 2 hours. The reaction mixture was concentrated in vacuo. Water was added to the residue, and ethyl acetate extraction was performed. The organic layer was dried over anhydrous magnesium sulfate and then concentrated in vacuo. Trifluoroacetic acid (10 mL) was added to the residue, and the mixture was stirred at room temperature for 2 hours. After the completion of the reaction, the reaction mixture was concentrated in vacuo. A saturated aqueous sodium hydrogen carbonate solution was added to the residue, and ethyl acetate extraction was performed. The organic layer was dried over anhydrous magnesium sulfate and then concentrated in vacuo. The residue was purified by silica gel column chromatography to give (E)-1-(6-amino-5-(methylsulfonyl)pyridin-2-yl)ethanone O-ethyl oxime (0.68 g, 2.6 mmol).
Yield: 75%

### Reference Production Example 6-6

Production of (E)-1-(6-chloro-5-(methylsulfonyl)pyridin-2-yl)ethanone O-ethyl oxime

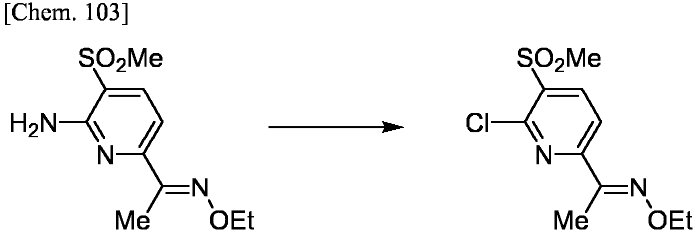

To a tetrahydrofuran solution (4.0 mL) of (E)-1-(6-amino-5-(methylsulfonyl)pyridin-2-yl)ethanone O-ethyl oxime (0.22 g, 0.84 mmol), copper(II) chloride (0.17 g, 1.3 mmol) and tert-butyl nitrite (0.20 mL, 1.7 mmol) were added at room temperature, and the mixture was stirred at 60°C for 1 hour. A saturated aqueous ammonium chloride solution was added, and ethyl acetate extraction was performed, followed by washing with saturated brine. The organic layer was dried over anhydrous magnesium sulfate and then concentrated in vacuo. The residue was purified by silica gel column chromatography to give (E)-1-(6-chloro-5-(methylsulfonyl)pyridin-2-yl)ethanone O-ethyl oxime (0.14 g, 0.51 mmol).
Yield: 60%
Physical property: ¹H-NMR (CDCl₃): δ 8.37 (d, 1H), 8.08 (d, 1H), 4.33 (q, 2H), 3.32 (s, 3H), 2.31 (s, 3H), 1.36 (t, 3H)

### Reference Example 7

Production of (Z)-4-((4-(tert-butyl)benzyl)thio)-3-(3-(2-chlorophenyl)-1-methyl-1H-1,2,4-triazol-5-yl)-N'-ethoxybenzimidamide (starting compound for Production Example 8)

### Reference Production Example 7-1

Production of 3-(2-chlorophenyl)-5-(2-fluoro-5-iodophenyl)-1-methyl-1H-1,2,4-triazole

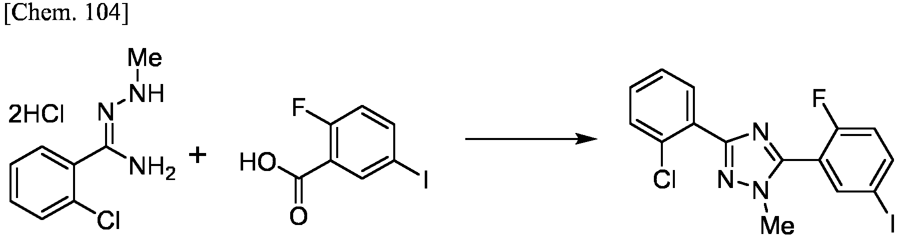

To a solution of 2-chloro-N'-methylbenzimidohydrazide dihydrochloride (3.5 g, 19 mmol) and 2-fluoro-5-iodobenzoic acid (5.1 g, 19 mmol) in pyridine (50 mL), N,N-dimethyl-4-aminopyridine (2.4 g, 19 mmol) and 1-(3-dimethylaminopropyl)-3-ethylcarbodiimide hydrochloride (4.1 g, 21 mmol) were added, and the mixture was stirred at room temperature overnight. After the completion of the reaction, the reaction mixture was concentrated in vacuo. 0.5 N Hydrochloric acid was added to the residue, and ethyl acetate extraction was performed. The organic layer was dried over anhydrous magnesium sulfate and then concentrated in vacuo. Acetic acid (50 mL) was added to the residue, and the mixture was stirred and heated under reflux for 1 hour. After the completion of the reaction, the reaction mixture was concentrated in vacuo. A saturated aqueous sodium hydrogen carbonate solution was added to the residue, and ethyl acetate extraction was performed. The organic layer was dried over anhydrous magnesium sulfate and then concentrated in vacuo. The residue was purified by silica gel column chromatography to give 3-(2-chlorophenyl)-5-(2-fluoro-5-iodophenyl)-1-methyl-1H-1,2,4-triazole (1.8 g, 4.4 mmol).
Yield: 23%
Physical property: ¹H-NMR (CDCl₃): δ 8.03 (dd, 1H), 7.91 (dd, 1H), 7.83 (ddd, 1H), 7.51 (dd, 1H), 7.38-7.33 (m, 2H), 7.03 (dd, 1H), 3.96 (s, 3H)

### Reference Production Example 7-2

Production of 3-(3-(2-chlorophenyl)-1-methyl-1H-1,2,4-triazol-5-yl)-4-fluorobenzonitrile

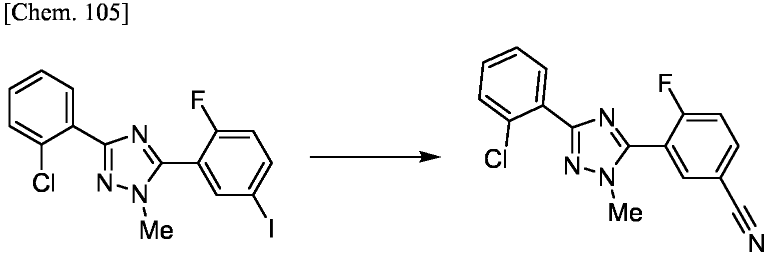

To a solution of 3-(2-chlorophenyl)-5-(2-fluoro-5-iodophenyl)-1-methyl-1H-1,2,4-triazole (1.6 g, 3.9 mmol) in N,N-dimethylacetamide (45 mL), triethylamine (0.81 mL, 5.9 mmol), tetrakis(triphenylphosphine)palladium(0) (0.45 g, 0.39 mmol), and zinc cyanide (0.70 g, 5.9 mmol) were added at room temperature, and the mixture was stirred at 110°C for 2 hours. After the completion of the reaction, the reaction mixture was filtered through Celite, followed by washing with ethyl acetate. The filtrate was concentrated in vacuo, and the residue was purified by silica gel column chromatography to give 3-(3-(2-chlorophenyl)-1-methyl-1H-1,2,4-triazol-5-yl)-4-fluorobenzonitrile (1.1 g, 3.5 mmol).
Yield: 90%
Physical property: ¹H-NMR (CDCl₃): δ 8.09 (dd, 1H), 7.92 (dd, 1H), 7.87 (ddd, 1H), 7.52 (dd, 1H), 7.41 (dd, 1H), 7.39-7.34 (m, 2H), 3.97 (s, 3H)

### Reference Production Example 7-3

Production of 4-((4-(tert-butyl)benzyl)thio)-3 -(3 -(2-chlorophenyl)-1 -methyl-1H-1,2,4-triazol-5-yl)benzonitrile

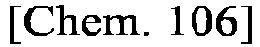

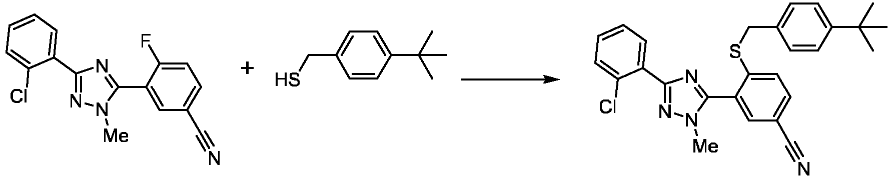

To a solution of 3-(3-(2-chlorophenyl)-1-methyl-1H-1,2,4-triazol-5-yl)-4-fluorobenzonitrile (0.50 g, 1.6 mmol) in N,N-dimethylacetamide (5.5 mL), cesium carbonate (0.78 g, 2.4 mmol) and (4-(tert-butyl)phenyl)methanethiol (0.35 mL, 1.9 mmol) were added at room temperature, and the mixture was stirred at the same temperature for 2 hours. After the completion of the reaction, a saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give 4-((4-(tert-butyl)benzyl)thio)-3-(3-(2-chlorophenyl)-1-methyl-1H-1,2,4-triazol-5-yl)benzonitrile (0.78 g, 1.6 mmol).
Yield: 100%
Physical property: ¹H-NMR (CDCl₃): δ 7.95 (dd, 1H), 7.71-7.67 (m, 2H), 7.52-7.48 (m, 2H), 7.36-7.31 (m, 4H), 7.24 (d, 2H), 4.15 (s, 2H), 3.80 (s, 3H), 1.29 (s, 9H)

### Reference Production Example 7-4

Production of (Z)-4-((4-(tert-butyl)benzyl)thio)-3-(3-(2-chlorophenyl)-1-methyl-1H-1,2,4-triazol-5-yl)-N'-hydroxybenzimidamide

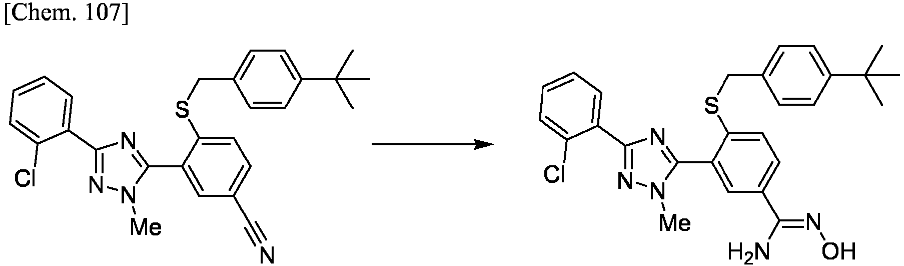

To a solution of 4-((4-(tert-butyl)benzyl)thio)-3-(3-(2-chlorophenyl)-1-methyl-1H-1,2,4-triazol-5-yl)benzonitrile (0.78 g, 1.6 mmol) in ethanol (8.2 mL), sodium acetate (0.21 g, 2.5 mmol) and hydroxylamine hydrochloride (0.17 g, 2.5 mmol) were added at room temperature, and the mixture was stirred at 80°C for 2 hours. After the completion of the reaction, water was added to the reaction mixture, and ethyl acetate extraction was performed. The organic layer was dried over anhydrous magnesium sulfate and then concentrated in vacuo to give (Z)-4-((4-(tert-butyl)benzyl)thio)-3-(3-(2-chlorophenyl)-1-methyl-1H-1,2,4-triazol-5-yl)-N'-hydroxybenzimidamide (0.72 g, 1.4 mmol).
Yield: 86%

### Reference Production Example 7-5

Production of (Z)-4-((4-(tert-butyl)benzyl)thio)-3-(3-(2-chlorophenyl)-1-methyl-1H -1,2,4-triazol-5-yl)-N'-ethoxybenzimidamide

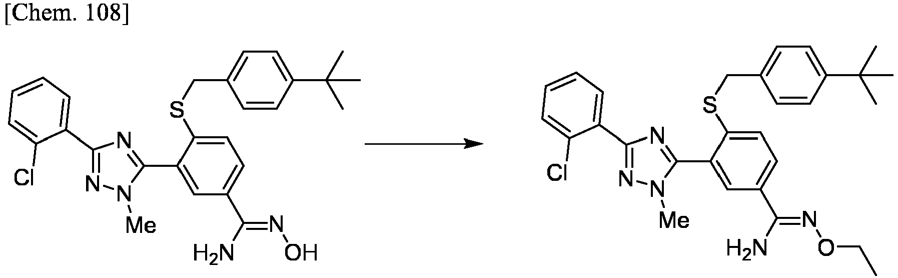

To a solution of (Z)-4-((4-(tert-butyl)benzyl)thio)-3-(3-(2-chlorophenyl)-1-methyl-1H-1,2,4-triazol-5-yl)-N'-hydroxybenzimidamide (0.28 g, 0.54 mmol) in N,N-dimethylformamide (3.0 mL), cesium carbonate (0.27 g, 0.82 mmol) and ethyl iodide (55 µL, 0.66 mmol) were added at room temperature, and the mixture was stirred at the same temperature for 2 hours. After the completion of the reaction, a saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give (Z)-4-((4-(tert-butyl)benzyl)thio)-3-(3-(2-chlorophenyl)-1-methyl-1H-1,2,4-triazol-5-yl)-N'-ethoxybenzimidamide (0.23 g, 0.43 mmol).
Yield: 78%

### Reference Example 8

Production of 6-cyano-N-methyl-2-(4-methyl-1-(2-nitrophenyl)-5-oxo-4,5-dihydro-1H-1,2,4-triazol-3-yl)pyridine-3-sulfonamide (starting compound for Production Example 10-1)

### Reference Production Example 8-1

Production of 3-((4-(tert-butyl)benzyl)thio)-6-chloropicolinohydrazide

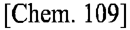

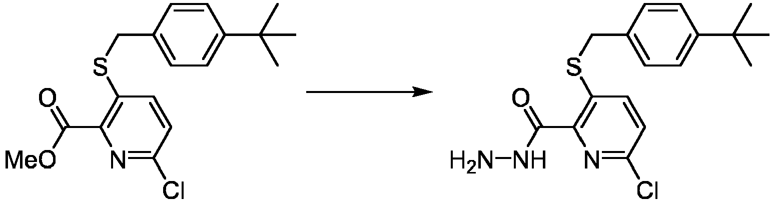

To a solution of methyl 3-((4-(tert-butyl)thio)-6-chloropicolinate (11 g, 30 mmol) in methanol (30 mL) and tetrahydrofuran (86 mL), hydrazine monohydrate (2.2 mL, 45 mmol) was added at room temperature, and the mixture was stirred overnight. The reaction mixture was concentrated in vacuo. The resulting solid was collected by filtration and washed with hexane to give 3-((4-(tert-butyl)benzyl)thio)-6-chloropicolinohydrazide (11 g, 30 mmol).
Yield: 100%

### Reference Production Example 8-2

Production of 3-(3-((4-(tert-butyl)benzyl)thio)-6-chloropyridin-2-yl)-4-methyl-1H-1,2,4-triazol-5(4H)-one

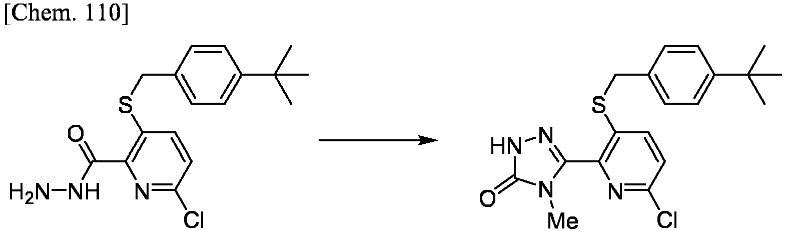

To a pyridine solution (30 mL) of 3-((4-(tert-butyl)benzyl)thio)-6-chloropicolinohydrazide (11 g, 30 mmol), methylcarbamoyl chloride (3.4 g, 36 mmol) was added with cooling in an ice bath, and the mixture was stirred at room temperature overnight. The reaction mixture was concentrated in vacuo, and water was added to the residue. The resulting solid was collected by filtration. This was dissolved in a 1 N aqueous sodium hydroxide solution (36 mL) and heated under reflux for 24 hours. 2 M Hydrochloric acid was added to the reaction mixture, and the resulting solid was collected by filtration and washed with methyl tert-butyl ether and hexane to give 3-(3-((4-(tert-butyl)benzyl)thio)-6-chloropyridin-2-yl)-4-methyl-1H-1,2,4-triazol-5(4H)-one (12 g, 30 mmol).
Yield: 100%

### Reference Production Example 8-3

Production of 3-(3-((4-(tert-butyl)benzyl)thio)-6-chloropyridin-2-yl)-4-methyl-1-(2-nitrophenol)-1H-1,2,4-triazol-5 (4H)-one

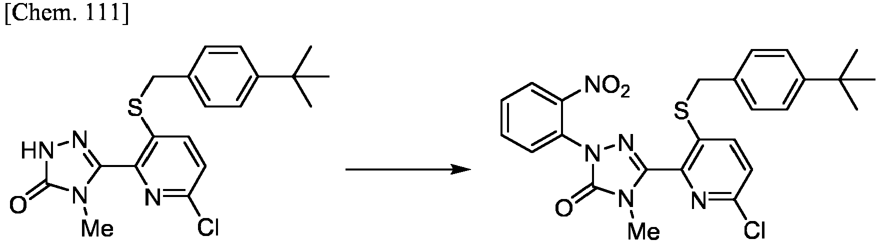

To a solution of 3-(3-((4-(tert-butyl)benzyl)thio)-6-chloropyridin-2-yl)-4-methyl-1H-1,2,4-triazol-5(4H)-one (5.6 g, 14.6 mmol) in dimethyl sulfoxide (70 mL), potassium carbonate (6.0 g, 44 mmol) and 2-fluoronitrobenzene (3.1 g, 22 mmol) were added, and the mixture was stirred at 80°C for 4 hours. Water was added to the reaction mixture, and ethyl acetate extraction was performed, followed by washing with saturated brine. The organic layer was concentrated in vacuo, and the residue was purified by silica gel column chromatography to give 3-(3-((4-(tert-butyl)benzyl)thio)-6-chloropyridin-2-yl)-4-methyl-1-(2-nitrophenol)-1H-1,2,4-triazol-5(4H)-one (2.8 g, 5.4 mmol).
Yield: 37%
Physical property: ¹H-NMR (CDCl₃): δ 7.99-7.94 (m, 1H), 7.83-7.79 (m, 1H), 7.72-7.66 (m, 2H), 7.51-7.45 (m, 1H), 7.36-7.25 (m, 5H), 4.13 (s, 2H), 3.47 (s, 3H), 1.30 (s, 9H)

### Reference Production Example 8-4

Production of 6-chloro-N-methyl-2-(4-methyl-1-(2-nitrophenyl)-5-oxo-4,5-dihydro-1H-1,2,4-triazol-3-yl)pyridine-3-sulfonamide

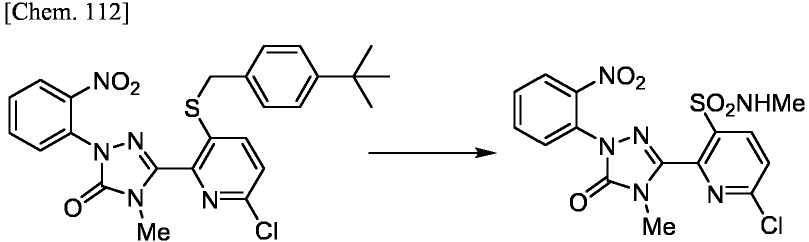

To a solution of 3-(3-((4-(tert-butyl)benzyl)thio)-6-chloropyridin-2-yl)-4-methyl-1-(2-nitrophenol)-1H-1,2,4-triazol-5(4H)-one (2.8 g, 5.4 mmol) in chloroform (20 mL), water (0.60 mL, 33 mmol) and acetic acid (1.0 mL, 16 mmol) were added, and 1,3-dichloro-5,5-dimethylhydantoin (3.2 g, 16 mmol) was added with cooling in an ice bath. After the disappearance of the starting compound was confirmed, the reaction mixture was added dropwise to a methanol solution of methylamine (9.8 mol/L, 20 mL) with cooling in an ice bath, and the mixture was stirred for 30 minutes. A saturated aqueous ammonium chloride solution was added, and ethyl acetate extraction was performed. The organic layer was concentrated, and the residue was purified by silica gel column chromatography to give 6-chloro-N-methyl-2-(4-methyl-1-(2-nitrophenyl)-5-oxo-4,5-dihydro-1H-1,2,4-triazol-3-yl)pyridine-3-sulfonamide (1.2 g, 2.8 mmol).
Yield: 53%

### Reference Production Example 8-5

Production of 6-cyano-N-methyl-2-(4-methyl-1-(2-nitrophenyl)-5-oxo-4,5-dihydro-1H-1,2,4-triazol-3-yl)pyridine-3-sulfonamide

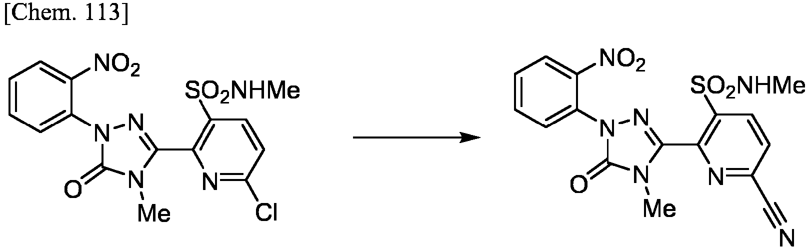

To a solution of 6-chloro-N-methyl-2-(4-methyl-1-(2-nitrophenyl)-5-oxo-4,5-dihydro-1H-1,2,4-triazol-3-yl)pyridine-3-sulfonamide (1.2 g, 2.8 mmol) in dimethyl sulfoxide (10 mL), sodium cyanide (0.17 g, 3.5 mmol) and 1,4-diazabicyclo[2.2.2]octane (0.16 g, 1.4 mmol) were added, and the mixture was stirred at 40°C for 2 hours. After the completion of the reaction, saturated brine was added to the reaction mixture, and ethyl acetate extraction was performed. The organic layer was concentrated in vacuo, and the residue was purified by silica gel column chromatography to give 6-cyano-N-methyl-2-(4-methyl-1-(2-nitrophenyl)-5-oxo-4,5-dihydro-1H-1,2,4-triazol-3-yl)pyridine-3-sulfonamide (1.0 g, 2.5 mmol).
Yield: 86%
Physical property: ¹H-NMR (CDCl₃): δ 8.66 (d, 1H), 8.00 (d, 1H), 7.94-7.90 (m, 2H), 7.79-7.73 (m, 1H), 6.06 (q, 1H), 3.51 (s, 3H), 2.75 (d, 3H)

### Reference Example 9

Production of (Z)-N'-ethoxy-6-(4-methyl-1H-pyrazol-3-yl)-5-(methylsulfonyl)picolinimidamide (starting compound for Production Example 13-1)

### Reference Production Example 9-1

Production of 6-chloro-N-methoxy-N-methyl-3-(methylthio)picolinamide

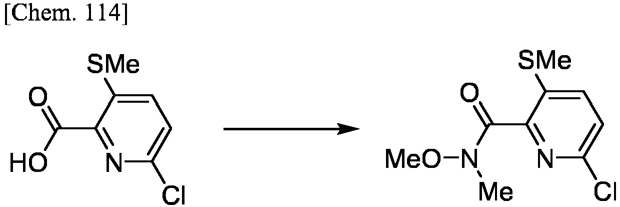

To a solution of 6-chloro-3-(methylthio)picolinic acid (16 g, 79 mmol) in THF (80 mL), N,N-dimethylformamide (0.6 mL, 7.8 mmol) and oxalyl chloride (7.4 mL, 86 mmol) were added at room temperature, and the mixture was stirred at room temperature for 30 minutes. After the completion of the reaction, the reaction mixture was concentrated in vacuo. Chloroform (80 mL), triethylamine (44 mL, 0.31 mol), and N-methoxy-N-methylamine hydrochloride (9.2 g, 94 mmol) were added to the residue at 0°C, and the mixture was stirred at room temperature for 2 hours. After the completion of the reaction, water was added to the reaction mixture, and ethyl acetate extraction was performed. The organic layer was dried over anhydrous magnesium sulfate and then concentrated in vacuo to give 6-chloro-N-methoxy-N-methyl-3-(methylthio)picolinamide (11 g, 44 mmol).
Yield: 56%
Physical property: ¹H-NMR (CDCl₃): δ 7.65 (d, 1H), 7.32 (d, 1H), 3.63 (s, 3H), 3.38 (s, 3H), 2.46 (s, 3H)

### Reference Production Example 9-2

Production of 1 -(6-chloro-3 -(methylthio)pyridin-2-yl)propan-1 -one

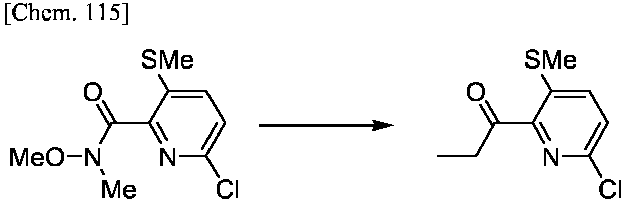

To a solution of 6-chloro-N-methoxy-N-methyl-3-(methylthio)picolinamide (10 g, 41 mmol) in THF (25 mL), ethylmagnesium bromide (solution in diethyl ether, 50 mL, 53 mmol) was added at 0°C, and the mixture was stirred for 2 hours. After the completion of the reaction, a saturated aqueous ammonium chloride solution was added to the reaction mixture, and ethyl acetate extraction was performed. The organic layer was dried over anhydrous magnesium sulfate and then concentrated in vacuo. The residue was purified by silica gel column chromatography to give 1-(6-chloro-3-(methylthio)pyridin-2-yl)propan-1-one (3.7 g, 17 mmol).
Yield: 41%
Physical property: ¹H-NMR (CDCl₃): δ 7.62 (d, 1H), 7.41 (d, 1H), 3.21 (q, 2H), 2.43 (s, 3H), 1.20 (t, 3H)

### Reference Production Example 9-3

Production of 6-chloro-2-(4-methyl-1H-pyrazol-3-yl)-3-(methylthio)pyridine

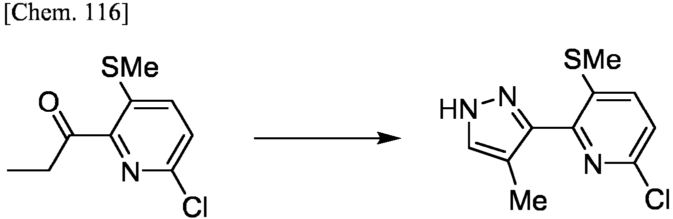

N,N-dimethylformamide dimethyl acetal (3.0 mL, 22 mmol) was added to 1-(6-chloro-3-(methylthio)pyridin-2-yl)propan-1-one (2.3 g, 11 mmol) at room temperature, and the mixture was stirred at 60°C for 2 hours. After the completion of the reaction, the reaction mixture was concentrated in vacuo. N,N-dimethylacetamide (10 mL) and hydrazine monohydrate (0.63 mL, 13 mmol) were added to the residue at room temperature, and the mixture was stirred at 120°C for 2 hours. After the completion of the reaction, water was added to the reaction mixture, and ethyl acetate extraction was performed. The organic layer was dried over anhydrous magnesium sulfate and then concentrated in vacuo. The residue was purified by silica gel column chromatography to give 6-chloro-2-(4-methyl-1H-pyrazol-3-yl)-3-(methylthio)pyridine (1.4 g, 5.6 mmol). Yield: 51%

### Reference Production Example 9-4

Production of 6-chloro-2-(4-methyl-1H-pyrazol-3-yl)-3-(methylsulfonyl)pyridine

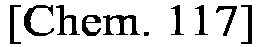

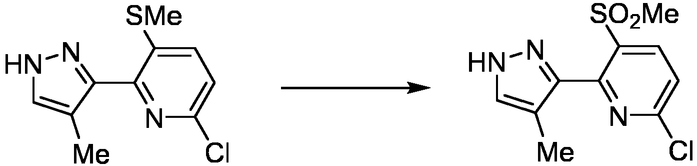

To a solution of 6-chloro-2-(4-methyl-1H-pyrazol-3-yl)-3-(methylthio)pyridine (1.1 g, 4.6 mmol) in ethyl acetate (4.0 mL), m-chloroperbenzoic acid (0.44 g, 14 mmol) was added, and the mixture was stirred at room temperature for 3 hours. After the completion of the reaction, a saturated aqueous sodium hydrogen carbonate solution and a saturated aqueous sodium thiosulfate solution were added to the reaction mixture, and ethyl acetate extraction was performed. The organic layer was dried over anhydrous magnesium sulfate and then concentrated in vacuo. The residue was purified by silica gel column chromatography to give 6-chloro-2-(4-methyl-1H-pyrazol-3-yl)-3-(methylsulfonyl)pyridine (1.2 g, 4.4 mmol).
Yield: 97%

### Reference Production Example 9-5

Production of 6-(4-methyl-1H-pyrazol-3-yl)-5-(methylsulfonyl)picolinonitrile

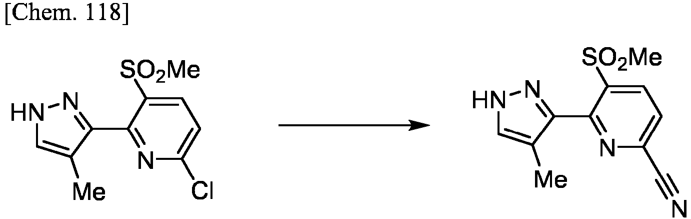

To a solution of 6-chloro-2-(4-methyl-1H-pyrazol-3-yl)-3-(methylsulfonyl)pyridine (1.4 g, 5.0 mmol) in dimethyl sulfoxide (25 mL), 1,4-diazabicyclo[2.2.2]octane (0.13 g, 1.1 mmol) was added at room temperature. Sodium cyanide (0.30 g, 6.0 mmol) dissolved in water (2.5 mL) was added gradually at room temperature, and the mixture was stirred at room temperature for 2 hours. After the completion of the reaction, water was added, and ethyl acetate extraction was performed. The organic layer was dried over anhydrous magnesium sulfate and then concentrated in vacuo. The residue was purified by silica gel column chromatography to give 6-(4-methyl-1H-pyrazol-3-yl)-5-(methylsulfonyl)picolinonitrile (0.68 g, 2.6 mmol).
Yield: 52%
Physical property: ¹H-NMR (CDCl₃): δ 8.70 (d, 1H), 7.86 (d, 1H), 7.52 (s, 1H), 3.47 (s, 3H), 2.23 (s, 3H)

### Reference Production Example 9-6

Production of (Z)-N'-ethoxy-6-(4-methyl-1H-pyrazol-3-yl)-5-(methylsulfonyl)picolinimidamide

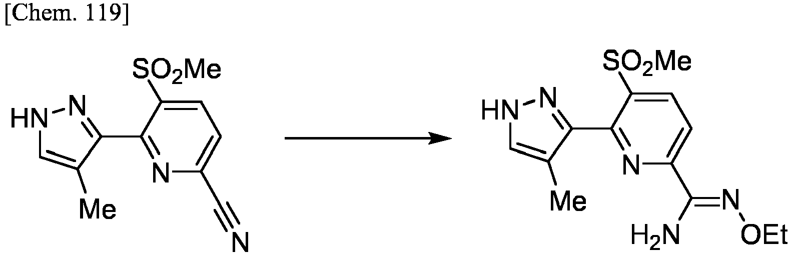

To a solution of 6-(4-methyl-1H-pyrazol-3-yl)-5-(methylsulfonyl)picolinonitrile (0.30 g, 1.1 mmol) in methanol (6.0 mL), sodium methoxide (40% solution in methanol, 0.14 mL, 1.4 mmol) was added, and the mixture was stirred at room temperature for 30 minutes. O-ethylhydroxylamine hydrochloride (0.14 g, 1.4 mmol) was added, and the mixture was stirred for 1 hour. After the completion of the reaction, water was added to the reaction mixture, and ethyl acetate extraction was performed. The organic layer was dried over anhydrous magnesium sulfate and then concentrated in vacuo. The residue was purified by silica gel column chromatography to give (Z)-N'-ethoxy-6-(4-methyl-1H-pyrazol-3-yl)-5-(methylsulfonyl)picolinimidamide (0.36 g, 1.1 mmol).
Yield: 98%
Physical property: ¹H-NMR (CDCl₃): δ 8.49 (d, 1H), 8.15 (d, 1H), 7.54 (s, 1H), 5.51 (s, 2H), 4.23 (q, 2H), 3.24 (s, 3H), 2.20 (s, 3H), 1.35 (t, 3H)

### Reference Production Example 10

Production of (Z)-N'-ethoxy-6-(5-methyl-2H-1,2,3-triazol-4-yl)-5-(methylsulfonyl)picolinimidamide (starting compound for Production Example 15)

### Reference Production Example 10-1

Production of (Z)-N'-ethoxy-5-(N-methylsulfonyl)-6-(prop-1-yn-1-yl)picolinimidamide

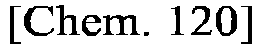

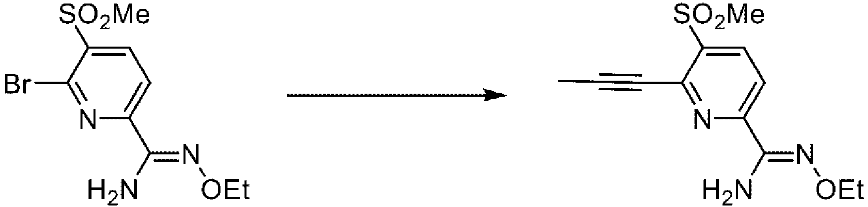

To a solution of (Z)-6-bromo-N'-ethoxy-5-(N-methylsulfonyl)picolinimidamide (0.40 g, 1.3 mmol) in triethyl (6.0 mL), a 5% N,N-dimethylacetamide solution (5.0 mL) of propine, dichlorobis(triphenylphosphine)palladium (44 mg, 0.063 mmol), and copper iodide (9.5 mg, 0.025 mmol) were added, and the mixture was stirred under an argon atmosphere at 75°C for 7 hours. After the disappearance of the starting compound was confirmed, the reaction mixture was cooled to room temperature, and ethyl acetate and water were added for extraction. The organic layer was concentrated in vacuo, and the residue was purified by silica gel chromatography to give (Z)-N'-ethoxy-5-(N-methylsulfonyl)-6-(prop-1-yn-1-yl)picolinimidamide (0.35 g, 1.2 mmol).
Yield: 99%
Physical property: ¹H-NMR (CDCl₃): δ 8.30 (d, 1H), 8.07 (d, 1H), 5.53 (brs, 2H), 4.20 (q, 2H), 3.32 (s, 3H), 2.24 (s, 3H), 1.34 (t, 3H)

### Reference Production Example 10-2

Production of (Z)-N'-ethoxy-6-(5-methyl-2H-1,2,3-triazol-4-yl)-5-(methylsulfonyl)picolinimidamide

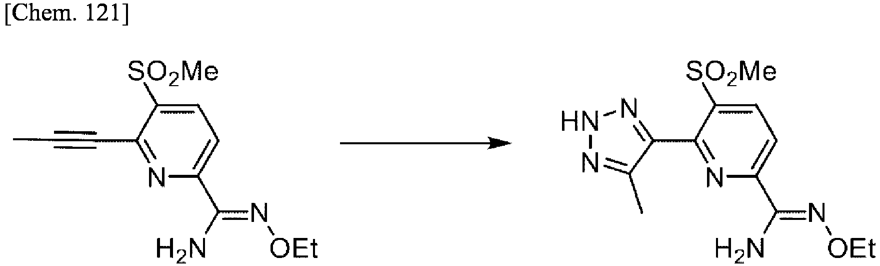

To a solution of (Z)-N'-ethoxy-5-(N-methylsulfonyl)-6-(prop-1-yn-1-yl)picolinimidamide (1.9 g, 6.6 mmol) in N,N-dimethylacetamide (67 mL), sodium azide (0.48 g, 7.4 mmol) and diacetoxy iodobenzene (2.6 g, 8.1 mmol) were added, and the mixture was stirred at 75°C for 5 hours. The reaction mixture was concentrated in vacuo, and the residue was purified by silica gel chromatography to give (Z)-N'-ethoxy-6-(5-methyl-2H-1,2,3-triazol-4-yl)-5-(methylsulfonyl)picolinimidamide (0.87 g, 2.7 mmol).
Yield: 40%
Physical property: Melting point: 126 to 130°C

Hereinafter, examples of formulations containing the compound of the present invention are shown, but the present invention is not limited thereto. In the formulation examples, "part" means part by weight.

### Formulation Example 1

| | |
|---|---|
| Compound of the present invention | 10 parts |
| Xylene | 70 parts |
| N-methylpyrrolidone | 10 parts |
| Mixture of polyoxyethylene nonylphenyl ether and calcium alkylbenzene sulfonate | 10 parts |

The above ingredients are uniformly mixed for dissolution to give an emulsifiable concentrate formulation.

### Formulation Example 2

| | |
|---|---|
| Compound of the present invention | 3 parts |
| Clay powder | 82 parts |
| Diatomite powder | 15 parts |

The above ingredients are uniformly mixed and then pulverized to give a dust formulation.

### Formulation Example 3

| | |
|---|---|
| Compound of the present invention | 5 parts |
| Mixture of bentonite powder and clay powder | 90 parts |
| Calcium lignosulfonate | 5 parts |

The above ingredients are uniformly mixed. After addition of an appropriate volume of water, the mixture is kneaded, granulated and dried to give a granular formulation.

### Formulation Example 4

| | |
|---|---|
| Compound of the present invention | 20 parts |
| Kaolin and synthetic high-dispersion silicic acid | 75 parts |
| Mixture of polyoxyethylene nonylphenyl ether and calcium alkylbenzene sulfonate | 5 parts |

The above ingredients are uniformly mixed and then pulverized to give a wettable powder formulation.

### Test Example 1: Test for post-emergence herbicidal effect against paddy weeds

Barnyard grass (*Echinochloa crus-galli*) was seeded and grown in test tubes containing hydroponic medium in an artificial climate chamber or a phytotron. Agrochemical formulations containing the compounds of the present invention as active ingredients prepared according to Formulation Example 1 were separately diluted with water so that the concentration of the active ingredient would be a predetermined concentration and used for drop treatment of the barnyard grass. The barnyard grass was grown in the artificial climate chamber at 30°C under full light conditions. Six days after agrochemical treatment, the herbicidal effect was evaluated as compared to the non-treatment group according to the following criteria.

### Criteria for herbicidal effect (degree of growth inhibition) and phytotoxicity

| | |
|---|---|
| Score 4 | 90% to 100% herbicidal effect |
| Score 3 | 70% to 89% herbicidal effect |
| Score 2 | 40% to 69% herbicidal effect |
| Score 1 | 1% to 39% herbicidal effect |
| Score 0 | 0% herbicidal effect |

As a result of Test Example 1, among the compounds represented by the general formula (1) of the present invention, compounds numbered 1-1, 1-2, 1-3, 1-4, 1-5, 1-6, 1-7, 1-8, 1-9, 1-11, 1-13, 1-14, 1-15, 1-17, 1-18, 1-19, 1-20, 1-21, 1-22, 1-23, 1-24, 1-26, 1-27, 1-28, 1-29, 1-30, 1-31, 1-32, 1-33, 1-34, 1-35, 1-36, 1-37, 1-38, 1-39, 1-40, 1-41, 1-42, 1-43, 1-44, 1-45, 1-46, 1-47, 1-48, 1-49, 1-50, 1-51, 1-53, 1-142, 1-143, 1-144, 1-145, 1-154, 1-186, 1-188, 1-189, 1-192, 1-197, 1-198, 1-200, 1-201, 1-206, 1-207, 1-209, 1-210, 1-211, 1-212, 1-220, 1-224, 1-225, 1-226, 1-227, 1-228, 1-229, 1-230, 1-233, 1-234, 1-237, 1-248, 1-249, 1-269, 1-270, 1-284, 1-285, 1-287, 1-288, 1-289, 1-290, 1-292, 1-293, 1-294, 1-295, 1-296, 1-299, 1-358, 1-359, 2-1, 2-2, 2-3, 2-4, 2-5, 2-6, 2-9, 2-10, 2-11, 2-12, 2-13, 2-14, 2-15, 2-18, 2-19, 2-21, 2-22, 2-23, 2-24, 2-26, 2-27, 2-28, 2-29, 2-31, 2-32, 2-33, 2-35, 2-38, 3-1, 3-2, 3-3, 3-4, 3-5, 3-6, 3-7, 3-8, 3-9, 3-10, 3-11, 3-12, 3-14, 3-15, 3-16, 3-17, 3-18, 3-19, 3-20, 3-21, 3-114, 3-115, 3-116, 3-117, 3-118, 3-119, 3-120, 3-121, 3-123, 3-124, 3-125, 3-168, 3-171, 3-176, 3-177, 3-179, 3-180, 3-191, 3-192, 3-193, 3-195, 3-197, 3-198, 3-200, 3-201, 3-203, 3-204, 3-205, 3-206, 3-208, 3-209, 3-229, 3-230, 3-250, 3-251, 3-265, 3-267, 3-268, 3-269, 3-270, 3-271, 3-272, 3-273, 3-274, 3-278, 3-279, 3-324, 3-377, 3-378, 4-1, 4-2, 4-3, 4-5, 4-6, 4-8, 4-9, 4-10, 4-12, 4-13, 4-14, 4-15, 4-16, 4-17, 4-19, 4-24, 4-52, 4-77, 4-78, 4-115, 4-131, 4-132, 4-134, 4-135, 4-149, 4-151, 4-152, 4-157, 4-158, 4-159, 4-160, 4-161, 4-162, 4-165, 4-168, 4-169, 4-171, 4-180, 4-181, 4-182, 4-183, 4-185, 4-187, 4-188, 4-189, 4-237, 4-238, 4-239, 4-240, 4-241, 4-242, 4-243, 4-247, 4-248, 4-249, 4-250, 4-254, 4-282, 4-283, 4-303, 4-304, 4-305, 4-306, 4-307, 4-308, 4-309, 5-1, 5-3, 5-21, 6-1, 7-1, 7-18, 7-20, 7-21, 7-22, 8-2, 8-17, 10-2, 10-16, 10-17, 10-18, 10-20, 11-95, 11-214, 12-2, 17-1, 17-2, 17-3, 17-6, 19-29, 20-17, 20-20, 20-62, 20-66, and 21-2 showed herbicidal effect against barnyard grass in score 3 or higher according to the above criteria when applied at an active ingredient concentration of 10 ppm.

### INDUSTRIAL APPLICABILITY

The nitrogen-containing heterocyclic compound of the present invention or a salt thereof is a highly effective agricultural or horticultural herbicide.

## Claims

1. A compound represented by the general formula (1): 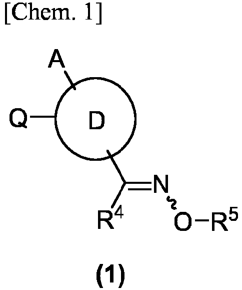 {wherein
Q represents a group represented by the following Q1, Q2, Q3, Q4, Q5, Q6, Q7, Q8, Q9, Q10, Q11, Q12, Q13, Q14, Q15, Q16, Q17, or Q18: 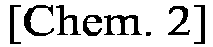 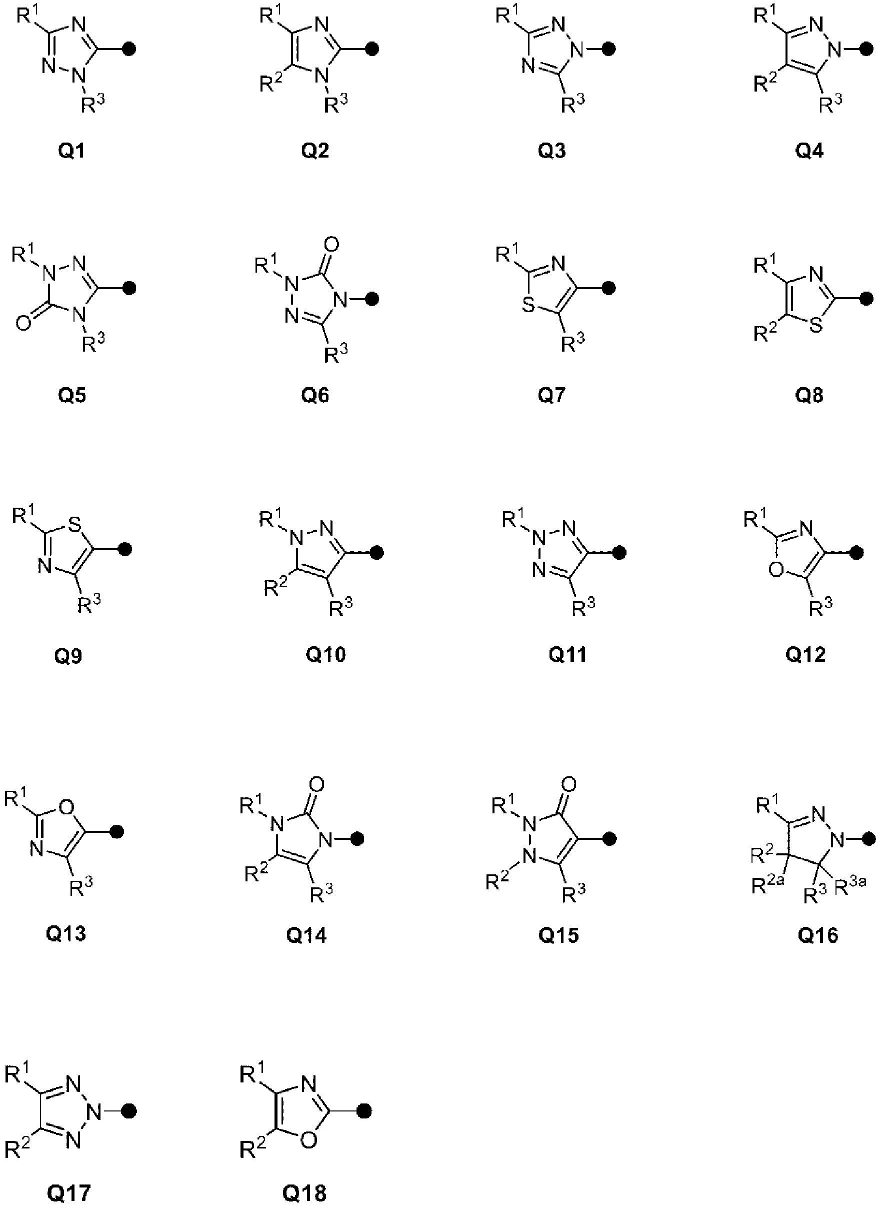 (wherein
R¹ represents
(a1) a halogen atom;
(a2) a (C₁-C₆) alkyl group;
(a3) a (C₂-C₆) alkenyl group;
(a4) a (C₂-C₆) alkynyl group;
(a5) a (C₃-C₆) cycloalkyl group;
(a6) a halo (C₁-C₆) alkyl group;
(a7) a halo (C₂-C₆) alkenyl group;
(a8) a halo (C₂-C₆) alkynyl group;
(a9) a halo (C₃-C₆) cycloalkyl group;
(a10) a dioxolanyl group;
(a11) a dioxanyl group;
(a12) a dioxepanyl group;
(a13) a dihydropyranyl group;
(a14) a tetrahydropyranyl group;
(a15) a tetrahydrothiopyranyl group;
(a16) a piperidinyl group;
(a17) a substituted piperidinyl group having 1 to 3 substituents on the ring, each independently selected from the group consisting of a (C₁-C₆) alkyl group, a (C₁-C₆) alkylcarbonyl group, and a (C₁-C₆) alkoxycarbonyl group;
(a18) a thienyl group;
(a19) a substituted thienyl group having 1 to 3 substituents on the ring, each independently selected from a set Y of substituents;
(a20) a thiazolyl group;
(a21) a substituted thiazolyl group having 1 or 2 substituents on the ring, each independently selected from the set Y of substituents;
(a22) a thiadiazolyl group;
(a23) a substituted thiadiazolyl group having one substituent on the ring, selected from the set Y of substituents;
(a24) a pyrazolyl group;
(a25) a substituted pyrazolyl group having 1 to 3 substituents on the ring, each independently selected from the set Y of substituents;
(a26) a phenyl group;
(a27) a substituted phenyl group having 1 to 5 substituents on the ring, each independently selected from the set Y of substituents;
(a28) a pyridyl group;
(a29) a substituted pyridyl group having 1 to 4 substituents on the ring, each independently selected from the set Y of substituents;
(a30) a pyridazinyl group;
(a31) a substituted pyridazinyl group having 1 to 3 substituents on the ring, each independently selected from the set Y of substituents;
(a32) a pyrimidinyl group;
(a33) a substituted pyrimidinyl group having 1 to 3 substituents on the ring, each independently selected from the set Y of substituents;
(a34) a pyrazinyl group;
(a35) a substituted pyrazinyl group having 1 to 3 substituents on the ring, each independently selected from the set Y of substituents;
(a36) an amino group;
(a37) a carboxyl group;
(a38) a (C₁-C₆) alkoxy group;
(a39) a halo (C₁-C₆) alkoxy group;
(a40) a (C₁-C₆) alkylsulfanyl group;
(a41) a (C₁-C₆) alkylsulfinyl group;
(a42) a (C₁-C₆) alkylsulfonyl group;
(a43) a (C₃-C₆) cycloalkenyl group;
(a44) a (C₁-C₆) alkoxy (C₁-C₆) alkyl group;
(a45) a (C₁-C₆) alkylsulfanyl (C₁-C₆) alkyl group;
(a46) a (C₁-C₆) alkylsulfinyl (C₁-C₆) alkyl group;
(a47) a (C₁-C₆) alkylsulfonyl (C₁-C₆) alkyl group;
(a48) a (C₁-C₆) alkylcarbonyl group;
(a49) a (C₁-C₆) alkoxycarbonyl group;
(a50) a halo (C₁-C₆) alkylcarbonyl group;
(a51) a halo (C₁-C₆) alkoxycarbonyl group;
(a52) a phenylcarbonyl group;
(a53) a substituted phenylcarbonyl group having 1 to 5 substituents on the ring, each independently selected from the set Y of substituents;
(a54) a phenylaminocarbonyl group;
(a55) a substituted phenylaminocarbonyl group having 1 to 5 substituents on the ring, each independently selected from the set Y of substituents;
(a56) a benzhydrylidene amino group;
(a57) an oxopyrrolidinyl group;
(a58) an oxopyridyl group;
(a59) a substituted dioxolanyl group having 1 to 4 substituents on the ring, each independently selected from the group consisting of a halogen atom and a (C₁-C₆) alkyl group;
(a60) a substituted dioxanyl group having 1 to 4 substituents on the ring, each independently selected from the group consisting of a halogen atom and a (C₁-C₆) alkyl group;
(a61) a furanyl group;
(a62) a substituted furanyl group having 1 to 3 substituents on the ring, each independently selected from the set Y of substituents;
(a63) an isothiazolyl group;
(a64) a substituted isothiazolyl group having 1 or 2 substituents on the ring, each independently selected from the set Y of substituents;
(a65) an oxazolyl group;
(a66) a substituted oxazolyl group having 1 or 2 substituents on the ring, each independently selected from the set Y of substituents;
(a67) an isoxazolyl group;
(a68) a substituted isoxazolyl group having 1 or 2 substituents on the ring, each independently selected from the set Y of substituents;
(a69) a quinolinyl group;
(a70) a substituted quinolinyl group having 1 to 6 substituents on the ring, each independently selected from the set Y of substituents;
(a71) a benzothienyl group;
(a72) a substituted benzothienyl group having 1 to 5 substituents on the ring, each independently selected from the set Y of substituents;
(a73) a phenoxy group;
(a74) a substituted phenoxy group having 1 to 5 substituents on the ring, each independently selected from the set Y of substituents;
(a75) a phenyl (C₁-C₆) alkyl group;
(a76) a substituted phenyl (C₁-C₆) alkyl group having 1 to 5 substituents on the ring, each independently selected from the set Y of substituents;
(a77) a phenyl (C₁-C₆) alkoxy group;
(a78) a substituted phenyl (C₁-C₆) alkoxy group having 1 to 5 substituents on the ring, each independently selected from the set Y of substituents;
(a79) a (C₁-C₆) alkylamino group;
(a80) a halo (C₁-C₆) alkoxycarbonylamino group;
(a81) an oxazolidinonyl group;
(a82) a (C₁-C₆) alkoxy (C₁-C₆) alkoxy group;
(a83) a tetrahydrofuranyl (C₁-C₆) alkoxy group; or
(a84) a (C₃-C₆) cycloalkyl (C₁-C₆) alkoxy group,
R² represents
(b1) a hydrogen atom;
(b2) a halogen atom; or
(b3) a (C₁-C₆) alkyl group,
R^{2a} represents
(b1') a hydrogen atom; or
(b2') a (C₁-C₆) alkyl group,
R³ represents
(c1) a hydrogen atom;
(c2) a halogen atom;
(c3) a hydroxy group;
(c4) a (C₁-C₆) alkyl group;
(c5) a (C₂-C₆) alkenyl group;
(c6) a (C₂-C₆) alkynyl group;
(c7) a (C₃-C₆) cycloalkyl group;
(c8) a halo (C₁-C₆) alkyl group;
(c9) a halo (C₂-C₆) alkenyl group;
(c10) a halo (C₂-C₆) alkynyl group;
(c11) a halo (C₃-C₆) cycloalkyl group;
(c12) a (C₁-C₆) alkoxy group;
(c13) a (C₁-C₆) alkylsulfanyl group;
(c14) a (C₁-C₆) alkylsulfinyl group;
(c15) a (C₁-C₆) alkylsulfonyl group;
(c16) a halo (C₁-C₆) alkoxy group;
(c17) a halo (C₁-C₆) alkylsulfanyl group;
(c18) a halo (C₁-C₆) alkylsulfinyl group;
(c19) a halo (C₁-C₆) alkylsulfonyl group;
(c20) a (C₁-C₆) alkoxycarbonyl group; or
(c21) a (C₁-C₆) alkylamino group, or
R² and R³ may be bound to each other to form a 5- or 6-membered ring,
R^{3a} represents
(c1') a hydrogen atom; or
(c2') a (C₁-C₆) alkyl group,
the set Y of substituents consists of
(d1) a halogen atom;
(d2) a cyano group;
(d3) a nitro group;
(d4) an amino group;
(d5) a (C₁-C₆) alkyl group;
(d6) a (C₂-C₆) alkenyl group;
(d7) a (C₂-C₆) alkynyl group;
(d8) a (C₃-C₆) cycloalkyl group;
(d9) a halo (C₁-C₆) alkyl group;
(d10) a halo (C₂-C₆) alkenyl group;
(d11) a halo (C₂-C₆) alkynyl group;
(d12) a halo (C₃-C₆) cycloalkyl group;
(d13) a (C₁-C₆) alkoxy group;
(d14) a (C₁-C₆) alkylsulfanyl group;
(d15) a (C₁-C₆) alkylsulfinyl group;
(d16) a (C₁-C₆) alkylsulfonyl group;
(d17) a halo (C₁-C₆) alkoxy group;
(d18) a halo (C₁-C₆) alkylsulfanyl group;
(d19) a halo (C₁-C₆) alkylsulfinyl group;
(d20) a halo (C₁-C₆) alkylsulfonyl group;
(d21) an N-((C₁-C₆) alkylcarbonyl)amino group;
(d22) an N,N-di-((C₁-C₆) alkylcarbonyl)amino group wherein the (C₁-C₆) alkylcarbonyl groups may be the same or different;
(d23) a di-(C₁-C₆) alkoxyphosphanyl group wherein the (C₁-C₆) alkoxy groups may be the same or different;
(d24) an imidazolyl group;
(d25) an imidazolyl group having 1 to 3 substituents on the ring, each independently selected from the group consisting of a halogen atom and a (C₁-C₆) alkyl group;
(d26) a phenyl group;
(d27) a phenyl group having 1 to 5 substituents on the ring, each independently selected from the group consisting of a halogen atom and a (C₁-C₆) alkyl group;
(d28) a pyridyl group;
(d29) a pyridyl group having 1 to 4 substituents on the ring, each independently selected from the group consisting of a halogen atom and a (C₁-C₆) alkyl group;
(d30) a di-(C₁-C₆) alkylamino group;
(d31) a (C₁-C₆) alkoxycarbonyl group; and
(d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group, and
each solid circle represents the position of binding to D),
R⁴ represents
(e1) a hydrogen atom;
(e2) an amino group;
(e3) a (C₁-C₆) alkyl group;
(e4) an N-(C₁-C₆) alkylamino group;
(e5) an N,N-di-(C₁-C₆) alkylamino group wherein the (C₁-C₆) alkyl groups may be the same or different;
(e6) an N-((C₁-C₆) alkylcarbonyl)amino group;
(e7) an N,N-di-((C₁-C₆) alkylcarbonyl)amino group wherein the (C₁-C₆) alkylcarbonyl groups may be the same or different;
(e8) an N-(halo (C₁-C₆) alkylcarbonyl)amino group;
(e9) an N-((C₁-C₆) alkoxycarbonyl)amino group; or
(e10) an N-(C₁-C₆) alkyl-N-((C₁-C₆) alkylcarbonyl)amino group,
R⁵ represents
(f1) a (C₁-C₆) alkyl group;
(f2) a (C₂-C₆) alkenyl group;
(f3) a (C₂-C₆) alkynyl group;
(f4) a (C₃-C₆) cycloalkyl group;
(f5) a halo (C₁-C₆) alkyl group;
(f6) a halo (C₂-C₆) alkenyl group;
(f7) a halo (C₂-C₆) alkynyl group;
(f8) a halo (C₃-C₆) cycloalkyl group;
(f9) a (C₃-C₆) cycloalkyl (C₁-C₆) alkyl group;
(f10) a (C₁-C₆) alkoxy (C₁-C₆) alkyl group;
(f11) a (C₁-C₆) alkylsulfanyl (C₁-C₆) alkyl group;
(f12) a (C₁-C₆) alkylsulfinyl (C₁-C₆) alkyl group;
(f13) a (C₁-C₆) alkylsulfonyl (C₁-C₆) alkyl group; or
(f14) a phenyl (C₁-C₆) alkyl group,
A represents
(g1) a (C₁-C₆) alkoxy group;
(g2) a halo (C₁-C₆) alkoxy group;
(g3) an N-(C₁-C₆) alkylaminocarbonyl group;
(g4) an N,N-di-(C₁-C₆) alkylaminocarbonyl group wherein the (C₁-C₆) alkylcarbonyl groups may be the same or different;
(g5) CO₂R⁷ wherein R⁷ represents
(h1) a hydrogen atom,
(h2) a (C₁-C₆) alkyl group,
(h3) a (C₂-C₆) alkenyl group,
(h4) a (C₂-C₆) alkynyl group,
(h5) a (C₃-C₆) cycloalkyl group,
(h6) a halo (C₁-C₆) alkyl group,
(h7) a halo (C₂-C₆) alkenyl group,
(h8) a halo (C₂-C₆) alkynyl group,
(h9) a halo (C₃-C₆) cycloalkyl group, or
(h10) a phenyl (C₁-C₆) alkyl group;
(g6) SOₙR⁸ wherein R⁸ represents
(i1) a (C₁-C₆) alkyl group,
(i2) a (C₂-C₆) alkenyl group,
(i3) a (C₂-C₆) alkynyl group,
(i4) a (C₃-C₆) cycloalkyl group,
(i5) a halo (C₁-C₆) alkyl group,
(i6) a halo (C₂-C₆) alkenyl group,
(i7) a halo (C₂-C₆) alkynyl group,
(i8) a halo (C₃-C₆) cycloalkyl group, or
(i9) a (C₁-C₆) alkylcarbonyl (C₁-C₆) alkyl group, and
n represents 0, 1, or 2; or
(g7) SO₂N(R⁹)R¹⁰ wherein R⁹ and R¹⁰ represent
(j 1) a hydrogen atom,
(j2) a (C₁-C₆) alkyl group,
(j3) a (C₂-C₆) alkenyl group,
(j4) a (C₂-C₆) alkynyl group,
(j5) a (C₃-C₆) cycloalkyl group,
(j6) a halo (C₁-C₆) alkyl group,
(j7) a halo (C₂-C₆) alkenyl group,
(j8) a halo (C₂-C₆) alkynyl group,
(j9) a halo (C₃-C₆) cycloalkyl group,
(j10) a phenyl (C₁-C₆) alkyl group
(j 11) a (C₁-C₆) alkylcarbonyl group,
(j12) a (C₁-C₆) alkoxycarbonyl group,
(j13) a halo (C₁-C₆) alkylcarbonyl group, or
(j14) a halo (C₁-C₆) alkoxycarbonyl group, and
R⁹ and R¹⁰ may be the same or different, and
D substituted with A, Q, and C(R⁴)=N~OR⁵ represents a ring represented by the following D1, D2, D3, or D4: 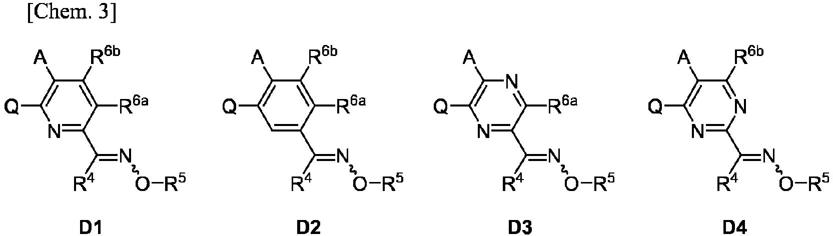 wherein R^{6a} and R^{6b} represent
(k1) a hydrogen atom;
(k2) a halogen atom; or
(k3) a (C₁-C₆) alkyl group, and
R^{6a} and R^{6b} may be the same or different}, or
a salt thereof.

2. The compound or the salt thereof according to claim 1, wherein
Q and D are as defined in claim 1,
R¹ is
(a1) a halogen atom;
(a2) a (C₁-C₆) alkyl group;
(a3) a (C₂-C₆) alkenyl group;
(a5) a (C₃-C₆) cycloalkyl group;
(a9) a halo (C₃-C₆) cycloalkyl group;
(a10) a dioxolanyl group;
(a11) a dioxanyl group;
(a12) a dioxepanyl group;
(a13) a dihydropyranyl group;
(a14) a tetrahydropyranyl group;
(a15) a tetrahydrothiopyranyl group;
(a16) a piperidinyl group;
(a17) a substituted piperidinyl group having 1 to 3 substituents on the ring, each independently selected from the group consisting of a (C₁-C₆) alkyl group, a (C₁-C₆) alkylcarbonyl group, and a (C₁-C₆) alkoxycarbonyl group;
(a18) a thienyl group;
(a19) a substituted thienyl group having 1 to 3 substituents on the ring, each independently selected from a set Y of substituents;
(a20) a thiazolyl group;
(a21) a substituted thiazolyl group having 1 or 2 substituents on the ring, each independently selected from the set Y of substituents;
(a22) a thiadiazolyl group;
(a23) a substituted thiadiazolyl group having one substituent on the ring, selected from the set Y of substituents;
(a24) a pyrazolyl group;
(a25) a substituted pyrazolyl group having 1 to 3 substituents on the ring, each independently selected from the set Y of substituents;
(a26) a phenyl group;
(a27) a substituted phenyl group having 1 to 5 substituents on the ring, each independently selected from the set Y of substituents;
(a28) a pyridyl group;
(a29) a substituted pyridyl group having 1 to 4 substituents on the ring, each independently selected from the set Y of substituents;
(a30) a pyridazinyl group;
(a31) a substituted pyridazinyl group having 1 to 3 substituents on the ring, each independently selected from the set Y of substituents;
(a32) a pyrimidinyl group;
(a33) a substituted pyrimidinyl group having 1 to 3 substituents on the ring, each independently selected from the set Y of substituents;
(a34) a pyrazinyl group;
(a35) a substituted pyrazinyl group having 1 to 3 substituents on the ring, each independently selected from the set Y of substituents;
(a36) an amino group;
(a37) a carboxyl group;
(a38) a (C₁-C₆) alkoxy group;
(a39) a halo (C₁-C₆) alkoxy group;
(a40) a (C₁-C₆) alkylsulfanyl group;
(a41) a (C₁-C₆) alkylsulfinyl group;
(a42) a (C₁-C₆) alkylsulfonyl group;
(a43) a (C₃-C₆) cycloalkenyl group;
(a44) a (C₁-C₆) alkoxy (C₁-C₆) alkyl group;
(a45) a (C₁-C₆) alkylsulfanyl (C₁-C₆) alkyl group;
(a46) a (C₁-C₆) alkylsulfinyl (C₁-C₆) alkyl group;
(a47) a (C₁-C₆) alkylsulfonyl (C₁-C₆) alkyl group;
(a48) a (C₁-C₆) alkylcarbonyl group;
(a49) a (C₁-C₆) alkoxycarbonyl group;
(a50) a halo (C₁-C₆) alkylcarbonyl group;
(a51) a halo (C₁-C₆) alkoxycarbonyl group;
(a52) a phenylcarbonyl group;
(a53) a substituted phenylcarbonyl group having 1 to 5 substituents on the ring, each independently selected from the set Y of substituents;
(a54) a phenylaminocarbonyl group;
(a55) a substituted phenylaminocarbonyl group having 1 to 5 substituents on the ring, each independently selected from the set Y of substituents;
(a56) a benzhydrylidene amino group;
(a57) an oxopyrrolidinyl group;
(a58) an oxopyridyl group;
(a59) a substituted dioxolanyl group having 1 to 4 substituents on the ring, each independently selected from the group consisting of a halogen atom and a (C₁-C₆) alkyl group;
(a60) a substituted dioxanyl group having 1 to 4 substituents on the ring, each independently selected from the group consisting of a halogen atom and a (C₁-C₆) alkyl group;
(a61) a furanyl group;
(a62) a substituted furanyl group having 1 to 3 substituents on the ring, each independently selected from the set Y of substituents;
(a63) an isothiazolyl group;
(a64) a substituted isothiazolyl group having 1 or 2 substituents on the ring, each independently selected from the set Y of substituents;
(a69) a quinolinyl group;
(a70) a substituted quinolinyl group having 1 to 6 substituents on the ring, each independently selected from the set Y of substituents;
(a71) a benzothienyl group;
(a72) a substituted benzothienyl group having 1 to 5 substituents on the ring, each independently selected from the set Y of substituents;
(a73) a phenoxy group;
(a74) a substituted phenoxy group having 1 to 5 substituents on the ring, each independently selected from the set Y of substituents;
(a75) a phenyl (C₁-C₆) alkyl group;
(a76) a substituted phenyl (C₁-C₆) alkyl group having 1 to 5 substituents on the ring, each independently selected from the set Y of substituents;
(a77) a phenyl (C₁-C₆) alkoxy group;
(a78) a substituted phenyl (C₁-C₆) alkoxy group having 1 to 5 substituents on the ring, each independently selected from the set Y of substituents;
(a79) a (C₁-C₆) alkylamino group;
(a80) a halo (C₁-C₆) alkoxycarbonylamino group;
(a81) an oxazolidinonyl group;
(a82) a (C₁-C₆) alkoxy (C₁-C₆) alkoxy group;
(a83) a tetrahydrofuranyl (C₁-C₆) alkoxy group; or
(a84) a (C₃-C₆) cycloalkyl (C₁-C₆) alkoxy group,
R² is
(b1) a hydrogen atom;
(b2) a halogen atom; or
(b3) a (C₁-C₆) alkyl group,
R^{2a} is
(b1') a hydrogen atom; or
(b2') a (C₁-C₆) alkyl group,
R³ is
(c1) a hydrogen atom;
(c2) a halogen atom;
(c3) a hydroxy group;
(c4) a (C₁-C₆) alkyl group;
(c5) a (C₂-C₆) alkenyl group;
(c6) a (C₂-C₆) alkynyl group;
(c7) a (C₃-C₆) cycloalkyl group;
(c8) a halo (C₁-C₆) alkyl group;
(c12) a (C₁-C₆) alkoxy group;
(c13) a (C₁-C₆) alkylsulfanyl group;
(c14) a (C₁-C₆) alkylsulfinyl group;
(c15) a (C₁-C₆) alkylsulfonyl group;
(c16) a halo (C₁-C₆) alkoxy group;
(c17) a halo (C₁-C₆) alkylsulfanyl group;
(c18) a halo (C₁-C₆) alkylsulfinyl group;
(c19) a halo (C₁-C₆) alkylsulfonyl group;
(c20) a (C₁-C₆) alkoxycarbonyl group; or
(c21) a (C₁-C₆) alkylamino group, or
R² and R³ may be bound to each other to form a 5- or 6-membered ring,
R^{3a} is
(c1') a hydrogen atom; or
(c2') a (C₁-C₆) alkyl group,
the set Y of substituents consists of
(d1) a halogen atom;
(d2) a cyano group;
(d3) a nitro group;
(d4) an amino group;
(d5) a (C₁-C₆) alkyl group;
(d8) a (C₃-C₆) cycloalkyl group;
(d9) a halo (C₁-C₆) alkyl group;
(d13) a (C₁-C₆) alkoxy group;
(d14) a (C₁-C₆) alkylsulfanyl group;
(d15) a (C₁-C₆) alkylsulfinyl group;
(d16) a (C₁-C₆) alkylsulfonyl group;
(d17) a halo (C₁-C₆) alkoxy group;
(d18) a halo (C₁-C₆) alkylsulfanyl group;
(d19) a halo (C₁-C₆) alkylsulfinyl group;
(d20) a halo (C₁-C₆) alkylsulfonyl group;
(d21) an N-((C₁-C₆) alkylcarbonyl)amino group;
(d22) an N,N-di-((C₁-C₆) alkylcarbonyl)amino group wherein the (C₁-C₆) alkylcarbonyl groups may be the same or different;
(d23) a di-(C₁-C₆) alkoxyphosphanyl group wherein the (C₁-C₆) alkoxy groups may be the same or different;
(d24) an imidazolyl group;
(d25) an imidazolyl group having 1 to 3 substituents on the ring, each independently selected from the group consisting of a halogen atom and a (C₁-C₆) alkyl group;
(d26) a phenyl group;
(d27) a phenyl group having 1 to 5 substituents on the ring, each independently selected from the group consisting of a halogen atom and a (C₁-C₆) alkyl group;
(d28) a pyridyl group;
(d29) a pyridyl group having 1 to 4 substituents on the ring, each independently selected from the group consisting of a halogen atom and a (C₁-C₆) alkyl group;
(d30) a di-(C₁-C₆) alkylamino group;
(d31) a (C₁-C₆) alkoxycarbonyl group; and
(d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group,
R⁴ is
(e1) a hydrogen atom;
(e2) an amino group;
(e3) a (C₁-C₆) alkyl group;
(e4) an N-(C₁-C₆) alkylamino group;
(e5) an N,N-di-(C₁-C₆) alkylamino group wherein the (C₁-C₆) alkyl groups may be the same or different;
(e6) an N-((C₁-C₆) alkylcarbonyl)amino group;
(e7) an N,N-di-((C₁-C₆) alkylcarbonyl)amino group wherein the (C₁-C₆) alkylcarbonyl groups may be the same or different; or
(e8) an N-(halo (C₁-C₆) alkylcarbonyl)amino group,
R⁵ is
(f1) a (C₁-C₆) alkyl group;
(f2) a (C₂-C₆) alkenyl group;
(f3) a (C₂-C₆) alkynyl group;
(f4) a (C₃-C₆) cycloalkyl group;
(f5) a halo (C₁-C₆) alkyl group;
(f9) a (C₃-C₆) cycloalkyl (C₁-C₆) alkyl group;
(f10) a (C₁-C₆) alkoxy (C₁-C₆) alkyl group;
(f11) a (C₁-C₆) alkylsulfanyl (C₁-C₆) alkyl group;
(f12) a (C₁-C₆) alkylsulfinyl (C₁-C₆) alkyl group; or
(f13) a (C₁-C₆) alkylsulfonyl (C₁-C₆) alkyl group,
A is
(g1) a (C₁-C₆) alkoxy group;
(g3) an N-(C₁-C₆) alkylaminocarbonyl group;
(g4) an N,N-di-(C₁-C₆) alkylaminocarbonyl group wherein the (C₁-C₆) alkylcarbonyl groups may be the same or different;
(g5') CO₂R⁷ wherein R⁷ represents
(h1) a hydrogen atom,
(h2) a (C₁-C₆) alkyl group,
(h3) a (C₂-C₆) alkenyl group,
(h4) a (C₂-C₆) alkynyl group,
(h5) a (C₃-C₆) cycloalkyl group,
(h6) a halo (C₁-C₆) alkyl group, or
(h10) a phenyl (C₁-C₆) alkyl group;
(g6') SOₙR⁸ wherein R⁸ represents
(i1) a (C₁-C₆) alkyl group,
(i2) a (C₂-C₆) alkenyl group,
(i3) a (C₂-C₆) alkynyl group,
(i4) a (C₃-C₆) cycloalkyl group,
(i5) a halo (C₁-C₆) alkyl group, or
(i9) a (C₁-C₆) alkylcarbonyl (C₁-C₆) alkyl group, and
n represents 0, 1, or 2; or
(g7') SO₂N(R⁹)R¹⁰ wherein R⁹ and R¹⁰ represent
(j 1) a hydrogen atom,
(j2) a (C₁-C₆) alkyl group,
(j3) a (C₂-C₆) alkenyl group,
(j4) a (C₂-C₆) alkynyl group,
(j5) a (C₃-C₆) cycloalkyl group,
(j 11) a (C₁-C₆) alkylcarbonyl group,
(j12) a (C₁-C₆) alkoxycarbonyl group,
(j13) a halo (C₁-C₆) alkylcarbonyl group, or
(j14) a halo (C₁-C₆) alkoxycarbonyl group, and
R⁹ and R¹⁰ may be the same or different, and
R^{6a} and R^{6b} are each
(k1) a hydrogen atom;
(k2) a halogen atom; or
(k3) a (C₁-C₆) alkyl group, and
R^{6a} and R^{6b} may be the same or different.

3. The compound or the salt thereof according to claim 1, wherein
Q and D are as defined in claim 1,
R¹ is
(a1) a halogen atom;
(a2) a (C₁-C₆) alkyl group;
(a3) a (C₂-C₆) alkenyl group;
(a5) a (C₃-C₆) cycloalkyl group;
(a13) a dihydropyranyl group;
(a14) a tetrahydropyranyl group;
(a16) a piperidinyl group;
(a18) a thienyl group;
(a19) a substituted thienyl group having 1 to 3 substituents on the ring, each independently selected from a set Y of substituents;
(a20) a thiazolyl group;
(a21) a substituted thiazolyl group having 1 or 2 substituents on the ring, each independently selected from the set Y of substituents;
(a22) a thiadiazolyl group;
(a23) a substituted thiadiazolyl group having one substituent on the ring, selected from the set Y of substituents;
(a24) a pyrazolyl group;
(a25) a substituted pyrazolyl group having 1 to 3 substituents on the ring, each independently selected from a set Y of substituents;
(a26) a phenyl group;
(a27) a substituted phenyl group having 1 to 5 substituents on the ring, each independently selected from the set Y of substituents;
(a28) a pyridyl group;
(a29) a substituted pyridyl group having 1 to 4 substituents on the ring, each independently selected from the set Y of substituents;
(a32) a pyrimidinyl group;
(a34) a pyrazinyl group;
(a36) an amino group;
(a37) a carboxyl group;
(a38) a (C₁-C₆) alkoxy group;
(a39) a halo (C₁-C₆) alkoxy group;
(a40) a (C₁-C₆) alkylsulfanyl group;
(a42) a (C₁-C₆) alkylsulfonyl group;
(a43) a (C₃-C₆) cycloalkenyl group;
(a44) a (C₁-C₆) alkoxy (C₁-C₆) alkyl group;
(a45) a (C₁-C₆) alkylsulfanyl (C₁-C₆) alkyl group;
(a46) a (C₁-C₆) alkylsulfinyl (C₁-C₆) alkyl group;
(a47) a (C₁-C₆) alkylsulfonyl (C₁-C₆) alkyl group;
(a48) a (C₁-C₆) alkylcarbonyl group;
(a49) a (C₁-C₆) alkoxycarbonyl group;
(a52) a phenylcarbonyl group;
(a54) a phenylaminocarbonyl group;
(a56) a benzhydrylidene amino group;
(a57) an oxopyrrolidinyl group;
(a58) an oxopyridyl group;
(a59) a substituted dioxolanyl group having 1 to 4 substituents on the ring, each independently selected from the group consisting of a halogen atom and a (C₁-C₆) alkyl group;
(a60) a substituted dioxanyl group having 1 to 4 substituents on the ring, each independently selected from the group consisting of a halogen atom and a (C₁-C₆) alkyl group;
(a61) a furanyl group;
(a63) an isothiazolyl group;
(a64) a substituted isothiazolyl group having 1 or 2 substituents on the ring, each independently selected from the set Y of substituents;
(a69) a quinolinyl group;
(a71) a benzothienyl group;
(a74) a substituted phenoxy group having 1 to 5 substituents on the ring, each independently selected from the set Y of substituents;
(a75) a phenyl (C₁-C₆) alkyl group;
(a82) a (C₁-C₆) alkoxy (C₁-C₆) alkoxy group;
(a83) a tetrahydrofuranyl (C₁-C₆) alkoxy group; or
(a84) a (C₃-C₆) cycloalkyl (C₁-C₆) alkoxy group,
R² is
(b1) a hydrogen atom;
(b2) a halogen atom; or
(b3) a (C₁-C₆) alkyl group,
R^{2a} is
(b1') a hydrogen atom,
R³ is
(c1) a hydrogen atom;
(c2) a halogen atom;
(c3) a hydroxy group;
(c4) a (C₁-C₆) alkyl group;
(c5) a (C₂-C₆) alkenyl group;
(c8) a halo (C₁-C₆) alkyl group;
(c12) a (C₁-C₆) alkoxy group;
(c13) a (C₁-C₆) alkylsulfanyl group;
(c14) a (C₁-C₆) alkylsulfinyl group;
(c15) a (C₁-C₆) alkylsulfonyl group; or
(c20) a (C₁-C₆) alkoxycarbonyl group, or
R² and R³ may be bound to each other to form a 5- or 6-membered ring,
R^{3a} is
(c1') a hydrogen atom,
the set Y of substituents consists of
(d1) a halogen atom;
(d2) a cyano group;
(d3) a nitro group;
(d4) an amino group;
(d5) a (C₁-C₆) alkyl group;
(d8) a (C₃-C₆) cycloalkyl group;
(d9) a halo (C₁-C₆) alkyl group;
(d13) a (C₁-C₆) alkoxy group;
(d14) a (C₁-C₆) alkylsulfanyl group;
(d17) a halo (C₁-C₆) alkoxy group;
(d21) an N-((C₁-C₆) alkylcarbonyl)amino group;
(d24) an imidazolyl group;
(d26) a phenyl group;
(d28) a pyridyl group;
(d30) a di-(C₁-C₆) alkylamino group;
(d31) a (C₁-C₆) alkoxycarbonyl group; and
(d32) a methylenedioxy group which is formed from two substituents from the set Y adjacently bound to each other and may be substituted by 1 or 2 substituents selected from the group consisting of a halogen atom, a phenyl group, and a (C₁-C₆) alkyl group,
R⁴ is
(e2) an amino group;
(e3) a (C₁-C₆) alkyl group;
(e6) an N-((C₁-C₆) alkylcarbonyl)amino group;
(e7) an N,N-di-((C₁-C₆) alkylcarbonyl)amino group wherein the (C₁-C₆) alkylcarbonyl groups may be the same or different; or
(e8) an N-(halo (C₁-C₆) alkylcarbonyl)amino group,
R⁵ is
(f1) a (C₁-C₆) alkyl group;
(f5) a halo (C₁-C₆) alkyl group; or
(f10) a (C₁-C₆) alkoxy (C₁-C₆) alkyl group,
A is
(g1) a (C₁-C₆) alkoxy group;
(g4) an N,N-di-(C₁-C₆) alkylaminocarbonyl group wherein the (C₁-C₆) alkylcarbonyl groups may be the same or different;
(g5") CO₂R⁷ wherein R⁷ represents
(h1) a hydrogen atom, or
(h2) a (C₁-C₆) alkyl group;
(g6") SOₙR⁸ wherein R⁸ represents
(i1) a (C₁-C₆) alkyl group, or
(i9) a (C₁-C₆) alkylcarbonyl (C₁-C₆) alkyl group, and
n represents 2; or
(g7") SO₂N(R⁹)R¹⁰ wherein R⁹ and R¹⁰ represent
(j 1) a hydrogen atom,
(j2) a (C₁-C₆) alkyl group,
(j 11) a (C₁-C₆) alkylcarbonyl group, or
(j12) a (C₁-C₆) alkoxycarbonyl group, and
R⁹ and R¹⁰ may be the same or different, and
R^{6a} and R^{6b} are each
(k1) a hydrogen atom.

4. The compound or the salt thereof according to claim 1, wherein
R¹, R², R³, R⁴, R⁵, R^{6a}, R^{6b}, A, and the set Y of substituents are as defined in claim 1,
Q is Q1, Q2, Q3, Q4, Q5, Q6, Q7, Q8, Q9, or Q10, and
D is D1 or D2.

5. The compound or the salt thereof according to claim 1, wherein
R¹, R², R³, R⁴, R⁵, R^{6a}, R^{6b}, A, and the set Y of substituents are as defined in claim 1,
Q is Q1, Q2, Q3, Q4, Q5, Q6, Q7, Q8, or Q10, and
D is D1.

6. The compound or the salt thereof according to claim 2, wherein
R¹, R², R³, R⁴, R⁵, R^{6a}, R^{6b}, A, and the set Y of substituents are as defined in claim 2,
Q is Q1, Q2, Q3, Q4, Q5, Q6, Q7, Q8, Q9, or Q10, and
D is D1 or D2.

7. The compound or the salt thereof according to claim 2, wherein
R¹, R², R³, R⁴, R⁵, R^{6a}, R^{6b}, A, and the set Y of substituents are as defined in claim 2,
Q is Q1, Q2, Q3, Q4, Q5, Q6, Q7, Q8, or Q10, and
D is D1.

8. The compound or the salt thereof according to claim 3, wherein
R¹, R², R³, R⁴, R⁵, R^{6a}, R^{6b}, A, and the set Y of substituents are as defined in claim 3,
Q is Q1, Q2, Q3, Q4, Q5, Q6, Q7, Q8, Q9, or Q10, and
D is D1 or D2.

9. The compound or the salt thereof according to claim 3, wherein
R¹, R², R³, R⁴, R⁵, R^{6a}, R^{6b}, A, and the set Y of substituents are as defined in claim 3,
Q is Q1, Q2, Q3, Q4, Q5, Q6, Q7, Q8, or Q10, and
D is D1.

10. An agricultural or horticultural herbicide comprising the compound or the salt thereof according to any one of claims 1 to 9 as an active ingredient.

11. A method for using an agricultural or horticultural herbicide, comprising treating weeds, soil, paddy fields, or growing media with an effective amount of the agricultural or horticultural herbicide according to claim 10.

12. A method for controlling weeds, comprising treating weeds, soil, paddy fields, or growing media with an effective amount of the agricultural or horticultural herbicide according to claim 10.
